# EUROPEAN PATENT SPECIFICATION

(11) **EP 4 192 814 B1**
(45) Date of publication and mention of the grant of the patent: **07.08.2024**
(21) Application number: 22741488.5
(22) Date of filing: 30.06.2022
(51) Int. Cl.: C07D 213/75, C07D 401/12, C07D 405/12, C07D 409/12, C07D 413/12, C07D 417/12, C07D 453/02, C07D 513/04, A61K 31/443, A61P 37/02, C07D 471/04

(54) **INHIBITORS OF TRANSGLUTAMINASES**
TRANSGLUTAMINASE-HEMMER
INHIBITEURS DE TRANSGLUTAMINASES

(30) Priority: 30.06.2021 EP 21182956; 01.07.2021 EP 21183316; 02.07.2021 US 202163217783 P; 17.12.2021 WO PCT/EP2021/086674; 07.06.2022 WO PCT/EP2022/065435
(43) Date of publication of application: 14.06.2023
(73) Proprietor: Zedira GmbH, 64293 Darmstadt (DE)
(72) Inventor: PASTERNACK, Ralf, 64347 Griesheim (DE); BÜCHOLD, Christian, 61184 Karben (DE); HILS, Martin, 64295 Darmstadt (DE); STIELER, Martin, 64285 Darmstadt (DE); GERLACH, Uwe, 65795 Hattersheim (DE)
(74) Representative: Arth, Hans-Lothar
(86) International application number: PCT/EP2022/068217
(87) International publication number: WO 2023/275337

(56) References cited:
- WO-A1-2018/122419
- US-B2- 11 072 634
- US-B2- 9 434 763
- SUBBAIAH MURUGAIAH A. M. ET AL: "Bioisosteres of the Phenyl Ring: Recent Strategic Applications in Lead Optimization and Drug Design", JOURNAL OF MEDICINAL CHEMISTRY, vol. 64, no. 19, 30 September 2021 (2021-09-30), US, pages 14046 - 14128, XP055933218, ISSN: 0022-2623, DOI: 10.1021/acs.jmedchem.1c01215
- TSE EDWIN G. ET AL: "Nonclassical Phenyl Bioisosteres as Effective Replacements in a Series of Novel Open-Source Antimalarials", JOURNAL OF MEDICINAL CHEMISTRY, vol. 63, no. 20, 17 July 2020 (2020-07-17), US, pages 11585 - 11601, XP055933193, ISSN: 0022-2623, Retrieved from the Internet <URL:http://pubs.acs.org/doi/pdf/10.1021/acs.jmedchem.0c00746> DOI: 10.1021/acs.jmedchem.0c00746

## Description

The invention relates to novel inhibitors of transglutaminases, in particular transglutaminase 2, methods for their synthesis and to their use for the prophylaxis and treatment of diseases associated with transglutaminases, in particular transglutaminase 2. Any references in the description to methods of treatment refer to the compounds and pharmaceutical compositions of the present invention for use in a method for treatment of the human body by therapy.

### Background of the invention

Transglutaminases are part of the class of transferases and according to EC nomenclature they are correctly designated as "protein-glutamine: amine γ-glutamyl transferases" (EC 2.3.2.13). They link the ε-amino group of the amino acid lysine and the γ-glutamyl group of the amino acid glutamine forming an isopeptide bond while ammonia is released. In the absence of suitable amines and/or under certain conditions, deamidation of the glutamine may occur resulting in the corresponding glutamic acid.

Additionally, transglutaminases play an important role in many therapeutic areas such as the cardiovascular diseases (thrombosis and atherosclerosis), autoimmune diseases (celiac disease, Duhring-Brocq-disease, gluten ataxia), neurodegenerative diseases (Alzheimer's disease, Parkinson's disease, Huntington's disease), dermatological diseases (ichthyosis, psoriasis, acne) as well as in wound healing and inflammatory diseases (e.g. tissue fibrosis) (J.M. Wodzinska, Mini-Reviews in medical chemistry, 2005, 5, 279 - 292).

Celiac disease, a gluten intolerance, however, is one of the most important indications.

Celiac disease is characterized by a chronic inflammation of the mucosa of the small intestine. In susceptible patients, the intestinal epithelium is successively destroyed after ingestion of gluten-containing food resulting in reduced absorption of nutrients which again has massive impact on the patients affected and is for example associated with symptoms such as loss of weight, anemia, diarrhea, nausea, vomiting, loss of appetite and fatigue. Due to these findings, there is a large demand for the development of a medicament for the treatment of celiac disease as well as of other diseases associated with tissue transglutaminase (transglutaminase 2, TG2, tTG). The tissue transglutaminase is a central element during pathogenesis. The endogenous enzyme catalyses the deamidation of gluten/gliadin in the small intestinal mucosa and thus triggers the inflammatory response. Therefore inhibitors of tissue transglutaminase are suitable to be used as active agents for medication.

Another very important group of indications for tissue transglutaminase inhibitors are fibrotic disorders. Fibrotic disorders are characterized by the accumulation of crosslinked extracellular matrix proteins. Diabetic nephropathy, cystic fibrosis, idiopathic pulmonary fibrosis, kidney fibrosis as well as liver fibrosis belong to the most important fibrotic disorders to be addressed with the compounds disclosed.

US 9,434,763 B2 discloses pyridinone derivatives having a warhead comprising at least one acceptor-substituted double bond, such as a Michael System, as irreversible transglutaminase inhibitors. Alkylacetamido and arylacetamido pyridinones showed inhibitory activity regarding tissue transglutaminase TG2 in nanomolar range (IC₅₀).

Tse et al. (J. Med. Chem. 2020, 63, 11585-11601) report on replacement of phenyl residues by non-classical bioisosteres, such as cubane and bicyclo[1.1.1]pentane (BCP), in anti-malarial triazolopyrazine compounds in order to alter compound solubility and metabolic stability. The authors further evaluated *in vitro* antiplasmodial activity of bioisosteric modified triazolopyrazines against the 3D7 strain of *P. falciparum.* Replacement of phenyl by bioisosteric saturated heterocyclic residues resulted in complete loss of activity. Adamantyl residues as well as other hydrocarbon-caged derivatives led to potency up to 2-9 times lower than the corresponding phenyl triazolopyrazine compounds. In contrast, higher potencies were achieved by replacing phenyl with closo-1,2- and 1,7-carborane isomers. The authors concluded that the effect of non classical bioiostere replacement on biological properties cannot be predicted accurately and that a considerable range of possible bioisosteres has to be tested first in order to identify a suitable replacement leading to the desired properties of a given molecule.

Subbaiah et al. (J. Med. Chem. 2021, 64, 19, 14046-14128) report on bioisosteres of the phenyl ring in lead optimization and drug design. It is noted that bioisosteric phenyl ring replacement with heterocyclic and carbocyclic moieties can lead to enhanced potency, solubility, and metabolic stability while reducing lipophilicity, plasma protein binding, phospholipidosis potential, and inhibition of cytochrome P450 enzymes and the hERG channel. However, this effect depends strongly on the properties of the compound itself and the addressed target.

US 11,072,634 B2 discloses reversible transglutaminase inhibitors comprising an aldehyde, a ketone, an α-ketoaldehyde, an α-ketoketone, an α-ketoacid, an α-ketoester, an α-ketoamide or a halogenmethylketone as warhead. The inhibitors showed inhibitory activity regarding tissue transglutaminase TG2 in nanomolar and micromolar range (IC₅₀).

The objective of the present invention is to provide novel, most probably reversible inhibitors of transglutaminases, in particular transglutaminase 2 and methods for the synthesis of said inhibitors as well as several uses of these inhibitors.

Said objective is solved by the technical teachings of the independent claims. Further advantageous embodiments, aspects and details of the invention are evident from the dependent claims, the description and the examples.

Surprisingly, it has been found that reversible inhibitors having a chemical warhead as disclosed herein inhibit effectively transglutaminases including tissue transglutaminase called transglutaminase 2 or TG2. Herein these terms are used synonymous.

Preferably, such chemical warhead moiety is particularly selected from reversible warheads such as α-ketoamides. The compounds of the present invention act as selective inhibitors of transglutaminase 2.

In order to prove inventiveness of the compounds of the present application, reference compounds were synthesized and tested in comparison to the most similar compounds of the present application. A skilled person might notice compound A8 from our patent US 9,434,763 B2 which we introduce as Ref. 3 to highlight the inventive and preferred features of the claimed compounds. From US 9,434,763 B2, it is clear, that aromatic moieties (C-terminal) restrict the efficacy of those compounds (compare to A1, **A8,** A37, A44, A47). In sharp contrast, branched alkyl moieties are highly preferred as indicated by more potent compounds (A28, A29, A59, A61, A63, A67, A68, A79).

To illustrate the advantage of branched alkyl moieties over aromatic moieties, we refer to reference compounds Ref. 2 (ZED1227, US 9,434,763 B2) and Ref. 3 (A8, ZED1047). Inhibition data were determined using the classical fluorescent transamidation assay (dansylcadaverine incorporation into methylated casein, DCC-assay) as described [Büchold, C.; Hils, M.; Gerlach, U.; Weber, J.; Pelzer, C.; Heil, A.; Aeschlimann, D.; Pasternack, R. Features of ZED1227: The First-In-Class Tissue Transglutaminase Inhibitor Undergoing Clinical Evaluation for the Treatment of Celiac Disease. Cells 2022, 11, 1667. https://doi.org/10.3390/cells11101667]. Casein is one of the best known high molecular weight (24 kDa) protein substrates for transglutaminases. Please note, the IC₅₀ value of Ref. 3 (A8) published in US 9,434,763 B2 cannot be compared to the present data, relying on a fluorogenic isopeptidase assay. Measured in the DCC-assay, Ref. 2 (IC₅₀= 53 nM) is 80-fold more potent compared to Ref. 3 (IC₅₀= 4,268 nM).

Accordingly, a person skilled in the art of medicinal chemistry would choose branched alkyl moieties as lead structures, such excluding aromatic moieties, e.g. the phenyl group. It is of common knowledge, that bridged cycloalkyl groups are non-classical bioisosters of the phenyl group. By replacement of the phenyl group in A8 by e.g. an adamantane group, a skilled person would expect similar physico-chemical or biochemical properties excluding to invest efforts. Since aromatic moieties are clearly not preferred, bridged cycloalkyl groups would not be considered improving the compounds.

This is further supported by additional reference compounds. ZED3641 (Ref. 1, as disclosed in US 11,072,634 B2; reversible acting α-ketomethylamide analogue to Ref. 2, ZED1227) is about 10-fold more potent compared to Ref. 6 (compare table 1). Ref. 6 is analogous to compound A8 disclosed in US 9,434,763 B2 with respect to the backbone proving again superiority of branched alkyl moieties compared to aromatic derivatives in combination with reversible acting warheads.

However, surprisingly, replacement of the preferred branched alkyl moieties by bridged cycloalkyl groups further significantly improve the potency of the compounds as shown in table 1. Therefore, we credit bridged cycloalkyl groups as disclosed with an outstanding inventive manner.

In summary, the inventive compounds rated "A" show efficacies of about 100-fold higher compared to Ref. 3 (A8, compare to table1).

Further, compounds with activities rated "B" or "C" are still preferred (lower IC₅₀ values) to Ref. 3 (A8). These compounds can also be considered inventive, since peripheric ligands affect physico-chemical or biochemical properties. Therefore, also less potent compounds might be of high value, depending on the application.

Thus, the present invention relates to compounds of the general formula (**I**): 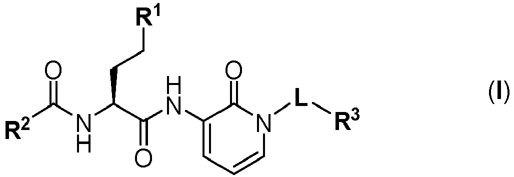 wherein
**L** represents **-L¹-** or **-L¹-L²-;** preferably **-L¹-L²-**;;
**L¹** represents -CH₂-, -CH₂CH₂-, -CH₂CH₂CH₂-, -CH₂CO-, or -CH₂CH₂CO-;
**L²** represents a bond, -NR^{N1}-, -NR^{N1}CH₂-, -NR^{N1}CH₂CH₂-, or -NR^{N1}CH(CH₃)-,
**R'** represents 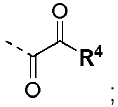
**R²** represents 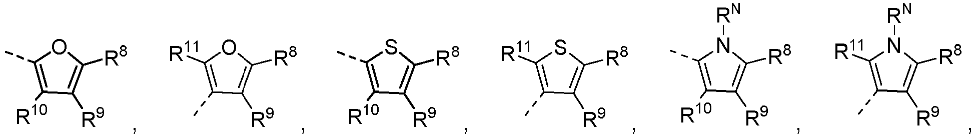 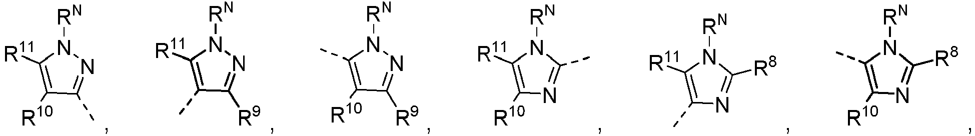 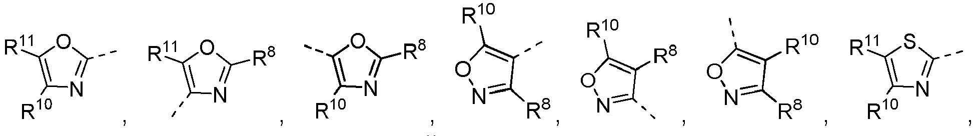 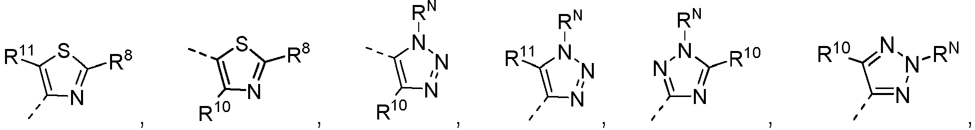 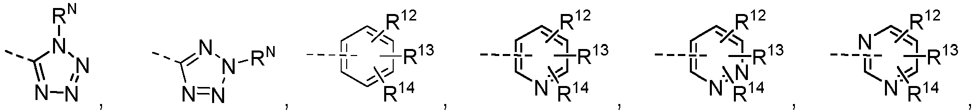 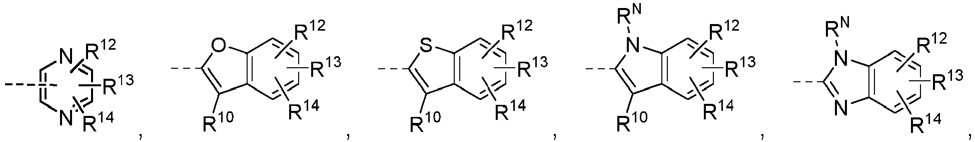 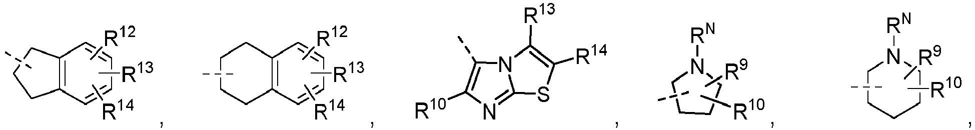 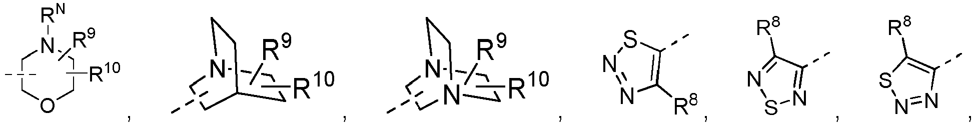 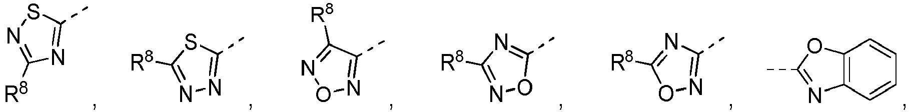 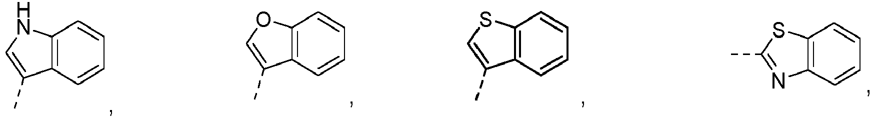 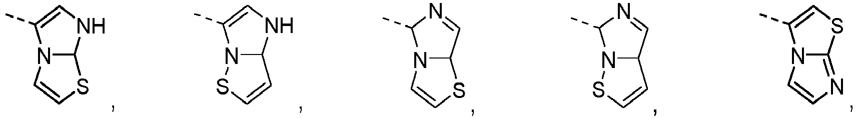 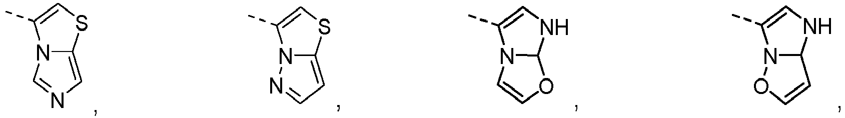 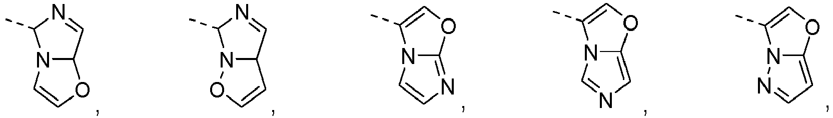 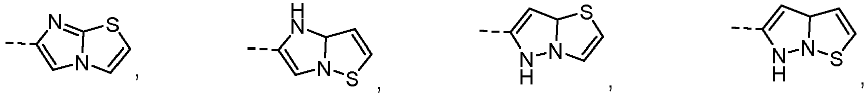 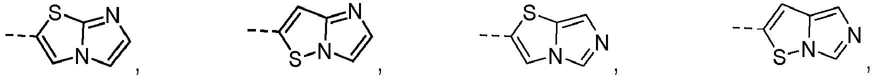 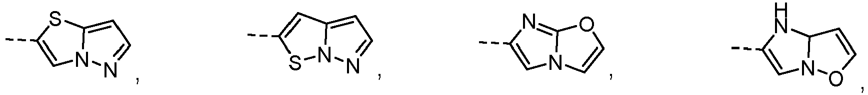 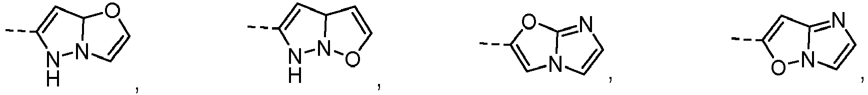 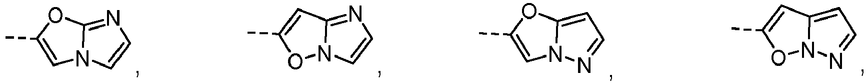 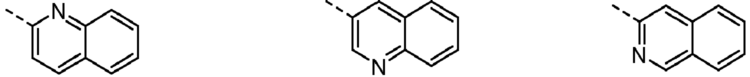 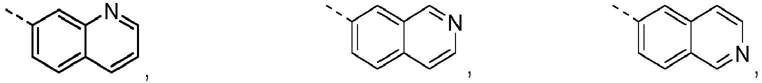 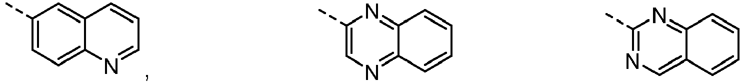 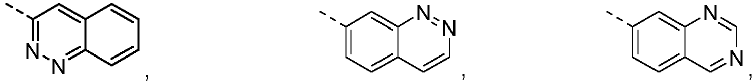 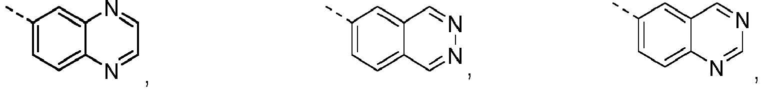 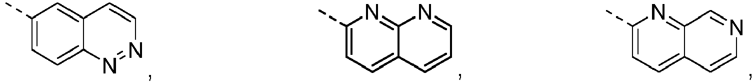 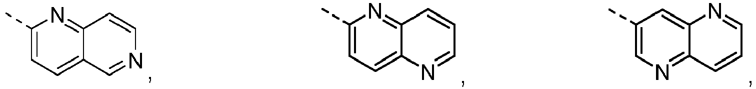 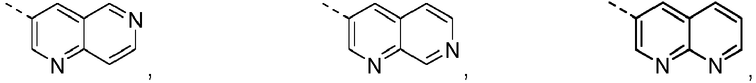 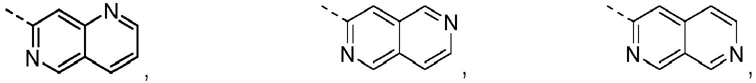 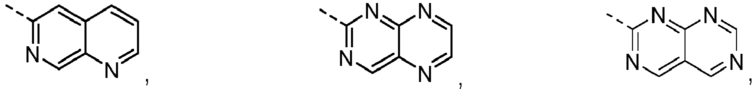 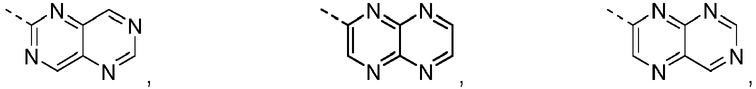 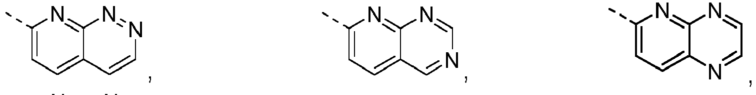 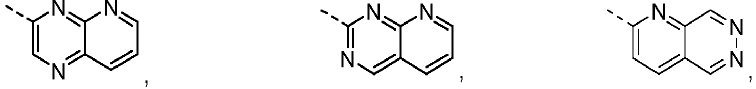 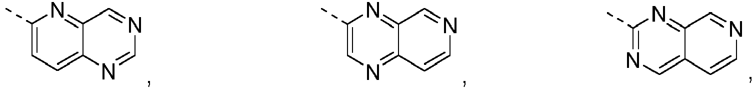 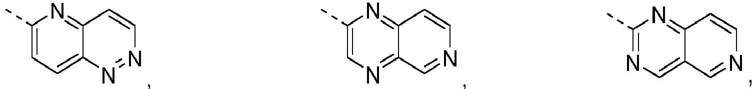 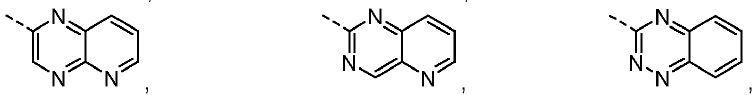 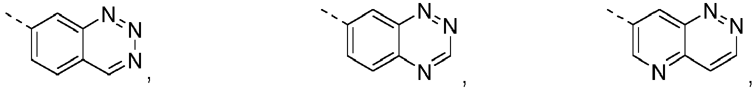 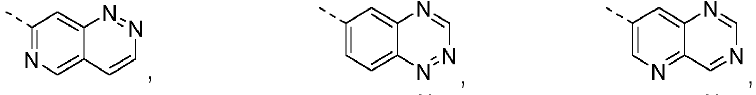 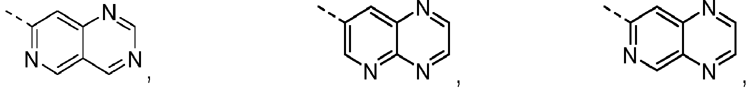 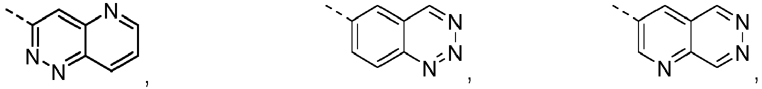 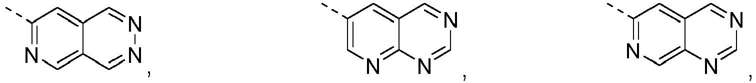 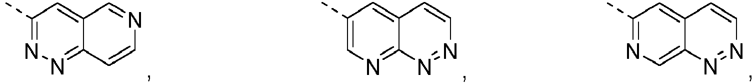 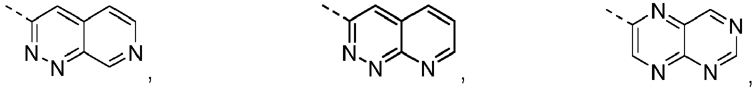 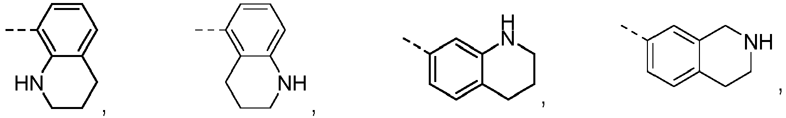 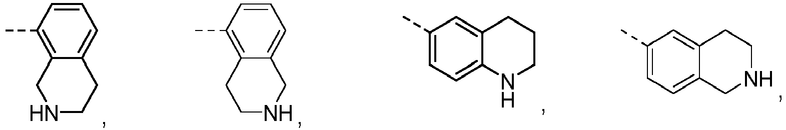 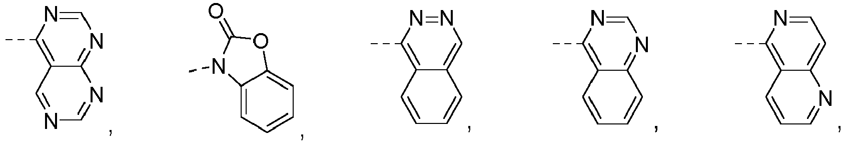 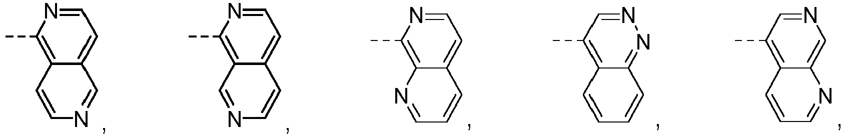 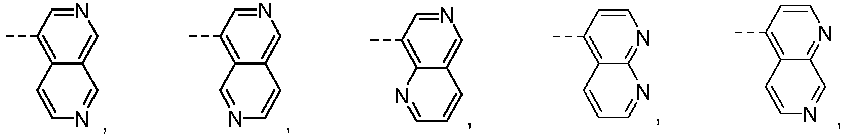 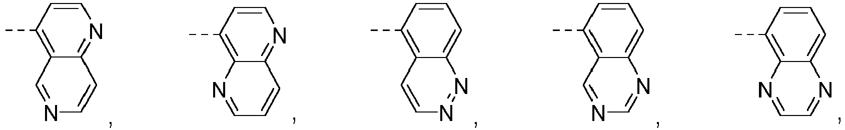 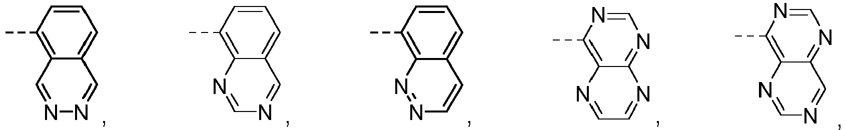 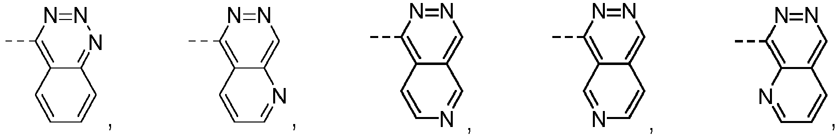 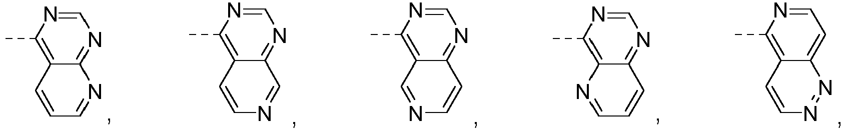 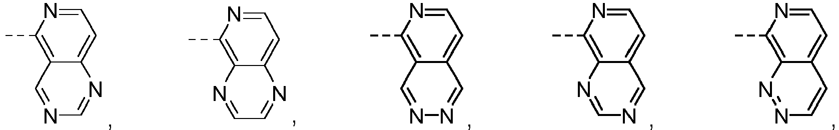 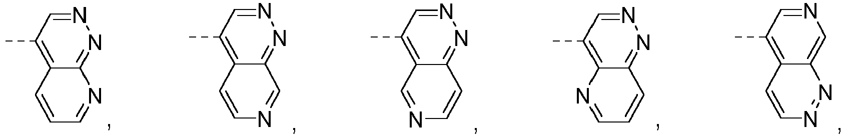 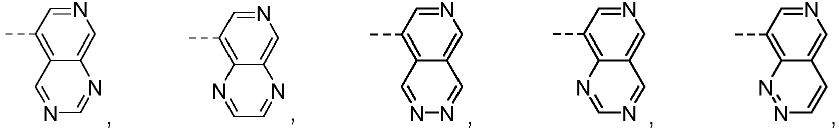 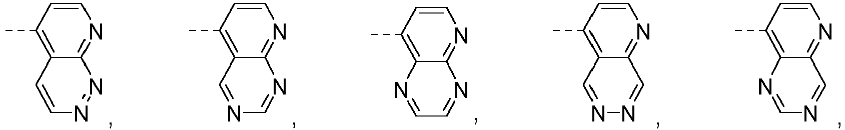 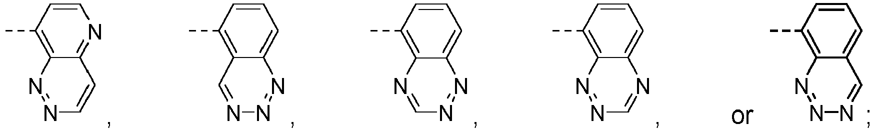
wherein the unsubstituted bicyclic residues can be substituted with 1 to 5 of the substituents **R⁹** - **R¹⁴** and **R^{N}** ; and preferably with 1 to 3 of the substituents **R¹¹** - **R¹³;**
**R³** represents bicyclo[1.1.1]pentyl, bicyclo[2.1.1]hexyl, bicyclo[2.2.1]heptyl, bicyclo[3.1.1]heptyl, bicyclo[2.2.2]octyl, bicyclo[3.2.1]octyl, bicyclo[3.2.2]nonyl, bicyclo[3.3.2]decyl, bicyclo[3.3.3]undecyl, 4-homoisotwistyl, adamantyl, diamantyl, hexamethylenetetraminyl and the afore-mentioned residues optionally contain one or more C=C double bond(s) and/or are optionally substituted by one or more of **R^{a}, R^{b}, R^{c}, R^{d},** and **R^{e};**
**R^{a}, R^{b}, R^{c}, R^{d},** and **R^{e}** represents independently of each other -H, -F, -Cl, -Br, -CN, -OH, -CH₃, -CH₂CH₃, -CH₂CH₂CH₃, -CH(CH₃)₂, -CHF₂, -CF₃, -CH₂CF₃, -COCH₃, -COCH₂CH₃, -CO₂H, -CO₂CH₃, -CO₂C₂H₅, -CONH₂, -CONHCH₃, -CON(CH₃)₂, -CONHC₂H₅, -CH₂CO₂H, -CH₂CO₂CH₃, -CH₂CO₂C₂H₅, -CH₂CONH₂, -CH₂CONHCH₃, -CH₂CON(CH₃)₂, -CH₂CONHC₂H₅, -NHCOCH₃, -NHCOC₂H₅, -NHCOCF₃, -NHCOCH₂CF₃, -NHSO₂CH₃, -NHSO₂C₂H₅, -NHSO₂CHF₂, -NHSO₂CF₃, -NHSO₂CH₂CF₃;
**R⁴** represents **-NR⁶R⁷;**
**R⁶** and **R⁷** represent independently of each other -H, -CH₃, -CH₂CH₂CH₃, -CH(CH₃)₂, -CH₂CH₂CH₂CH₃, -CH₂CH₂CH₂CH₂CH₃, -CH₂CH(CH₃)₂, -C(CH₃)₃, -CH₂CH=CH₂, -CH₂CH=CH(CH₃), -CH₂CH=C(CH₃)₂, -CH₂CH=CHCH₂CH₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -Ph, -CH₂-Ph, -CH₂OCH₃, -CH₂OCH₂CH₃, -CH₂CH₂OCH₃, -CH₂CH₂OCH₂CH₃, -CH₂CH₂NHCH₃, -CH₂CH₂N(CH₃)₂,
or **-NR⁶R⁷** is -N(C₂H₅)₂, 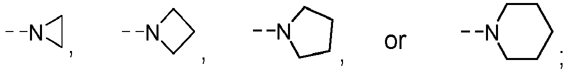
**R⁸, R⁹, R¹⁰, R¹¹, R¹², R¹³,** and **R¹⁴** represent independently of each other -H, -F, -Cl, -Br, -I, -OH, -CN, -NO₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -CH₂-cyclo-C₃H₅, -CH₂OH, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -OCH₃, -OC₂H₅, -OC₃H₇, -OCH(CH₃)₂, -OC(CH₃)₃, -OC₄H₉, -OCHF₂, -OCF₃, -OCH₂CF₃, -OC₂F₅, -OCH₂OCH₃, -O-cyclo-C₃H₅, -OCH₂-cyclo-C₃H₅, -O-C₂H₄-cyclo-C₃H₅, -CHO, -COCH₃, -COCF₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, -COOC(CH₃)₃, -OOC-CH₃, -OOC-CF₃, -OOC-C₂H₅, -OOC-C₃H₇, -OOC-CH(CH₃)₂, -OOC-C(CH₃)₃, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NHCOCH₃, -NHCOCF₃, -NHCOC₂H₅, -NHCOC₃H₇, -NHCOCH(CH₃)₂, -NHCOC(CH₃)₃, -CONH₂, -CONHCH₃, -CONHC₂H₅, -CONHC₃H₇, -CONHCH(CH₃)₂, -CONH-cyclo-C₃H₅, -CONHC(CH₃)₃, -CON(CH₃)₂, -CON(C₂H₅)₂, -CON(C₃H₇)₂, -CON[CH(CH₃)₂]₂, -CON[C(CH₃)₃]₂, -SO₂NH₂, -SO₂NHCH₃, -SO₂NHC₂H₅, -SO₂NHC₃H₇, -SO₂NHCH(CH₃)₂, -SO₂NH-cyclo-C₃H₅, -SO₂NHC(CH₃)₃, -SO₂N(CH₃)₂, -SO₂N(C₂H₅)₂, -SO₂N(C₃H₇)₂, -SO₂N[CH(CH₃)₂]₂, -SO₂N[C(CH₃)₃]₂, -NHSO₂CH₃, -NHSO₂CF₃, -NHSO₂C₂H₅, -NHSO₂C₃H₇, -NHSO₂CH(CH₃)₂, -NHSO₂C(CH₃)₃, -CH=CH₂, -CH₂-CH=CH₂, -C(CH₃)=CH₂, -CH=CH-CH₃, -C≡CH, -C≡C-CH₃, -CH₂-C≡CH, -Ph, -O-Ph, -O-CH₂-Ph, 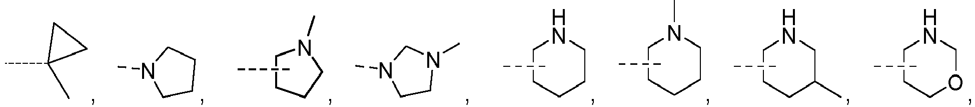 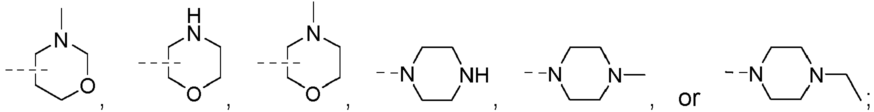
or **R⁸** and **R⁹** or **R⁹** and **R¹⁰** can form together one of the following five-membered or six-membered rings: 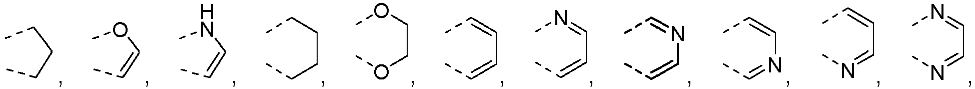 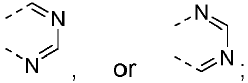
or **R¹²** and **R¹³** or **R¹³** and **R¹⁴** can form together one of the following five-membered or six-membered rings: 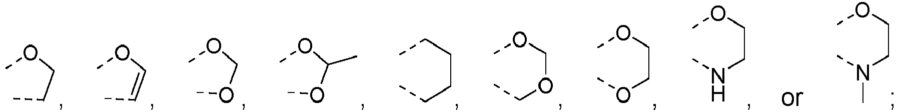
**R^{N}** represents -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -cyclo-C₄H₇, -cyclo-C₅H₉, -CH₂-cyclo-C₃H₅, -CH₂-cyclo-C₄H₇, -CH₂-cyclo-C₅H₉, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -CH₂-CH=CH₂, -CH₂-C≡CH, -CHO, -COCH₃, -COC₂H₅, -COC₃H₇, -COCH(CH₃)₂, -COC(CH₃)₃, -CO-cyclo-C₃H₅, -CO-cyclo-C₄H₇, -CO-cyclo-C₅H₉, -COOCH₃, -COOC₂H₅, -COOC₃H₇, -COOCH(CH₃)₂, -COOC(CH₃)₃, -COOCH₂Ph, -SO₂CH₃, -SO₂CF₃, -SO₂C₂H₅, -SO₂C₃H₇, -SO₂CH(CH₃)₂, -SO₂-cyclo-C₃H₅, or -SO₂C(CH₃)₃;
**R^{N1}** represent -H, -CH₃, or -CH₂CH₃;
or a diastereomer, an enantiomer, a mixture of diastereomers, a mixture of enantiomers, a racemate, a solvate, a hydrate, or a pharmaceutically acceptable salt thereof.

The inventors have found that the reversible inhibitors of formula (I) disclosed herein having a bridged bicyclic residue **R³** exhibit increased potency over the compounds of the prior art. Particularly, it is demonstrated herein, that the inventive compounds have an improved inhibitory activity compared to the known compounds bearing aromatic moieties **R³** instead of bridged bicyclic residues. In order to prove inventiveness of the compounds of the present application, known compounds from US 9,434,763 B2 and US 11,072,634 B2 (Reference 1 (E16 from US 11,072,634 B2), Reference 3 (A8 from US 9,434,763 B2), and Reference 6) were synthesized and tested as reference compounds in comparison to the most similar compounds of the present application. To this extent, inhibition data were determined using the classical fluorescent transamidation assay (dansylcadaverine incorporation into methylated casein, DCC-assay) as described in Büchold et al. [Büchold, C.; Hils, M.; Gerlach, U.; Weber, J.; Pelzer, C.; Heil, A.; Aeschlimann, D.; Pasternack, R. Features of ZED1227: The First-In-Class Tissue Transglutaminase Inhibitor Undergoing Clinical Evaluation for the Treatment of Celiac Disease. Cells 2022, 11, 1667. https://doi.org/10.3390/cells11101667]. Casein is one of the best known high molecular weight (24 kDa) protein substrates for transglutaminases. Inhibition data of the inventive compounds was compared with inhibition of compounds disclosed in US 9,434,763 B2, particularly, compound A8, which is denoted herein as Reference 3. It is noteworthy that the IC₅₀ values of compounds A8 published in US 9,434,763 B2 and E16 from US 11,072,634 B2 cannot be compared to the present data, relying on a fluorogenic isopeptidase assay.

Thus, the inventive compounds of formula (I) rated "A" showed efficacies of about 100-fold higher compared to Ref. 3 (A8). The same argumentation applies to Ref. 6 which is except of the phenylethyl group identical to compound **II-111.** Ref. 6 is more than 25-times less potent in comparison to compound **II-111** as evident from Table 1.

This finding was particularly surprising as a skilled person would not have expected an improved inhibitory activity of the inventive compounds bearing a bridged bicyclic residue over the compounds of the prior art bearing aromatic residues since it is common knowledge that bridged bicyclic groups or bridged cycloalkyl groups are non-classical bioisosters of the phenyl group, such that a skilled person would only expect to obtain a compound having similar physico-chemical and biological properties, including inhibitory activity, when replacing a phenyl group with a bridged bicyclic group. As aromatic moieties showed lower potency, bridged cycloalkyl groups would not be considered improving the physico-chemical and biological properties of the compounds.

The residues bicyclo[1.1.1]pentyl, bicyclo[2.1.1]hexyl, bicyclo[2.2.1]heptyl, bicyclo[3.1.1]heptyl, bicyclo[2.2.2]octyl, bicyclo[3.2.1]octyl, bicyclo[3.2.2]nonyl, bicyclo[3.3.2]decyl, bicyclo[3.3.3]undecyl, 4-homoisotwistyl, adamantyl, diamantyl, and hexamethylenetetraminyl used herein, have the following parent structures respectively:

| | | |
|---|---|---|
| bicyclo[1.1.1]pentyl | bicyclo[2.1.1]hexyl | bicyclo[2.2.1]heptyl |
| 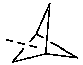 | 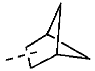 | 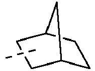 |
| bicyclo[3.1.1]heptyl | bicyclo[2.2.2]octyl | bicyclo[3.2.1]octyl |
| 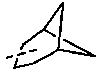 | 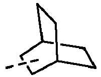 | 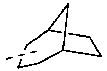 |
| bicyclo[3.2.2]nonyl | bicyclo[3.3.2]decyl | bicyclo[3.3.3]undecyl |
| 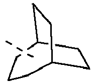 | 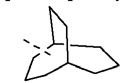 | 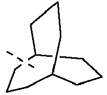 |
| 4-homoisotwistyl | adamantyl | diamantyl |
| 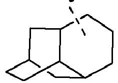 | 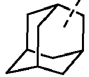 | 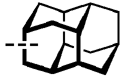 |
| hexamethylenetetraminyl | | |
| 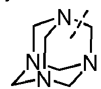 | | |

and the afore-mentioned residues optionally contain one or more C=C double bond(s) such as bicyclo[2.2.1]hept-5-enyl (s. **II-97**) and/or are optionally substituted by one or more of **R^{a}, R^{b}, R^{c}, R^{d},** and **R^{e}.**

The unsubstituted bicyclic residues which can be substituted with 1 to 5 of the substituents **R⁹** - **R¹⁴** and **R^{N};** have the following structure and the substituents **R⁹** - **R¹⁴** and **R^{N}** have the meanings as defined herein:

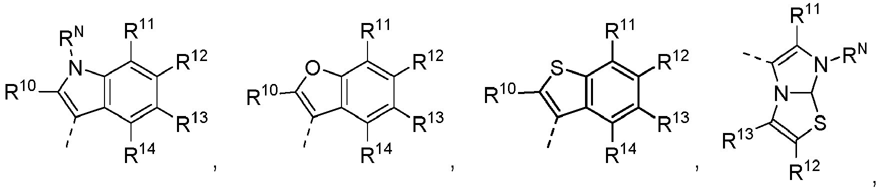

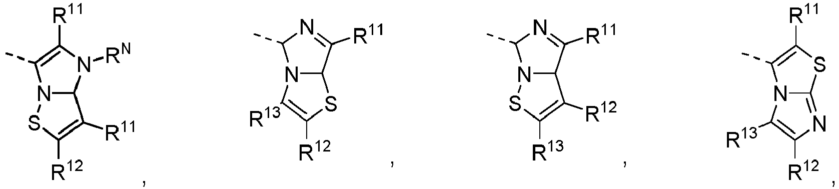

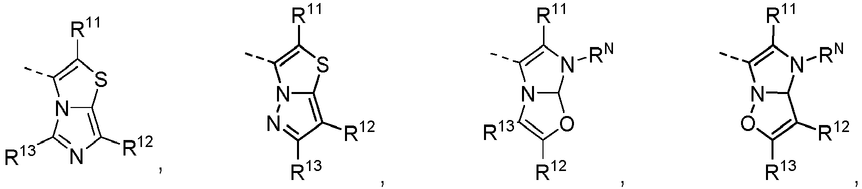

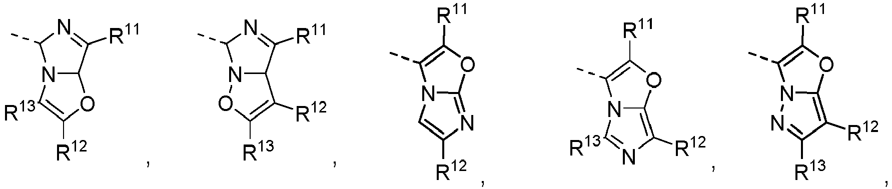

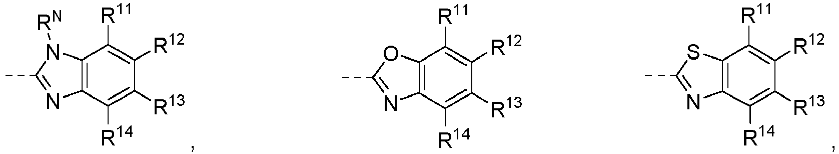

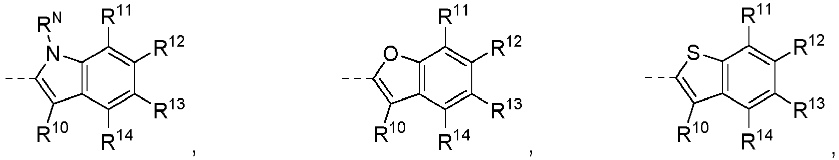

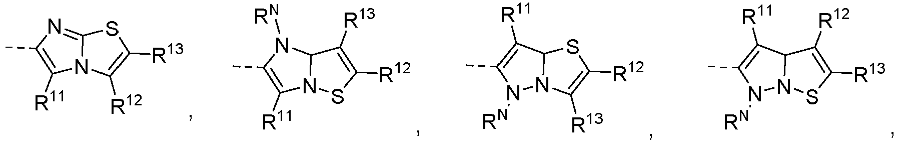

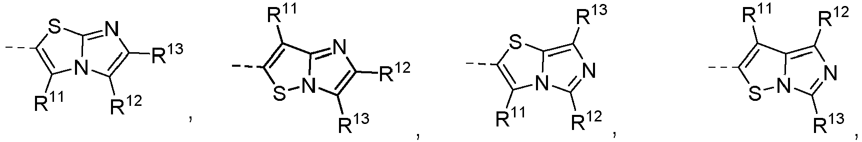

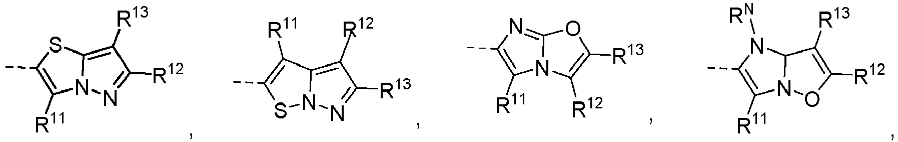

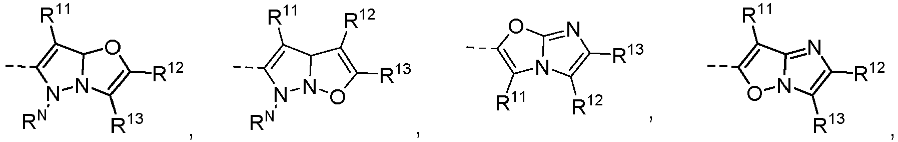

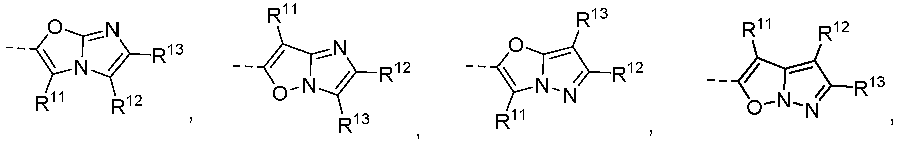

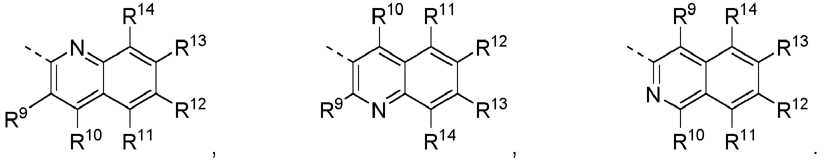

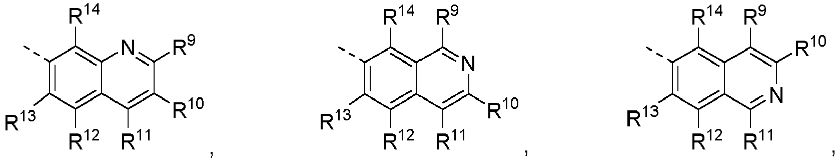

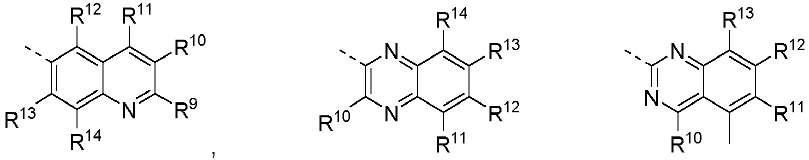

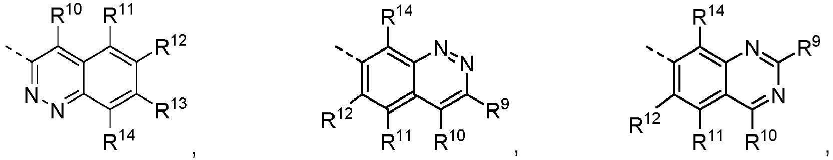

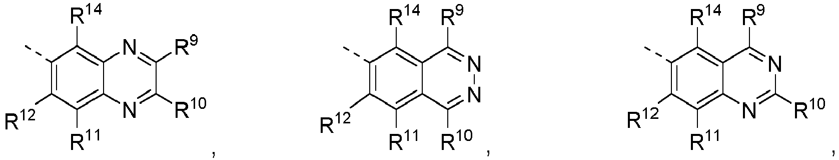

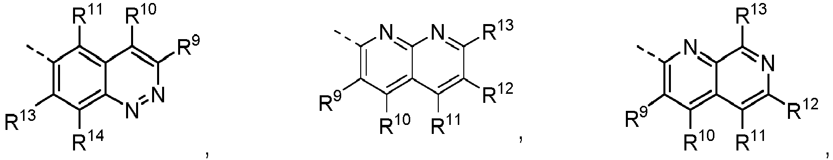

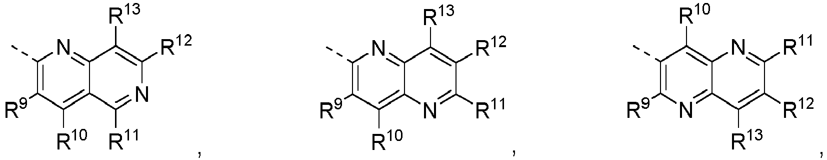

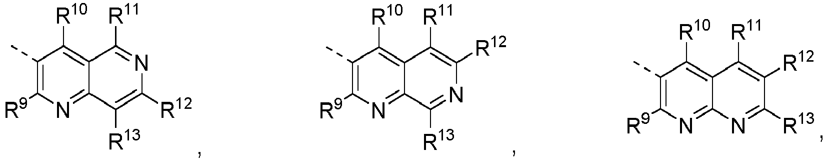

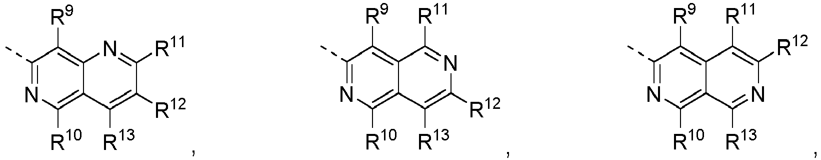

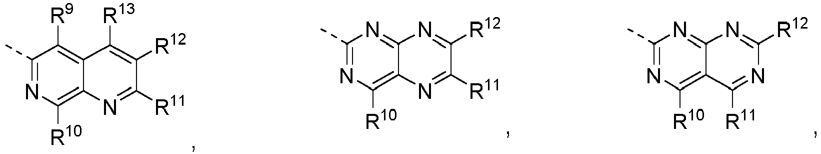

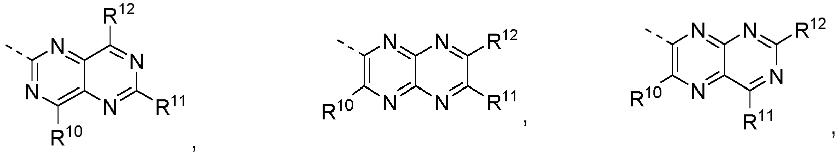

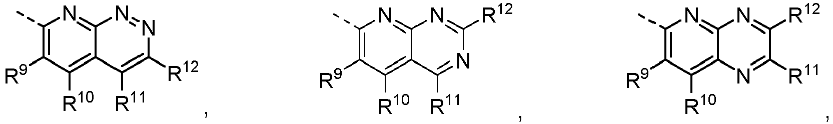

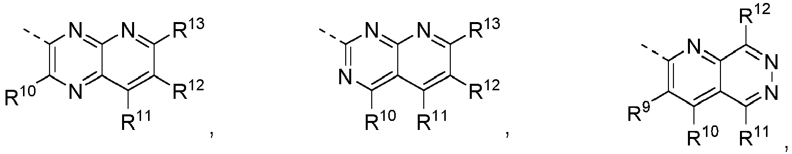

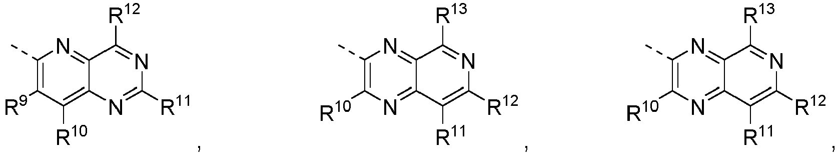

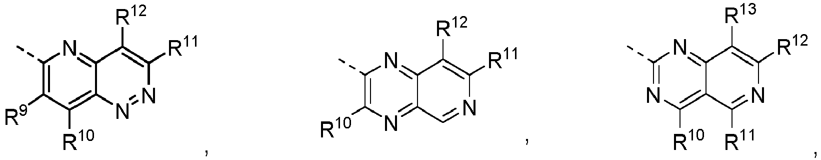

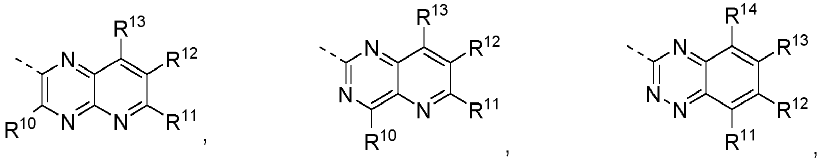

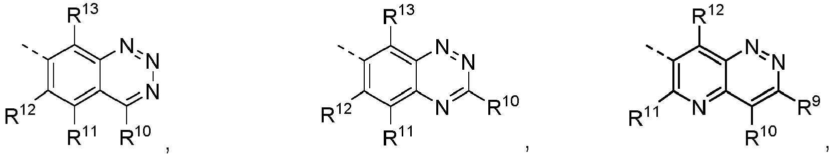

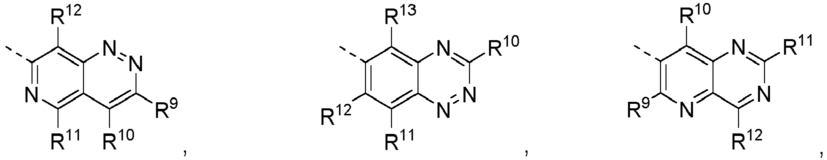

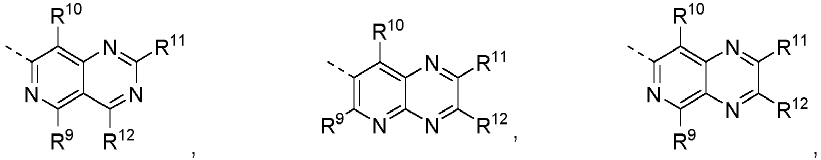

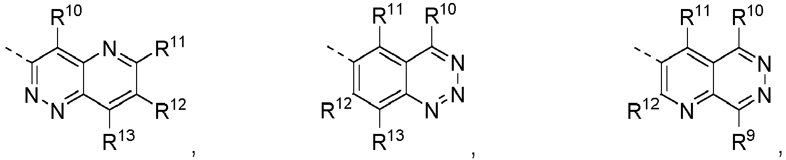

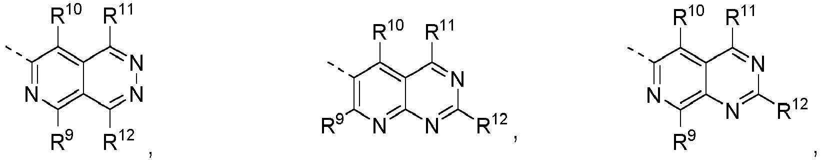

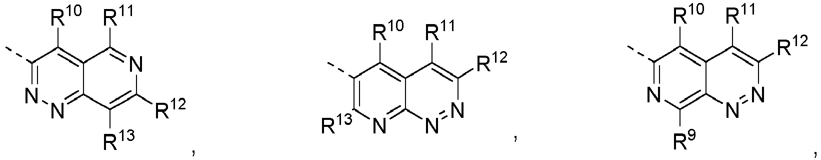

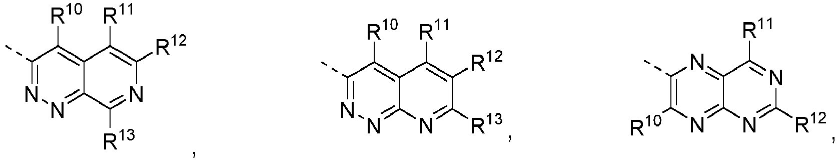

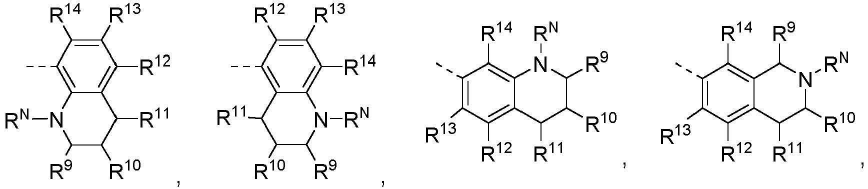

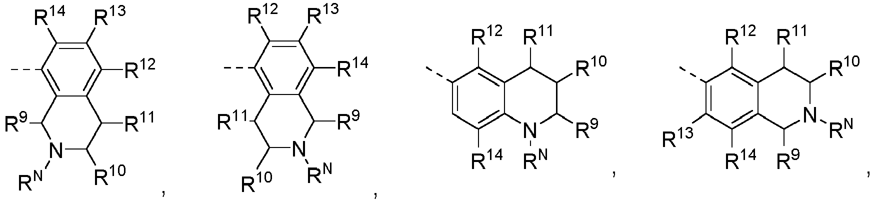

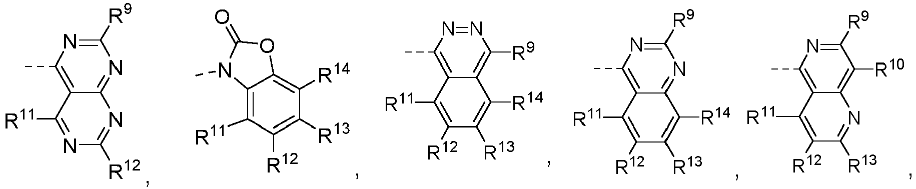

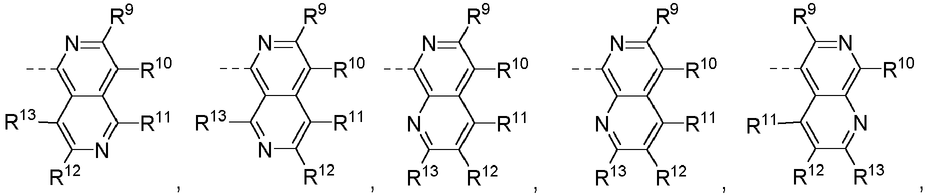

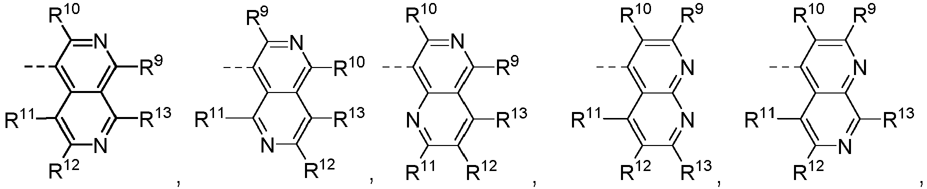

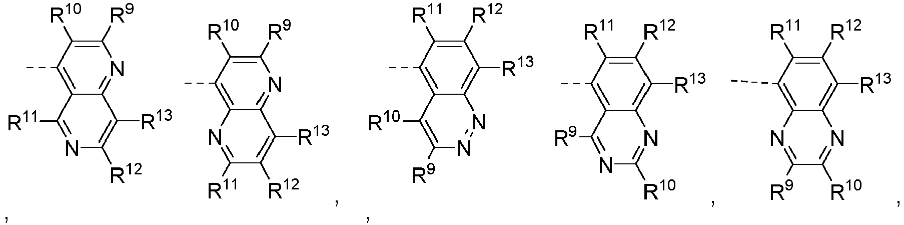

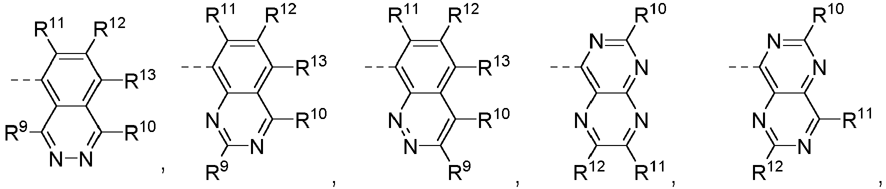

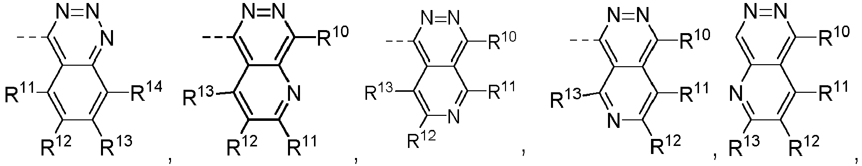

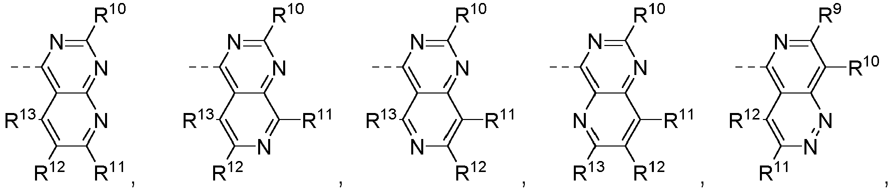

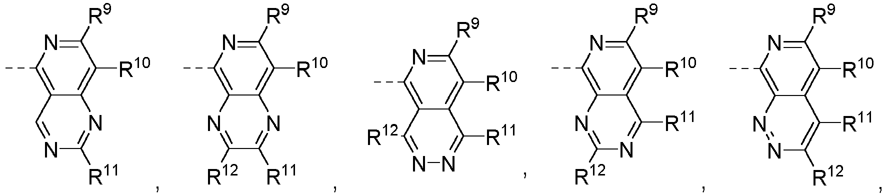

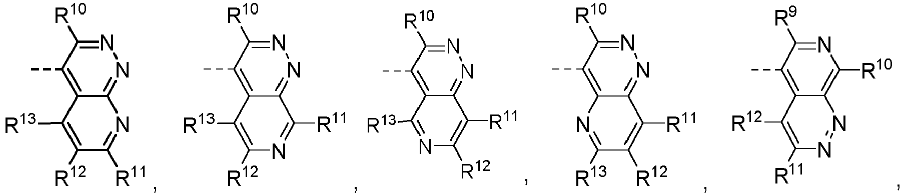

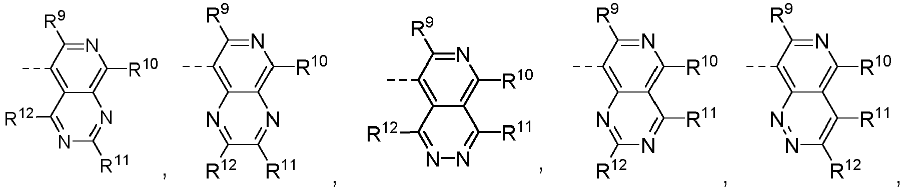

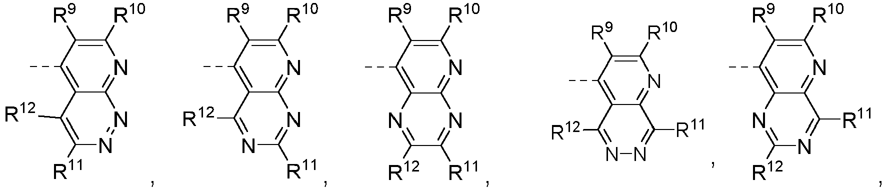

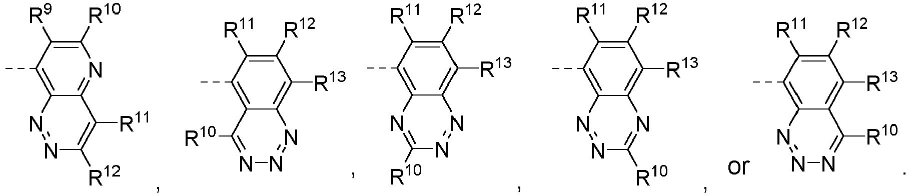

One embodiment is directed to compounds of the general formula (**I**): 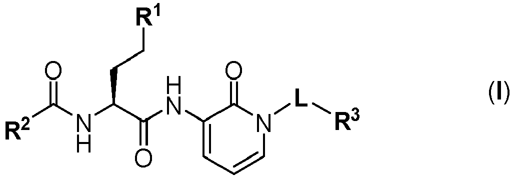 wherein
**L** represents **-L¹-** or **-L¹-L²-;** preferably **-L¹-L²-;**
**L¹** represents -CH₂-, -CH₂CH₂-, -CH₂CH₂CH₂-, -CH₂CO-, or -CH₂CH₂CO-;
**R¹** represents 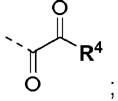
**R²** represents 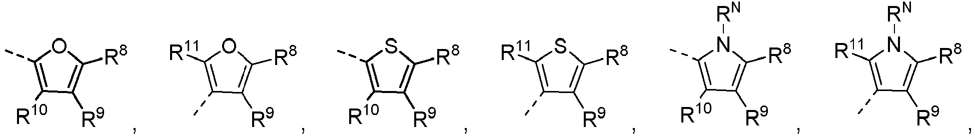 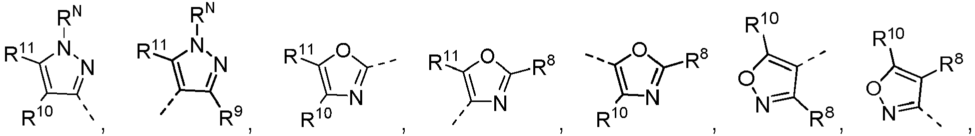 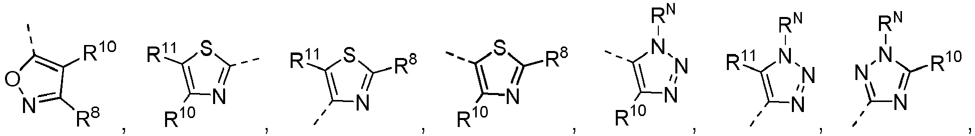 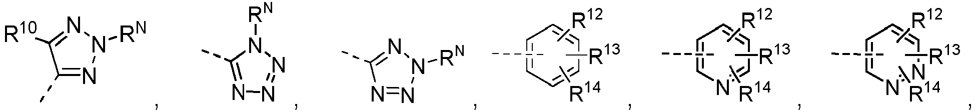 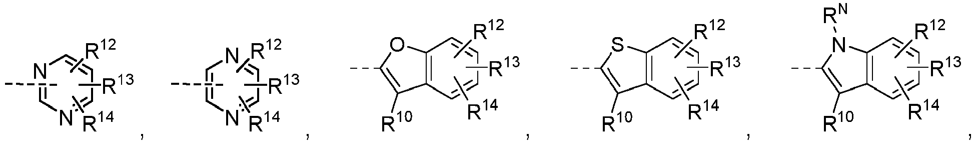 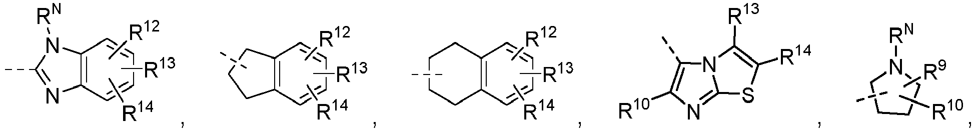 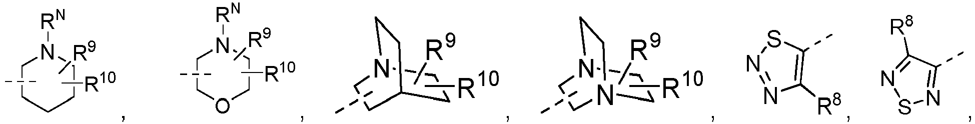 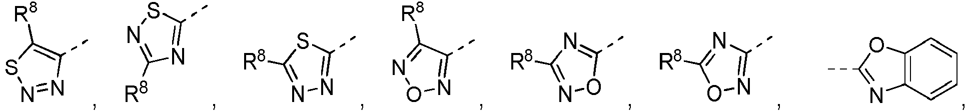 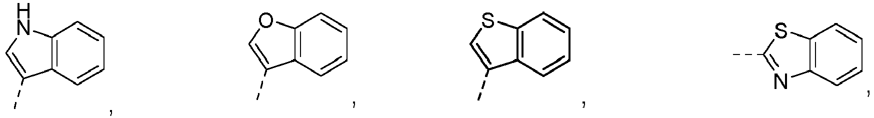 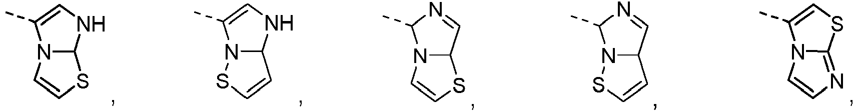 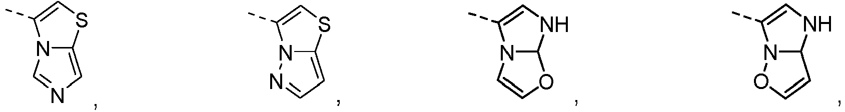 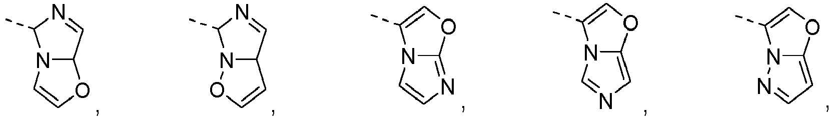 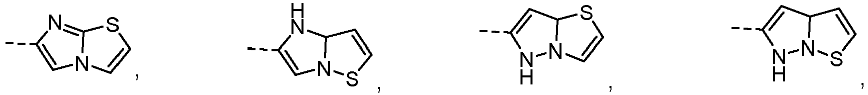 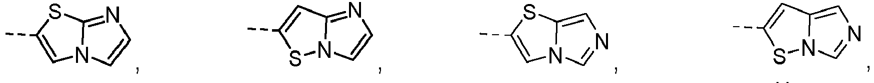 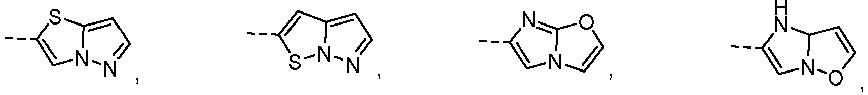 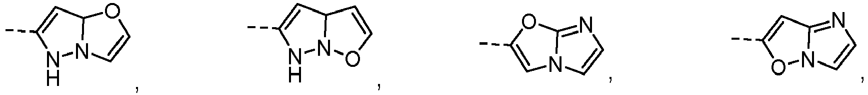 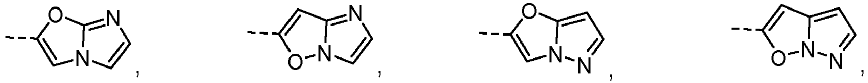 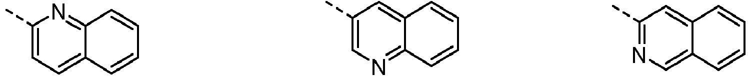 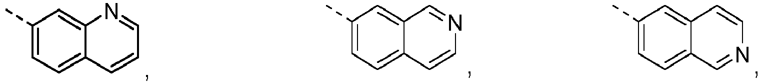 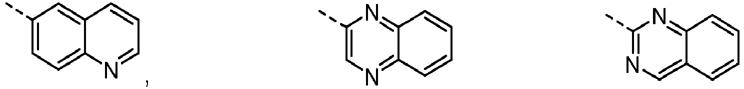 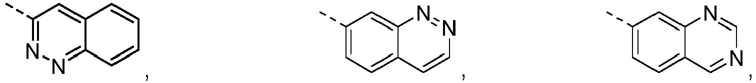 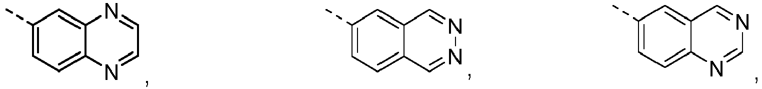 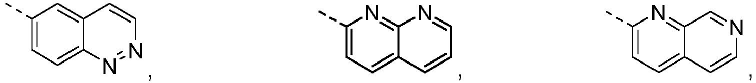 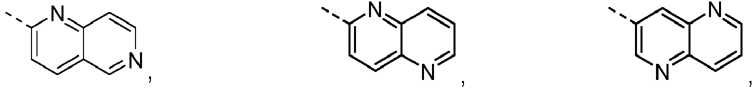 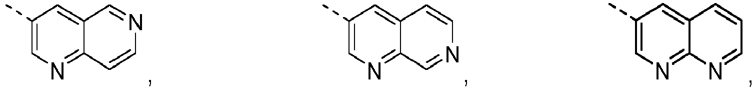 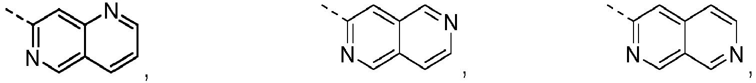 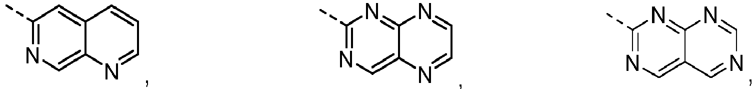 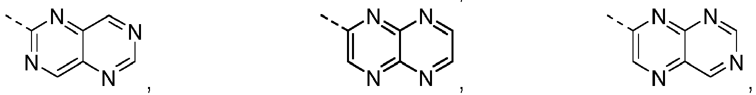 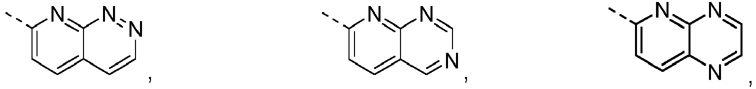 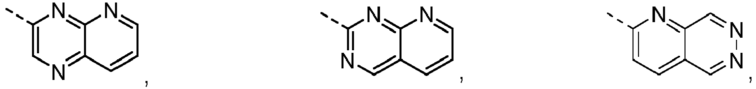 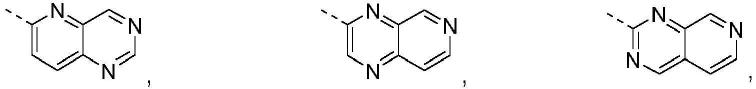 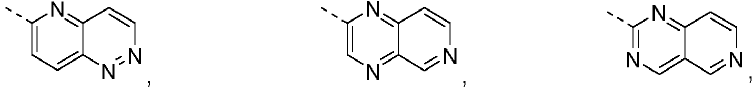 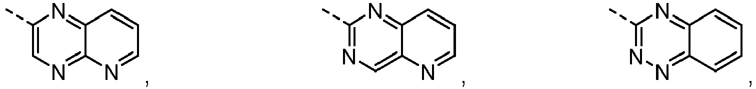 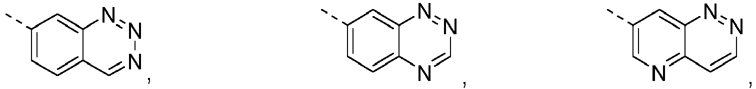 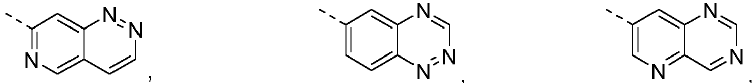 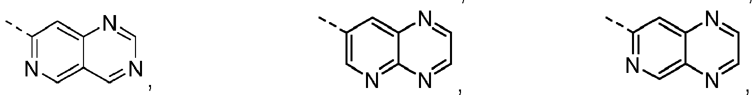 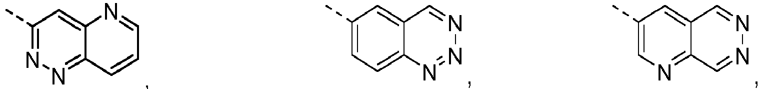 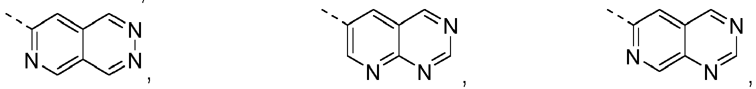 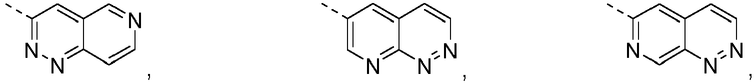 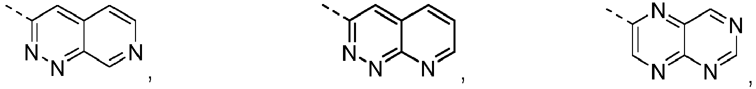 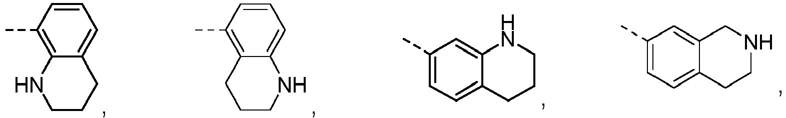 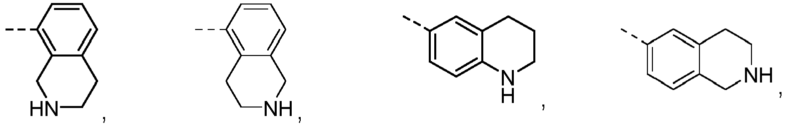 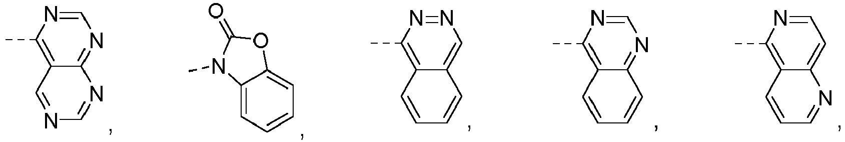 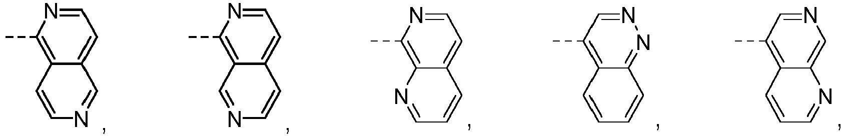 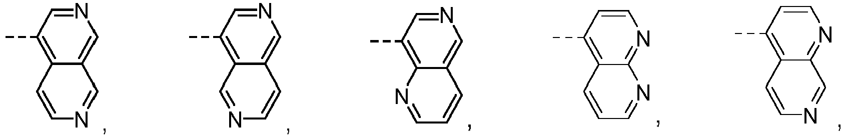 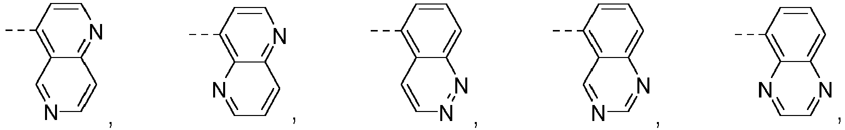 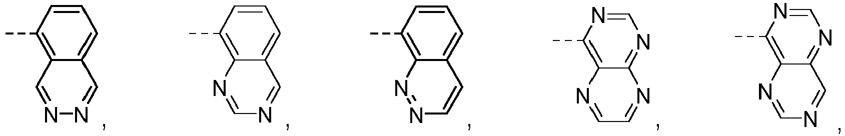 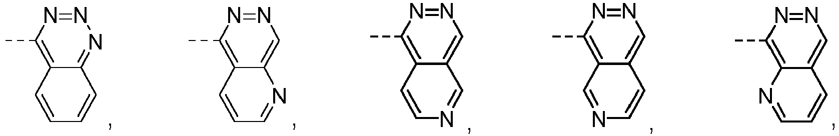 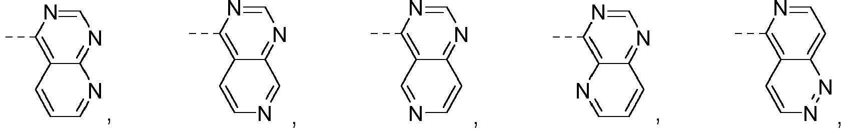 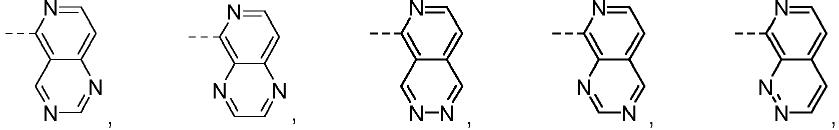 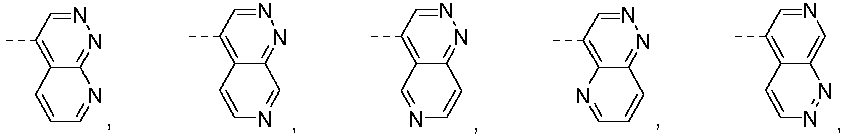 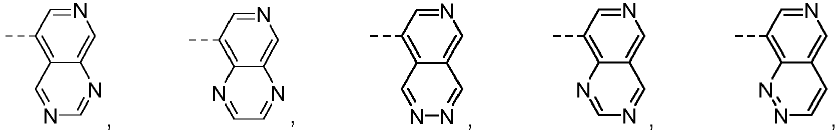 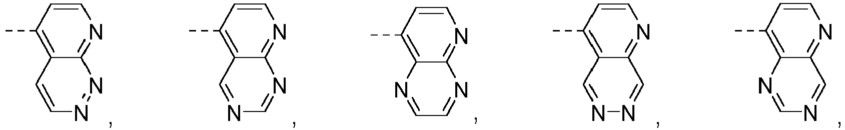 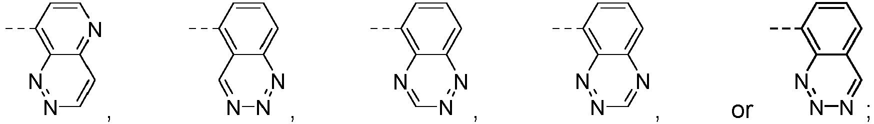
wherein the unsubstituted bicyclic residues can be substituted with 1 to 5 of the substituents **R⁹** - **R¹⁴** and **R^{N}** ; and preferably with 1 to 3 of the substituents **R¹¹** - **R¹³;**
wherein
   i) **L²** represents a bond, -**NR^{N1}**CH₂-, -**NR^{N1}**CH₂CH₂-, or -**NR^{N1}**CH(CH₃)-; and **R³** represents bicyclo[1.1.1]pentyl, bicyclo[2.1.1]hexyl, bicyclo[2.2.1]heptyl, 1-bicyclo[3.1.1]heptyl, 3-bicyclo[3.1.1]heptyl, bicyclo[2.2.2]octyl, bicyclo[3.2.1]octyl, bicyclo[3.2.2]nonyl, bicyclo[3.3.2]decyl, bicyclo[3.3.3]undecyl, 4-homoisotwistyl, diamantyl, hexamethylenetetraminyl and the afore-mentioned residues optionally contain one or more C=C double bond(s) and/or are optionally substituted by one or more of **R^{a}, R^{b}, R^{c}, R^{d},** and **R^{e};** or
   ii) **L²** represents a bond, **-NR^{N1}-,** -**NR^{N1}**CH₂CH₂-, or -**NR^{N1}**CH(CH₃)-; and **R³** represents bicyclo[1.1.1]pentyl, bicyclo[2.1.1]hexyl, bicyclo[2.2.1]heptyl, bicyclo[2.2.2]octyl, bicyclo[3.2.1]octyl, bicyclo[3.2.2]nonyl, bicyclo[3.3.2]decyl, bicyclo[3.3.3]undecyl, 4-homoisotwistyl, 2-adamantyl, diamantyl, hexamethylenetetraminyl and the afore-mentioned residues optionally contain one or more C=C double bond(s) and/or are optionally substituted by one or more of **R^{a}, R^{b}, R^{c}, R^{d},** and **R^{e};**
**R^{a}, R^{b}, R^{c}, R^{d},** and **R^{e}** represents independently of each other -H, -F, -Cl, -Br, -CN, -OH, -CH₃, -CH₂CH₃, -CH₂CH₂CH₃, -CH(CH₃)₂, -CHF₂, -CF₃, -CH₂CF₃, -COCH₃, -COCH₂CH₃, -CO₂H, -CO₂CH₃, -CO₂C₂H₅, -CONH₂, -CONHCH₃, -CON(CH₃)₂, -CONHC₂H₅, -CH₂CO₂H, -CH₂CO₂CH₃, -CH₂CO₂C₂H₅, -CH₂CONH₂, -CH₂CONHCH₃, -CH₂CON(CH₃)₂, -CH₂CONHC₂H₅, -NHCOCH₃, -NHCOC₂H₅, -NHCOCF₃, -NHCOCH₂CF₃, -NHSO₂CH₃, -NHSO₂C₂H₅, -NHSO₂CHF₂, -NHSO₂CF₃, -NHSO₂CH₂CF₃;
**R⁴** represents **-NR⁶R⁷;**
**R⁶** and **R⁷** represent independently of each other -H, -CH₃, -CH₂CH₂CH₃, -CH(CH₃)₂, -CH₂CH₂CH₂CH₃, -CH₂CH₂CH₂CH₂CH₃, -CH₂CH(CH₃)₂, -C(CH₃)₃, -CH₂CH=CH₂, -CH₂CH=CH(CH₃), -CH₂CH=C(CH₃)₂, -CH₂CH=CHCH₂CH₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -Ph, -CH₂-Ph, -CH₂OCH₃, -CH₂OCH₂CH₃, -CH₂CH₂OCH₃, -CH₂CH₂OCH₂CH₃, -CH₂CH₂NHCH₃, -CH₂CH₂N(CH₃)₂,
or **-NR⁶R⁷** is -N(C₂H₅)₂, 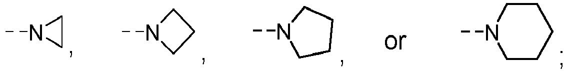
**R⁸, R⁹, R¹⁰, R¹¹, R¹², R¹³,** and **R¹⁴** represent independently of each other -H, -F, -Cl, -Br, -I, -OH, -CN, -NO₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -CH₂-cyclo-C₃H₅, -CH₂OH, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -OCH₃, -OC₂H₅, -OC₃H₇, -OCH(CH₃)₂, -OC(CH₃)₃, -OC₄H₉, -OCHF₂, -OCF₃, -OCH₂CF₃, -OC₂F₅, -OCH₂OCH₃, -O-cyclo-C₃H₅, -OCH₂-cyclo-C₃H₅, -O-C₂H₄-cyclo-C₃H₅, -CHO, -COCH₃, -COCF₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, -COOC(CH₃)₃, -OOC-CH₃, -OOC-CF₃, -OOC-C₂H₅, -OOC-C₃H₇, -OOC-CH(CH₃)₂, -OOC-C(CH₃)₃, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NHCOCH₃, -NHCOCF₃, -NHCOC₂H₅, -NHCOC₃H₇, -NHCOCH(CH₃)₂, -NHCOC(CH₃)₃, -CONH₂, -CONHCH₃, -CONHC₂H₅, -CONHC₃H₇, -CONHCH(CH₃)₂, -CONH-cyclo-C₃H₅, -CONHC(CH₃)₃, -CON(CH₃)₂, -CON(C₂H₅)₂, -CON(C₃H₇)₂, -CON[CH(CH₃)₂]₂, -CON[C(CH₃)₃]₂, -SO₂NH₂, -SO₂NHCH₃, -SO₂NHC₂H₅, -SO₂NHC₃H₇, -SO₂NHCH(CH₃)₂, -SO₂NH-cyclo-C₃H₅, -SO₂NHC(CH₃)₃, -SO₂N(CH₃)₂, -SO₂N(C₂H₅)₂, -SO₂N(C₃H₇)₂, -SO₂N[CH(CH₃)₂]₂, -SO₂N[C(CH₃)₃]₂, -NHSO₂CH₃, -NHSO₂CF₃, -NHSO₂C₂H₅, -NHSO₂C₃H₇, -NHSO₂CH(CH₃)₂, -NHSO₂C(CH₃)₃, -CH=CH₂, -CH₂-CH=CH₂, -C(CH₃)=CH₂, -CH=CH-CH₃, -C≡CH, -C≡C-CH₃, -CH₂-C≡CH, -Ph, -O-Ph, -O-CH₂-Ph, 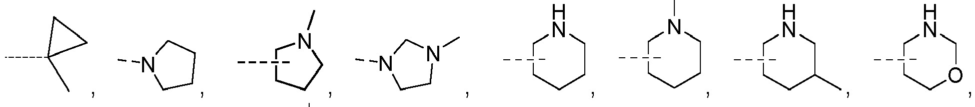 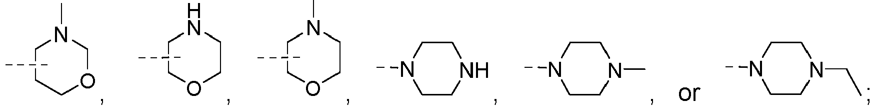
or **R⁸** and **R⁹** or **R⁹** and **R¹⁰** can form together one of the following five-membered or six-membered rings: 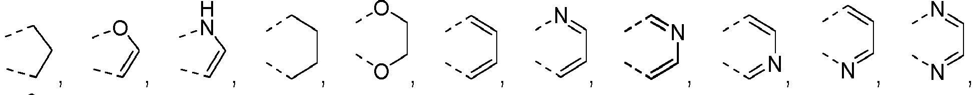 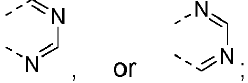
or **R¹²** and **R¹³** or **R¹³** and **R¹⁴** can form together one of the following five-membered or six-membered rings: 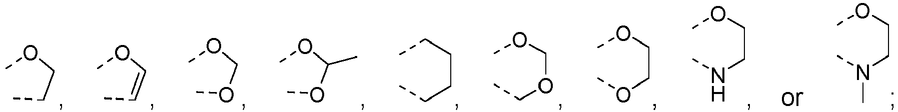
**R^{N}** represents -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -cyclo-C₄H₇, -cyclo-C₅H₉, -CH₂-cyclo-C₃H₅, -CH₂-cyclo-C₄H₇, -CH₂-cyclo-C₅H₉, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -CH₂-CH=CH₂, -CH₂-C≡CH, -CHO, -COCH₃, -COC₂H₅, -COC₃H₇, -COCH(CH₃)₂, -COC(CH₃)₃, -CO-cyclo-C₃H₅, -CO-cyclo-C₄H₇, -CO-cyclo-C₅H₉, -COOCH₃, -COOC₂H₅, -COOC₃H₇, -COOCH(CH₃)₂, -COOC(CH₃)₃, -COOCH₂Ph, -SO₂CH₃, -SO₂CF₃, -SO₂C₂H₅, -SO₂C₃H₇, -SO₂CH(CH₃)₂, -SO₂-cyclo-C₃H₅, or -SO₂C(CH₃)₃;
**R^{N1}** represent -H, -CH₃, or -CH₂CH₃;
or a diastereomer, an enantiomer, a mixture of diastereomers, a mixture of enantiomers, a racemate, a solvate, a hydrate, or a pharmaceutically acceptable salt thereof.

In one embodiment the herein disclosed inventive compounds, the moiety **-L-R³** is not

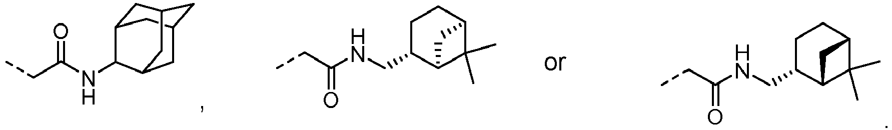

In one embodiment of the herein disclosed inventive compounds, **L** represents **-L¹-L²-;**
**L¹** represents -CH₂-, or -CH₂CO-; and
**L²** represents a bond, -NH-, -NHCH₂-, -NHCH₂CH₂-, or -NHCH(CH₃)-.

In one embodiment of the herein disclosed inventive compounds, **L** represents -CH₂-, -CH₂CO-NH-, -CH₂CO-NH-CH₂-, or -CH₂CO-NH-CH(CH₃)-.

In preferred embodiments of the inventive compound of formula (**I**), **R²** represents 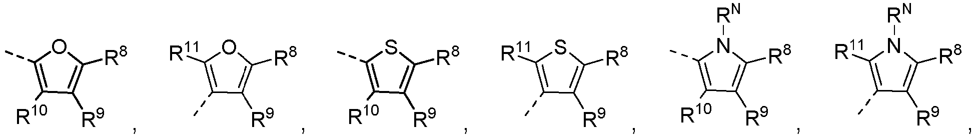 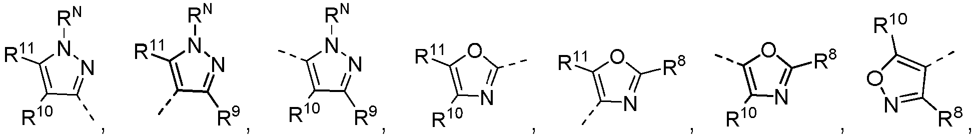 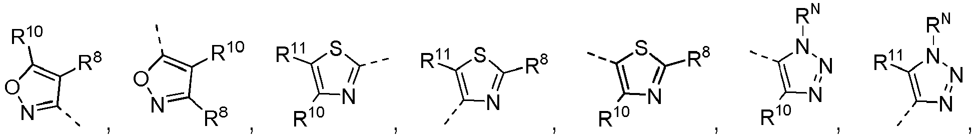 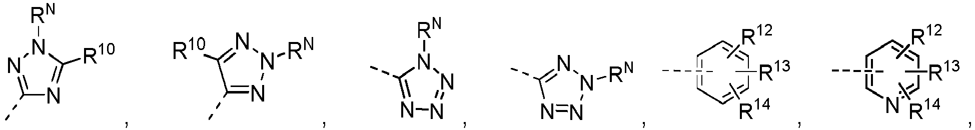 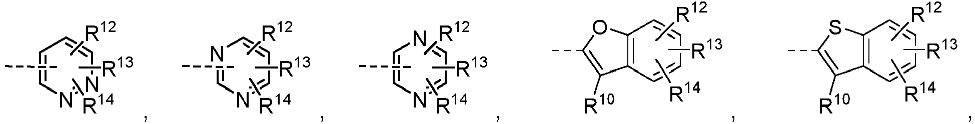 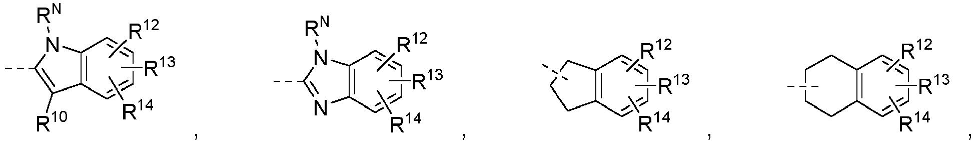 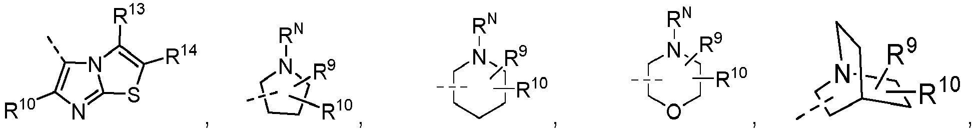 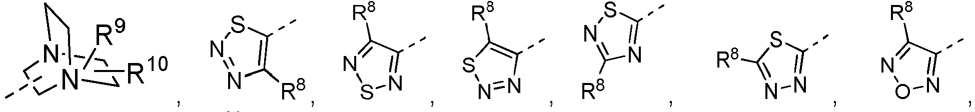 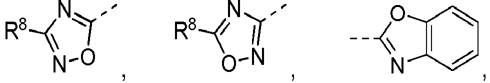 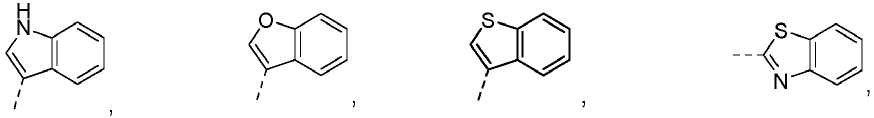 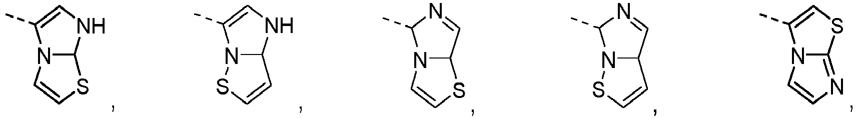 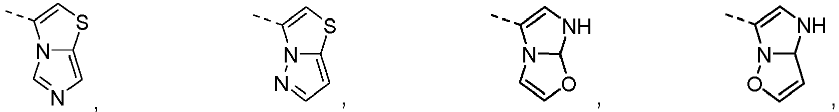 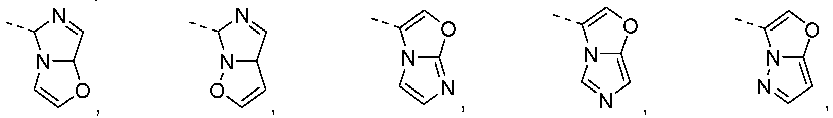 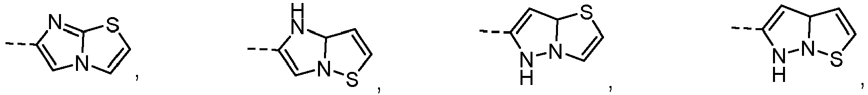 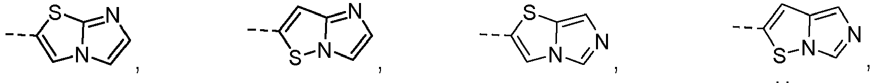 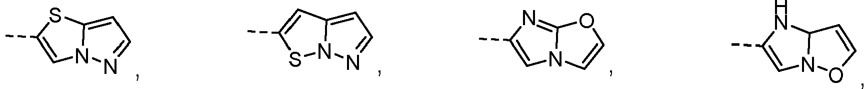 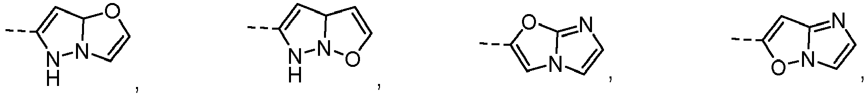 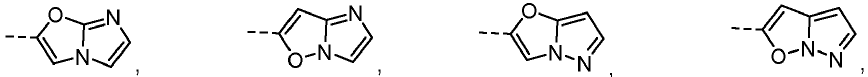 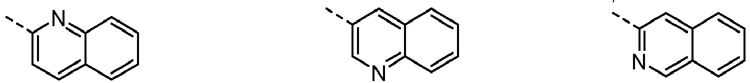 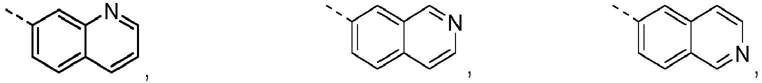 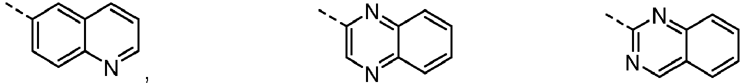 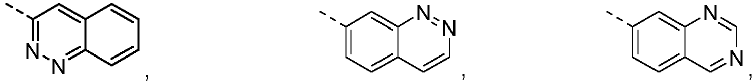 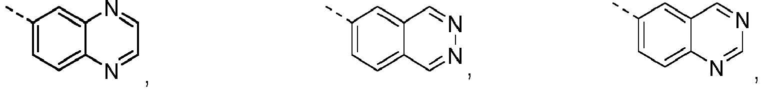 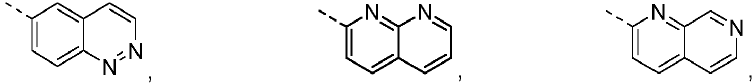 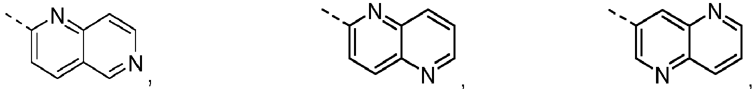 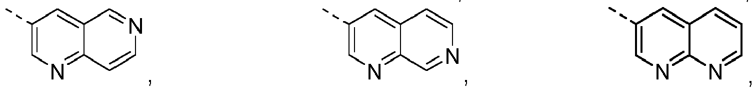 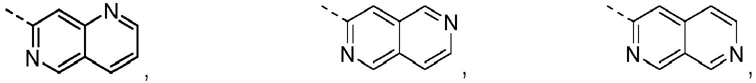 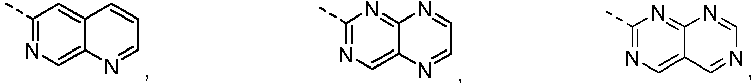 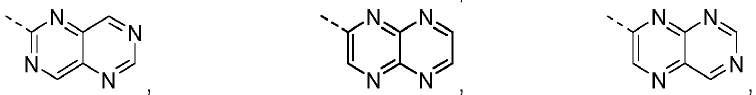 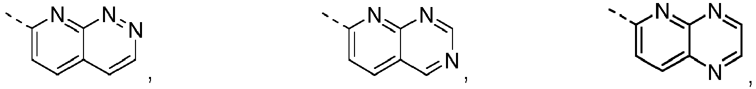 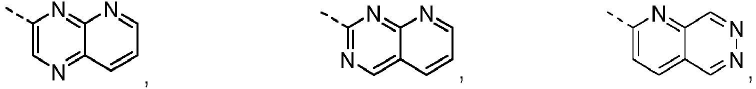 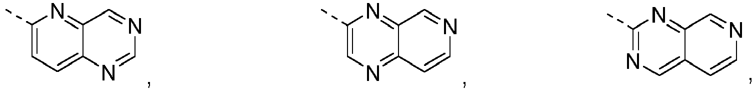 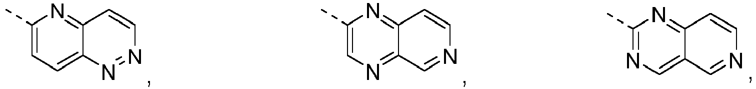 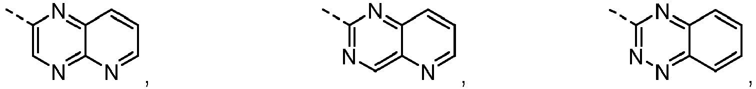 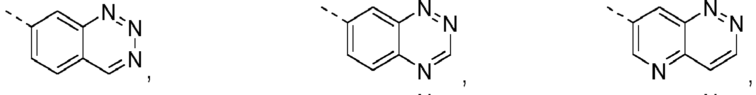 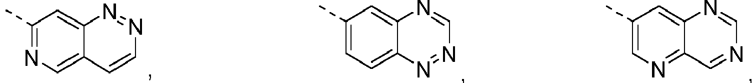 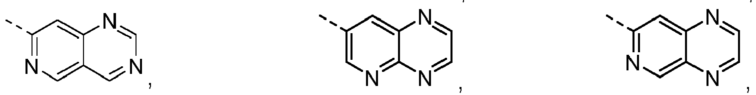 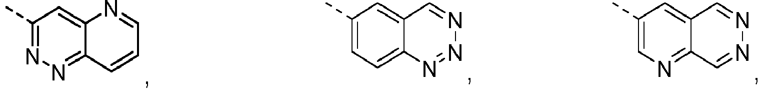 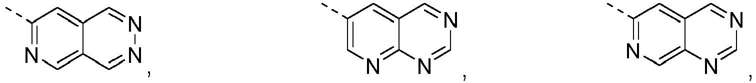 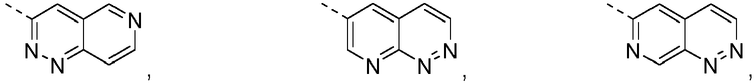 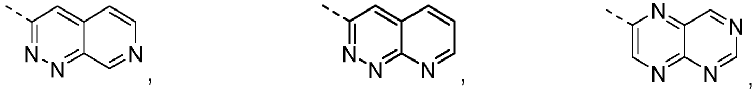 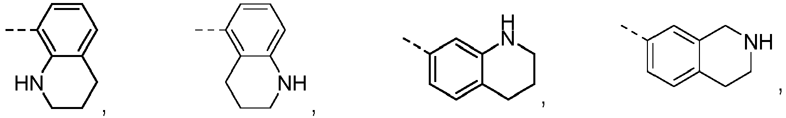 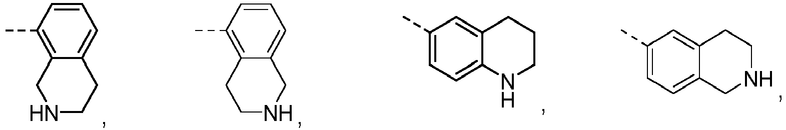 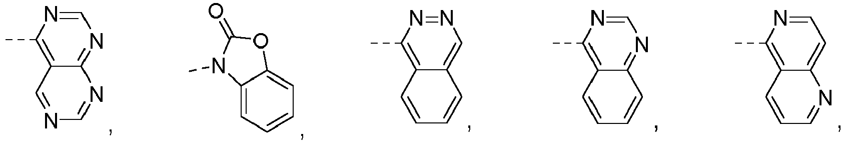 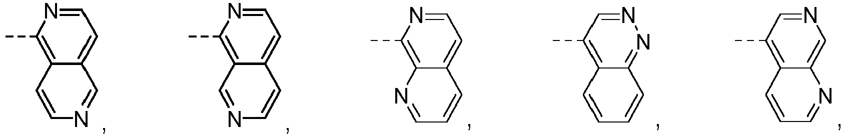 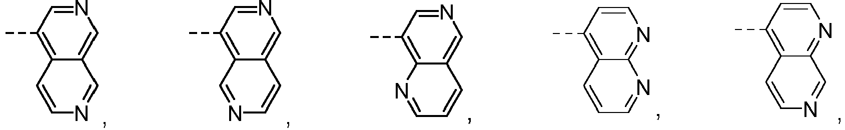 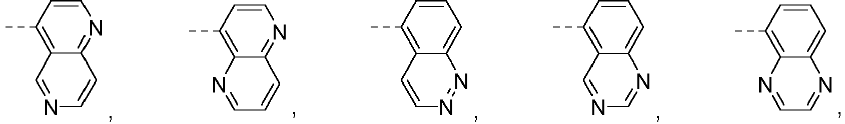 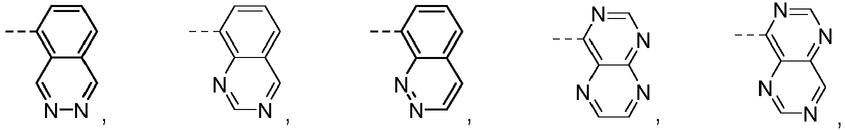 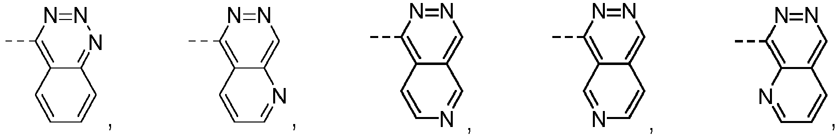 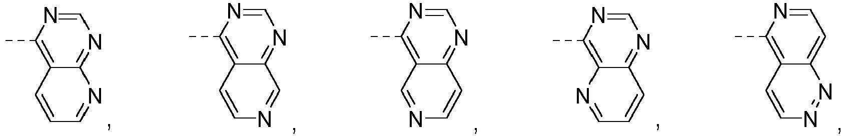 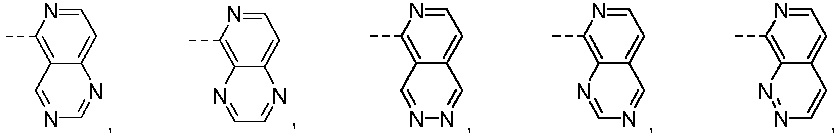 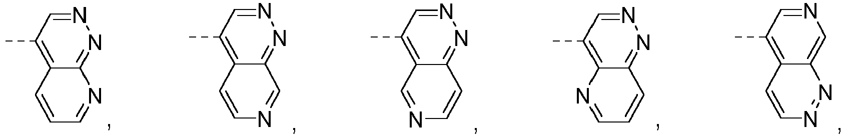 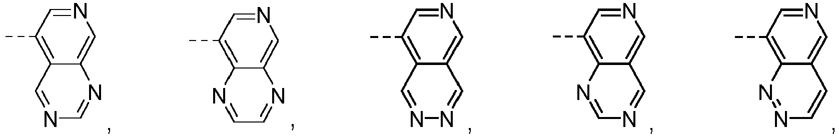 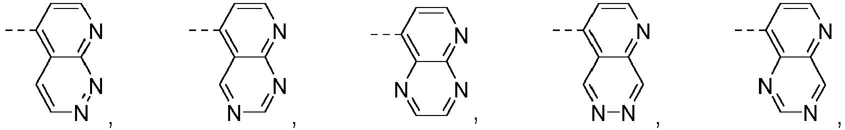 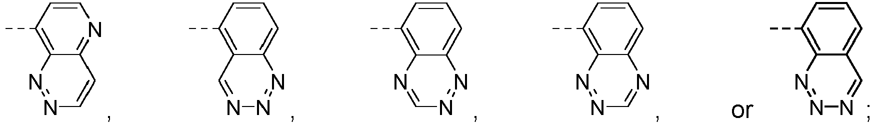 wherein the unsubstituted bicyclic residues can be substituted with 1 to 5 of the substituents **R⁹** - **R¹⁴** and **R^{N}** ; and preferably with 1 to 3 of the substituents **R¹¹** - **R¹³** and the substituents **R⁹** - **R¹⁴** and **R^{N3}** have the meanings as defined herein.

In even more preferred embodiments, **R²** represents: 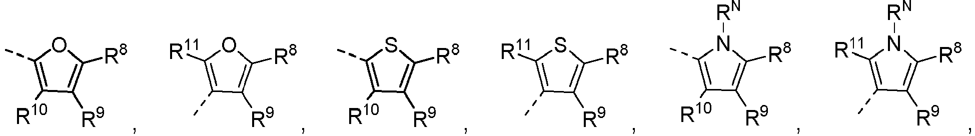 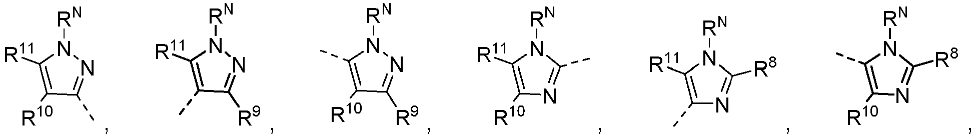 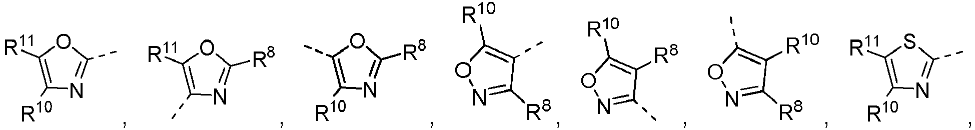 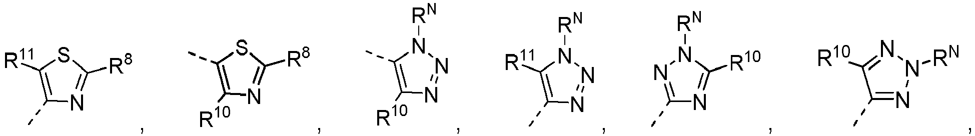 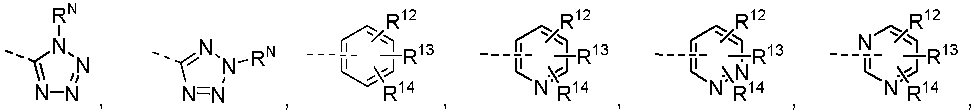 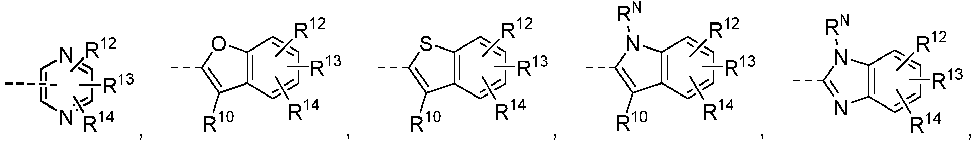 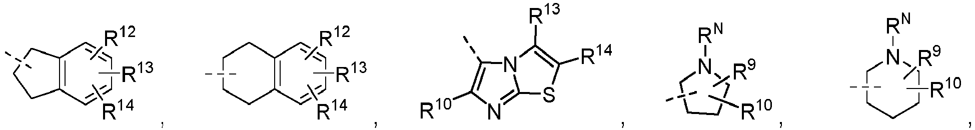 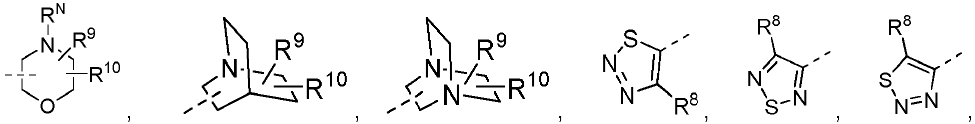 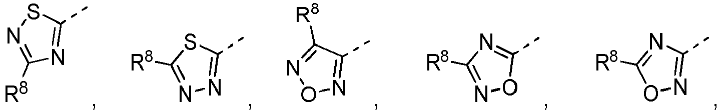 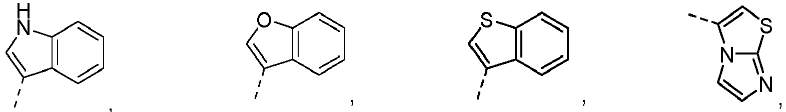 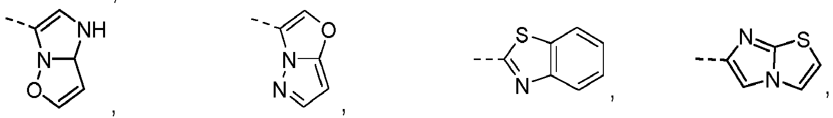 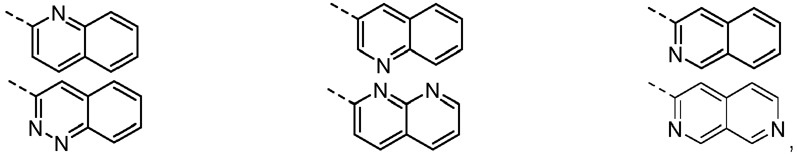 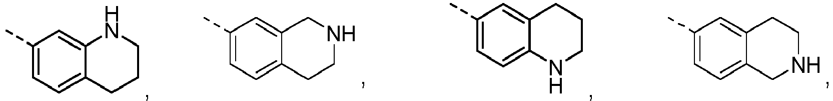 or 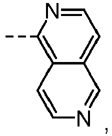 wherein the unsubstituted bicyclic residues can be substituted with 1 to 5 of the substituents **R⁹** - **R¹⁴** and **R^{N}** ; and preferably with 1 to 3 of the substituents **R¹¹** - **R¹³** and the substituents **R⁹** - **R¹⁴** and **R^{N}** have the meanings as defined herein.

Thus, the present invention relates to compounds of the general formula (I): 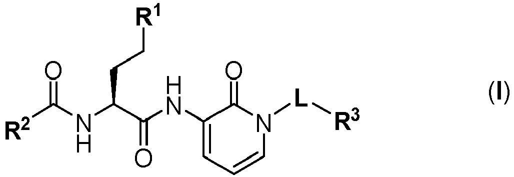 wherein
**L** represents **-L¹-** or **-L¹-L²-;** preferably **-L¹-L²-;**
**L¹** represents -CH₂-, or -CH₂CO-;
**L²** represents a bond, -NH-, -NHCH₂-, -NHCH₂CH₂-, or -NHCH(CH₃)-,
**R¹** represents 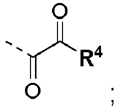
**R²** represents 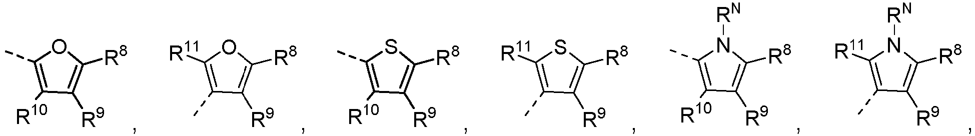 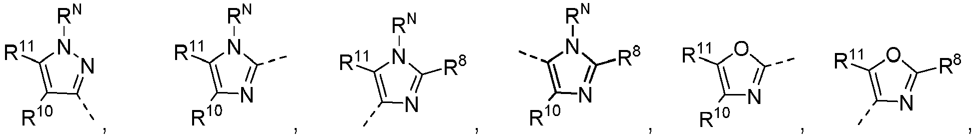 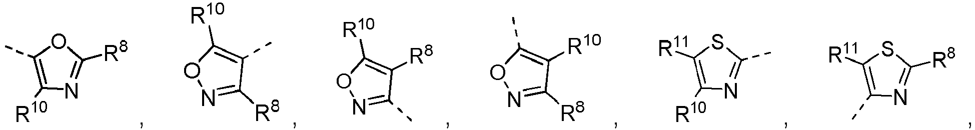 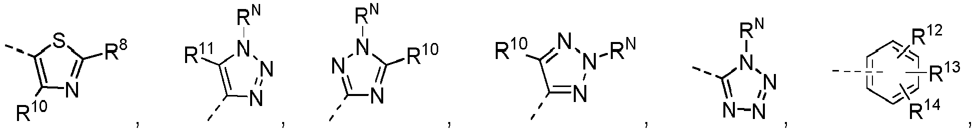 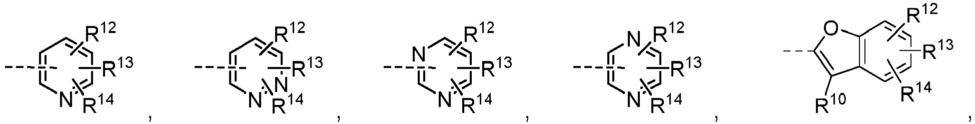 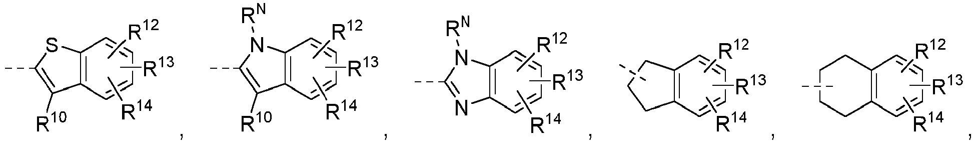 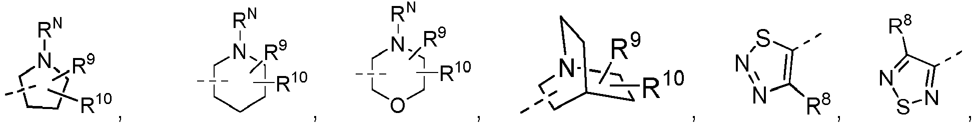 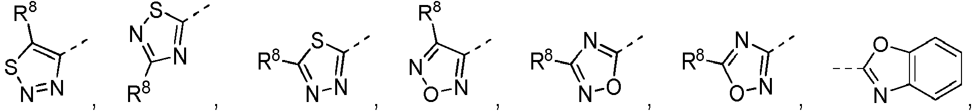 or 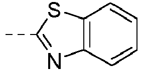
**R³** represents bicyclo[1.1.1]pentyl, bicyclo[2.1.1]hexyl, bicyclo[2.2.1]heptyl, bicyclo[3.1.1]heptyl, bicyclo[2.2.2]octyl, bicyclo[3.2.1]octyl, bicyclo[3.2.2]nonyl, bicyclo[3.3.2]decyl, bicyclo[3.3.3]undecyl, 4-homoisotwistyl, adamantyl, diamantyl, hexamethylenetetraminyl and the afore-mentioned residues optionally contain one or more C=C double bond(s) and/or are optionally substituted by one or more of **R^{a}, R^{b}, R^{c}, R^{d},** and **R^{e};**
**R^{a}, R^{b}, R^{c}, R^{d},** and **R^{e}** represents independently of each other -H, -F, -Cl, -Br, -CN, -OH, -CH₃, -CH₂CH₃, -CH₂CH₂CH₃, -CH(CH₃)₂, -CHF₂, -CF₃, -CH₂CF₃, -COCH₃, -COCH₂CH₃, -CO₂H, -CO₂CH₃, -CO₂C₂H₅, -CONH₂, -CONHCH₃, -CON(CH₃)₂, -CONHC₂H₅, -CH₂CO₂H, -CH₂CO₂CH₃, -CH₂CO₂C₂H₅, -CH₂CONH₂, -CH₂CONHCH₃, -CH₂CON(CH₃)₂, -CH₂CONHC₂H₅, -NHCOCH₃, -NHCOC₂H₅, -NHCOCF₃, -NHCOCH₂CF₃, -NHSO₂CH₃, -NHSO₂C₂H₅, -NHSO₂CHF₂, -NHSO₂CF₃, -NHSO₂CH₂CF₃;
**R⁴** represents **-NR⁶R⁷;**
**R⁶** and **R⁷** represent independently of each other -H, -CH₃, -CH₂CH₂CH₃, -CH(CH₃)₂, -CH₂CH₂CH₂CH₃, -CH₂CH₂CH₂CH₂CH₃, -CH₂CH(CH₃)₂, -CH₂CH=CH₂, -CH₂CH=CH(CH₃), -CH₂CH=C(CH₃)₂, -CH₂CH=CHCH₂CH₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -CH₂OCH₃, -CH₂OCH₂CH₃, -CH₂CH₂OCH₃, -CH₂CH₂OCH₂CH₃, -CH₂CH₂NHCH₃, -CH₂CH₂N(CH₃)₂,
or **-NR⁶R⁷** is -N(C₂H₅)₂, 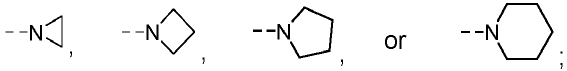
**R⁸, R⁹, R¹⁰, R¹¹, R¹², R¹³,** and **R¹⁴** represent independently of each other -H, -F, -Cl, -Br, -OH, -CN, -NO₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -CH₂-cyclo-C₃H₅, -CH₂OH, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -OCH₃, -OC₂H₅, -OC₃H₇, -OCH(CH₃)₂, -OC(CH₃)₃, -OC₄H₉, -OCHF₂, -OCF₃, -OCH₂CF₃, -OC₂F₅, -OCH₂OCH₃, -O-cyclo-C₃H₅, -OCH₂-cyclo-C₃H₅, -O-C₂H₄-cyclo-C₃H₅, -CHO, -COCH₃, -COCF₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, -COOC(CH₃)₃, -OOC-CH₃, -OOC-CF₃, -OOC-C₂H₅, -OOC-C₃H₇, -OOC-CH(CH₃)₂, -OOC-C(CH₃)₃, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NHCOCH₃, -NHCOCF₃, -NHCOC₂H₅, -NHCOC₃H₇, -NHCOCH(CH₃)₂, -NHCOC(CH₃)₃, -CONH₂, -CONHCH₃, -CONHC₂H₅, -CONHC₃H₇, -CONHCH(CH₃)₂, -CONH-cyclo-C₃H₅, -CONHC(CH₃)₃, -CON(CH₃)₂, -CON(C₂H₅)₂, -CON(C₃H₇)₂, -CON[CH(CH₃)₂]₂, -CON[C(CH₃)₃]₂, -SO₂NH₂, -SO₂NHCH₃, -SO₂NHC₂H₅, -SO₂NHC₃H₇, -SO₂NHCH(CH₃)₂, -SO₂NH-cyclo-C₃H₅, -SO₂NHC(CH₃)₃, -SO₂N(CH₃)₂, -SO₂N(C₂H₅)₂, -SO₂N(C₃H₇)₂, -SO₂N[CH(CH₃)₂]₂, -SO₂N[C(CH₃)₃]₂, -NHSO₂CH₃, -NHSO₂CF₃, -NHSO₂C₂H₅, -NHSO₂C₃H₇, -NHSO₂CH(CH₃)₂, -NHSO₂C(CH₃)₃, -CH=CH₂, -CH₂-CH=CH₂, -C(CH₃)=CH₂, -CH=CH-CH₃, -C≡CH, -C≡C-CH₃, -CH₂-C≡CH, -Ph, -O-Ph, -O-CH₂-Ph, 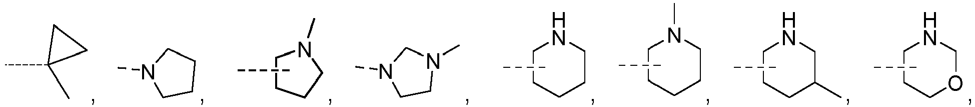 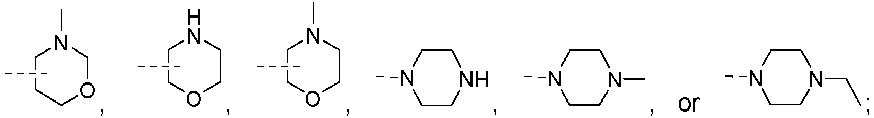
or **R⁸** and **R⁹** or **R⁹** and **R¹⁰** can form together one of the following five-membered or six-membered rings: 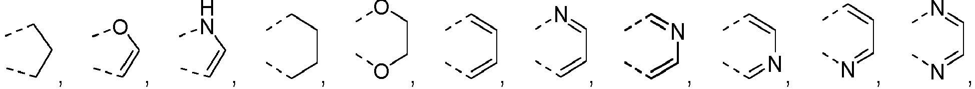 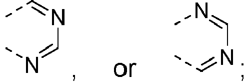
or R¹² and R¹³ or R¹³ and R¹⁴ can form together one of the following five-membered or six-membered rings: 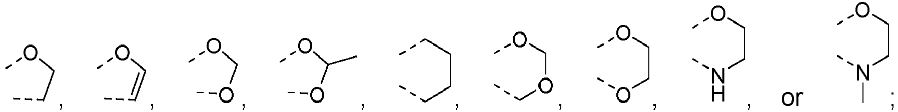
**R^{N}** represents -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -cyclo-C₄H₇, -cyclo-C₅H₉, -CH₂-cyclo-C₃H₅, -CH₂-cyclo-C₄H₇, -CH₂-cyclo-C₅H₉, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -CH₂-CH=CH₂, -CH₂-C≡CH, -CHO, -COCH₃, -COC₂H₅, -COC₃H₇, -COCH(CH₃)₂, -COC(CH₃)₃, -CO-cyclo-C₃H₅, -CO-cyclo-C₄H₇, -CO-cyclo-C₅H₉, -COOCH₃, -COOC₂H₅, -COOC₃H₇, -COOCH(CH₃)₂, -COOC(CH₃)₃, -COOCH₂Ph, -SO₂CH₃, -SO₂CF₃, -SO₂C₂H₅, -SO₂C₃H₇, -SO₂CH(CH₃)₂, -SO₂-cyclo-C₃H₅, or -SO₂C(CH₃)₃;
or a diastereomer, an enantiomer, a mixture of diastereomers, a mixture of enantiomers, a racemate, a solvate, a hydrate, or a pharmaceutically acceptable salt thereof.

Preferred are the compounds of the formula (**Ib**): 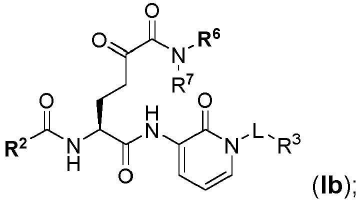 and **L, R², R³, R⁶, R⁷** have the same meanings as defined in the formula (**I**)

Preferred are the compounds of the formula (**Ib**): 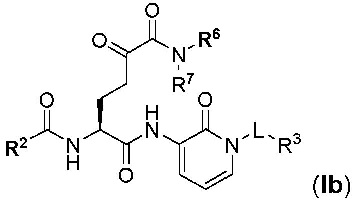 wherein
**L** represents **-L¹-** or **-L¹-L²-;** preferably **-L¹-L²-;;**
**L¹** represents -CH₂-, -CH₂CH₂-, -CH₂CH₂CH₂-, -CH₂CO-, or -CH₂CH₂CO-;
**L²** represents a bond, -NR^{N1}-, -NR^{N1}CH₂-, -NR^{N1}CH₂CH₂-, or -NR^{N1}CH(CH₃)-;
**R²** represents 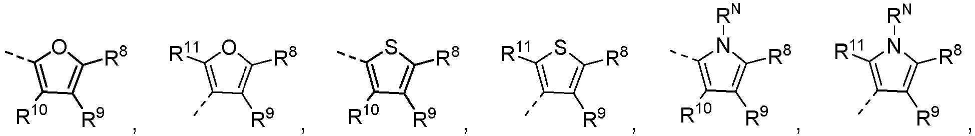 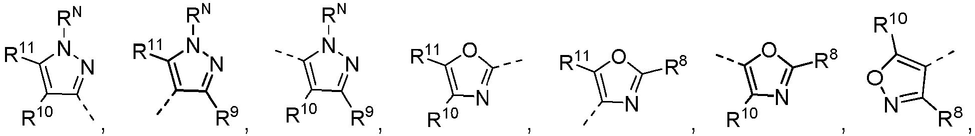 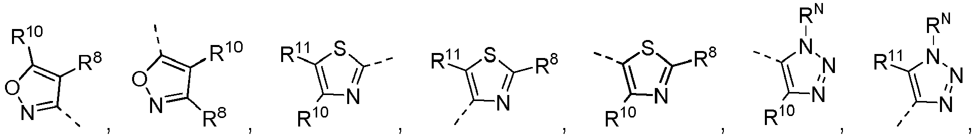 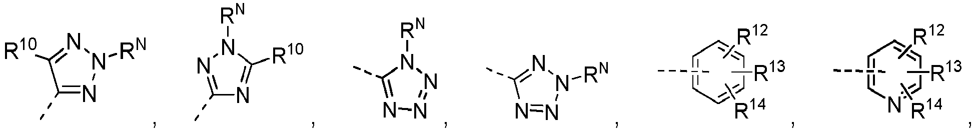 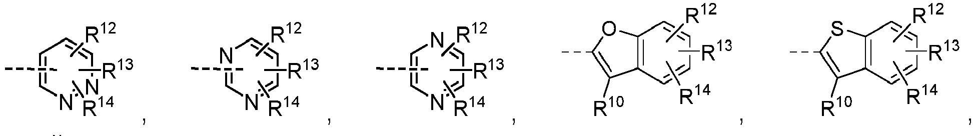 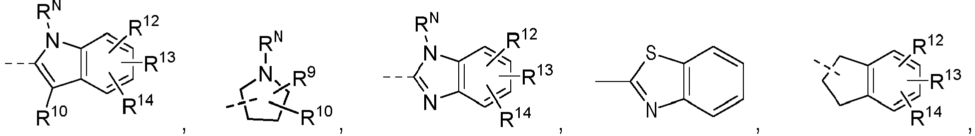 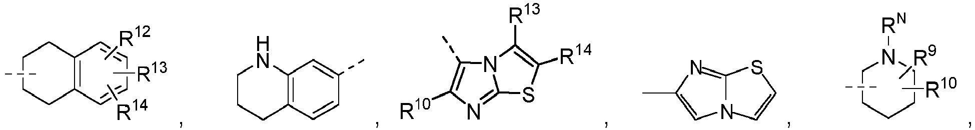 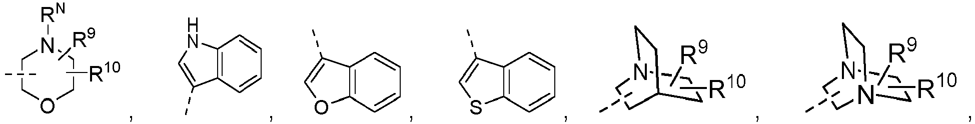 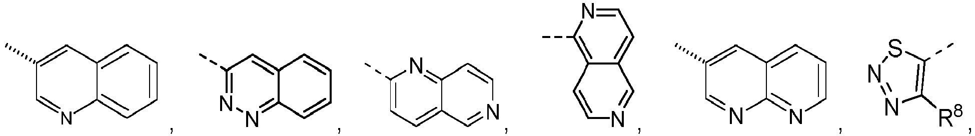 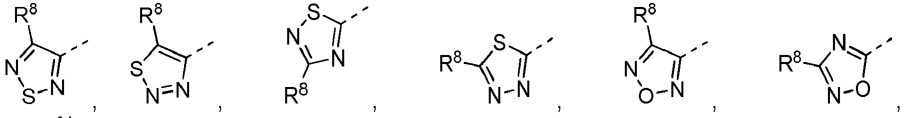 or 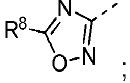
**R³** represents bicyclo[1.1.1]pentyl, bicyclo[2.1.1]hexyl, bicyclo[2.2.1]heptyl, bicyclo[3.1.1]heptyl, bicyclo[2.2.2]octyl, bicyclo[3.2.1]octyl, bicyclo[3.2.2]nonyl, bicyclo[3.3.2]decyl, bicyclo[3.3.3]undecyl, 4-homoisotwistyl, adamantyl, diamantyl, hexamethylenetetraminyl, and the afore-mentioned residues optionally contain one or more C=C double bond(s) and/or are optionally substituted with one or more of **R^{a}, R^{b}, R^{c}, R^{d},** and **R^{e};**
**R^{a}, R^{b}, R^{c}, R^{d},** and **R^{e}** represents independently of each other -H, -F, -Cl, -Br, -CN, -OH, -CH₃, -CH₂CH₃, -CH₂CH₂CH₃, -CH(CH₃)₂, -CHF₂, -CF₃, -CH₂CF₃, -COCH₃, -COCH₂CH₃, -CO₂H, -CO₂CH₃, -CO₂C₂H₅, -CONH₂, -CONHCH₃, -CON(CH₃)₂, -CONHC₂H₅, -CH₂CO₂H, -CH₂CO₂CH₃, -CH₂CO₂C₂H₅, -CH₂CONH₂, -CH₂CONHCH₃, -CH₂CON(CH₃)₂, -CH₂CONHC₂H₅, -NHCOCH₃, -NHCOC₂H₅, -NHCOCF₃, -NHCOCH₂CF₃, -NHSO₂CH₃, -NHSO₂C₂H₅, -NHSO₂CHF₂, -NHSO₂CF₃, -NHSO₂CH₂CF₃;
**R⁴** represents **-NR⁶R⁷;**
**R⁶** and **R⁷** represent independently of each other -H, -CH₃, -CH₂CH₂CH₃, -CH(CH₃)₂, -CH₂CH₂CH₂CH₃, -CH₂CH₂CH₂CH₂CH₃, -CH₂CH(CH₃)₂, -C(CH₃)₃, -CH₂CH=CH₂, -CH₂CH=CH(CH₃), -CH₂CH=C(CH₃)₂, -CH₂CH=CHCH₂CH₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -Ph, -CH₂-Ph, -CH₂OCH₃, -CH₂OCH₂CH₃, -CH₂CH₂OCH₃, -CH₂CH₂OCH₂CH₃, -CH₂CH₂NHCH₃, -CH₂CH₂N(CH₃)₂,
or -N**R⁶R⁷** is -N(C₂H₅)₂, 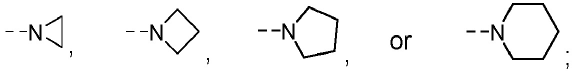
**R^{N}** represents -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -cyclo-C₄H₇, -cyclo-C₅H₉, -CH₂-cyclo-C₃H₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -CH₂-CH=CH₂, -CH₂-C=CH, -CHO, -COCH₃, -COC₂H₅, -COC₃H₇, -COCH(CH₃)₂, -COC(CH₃)₃, -COOCH₃, -COOC₂H₅, -COOC₃H₇, -COOCH(CH₃)₂, -COOC(CH₃)₃, -COOCH₂Ph, -SO₂CH₃, -SO₂CF₃, -SO₂C₂H₅, -SO₂C₃H₇, -SO₂CH(CH₃)₂, or -SO₂C(CH₃)₃;
**R^{N1}** represent -H, -CH₃, or -CH₂CH₃;
and **R⁸** - **R¹⁴** have the meanings as defined above for formula (**I**);
or a diastereomer, an enantiomer, a mixture of diastereomers, a mixture of enantiomers, a racemate, a solvate, a hydrate, or a pharmaceutically acceptable salt thereof.

Preferably, **R²** represents

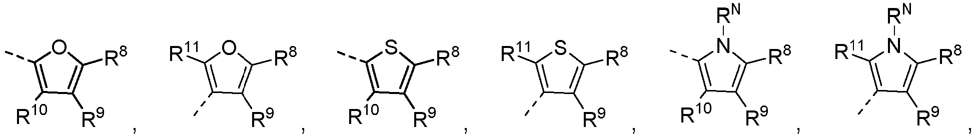

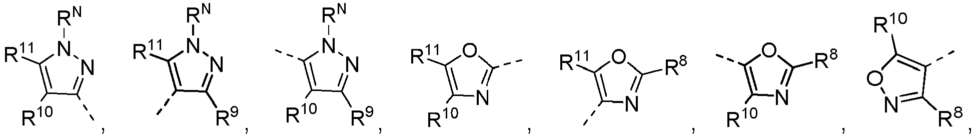

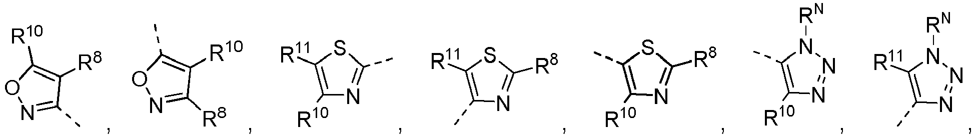

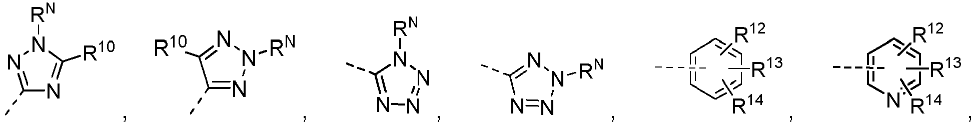

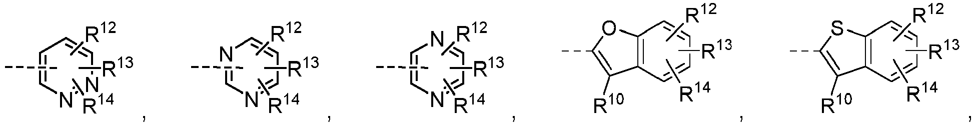

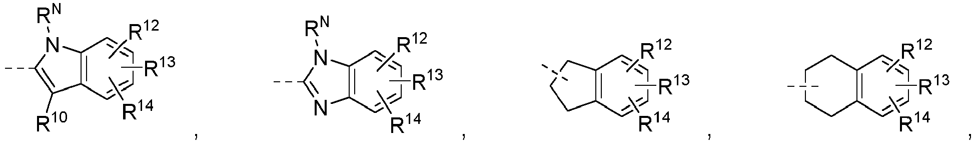

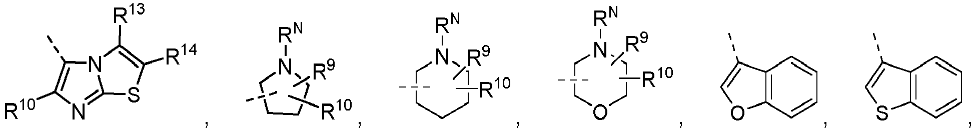

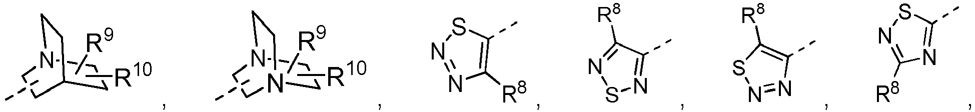

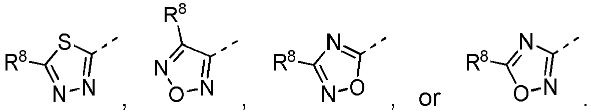

Preferably, -N**R⁶R⁷** of the formula (**Ib**) represents -NH₂, -NHCH₃, -N(CH₃)₂, -NHCH(CH₃)₂, -NHCH₂CH₂CH₃, -NH-CH₂CH=CH₂, -NHCH₂CH₂CH₂CH₃, -NHCH₂CH(CH₃)₂, -NHC(CH₃)₃, -NHCH₂CH₂CH₂CH₂CH₃, -NH-cyclo-C₃H₅, -NH-cyclo-C₄H₇, -NH-cyclo-C₅H₉, -NH-cyclo-C₆H₁₁, -NHCH₂-cyclo-C₃H₅, -NHCH₂-cyclo-C₄H₇, -NHCH₂-cyclo-C₅H₉, -NHCH₂-cyclo-C₆H₁₁, -NHCH₂-Ph, -NHCH₂OCH₃, -NHCH₂OCH₂CH₃, -NHCH₂CH₂OCH₃, -NHCH₂CH₂NHCH₃, -NHCH₂CH₂N(CH₃)₂,

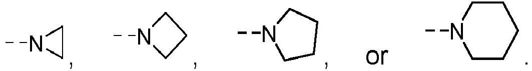

In some embodiments, the present invention relates to the compound of the formula (**I**), 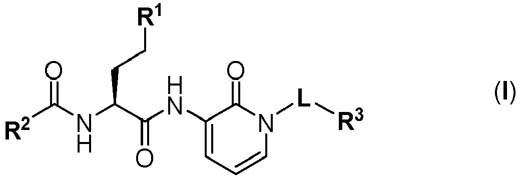 wherein
**L** represents -**L¹**- or -**L¹**-**L²**-; preferably -**L¹**-**L²**-;;
**L¹** represents -CH₂-, -CH₂CH₂-, -CH₂CH₂CH₂-, -CH₂CO-, or -CH₂CH₂CO-;
**L²** represents a bond, -NR^{N1}-, -NR^{N1}CH₂-, -NR^{N1}CH₂CH₂-, or -NR^{N1}CH(CH₃)-;
**R¹** represents 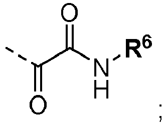
**R²** represents 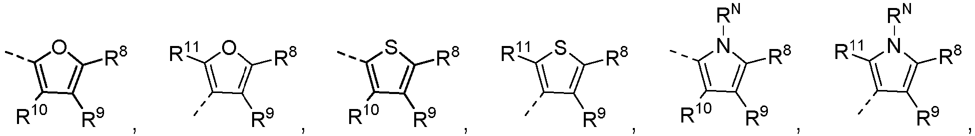 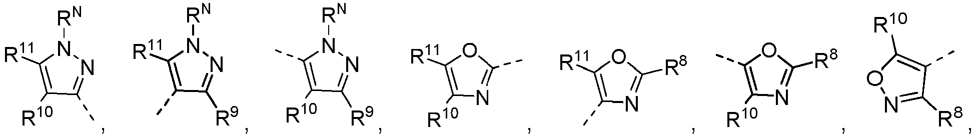 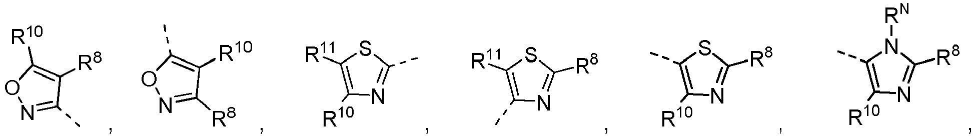 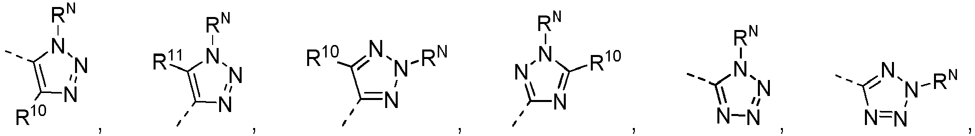 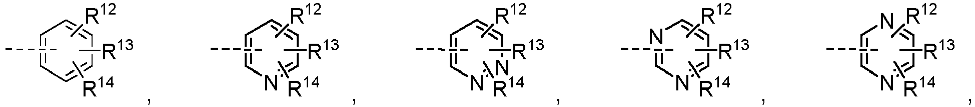 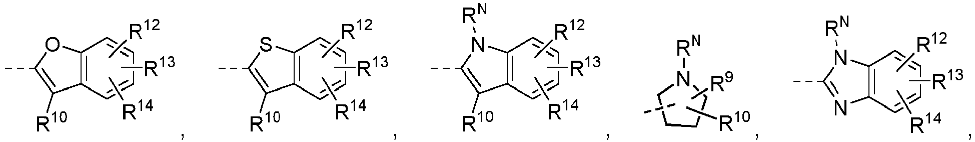 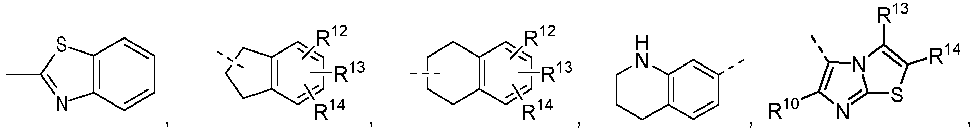 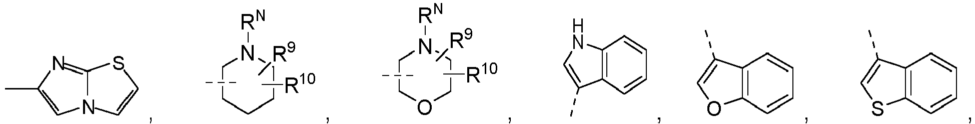 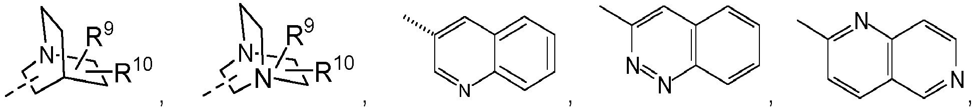 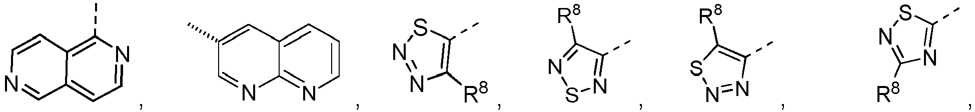 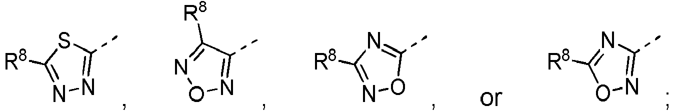
**R³** represents bicyclo[1.1.1]pentyl, bicyclo[2.1.1]hexyl, bicyclo[2.2.1]heptyl, bicyclo[3.1.1]heptyl, bicyclo[2.2.2]octyl, bicyclo[3.2.1]octyl, bicyclo[3.2.2]nonyl, bicyclo[3.3.2]decyl, bicyclo[3.3.3]undecyl, 4-homoisotwistyl, adamantyl, diamantyl, hexamethylenetetraminyl, and the afore-mentioned residues optionally contain one or more C=C double bond(s) and/or are substituted with one or more of **R^{a}, R^{b}, R^{c}, R^{d},** and **R^{e}**;
**R⁶** represents -H, -CH₃, -CH₂CH=CH₂, -cyclo-C₃H₅, -CH₂CH₂CH₂CH₂CH₃;
and **R⁸** - **R¹⁴, R^{a}, R^{b}, R^{c}, R^{d}, R^{e}, R^{N},** and **R^{N1}** have the meanings and preferred meanings as defined herein.

Also preferred are compounds of the general formula (**I**), 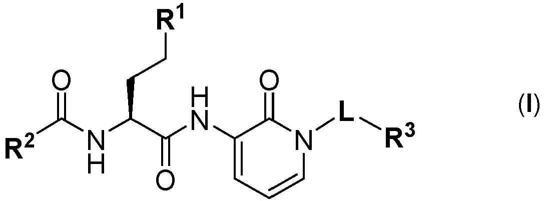 wherein
**L** represents -**L¹**- or -**L¹**-**L²**- ;
**L¹** represents -CH₂-, -CH₂CH₂-, -CH₂CH₂CH₂-, -CH₂CO-, -CH₂CH₂CO-;
**L²** is a bond, -N**R^{N1}**-, -N**R^{N1}**CH₂-, -N**R^{N1}**CH₂CH₂-, or -NR^{N1}CH(CH₃)-;
**R¹** represents 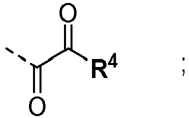
**R²** represents 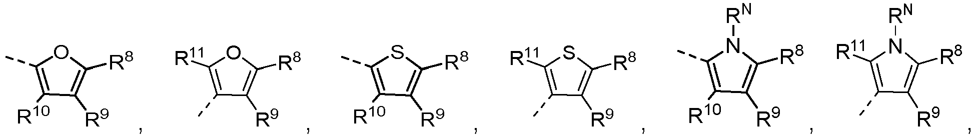 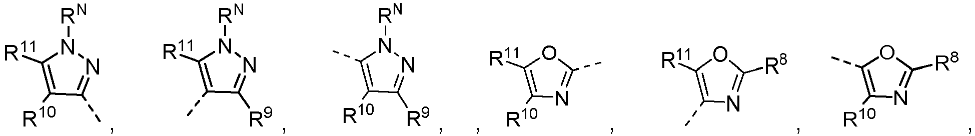 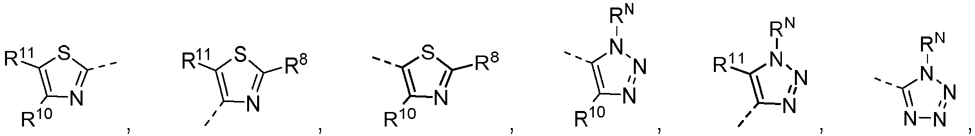 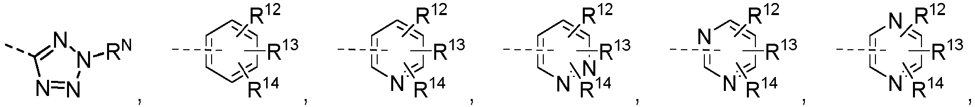 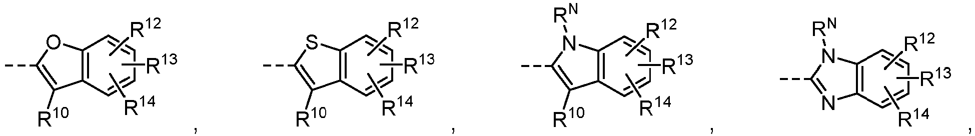 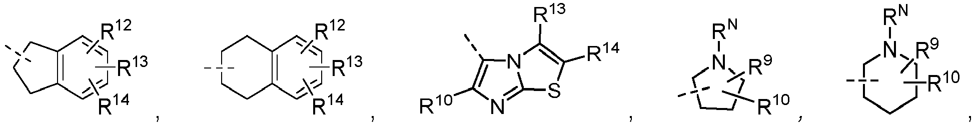 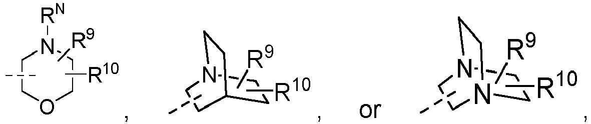
**R³** represents bicyclo[1.1.1]pentyl, bicyclo[2.1.1]hexyl, bicyclo[2.2.1]heptyl, bicyclo[3.1.1]heptyl, bicyclo[2.2.2]octyl, bicyclo[3.2.1]octyl, bicyclo[3.2.2]nonyl, bicyclo[3.3.2]decyl, bicyclo[3.3.3]undecyl, 4-homoisotwistyl, adamantyl, diamantyl, hexamethylenetetraminyl, and the afore-mentioned residues optionally contain one or more C=C double bond and/or are substituted one or more of **R^{a}, R^{b}, R^{c}, R^{d},** and **R^{e}**;
**R^{a}, R^{b}, R^{c}, R^{d},** and **R^{e}** represents independently of each other -H, -F, -Cl, -Br, -CN, -OH, -CH₃, -CH₂CH₃, -CH₂CH₂CH₃, -CH(CH₃)₂, -CHF₂, -CF₃, -CH₂CF₃, -COCH₃, -COCH₂CH₃, -CO₂H, -CO₂CH₃, -CO₂C₂H₅, -CONH₂, -CONHCH₃, -CON(CH₃)₂, -CONHC₂H₅, -CH₂CO₂H, -CH₂CO₂CH₃, -CH₂CO₂C₂H₅, -CH₂CONH₂, -CH₂CONHCH₃, -CH₂CON(CH₃)₂, -CH₂CONHC₂H₅, -NHCOCH₃, -NHCOC₂H₅, -NHCOCF₃, -NHCOCH₂CF₃, -NHSO₂CH₃, -NHSO₂C₂H₅, -NHSO₂CHF₂, -NHSO₂CF₃, -NHSO₂CH₂CF₃;
**R⁴** represents -N**R⁶R⁷**;
**R⁶** and **R⁷** represent independently of each other -H, -CH₃, -CH₂CH₂CH₃, -CH(CH₃)₂, -CH₂CH₂CH₂CH₃, -CH₂CH₂CH₂CH₂CH₃, -CH₂CH(CH₃)₂, -C(CH₃)₃, -CH₂CH=CH₂, -CH₂CH=CH(CH₃), -CH₂CH=C(CH₃)₂, -CH₂CH=CHCH₂CH₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -CH₂-Ph, -CH₂OCH₃, -CH₂OCH₂CH₃, -CH₂CH₂OCH₃, -CH₂CH₂OCH₂CH₃, -CH₂CH₂NHCH₃, -CH₂CH₂N(CH₃)₂,
or -N**R⁶R⁷** represents 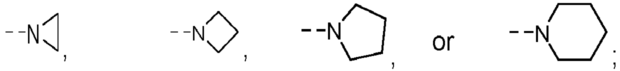
**R^{N}** represents -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -cyclo-C₄H₇, -cyclo-C₅H₉, -CH₂-cyclo-C₃H₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -CH₂-CH=CH₂, -CH₂-C≡CH, -CHO, -COCH₃, -COC₂H₅, -COC₃H₇, -COCH(CH₃)₂, -COC(CH₃)₃, -COOCH₃, -COOC₂H₅, -COOC₃H₇, -COOCH(CH₃)₂, -COOC(CH₃)₃, -COOCH₂Ph, -SO₂CH₃, -SO₂CF₃, -SO₂C₂H₅, -SO₂C₃H₇, -SO₂CH(CH₃)₂, or -SO₂C(CH₃)₃;
**R^{N1}** represent -H, -CH₃, or -CH₂CH₃;
or a diastereomer, an enantiomer, a mixture of diastereomers, a mixture of enantiomers, a racemate, a solvate, a hydrate, or a pharmaceutically acceptable salt thereof.

Also preferred are compounds of the formula (**I**) or (**Ib**), wherein
**L¹** represents -CH₂-, or -CH₂CO-;
**L²** represents a bond, -N**R^{N1}**-, -N**R^{N1}**CH₂-, or -NR^{N1}CH(CH₃)-;
**R³** represents bicyclo[1.1.1]pentyl, bicyclo[2.1.1]hexyl, bicyclo[2.2.1]heptyl, bicyclo[3.1.1]heptyl, bicyclo[2.2.2]octyl, 4-homoisotwistyl, adamantyl, or diamantyl, and the afore-mentioned residues optionally contain one or more C=C double bond(s) and/or are substituted by one or more of **R^{a}, R^{b}, R^{c}, R^{d},** and **R^{e}**; and
**R^{a}, R^{b}, R^{c}, R^{d}, R^{e}** and **R^{N1}** have the same meanings as defined herein.

Preferably, **R²** of the formula (**I**) or (**Ib**) represents 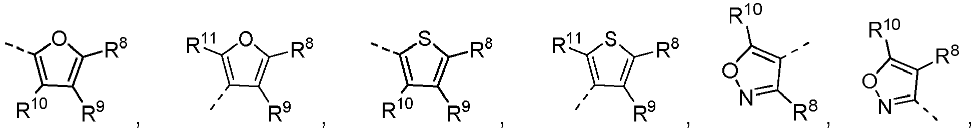 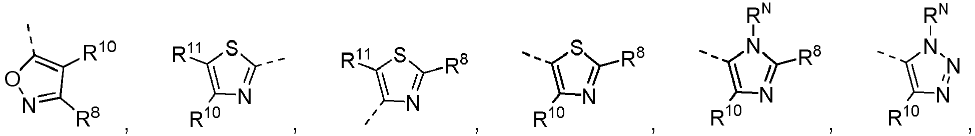 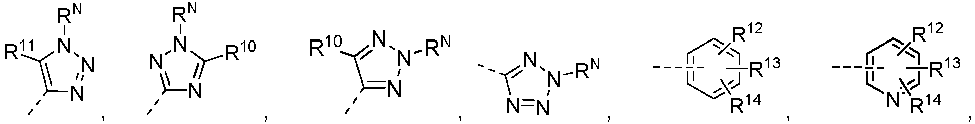 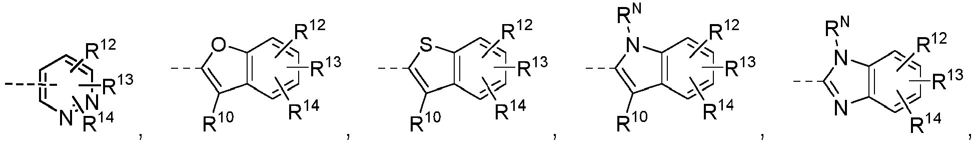 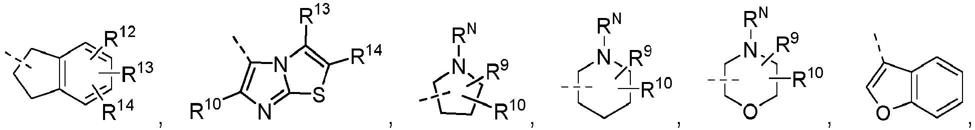 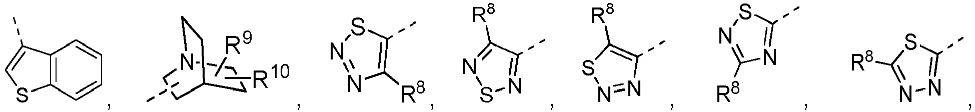 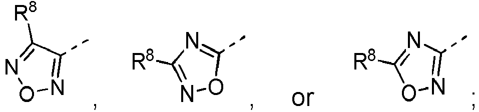 and **R⁸** - **R¹⁴** and **R^{N}** have the meanings as defined in formula (**I**) or (**Ib**).

Preferred are compounds having any one of the formulae (**IV-a**) - (**IV-o**) and (**V-a**) - (**V-d**): 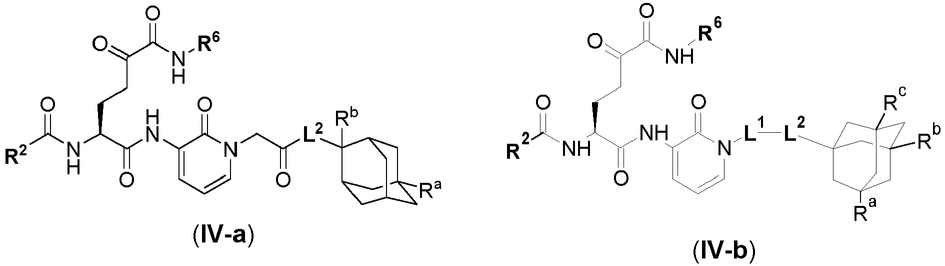 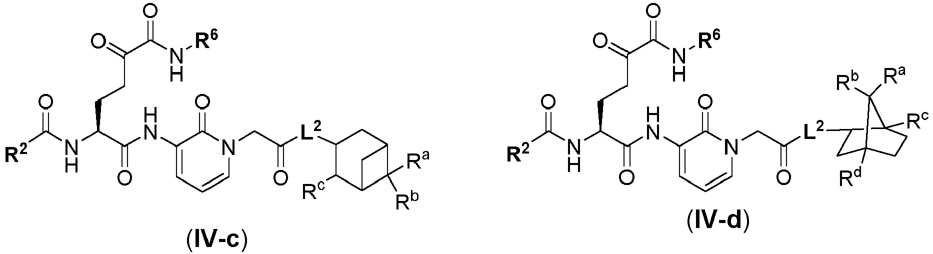 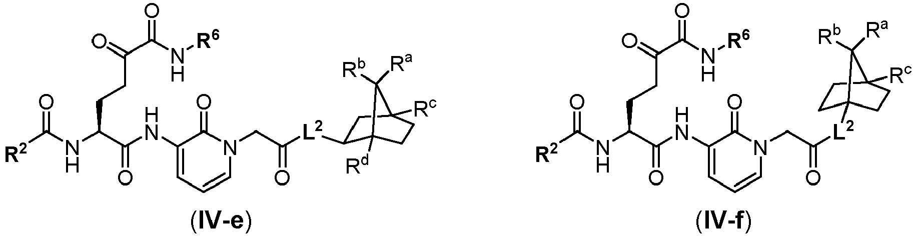 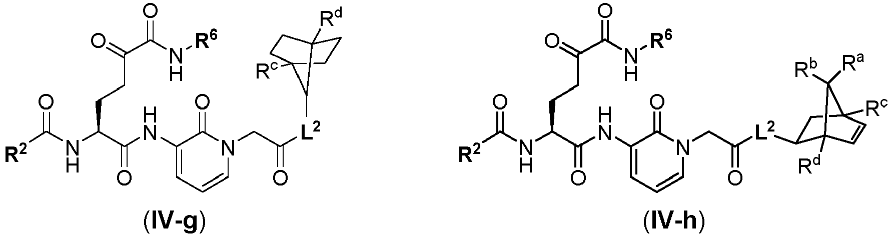 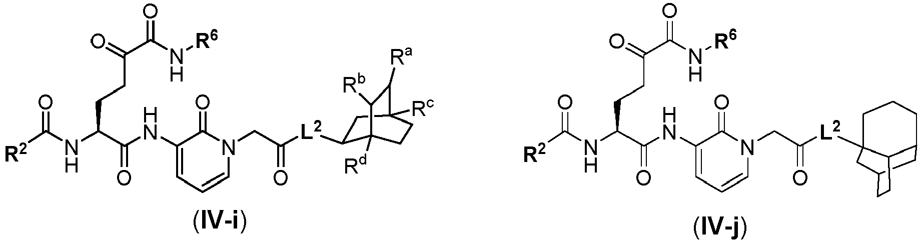 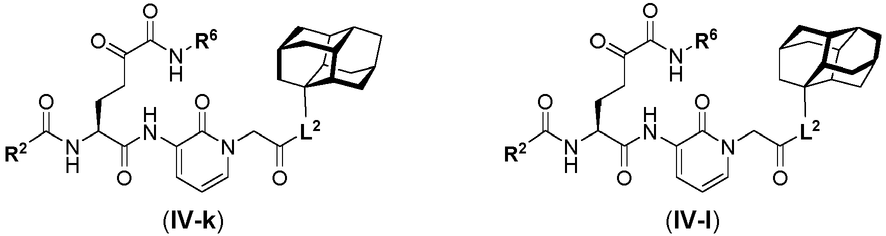 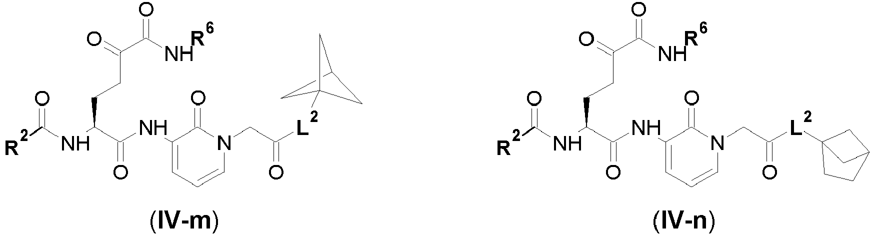 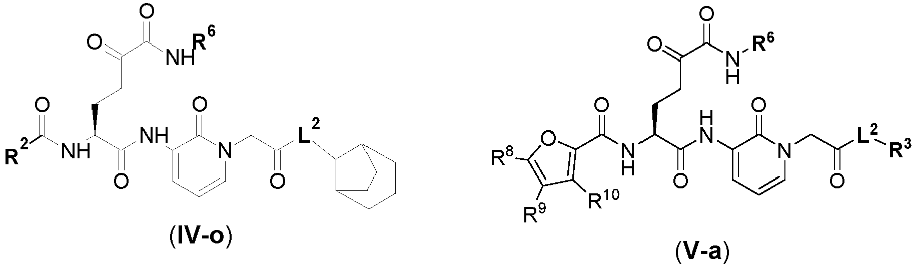 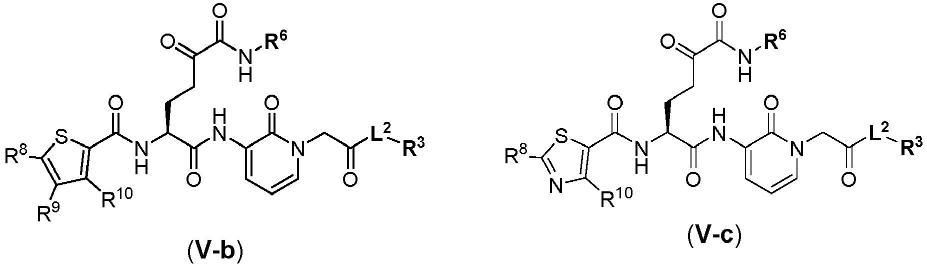 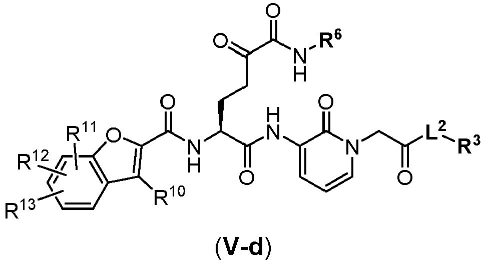 and **R²**, **R³**, **R⁶**, **R⁸**, **R⁹**, **R¹⁰**, **R¹¹**, **R¹²**, **R¹³**, **R^{a}**, **R^{b}**, **R^{c}**, **R^{d}** and **L²** have the same meanings as defined herein, preferably as defined in formula (**I**) or (**Ib**).

Also preferred are the compounds of any of the formula (**IVa-1**), 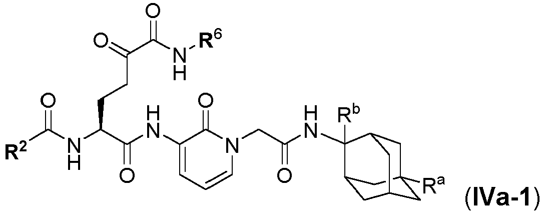 wherein
**R⁶** represents -H, -CH₃, -CH(CH₃)₂, -CH₂CH₂CH₃, -CH₂CH=CH₂, -CH₂CH₂CH₂CH₃, -CH₂CH(CH₃)₂, -C(CH₃)₃, -CH₂CH₂CH₂CH₂CH₃, -cyclo-C₃H₅, -cyclo-C₅H₉, -cyclo-C₆H₁₁ or -CH₂-cyclo-C₃H₅.
and **R²**, **R^{a},** and **R^{b}** have the same meanings as defined above.

Preferably, in the compounds of formula (**I**) or (**Ib**), **R^{a}** and **R^{b}** represent independently of each other -H, -F, -Cl, -Br, -OH, -CN, -CH₃, -C₂H₅, or -CO₂Me.

Preferably, in any of the formula (**I**), (**Ib**), (**IV-a**) - (**IV-o**), or (**IVa-1**):
**R²** represents 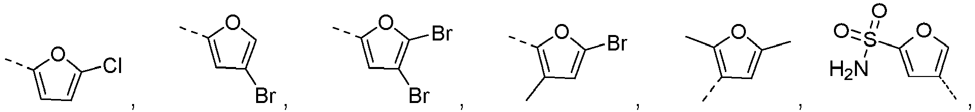 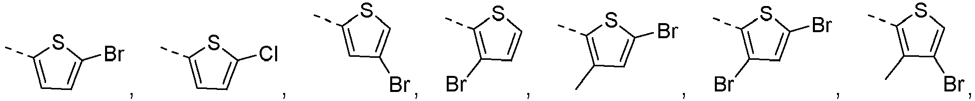 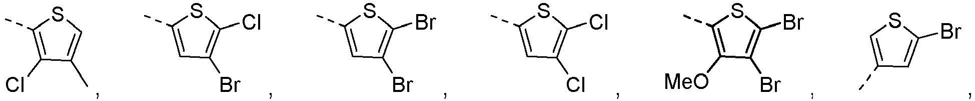 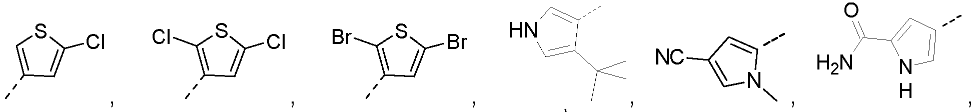 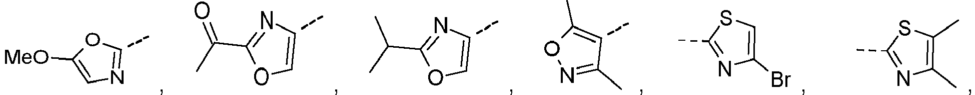 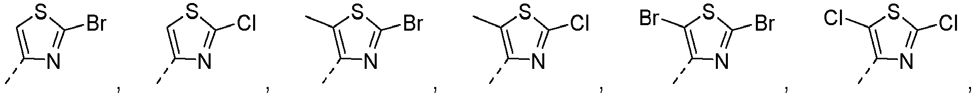 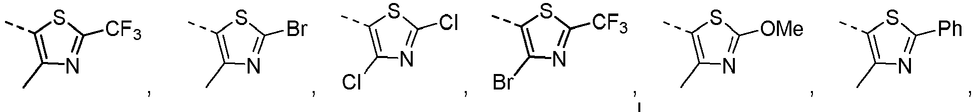 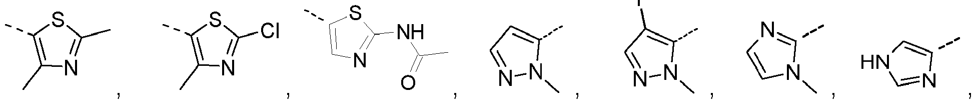 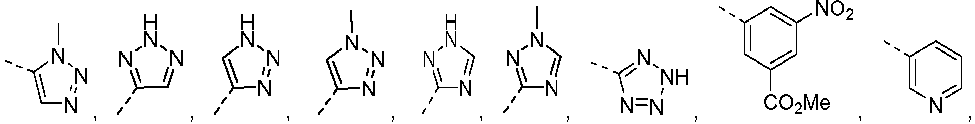 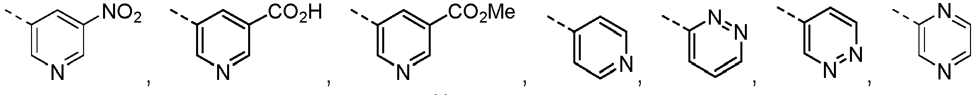 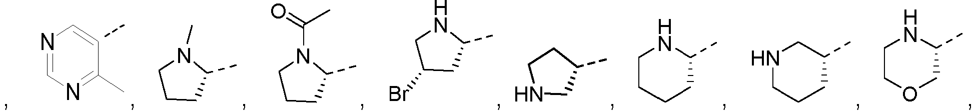 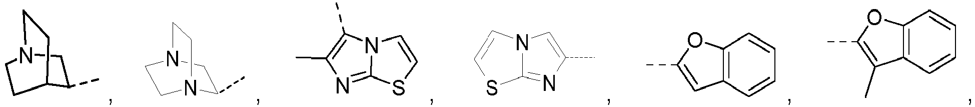 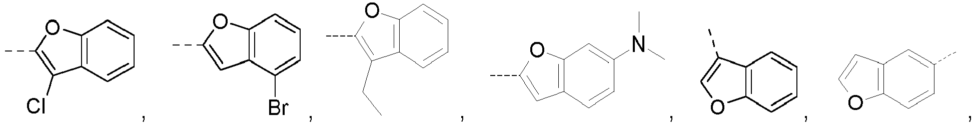 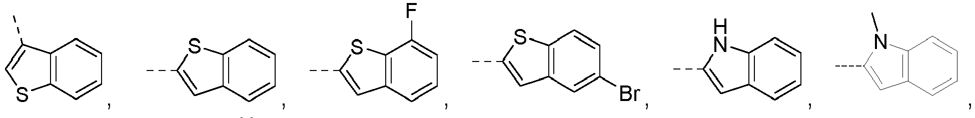 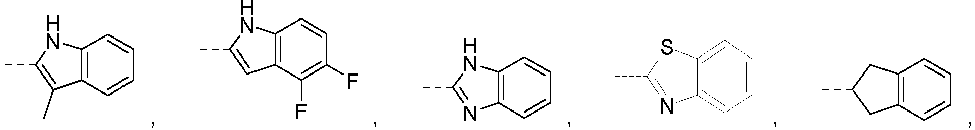 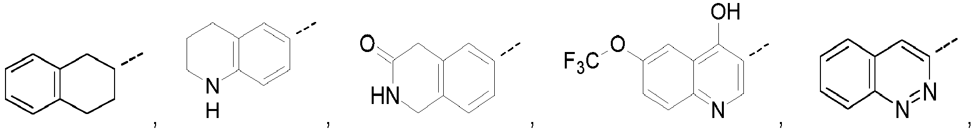 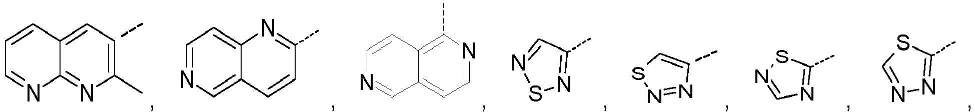 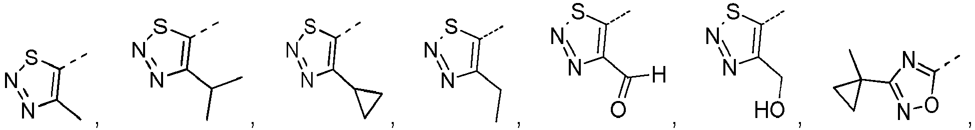 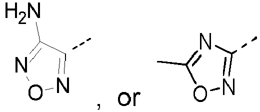 and
**R⁶** represents -H, -CH₃, -CH(CH₃)₂, -CH₂CH₂CH₃, -CH₂CH=CH₂, -CH₂CH₂CH₂CH₃, -CH₂CH(CH₃)₂, -C(CH₃)₃, -CH₂CH₂CH₂CH₂CH₃, -cyclo-C₃H₅, -cyclo-C₅H₉, -cyclo-C₆H₁₁ or -CH₂-cyclo-C₃H₅.

Due to the specially selected substituents **R²** on the N-terminal side and substituents **R³** on the C-terminal side and of the inventive compound according to the invention the steric dimension can be adjusted very precisely, so that a binding pocket of a desired target molecule may be addressed with highly matching measurements.

Preferred, are the compound of any of the formulae (**I**), (**Ib**), (**IV-a**) - (**IV-o**), and (**V-a**) - (**V-d**), wherein
**R³** represents

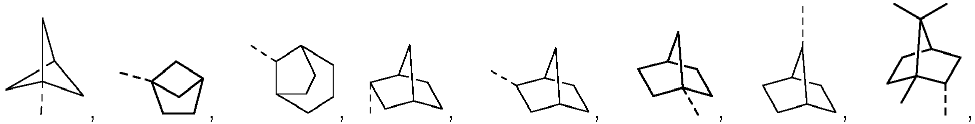

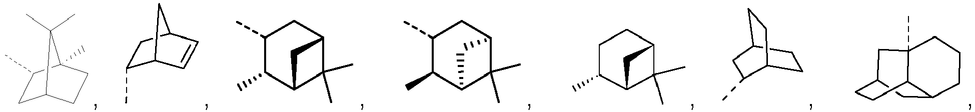

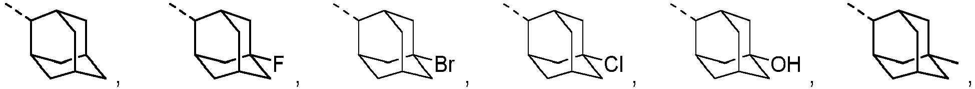

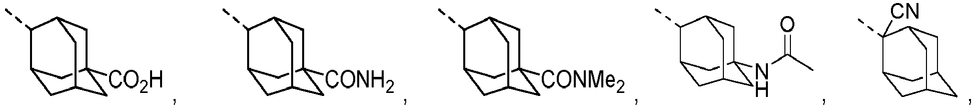

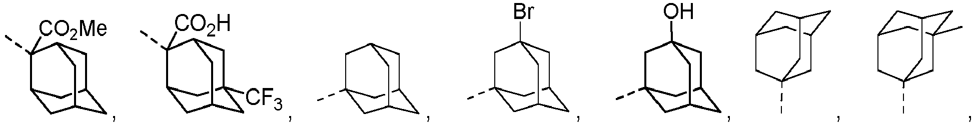

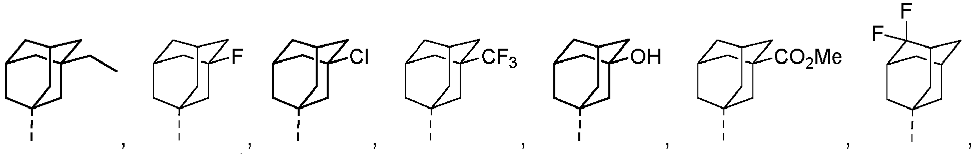

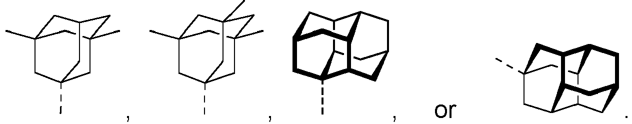

Surprisingly, it was found that the inventive compounds bound to the transglutaminase 2 reversibly and inhibit the transglutaminase effectively. The electrophilic warheads in combination with the preferred embodiment specifically react with highly nucleophilic thiols in the active site of the transglutaminase 2. Accordingly, it was found that potential unspecific reactions with off-targets are reduced.

In one embodiment, the present invention refers to the compound selected from the group consisting of:

| | | |
|---|---|---|
| **II-2:** | 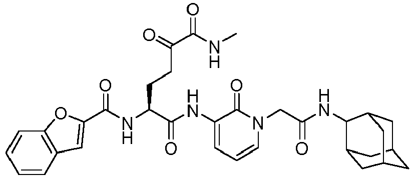 | (S)-2-(benzofuran-2-carboxamido)-N1-(1-(2-(2-adamantylamino)-2-oxoethyl)-2-oxo-1,2-dihydropyridin-3-yl)-N6-methyl-5-oxohexanediamide |
| **II-3:** | 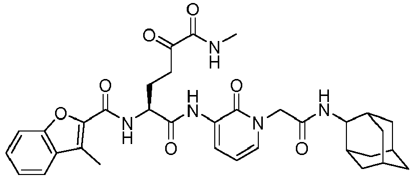 | (S)-N1-(1-(2-(2-adamantylamino)-2-oxoethyl)-2-oxo-1,2-dihydropyridin-3-yl)-N6-methyl-2-(3-methylbenzofuran-2-carboxamido)-5-oxohexanediamide |
| **II-4:** | 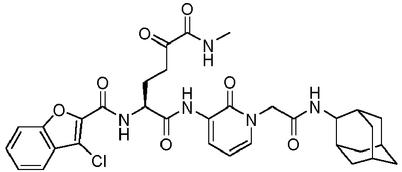 | (S)-2-(3-chlorobenzofuran-2-carboxamido)-N1-(1-(2-(2-adamantylamino)-2-oxoethyl)-2-oxo-1,2-dihydropyridin-3-yl)-N6-methyl-5-oxohexanediamide |
| **II-5:** | 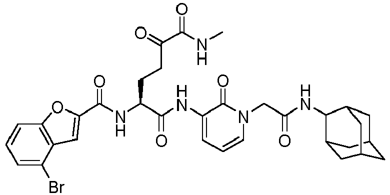 | (S)-2-(4-bromobenzofuran-2-carboxamido)-N1-(1-(2-(2-adamantylamino)-2-oxoethyl)-2-oxo-1,2-dihydropyridin-3-yl)-N6-methyl-5-oxohexanediamide |
| **II-6**: | 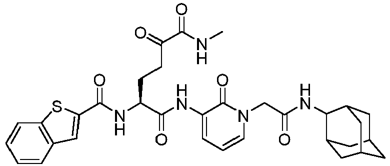 | (S)-2-(4-bromobenzofuran-2-carboxam ido)-N1-(1-(2-(2-adamantylamino)-2-oxoethyl)-2-oxo-1,2-dihydropyridin-3-yl)-N6-methyl-5-oxohexanediamide |
| **II-7:** | 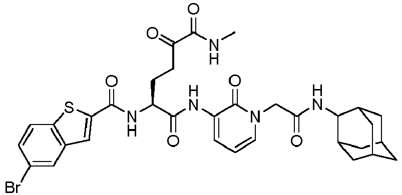 | (S)-2-(benzo[b]thiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-8**: | 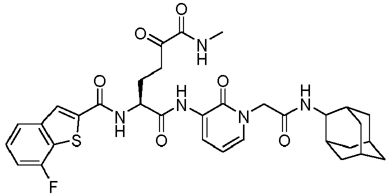 | (S)-2-(5-bromobenzo[b]thiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-9:** | 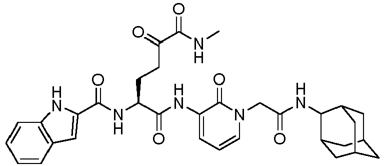 | (S)-2-(1H-indole-2-carboxamido)-N1-(1-(2-(2-adamantylamino)-2-oxoethyl)-2-oxo-1,2-dihydropyridin-3-yl)-N6-methyl-5-oxohexanediamide |
| **II-10:** | 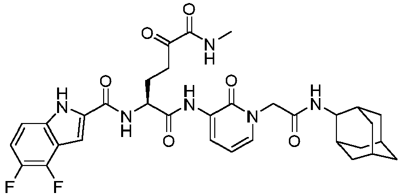 | (S)-2-(4,5-difluoro-1H-indole-2-carboxam ido)-N1-(1-(2-(2-adamantylamino)-2-oxoethyl)-2-oxo-1,2-dihydropyridin-3-yl)-N6-methyl-5-oxohexanediamide |
| **II-11**: | 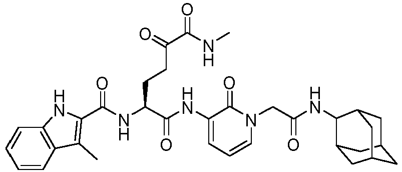 | (S)-N1-(1-(2-(2-adamantylamino)-2-oxoethyl)-2-oxo-1,2-dihydropyridin-3-yl)-N6-methyl-2-(3-methyl-1H-indole-2-carboxamido)-5-oxohexanediamide |
| **II-12**: | 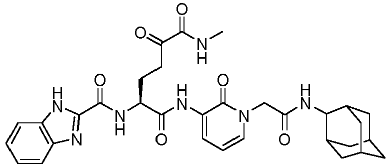 | (S)-2-(1H-benzo[d]imidazole-2-carboxamido)-N1-(1-(2-(2-adamantylamino)-2-oxoethyl)-2-oxo-1,2-dihydropyridin-3-yl)-N6-methyl-5-oxohexanediamide |
| **II-13**: | 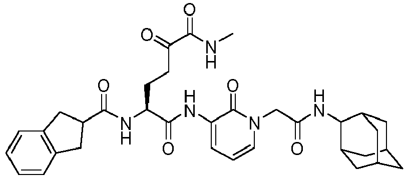 | (S)-2-(2,3-dihydro-1H-indene-2-carboxam ido)-N 1-(1-(2-(2-adamantylamino)-2-oxoethyl)-2-oxo-1,2-dihydropyridin-3-yl)-N6-methyl-5-oxohexanediamide |
| **II-14:** | 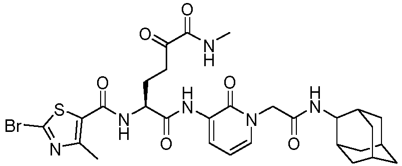 | (S)--(2-bromo-4-methylthiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-15**: | 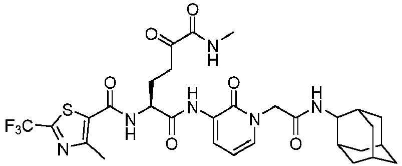 | (S)-N 1-(1-(2-(2-adamantylam i no)-2-oxoethyl)-2-oxo-1,2-dihydropyridin-3-yl)-N6-methyl-2-(4-methyl-2-(trifluoromethyl)thiazole-5-carboxamido)-5-oxohexanediamide |
| **II-16**: | 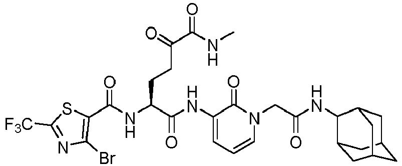 | (S)-2-(4-bromo-2-(trifluoromethyl)thiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-17**: | 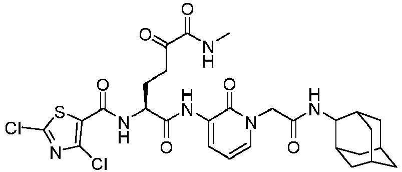 | (S)-2-(2,4-dichlorothiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-18**: | 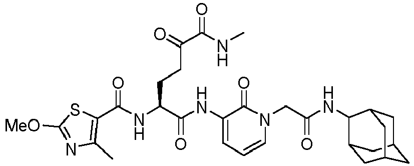 | (S)-N 1-(1-(2-(2-adamantylam i no)-2-oxoethyl)-2-oxo-1,2-dihydropyridin-3-yl)-2-(2-methoxy-4-methylthiazole-5-carboxamido)-N6-methyl-5-oxohexanediamide |
| **II-19**: | 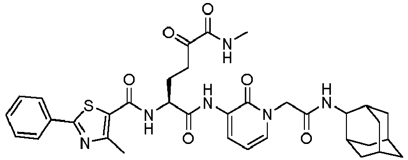 | (S)-N 1-(1-(2-(2-adamantylam i no)-2-oxoethyl)-2-oxo-1,2-dihydropyridin-3-yl)-N6-methyl-2-(4-methyl-2-phenylthiazole-5-carboxamido)-5-oxohexanediamide |
| **II-20:** | 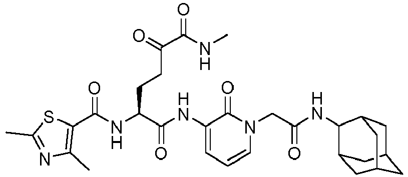 | (S)-2-(2,4-dimethylthiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-21**: | 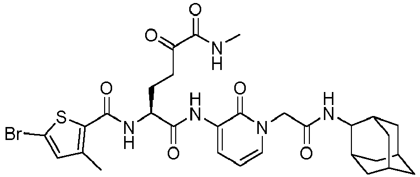 | (S)-2-(5-bromo-3-methylthiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-22:** | 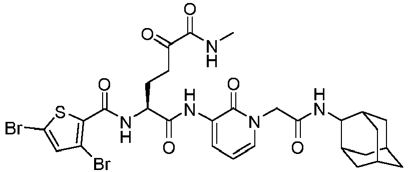 | (S)-2-(3,5-dibromothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-23:** | 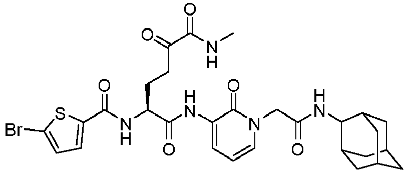 | (S)-2-(5-bromothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-24:** | 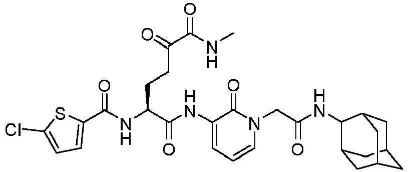 | (S)-2-(5-chlorothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-25:** | 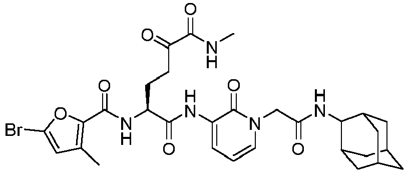 | (S)-2-(5-bromo-3-methylfuran-2-carboxam ido)-N1-(1-(2-(2-adamantylamino)-2-oxoethyl)-2-oxo-1,2-dihydropyridin-3-yl)-N6-methyl-5-oxohexanediamide |
| **II-26:** | 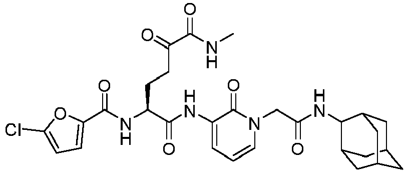 | (S)-2-(5-chlorofuran-2-carboxamido)-N1-(1-(2-(2-adamantylamino)-2-oxoethyl)-2-oxo-1,2-dihydropyridin-3-yl)-N6-methyl-5-oxohexanediamide |
| **II-27:** | 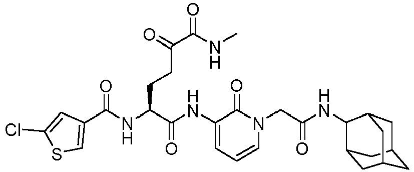 | (S)-2-(5-chlorothiophene-3-carboxamido)-N1-(1-(2-(2-adamantylamino)-2-oxoethyl)-2-oxo-1,2-dihydropyridin-3-yl)-N6-methyl-5-oxohexanediamide |
| **II-28**: | 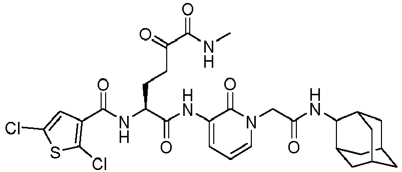 | (S)-2-(2,5-dichlorothiophene-3-carboxamido)-N1-(1-(2-(2-adamantylamino)-2-oxoethyl)-2-oxo-1,2-dihydropyridin-3-yl)-N6-methyl-5-oxohexanediamide |
| **II-29:** | 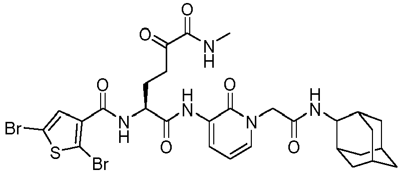 | (S)-2-(2,5-dibromothiophene-3-carboxamido)-N1-(1-(2-(2-adamantylamino)-2-oxoethyl)-2-oxo-1,2-dihydropyridin-3-yl)-N6-methyl-5-oxohexanediamide |
| **II-30**: | 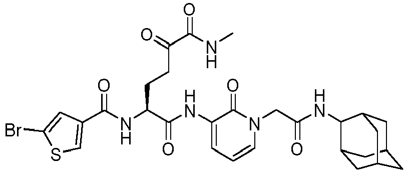 | (S)-2-(5-bromothiophene-3-carboxamido)-N1-(1-(2-(2-adamantylam i no)-2-oxoethyl)-2-oxo-1,2-dihydropyridin-3-yl)-N6-methyl-5-oxohexanediamide |
| **II-31**: | 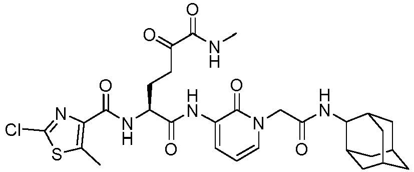 | (S)-2-(2-chloro-5-methylthiazole-4-carboxamido)-N1-(1-(2-(2-adamantylamino)-2-oxoethyl)-2-oxo-1,2-dihydropyridin-3-yl)-N6-methyl-5-oxohexanediamide |
| **II-32:** | 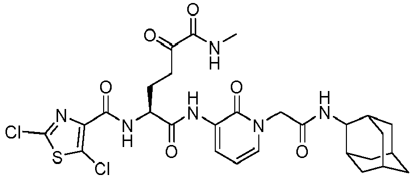 | (S)-2-(2,5-dichlorothiazole-4-carboxamido)-N1-(1-(2-(2-adamantylamino)-2-oxoethyl)-2-oxo-1,2-dihydropyridin-3-yl)-N6-methyl-5-oxohexanediamide |
| **II-33:** | 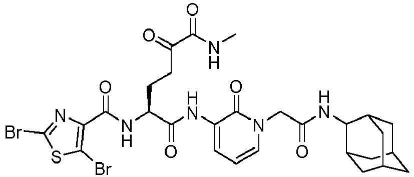 | (S)-2-(2,5-dibromothiazole-4-carboxamido)-N1-(1-(2-(2-adamantylamino)-2-oxoethyl)-2-oxo-1,2-dihydropyridin-3-yl)-N6-methyl-5-oxohexanediamide |
| **II-34:** | 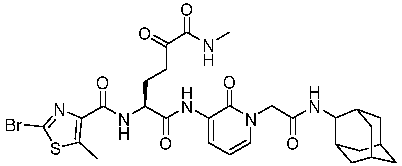 | (S)-2-(2-bromo-5-methylthiazole-4-carboxamido)-N1-(1-(2-(2-adamantylamino)-2-oxoethyl)-2-oxo-1,2-dihydropyridin-3-yl)-N6-methyl-5-oxohexanediamide |
| **II-35:** | 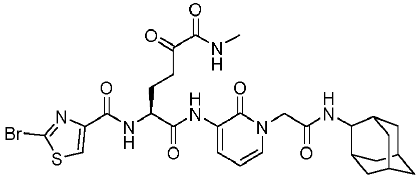 | (S)-2-(2-bromothiazole-4-carboxamido)-N1-(1-(2-(2-adamantylamino)-2-oxoethyl)-2-oxo-1,2-dihydropyridin-3-yl)-N6-methyl-5-oxohexanediamide |
| **II-36:** | 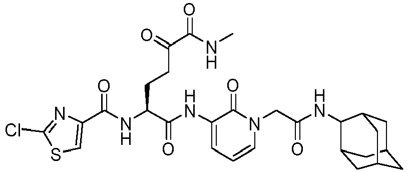 | (S)-2-(2-chlorothiazole-4-carboxamido)-N1-(1-(2-(2-adamantylamino)-2-oxoethyl)-2-oxo-1,2-dihydropyridin-3-yl)-N6-methyl-5-oxohexanediamide |
| **II-37:** | 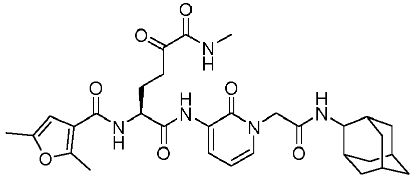 | (S)-2-(2,5-dimethylfuran-3-carboxamido)-N1-(1-(2-(2-adamantylami no)-2-oxoethyl)-2-oxo-1,2-dihydropyridin-3-yl)-N6-methyl-5-oxohexanediamide |
| **II-38:** | 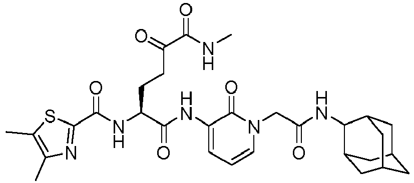 | (S)-2-(4,5-dimethylthiazole-2-carboxamido)-N1-(1-(2-(2-adamantylamino)-2-oxoethyl)-2-oxo-1,2-dihydropyridin-3-yl)-N6-methyl-5-oxohexanediamide |
| **II-39:** | 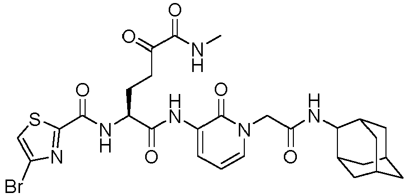 | (S)-2-(4-bromothiazole-2-carboxamido)-N1-(1-(2-(2-adamantylamino)-2-oxoethyl)-2-oxo-1,2-dihydropyridin-3-yl)-N6-methyl-5-oxohexanediamide |
| **II-40:** | 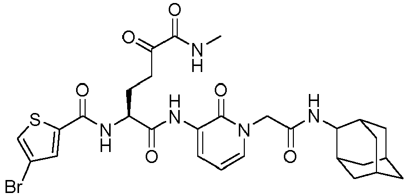 | (S)-2-(4-bromothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-41**: | 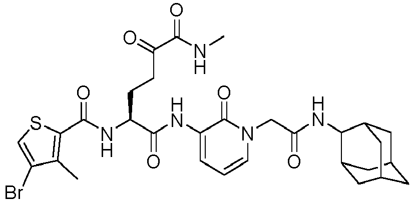 | (S)-2-(4-bromo-3-methylthiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-42:** | 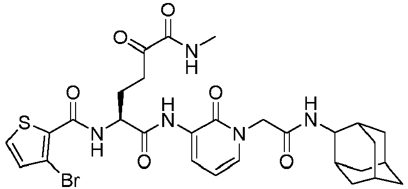 | (S)-2-(3-bromothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-43:** | 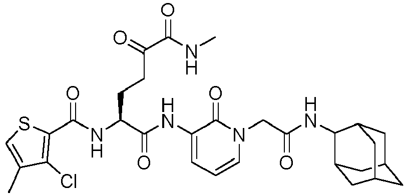 | (S)-2-(3-chloro-4-methylthiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-44:** | 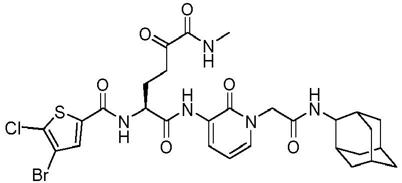 | (S)-2-(4-bromo-5-chlorothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-45:** | 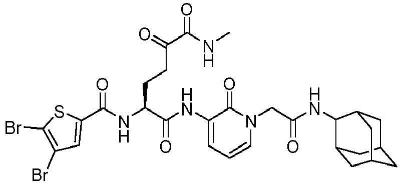 | (S)-2-(4,5-dibromothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-46:** | 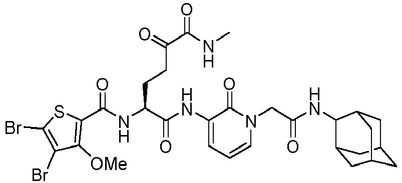 | (S)-2-(4,5-dibromo-3-methoxythiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-47:** | 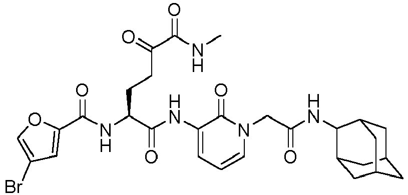 | (S)-2-(4-bromofuran-2-carboxam ido)-N1-(1-(2-(2-adamantylamino)-2-oxoethyl)-2-oxo-1,2-dihydropyridin-3-yl)-N6-methyl-5-oxohexanediamide |
| **II-48:** | 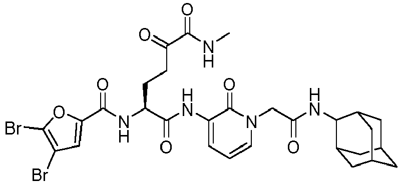 | (S)-2-(4,5-dibromofuran-2-carboxamido)-N1-(1-(2-(2-adamantylami no)-2-oxoethyl)-2-oxo-1,2-dihydropyridin-3-yl)-N6-methyl-5-oxohexanediamide |
| **II-49:** | 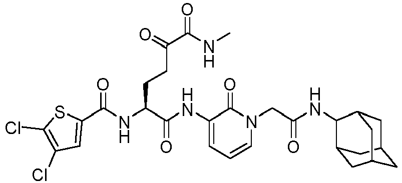 | (S)-2-(4,5-dichlorothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-50:** | 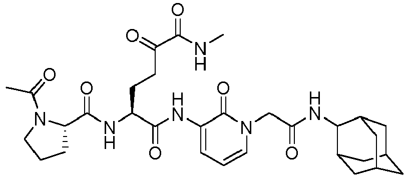 | (S)-2-((S)-1-acetylpyrrolidine-2-carboxamido)-N1-(1-(2-(2-adamantylamino)-2-oxoethyl)-2-oxo-1,2-dihydropyridin-3-yl)-N6-methyl-5-oxohexanediamide |
| **II-51:** | 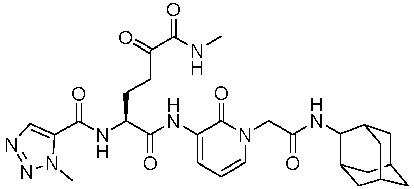 | (S)-N1-(1-(2-(2-adamantylamino)-2-oxoethyl)-2-oxo-1,2-dihydropyridin-3-yl)-N6-methyl-2-(1-methyl-1H-1,2,3-triazole-5-carboxamido)-5-oxohexanediamide |
| **II-52:** | 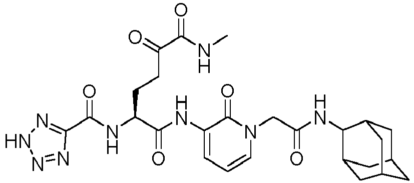 | (S)-N1-(1-(2-(2-adamantylamino)-2-oxoethyl)-2-oxo-1,2-dihydropyridin-3-yl)-N6-methyl-5-oxo-2-(2H-tetrazole-5-carboxamido)hexanediamide |
| **II-53**: | 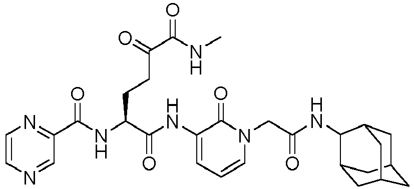 | (S)-N1-(1-(2-(2-adamantylamino)-2-oxoethyl)-2-oxo-1,2-dihydropyridin-3-yl)-N6-methyl-5-oxo-2-(pyrazine-2-carboxamido)hexanediamide |
| **II-54:** | 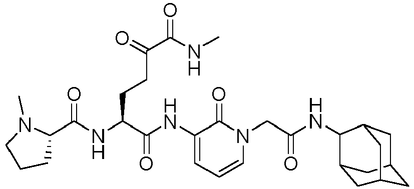 | (S)-N1-(1-(2-(2-adamantylamino)-2-oxoethyl)-2-oxo-1,2-dihydropyridin-3-yl)-N6-methyl-2-((S)-1-methylpyrrolidine-2-carboxamido)-5-oxohexanediamide |
| **II-55**: | 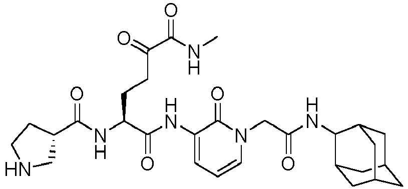 | (S)-N1-(1-(2-(2-adamantylamino)-2-oxoethyl)-2-oxo-1,2-dihydropyridin-3-yl)-N6-methyl-5-oxo-2-((S)-pyrrolidine-3-carboxamido)hexanediamide |
| **II-56**: | 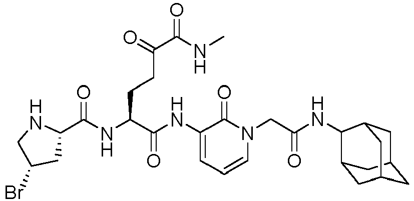 | (S)-2-((2S,4S)-4-bromopyrrolidine-2-carboxamido)-N1-(1-(2-(2-adamantylamino)-2-oxoethyl)-2-oxo-1,2-dihydropyridin-3-yl)-N6-methyl-5-oxohexanediamide |
| **II-58**: | 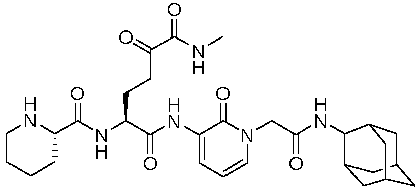 | (S)-N1-(1-(2-(2-adamantylamino)-2-oxoethyl)-2-oxo-1,2-dihydropyridin-3-yl)-N6-methyl-5-oxo-2-((S)-piperidine-2-carboxamido)hexanediamide |
| **II-59:** | 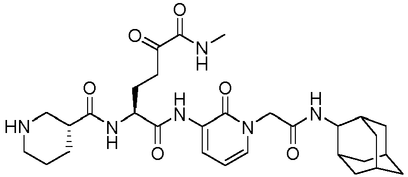 | (S)-N1-(1-(2-(2-adamantylamino)-2-oxoethyl)-2-oxo-1,2-dihydropyridin-3-yl)-N6-methyl-5-oxo-2-((R)-piperidine-3-carboxamido)hexanediamide |
| **II-60**: | 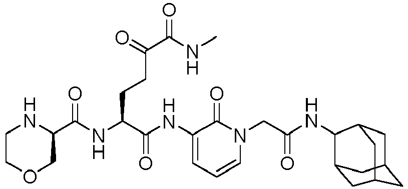 | (S)-N1-(1-(2-(2-adamantylamino)-2-oxoethyl)-2-oxo-1,2-dihydropyridin-3-yl)-N6-methyl-2-((R)-morpholine-3-carboxamido)-5-oxohexanediamide |
| **II-61**: | 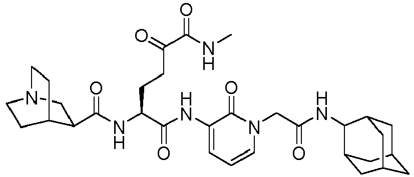 | (S)-N1-(1-(2-(2-adamantylamino)-2-oxoethyl)-2-oxo-1,2-dihydropyridin-3-yl)-N6-methyl-5-oxo-2-(quinuclidine-3-carboxamido)hexanediamide |
| **II-62**: | 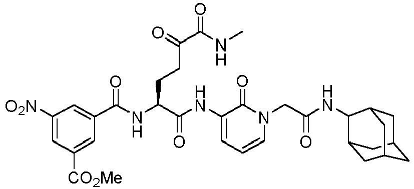 | (S)-methyl 3-(1-(1-(2-(2-adamantylamino)-2-oxoethyl)-2-oxo-1,2-dihydropyridin-3-ylamino)-6-(methylamino)-1,5,6-trioxohexan-2-ylcarbamoyl)-5-nitrobenzoate |
| **II-63**: | 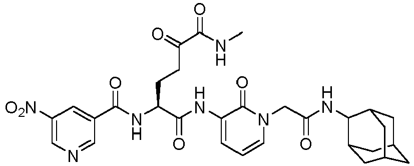 | (S)-N1-(1-(2-(2-adamantylamino)-2-oxoethyl)-2-oxo-1,2-dihydropyridin-3-yl)-N6-methyl-2-(5-nitronicotinamido)-5-oxohexanediamide |
| **II-64:** | 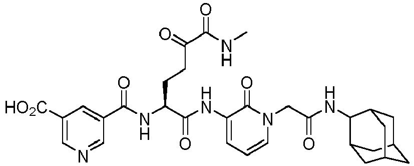 | (S)-5-(1-(1-(2-(2-adamantylamino)-2-oxoethyl)-2-oxo-1,2-dihydropyridin-3-ylamino)-6-(methylamino)-1,5,6-trioxohexan-2-ylcarbamoyl)nicotinic acid |
| **II-65**: | 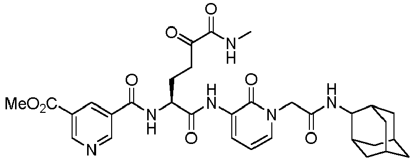 | (S)-methyl 5-(1-(1-(2-(2-adamantylamino)-2-oxoethyl)-2-oxo-1,2-dihydropyridin-3-ylamino)-6-(methylamino)-1,5,6-trioxohexan-2-ylcarbamoyl)nicotinate |
| **II-66**: | 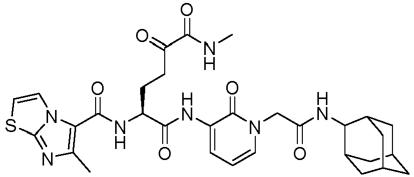 | (S)-N1-(1-(2-(2-adamantylamino)-2-oxoethyl)-2-oxo-1,2-dihydropyridin-3-yl)-N6-methyl-2-(6-methylimidazo[2,1-b]thiazole-5-carboxamido)-5-oxohexanediamide |
| **II-67:** | 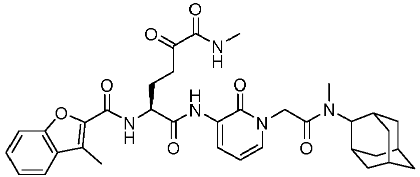 | (S)-N 1-(1-(2-(2-adamantyl(methyl)amino)-2-oxoethyl)-2-oxo-1,2-dihydropyridin-3-yl)-N6-methyl-2-(3-methylbenzofuran-2-carboxamido)-5-oxohexanediamide |
| **II-68**: | 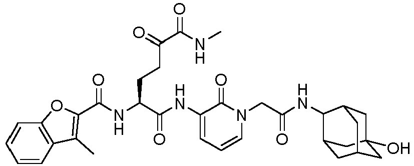 | (S)-N1-(1-(2-(5-hydroxyadamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-69**: | 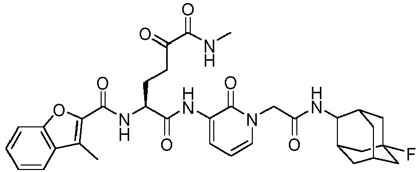 | (S)-N1-(1-(2-(5-fluoroadamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-70**: | 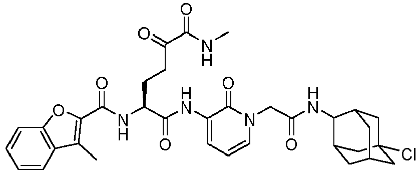 | (S)-N1-(1-(2-(5-chloroadamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-71**: | 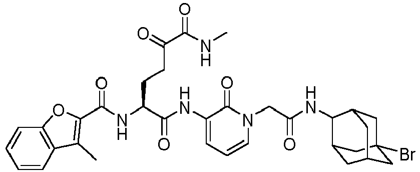 | (S)-N1-(1-(2-(5-bromoadamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-72**: | 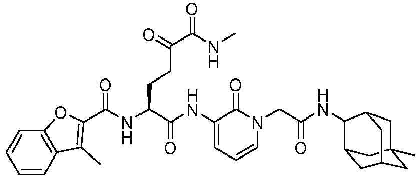 | (S)-N1-(1-(2-(5-methyladamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-73:** | 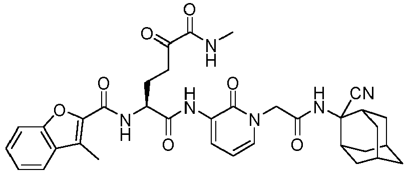 | (S)-N1-(1-(2-(2-carbonitrileadamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-74:** | 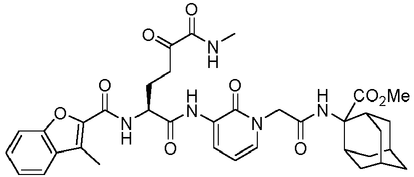 | (S)-N1-(1-(2-(2-methyl adamantane-2-carboxylate-2-amino)-2-oxoethyl)-2-oxo-1,2-dihydropyridin-3-yl)-N6-methyl-2-(3-methylbenzofuran-2-carboxamido)-5-oxohexanediamide |
| **II-87:** | 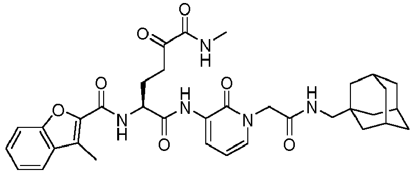 | (S)-N1-(1-(2-(1-adamantylmethylamino)-2-oxoethyl)-2-oxo-1,2-dihydropyridin-3-yl)-N6-methyl-2-(3-methylbenzofuran-2-carboxamido)-5-oxohexanediamide |
| **II-88:** | 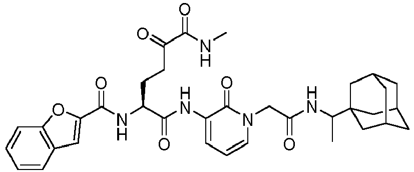 | (S)-N 1-(1-(2-(1-(1-adamantyl)ethanamino)-2-oxoethyl)-2-oxo-1,2-dihydropyridin-3-yl)-N6-methyl-2-(3-methylbenzofuran-2-carboxamido)-5-oxohexanediamide |
| **II-90:** | 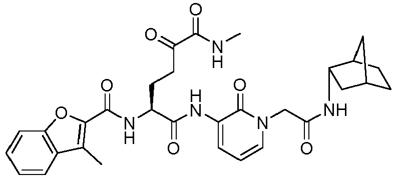 | (S)-N1-(1-(2-((1R,2S,4S)-bicyclo[2.2.1]heptan-2-ylamino)-2-oxoethyl)-2-oxo-1,2-dihydropyridin-3-yl)-N6-methyl-2-(3-methylbenzofuran-2-carboxamido)-5-oxohexanediamide |
| **II-92**: | 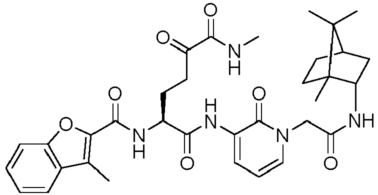 | (S)-N1-methyl-5-(3-methylbenzofuran-2-carboxamido)-2-oxo-N6-(2-oxo-1-(2-oxo-2-((1S,2S,4R)-1,7,7-trimethylbicyclo[2.2.1]heptan-2-ylamino)ethyl)-1,2-dihydropyridin-3-yl)hexanediamide |
| **II-94:** | 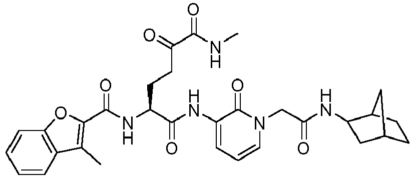 | (S)-N1-(1-(2-((1R,2R,4S)-bicyclo[2.2.1]heptan-2-ylamino)-2-oxoethyl)-2-oxo-1,2-dihydropyridin-3-yl)-N6-methyl-2-(3-methylbenzofuran-2-carboxamido)-5-oxohexanediamide |
| **II-95**: | 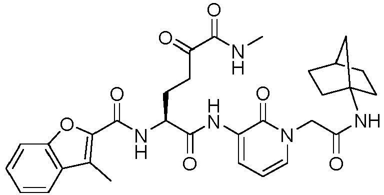 | (S)-N1-(1-(2-(bicyclo[2.2.1]heptan-1-ylamino)-2-oxoethyl)-2-oxo-1,2-dihydropyridin-3-yl)-N6-methyl-2-(3-methylbenzofuran-2-carboxamido)-5-oxohexanediamide |
| **II-96**: | 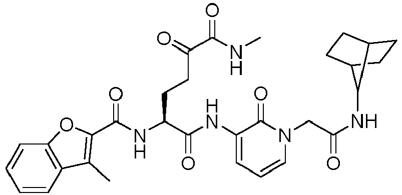 | (S)-N1-(1-(2-(bicyclo[2.2.1]heptan-7-ylamino)-2-oxoethyl)-2-oxo-1,2-dihydropyridin-3-yl)-N6-methyl-2-(3-methylbenzofuran-2-carboxamido)-5-oxohexanediamide |
| **II-97:** | 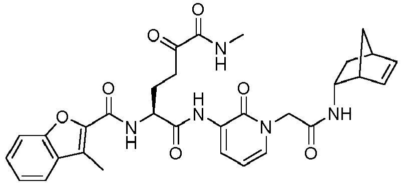 | (S)-N1-(1-(2-(bicyclo[2.2.1]hept-5-en-2-ylamino)-2-oxoethyl)-2-oxo-1,2-dihydropyridin-3-yl)-N6-methyl-2-(3-methylbenzofuran-2-carboxamido)-5-oxohexanediamide |
| **II-98**: | 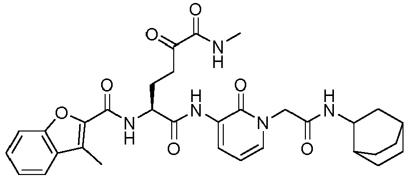 | (2S)-N1-(1-(2-(bicyclo[2.2.2]octan-2-ylamino)-2-oxoethyl)-2-oxo-1,2-dihydropyridin-3-yl)-N6-methyl-2-(3-methylbenzofuran-2-carboxamido)-5-oxohexanediamide |
| **II-99**: | 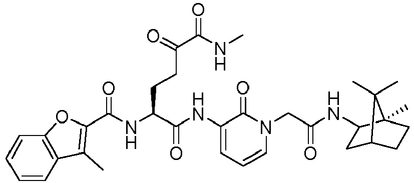 | (S)-N 1-methyl-5-(3-methylbenzofuran-2-carboxamido)-2-oxo-N6-(2-oxo-1-(2-oxo-2-((1R,2R,4R)-1,7,7-trimethylbicyclo[2.2.1]heptan-2-ylamino)ethyl)-1,2-dihydropyridin-3-yl)hexanediamide |
| **II-100:** | 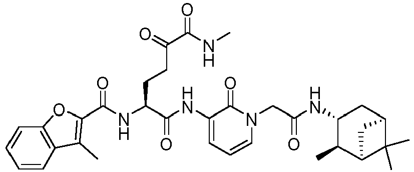 | (S)-N1-methyl-5-(3-methylbenzofuran-2-carboxamido)-2-oxo-N6-(2-oxo-1-(2-oxo-2-((1R,2R,3R,5S)-2,6,6-trimethylbicyclo[3.1.1]heptan-3-ylamino)ethyl)-1,2-dihydropyridin-3-yl)hexanediamide |
| **II-101**: | 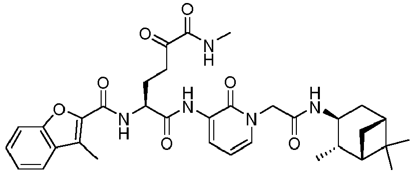 | (S)-N1-methyl-5-(3-methylbenzofuran-2-carboxamido)-2-oxo-N6-(2-oxo-1-(2-oxo-2-((1S,2S,3S,5R)-2,6,6-trimethylbicyclo[3.1.1]heptan-3-ylamino)ethyl)-1,2-dihydropyridin-3-yl)hexanediamide |
| **II-103:** | 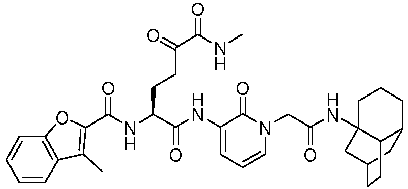 | (S)-N1-(1-(2-(4-homoisotwistane-3-amino)-2-oxoethyl)-2-oxo-1,2-dihydropyridin-3-yl)-N6-methyl-2-(3-methylbenzofuran-2-carboxamido)-5-oxohexanediamide |
| **II-104:** | 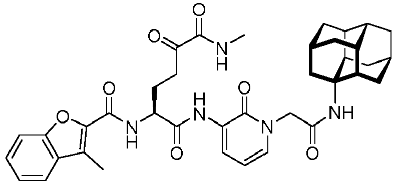 | (S)-N1-(1-(2-(diamantane-1-amino)-2-oxoethyl)-2-oxo-1,2-dihydropyridin-3-yl)-N6-methyl-2-(3-methylbenzofuran-2-carboxamido)-5-oxohexanediamide |
| **II-105:** | 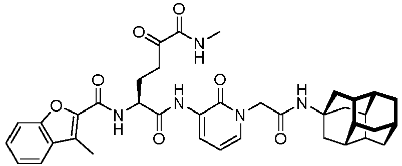 | (S)-N1-(1-(2-(diamantane-4-amino)-2-oxoethyl)-2-oxo-1,2-dihydropyridin-3-yl)-N6-methyl-2-(3-methylbenzofuran-2-carboxamido)-5-oxohexanediamide |
| **II-107:** | 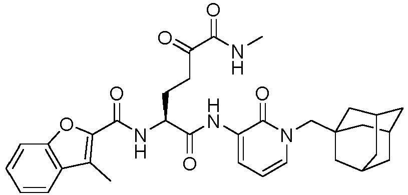 | (S)-N1-(1-(1-adamantylmethyl)-2-oxo-1,2-dihydropyridin-3-yl)-N6-methyl-2-(3-methylbenzofuran-2-carboxamido)-5-oxohexanediamide |
| **II-108:** | 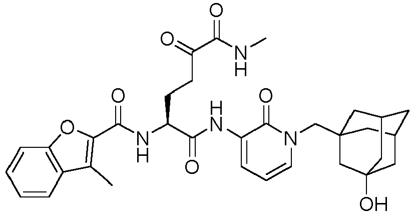 | (2S)-N1-(1-((3-hydroxy-1-adamantyl)methyl)-2-oxo-1,2-dihydropyridin-3-yl)-N6-methyl-2-(3-methylbenzofuran-2-carboxamido)-5-oxohexanediamide |
| **II-109:** | 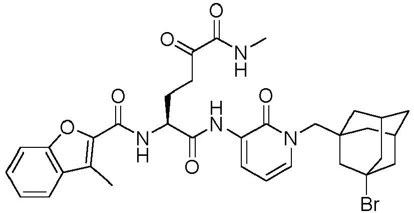 | (2S)-N1-(1-((3-bromo-1-adamantyl)methyl)-2-oxo-1,2-dihydropyridin-3-yl)-N6-methyl-2-(3-methylbenzofuran-2-carboxamido)-5-oxohexanediamide |
| **II-110:** | 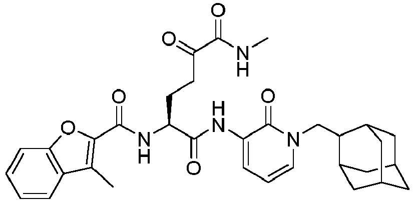 | (S)-N1-(1-(2-adamantylmethyl)-2-oxo-1,2-dihydropyridin-3-yl)-N6-methyl-2-(3-methylbenzofuran-2-carboxamido)-5-oxohexanediamide |
| **II-111**: | 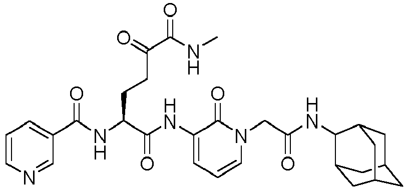 | (S)-N1-(1-(2-(2-adamantylamino)-2-oxoethyl)-2-oxo-1,2-dihydropyridin-3-yl)-N6-methyl-2-(nicotinamido)-5-oxohexanediamide |
| **II-112**: | 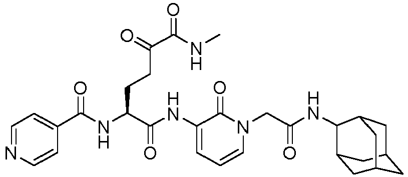 | (S)-2-(isonicotinamido)-N1-(1-(2-(2-adamantylamino)-2-oxoethyl)-2-oxo-1,2-dihydropyridin-3-yl)-N6-methyl-5-oxohexanediamide |
| **II-113**: | 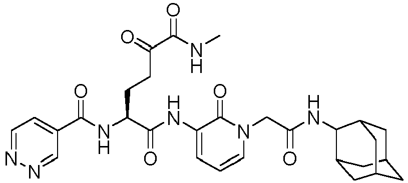 | (S)-N1-(1-(2-(2-adamantylam i no)-2-oxoethyl)-2-oxo-1,2-dihydropyridin-3-yl)-N6-methyl-5-oxo-2-(pyridazine-4-carboxamido)hexanediamide |
| **II-114:** | 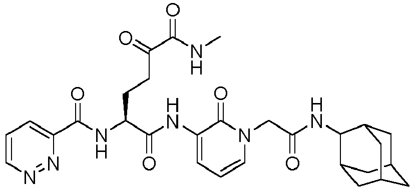 | (S)-N1-(1-(2-(2-adamantylamino)-2-oxoethyl)-2-oxo-1,2-dihydropyridin-3-yl)-N6-methyl-5-oxo-2-(pyridazine-3-carboxamido)hexanediamide |
| **II-115**: | 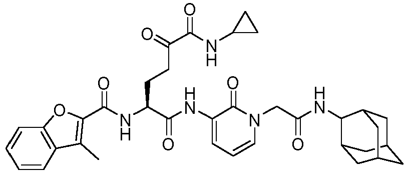 | (S)-N1-cyclopropyl-N6-(1-(2-(2-adamantylamino)-2-oxoethyl)-2-oxo-1,2-dihydropyridin-3-yl)-5-(3-methylbenzofuran-2-carboxamido)-2-oxohexanediamide |
| **II-116**: | 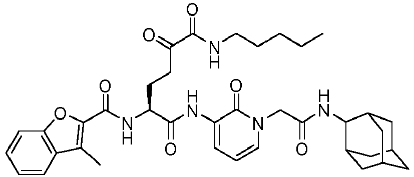 | (S)-N1-(1-(2-(2-adamantylamino)-2-oxoethyl)-2-oxo-1,2-dihydropyridin-3-yl)-2-(3-methylbenzofuran-2-carboxamido)-5-oxo-N6-pentylhexanediamide |
| **II-117**: | 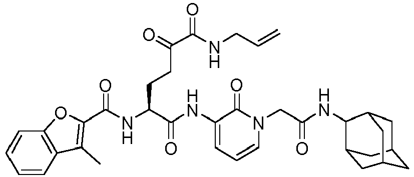 | (S)-N1-allyl-N6-(1-(2-(2-adamantylamino)-2-oxoethyl)-2-oxo-1,2-dihydropyridin-3-yl)-5-(3-methylbenzofuran-2-carboxamido)-2-oxohexanediamide |
| **II-118**: | 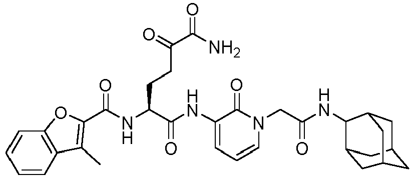 | (S)-N1-(1-(2-(2-adamantylamino)-2-oxoethyl)-2-oxo-1,2-dihydropyridin-3-yl)-2-(3-methylbenzofuran-2-carboxamido)-5-oxohexanediamide |
| **II-119**: | 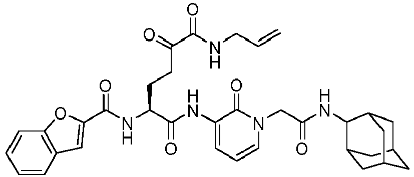 | (S)-N1-allyl-5-(benzofuran-2-carboxamido)-N6-(1-(2-(2-adamantylamino)-2-oxoethyl)-2-oxo-1,2-dihydropyridin-3-yl)-2-oxohexanediamide |
| **II-120**: | 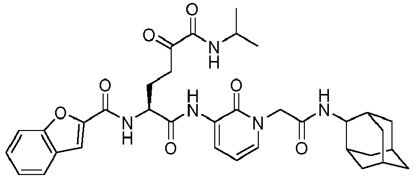 | (S)-2-(benzofuran-2-carboxamido)-N6-isopropyl-N1-(1-(2-(2-adamantylamino)-2-oxoethyl)-2-oxo-1,2-dihydropyridin-3-yl)-5-oxohexanediamide |
| **II-121**: | 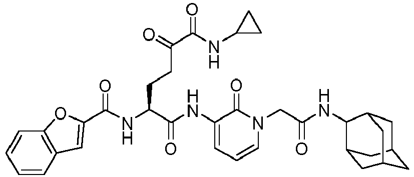 | (S)-2-(benzofuran-2-carboxamido)-N6-cyclopropyl-N1-(1-(2-(2-adamantylamino)-2-oxoethyl)-2-oxo-1,2-dihydropyridin-3-yl)-5-oxohexanediamide |
| **II-122**: | 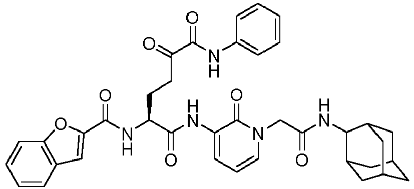 | (S)-2-(benzofuran-2-carboxamido)-N1-(1-(2-(2-adamantylamino)-2-oxoethyl)-2-oxo-1,2-dihydropyridin-3-yl)-5-oxo-N6-phenylhexanediamide |
| **II-123**: | 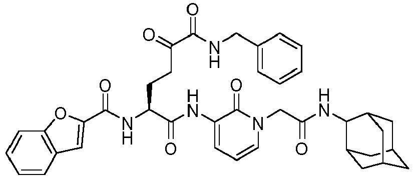 | (S)-2-(benzofuran-2-carboxamido)-N6-benzyl-N1-(1-(2-(2-adamantylamino)-2-oxoethyl)-2-oxo-1,2-dihydropyridin-3-yl)-5-oxohexanediamide |
| **II-124:** | 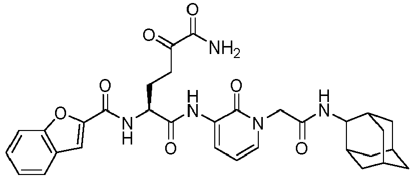 | (S)-2-(benzofuran-2-carboxamido)-N1-(1-(2-(2-adamantylamino)-2-oxoethyl)-2-oxo-1,2-dihydropyridin-3-yl)-5-oxohexanediamide |
| **II-125**: | 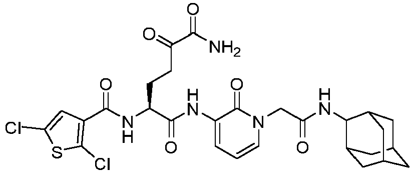 | (S)-2-(2,5-dichlorothiophene-3-carboxamido)-N1-(1-(2-(2-adamantylamino)-2-oxoethyl)-2-oxo-1,2-dihydropyridin-3-yl)-5-oxohexanediamide |
| **II-126**: | 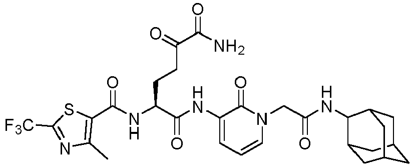 | (S)-N1-(1-(2-(2-adamantylamino)-2-oxoethyl)-2-oxo-1,2-dihydropyridin-3-yl)-2-(4-methyl-2-(trifluoromethyl)thiazole-5-carboxamido)-5-oxohexanediamide |
| **II-127**: | 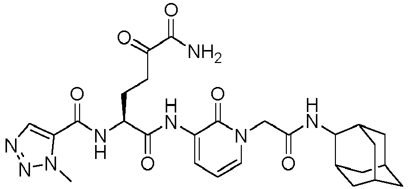 | (S)-N1-(1-(2-(2-adamantylamino)-2-oxoethyl)-2-oxo-1,2-dihydropyridin-3-yl)-2-(1-methyl-1H-1,2,3-triazole-5-carboxamido)-5-oxohexanediamide |
| **II-128**: | 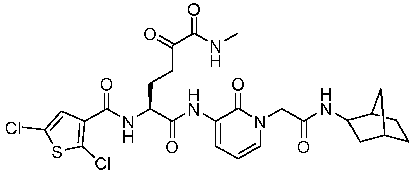 | (2S)-N1-(1-(2-((1R,2R,4S)-bicyclo[2.2.1]heptan-2-ylamino)-2-oxoethyl)-2-oxo-1,2-dihydropyridin-3-yl)-2-(2,5-dichlorothiophene-3-carboxamido)-N6-methyl-5-oxohexanediamide |
| **II-129**: | 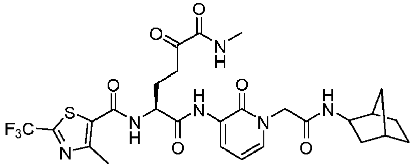 | (2S)-N1-(1-(2-((1R,2R,4S)-bicyclo[2.2.1]heptan-2-ylamino)-2-oxoethyl)-2-oxo-1,2-dihydropyridin-3-yl)-N6-methyl-2-(4-methyl-2-(trifluoromethyl)thiazole-5-carboxamido)-5-oxohexanediamide |
| **II-130**: | 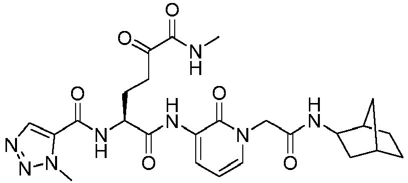 | (2S)-N1-(1-(2-((1R,2R,4S)-bicyclo[2.2.1]heptan-2-ylamino)-2-oxoethyl)-2-oxo-1,2-dihydropyridin-3-yl)-N6-methyl-2-(1-methyl-1H-1,2,3-triazole-5-carboxamido)-5-oxohexanediamide |
| **II-131**: | 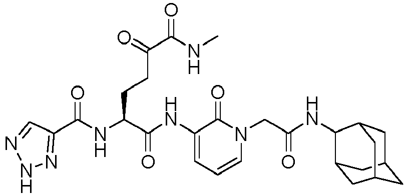 | (S)-N1-(1-(2-(2-adamantylamino)-2-oxoethyl)-2-oxo-1,2-dihydropyridin-3-yl)-N6-methyl-5-oxo-2-(2H-1,2,3-triazole-4-carboxamido)hexanediamide |
| **II-132**: | 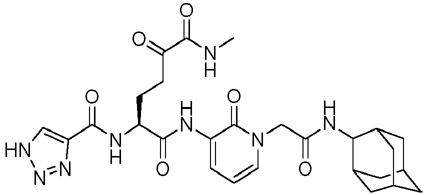 | (S)-N1-(1-(2-(2-adamantylamino)-2-oxoethyl)-2-oxo-1,2-dihydropyridin-3-yl)-N6-methyl-5-oxo-2-(1H-1,2,3-triazole-4-carboxamido)hexanediamide |
| **II-133**: | 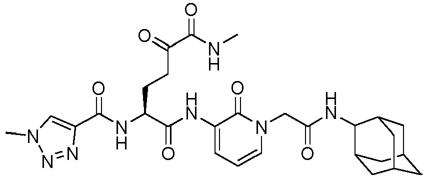 | (S)-N1-(1-(2-(2-adamantylamino)-2-oxoethyl)-2-oxo-1,2-dihydropyridin-3-yl)-N6-methyl-2-(1-methyl-1H-1,2,3-triazole-4-carboxamido)-5-oxohexanediamide |
| **II-134**: | 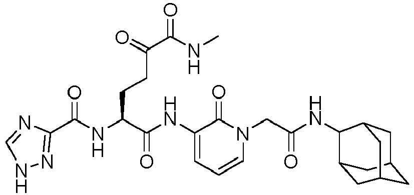 | (S)-N1-(1-(2-(2-adamantylamino)-2-oxoethyl)-2-oxo-1,2-dihydropyridin-3-yl)-N6-methyl-5-oxo-2-(1H-1,2,4-triazole-3-carboxamido)hexanediamide |
| **II-135**: | 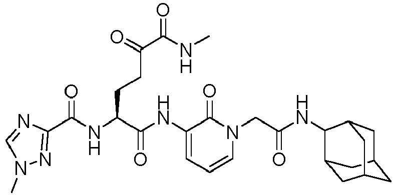 | (S)-N1-(1-(2-(2-adamantylamino)-2-oxoethyl)-2-oxo-1,2-dihydropyridin-3-yl)-N6-methyl-2-(1-methyl-1H-1,2,4-triazole-3-carboxamido)-5-oxohexanediamide |
| **II-136:** | 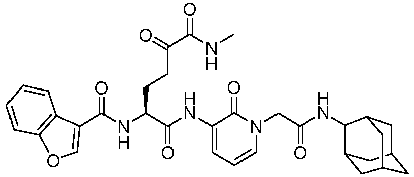 | (S)-2-(benzofuran-3-carboxamido)-N1-(1-(2-(2-adamantylamino)-2-oxoethyl)-2-oxo-1,2-dihydropyridin-3-yl)-N6-methyl-5-oxohexanediamide |
| **II-137**: | 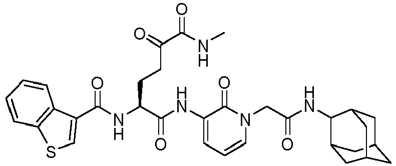 | (S)-2-(benzo[b]thiophene-3-carboxamido)-N1-(1-(2-(2-adamantylamino)-2-oxoethyl)-2-oxo-1,2-dihydropyridin-3-yl)-N6-methyl-5-oxohexanediamide |
| **II-138** | 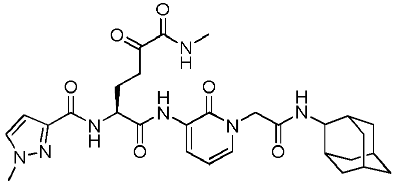 | (S)-N1-(1-(2-(2-adamantylamino)-2-oxoethyl)-2-oxo-1,2-dihydropyridin-3-yl)-N6-methyl-2-(1-methyl-1H-pyrazole-3-carboxamido)-5-oxohexanediamide |
| **II-139** | 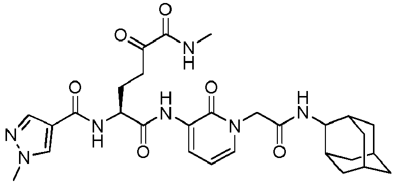 | (S)-N1-(1-(2-(2-adamantylamino)-2-oxoethyl)-2-oxo-1,2-dihydropyridin-3-yl)-N6-methyl-2-(1-methyl-1H-pyrazole-4-carboxamido)-5-oxohexanediamide |
| **II-140** | 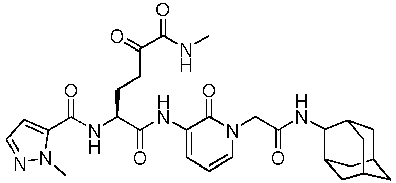 | (S)-N1-(1-(2-(2-adamantylamino)-2-oxoethyl)-2-oxo-1,2-dihydropyridin-3-yl)-N6-methyl-2-(1-methyl-1H-pyrazole-5-carboxamido)-5-oxohexanediamide |
| **II-141** | 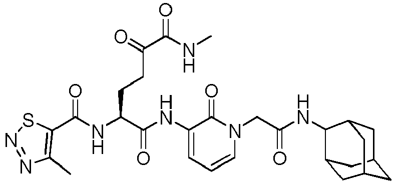 | (S)-N1-(1-(2-(2-adamantylamino)-2-oxoethyl)-2-oxo-1,2-dihydropyridin-3-yl)-N6-methyl-2-(4-methyl-1,2,3-thiadiazole-5-carboxamido)-5-oxohexanediamide |
| **II-142** | 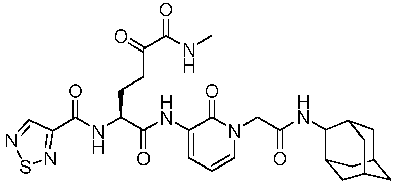 | (S)-N1-(1-(2-(2-adamantylamino)-2-oxoethyl)-2-oxo-1,2-dihydropyridin-3-yl)-N6-methyl-5-oxo-2-(1,2,5-thiadiazole-3-carboxamido)hexanediamide |
| **II-143** | 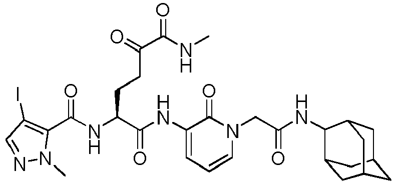 | (S)-2-(4-iodo-1-methyl-1H-pyrazole-5-carboxamido)-N1-(1-(2-(2-adamantylamino)-2-oxoethyl)-2-oxo-1,2-dihydropyridin-3-yl)-N6-methyl-5-oxohexanediamide |
| **II-144** | 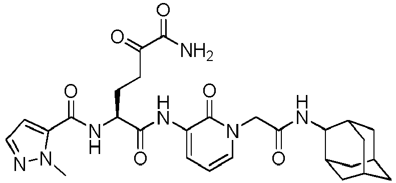 | (S)-N1-(1-(2-(2-adamantylamino)-2-oxoethyl)-2-oxo-1,2-dihydropyridin-3-yl)-2-(1-methyl-1H-pyrazole-5-carboxamido)-5-oxohexanediamide |
| **II-145** | 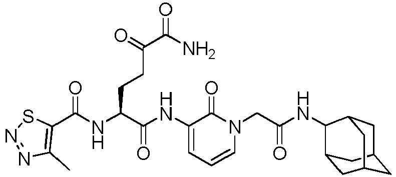 | (S)-N1-(1-(2-(2-adamantylamino)-2-oxoethyl)-2-oxo-1,2-dihydropyridin-3-yl)-2-(4-methyl-1,2,3-thiadiazole-5-carboxamido)-5-oxohexanediamide |
| **II-146** | 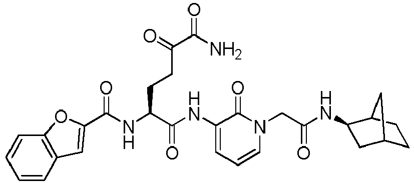 | (S)-2-(benzofuran-2-carboxamido)-N1-(1- (2-((1R,2R,4S)-bicyclo[2.2.1]heptan-2-ylamino)-2-oxoethyl)-2-oxo-1,2-dihydropyridin-3-yl)-5-oxohexanediamide |
| **II-147** | 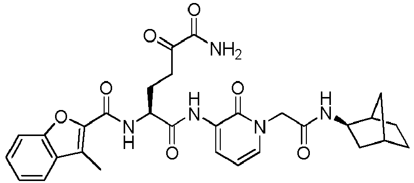 | (S)-N1-(1-(2-((1R,2R,4S)-bicyclo[2.2.1]heptan-2-ylamino)-2-oxoethyl)-2-oxo-1,2-dihydropyridin-3-yl)-2-(3-methylbenzofuran-2-carboxamido)-5-oxohexanediamide |
| **II-148** | 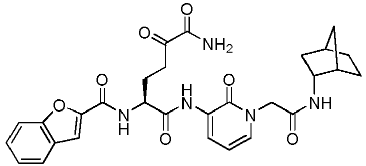 | (S)-2-(benzofu ran-2-carboxam ido)-N1-(1- (2-((1S,2R,4R)-bicyclo[2.2.1]heptan-2-ylamino)-2-oxoethyl)-2-oxo-1,2-dihydropyridin-3-yl)-5-oxohexanediamide |
| **II-149** | 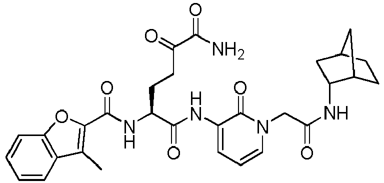 | (S)-N1-(1-(2-((1S,2R,4R)-bicyclo[2.2.1]heptan-2-ylamino)-2-oxoethyl)-2-oxo-1,2-dihydropyridin-3-yl)-2-(3-methylbenzofuran-2-carboxamido)-5-oxohexanediamide |
| **II-150** | 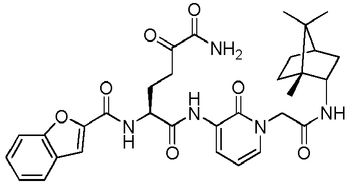 | (S)-2-(benzofuran-2-carboxamido)-5-oxoN1-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-151** | 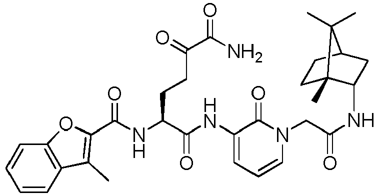 | (S)-2-(3-methylbenzofuran-2-carboxamido)-5-oxo-N1-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-152** | 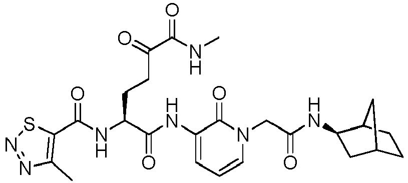 | (S)-N1-(1-(2-((1R,2R,4S)-bicyclo[2.2.1]heptan-2-ylamino)-2-oxoethyl)-2-oxo-1,2-dihydropyridin-3-yl)-N6-methyl-2-(4-methyl-1,2,3-thiadiazole-5-carboxamido)-5-oxohexanediamide |
| **II-153** | 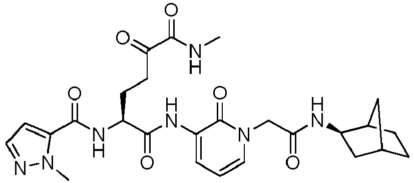 | (S)-N1-(1-(2-((1R,2R,4S)-bicyclo[2.2.1]heptan-2-ylamino)-2-oxoethyl)-2-oxo-1,2-dihydropyridin-3-yl)-N6-methyl-2-(1-methyl-1H-pyrazole-5-carboxamido)-5-oxohexanediamide |
| **II-154** | 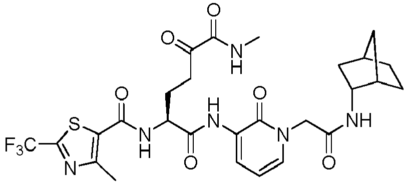 | (S)-N1-(1-(2-((1S,2R,4R)-bicyclo[2.2.1]heptan-2-ylamino)-2-oxoethyl)-2-oxo-1,2-dihydropyridin-3-yl)-N6-methyl-2-(4-methyl-2-(trifluoromethyl)thiazole-5-carboxamido)-5-oxohexanediamide |
| **II-155** | 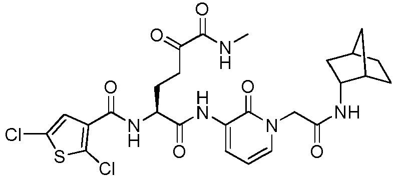 | (S)-N1-(1-(2-((1S,2R,4R)-bicyclo[2.2.1]heptan-2-ylamino)-2-oxoethyl)-2-oxo-1,2-dihydropyridin-3-yl)-2-(2,5-dichlorothiophene-3-carboxamido)-N6-methyl-5-oxohexanediamide |
| **II-156** | 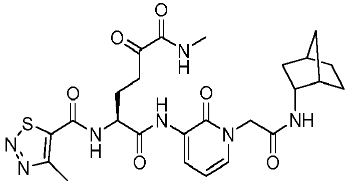 | (S)-N1-(1-(2-((1S,2R,4R)-bicyclo[2.2.1]heptan-2-ylamino)-2-oxoethyl)-2-oxo-1,2-dihydropyridin-3-yl)-N6-methyl-2-(4-methyl-1,2,3-thiadiazole-5-carboxamido)-5-oxohexanediamide |
| **II-157** | 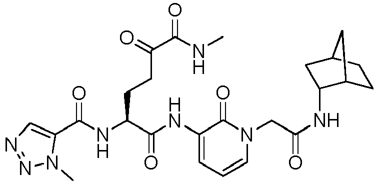 | (S)-N1-(1-(2-((1S,2R,4R)-bicyclo[2.2.1]heptan-2-ylamino)-2-oxoethyl)-2-oxo-1,2-dihydropyridin-3-yl)-N6-methyl-2-(1-methyl-1H-1,2,3-triazole-5-carboxamido)-5-oxohexanediamide |
| **II-158** | 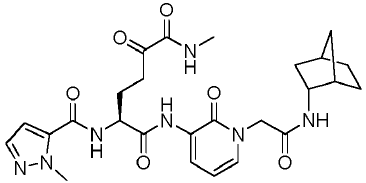 | (S)-N 1-(1-(2-((1S,2R,4R)-bicyclo[2.2.1]heptan-2-ylamino)-2-oxoethyl)-2-oxo-1,2-dihydropyridin-3-yl)-N6-methyl-2-(1-methyl-1H-pyrazole-5-carboxamido)-5-oxohexanediamide |
| **II-159** | 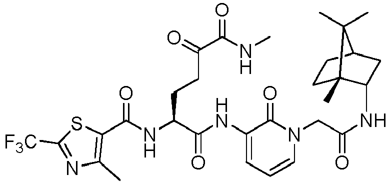 | (S)-N1-methyl-5-(4-methyl-2-(trifluoromethyl)thiazole-5-carboxamido)-2-oxo-N6-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-160** | 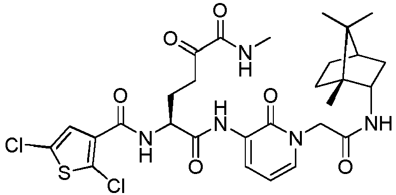 | (S)-2-(2,5-dichlorothiophene-3-carboxamido)-N6-methyl-5-oxo-N1-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-161** | 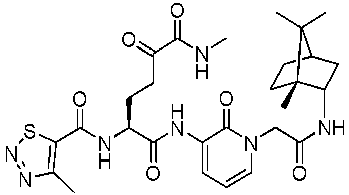 | (S)-N1-methyl-5-(4-methyl-1,2,3-thiadiazole-5-carboxamido)-2-oxo-N6-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-162** | 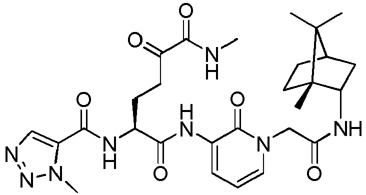 | (S)-N1-methyl-5-(1-methyl-1H-1,2,3-triazole-5-carboxamido)-2-oxo-N6-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-163** | 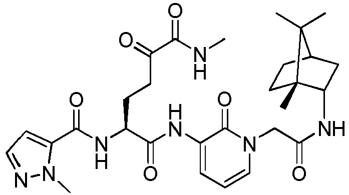 | (S)-N1-methyl-5-(1-methyl-1H-pyrazole-5-carboxamido)-2-oxo-N6-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-164** | 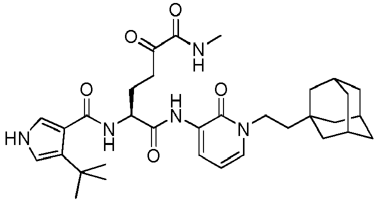 | (2S)-2-(4-tert-butyl-1H-pyrrole-3-carboxamido)-N6-methyl-N1-(1-(2-(1-adamantylamino)ethyl)-2-oxo-1,2-dihydropyridin-3-yl)-5-oxohexanediamide |
| **II-165** | 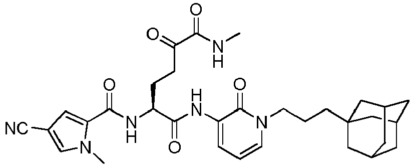 | (2S)-2-(4-cyano-1-methyl-1H-pyrrole-2-carboxamido)-N6-methyl-N1-(1-(3-(1-adamantylamino)propyl)-2-oxo-1,2-dihydropyridin-3-yl)-5-oxohexanediamide |
| **II-166** | 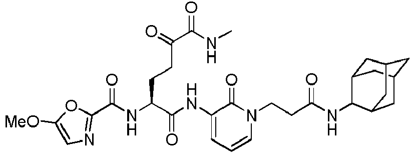 | (S)-N1-(1-(3-(2-adamantylamino)-3-oxopropyl)-2-oxo-1,2-dihydropyridin-3-yl)-2-(5-methoxyoxazole-2-carboxamido)-N6-methyl-5-oxohexanediamide |
| **II-167** | 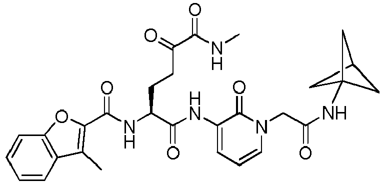 | (S)-N1-(1-(2-(bicyclo[1.1.1]pentan-1- ylamino)-2-oxoethyl)-2-oxo-1,2-dihydropyridin-3-yl)-N6-methyl-2-(3-methylbenzofuran-2-carboxamido)-5-oxohexanediamide |
| **II-168** | 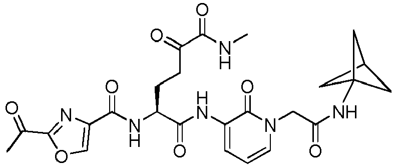 | (S)-2-(2-acetyloxazole-4-carboxamido)-N1-(1-(2-(bicyclo[1.1.1]pentan-1-ylamino)-2-oxoethyl)-2-oxo-1,2-dihydropyridin-3-yl)-N6-methyl-5-oxohexanediamide |
| **II-169** | 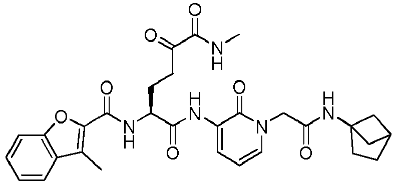 | (S)-N1-(1-(2-(bicyclo[2.1.1]hexan-1-ylamino)-2-oxoethyl)-2-oxo-1,2-dihydropyridin-3-yl)-N6-methyl-2-(3-methylbenzofuran-2-carboxamido)-5-oxohexanediamide |
| **II-170** | 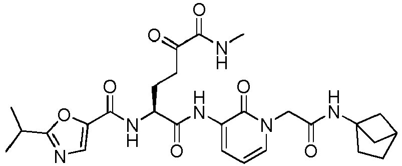 | (S)-N1-(1-(2-(bicyclo[2.1.1]hexan-1-ylamino)-2-oxoethyl)-2-oxo-1,2-dihydropyridin-3-yl)-2-(2-isopropyloxazole-5-carboxamido)-N6-methyl-5-oxohexanediamide |
| **II-171** | 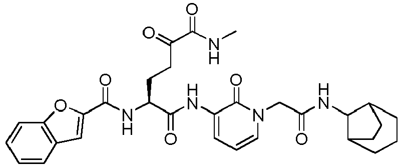 | (2S)-2-(benzofuran-2-carboxamido)-N1-(1-(2-(bicyclo[3.2.1]octan-8-ylamino)-2-oxoethyl)-2-oxo-1,2-dihydropyridin-3-yl)-N6-methyl-5-oxohexanediamide |
| **II-172** | 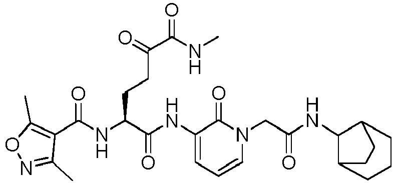 | (2S)-N1-(1-(2-(bicyclo[3.2.1]octan-8-ylamino)-2-oxoethyl)-2-oxo-1,2-dihydropyridin-3-yl)-2-(3,5-dimethylisoxazole-4-carboxamido)-N6-methyl-5-oxohexanediamide |
| **II-173** | 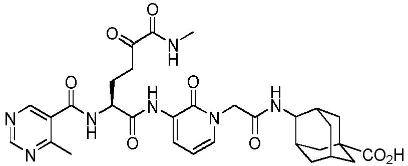 | (S)-N1-(1-(2-(5-carboxy-2-aminoadamantane)-2-oxoethyl)-2-oxo-1,2-dihydropyridin-3-yl)-N6-methyl-2-(4-methylpyrimidine-5-carboxamido)-5-oxohexanediamide |
| **II-174** | 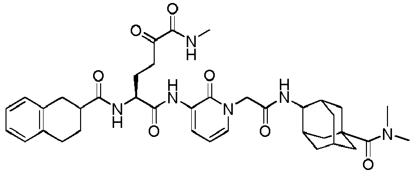 | (2S)-N1-(1-(2-(4-aminoadamantane-N, N-dimethyl-1-carboxamide)-2-oxoethyl)-2-oxo-1,2-dihydropyridin-3-yl)-N6-methyl-5-oxo-2-(1,2,3,4-tetrahydronaphthalene-2-carboxamido)hexanediamide |
| **II-175** | 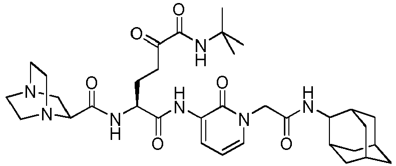 | (S)-2-((S)-1,4-diazabicyclo[2.2.2]octane-2-carboxamido)-N6-tert-butyl-N1-(1-(2-(2-adamantylamino)-2-oxoethyl)-2-oxo-1,2-dihydropyridin-3-yl)-5-oxohexanediamide |
| **II-176** | 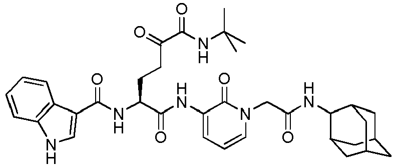 | (S)-N1-tert-butyl-5-(1H-indole-3-carboxamido)-N6-(1-(2-(2-adamantylamino)-2-oxoethyl)-2-oxo-1,2-dihydropyridin-3-yl)-2-oxohexanediamide |
| **II-177** | 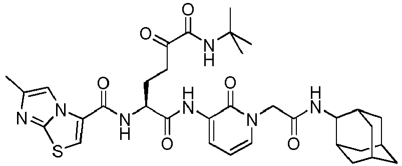 | (S)-N1-tert-butyl-N6-(1-(2-(2-adamantylamino)-2-oxoethyl)-2-oxo-1,2-dihydropyridin-3-yl)-5-(6-methylimidazo[2,1-b]thiazole-3-carboxamido)-2-oxohexanediamide |
| **II-178** | 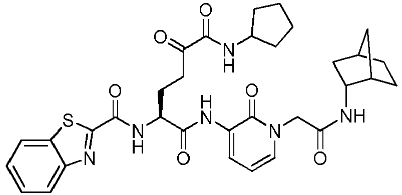 | (S)-2-(benzo[d]thiazole-2-carboxamido)-N1-(1-(2-((1S,2R,4R)-bicyclo[2.2.1]heptan-2-ylamino)-2-oxoethyl)-2-oxo-1,2-dihydropyridin-3-yl)-N6-cyclopentyl-5-oxohexanediamide |
| **II-179** | 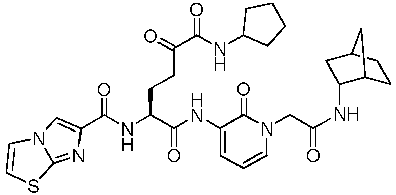 | (S)-N1-(1-(2-((1S,2R,4R)-bicyclo[2.2.1]heptan-2-ylamino)-2-oxoethyl)-2-oxo-1,2-dihydropyridin-3-yl)-N6-cyclopentyl-2-(imidazo[2,1-b]thiazole-6-carboxamido)-5-oxohexanediamide |
| **II-180** | 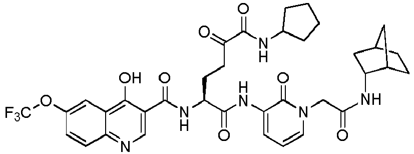 | (S)-N1-(1-(2-((1S,2R,4R)-bicyclo[2.2.1]heptan-2-ylamino)-2-oxoethyl)-2-oxo-1,2-dihydropyridin-3-yl)-N6-cyclopentyl-2-(4-hydroxy-6-(trifluoromethoxy)quinoline-3-carboxamido)-5-oxohexanediamide |
| **II-181** | 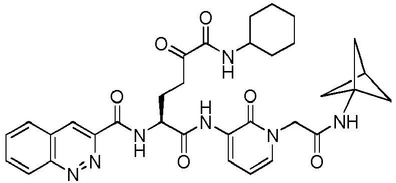 | (S)-N1-(1-(2-(bicyclo[1.1.1]pentan-1-ylamino)-2-oxoethyl)-2-oxo-1,2-dihydropyridin-3-yl)-2-(cinnoline-3-carboxam ido)-N6-cyclohexyl-5-oxohexanediamide |
| **II-182** | 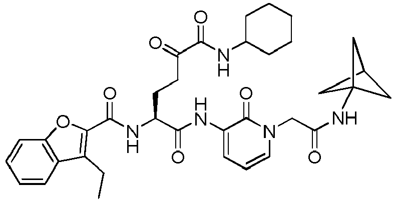 | (S)-N1-(1-(2-(bicyclo[1.1.1]pentan-1- ylamino)-2-oxoethyl)-2-oxo-1,2-dihydropyridin-3-yl)-N6-cyclohexyl-2-(3-ethylbenzofuran-2-carboxamido)-5-oxohexanediamide |
| **II-183** | 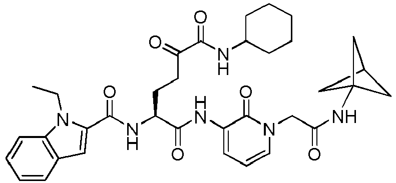 | (S)-N1-(1-(2-(bicyclo[1.1.1]pentan-1-ylamino)-2-oxoethyl)-2-oxo-1,2-dihydropyridin-3-yl)-N6-cyclohexyl-2-(1-ethyl-1H-indole-2-carboxamido)-5-oxohexanediamide |
| **II-184** | 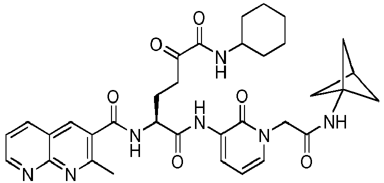 | (S)-N1-(1-(2-(bicyclo[1.1.1]pentan-1-ylamino)-2-oxoethyl)-2-oxo-1,2-dihydropyridin-3-yl)-N6-cyclohexyl-2-(2-methyl-1,8-naphthyridine-3-carboxamido)-5-oxohexanediamide |
| **II-185** | 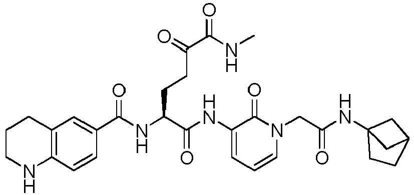 | (S)-N1-(1-(2-(bicyclo[2.1.1]hexan-1-ylamino)-2-oxoethyl)-2-oxo-1,2-dihydropyridin-3-yl)-N6-methyl-5-oxo-2-(1,2,3,4-tetrahydroquinoline-6-carboxamido)hexanediamide |
| **II-186** | 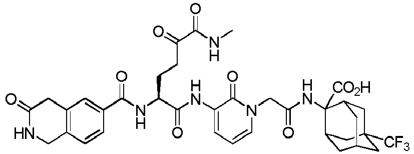 | (S)-N1-(1-(2-(2-carboxy-2-amino-5-(trifluoromethyl)adamantane)-2-oxoethyl)-2-oxo-1,2-dihydropyridin-3-yl)-N6-methyl-5-oxo-2-(3-oxo-1,2,3,4-tetrahydroisoquinoline-6-carboxamido)hexanediamide |
| **II-187** | 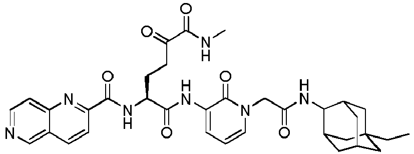 | (S)-N1-(1-(2-(5-ethyladamantane-2-amino)-2-oxoethyl)-2-oxo-1,2-dihydropyridin-3-yl)-N6-methyl-2-(1,6-naphthyridine-2-carboxamido)-5-oxohexanediamide |
| **II-188** | 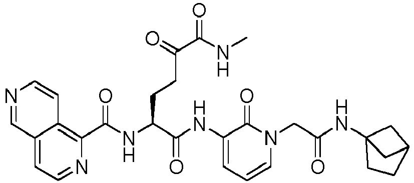 | (S)-N1-(1-(2-(bicyclo[2.1.1]hexan-1-ylamino)-2-oxoethyl)-2-oxo-1,2-dihydropyridin-3-yl)-N6-methyl-2-(2,6-naphthyridine-1-carboxamido)-5-oxohexanediamide |
| **II-189** | 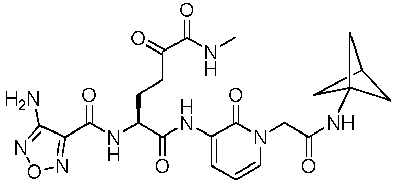 | (S)-2-(4-amino-1,2,5-oxadiazole-3-carboxamido)-N1-(1-(2-(bicyclo[1.1.1]pentan-1-ylamino)-2-oxoethyl)-2-oxo-1,2-dihydropyridin-3-yl)-N6-methyl-5-oxohexanediamide |
| **11-190** | 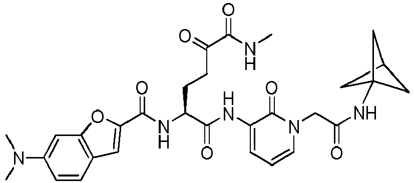 | (S)-N1-(1-(2-(bicyclo[1.1.1]pentan-1- ylamino)-2-oxoethyl)-2-oxo-1,2-dihydropyridin-3-yl)-2-(6-(dimethylamino)benzofuran-2-carboxam ido)-N6-methyl-5-oxohexanediamide |
| **II-191** | 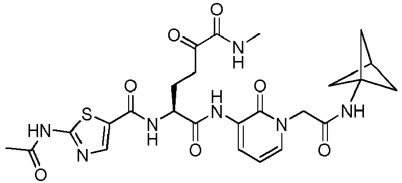 | (S)-2-(2-acetamidothiazole-5-carboxamido)-N1-(1-(2-(bicyclo[1.1.1]pentan-1-ylamino)-2-oxoethyl)-2-oxo-1,2-dihydropyridin-3-yl)-N6-methyl-5-oxohexanediamide |
| **II-192** | 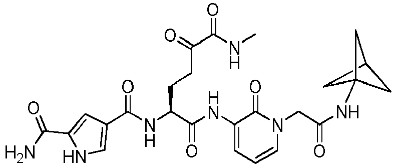 | (S)-N4-(1-(1-(2-(bicyclo[1.1.1]pentan-1-ylamino)-2-oxoethyl)-2-oxo-1,2-dihydropyridin-3-ylamino)-6-(methylamino)-1,5,6-trioxohexan-2-yl)-1H-pyrrole-2,4-dicarboxamide |
| **II-193** | 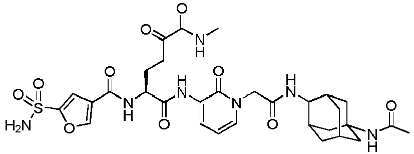 | (S)-N1-(1-(2-(1-acetylamino-4-aminoadamantane)-2-oxoethyl)-2-oxo-1,2-dihydropyridin-3-yl)-N6-methyl-5-oxo-2-(5-sulfamoylfuran-3-carboxamido)hexanediamide |
| **II-194** | 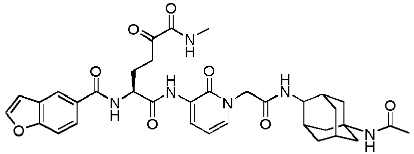 | (S)-2-(benzofuran-5-carboxamido)-N1-(1-(2-(1-acetylamino-4-aminoadamantane)-2-oxoethyl)-2-oxo-1,2-dihydropyridin-3-yl)-N6-methyl-5-oxohexanediamide |
| **II-195** | 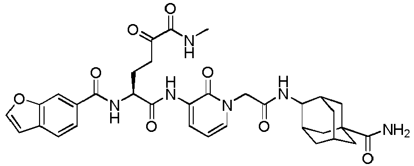 | (S)-2-(benzofuran-6-carboxamido)-N1-(1- (2-(4-aminoadamantane-1-carboxamide)-2-oxoethyl)-2-oxo-1,2-dihydropyridin-3-yl)-N6-methyl-5-oxohexanediamide |
| **II-196** | 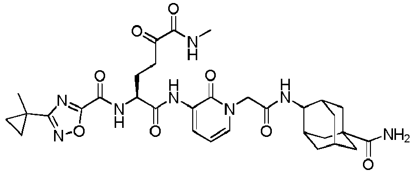 | (S)-N1-(1-(2-(4-aminoadamantane-1-carboxam ide)-2-oxoethyl)-2-oxo-1,2-dihydropyridin-3-yl)-N6-methyl-2-(3-(1-methylcyclopropyl)-1,2,4-oxadiazole-5-carboxamido)-5-oxohexanediamide |
| **II-197** | 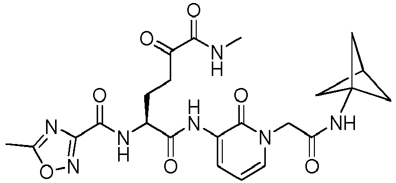 | (S)-N1-(1-(2-(bicyclo[1.1.1]pentan-1- ylamino)-2-oxoethyl)-2-oxo-1,2-dihydropyridin-3-yl)-N6-methyl-2-(5-methyl-1,2,4-oxadiazole-3-carboxamido)-5-oxohexanediamide |
| **II-198** | 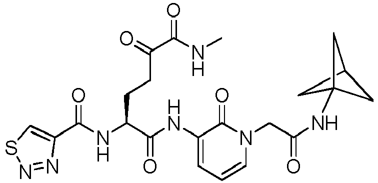 | (S)-N1-(1-(2-(bicyclo[1.1.1]pentan-1- ylamino)-2-oxoethyl)-2-oxo-1,2-dihydropyridin-3-yl)-N6-methyl-5-oxo-2-(1,2,3-thiadiazole-4-carboxamido)hexanediamide |
| **II-199** | 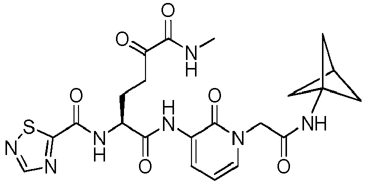 | (S)-N1-(1-(2-(bicyclo[1.1.1]pentan-1- ylamino)-2-oxoethyl)-2-oxo-1,2-dihydropyridin-3-yl)-N6-methyl-5-oxo-2-(1,2,4-thiadiazole-5-carboxamido)hexanediamide |
| **II-200** | 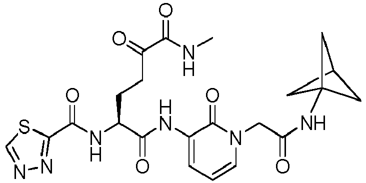 | (S)-N1-(1-(2-(bicyclo[1.1.1]pentan-1-ylamino)-2-oxoethyl)-2-oxo-1,2-dihydropyridin-3-yl)-N6-methyl-5-oxo-2-(1,3,4-thiadiazole-2-carboxamido)hexanediamide |
| **II-201** | 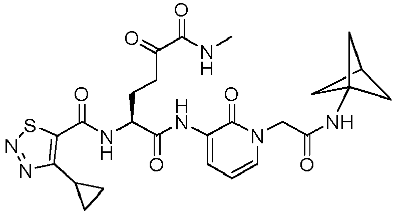 | (S)-N1-(1-(2-(bicyclo[1.1.1]pentan-1-ylamino)-2-oxoethyl)-2-oxo-1,2-dihydropyridin-3-yl)-2-(4-cyclopropyl-1,2,3-thiadiazole-5-carboxamido)-N6-methyl-5-oxohexanediamide |
| **II-202** | 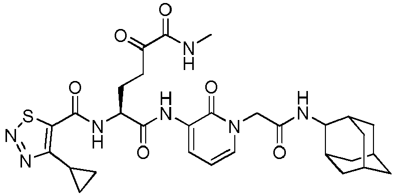 | (S)-2-(4-cyclopropyl-1,2,3-thiadiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-203** | 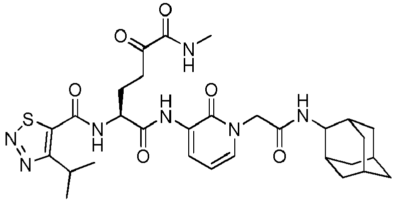 | (S)-2-(4-isopropyl-1,2,3-thiadiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-204** | 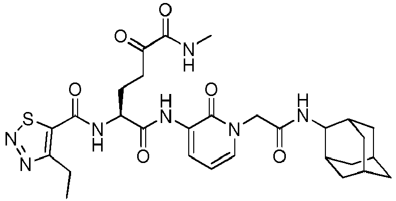 | (S)-2-(4-ethyl-1,2,3-thiadiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-205** | 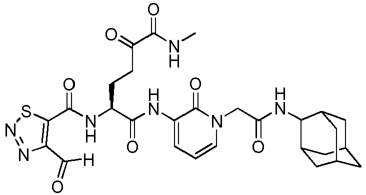 | (S)-2-(4-formyl-1,2,3-thiadiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-206** | 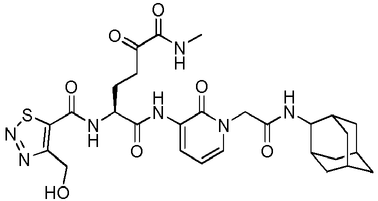 | (S)-2-(4-(hydroxymethyl)-1,2,3-thiadiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-207** | 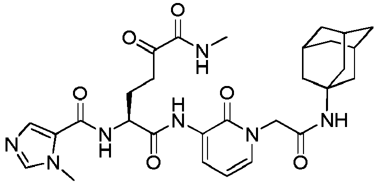 | (S)-N1-(1-(2-(1-adamantylamino)-2-oxoethyl)-2-oxo-1,2-dihydropyridin-3-yl)-N6-methyl-2-(1-methyl-1H-imidazole-5-carboxamido)-5-oxohexanediamide |
| **II-208** | 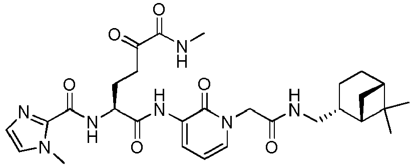 | (S)-N1-(1-(2-(((1S,2R,5S)-6,6-dimethylbicyclo[3.1.1]heptan-2-yl)methylamino)-2-oxoethyl)-2-oxo-1,2-dihydropyridin-3-yl)-N6-methyl-2-(1-methyl-1H-imidazole-2-carboxamido)-5-oxohexanediamide |
| **II-209** | 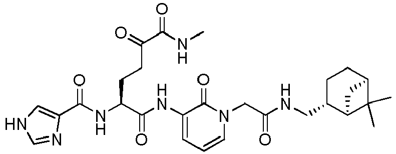 | (S)-N1-(1-(2-(((1R,2R,5R)-6,6-dimethylbicyclo[3.1.1]heptan-2-yl)methylamino)-2-oxoethyl)-2-oxo-1,2-dihydropyridin-3-yl)-2-(1H-imidazole-4-carboxam ido)-N6-methyl-5-oxohexanediamide |
| **II-210** | 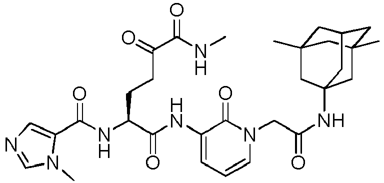 | (S)-N1-(1-(2-(3,5-dimethyladamantane-1-amino)-2-oxoethyl)-2-oxo-1,2-dihydropyridin-3-yl)-N6-methyl-2-(1-methyl-1H-imidazole-5-carboxamido)-5-oxohexanediamide |
| **II-211** | 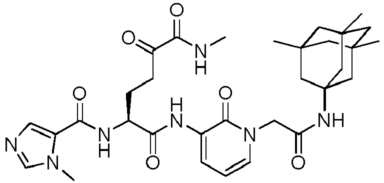 | (S)-N1-(1-(2-(3,5,7-trimethyl-1-adamantylamino)-2-oxoethyl)-2-oxo-1,2-dihydropyridin-3-yl)-N6-methyl-2-(1-methyl-1H-imidazole-5-carboxamido)-5-oxohexanediamide |

or a pharmaceutically acceptable salt thereof.

Especially preferred are the followinQ compounds:

| | |
|---|---|
| **II-2**: | (S)-2-(benzofuran-2-carboxamido)-N1-(1-(2-(2-adamantylamino)-2-oxoethyl)-2-oxo-1,2-dihydropyridin-3-yl)-N6-methyl-5-oxohexanediamide |
| **II-3**: | (S)-N1-(1-(2-(2-adamantylamino)-2-oxoethyl)-2-oxo-1,2-dihydropyridin-3-yl)-N6-methyl-2-(3-methylbenzofuran-2-carboxamido)-5-oxohexanediamide |
| **II-4**: | (S)-2-(3-chlorobenzofuran-2-carboxamido)-N1-(1-(2-(2-adamantylamino)-2-oxoethyl)-2-oxo-1,2-dihydropyridin-3-yl)-N6-methyl-5-oxohexanediamide |
| **II-5**: | (S)-2-(4-bromobenzofuran-2-carboxamido)-N1-(1-(2-(2-adamantylamino)-2-oxoethyl)-2-oxo-1,2-dihydropyridin-3-yl)-N6-methyl-5-oxohexanediamide |
| **II-6**: | (S)-2-(4-bromobenzofuran-2-carboxamido)-N1-(1-(2-(2-adamantylamino)-2-oxoethyl)-2-oxo-1,2-dihydropyridin-3-yl)-N6-methyl-5-oxohexanediamide |
| **II-7**: | (S)-2-(benzo[b]thiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-8**: | (S)-2-(5-bromobenzo[b]thiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-9**: | (S)-2-(1H-indole-2-carboxamido)-N1-(1-(2-(2-adamantylamino)-2-oxoethyl)-2-oxo-1,2-dihydropyridin-3-yl)-N6-methyl-5-oxohexanediamide |
| **II-10:** | (S)-2-(4,5-difluoro-1H-indole-2-carboxamido)-N1-(1-(2-(2-adamantylamino)-2-oxoethyl)-2-oxo-1,2-dihydropyridin-3-yl)-N6-methyl-5-oxohexanediamide |
| **II-11**: | (S)-N1-(1-(2-(2-adamantylamino)-2-oxoethyl)-2-oxo-1 ,2-dihydropyridin-3-yl)-N6-methyl-2-(3-methyl-1H-indole-2-carboxamido)-5-oxohexanediamide |
| **II-12**: | (S)-2-(1H-benzo[d]imidazole-2-carboxamido)-N1-(1-(2-(2-adamantylamino)-2-oxoethyl)-2-oxo-1,2-dihydropyridin-3-yl)-N6-methyl-5-oxohexanediamide |
| **II-13**: | (S)-2-(2,3-dihydro-1H-indene-2-carboxamido)-N1-(1-(2-(2-adamantylamino)-2-oxoethyl)-2-oxo-1,2-dihydropyridin-3-yl)-N6-methyl-5-oxohexanediamide |
| **II-14:** | (S)--(2-bromo-4-methylthiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-15**: | (S)-N1-(1-(2-(2-adamantylamino)-2-oxoethyl)-2-oxo-1,2-dihydropyridin-3-yl)-N6-methyl-2-(4-methyl-2-(trifluoromethyl)thiazole-5-carboxamido)-5-oxohexanediamide |
| **II-16:** | (S)-2-(4-bromo-2-(trifluoromethyl)thiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-17:** | (S)-2-(2,4-dichlorothiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-18:** | (S)-N1-(1-(2-(2-adamantylamino)-2-oxoethyl)-2-oxo-1,2-dihydropyridin-3-yl)-2-(2-methoxy-4-methylthiazole-5-carboxamido)-N6-methyl-5-oxohexanediamide |
| **II-19:** | (S)-N1-(1-(2-(2-adamantylamino)-2-oxoethyl)-2-oxo-1,2-dihydropyridin-3-yl)-N6-methyl-2-(4-methyl-2-phenylthiazole-5-carboxamido)-5-oxohexanediamide |
| **II-21:** | (S)-2-(5-bromo-3-methylthiophene-2-carboxamido)-N 1-(1-(2-(2-adamantylamino)-2-oxoethyl)-2-oxo-1,2-dihydropyridin-3-yl)-N6-methyl-5-oxohexanediamide |
| **II-22:** | (S)-2-(3,5-dibromothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-23:** | (S)-2-(5-bromothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-24:** | (S)-2-(5-chlorothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-25:** | (S)-2-(5-bromo-3-methylfuran-2-carboxamido)-N1-(1-(2-(2-adamantylamino)-2-oxoethyl)-2-oxo-1,2-dihydropyridin-3-yl)-N6-methyl-5-oxohexanediamide |
| **II-26:** | (S)-2-(5-chlorofuran-2-carboxamido)-N1-(1-(2-(2-adamantylamino)-2-oxoethyl)-2-oxo-1,2-dihydropyridin-3-yl)-N6-methyl-5-oxohexanediamide |
| **II-27:** | (S)-2-(5-chlorothiophene-3-carboxamido)-N1-(1-(2-(2-adamantylamino)-2-oxoethyl)-2-oxo-1,2-dihydropyridin-3-yl)-N6-methyl-5-oxohexanediamide |
| **II-28:** | (S)-2-(2,5-dichlorothiophene-3-carboxamido)-N1-(1-(2-(2-adamantylamino)-2-oxoethyl)-2-oxo-1,2-dihydropyridin-3-yl)-N6-methyl-5-oxohexanediamide |
| **II-29:** | (S)-2-(2,5-dibromothiophene-3-carboxamido)-N1-(1-(2-(2-adamantylamino)-2-oxoethyl)-2-oxo-1,2-dihydropyridin-3-yl)-N6-methyl-5-oxohexanediamide |
| **II-30:** | (S)-2-(5-bromothiophene-3-carboxamido)-N1-(1-(2-(2-adamantylamino)-2-oxoethyl)-2-oxo-1,2-dihydropyridin-3-yl)-N6-methyl-5-oxohexanediamide |
| **II-31**: | (S)-2-(2-chloro-5-methylthiazole-4-carboxamido)-N 1-(1-(2-(2-adamantylamino)-2-oxoethyl)-2-oxo-1,2-dihydropyridin-3-yl)-N6-methyl-5-oxohexanediamide |
| **II-32:** | (S)-2-(2,5-dichlorothiazole-4-carboxamido)-N1-(1-(2-(2-adamantylamino)-2-oxoethyl)-2-oxo-1,2-dihydropyridin-3-yl)-N6-methyl-5-oxohexanediamide |
| **II-33:** | (S)-2-(2,5-dibromothiazole-4-carboxamido)-N1-(1-(2-(2-adamantylamino)-2-oxoethyl)-2-oxo-1,2-dihydropyridin-3-yl)-N6-methyl-5-oxohexanediamide |
| **II-34:** | (S)-2-(2-bromo-5-methylthiazole-4-carboxamido)-N 1-(1-(2-(2-adamantylamino)-2-oxoethyl)-2-oxo-1,2-dihydropyridin-3-yl)-N6-methyl-5-oxohexanediamide |
| **II-35:** | (S)-2-(2-bromothiazole-4-carboxamido)-N1-(1-(2-(2-adamantylamino)-2-oxoethyl)-2-oxo-1,2-dihydropyridin-3-yl)-N6-methyl-5-oxohexanediamide |
| **II-36:** | (S)-2-(2-chlorothiazole-4-carboxamido)-N1-(1-(2-(2-adamantylamino)-2-oxoethyl)-2-oxo-1,2-dihydropyridin-3-yl)-N6-methyl-5-oxohexanediamide |
| **II-37:** | (S)-2-(2,5-dimethylfuran-3-carboxamido)-N1-(1-(2-(2-adamantylamino)-2-oxoethyl)-2-oxo-1,2-dihydropyridin-3-yl)-N6-methyl-5-oxohexanediamide |
| **II-39:** | (S)-2-(4-bromothiazole-2-carboxamido)-N1-(1-(2-(2-adamantylamino)-2-oxoethyl)-2-oxo-1,2-dihydropyridin-3-yl)-N6-methyl-5-oxohexanediamide |
| **II-40:** | (S)-2-(4-brom othiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-41:** | (S)-2-(4-bromo-3-methylthiophene-2-carboxamido)-N 1-(1-(2-(2-adamantylamino)-2-oxoethyl)-2-oxo-1,2-dihydropyridin-3-yl)-N6-methyl-5-oxohexanediamide |
| **II-42:** | (S)-2-(3-brom othiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-44:** | (S)-2-(4-bromo-5-chlorothiophene-2-carboxamido)-N 1-(1-(2-(2-adamantylamino)-2-oxoethyl)-2-oxo-1,2-dihydropyridin-3-yl)-N6-methyl-5-oxohexanediamide |
| **II-45:** | (S)-2-(4,5-dibromothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-46:** | (S)-2-(4,5-dibromo-3-methoxythiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-47:** | (S)-2-(4-bromofuran-2-carboxamido)-N1-(1-(2-(2-adamantylamino)-2-oxoethyl)-2-oxo-1,2-dihydropyridin-3-yl)-N6-methyl-5-oxohexanediamide |
| **II-48:** | (S)-2-(4,5-dibromofuran-2-carboxamido)-N1-(1-(2-(2-adamantylamino)-2-oxoethyl)-2-oxo-1,2-dihydropyridin-3-yl)-N6-methyl-5-oxohexanediamide |
| **II-49:** | (S)-2-(4,5-dichlorothiophene-2-carboxamido)-N 1-(1-(2-(2-adamantylamino)-2-oxoethyl)-2-oxo-1,2-dihydropyridin-3-yl)-N6-methyl-5-oxohexanediamide |
| **II-50:** | (S)-2-((S)-1-acetylpyrrolidine-2-carboxamido)-N 1-(1-(2-(2-adamantylamino)-2-oxoethyl)-2-oxo-1,2-dihydropyridin-3-yl)-N6-methyl-5-oxohexanediamide |
| **II-51:** | (S)-N1-(1-(2-(2-adamantylamino)-2-oxoethyl)-2-oxo-1,2-dihydropyridin-3-yl)-N6-methyl-2-(1-methyl-1H-1,2,3-triazole-5-carboxamido)-5-oxohexanediamide |
| **II-53:** | (S)-N1-(1-(2-(2-adamantylamino)-2-oxoethyl)-2-oxo-1,2-dihydropyridin-3-yl)-N6-methyl-5-oxo-2-(pyrazine-2-carboxamido)hexanediamide |
| **II-54:** | (S)-N 1-(1-(2-(2-adamantylamino)-2-oxoethyl)-2-oxo-1,2-dihydropyridin-3-yl)-N6-methyl-2-((S)-1-methylpyrrolidine-2-carboxamido)-5-oxohexanediamide |
| **II-55:** | (S)-N1-(1-(2-(2-adamantylamino)-2-oxoethyl)-2-oxo-1,2-dihydropyridin-3-yl)-N6-methyl-5-oxo-2-((S)-pyrrolidine-3-carboxamido)hexanediamide |
| **II-56:** | (S)-2-((2S,4S)-4-bromopyrrolidine-2-carboxamido)-N1-(1-(2-(2-adamantylamino)-2-oxoethyl)-2-oxo-1,2-dihydropyridin-3-yl)-N6-methyl-5-oxohexanediamide |
| **II-58:** | (S)-N1-(1-(2-(2-adamantylamino)-2-oxoethyl)-2-oxo-1,2-dihydropyridin-3-yl)-N6-methyl-5-oxo-2-((S)-piperidine-2-carboxamido)hexanediamide |
| **II-59:** | (S)-N1-(1-(2-(2-adamantylamino)-2-oxoethyl)-2-oxo-1,2-dihydropyridin-3-yl)-N6-methyl-5-oxo-2-((R)-piperidine-3-carboxamido)hexanediamide |
| **II-60** | (S)-N1-(1-(2-(2-adamantylamino)-2-oxoethyl)-2-oxo-1,2-dihydropyridin-3-yl)-N6-methyl-2-((R)-morpholine-3-carboxamido)-5-oxohexanediamide |
| **II-61** : | (S)-N1-(1-(2-(2-adamantylamino)-2-oxoethyl)-2-oxo-1,2-dihydropyridin-3-yl)-N6-methyl-5-oxo-2-(quinuclidine-3-carboxamido)hexanediamide |
| **II-62:** | (S)-methyl 3-(1-(1-(2-(2-adamantylamino)-2-oxoethyl)-2-oxo-1,2-dihydropyridin-3-ylamino)-6-(methylamino)-1,5,6-trioxohexan-2-ylcarbamoyl)-5-nitrobenzoate |
| **II-63:** | (S)-N1-(1-(2-(2-adamantylamino)-2-oxoethyl)-2-oxo-1 ,2-dihydropyridin-3-yl)-N6-methyl-2-(5-nitronicotinamido)-5-oxohexanediamide |
| **II-64:** | (S)-5-(1-(1-(2-(2-adamantylamino)-2-oxoethyl)-2-oxo-1,2-dihydropyridin-3-ylamino)-6-(methylamino)-1,5,6-trioxohexan-2-ylcarbamoyl)nicotinic acid |
| **II-65:** | (S)-methyl 5-(1-(1-(2-(2-adamantylamino)-2-oxoethyl)-2-oxo-1,2-dihydropyridin-3-ylamino)-6-(methylamino)-1,5,6-trioxohexan-2-ylcarbamoyl)nicotinate |
| **II-68:** | (S)-N1-(1-(2-(5-hydroxyadamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-69:** | (S)-N1-(1-(2-(5-fluoroadamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-70:** | (S)-N1-(1-(2-(5-chloroadamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-71:** | (S)-N1-(1-(2-(5-bromoadamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-72:** | (S)-N1-(1-(2-(5-methyladamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-73:** | (S)-N1-(1-(2-(2-carbonitrileadamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-87:** | (S)-N1-(1-(2-(1-adamantylmethylamino)-2-oxoethyl)-2-oxo-1,2-dihydropyridin-3-yl)-N6-methyl-2-(3-methylbenzofuran-2-carboxamido)-5-oxohexanediamide |
| **II-88:** | (S)-N1-(1-(2-(1-(1-adamantyl)ethanamino)-2-oxoethyl)-2-oxo-1,2-dihydropyridin-3-yl)-N6-methyl-2-(3-methylbenzofuran-2-carboxamido)-5-oxohexanediamide |
| **II-90:** | (S)-N1-(1-(2-((1R,2S,4S)-bicyclo[2.2.1]heptan-2-ylamino)-2-oxoethyl)-2-oxo-1,2-dihydropyridin-3-yl)-N6-methyl-2-(3-methylbenzofuran-2-carboxamido)-5-oxohexanediamide |
| **II-92:** | (S)-N1-methyl-5-(3-methylbenzofuran-2-carboxamido)-2-oxo-N6-(2-oxo-1-(2-oxo-2-((1S,2S,4R)-1,7,7-trimethylbicyclo[2.2.1]heptan-2-ylamino)ethyl)-1,2-dihydropyridin-3-yl)hexanediamide |
| **II-94:** | (S)-N1-(1-(2-((1R,2R,4S)-bicyclo[2.2.1]heptan-2-ylamino)-2-oxoethyl)-2-oxo-1,2-dihydropyridin-3-yl)-N6-methyl-2-(3-methylbenzofuran-2-carboxamido)-5-oxohexanediamide |
| **II-95:** | (S)-N1-(1-(2-(bicyclo[2.2.1]heptan-1-ylamino)-2-oxoethyl)-2-oxo-1,2-dihydropyridin-3-yl)-N6-methyl-2-(3-methylbenzofuran-2-carboxamido)-5-oxohexanediamide |
| **II-96:** | (S)-N1-(1-(2-(bicyclo[2.2.1]heptan-7-ylamino)-2-oxoethyl)-2-oxo-1,2-dihydropyridin-3-yl)-N6-methyl-2-(3-methylbenzofuran-2-carboxamido)-5-oxohexanediamide |
| **II-97:** | (S)-N1-(1-(2-(bicyclo[2.2.1]hept-5-en-2-ylamino)-2-oxoethyl)-2-oxo-1,2-dihydropyridin-3-yl)-N6-methyl-2-(3-methylbenzofuran-2-carboxamido)-5-oxohexanediamide |
| **II-98:** | (2S)-N1-(1-(2-(bicyclo[2.2.2]octan-2-ylamino)-2-oxoethyl)-2-oxo-1,2-dihydropyridin-3-yl)-N6-methyl-2-(3-methylbenzofuran-2-carboxamido)-5-oxohexanediamide |
| **II-99:** | (S)-N1-methyl-5-(3-methylbenzofuran-2-carboxamido)-2-oxo-N6-(2-oxo-1-(2-oxo-2-((1R,2R,4R)-1,7,7-trimethylbicyclo[2.2.1]heptan-2-ylamino)ethyl)-1,2-dihydropyridin-3-yl)hexanediamide |
| **II-100:** | (S)-N1-methyl-5-(3-methylbenzofuran-2-carboxamido)-2-oxo-N6-(2-oxo-1-(2-oxo-2-((1R,2R,3R,5S)-2,6,6-trimethylbicyclo[3.1.1]heptan-3-ylamino)ethyl)-1,2-dihydropyridin-3-yl)hexanediamide |
| **II-101**: | (S)-N1-methyl-5-(3-methylbenzofuran-2-carboxamido)-2-oxo-N6-(2-oxo-1-(2-oxo-2-((1S,2S,3S,5R)-2,6,6-trimethylbicyclo[3.1.1]heptan-3-ylamino)ethyl)-1,2-dihydropyridin-3-yl)hexanediamide |
| **II-103:** | (S)-N 1-(1-(2-(4-homoisotwistane-3-amino)-2-oxoethyl)-2-oxo-1,2-dihydropyridin-3-yl)-N6-methyl-2-(3-methylbenzofuran-2-carboxamido)-5-oxohexanediamide |
| **II-104:** | (S)-N1-(1-(2-(diamantane-1-amino)-2-oxoethyl)-2-oxo-1,2-dihydropyridin-3-yl)-N6-methyl-2-(3-methylbenzofuran-2-carboxamido)-5-oxohexanediamide |
| **II-105:** | (S)-N1-(1-(2-(diamantane-4-amino)-2-oxoethyl)-2-oxo-1,2-dihydropyridin-3-yl)-N6-methyl-2-(3-methylbenzofuran-2-carboxamido)-5-oxohexanediamide |
| **II-107:** | (S)-N1-(1-(1-adamantylmethyl)-2-oxo-1,2-dihydropyridin-3-yl)-N6-methyl-2-(3-methylbenzofuran-2-carboxamido)-5-oxohexanediamide |
| **II-108:** | (2S)-N1-(1-((3-hydroxy-1-adamantyl)methyl)-2-oxo-1,2-dihydropyridin-3-yl)-N6-methyl-2-(3-methylbenzofuran-2-carboxamido)-5-oxohexanediamide |
| **II-109:** | (2S)-N1-(1-((3-bromo-1-adamantyl)methyl)-2-oxo-1,2-dihydropyridin-3-yl)-N6-methyl-2-(3-methylbenzofuran-2-carboxamido)-5-oxohexanediamide |
| **II-110:** | (S)-N1-(1-(2-adamantylmethyl)-2-oxo-1,2-dihydropyridin-3-yl)-N6-methyl-2-(3-methylbenzofuran-2-carboxamido)-5-oxohexanediamide |
| **II-111:** | (S)-N1-(1-(2-(2-adamantylamino)-2-oxoethyl)-2-oxo-1,2-dihydropyridin-3-yl)-N6-methyl-2-(nicotinamido)-5-oxohexanediamide |
| **II-112:** | (S)-2-(isonicotinamido)-N1-(1-(2-(2-adamantylamino)-2-oxoethyl)-2-oxo-1,2-dihydropyridin-3-yl)-N6-methyl-5-oxohexanediamide |
| **II-113:** | (S)-N1-(1-(2-(2-adamantylamino)-2-oxoethyl)-2-oxo-1,2-dihydropyridin-3-yl)-N6-methyl-5-oxo-2-(pyridazine-4-carboxamido)hexanediamide |
| **II-114:** | (S)-N1-(1-(2-(2-adamantylamino)-2-oxoethyl)-2-oxo-1,2-dihydropyridin-3-yl)-N6-methyl-5-oxo-2-(pyridazine-3-carboxamido)hexanediamide |
| **II-115:** | (S)-N1-cyclopropyl-N6-(1-(2-(2-adamantylamino)-2-oxoethyl)-2-oxo-1,2-dihydropyridin-3-yl)-5-(3-methylbenzofuran-2-carboxamido)-2-oxohexanediamide |
| **II-116:** | (S)-N1-(1-(2-(2-adamantylamino)-2-oxoethyl)-2-oxo-1,2-dihydropyridin-3-yl)-2-(3-methylbenzofuran-2-carboxamido)-5-oxo-N6-pentylhexanediamide |
| **II-117:** | (S)-N1-allyl-N6-(1-(2-(2-adamantylamino)-2-oxoethyl)-2-oxo-1,2-dihydropyridin-3-yl)-5-(3-methylbenzofuran-2-carboxamido)-2-oxohexanediamide |
| **II-118:** | (S)-N1-(1-(2-(2-adamantylamino)-2-oxoethyl)-2-oxo-1,2-dihydropyridin-3-yl)-2-(3-methylbenzofuran-2-carboxamido)-5-oxohexanediamide |
| **II-119:** | (S)-N1-allyl-5-(benzofuran-2-carboxamido)-N6-(1-(2-(2-adamantylamino)-2-oxoethyl)-2-oxo-1,2-dihydropyridin-3-yl)-2-oxohexanediamide |
| **II-120:** | (S)-2-(benzofuran-2-carboxamido)-N6-isopropyl-N1-(1-(2-(2-adamantylamino)-2-oxoethyl)-2-oxo-1,2-dihydropyridin-3-yl)-5-oxohexanediamide |
| **II-121**: | (S)-2-(benzofuran-2-carboxamido)-N6-cyclopropyl-N1-(1-(2-(2-adamantylamino)-2-oxoethyl)-2-oxo-1,2-dihydropyridin-3-yl)-5-oxohexanediamide |
| **II-124:** | (S)-2-(benzofuran-2-carboxamido)-N 1-(1-(2-(2-adamantylamino)-2-oxoethyl)-2-oxo-1,2-dihydropyridin-3-yl)-5-oxohexanediamide |
| **II-125:** | (S)-2-(2,5-dichlorothiophene-3-carboxamido)-N1-(1-(2-(2-adamantylamino)-2-oxoethyl)-2-oxo-1,2-dihydropyridin-3-yl)-5-oxohexanediamide |
| **II-126:** | (S)-N1-(1-(2-(2-adamantylamino)-2-oxoethyl)-2-oxo-1,2-dihydropyridin-3-yl)-2-(4-methyl-2-(trifluoromethyl)thiazole-5-carboxamido)-5-oxohexanediamide |
| **II-127:** | (S)-N1-(1-(2-(2-adamantylamino)-2-oxoethyl)-2-oxo-1,2-dihydropyridin-3-yl)-2-(1-methyl-1H-1,2,3-triazole-5-carboxamido)-5-oxohexanediamide |
| **II-128:** | (2S)-N1-(1-(2-((1R,2R,4S)-bicyclo[2.2.1]heptan-2-ylamino)-2-oxoethyl)-2-oxo-1,2-dihydropyridin-3-yl)-2-(2,5-dichlorothiophene-3-carboxamido)-N6-methyl-5-oxohexanediamide |
| **II-129:** | (2S)-N1-(1-(2-((1R,2R,4S)-bicyclo[2.2.1]heptan-2-ylamino)-2-oxoethyl)-2-oxo-1,2-dihydropyridin-3-yl)-N6-methyl-2-(4-methyl-2-(trifluoromethyl)thiazole-5-carboxamido)-5-oxohexanediamide |
| **II-130:** | (2S)-N1-(1-(2-((1R,2R,4S)-bicyclo[2.2.1]heptan-2-ylamino)-2-oxoethyl)-2-oxo-1,2-dihydropyridin-3-yl)-N6-methyl-2-(1-methyl-1H-1,2,3-triazole-5-carboxamido)-5-oxohexanediamide |
| **II-131:** | (S)-N1-(1-(2-(2-adamantylamino)-2-oxoethyl)-2-oxo-1,2-dihydropyridin-3-yl)-N6-methyl-5-oxo-2-(2H-1,2,3-triazole-4-carboxamido)hexanediamide |
| **II-135:** | (S)-N1-(1-(2-(2-adamantylamino)-2-oxoethyl)-2-oxo-1,2-dihydropyridin-3-yl)-N6-methyl-2-(1-methyl-1H-1,2,4-triazole-3-carboxamido)-5-oxohexanediamide |
| **II-136:** | (S)-2-(benzofuran-3-carboxamido)-N 1-(1-(2-(2-adamantylamino)-2-oxoethyl)-2-oxo-1,2-dihydropyridin-3-yl)-N6-methyl-5-oxohexanediamide |
| **II-137:** | (S)-2-(benzo[b]thiophene-3-carboxamido)-N1-(1-(2-(2-adamantylamino)-2-oxoethyl)-2-oxo-1,2-dihydropyridin-3-yl)-N6-methyl-5-oxohexanediamide |
| **II-138** | (S)-N1-(1-(2-(2-adamantylamino)-2-oxoethyl)-2-oxo-1,2-dihydropyridin-3-yl)-N6-methyl-2-(1-methyl-1H-pyrazole-3-carboxamido)-5-oxohexanediamide |
| **II-139** | (S)-N1-(1-(2-(2-adamantylamino)-2-oxoethyl)-2-oxo-1,2-dihydropyridin-3-yl)-N6-methyl-2-(1-methyl-1H-pyrazole-4-carboxamido)-5-oxohexanediamide |
| **II-140** | (S)-N1-(1-(2-(2-adamantylamino)-2-oxoethyl)-2-oxo-1,2-dihydropyridin-3-yl)-N6-methyl-2-(1-methyl-1H-pyrazole-5-carboxamido)-5-oxohexanediamide |
| **II-141** | (S)-N1-(1-(2-(2-adamantylamino)-2-oxoethyl)-2-oxo-1,2-dihydropyridin-3-yl)-N6-methyl-2-(4-methyl-1,2,3-thiadiazole-5-carboxamido)-5-oxohexanediamide |
| **II-142** | (S)-N1-(1-(2-(2-adamantylamino)-2-oxoethyl)-2-oxo-1,2-dihydropyridin-3-yl)-N6-methyl-5-oxo-2-(1,2,5-thiadiazole-3-carboxamido)hexanediamide |
| **II-145** | (S)-N1-(1-(2-(2-adamantylamino)-2-oxoethyl)-2-oxo-1,2-dihydropyridin-3-yl)-2-(4-methyl-1,2,3-thiadiazole-5-carboxamido)-5-oxohexanediamide |
| **II-146** | (S)-2-(benzofuran-2-carboxamido)-N1-(1-(2-((1R,2R,4S)-bicyclo[2.2.1]heptan-2-ylamino)-2-oxoethyl)-2-oxo-1,2-dihydropyridin-3-yl)-5-oxohexanediamide |
| **II-147** | (S)-N1-(1-(2-((1R,2R,4S)-bicyclo[2.2.1]heptan-2-ylamino)-2-oxoethyl)-2-oxo-1,2-dihydropyridin-3-yl)-2-(3-methylbenzofuran-2-carboxamido)-5-oxohexanediamide |
| **II-148** | (S)-2-(benzofuran-2-carboxamido)-N1-(1-(2-((1S,2R,4R)-bicyclo[2.2.1]heptan-2-ylamino)-2-oxoethyl)-2-oxo-1,2-dihydropyridin-3-yl)-5-oxohexanediamide |
| **II-149** | (S)-N1-(1-(2-((1S,2R,4R)-bicyclo[2.2.1]heptan-2-ylamino)-2-oxoethyl)-2-oxo-1,2-dihydropyridin-3-yl)-2-(3-methylbenzofuran-2-carboxamido)-5-oxohexanediamide |
| **II-150** | (S)-2-(benzofuran-2-carboxamido)-5-oxo-N1-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-151** | (S)-2-(3-methylbenzofuran-2-carboxamido)-5-oxo-N1-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-152** | (S)-N1-(1-(2-((1R,2R,4S)-bicyclo[2.2.1]heptan-2-ylamino)-2-oxoethyl)-2-oxo-1,2-dihydropyridin-3-yl)-N6-methyl-2-(4-methyl-1,2,3-thiadiazole-5-carboxamido)-5-oxohexanediamide |
| **II-154** | (S)-N1-(1-(2-((1S,2R,4R)-bicyclo[2.2.1]heptan-2-ylamino)-2-oxoethyl)-2-oxo-1,2-dihydropyridin-3-yl)-N6-methyl-2-(4-methyl-2-(trifluoromethyl)thiazole-5-carboxamido)-5-oxohexanediamide |
| **II-155** | (S)-N1-(1-(2-((1S,2R,4R)-bicyclo[2.2.1]heptan-2-ylamino)-2-oxoethyl)-2-oxo-1,2-dihydropyridin-3-yl)-2-(2,5-dichlorothiophene-3-carboxamido)-N6-methyl-5-oxohexanediamide |
| **II-156** | (S)-N1-(1-(2-((1S,2R,4R)-bicyclo[2.2.1]heptan-2-ylamino)-2-oxoethyl)-2-oxo-1,2-dihydropyridin-3-yl)-N6-methyl-2-(4-methyl-1,2,3-thiadiazole-5-carboxamido)-5-oxohexanediamide |
| **II-157** | (S)-N1-(1-(2-((1S,2R,4R)-bicyclo[2.2.1]heptan-2-ylamino)-2-oxoethyl)-2-oxo-1,2-dihydropyridin-3-yl)-N6-methyl-2-(1-methyl-1H-1,2,3-triazole-5-carboxamido)-5-oxohexanediamide |
| **II-158** | (S)-N1-(1-(2-((1S,2R,4R)-bicyclo[2.2.1]heptan-2-ylamino)-2-oxoethyl)-2-oxo-1,2-dihydropyridin-3-yl)-N6-methyl-2-(1-methyl-1H-pyrazole-5-carboxamido)-5-oxohexanediamide |
| **II-159** | (S)-N1-methyl-5-(4-methyl-2-(trifluoromethyl)thiazole-5-carboxamido)-2-oxo-N6-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-160** | (S)-2-(2,5-dichlorothiophene-3-carboxamido)-N6-methyl-5-oxo-N1-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-161** | (S)-N1-methyl-5-(4-methyl-1,2,3-thiadiazole-5-carboxamido)-2-oxo-N6-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-162** | (S)-N1-methyl-5-(1-methyl-1H-1,2,3-triazole-5-carboxamido)-2-oxo-N6-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-163** | (S)-N1-methyl-5-(1-methyl-1H-pyrazole-5-carboxamido)-2-oxo-N6-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-167** | (S)-N1-(1-(2-(bicyclo[1.1.1]pentan-1-ylamino)-2-oxoethyl)-2-oxo-1,2-dihydropyridin-3-yl)-N6-methyl-2-(3-methylbenzofuran-2-carboxamido)-5-oxohexanediamide |
| **II-168** | (S)-2-(2-acetyloxazole-4-carboxamido)-N1-(1-(2-(bicyclo[1.1.1]pentan-1-ylamino)-2-oxoethyl)-2-oxo-1,2-dihydropyridin-3-yl)-N6-methyl-5-oxohexanediamide |
| **II-169** | (S)-N1-(1-(2-(bicyclo[2.1.1]hexan-1-ylamino)-2-oxoethyl)-2-oxo-1,2-dihydropyridin-3-yl)-N6-methyl-2-(3-methylbenzofuran-2-carboxamido)-5-oxohexanediamide |
| **II-170** | (S)-N1-(1-(2-(bicyclo[2.1.1]hexan-1-ylamino)-2-oxoethyl)-2-oxo-1,2-dihydropyridin-3-yl)-2-(2-isopropyloxazole-5-carboxamido)-N6-methyl-5-oxohexanediamide |
| **II-171** | (2S)-2-(benzofuran-2-carboxamido)-N1-(1-(2-(bicyclo[3.2.1]octan-8-ylamino)-2-oxoethyl)-2-oxo-1,2-dihydropyridin-3-yl)-N6-methyl-5-oxohexanediamide |
| **II-172** | (2S)-N1-(1-(2-(bicyclo[3.2.1]octan-8-ylamino)-2-oxoethyl)-2-oxo-1,2-dihydropyridin-3-yl)-2-(3,5-dimethylisoxazole-4-carboxamido)-N6-methyl-5-oxohexanediamide |
| **II-173** | (S)-N1-(1-(2-(5-carboxy-2-aminoadamantane)-2-oxoethyl)-2-oxo-1,2-dihydropyridin-3-yl)-N6-methyl-2-(4-methylpyrimidine-5-carboxamido)-5-oxohexanediamide |
| **II-174** | (2S)-N1-(1-(2-(4-aminoadamantane-N,N-dimethyl-1-carboxamide)-2-oxoethyl)-2-oxo-1,2-dihydropyridin-3-yl)-N6-methyl-5-oxo-2-(1,2,3,4-tetrahydronaphthalene-2-carboxamido)hexanediamide |
| **II-178** | (S)-2-(benzo[d]thiazole-2-carboxamido)-N1-(1-(2-((1S,2R,4R)-bicyclo[2.2.1]heptan-2-ylamino)-2-oxoethyl)-2-oxo-1,2-dihydropyridin-3-yl)-N6-cyclopentyl-5-oxohexanediamide |
| **II-182** | (S)-N1-(1-(2-(bicyclo[1.1.1]pentan-1-ylamino)-2-oxoethyl)-2-oxo-1,2-dihydropyridin-3-yl)-N6-cyclohexyl-2-(3-ethylbenzofuran-2-carboxamido)-5-oxohexanediamide |
| **II-183** | (S)-N1-(1-(2-(bicyclo[1.1.1]pentan-1-ylamino)-2-oxoethyl)-2-oxo-1,2-dihydropyridin-3-yl)-N6-cyclohexyl-2-(1-ethyl-1H-indole-2-carboxamido)-5-oxohexanediamide |
| **II-194** | (S)-2-(benzofuran-5-carboxamido)-N1-(1-(2-(1-acetylamino-4-aminoadamantane)-2-oxoethyl)-2-oxo-1,2-dihydropyridin-3-yl)-N6-methyl-5-oxohexanediamide |
| **II-195** | (S)-2-(benzofuran-6-carboxamido)-N1-(1-(2-(4-aminoadamantane-1-carboxamide)-2-oxoethyl)-2-oxo-1,2-dihydropyridin-3-yl)-N6-methyl-5-oxohexanediamide |
| **II-198** | (S)-N1-(1-(2-(bicyclo[1.1.1]pentan-1-ylamino)-2-oxoethyl)-2-oxo-1,2-dihydropyridin-3-yl)-N6-methyl-5-oxo-2-(1,2,3-thiadiazole-4-carboxamido)hexanediamide |
| **II-199** | (S)-N1-(1-(2-(bicyclo[1.1.1]pentan-1-ylamino)-2-oxoethyl)-2-oxo-1,2-dihydropyridin-3-yl)-N6-methyl-5-oxo-2-(1,2,4-thiadiazole-5-carboxamido)hexanediamide |
| **II-200** | (S)-N1-(1-(2-(bicyclo[1.1.1]pentan-1-ylamino)-2-oxoethyl)-2-oxo-1,2-dihydropyridin-3-yl)-N6-methyl-5-oxo-2-(1,3,4-thiadiazole-2-carboxamido)hexanediamide |
| **II-201** | (S)-N1-(1-(2-(bicyclo[1.1.1]pentan-1-ylamino)-2-oxoethyl)-2-oxo-1,2-dihydropyridin-3-yl)-2-(4-cyclopropyl-1,2,3-thiadiazole-5-carboxamido)-N6-methyl-5-oxohexanediamide |
| **II-202** | (S)-2-(4-cyclopropyl-1,2,3-thiadiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-203** | (S)-2-(4-isopropyl-1,2,3-thiadiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-204** | (S)-2-(4-ethyl-1,2,3-thiadiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-205** | (S)-2-(4-formyl-1,2,3-thiadiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-206** | (S)-2-(4-(hydroxymethyl)-1,2,3-thiadiazole-5-carboxamido)-N 1-(1-(2-(2-adamantylamino)-2-oxoethyl)-2-oxo-1,2-dihydropyridin-3-yl)-N6-methyl-5-oxohexanediamide |
| **II-207** | (S)-N1-(1-(2-(1-adamantylamino)-2-oxoethyl)-2-oxo-1,2-dihydropyridin-3-yl)-N6-methyl-2-(1-methyl-1H-imidazole-5-carboxamido)-5-oxohexanediamide |
| **II-210** | (S)-N1-(1-(2-(3,5-dimethyladamantane-1-amino)-2-oxoethyl)-2-oxo-1,2-dihydropyridin-3-yl)-N6-methyl-2-(1-methyl-1H-imidazole-5-carboxamido)-5-oxohexanediamide |
| **II-211** | (S)-N1-(1-(2-(3,5,7-trimethyl-1-adamantylamino)-2-oxoethyl)-2-oxo-1,2-dihydropyridin-3-yl)-N6-methyl-2-(1-methyl-1H-imidazole-5-carboxamido)-5-oxohexanediamide |

or a pharmaceutically acceptable salt thereof.

### Method for production of inventive compounds

In some embodiments, the present invention relates to a method for the synthesis of a compound of formula (**I**), especially any compound of the formula (**Ib**):

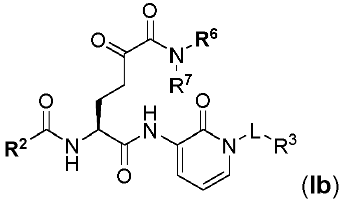

The compound of the formula (**Ib**) can be produced and thus, the present invention relates to a method for producing the compound of formula (**Ib**) comprising the following steps in the following order:
**Step 1B**: providing a compound **4b** 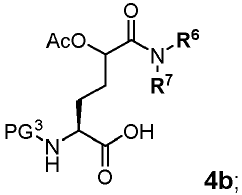
**Step 2B**: performing coupling reaction of the compound **4b** with a compound **5** 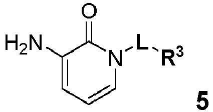 to obtain a compound **6b** 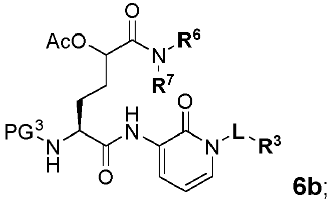
**Step 3B**: deprotecting an amino protecting group PG³ to obtain a compound **7b** 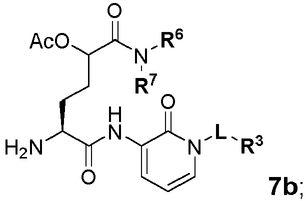
**Step 4B:** performing coupling reaction of the compound **7b** with a carboxylic acid (**R²**-CO₂H **8**) to obtain a compound **9b** 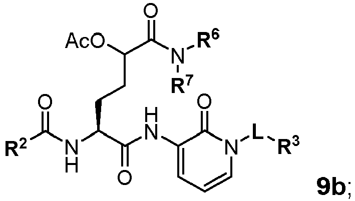
**Step 5B:** performing oxidation reaction of the compound **9b** to produce the compound of the formula (**Ib**) 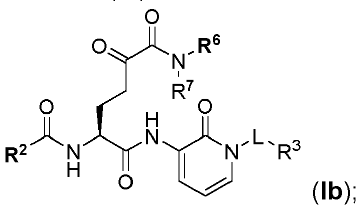
wherein L, **R², R³, R⁶**and **R⁷** have the same meanings as defined above in the formula (**Ib**), and **PG³** is an amino protecting group.

In the step **58** the chemical warhead precursor 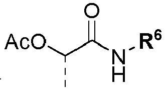 may be firstly converted to 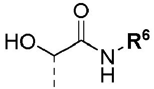 under a basic condition such as treating with K₂CO₃, and then 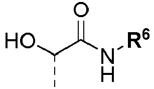 is converted to the corresponding chemical warhead 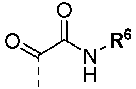 by an oxidation method, preferably by using Dess-Martin periodinane (DMP), iodoxybenzoic acid (IBX), or hypochlorite/TEMPO (2,2,6,6-tetramethylpiperidine-1-oxyl) in a polar solvent, as described in the chemical examples.

In an alternative route first all protecting groups PG¹ and PG² are simultaneously removed and the protecting group PG³ is selectively introduced. Preferably, PG¹ and PG³ are same.

The term "protecting groups" as used herein refers to commonly used protection groups in organic synthesis, preferably for amino and carboxyl groups. PG¹, PG³, and PG⁵ preferably are suitable protecting groups for amino groups. PG² and PG⁴ preferably are suitable protecting groups for carboxyl groups. Preferably, PG¹, PG³, and PG⁵ may be selected from the group consisting of or comprising: acetyl, benzoyl, benzyloxycarbonyl (Cbz), tert-butylcarbonyl, tert-butyloxycarbonyl (Boc), and fluorenylmethylenoxy group (Fmoc). PG² and PG⁴ may be selected from the group consisting of or comprising: methoxy, ethoxy, isobutoxy, tert-butoxy, benzyloxy; preferably, tert-butoxy group.

In Step **2B**, to promote the coupling reaction with amino group of intermediate compound, activating reagents are commonly used to activating carboxylic acid (*"*Peptide Coupling Reagents, More than a Letter Soup", Ayman EI-Faham and Fernando Albericio, Chemical Reviews, 2011, 111(11), p.6557-6602). The activation may be introduced separate reaction or *in situ* reaction. Preferably, any of the following coupling reagent can be used to activate carobxylic acid group: BOP (Benzotriazole-1-yl-oxy-tris-(dimethylamino)-phosphonium hexafluorophosphate), PyBOP (Benzotriazole-1-yl-oxy-tris-pyrrolidino-phosphonium hexafluorophosphate), AOP (7-(Azabenzotriazol-1-yl)oxy tris(dimethylamino)phosphonium hexafluorophosphate), PyAOP ((7-Azabenzotriazol-1-yloxy)tripyrrolidinophosphonium hexafluorophosphate), TBTU (2-(1H-Benzotriazole-1-yl)-1,1,3,3-tetramethylaminium tetrafluoroborate), EEDQ (*N-*Ethoxycarbonyl-2-ethoxy-1,2-dihydroquinoline), Polyphosphoric Acid (PPA), DPPA (Diphenyl phosphoryl azide), HATU (1-[Bis(dimethylamino)methylene]-1H-1,2,3-triazolo[4,5-b]pyridinium 3-oxide hexafluorophosphate), HBTU (0-Benzotriazol-1-yl-N,N,N',N'-tetramethyluronium hexafluorophosphate), HOBt (1-Hydroxybenzotriazole), HOAt (1-Hydroxy-7-azabenzotriazole), DCC (*N,N'*-Dicyclohexylcarbodiimide), EDC (or EDAC or EDCI, 1-Ethyl-3-(3-dimethylaminopropyl)carbodiimide), BOP-CI (Bis(2-oxo-3-oxazolidinyl)phosphinic chloride), TFFH (Tetramethylfluoroformamidinium hexafluorophosphate), BroP (Bromo tris(dimethylamino) phosphonium hexafluorophosphate), PyBroP (Bromo-tris-pyrrolidino-phosphonium hexafluorophosphate) and CIP (2-Chloro-1,3-dimethylimidazolidinium hexafluorophosphate), or further, similar acting reagents, providing an activated intermediate, or a mixture thereof.

### Pharmaceutical Composition & Medical Use

Therefore another aspect of the present invention relates to compounds according to the general formula (**I**) as medicine as well as their use in medicine. Especially preferred is the use as inhibitors of transglutaminases, in particular transglutaminase 2 (TG2).

Thus the compounds of formula (I) described herein or according to the present invention may be administered themselves or in form of a pharmacologically acceptable salt.

The compounds of the present invention may form of a pharmacologically acceptable salt with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, trifluoroacetic acid, and other mineral or carboxylic acids well known to those skilled in the art. The salts are prepared by contacting the free base form with a sufficient amount of the desired acid to produce a salt in the conventional manner. Preferred is the mesylate salt, hydrochloride salt and the trifluoroacetate salt and especially preferred is the trifluoroacetate salt and the hydrochloride salt.

In the case the inventive compounds bear acidic groups, salts could also be formed with inorganic or organic bases. Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

### Methods of Use

In a further aspect of the present invention, the novel compounds according to the general formula (I) are used as pharmaceutically active agent, i.e. the compound of the formula (I) is used in medicine.

Furthermore, the present invention relates to a pharmaceutical composition comprising at least one compound according to the general formula (I), as an active ingredient or a pharmacologically acceptable salts thereof as an active ingredient, together with at least one pharmacologically acceptable carrier, excipient and/or diluent.

The compounds according to general formula (I) described herein are especially suitable for the treatment and prophylaxis of diseases associated with and/or caused by transglutaminase 2.

Celiac disease, a gluten intolerance is associated with tissue transglutaminase (TG 2). Another very important group of indications for tissue transglutaminase inhibitors are fibrotic disorders. Fibrotic disorders are characterized by the accumulation of crosslinked extracellular matrix proteins. Diabetic nephropathy, cystic fibrosis, idiopathic pulmonary fibrosis, kidney fibrosis as well as liver fibrosis belong to the most important fibrotic disorders to be addressed with the compounds disclosed.

In the biological example B-1, it is proven that the inventive compounds as reversible and irreversible TG inhibitors effectively inhibit the activity of TGs, especially TG2.

As used herein the term "inhibiting" or "inhibition" refers to the ability of a compound to downregulate, decrease, reduce, suppress, inactivate, or inhibit at least partially the activity of an enzyme, or the expression of an enzyme or protein.

Therefore, another aspect of the present invention is the use of the inventive compounds of the general formula (I), or the pharmaceutical composition thereof as described in the treatment or prophylaxis of autoimmune and inflammatory diseases, vascular diseases, fibrotic diseases, liver diseases, cholestatic liver diseases, cancer, neurodegenerative diseases, ocular diseases, and skin disorders.

Further aspects of the present invention relate to the use of the compounds of general formula (I) for the preparation of a pharmaceutical composition useful for prophylaxis and/or treatment of autoimmune and inflammatory diseases, vascular diseases, fibrotic diseases, liver diseases, cholestatic liver diseases, cancer, neurodegenerative diseases, ocular diseases, and skin disorders.

In a further aspect of the present invention, a method for preventing and/or treating autoimmune and inflammatory diseases, vascular diseases, fibrotic diseases, liver diseases, cholestatic liver diseases, cancer, neurodegenerative diseases, ocular diseases, and skin disorders, which method comprises administering to a subject, in particular a human, a pharmaceutically effective amount of at least one compound of the general formula (I), to prevent and/or treat said autoimmune and inflammatory diseases, vascular diseases, fibrotic diseases, liver diseases, cholestatic liver diseases, cancer, neurodegenerative diseases, ocular diseases, and skin disorders.

Preferred, the autoimmune and inflammatory diseases comprises multiple sclerosis, celiac disease, Duhring-Brocq-disease (dermatitis herpetiformis), gluten ataxia, gluten neuropathy, diabetes, rheumatoid arthritis, Graves' disease, inflammatory bowel disease, systemic lupus erythematosus psoriasis, and gingivitis;
the vascular diseases comprise atherosclerosis, thrombosis, vascular stiffness;
the fibrotic diseases affecting the lung, the kidney, the liver, the skin or the gut like cystic fibrosis, kidney fibrosis and diabetic nephropathy, intestinal fibrosis, idiopathic lung fibrosis, liver fibrosis;
the liver diseases like alcoholic hepatitis, alcoholic steatohepatitis, nonalcoholic steatohepatitis, non-alcoholic fatty liver disease, liver cirrhosis, autoimmune hepatitis or liver inflammation;
the cholestatic liver diseases comprise primary biliary cholangitis and primary sclerosing cholangitis;
the cancer comprises glioblastoma, melanoma, pancreatic cancer, renal cell carcinoma, meningioma, and breast cancer,
the neurodegenerative diseases comprise Parkinson's disease, Huntington's disease, or Alzheimer's disease,
the ocular diseases comprise glaucoma, cataracts, macular degeneration, or uveitis; the skin disorders comprise acne, psoriasis, scarring, and skin aging.

More preferred, the compound of the formula (I), or the pharmaceutical composition thereof is useful in the treatment or prophylaxis of celiac disease.

Furthermore, the compounds of the general formula (I), can be administered in form of their pharmaceutically active salts, optionally using essentially non-toxic pharmaceutically acceptable carriers, adjuvants or extenders. Medications are prepared in a known manner in a conventional solid or fluid carrier or in extenders and a conventional pharmaceutically acceptable adjuvant/expedient in a suitable dose. The preferred preparations are provided in an administrable form suitable for oral application, such as pills, tablets, film tablets, coated tablets, capsules and powders.

Tablets, film tablets, coated tablets, gelatine capsules and opaque capsules are the preferred pharmaceutical formulations. Any pharmaceutical compositions contains at least one compound of the general formula (I), and/or pharmaceutically acceptable salts thereof in an amount of 5 mg to 500 mg, preferably 10 mg to 250 mg and most preferred in an amount of 10 to 100 mg per formulation.

Besides, the object of the present invention also includes pharmaceutical preparations for oral, parenteral, dermal, intradermal, intragastric, intracutaneous, intravascular, intravenous, intramuscular, intraperitoneal, intranasal, intravaginal, intrabuccal, percutaneous, rectal, subcutaneous, sublingual, topic, transdermal or inhalative application, containing, in addition to typical vehicles and extenders, a compound of the general formula (I), and/or a pharmaceutically acceptable salt thereof as active component.

The pharmaceutical compositions of the present invention contain one of the compounds of the formula (I) disclosed herein as active component, typically mixed with suitable carrier materials, selected with respect to the intended form of administration, i.e. tablets to be administered orally, capsules (filled either with a solid, a semi-solid or a liquid), powders, orally administrable gels, elixirs, dispersible granulates, syrups, suspensions and the like in accordance with conventional pharmaceutical practices. For example, the compound of the formula (I) can as active agent component be combined with any oral, non-toxic, pharmaceutically acceptable, inert carrier, such as lactose, starch, sucrose, cellulose, magnesium stearate, dicalcium phosphate, calcium sulfate, talc, mannitol, ethyl alcohol (liquid forms) and the like for the oral administration in form of tablets or capsules. Moreover, suitable binders, lubricants, disintegrants and colorants can be added to the mixture if required. Powders and tablets can consist of said inert carriers to an extent from about 5% per weight to about 95% per weight of the inventive composition.

Suitable binders include starch, gelatine, natural sugars, sweeteners made of corn, natural and synthetic gums, such as acacia gum, sodium alginate, carboxymethylcellulose, polyethylene glycol and waxes. Possible lubricants for the use in said dosage forms include boric acid, sodium benzoate, sodium acetate, sodium chloride and the like. Disintegrants include starch, methylcellulose, cyclodextrins, guar gum and the like. If required, sweeteners and flavor additives and preservatives can also be included. Some of the terms used above, namely disintegrants, extenders, lubricants, binders and the like are discussed in greater detail below.

Additionally, the compositions of the present invention can be formulated in a form with sustained release to provide a controlled release rate of any one or more components or active components, in order to optimize the therapeutic effect, i.e. the inhibitory activity and the like. Suitable dosage forms for sustained release include layered tablets containing layers with varying degradation rates or controlled release polymeric matrices impregnated with the active components and in the form of a tablet or capsule containing such impregnated or encapsulated porous polymeric matrices.

Preparations in fluid form include solutions, suspensions and emulsions. Exemplarily mentioned are water or water propylene glycol solutions for parenteral injections or the addition of sweeteners and opacifiers for oral solutions, suspensions, and emulsions.

Aerosol preparations suitable for inhalation may include solutions and solids in the form of powders which can be combined with a pharmaceutically acceptable carrier, such as a compressed inert gas, e.g. nitrogen.

For the preparation of suppositories a low melting wax, such as a mixture of fatty acid glycerides, e.g. cocoa butter, is melted firstly and the active component is homogenously dispersed therein by stirring or similar mixing operations. The melted homogenous mixture is then poured in fitting forms, cooled and thus hardened.

Further preparations in solid form which are to be converted into preparations in fluid form for either oral or parenteral administration shortly before use are included. Such fluid forms include solutions, suspensions and emulsions.

Furthermore, the compounds of the present invention may be administered via transdermal application. The transdermal compositions can have the form of cremes, lotions, aerosols and/or emulsions.

The term capsule refers to a special container or casing composed of methylcellulose, polyvinyl alcohols or denatured gelatins or starches, in which the active agents can be enclosed. Typically, hard shell capsules are prepared from mixtures of bones and porcine skin gelatins having comparatively high gel strength. The capsule itself can contain small amounts of colorants, opacifiers, softening agents and preservatives.

Tablet means a compressed or cast solid dosage form containing the active components with suitable extenders. The tablet can be produced by compressing mixtures or granulates obtained by wet granulation, dry granulation or compaction, which are known to the one skilled in the art.

Oral gels refer to the active components dispersed or solubilized in a hydrophilic semi-solid matrix.

Powders for compositions refer to powder mixtures containing the active components and suitable extenders which can be suspended in water or juices.

Suitable extenders are substances which usually form the largest part of the composition or dosage form. Suitable extenders include sugars such as lactose, sucrose, mannitol and sorbitol; starches derived from wheat, corn, rice and potatoes; and celluloses such as microcrystalline cellulose. The amount of extenders in the composition can range from about 5 to about 95% per weight of the total composition, preferably form about 25 to about 75% per weight and further preferred from about 30 to about 60% per weight.

The term disintegrants refers to materials added to the composition in order to support disintegration and release of the medicinal substance. Suitable disintegrants include starches, modified starches which are soluble in cold water, such as sodium carboxymethyl starch; natural and synthetic gums such as locust bean gum, caraya, guar gum, tragacanth and agar; cellulose derivatives such as methylcellulose and sodium carboxymethylcellulose, microcrystalline celluloses and crosslinked microcrystalline celluloses such as croscarmellose sodium; alginates such as alginic acid and sodium alginate; clays such as bentonites and foaming mixtures. The amount of disintegrants used in the composition can range from about 2 to 20% per weight of the composition and further preferred from about 5 to about 10% per weight.

Binders characterize substances binding or "gluing" powders to each other and they consequently serve as "glue" in the formulation. Binders add a cohesion starch which is already available in the extenders or the disintegrant. Suitable binders include sugar, such as sucrose; starches derived from wheat, corn, rice and potatoes; natural gums such as acacia gum, gelatine and tragacanth; derivatives of sea weed such as alginic acid, sodium alginate and ammonium calcium alginate, cellulose materials such as methyl cellulose and sodium carboxymethylcellulose and hydroxypropyl methylcellulose, polyvinylpyrrolidone and inorganic compounds, such as magnesium aluminium silicate. The amount of binders in the composition can range from about 2 to about 20% per weight of the total composition, preferably form about 3 to about 10% per weight and further preferred from about 3 to about 6% per weight.

The term lubricant refers to a substance added to the dosage form in order to allow for the tablet, granulate, etc. to be released from the casting mold or pressing mold, after compression, by reducing the friction. Suitable lubricants include metallic stearates such as magnesium stearate, calcium stearate or potassium stearate; stearic acid; waxes with high melting points and water soluble lubricants such as sodium chloride, sodium benzoate, sodium acetate, sodium oleate, polyethylene glycols and D,L-leucine. Due to the fact that lubricants have to be present on the surface of the granulates as well as between the granulates and parts of the tablet press they are typically added during the last step prior to compression. The amount of lubricants in the composition can range from about 0.2 to about 5% per weight of the total composition, preferably form about 0.5 to about 2% per weight and further preferred from about 0.3 to about 1.5 % per weight.

Lubricants are materials preventing caking and improving the flow characteristics of granulates so that the flow is smooth and uniform. Suitable lubricants include silicon dioxide and talc. The amount of lubricants in the composition can range from about 0.1 to about 5 % per weight of the total composition, preferably form about 0.5 to about 2 % per weight.

Colorants are adjuvants coloring the composition or dosage form. Such adjuvants can include colorants having food quality which are adsorbed on a suitable adsorption means, such as clay or aluminium oxide. The amount of the colorant used can vary from about 0.1 to about 5% per weight of the composition and preferably from about 0.1 to about 1 % per weight.

As used herein, a "pharmaceutically effective amount" of a transglutaminase inhibitor is the amount or activity effective for achieving the desired physiological result, either in cells treated *in vitro* or in a patient treated *in vivo.* Specifically, a pharmaceutical effective amount is such an amount which is sufficient for inhibiting, for a certain period of time, one or more of the clinically defined pathological processes associated with transglutaminase 2. The effective amount can vary according to the specific compound of the formula (I) and additionally depends on a plurality of factors and conditions related to the subject to be treated and the severity of the disease. If, for example, an inhibitor is to be administered *in vivo,* factors such as age, weight and health of the patients as well as dose reaction curves and data regarding toxicity obtained from preclinical animal studies are amongst the data to be considered. If the inhibitor in form of the compound of the formula (I) described herein is to be brought in contact with the cells *in vivo,* a plurality of preclinical *in vitro* studies would be designed in order to determine parameters such as absorption, half-life, dose, toxicity, etc. Determining a pharmaceutically effective amount for a given pharmaceutically active ingredient is part of the ordinary skills of the one skilled in the art.

### Examples

Following abbreviations used in the examples have the following meaning.

Boc (tert-butoxycarbonyl), BocOSu (N-tert-butoxycarbonyloxy-succinimide) DCM (dichloromethane), DMAP (4-(Dimethylamino)-pyridine), TEA (triethylamine), DMF (dimethylformamide), DMP (Dess-Martin periodiane), DIPEA (N-Ethyldiisopropylamine), Glu (glutamic acid), EDC (1-ethyl-3-(3'-dimethylaminopropyl)carbodiimide), TFA (trifluoroacetic acid), THF (tetrahydrofuran), EtOAc (ethyl acetate), HATU (1-[Bis(dimethylamino)methylene]-1H-1,2,3-triazolo[4,5-b]pyridinium 3-oxid hexafluorophosphate), HOBt (hydroxybenzotriazole), MTBE (methyl tert-butyl ether), tBu (tert-butyl),

### Chemical Examples

The following examples are intended to illustrate the invention with selected compounds without limiting the protecting scope of the present intellectual property right on these concrete examples. It is clear for a person skilled in the art that analogous compounds and compounds produced according to analogous synthetic ways fall under the protecting scope of the present intellectual property right.

### Example II. Synthetic method II

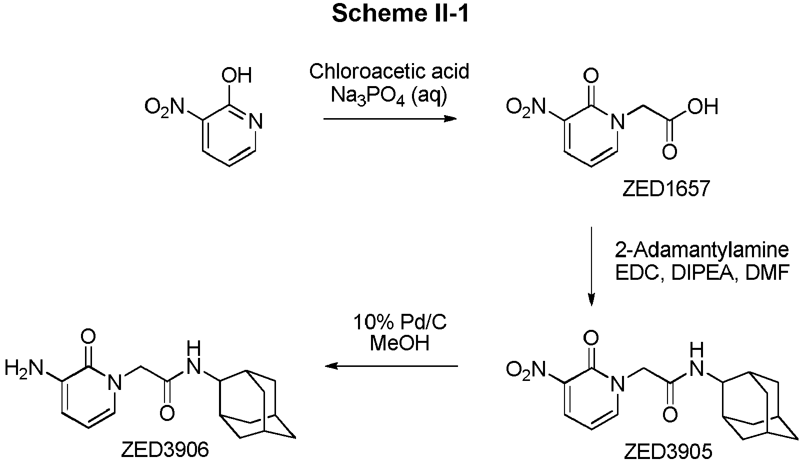

### 1 Preparation of compound ZED1657

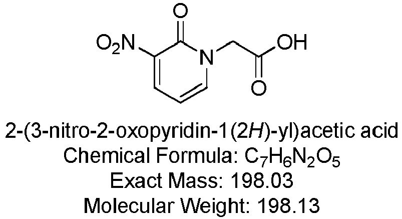

30.0 g (214 mmol) of 2-hydroxy-3-nitropyridine and 40.5 g (2 eq) of chloroacetic acid were suspended in 600 mL water. At 40°C, 245 g (3 eq) trisodium phosphate dodecahydrate were added, and the reaction was stirred at room temperature overnight. 250 mL HCl (32%) were added, and the suspension was stirred for another night at 4°C. The precipitate was filtered and dried. Yield: 41.2 g, 97% ESI-MS: 199.3 [M+H]⁺

### 2 Preparation of compound ZED3905

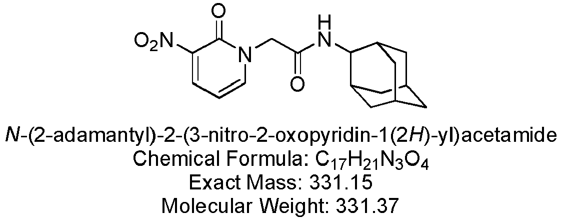

17.0 g (85.8 mmol) of ZED1657, 16.1 g (1 eq) of 2-adamantanamine hydrochloride and 11.6 g (1 eq) of HOBt were dissolved in 200 mL DMF and 17.9 mL (1.2 eq) DIPEA. 18.1 g (1.1 eq) of 1-ethyl-3-(3'-dimethylaminopropyl)carbodiimide hydrochloride were added and the reaction was stirred at room temperature overnight. The solvent was evaporated, and the residue was dissolved in 500 mL DCM. The solution was washed with each 200 mL citric acid solution (10%), NaHCO₃ solution (10%) and brine. The organic phase was dried over Na₂SO₄, filtered and the solvent was evaporated.
Yield: 24.1 g, 85% ESI-MS: 332.4 [M+H]⁺

### 3 Preparation of compound ZED3906

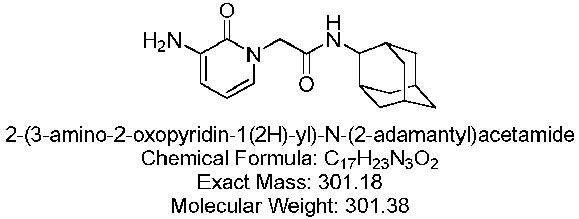

24.2 g (73.0 mmol) of ZED3905 were suspended in 600 mL MeOH before 2.42 g of palladium (10%) on activated carbon (unreduced) were added. The suspension was stirred overnight at room temperature under an atmosphere of hydrogen. The catalyst was filtered, and the solvent was evaporated. Yield: 15.7 g, 71% ESI-MS: 302.4 [M+H]⁺

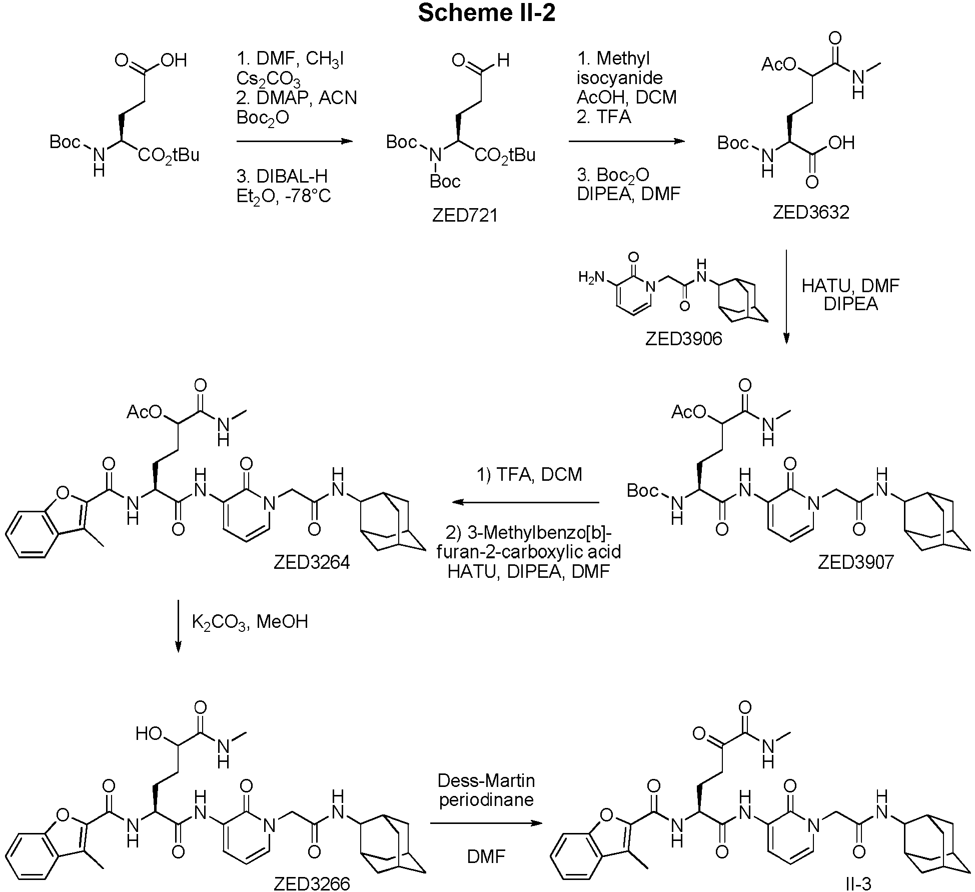

### Preparation of compound ZED788

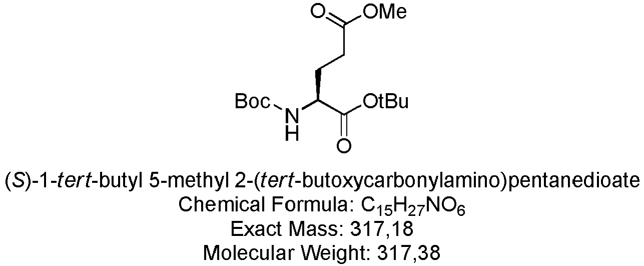

12.0 g of Boc-L-Glu-OtBu (39.6 mmol) and 7.09 g of cesium carbonate (21.8 mmol, 0.55 eq) were suspended in 100 ml of DMF and stirred for 1 h at room temperature. 2.47 ml iodomethane (39.6 mmol) we added, and the mixture was stirred at room temperature overnight. The solvent was evaporated, and the residue was dissolved in ethyl acetate and washed twice with each citric acid solution (10%), NaHCO₃ solution (10%) and brine. The organic phase was dried over Na₂SO₄, filtered and the solvent was evaporated. The raw product was used without further purification.
Yield: 13.4 g, >100%
ESI-MS: 318.3 [M+H]⁺

### Preparation of compound ZED720

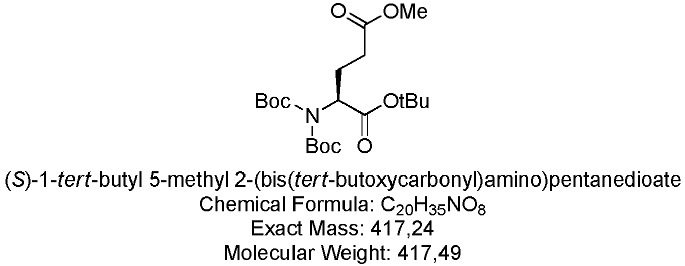

13.4 g of ZED788 (-39,6 mmol) and 986 mg of N,N-dimethyl-4-aminopyridine (DMAP) were dissolved in 30 ml of acetonitrile. 17.6 g of di-tert-butyl bicarbonate (77.1 mmol) in 100 ml of acetonitrile was added and the solution was stirred at room temperature overnight. The solvent was evaporated, and the residue was dissolved in ethyl acetate and washed twice with each citric acid solution (10%), NaHCO₃ solution (10%) and brine. The organic phase was dried over Na₂SO₄, filtered and the solvent was evaporated. The raw product was used without further purification.
Yield: 13.7 g, 83%
ESI-MS: 418.3 [M+H]⁺

### Preparation of compound ZED721

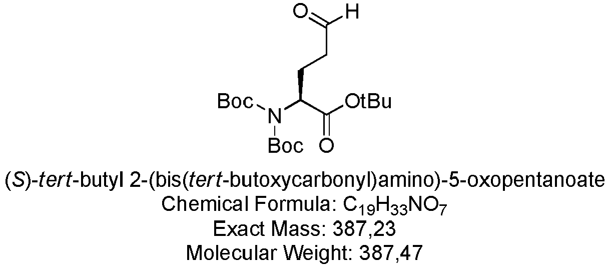

13.7 g of ZED720 (32.8 mmol) were dissolved in 200 ml of dry diethyl ether and cooled to -78°C under argon atmosphere. 36.1 ml of diisobutylaluminum hydride (1M in hexane) were added dropwise and the solution was stirred for 30 min at -78°C before being quenched with potassium sodium tartrate (Rochelle salt) solution. The organic layer was separated, dried over Na₂SO₄, filtered, and concentrated to dryness. The raw product was used without further purification.
Yield: 13.3 g, >100%
ESI-MS: 388.3 [M+H]⁺

**4 Preparation of compound ZED3632**

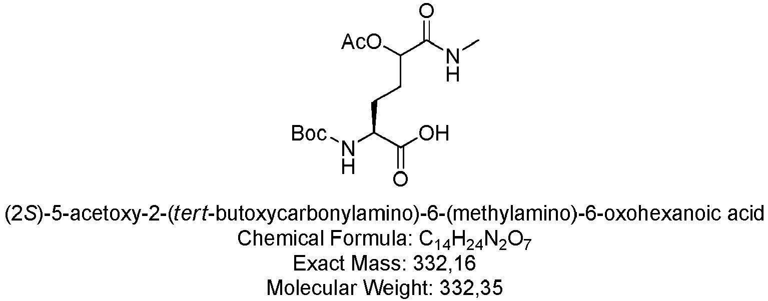

15.0 g (38.7 mmol) of the aldehyde (S)-tert-butyl 2-(bis(tert-butoxycarbonyl)amino)-5-oxopentanoate **(ZED721)** were dissolved in 60 mL DCM. At 0°C 2.42 mL (1.05 eq) methyl isocyanide and 2.33 mL (1.05 eq) acetic acid were added, and the reaction was stirred at room temperature overnight. 75 mL TFA were added, and the reaction was stirred for another 3 h. The solvent was evaporated, and the residue was dissolved in 40 mL DMF. 13.2 mL (2 eq) DIPEA and 10.4 g (46.6 mmol) di-tert-butyl dicarbonate in 10 mL DMF were added and the reaction was stirred at room temperature overnight. The solvent was evaporated, and the residue was dissolved in DCM. After extraction with NaHCO₃ solution (1.05 eq in water), 1.5 eq citric acid was added to the aqueous phase, followed by re-extraction with DCM. The organic phase was dried over Na₂SO₄, filtered and the solvent was evaporated. The residue was purified by flash chromatography.
Yield: 12.5 g, 95%
ESI-MS: 333.5 [M+H]⁺

### 5 Preparation of compound ZED3907

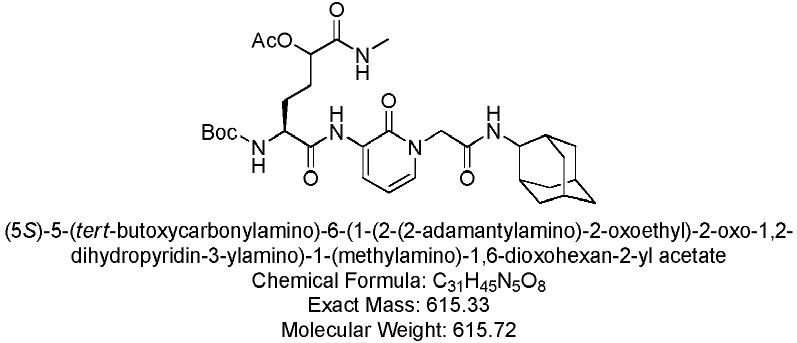

19.8 g (59.5 mmol) of ZED3632, 22.6 g (1 eq) HATU and 17.9 g (1 eq) ZED3906 were dissolved in 400 mL DMF and 20.8 mL DIPEA (2 eq) and stirred at 45°C overnight. The solvent was evaporated; the residue was dissolved in 200 mL EtOAc and washed twice with each 150 mL citric acid solution (10%), NaHCO₃ solution (10%) and brine. The organic phase was dried over Na₂SO₄, filtered and the solvent was evaporated.
Yield: 27.4 g, 75%
ESI-MS: 616.4 [M+H]⁺

### 6 Preparation of compound ZED3264

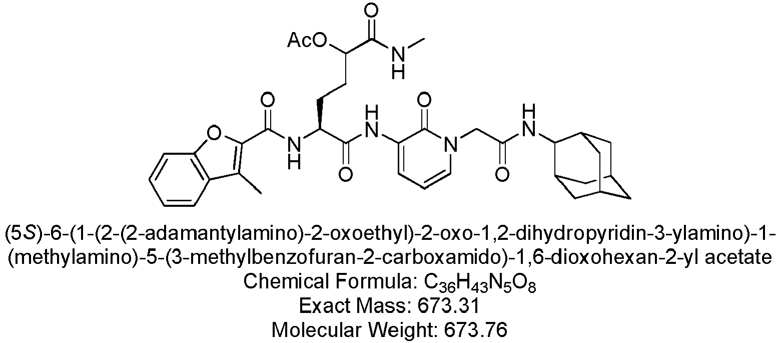

480 mg (0.78 mmol) of **ZED3907** were dissolved in 4 ml DCM/TFA (1:1) and stirred at room temperature for 1 h. The solvent was evaporated, and the residue was dissolved in 4 ml DMF. 137 mg (1 eq) 3-methylbenzo[b]furan-2-carboxylic acid, 296 mg (1 eq) HATU and 272 µl (2 eq) DIPEA were added, and the reaction was stirred at room temperature overnight. The solvent was evaporated; the residue was dissolved in 20 mL EtOAc and washed with each 10 mL citric acid solution (10%), NaHCO₃ solution (10%) and brine. The organic phase was dried over Na₂SO₄, filtered and the solvent was evaporated.
Yield: 409 mg, 78%
ESI-MS: 674.4 [M+H]⁺

### 7 Preparation of compound ZED3266

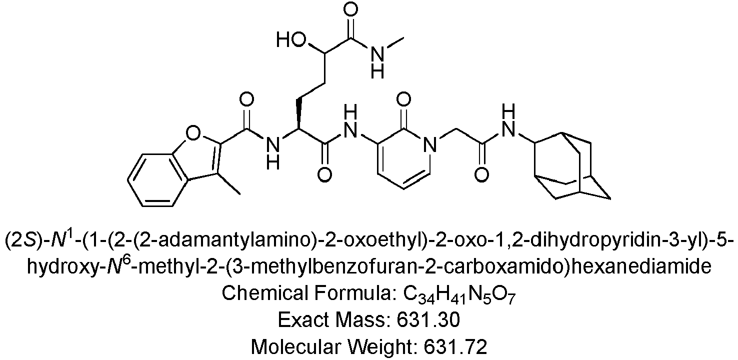

409 mg (0.61 mmol) of ZED3264 were dissolved in 5 ml MeOH. 126 mg (1.5 eq) potassium carbonate were added, and the reaction was stirred at room temperature for 1 h. The solution was diluted with DCM and washed with water. The organic phase was dried over Na₂SO₄, filtered and the solvent was evaporated.
Yield: 377 mg, 98%
ESI-MS: 632.4 [M+H]⁺

### 8 Preparation of compound II-3

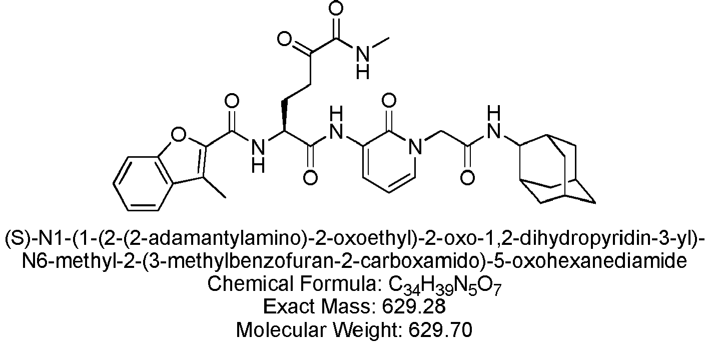

377 mg (0.60 mmol) of ZED3266 were dissolved in 2 ml DMF. 405 mg (1.6 eq) Dess-Martin periodinane (DMP) were added and the reaction was stirred at room temperature over 2 h. The precipitate was filtered off and the filtrate was evaporated. The residue was purified by HPLC.
Yield: 314 mg, 67%
ESI-MS: 630.4 [M+H]⁺
¹H-NMR (DMSO-D₆, 500 MHz, δ [ppm]: 1.46 // 1.98 (d // d, 2H // 2H, adamantyl-C4-H₂), 1.68 // 1.78 (m, 4H, adamantyl-C4-H₂), 1.71 (m, 2H, adamantyl-C1-H), 1.75 (m, 2H, adamantyl-C6-H₂), 1.78 (m, 2H, adamantyl-C5-H), 2.05 // 2.16 (m // m, 1H // 1H, β-CH₂), 2.53 (s, 3H, benzofuran-CH3), 2.64 (d, 3H, amide-N-CH₃), 2.96 (t, 2H, γ-CH₂), 3.82 (m, 1H, adamantyl-C2-H), 4.64 (s, 2H, N-CH₂), 4.70 (ddd, 1H, α-CH₂), 6.25 (t, 1H, pyridinone-C5-H), 7.33 (d, 1H, pyridinone-C6-H), 7.36 (t, 1H, benzofuran-CH), 7.51 (t, 1H, benzofuran-CH), 7.63 (d, 1H, benzofuran-CH), 7.76 (d, 1H, benzofuran-CH), 8.06 (d, 1H, adamantyl-NH), 8.21 (d, 1H, pyridinone-C4-H), 8.54 (q, 1H, methylamide-NH), 8.87 (d, 1H, α-NH), 9.36 (s, 1H, pyridinone-NH).
¹³C-NMR (DMSO-D6, 500 MHz, δ [ppm]: 8.62 (benzofuran-CH3), 24.50 (β-CH₂), 25.37 (amide-N-CH₃), 26.57 // 26.62 (adamantyl-C5-H), 30.83 (adamantyl-C4-H₂), 31.35 (adamantyl-C1-H), 33.61 (γ-CH₂), 36.66 (adamantyl-C4'-H₂), 37.01 (adamantyl-C6-H₂), 51.64 (N-CH₂), 52.80 (α-CH₂), 53.24 (adamantyl-C2-H), 104.51 (pyridinone-C5-H), 111.55 (benzofuran-CH), 121.09 (benzofuran-CH), 121.72 (benzofuran-Cq), 122.53 (pyridinone-C4-H), 123.19 (benzofuran-CH), 127.28 (pyridinone-N-Cq), 127.89 (benzofuran-CH), 129.02 (benzofuran-Cq), 133.27 (pyridinone-C6-H), 142.31 (benzofuran-Cq), 152.68 (benzofuran-Cq), 156.55 (pyridinone-C=O), 159.59 (benzofuran-C=O), 161.32 (C=O-NH-CH₃), 165.65 (C=O-adamantylamide), 170.42 (C=O-NH-pyridinone), 198.06 (C=O-methylamide).

### 9 Preparation of compound II-2

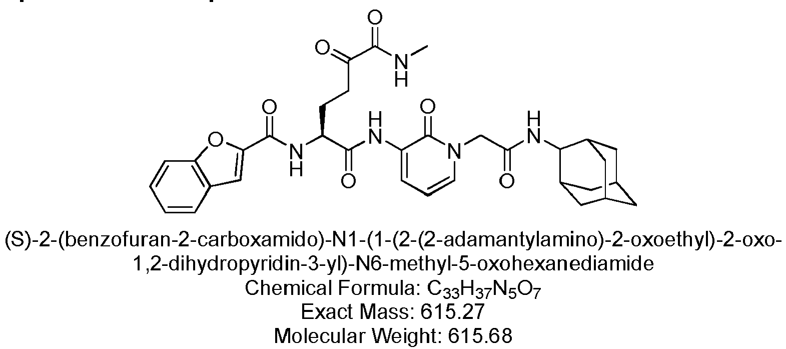

To the α-hydroxyester precursor of compound II-2 (242 mg, 0.39 mmol, prepared by using benzofuran-2-carboxylic acid in step 6 according to compound ZED3264) in 8 mL of acetonitrile, 1 mg of TEMPO (2,2,6,6-tetramethylpiperidine-1-oxyl, 1 mol%) were added. 56 mg of calcium hypochlorite (1 eq) were added at 0°C and the reaction mixture was stirred at 25°C for 2 h. The suspension was filtered, diluted with ethyl acetate and washed with NaHCO₃ solution (10%) and brine. The organic phase was dried over Na₂SO₄, filtered and the solvent was evaporated. The residue was purified by HPLC.
Yield: 102 mg, 42%
ESI-MS: 616.3 [M+H]⁺

### 10 Preparation of compound II-4

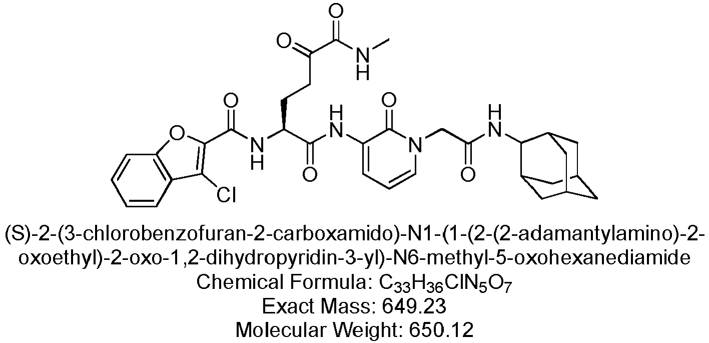

To the α-hydroxyester precursor of compound II-4 (124 mg, 0.19 mmol, prepared by using 3-chlorobenzofuran-2-carboxylic acid in step 6 according to compound ZED3264) in 4 ml DMSO, 106 mg of 2-iodoxybenzoic acid (IBX, 2 eq) were added and the reaction mixture was stirred at room temperature for 3 h. NaHCO₃ solution (10%) was added and the suspension was extracted with EtOAc. The organic phase was dried over Na₂SO₄, filtered and the solvent was evaporated. The residue was purified by HPLC.
Yield: 37 mg, 30% (last step)
ESI-MS: 650.3 / 652.3 [M+H]⁺

### 11 Preparation of compound II-5

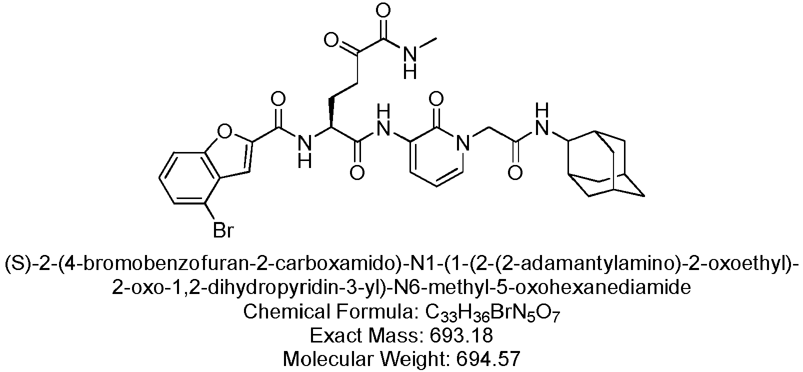

The synthesis of compound **II-5** was performed according to compound **II-3,** using 4-bromo-1-benzofuran-2-carboxylic acid instead of 3-methylbenzo[b]furan-2-carboxylic acid in step 6 (according to ZED3264).
Yield: 69 mg, 72% (last step)
ESI-MS: 694.3 / 696.3 [M+H]⁺

### 12 Preparation of compound II-6

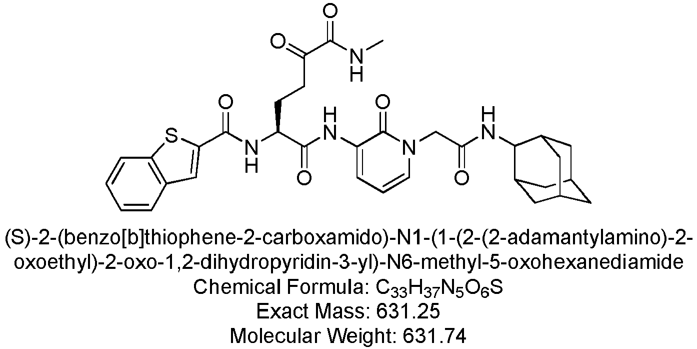

The synthesis of compound **II-6** was performed according to compound **II-3,** using benzo[b]thiophene-2-carboxylic acid instead of 3-methylbenzo[b]furan-2-carboxylic acid in step 6 (according to ZED3264).
Yield: 287 mg, 76% (last step)
ESI-MS: 632.3 [M+H]⁺

### 13 Preparation of compound II-7

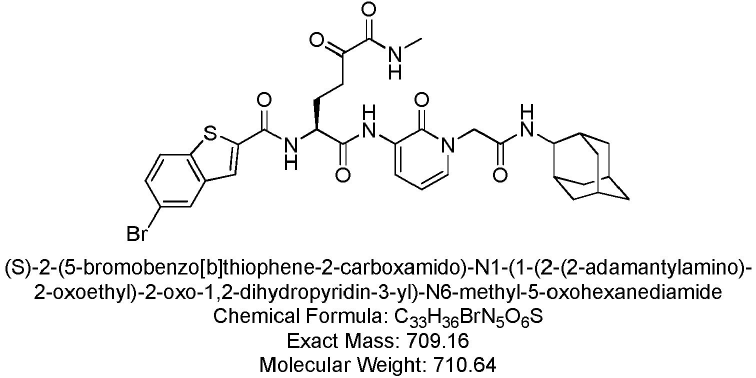

The synthesis of compound **II-7** was performed according to compound **II-3,** using 5-bromobenzo[b]thiophene-2-carboxylic acid instead of 3-methylbenzo[b]furan-2-carboxylic acid in step 6 (according to ZED3264).
Yield: 145 mg, 59% (last step)
ESI-MS: 710.2 / 712.2 [M+H]⁺

### 14 Preparation of compound II-8

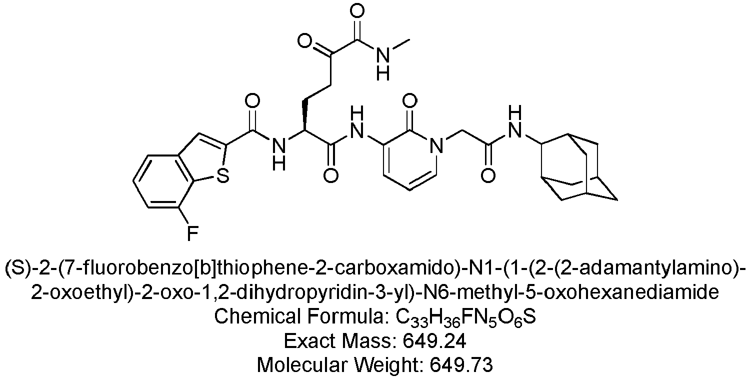

The synthesis of compound **II-8** was performed according to compound **II-3,** using 7-fluorobenzo[b]thiophene-2-carboxylic acid instead of 3-methylbenzo[b]furan-2-carboxylic acid in step 6 (according to ZED3264).
Yield: 78 mg, 71% (last step)
ESI-MS: 650.3 [M+H]⁺

### 15 Preparation of compound II-9

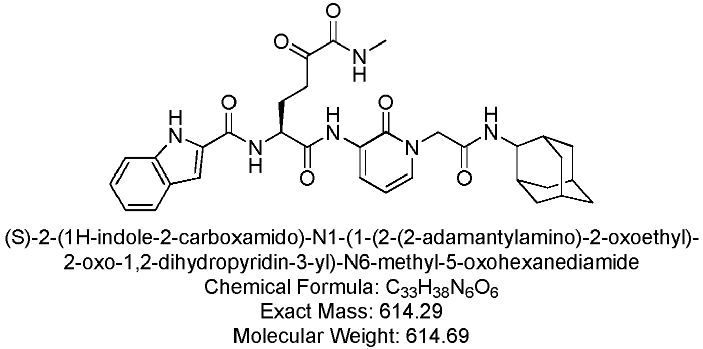

The synthesis of compound **II-9** was performed according to compound **II-3,** using 1H-indole-2-carboxylic acid instead of 3-methylbenzo[b]furan-2-carboxylic acid in step 6 (according to ZED3264).
Yield: 57 mg, 69% (last step)
ESI-MS: 615.4 [M+H]⁺

### 16 Preparation of compound II-10

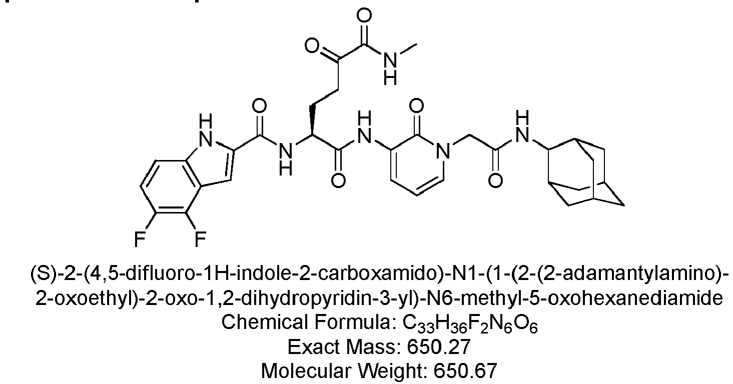

The synthesis of compound **II-10** was performed according to compound **II-3,** using 4,5-difluoro-1H-indole-2-carboxylic acid instead of 3-methylbenzo[b]furan-2-carboxylic acid in step 6 (according to ZED3264).
Yield: 47 mg, 65% (last step)
ESI-MS: 651.3 [M+H]⁺

### 17 Preparation of compound II-11

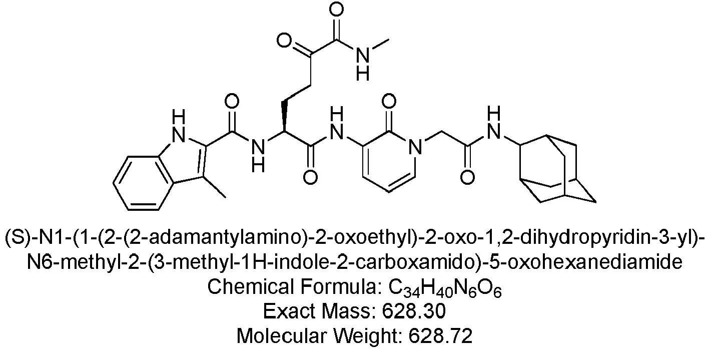

The synthesis of compound **II-11** was performed according to compound **II-3,** using 3-methyl-1H-indole-2-carboxylic acid instead of 3-methylbenzo[b]furan-2-carboxylic acid in step 6 (according to ZED3264).
Yield: 58 mg, 72% (last step)
ESI-MS: 629.4 [M+H]⁺

### 18 Preparation of compound II-12

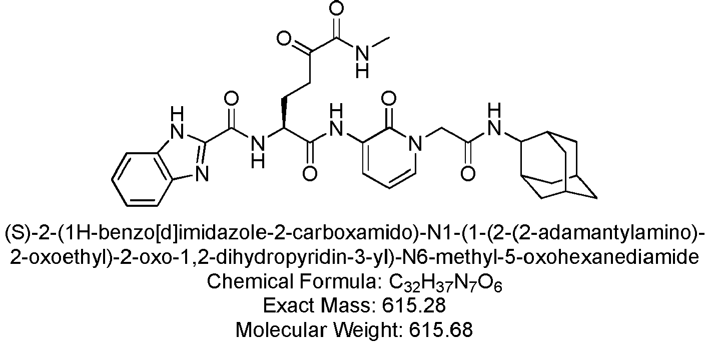

The synthesis of compound **II-12** was performed according to compound **II-3,** using 1H-benzo[d]imidazole-2-carboxylic acid instead of 3-methylbenzo[b]furan-2-carboxylic acid in step 6 (according to ZED3264).
Yield: 27 mg, 48% (last step)
ESI-MS: 616.4 [M+H]⁺

### 19 Preparation of compound II-13

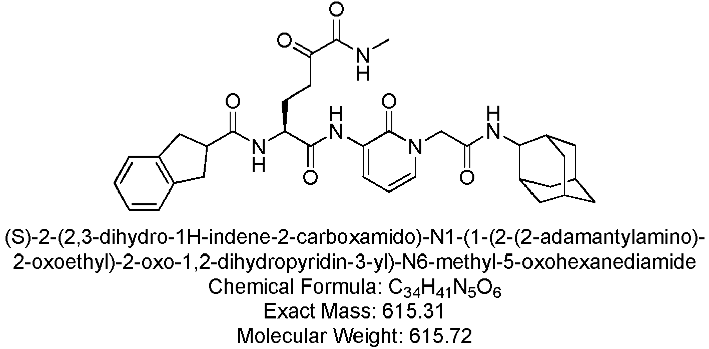

The synthesis of compound **II-13** was performed according to compound **II-3,** using 2,3-dihydro-1H-indene-2-carboxylic acid instead of 3-methylbenzo[b]furan-2-carboxylic acid in step 6 (according to ZED3264).
Yield: 38 mg, 61% (last step)
ESI-MS: 616.4 [M+H]⁺

### 20 Preparation of compound II-14

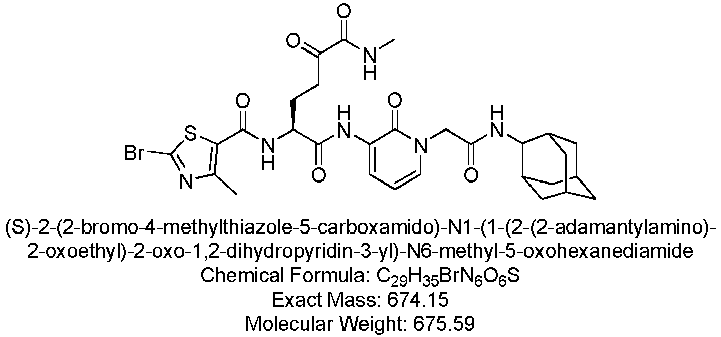

The synthesis of compound **II-14** was performed according to compound **II-3,** using 2-bromo-4-methylthiazole-5-carboxylic acid instead of 3-methylbenzo[b]furan-2-carboxylic acid in step 6 (according to ZED3264).
Yield: 98 mg, 70% (last step)
ESI-MS: 675.2 / 677.2 [M+H]⁺

### 21 Preparation of compound II-15

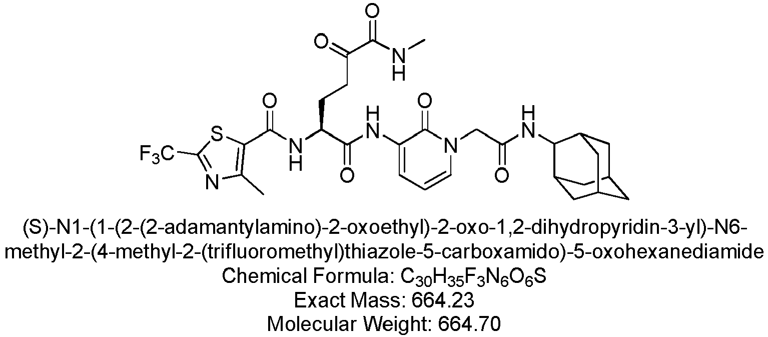

The synthesis of compound **II-15** was performed according to compound **II-3,** using 4-methyl-2-(trifluoromethyl)thiazole-5-carboxylic acid instead of 3-methylbenzo[b]furan-2-carboxylic acid in step 6 (according to ZED3264).
Yield: 67 mg, 51% (last step)
ESI-MS: 665.4 [M+H]⁺

### 22 Preparation of compound II-16

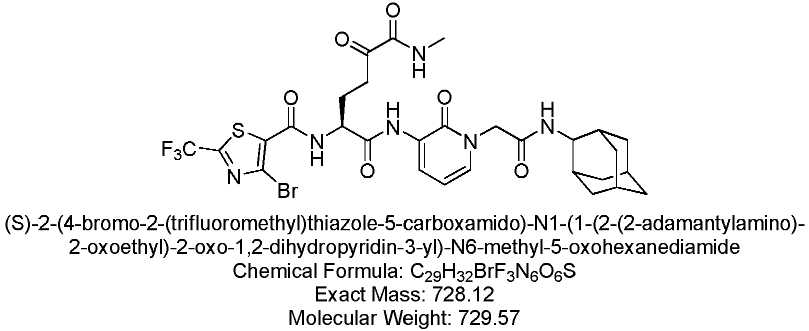

The synthesis of compound **II-16** was performed according to compound **II-3**, using 4-bromo-2-(trifluoromethyl)thiazole-5-carboxylic acid instead of 3-methylbenzo[b]furan-2-carboxylic acid in step 6 (according to ZED3264).
Yield: 136 mg, 63% (last step)
ESI-MS: 729.3/731.3 [M+H]⁺

### 23 Preparation of compound II-17

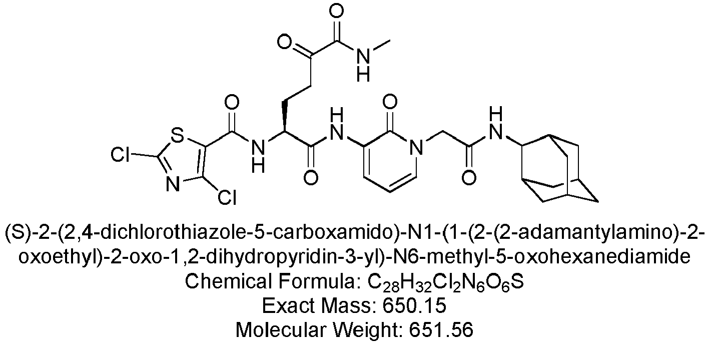

The synthesis of compound **II-17** was performed according to compound **II-3,** using 2,4-dichlorothiazole-5-carboxylic acid instead of 3-methylbenzo[b]furan-2-carboxylic acid in step 6 (according to ZED3264).
Yield: 102 mg, 71% (last step)
ESI-MS: 651.2 / 653.2 [M+H]⁺

### 24 Preparation of compound II-18

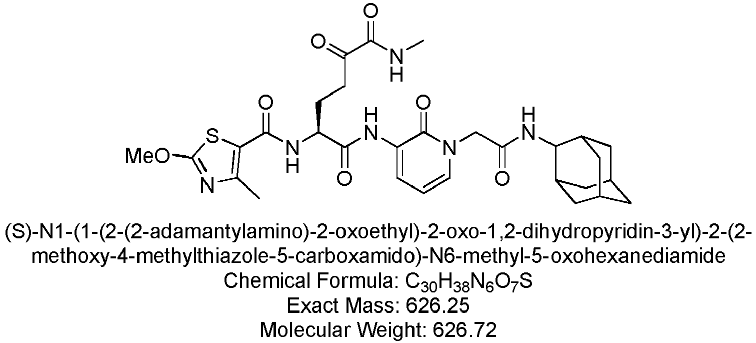

The synthesis of compound **II-18** was performed according to compound **II-3,** using 2-methoxy-4-methylthiazole-5-carboxylic acid instead of 3-methylbenzo[b]furan-2-carboxylic acid in step 6 (according to ZED3264).
Yield: 56 mg, 63% (last step)
ESI-MS: 627.3 [M+H]⁺

### 25 Preparation of compound II-19

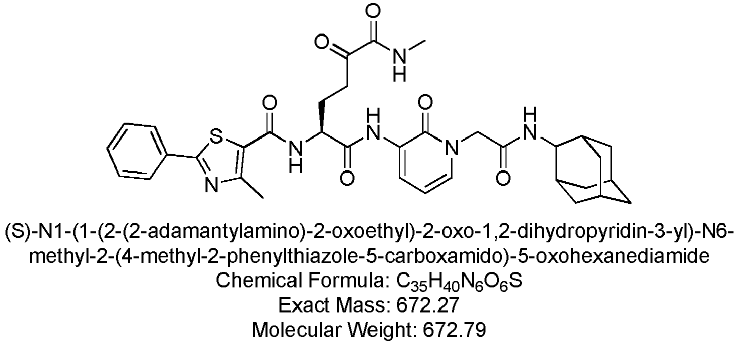

The synthesis of compound **II-19** was performed according to compound **II-3,** using 4-methyl-2-phenylthiazole-5-carboxylic acid instead of 3-methylbenzo[b]furan-2-carboxylic acid in step 6 (according to ZED3264).
Yield: 46 mg, 67% (last step)
ESI-MS: 673.4 [M+H]⁺

### 26 Preparation of compound II-20

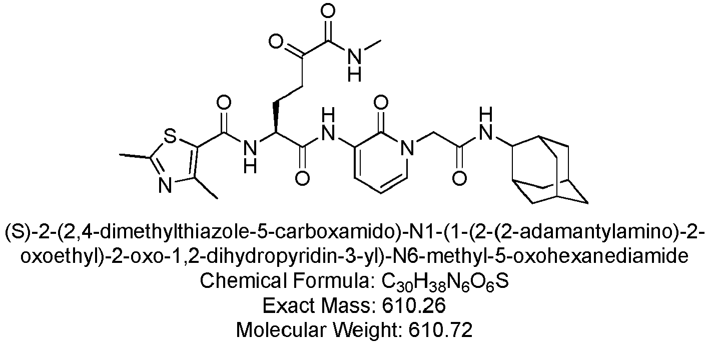

The synthesis of compound **II-20** was performed according to compound **II-3,** using 2,4-dimethylthiazole-5-carboxylic acid instead of 3-methylbenzo[b]furan-2-carboxylic acid in step 6 (according to ZED3264).
Yield: 216 mg, 77% (last step)
ESI-MS: 611.4 [M+H]⁺

### 27 Preparation of compound II-21

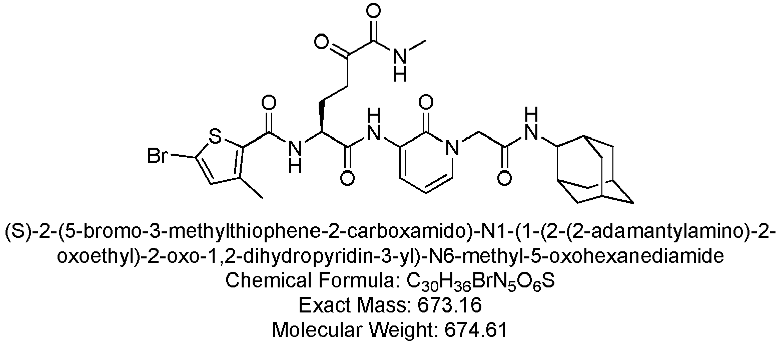

The synthesis of compound **II-21** was performed according to compound **II-3,** using 5-bromo-3-methylthiophene-2-carboxylic acid instead of 3-methylbenzo[b]furan-2-carboxylic acid in step 6 (according to ZED3264).
Yield: 178 mg, 79% (last step)
ESI-MS: 674.2 / 676.2.4 [M+H]⁺

### 28 Preparation of compound II-22

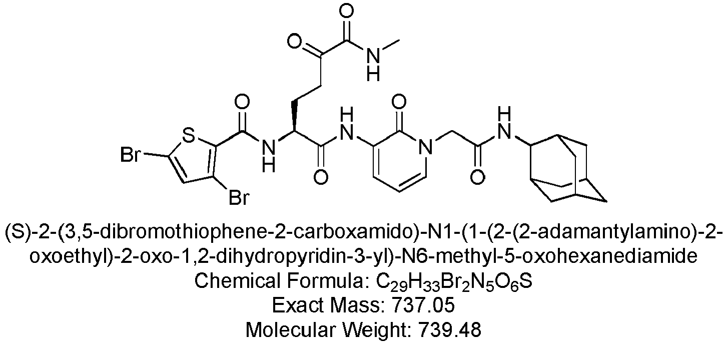

The synthesis of compound **II-22** was performed according to compound **II-3,** using 3,5-dibromothiophene-2-carboxylic acid instead of 3-methylbenzo[b]furan-2-carboxylic acid in step 6 (according to ZED3264).
Yield: 89 mg, 67% (last step)
ESI-MS: 738.2 / 740.2 / 742.2 [M+H]⁺

### 29 Preparation of compound II-23

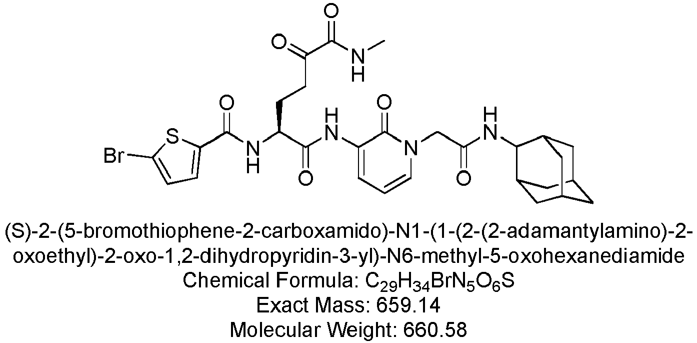

The synthesis of compound **II-23** was performed according to compound **II-3,** using 5-bromothiophene-2-carboxylic acid instead of 3-methylbenzo[b]furan-2-carboxylic acid in step 6 (according to ZED3264).
Yield: 141 mg, 72% (last step)
ESI-MS: 660.2 / 662.2 [M+H]⁺

### 30 Preparation of compound II-24

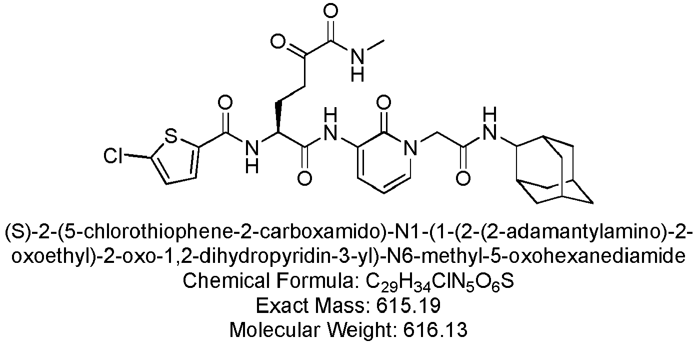

The synthesis of compound **II-24** was performed according to compound **II-3,** using 5-chlorothiophene-2-carboxylic acid instead of 3-methylbenzo[b]furan-2-carboxylic acid in step 6 (according to ZED3264).
Yield: 117 mg, 78% (last step)
ESI-MS: 616.3 / 618.3 [M+H]⁺

### 31 Preparation of compound II-25

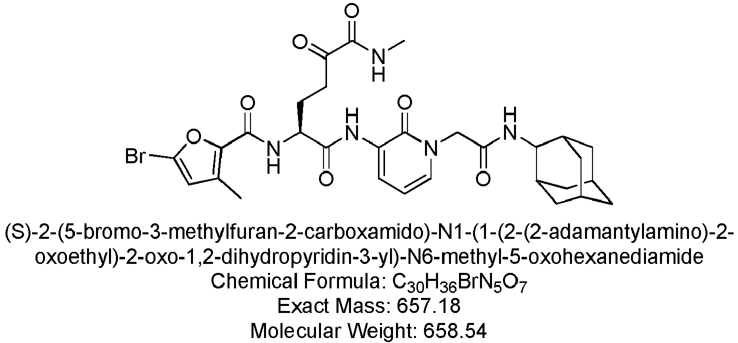

The synthesis of compound **II-25** was performed according to compound **II-3,** using 5-bromo-3-methylfuran-2-carboxylic acid instead of 3-methylbenzo[b]furan-2-carboxylic acid in step 6 (according to ZED3264).
Yield: 173 mg, 72% (last step)
ESI-MS: 658.2 / 660.2 [M+H]⁺

### 32 Preparation of compound II-26

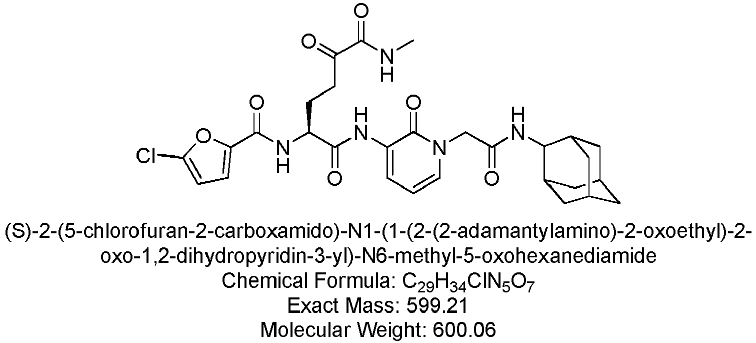

The synthesis of compound **II-26** was performed according to compound **II-3,** using 5-chlorofuran-2-carboxylic acid instead of 3-methylbenzo[b]furan-2-carboxylic acid in step 6 (according to ZED3264).
Yield: 127 mg, 56% (last step)
ESI-MS: 600.3 / 602.3 [M+H]⁺

### 33 Preparation of compound II-27

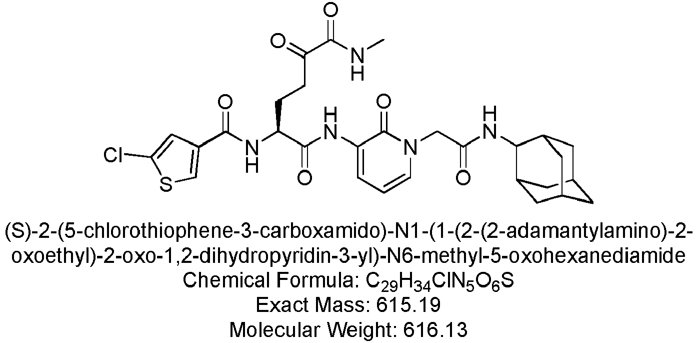

The synthesis of compound **II-27** was performed according to compound **II-3,** using 5-chlorothiophene-3-carboxylic acid instead of 3-methylbenzo[b]furan-2-carboxylic acid in step 6 (according to ZED3264).
Yield: 112 mg, 65% (last step)
ESI-MS: 616.3 / 618.3 [M+H]⁺

### 34 Preparation of compound II-28

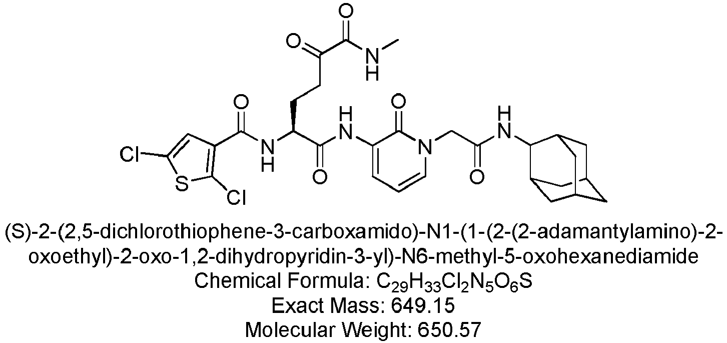

The synthesis of compound **II-28** was performed according to compound **II-3,** using 2,5-dichlorothiophene-3-carboxylic acid instead of 3-methylbenzo[b]furan-2-carboxylic acid in step 6 (according to ZED3264).
Yield: 319 mg, 77% (last step)
ESI-MS: 650.3 / 652.3 [M+H]⁺

### 35 Preparation of compound II-29

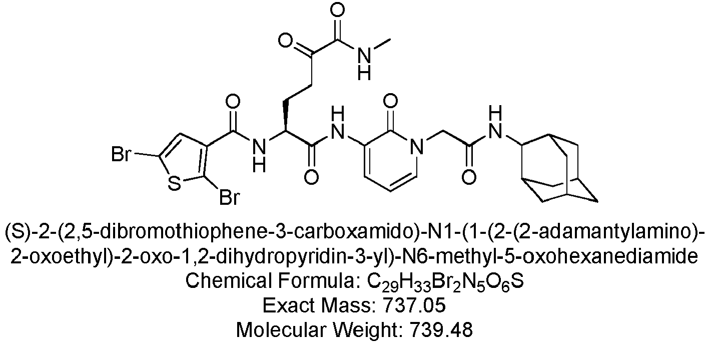

The synthesis of compound **II-29** was performed according to compound **II-3,** using 2,5-dibromothiophene-3-carboxylic acid instead of 3-methylbenzo[b]furan-2-carboxylic acid in step 6 (according to ZED3264).
Yield: 98 mg, 52% (last step)
ESI-MS: 738.2 / 740.2 / 742.2 [M+H]⁺

### 36 Preparation of compound II-30

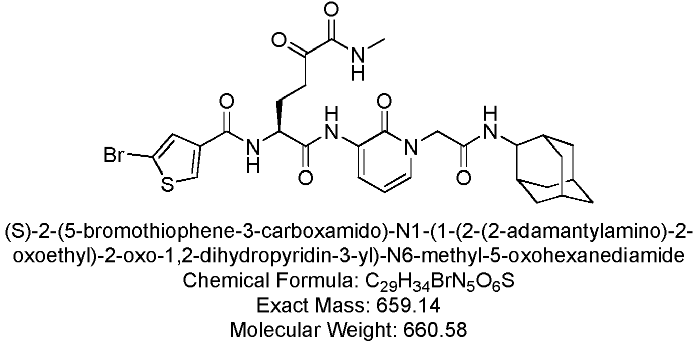

The synthesis of compound **II-30** was performed according to compound **II-3,** using 5-bromothiophene-3-carboxylic acid instead of 3-methylbenzo[b]furan-2-carboxylic acid in step 6 (according to ZED3264).
Yield: 171 mg, 73% (last step)
ESI-MS: 660.2 / 662.2 [M+H]⁺

### 37 Preparation of compound II-31

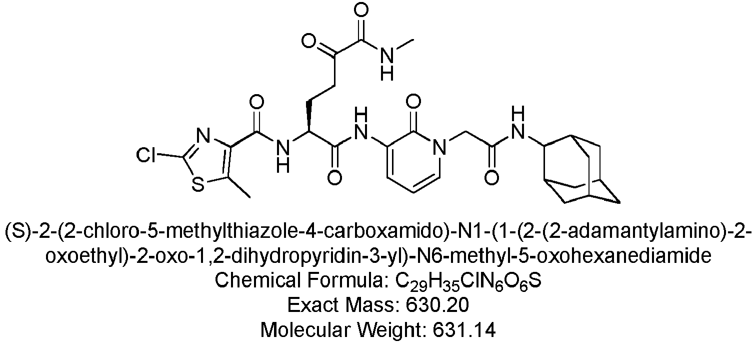

The synthesis of compound **II-31** was performed according to compound **II-3,** using 2-chloro-5-methylthiazole-4-carboxylic acid instead of 3-methylbenzo[b]furan-2-carboxylic acid in step 6 (according to ZED3264).
Yield: 32 mg, 41% (last step)
ESI-MS: 631.3 / 633.3 [M+H]⁺

### 38 Preparation of compound II-32

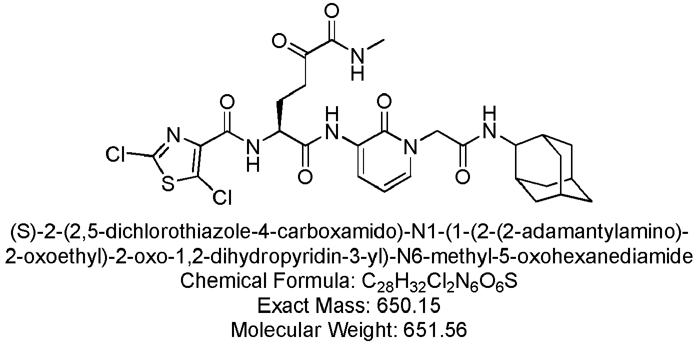

The synthesis of compound **II-32** was performed according to compound **II-3,** using 2,5-dichlorothiazole-4-carboxylic acid instead of 3-methylbenzo[b]furan-2-carboxylic acid in step 6 (according to ZED3264).
Yield: 41 mg, 35% (last step)
ESI-MS: 651.2 / 653.2 [M+H]⁺

### 39 Preparation of compound II-33

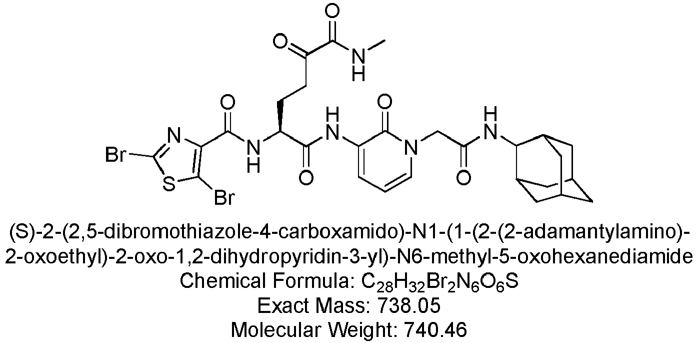

The synthesis of compound **II-33** was performed according to compound **II-3,** using 2,5-dibromothiazole-4-carboxylic acid instead of 3-methylbenzo[b]furan-2-carboxylic acid in step 6 (according to ZED3264).
Yield: 21 mg, 32% (last step)
ESI-MS: 739.2 / 741.2 / 743.2 [M+H]⁺

### 40 Preparation of compound II-34

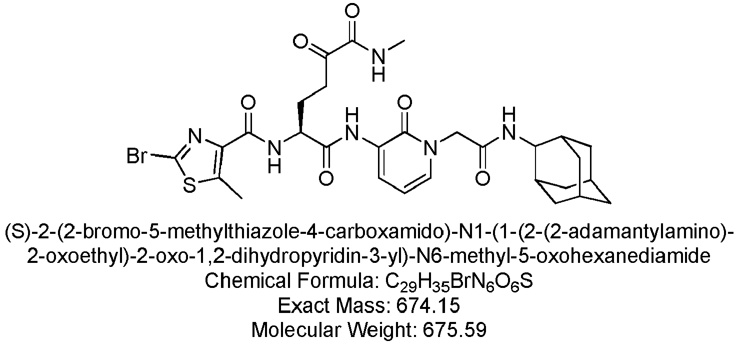

The synthesis of compound **II-34** was performed according to compound **II-3**, using 2-bromo-5-methylthiazole-4-carboxylic acid instead of 3-methylbenzo[b]furan-2-carboxylic acid in step 6 (according to ZED3264).
Yield: 42 mg, 57% (last step)
ESI-MS: 675.2 / 677.2 [M+H]⁺

### 41 Preparation of compound II-35

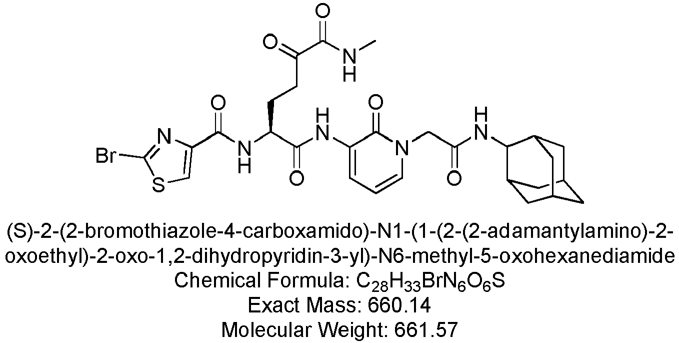

The synthesis of compound **II-35** was performed according to compound **II-3,** using 2-bromothiazole-4-carboxylic acid instead of 3-methylbenzo[b]furan-2-carboxylic acid in step 6 (according to ZED3264).
Yield: 66 mg, 46% (last step)
ESI-MS: 661.2 / 663.2 [M+H]⁺

### 42 Preparation of compound II-36

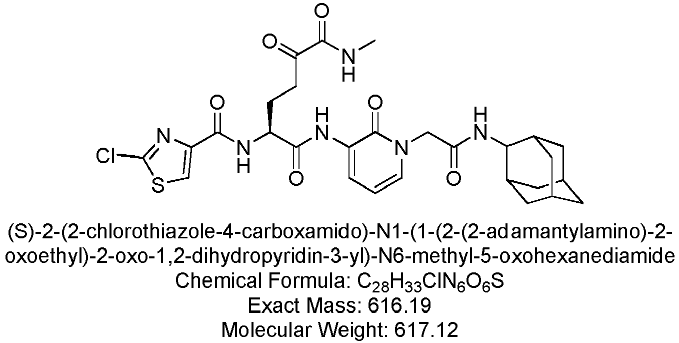

The synthesis of compound **II-36** was performed according to compound **II-3**, using 2-chlorothiazole-4-carboxylic acid instead of 3-methylbenzo[b]furan-2-carboxylic acid in step 6 (according to ZED3264).
Yield: 74 mg, 58% (last step)
ESI-MS: 617.3 / 619.3 [M+H]⁺

### 43 Preparation of compound II-37

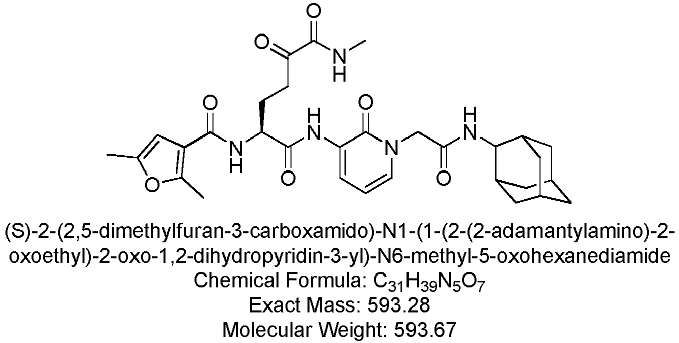

The synthesis of compound **II-37** was performed according to compound **II-3,** using 2,5-dimethylfuran-3-carboxylic acid instead of 3-methylbenzo[b]furan-2-carboxylic acid in step 6 (according to ZED3264).
Yield: 152 mg, 73% (last step)
ESI-MS: 594.4 [M+H]⁺

### 44 Preparation of compound II-38

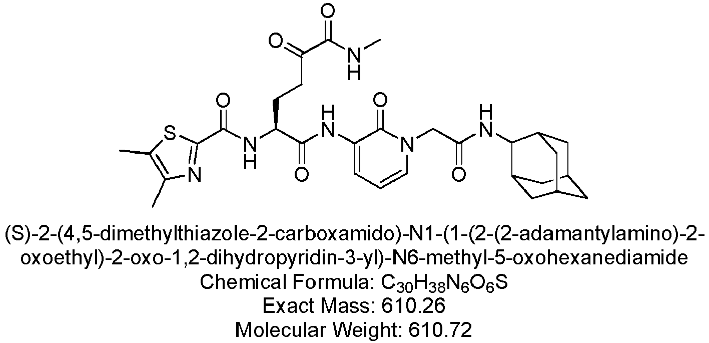

The synthesis of compound **II-38** was performed according to compound **II-3,** using 4,5-dimethylthiazole-2-carboxylic acid instead of 3-methylbenzo[b]furan-2-carboxylic acid in step 6 (according to ZED3264).
Yield: 46 mg, 38% (last step)
ESI-MS: 611.4 [M+H]⁺

### 45 Preparation of compound II-39

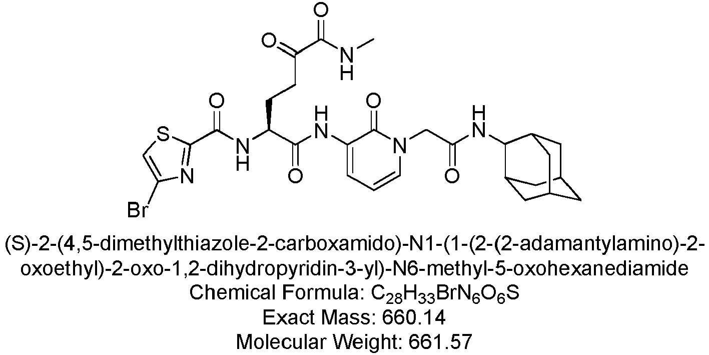

The synthesis of compound **II-39** was performed according to compound **II-3,** using 4-bromothiazole-2-carboxylic acid instead of 3-methylbenzo[b]furan-2-carboxylic acid in step 6 (according to ZED3264).
Yield: 84 mg, 68% (last step)
ESI-MS: 661.2 / 663.2 [M+H]⁺

### 46 Preparation of compound II-40

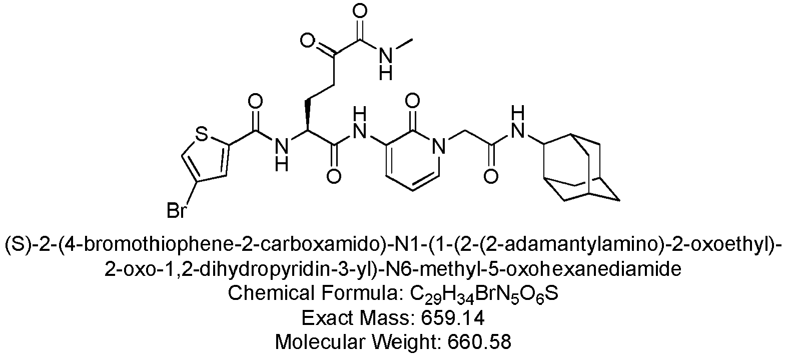

The synthesis of compound **II-40** was performed according to compound **II-3,** using 4-bromothiophene-2-carboxylic acid instead of 3-methylbenzo[b]furan-2-carboxylic acid in step 6 (according to ZED3264).
Yield: 163 mg, 73% (last step)
ESI-MS: 660.2 / 662.2 [M+H]⁺

### 47 Preparation of compound II-41

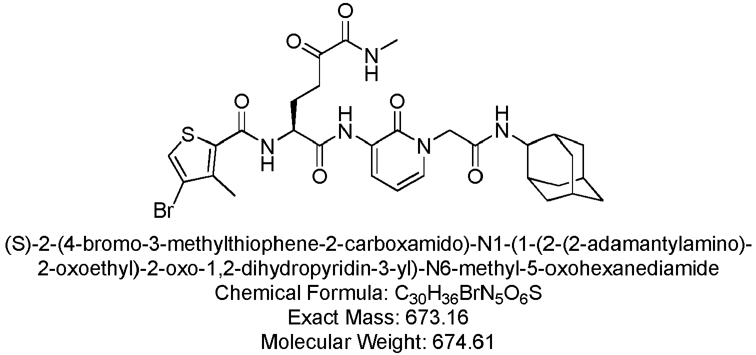

The synthesis of compound **II-41** was performed according to compound **II-3,** using 4-bromo-3-methylthiophene-2-carboxylic acid instead of 3-methylbenzo[b]furan-2-carboxylic acid in step 6 (according to ZED3264).
Yield: 114 mg, 68% (last step)
ESI-MS: 674.2 / 676.2 [M+H]⁺

### 48 Preparation of compound II-42

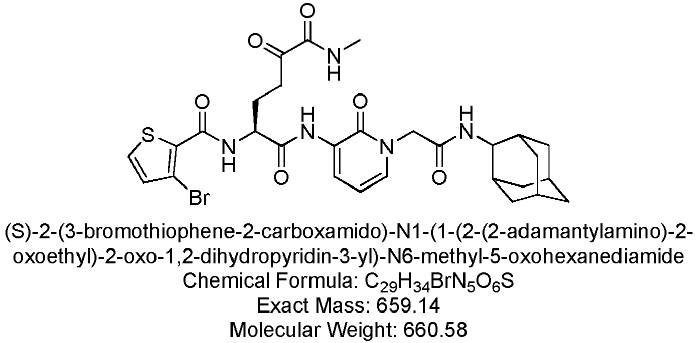

The synthesis of compound **II-42** was performed according to compound **II-3,** using 3-bromothiophene-2-carboxylic acid instead of 3-methylbenzo[b]furan-2-carboxylic acid in step 6 (according to ZED3264).
Yield: 149 mg, 76% (last step)
ESI-MS: 660.2 / 662.2 [M+H]⁺

### 49 Preparation of compound II-43

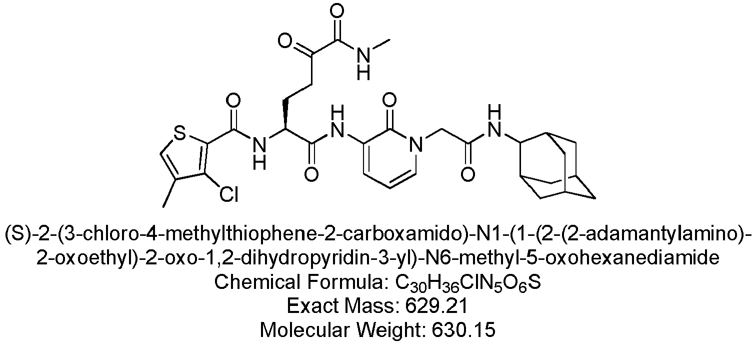

The synthesis of compound **II-43** was performed according to compound **II-3,** using 3-chloro-4-methylthiophene-2-carboxylic acid instead of 3-methylbenzo[b]furan-2-carboxylic acid in step 6 (according to ZED3264).
Yield: 110 mg, 64% (last step)
ESI-MS: 630.3 / 632.3 [M+H]⁺

### 50 Preparation of compound II-44

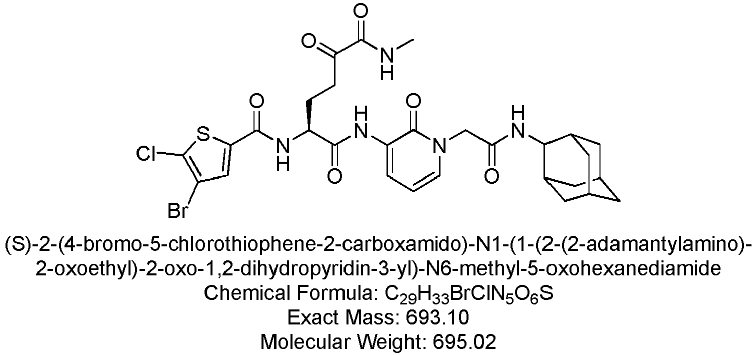

The synthesis of compound **II-44** was performed according to compound **II-3,** using 4-bromo-5-chlorothiophene-2-carboxylic acid instead of 3-methylbenzo[b]furan-2-carboxylic acid in step 6 (according to ZED3264).
Yield: 126 mg, 59% (last step)
ESI-MS: 694.2 / 696.2 / 698.2 [M+H]⁺

### 51 Preparation of compound II-45

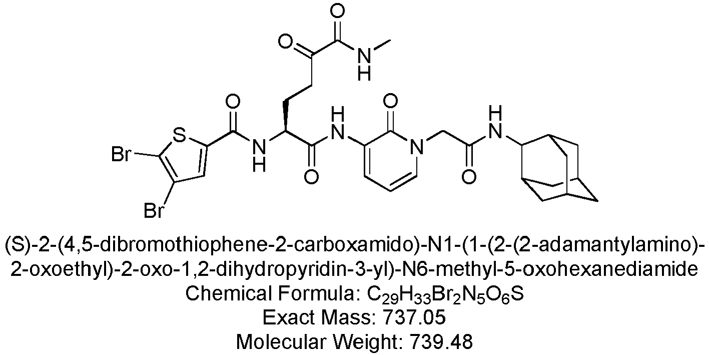

The synthesis of compound **II-45** was performed according to compound **II-3,** using 4,5-dibromothiophene-2-carboxylic acid instead of 3-methylbenzo[b]furan-2-carboxylic acid in step 6 (according to ZED3264).
Yield: 89 mg, 51% (last step)
ESI-MS: 738.2 / 740.2 / 742.2 [M+H]⁺

### 52 Preparation of compound II-46

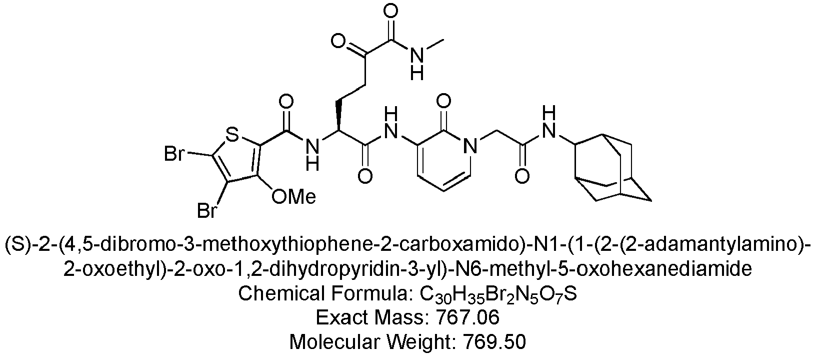

The synthesis of compound **II-46** was performed according to compound **II-3,** using 4,5-dibromo-3-methoxythiophene-2-carboxylic acid instead of 3-methylbenzo[b]furan-2-carboxylic acid in step 6 (according to ZED3264).
Yield: 59 mg, 40% (last step)
ESI-MS: 768.2 / 770.2 / 772.2 [M+H]⁺

### 53 Preparation of compound II-47

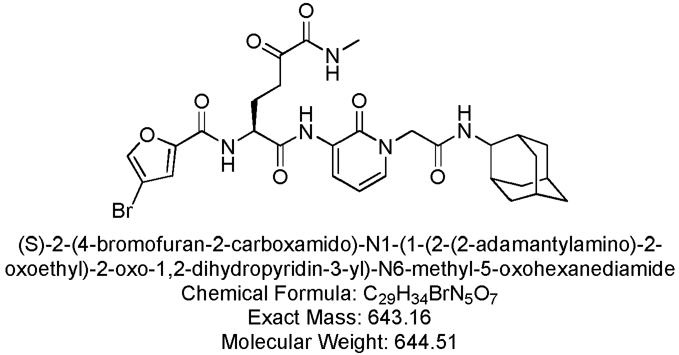

The synthesis of compound **II-47** was performed according to compound **II-3,** using 4-bromofuran-2-carboxylic acid instead of 3-methylbenzo[b]furan-2-carboxylic acid in step 6 (according to ZED3264).
Yield: 90 mg, 69% (last step)
ESI-MS: 644.3 / 646.3 [M+H]⁺

### 54 Preparation of compound II-48

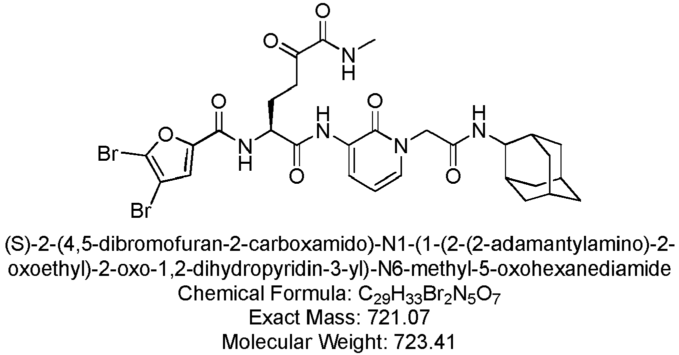

The synthesis of compound **II-48** was performed according to compound **II-3,** using 4,5-dibromofuran-2-carboxylic acid instead of 3-methylbenzo[b]furan-2-carboxylic acid in step 6 (according to ZED3264).
Yield: 72 mg, 56% (last step)
ESI-MS: 722.2 / 724.2 / 726.2 [M+H]⁺

### 55 Preparation of compound II-49

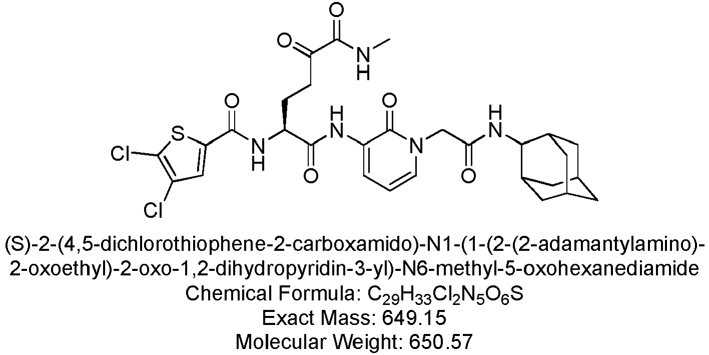

The synthesis of compound **II-49** was performed according to compound **II-3,** using 4,5-dichlorothiophene-2-carboxylic acid instead of 3-methylbenzo[b]furan-2-carboxylic acid in step 6 (according to ZED3264).
Yield: 55 mg, 48% (last step)
ESI-MS: 650.3/652.3 [M+H]⁺

### 56 Preparation of compound II-50

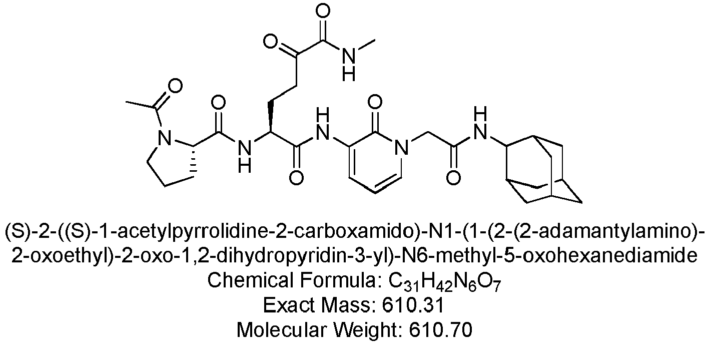

The synthesis of compound **II-50** was performed according to compound **II-3,** using (S)-1-acetylpyrrolidine-2-carboxylic acid instead of 3-methylbenzo[b]furan-2-carboxylic acid in step 6 (according to ZED3264).
Yield: 190 mg, 78% (last step)
ESI-MS: 611.4 [M+H]⁺

### 57 Preparation of compound II-51

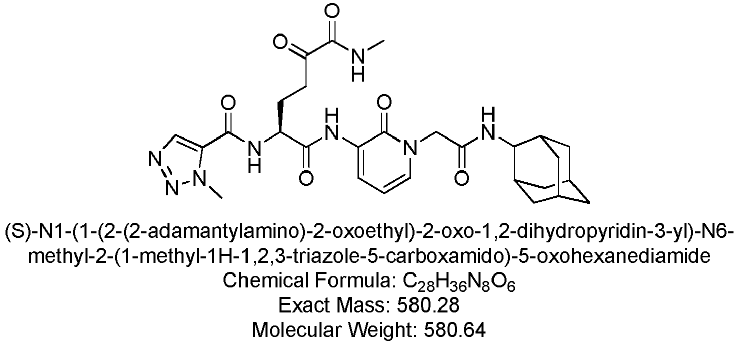

The synthesis of compound **II-51** was performed according to compound **II-3**, using 1-methyl-1H-1,2,3-triazole-5-carboxylic acid instead of 3-methylbenzo[b]furan-2-carboxylic acid in step 6 (according to ZED3264).
Yield: 28 mg, 35% (last step)
ESI-MS: 581.4 [M+H]⁺

### 58 Preparation of compound II-52

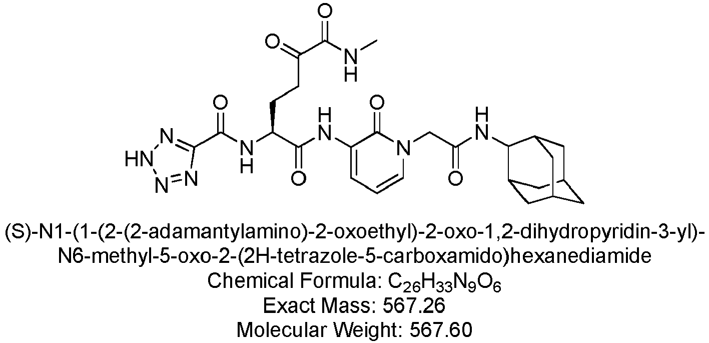

The synthesis of compound **II-52** was performed according to compound **II-3,** using 2H-tetrazole-5-carboxylic acid instead of 3-methylbenzo[b]furan-2-carboxylic acid in step 6 (according to ZED3264).
Yield: 23 mg, 31% (last step)
ESI-MS: 568.4 [M+H]⁺

### 59 Preparation of compound II-53

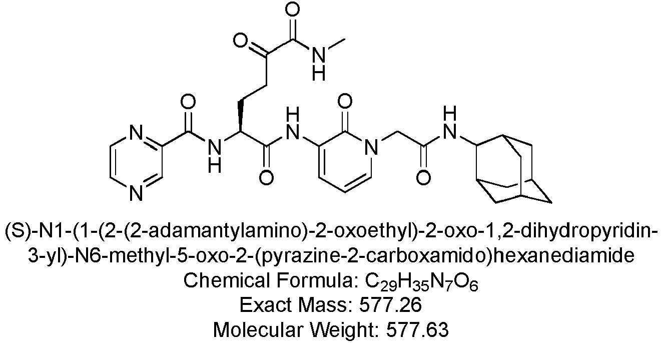

The synthesis of compound **II-53** was performed according to compound **II-3,** using pyrazine-2-carboxylic acid instead of 3-methylbenzo[b]furan-2-carboxylic acid in step 6 (according to ZED3264).
Yield: 79 mg, 74% (last step)
ESI-MS: 578.3 [M+H]⁺

### 60 Preparation of compound II-54

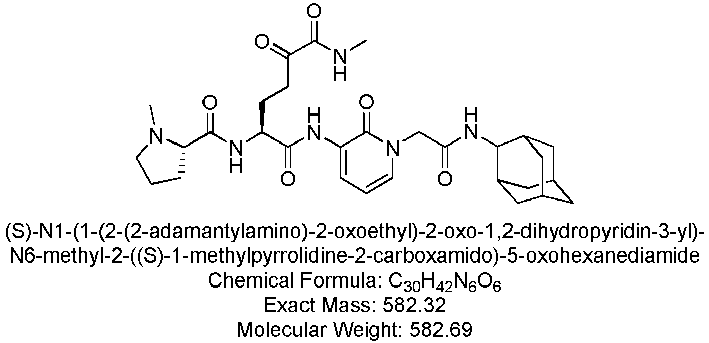

The synthesis of compound **II-54** was performed according to compound **II-3,** using (S)-1-methylpyrrolidine-2-carboxylic acid instead of 3-methylbenzo[b]furan-2-carboxylic acid in step 6 (according to ZED3264).
Yield: 68 mg, 82% (last step)
ESI-MS: 583.4 [M+H]⁺

### 61 Preparation of compound II-55

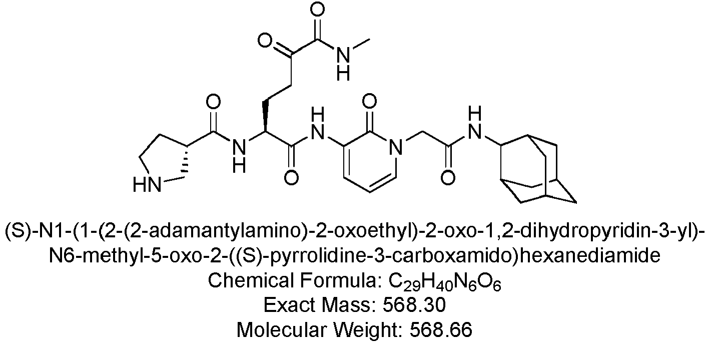

The synthesis of compound **II-55** was performed according to compound **II-3,** using (S)-1-Boc-pyrrolidine-3-carboxylic acid instead of 3-methylbenzo[b]furan-2-carboxylic acid in step 6 (according to ZED3264). The final product was obtained by deprotection (DCM/TFA) as described above and purified by HPLC.
Yield: 43 mg, 79% (last step)
ESI-MS: 569.4 [M+H]⁺

### 62 Preparation of compound II-56

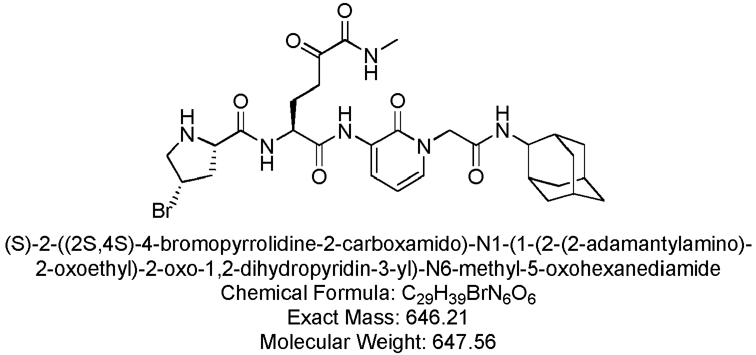

The synthesis of compound **II-56** was performed according to compound **II-3**, using (2S,4S)-1-Boc-4-bromopyrrolidine-2-carboxylic acid instead of 3-methylbenzo[b]furan-2-carboxylic acid in step 6 (according to ZED3264). The final product was obtained by deprotection (DCM/TFA) as described above and purified by HPLC.
Yield: 45 mg, 73% (last step)
ESI-MS: 647.3 / 649.3 [M+H]⁺

### 63 Preparation of compound II-58

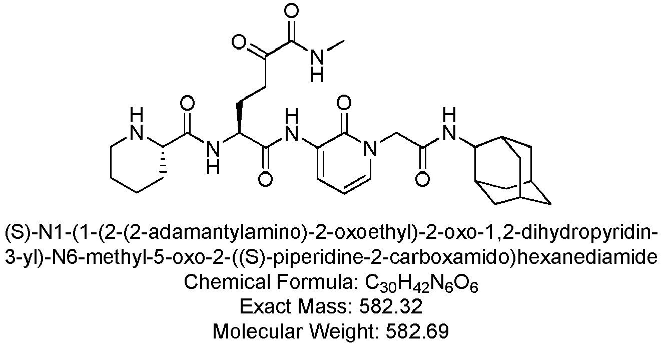

The synthesis of compound **II-58** was performed according to compound **II-3,** using (S)-1-Boc-piperidine-2-carboxylic acid instead of 3-methylbenzo[b]furan-2-carboxylic acid in step 6 (according to ZED3264). The final product was obtained by deprotection (DCM/TFA) as described above and purified by HPLC.
Yield: 53 mg, 86% (last step)
ESI-MS: 583.4 [M+H]⁺

### 64 Preparation of compound II-59

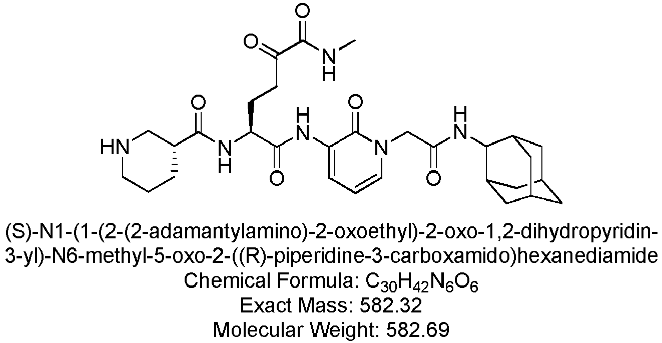

The synthesis of compound **II-59** was performed according to compound **II-3**, using (R)-1-Boc-piperidine-3-carboxylic acid instead of 3-methylbenzo[b]furan-2-carboxylic acid in step 6 (according to ZED3264). The final product was obtained by deprotection (DCM/TFA) as described above and purified by HPLC.
Yield: 43 mg, 77% (last step)
ESI-MS: 583.4 [M+H]⁺

### 65 Preparation of compound II-60

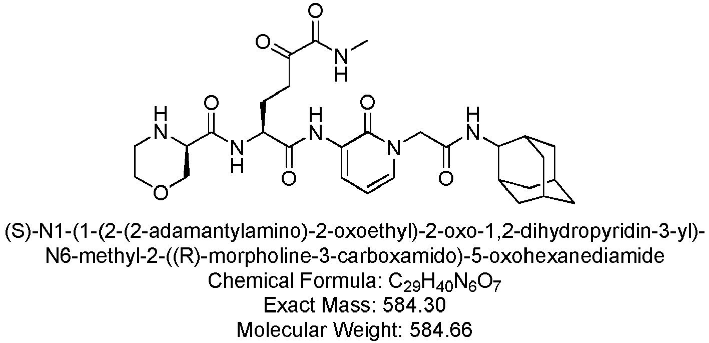

The synthesis of compound **II-60** was performed according to compound **II-3,** using (R)-4-Boc-morpholine-3-carboxylic acid instead of 3-methylbenzo[b]furan-2-carboxylic acid in step 6 (according to ZED3264). The final product was obtained by deprotection (DCM/TFA) as described above and purified by HPLC.
Yield: 67 mg, 85% (last step)
ESI-MS: 585.4 [M+H]⁺

### 66 Preparation of compound II-61

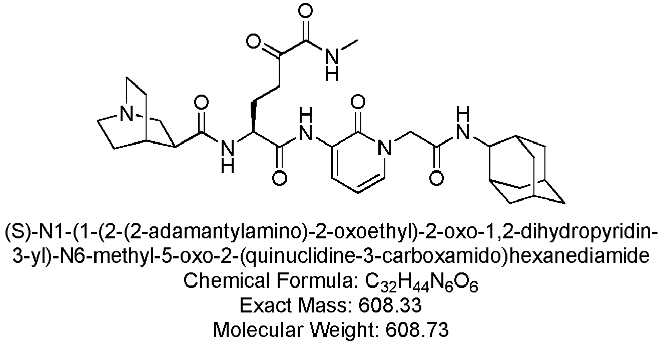

The synthesis of compound **II-61** was performed according to compound **II-3**, using quinuclidine-3-carboxylic acid instead of 3-methylbenzo[b]furan-2-carboxylic acid in step 6 (according to ZED3264).
Yield: 24 mg, 54% (last step)
ESI-MS: 609.4 [M+H]⁺

### 67 Preparation of compound II-62

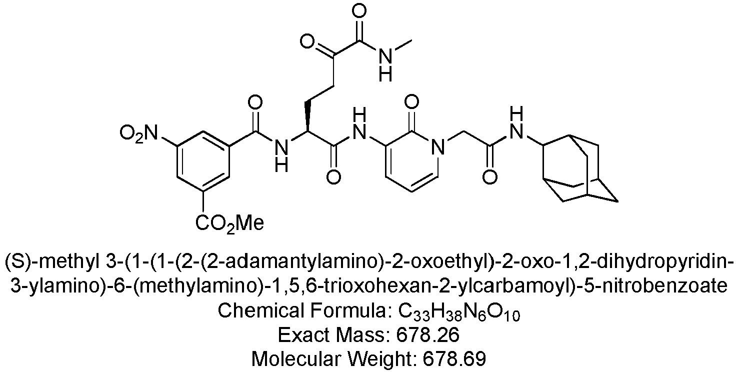

The synthesis of compound **II-62** was performed according to compound **II-3**, using mono-methyl 5-nitroisophthalate instead of 3-methylbenzo[b]furan-2-carboxylic acid in step 6 (according to ZED3264).
Yield: 57 mg, 66% (last step)
ESI-MS: 679.3 [M+H]⁺

### 68 Preparation of compound II-63

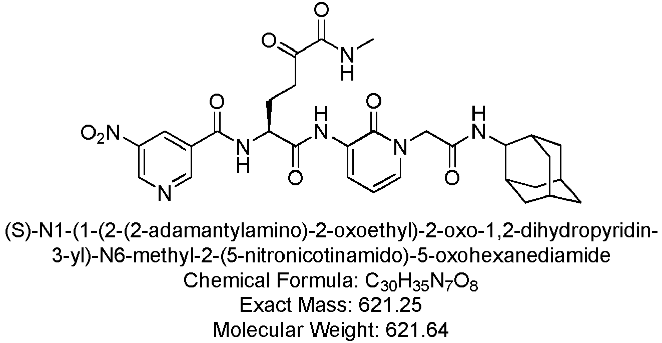

The synthesis of compound **II-63** was performed according to compound **II-3**, using 5-nitronicotinic acid instead of 3-methylbenzo[b]furan-2-carboxylic acid in step 6 (according to ZED3264).
Yield: 76 mg, 65% (last step)
ESI-MS: 622.3 [M+H]⁺

### 69 Preparation of compound II-64

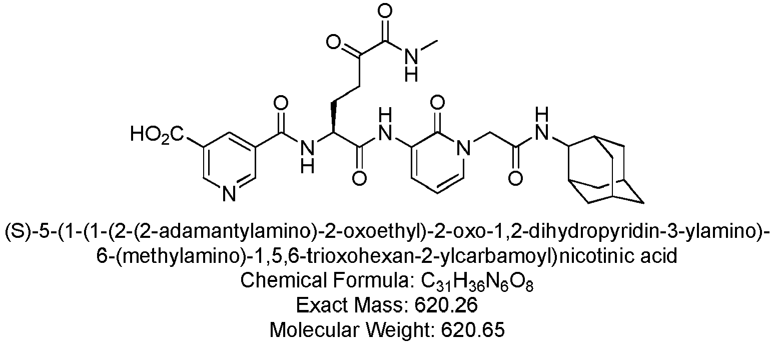

The synthesis of compound **II-64** was performed according to compound **II-3**, using 3,5-pyridinedicarboxylic acid instead of 3-methylbenzo[b]furan-2-carboxylic acid in step 6 (according to ZED3264).
Yield: 16 mg, 52% (last step)
ESI-MS: 621.3 [M+H]⁺

### 70 Preparation of compound II-65

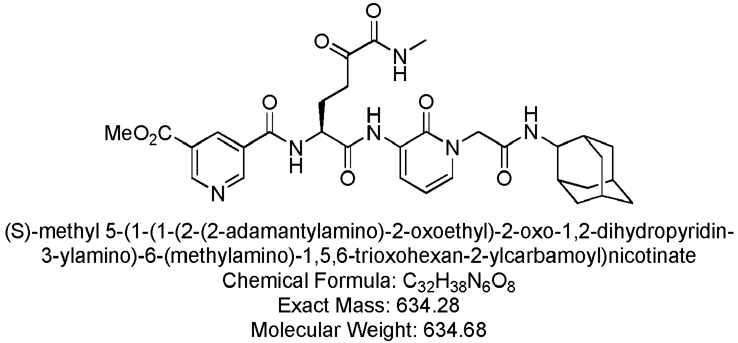

The synthesis of compound **II-65** was performed according to compound **II-3**, using 5-(methoxycarbonyl)nicotinic acid instead of 3-methylbenzo[b]furan-2-carboxylic acid in step 6 (according to ZED3264).
Yield: 34 mg, 62% (last step)
ESI-MS: 635.3 [M+H]⁺

### 71 Preparation of compound II-66

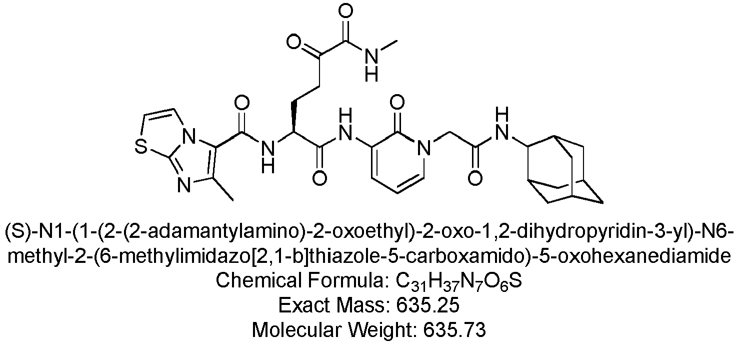

The synthesis of compound **II-66** was performed according to compound **II-3**, using 6-methylimidazo[2,1-b]thiazole-5-carboxylic acid instead of 3-methylbenzo[b]furan-2-carboxylic acid in step 6 (according to ZED3264).
Yield: 45 mg, 37% (last step)
ESI-MS: 636.4 [M+H]⁺

### 72 Preparation of compound II-67

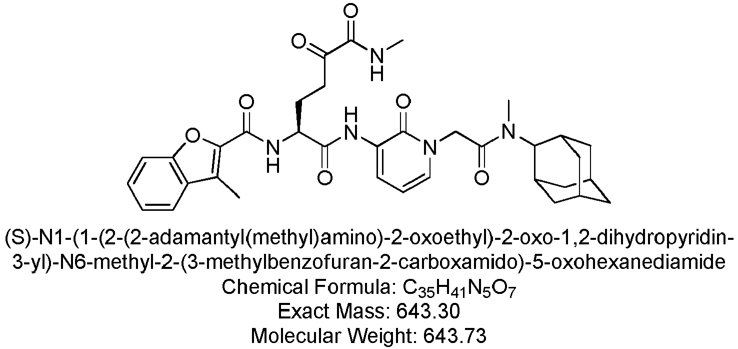

The synthesis of compound **II-67** was performed according to compound **II-3**, using N-methyl-2-adamantanamine instead of 2-adamantanamine in step 2 (according to ZED3905).
Yield: 66 mg, 45% (last step)
ESI-MS: 644.4 [M+H]⁺

### 73 Preparation of compound II-68

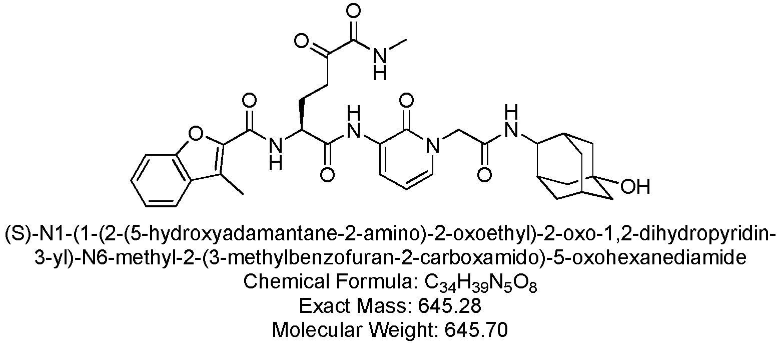

The synthesis of compound **II-68** was performed according to compound **II-3,** using 5-hydroxy-2-adamantanamine instead of 2-adamantanamine in step 2 (according to ZED3905).
Yield: 21 mg, 34% (last step)
ESI-MS: 646.4 [M+H]⁺

### 74 Preparation of compound II-69

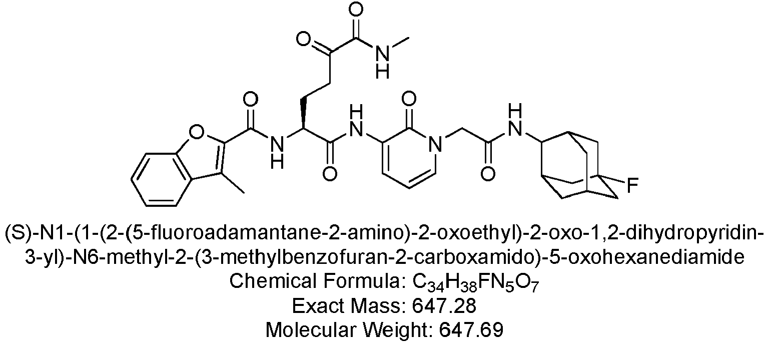

The synthesis of compound **II-69** was performed according to compound **II-3**, using 5-fluoro-2-adamantanamine instead of 2-adamantanamine in step 2 (according to ZED3905).
Yield: 48 mg, 57% (last step)
ESI-MS: 648.4 [M+H]⁺

### 75 Preparation of compound II-70

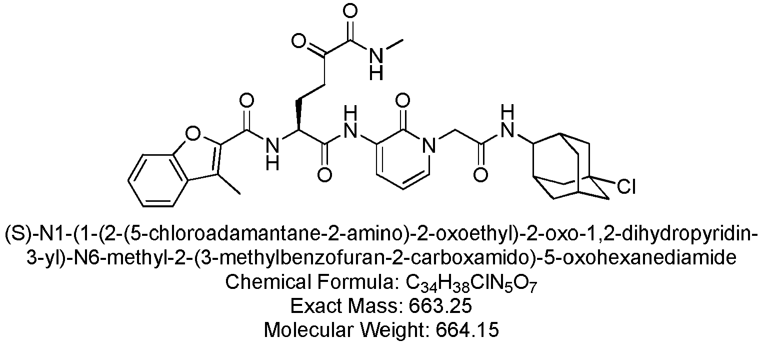

The synthesis of compound **II-70** was performed according to compound **II-3,** using 5-chloro-2-adamantanamine instead of 2-adamantanamine in step 2 (according to ZED3905).
Yield: 45 mg, 35% (last step)
ESI-MS: 664.3 / 666.3 [M+H]⁺

### 76 Preparation of compound II-71

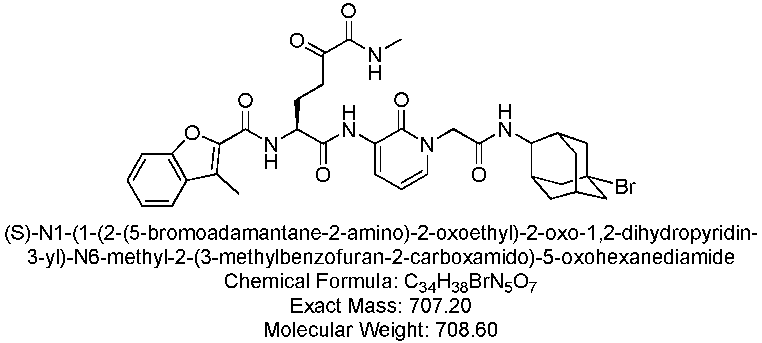

The synthesis of compound **II-71** was performed according to compound **II-3**, using 5-bromo-2-adamantanamine instead of 2-adamantanamine in step 2 (according to ZED3905).
Yield: 31 mg, 34% (last step)
ESI-MS: 708.3 / 710.3 [M+H]⁺

### 77 Preparation of compound II-72

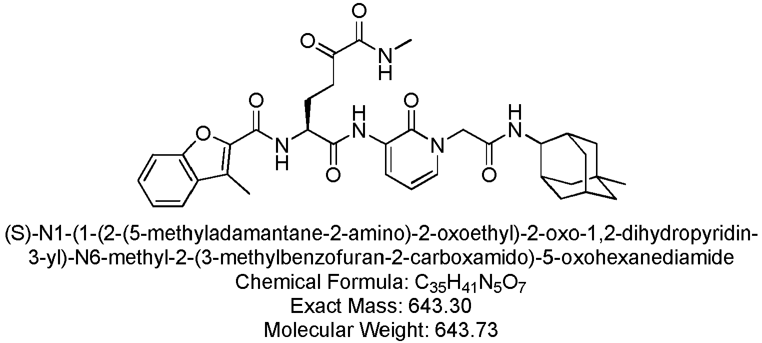

The synthesis of compound **II-72** was performed according to compound **II-3**, using 5-methyl-2-adamantanamine instead of 2-adamantanamine in step 2 (according to ZED3905).
Yield: 68 mg, 54% (last step)
ESI-MS: 644.4 [M+H]⁺

### 78 Preparation of compound II-73

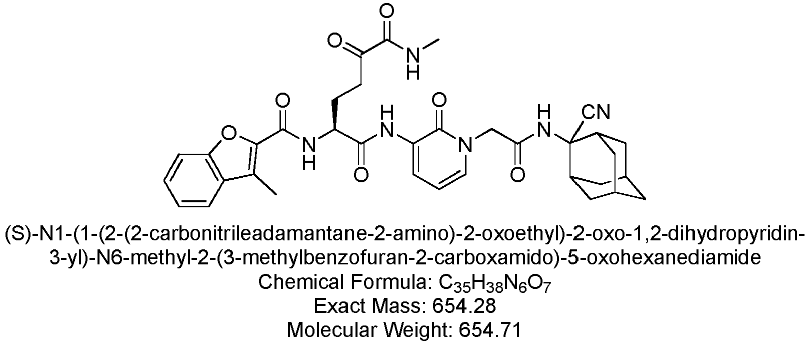

The synthesis of compound **II-73** was performed according to compound **II-3**, using 2-aminoadamantane-2-carbonitrile instead of 2-adamantanamine in step 2 (according to ZED3905).
Yield: 26 mg, 46% (last step)
ESI-MS: 655.4 [M+H]⁺

### 79 Preparation of compound II-74

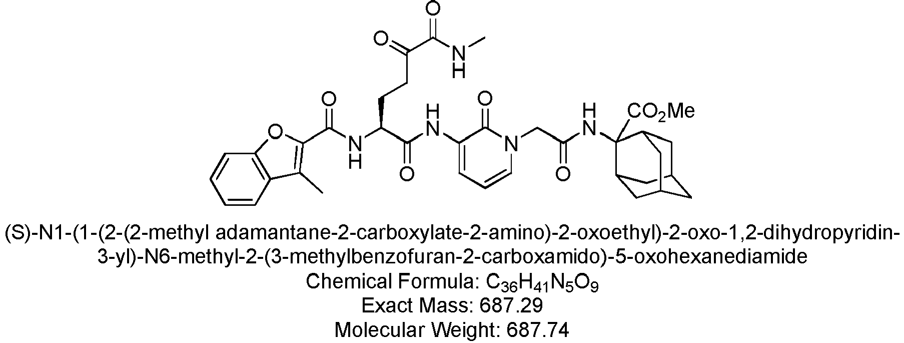

The synthesis of compound **II-74** was performed according to compound **II-3,** using 2-methyl 2-aminoadamantane-2-carboxylate instead of 2-adamantanamine in step 2 (according to ZED3905).
Yield: 38 mg, 61% (last step)
ESI-MS: 688.4 [M+H]⁺

### 80 Preparation of compound II-87

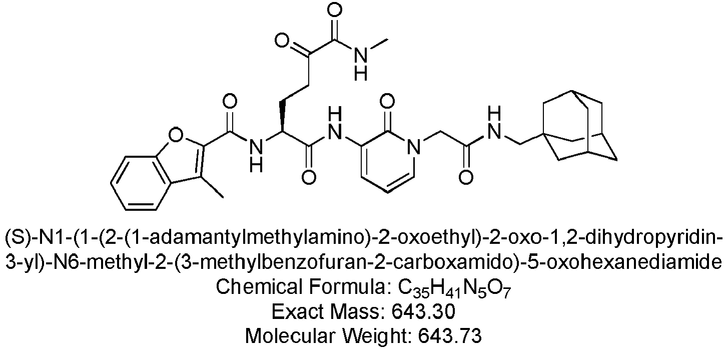

The synthesis of compound **II-87** was performed according to compound **II-3**, using 1-adamantanemethylamine instead of 2-adamantanamine in step 2 (according to ZED3905).
Yield: 43 mg, 53% (last step)
ESI-MS: 644.4 [M+H]⁺

### 81 Preparation of compound II-88

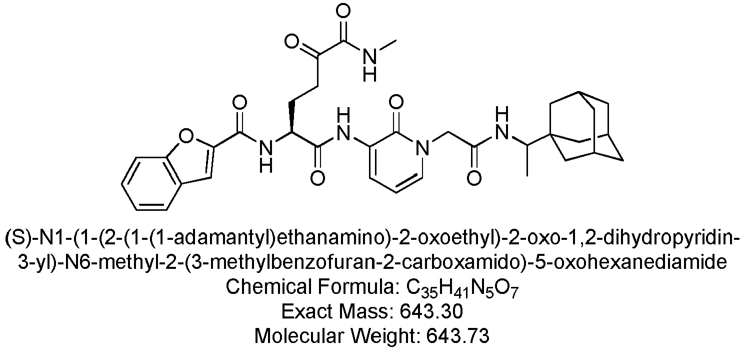

The synthesis of compound **II-88** was performed according to compound **II-2**, using 1-rimantadine instead of 2-adamantanamine in step 2 (according to ZED3905).
Yield: 31 mg, 41% (last step)
ESI-MS: 644.4 [M+H]⁺

### 82 Preparation of compound II-90

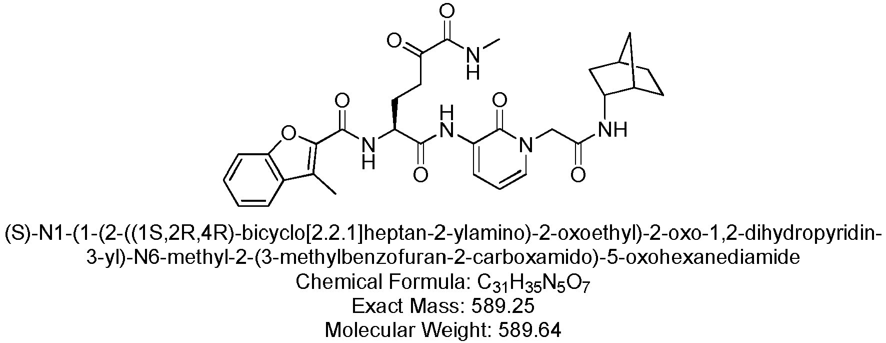

The synthesis of compound **II-90** was performed according to compound **II-3**, using (±)-endo-2-norbornylamine instead of 2-adamantanamine in step 2 (according to ZED3905).
Yield: 67 mg, 65% (last step)
ESI-MS: 590.4 [M+H]⁺

### 83 Preparation of compound II-92

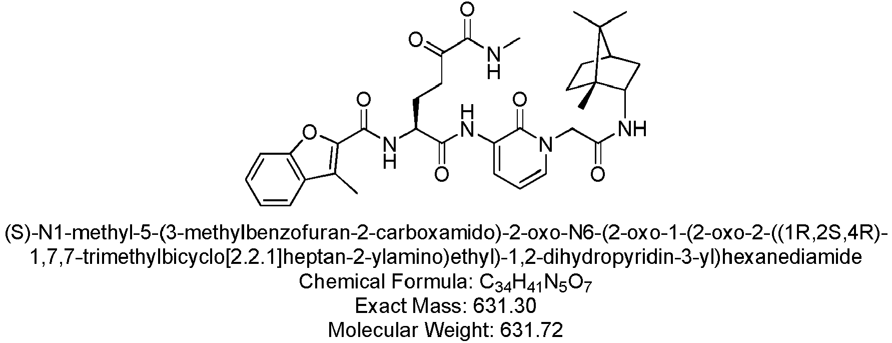

The synthesis of compound **II-92** was performed according to compound **II-3,** using (R)-(+)-bornylamine instead of 2-adamantanamine in step 2 (according to ZED3905).
Yield: 52 mg, 66% (last step)
ESI-MS: 632.5 [M+H]⁺

### 84 Preparation of compound II-94

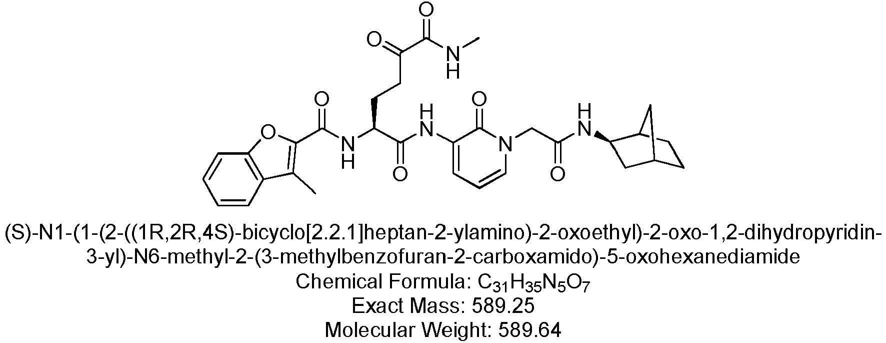

The synthesis of compound **II-94** was performed according to compound **II-3,** using exo-2-aminonorbornane instead of 2-adamantanamine in step 2 (according to ZED3905).
Yield: 62 mg, 68% (last step)
ESI-MS: 590.4 [M+H]⁺

### 85 Preparation of compound II-95

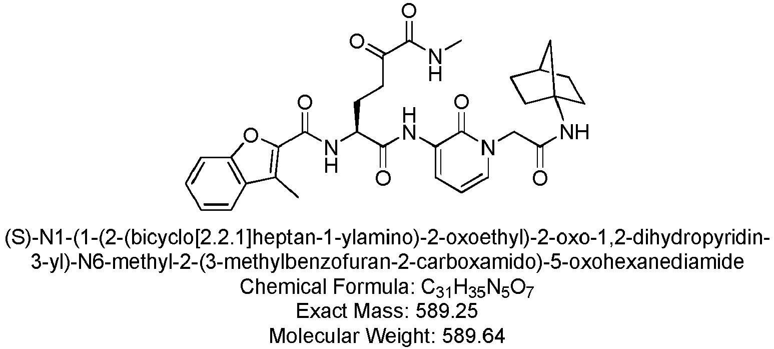

The synthesis of compound **II-95** was performed according to compound **II-3**, using bicyclo[2.2.1]heptan-1-ylamine instead of 2-adamantanamine in step 2 (according to ZED3905).
Yield: 14 mg, 32% (last step)
ESI-MS: 590.4 [M+H]⁺

### 86 Preparation of compound II-96

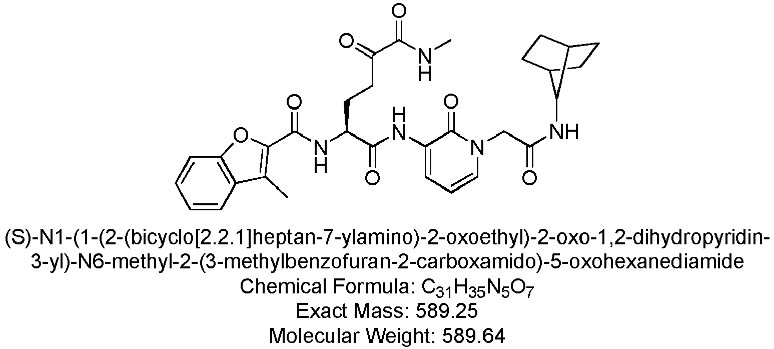

The synthesis of compound **II-96** was performed according to compound **II-3**, using bicyclo[2.2.1]heptan-7-ylamine instead of 2-adamantanamine in step 2 (according to ZED3905).
Yield: 36 mg, 53% (last step)
ESI-MS: 590.4 [M+H]⁺

### 87 Preparation of compound II-97

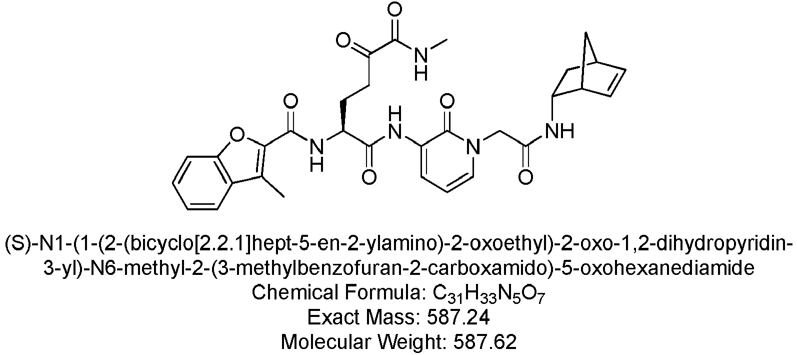

The synthesis of compound **II-97** was performed according to compound **II-3,** using bicyclo[2.2.1]hept-5-en-2-amine instead of 2-adamantanamine in step 2 (according to ZED3905).
Yield: 21 mg, 44% (last step)
ESI-MS: 588.4 [M+H]⁺

### 88 Preparation of compound II-98

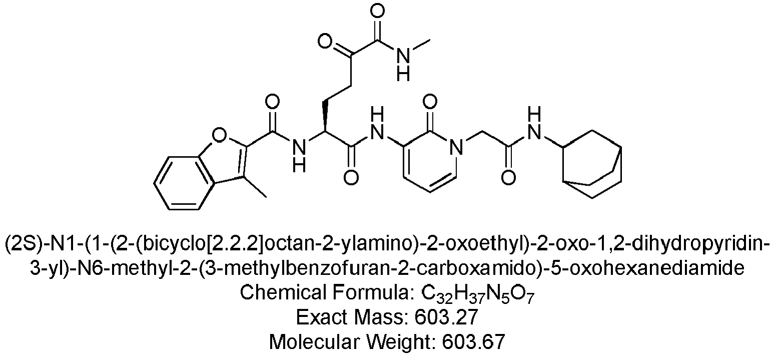

The synthesis of compound **II-98** was performed according to compound **II-3**, using bicyclo[2.2.2]oct-2-ylamine instead of 2-adamantanamine in step 2 (according to ZED3905).
Yield: 25 mg, 41% (last step)
ESI-MS: 604.4 [M+H]⁺

### 89 Preparation of compound II-99

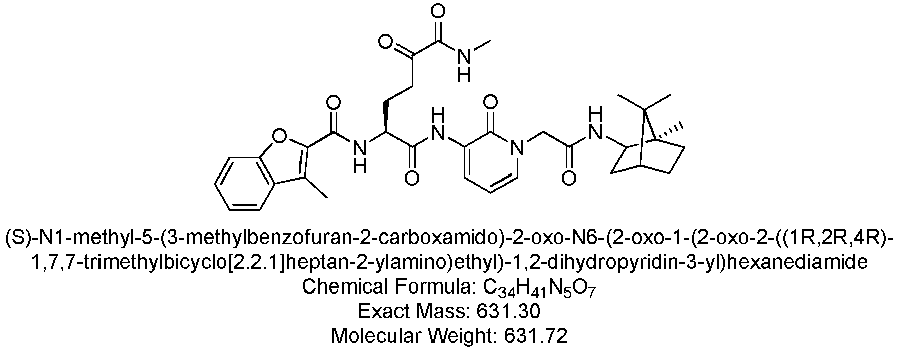

The synthesis of compound **II-99** was performed according to compound **II-3,** using (R)-(-)-isobornylamine instead of 2-adamantanamine in step 2 (according to ZED3905).
Yield: 27 mg, 48% (last step)
ESI-MS: 632.5 [M+H]⁺

### 90 Preparation of compound II-100

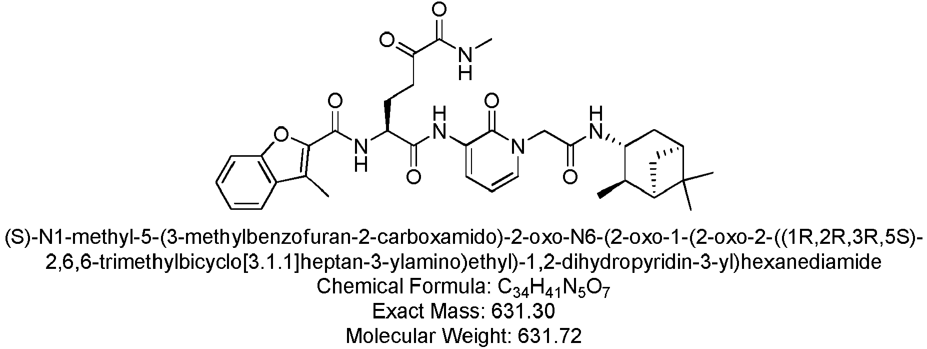

The synthesis of compound **II-100** was performed according to compound **II-3**, using (1R,2R,3R,5S)-(-)-isopinocampheylamine instead of 2-adamantanamine in step 2 (according to ZED3905).
Yield: 17 mg, 39% (last step)
ESI-MS: 632.5 [M+H]⁺

### 91 Preparation of compound II-101

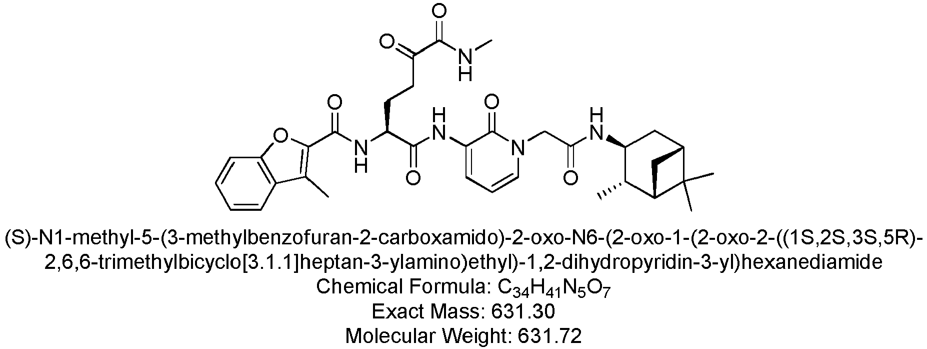

The synthesis of compound **II-101** was performed according to compound **II-3**, using (1S,2S,3S,5R)-(+)-isopinocampheylamine instead of 2-adamantanamine in step 2 (according to ZED3905).
Yield: 25 mg, 41% (last step)
ESI-MS: 632.5 [M+H]⁺

### 92 Preparation of compound II-103

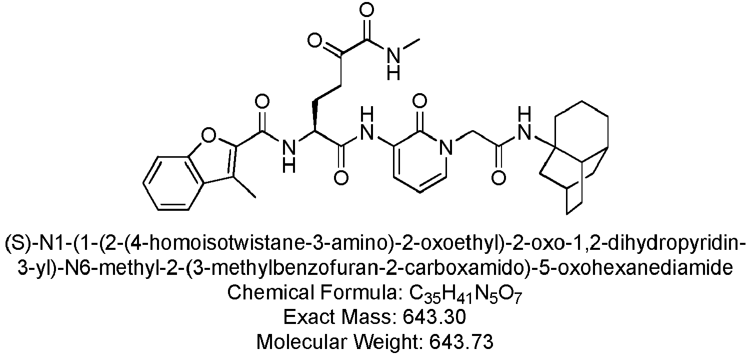

The synthesis of compound **II-103** was performed according to compound **II-3**, using 3-amino-4-homoisotwistane instead of 2-adamantanamine in step 2 (according to ZED3905).
Yield: 12 mg, 28% (last step)
ESI-MS: 644.5 [M+H]⁺

### 93 Preparation of compound II-104

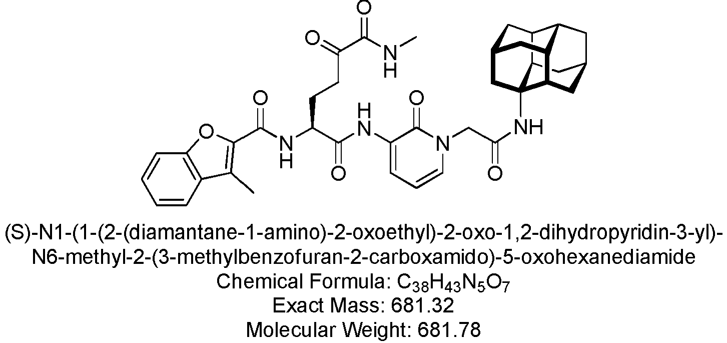

The synthesis of compound **II-104** was performed according to compound **II-3,** using 1-aminodiamantane instead of 2-adamantanamine in step 2 (according to ZED3905).
Yield: 17 mg, 35% (last step)
ESI-MS: 682.5 [M+H]⁺

### 94 Preparation of compound II-105

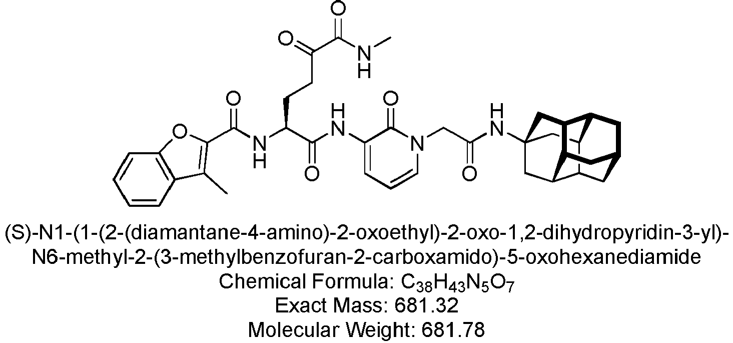

The synthesis of compound **II-105** was performed according to compound **II-3**, using 4-aminodiamantane instead of 2-adamantanamine in step 2 (according to ZED3905).
Yield: 8 mg, 26% (last step)
ESI-MS: 682.5 [M+H]⁺

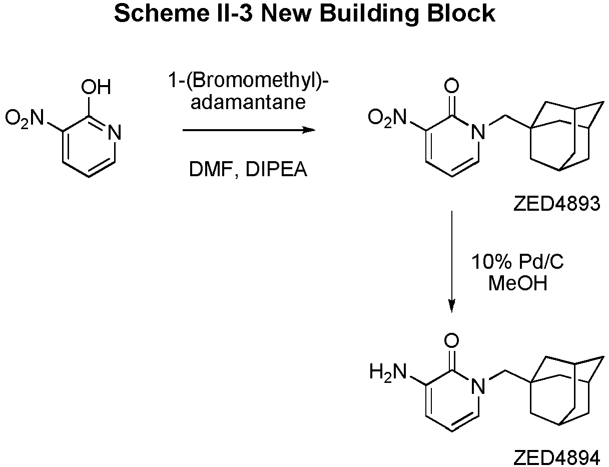

### 95 Preparation of compound ZED4893

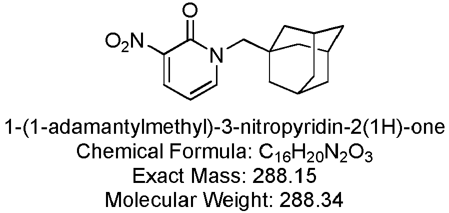

500 mg (3.57 mmol) of 2-hydroxy-3-nitropyridine and 818 mg (1 eq) of 1-(bromomethyl)adamantane were dissolved in 10 mL DMF and 1.24 mL DIPEA (2 eq) and stirred at room temperature overnight. The solvent was evaporated; the residue was dissolved in 30 mL EtOAc and washed twice with each 10 mL citric acid solution (10%), NaHCO₃ solution (10%) and brine. The organic phase was dried over Na₂SO₄, filtered and the solvent was evaporated. The residue was purified by HPLC.
Yield: 484 mg, 47% ESI-MS: 289.3 [M+H]⁺

### 96 Preparation of compound ZED4894

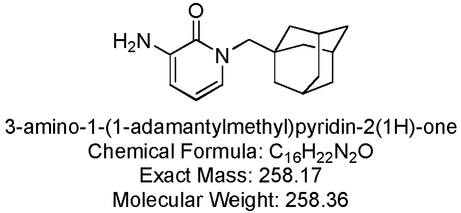

484 mg (1.68 mmol) of ZED4893 were suspended in 30 mL MeOH before 50 mg of palladium (10%) on activated carbon (unreduced) were added. The suspension was stirred for 3 h at room temperature under an atmosphere of hydrogen. The catalyst was filtered, and the solvent was evaporated.
Yield: 339 mg, 78%
ESI-MS: 259.4 [M+H]⁺

### 97 Preparation of compound II-107

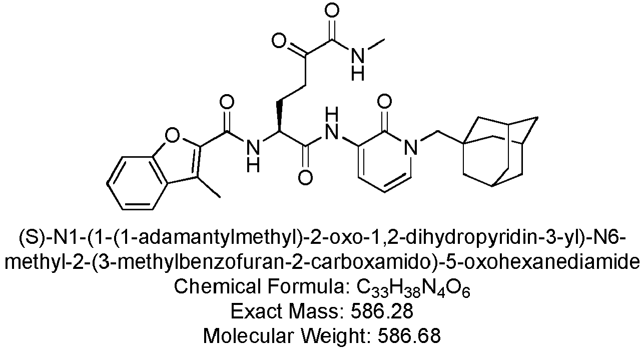

The synthesis of compound **II-107** was performed according to compound **II-3,** using **ZED4894** instead of **ZED3906** in step 5 (according to ZED3907).
Yield: 41 mg, 49% (last step)
ESI-MS: 587.4 [M+H]⁺

### 98 Preparation of compound II-108

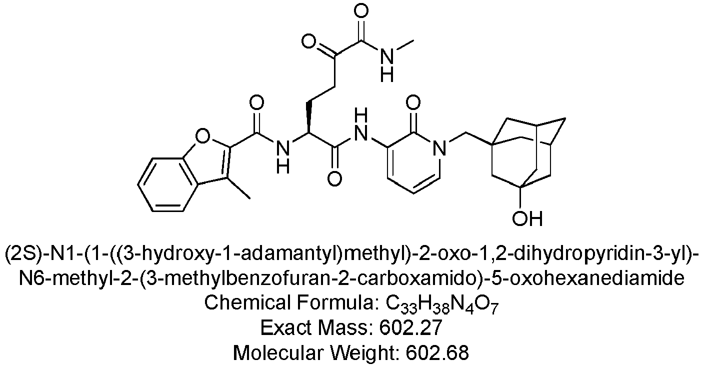

The synthesis of compound **II-108** was performed according to compound **II-107**, using 3-(bromomethyl)-1-adamantanol instead of 1-(bromomethyl)adamantane (according to ZED4893).
Yield: 16 mg, 36% (last step)
ESI-MS: 603.4 [M+H]⁺

### 99 Preparation of compound II-109

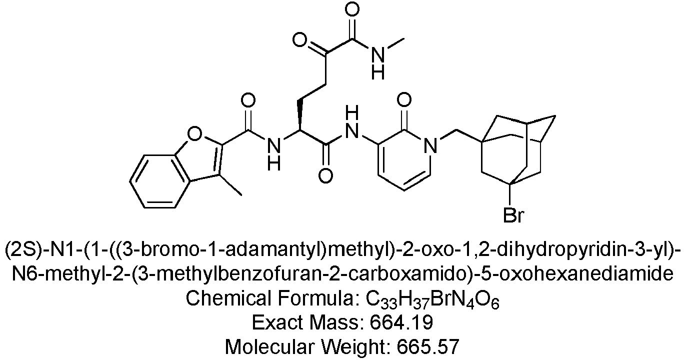

The synthesis of compound **II-109** was performed according to compound **II-107**, using 1-bromo-3-(bromomethyl)adamantane instead of 1-(bromomethyl)adamantane (according to ZED4893).
Yield: 24 mg, 41% (last step)
ESI-MS: 665.3 / 667.3 [M+H]⁺

### 100 Preparation of compound II-110

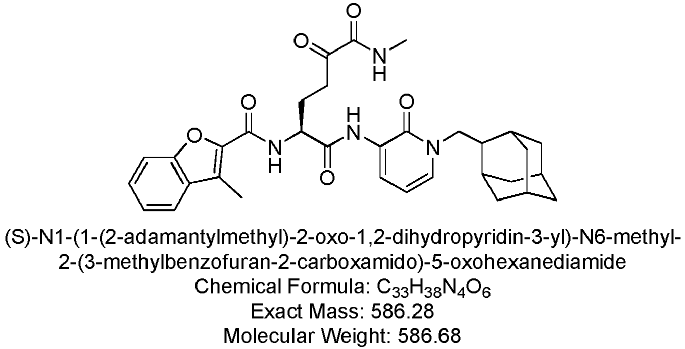

The synthesis of compound **II-110** was performed according to compound **II-107**, using 2-(bromomethyl)adamantane instead of 1-(bromomethyl)adamantane (according to ZED4893).
Yield: 46 mg, 62% (last step)
ESI-MS: 587.4 [M+H]⁺

### 101 Preparation of compound II-111

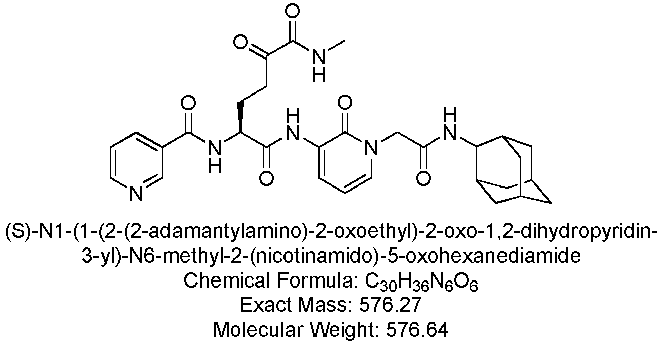

The synthesis of compound **II-111** was performed according to compound II-3, using nicotinic acid instead of 3-methylbenzo[b]furan-2-carboxylic acid in step 6 (according to ZED3264).
Yield: 65 mg, 46% (last step)
ESI-MS: 577.4 [M+H]⁺

### 102 Preparation of compound II-112

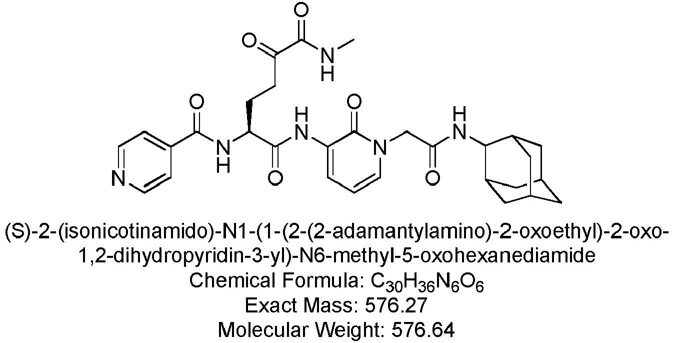

The synthesis of compound **II-112** was performed according to compound **II-3**, using isonicotinic acid instead of 3-methylbenzo[b]furan-2-carboxylic acid in step 6 (according to ZED3264).
Yield: 47 mg, 52% (last step)
ESI-MS: 577.4 [M+H]⁺

### 103 Preparation of compound II-113

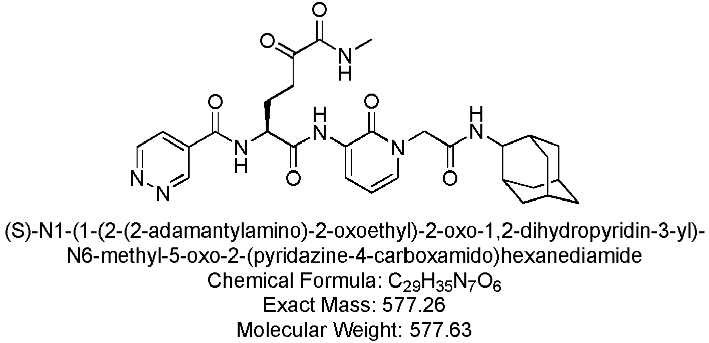

The synthesis of compound **II-113** was performed according to compound **II-3,** using pyridazine-4-carboxylic acid instead of 3-methylbenzo[b]furan-2-carboxylic acid in step 6 (according to ZED3264).
Yield: 34 mg, 46% (last step)
ESI-MS: 578.4 [M+H]⁺

### 104 Preparation of compound II-114

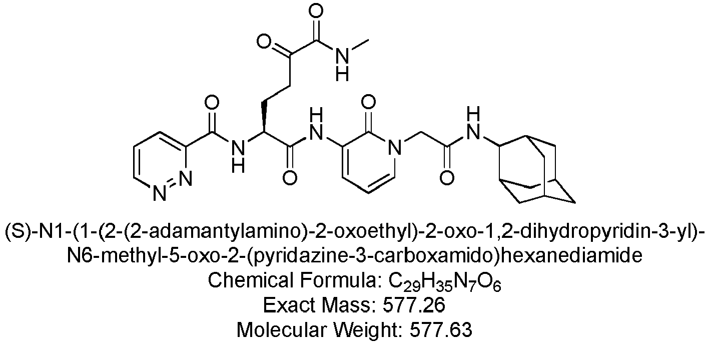

The synthesis of compound **II-114** was performed according to compound **II-3,** using pyridazine-3-carboxylic acid instead of 3-methylbenzo[b]furan-2-carboxylic acid in step 6 (according to ZED3264).
Yield: 43 mg, 56% (last step)
ESI-MS: 578.4 [M+H]⁺

### 105 Preparation of compound II-115

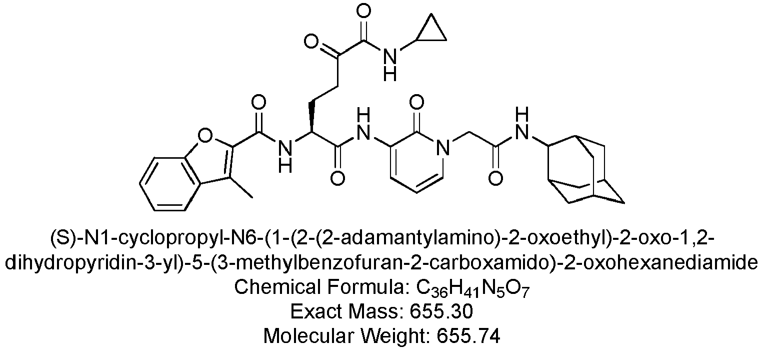

The synthesis of compound **II-115** was performed according to compound **II-3**, using cyclopropyl isocyanide instead of methyl isocyanide in step 4 (according to ZED3632).
Yield: 47 mg, 64% (last step)
ESI-MS: 656.5 [M+H]⁺

### 106 Preparation of compound II-116

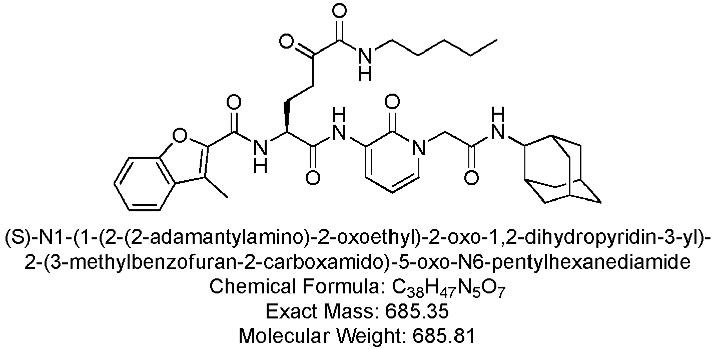

The synthesis of compound **II-116** was performed according to compound **II-3**, using pentyl isocyanide instead of methyl isocyanide in step 4 (according to ZED3632).
Yield: 87 mg, 71% (last step)
ESI-MS: 686.5 [M+H]⁺

### 107 Preparation of compound II-117

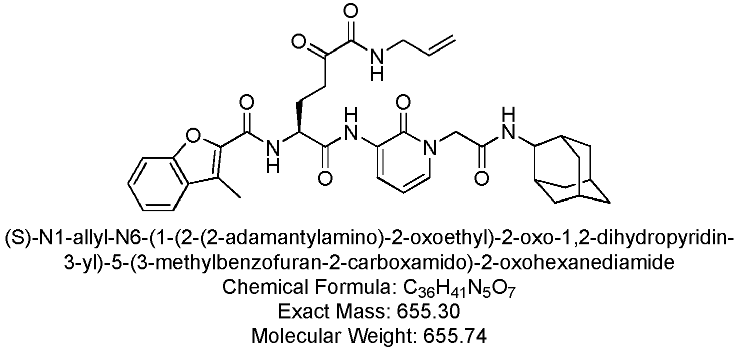

The synthesis of compound **II-117** was performed according to compound **II-3**, using allyl isocyanide instead of methyl isocyanide in step 4 (according to ZED3632).
Yield: 42 mg, 63% (last step)
ESI-MS: 656.5 [M+H]⁺

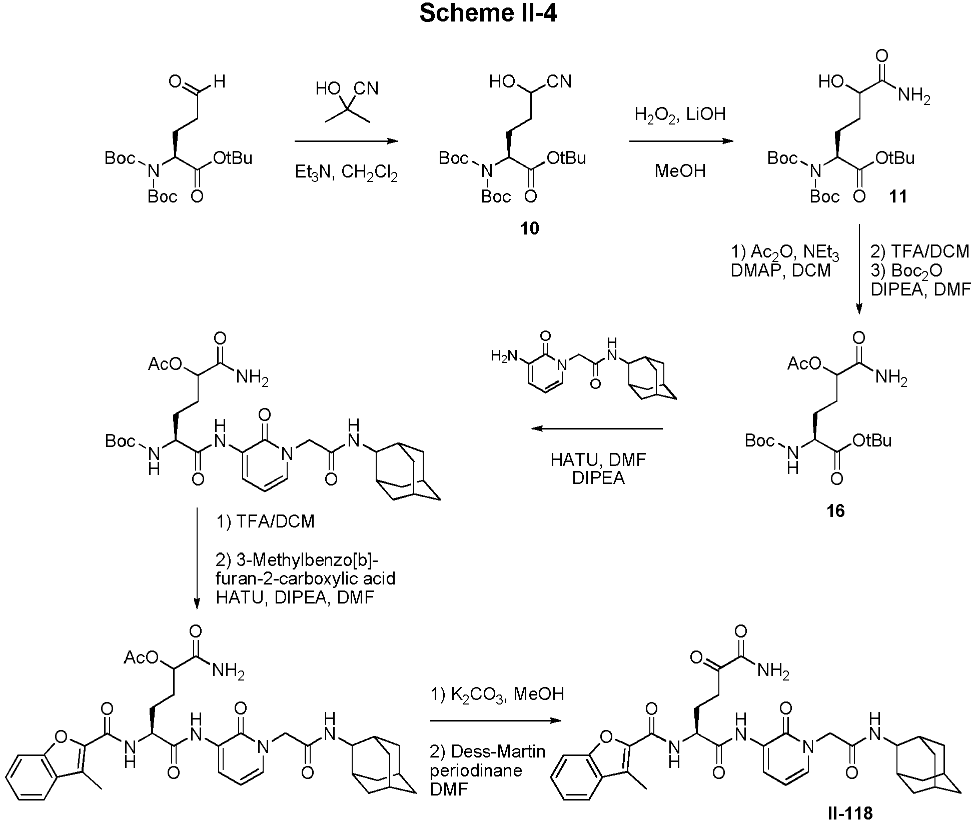

### Preparation of compound 10

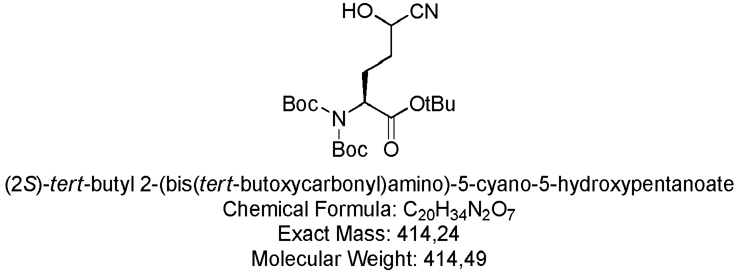

15.0 g (38.7 mmol) of the aldehyde (S)-tert-butyl 2-(bis(tert-butoxycarbonyl)amino)-5-oxopentanoate (**ZED721**) were dissolved in 150 ml DCM. 6.42 ml (46.3 mmol) trimethylamine and 7.37 ml (79.9 mmol) acetone cyanohydrin were added, and the reaction was stirred at room temperature overnight. The solution was washed twice with each citric acid solution (10%) and brine. The organic phase was dried over Na₂SO₄, filtered and the solvent was evaporated. The residue was purified by flash chromatography.
Yield: 16.2 g, >100%
ESI-MS: 437.6 [M+Na]⁺

### Preparation of compound 11

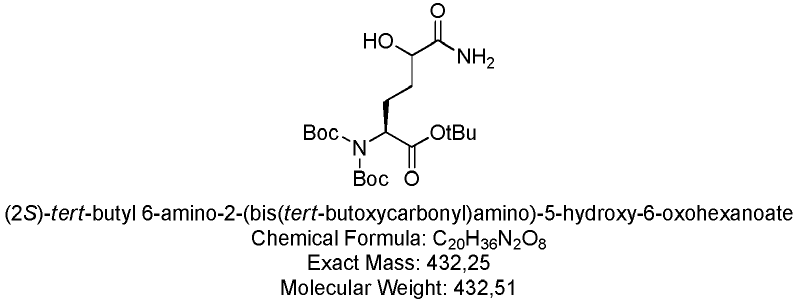

16.2 g (-38.6 mmol) of cyanohydrin **10** were dissolved in 95 ml MeOH at 4 °C and 1.91 g (45.5 mmol) lithium hydroxide monohydrate were added. 18.6 ml hydrogen peroxide (35%) were added dropwise, and the reaction was stirred at room temperature for 1.5 h before quenching with sodium thiosulfate solution (5%). The aqueous phase was extracted with DCM. The combined organic phases were dried over Na₂SO₄, filtered and the solvent was evaporated. The residue was purified by flash chromatography.
Yield: 8.61 g, 52%
ESI-MS: 455.2 [M+Na]⁺

### Preparation of compound 15

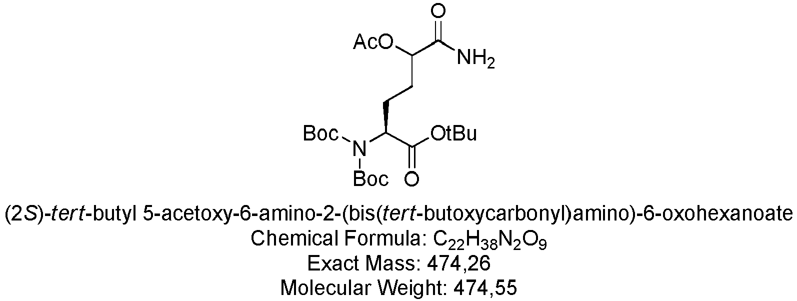

8.61 g (19.9 mmol) of hydroxyamide **10** were dissolved in 55 ml DCM. 3.45 ml (24.9 mmol) 1.91 g (45.5 mmol) trimethylamine, 2.12 ml acetic anhydride and 62 mg (0.50 mmol) DMAP were added, and the reaction was stirred at room temperature for 3 h. After washing with water and brine, the organic phase was dried over Na₂SO₄, filtered and the solvent was evaporated. The product precipitates from MTBE solution by addition of hexane.
Yield: 8.08 g, 86%
ESI-MS: 475.5 [M+H]⁺

### Preparation of compound 16

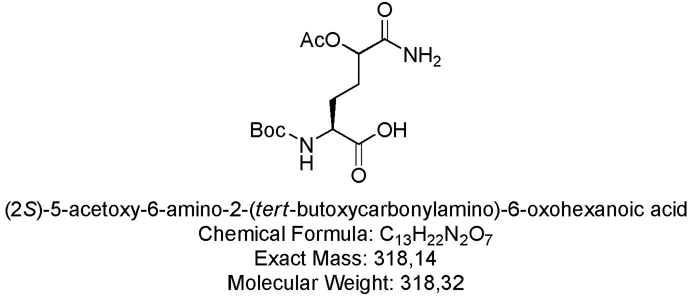

8.08 g (17.0 mmol) of **15** were dissolved in 140 ml DCM/TFA (1:1) and stirred at room temperature for 3 h. The solvent was evaporated, and the residue was dissolved in 40 ml DMF. 5.80 ml (2 eq) DIPEA and 4.55 g (20.4 mmol) di-tert-butyl dicarbonate in 20 ml DMF were added and the reaction was stirred at room temperature overnight. The solvent was evaporated, and the residue was dissolved in 80 ml EtOAc. After extraction with NaHCO₃ solution (1.05 eq in water), the product precipitates from the aqueous phase by addition of 1.5 eq citric acid.
Yield: 1.64 g, 30%
ESI-MS: 319.4 [M+H]⁺

### 108 Preparation of compound II-118

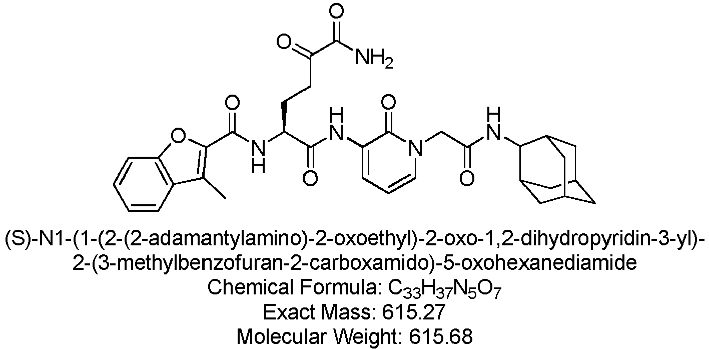

The synthesis of compound **II-118** was performed according to compound **II-3,** using compound **16** instead of ZED3632 in step 5 (according to ZED3907).
Yield: 158 mg, 56% (last step)
ESI-MS: 616.4 [M+H]⁺

### 109 Preparation of compound II-119

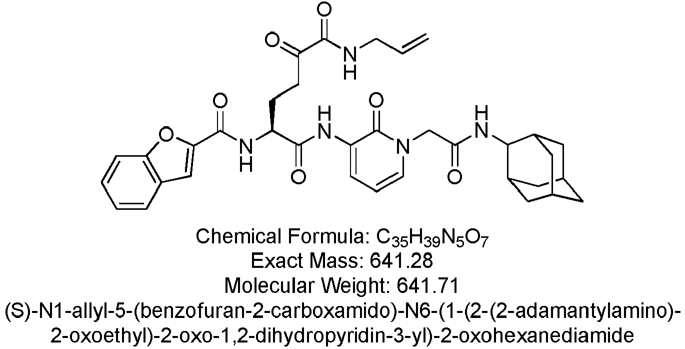

The synthesis of compound **II-119** was performed according to compound **II-2**, using allyl isocyanide instead of methyl isocyanide in step 4 (according to ZED3632).
Yield: 56 mg, 71% (last step)
ESI-MS: 642.4 [M+H]⁺

### 110 Preparation of compound II-120

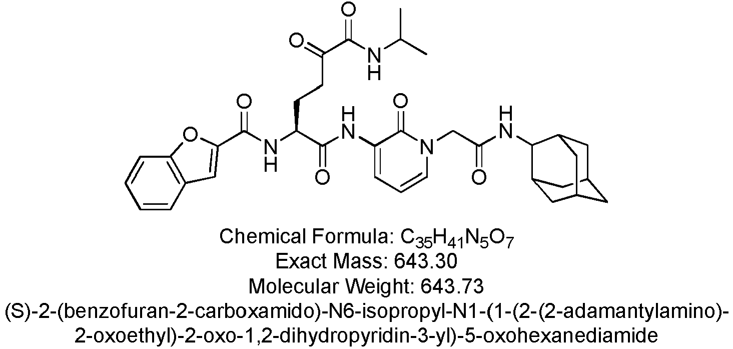

The synthesis of compound **II-120** was performed according to compound **II-2,** using isopropyl isocyanide instead of methyl isocyanide in step 4 (according to ZED3632).
Yield: 62 mg, 65% (last step)
ESI-MS: 644.5 [M+H]⁺

### 111 Preparation of compound II-121

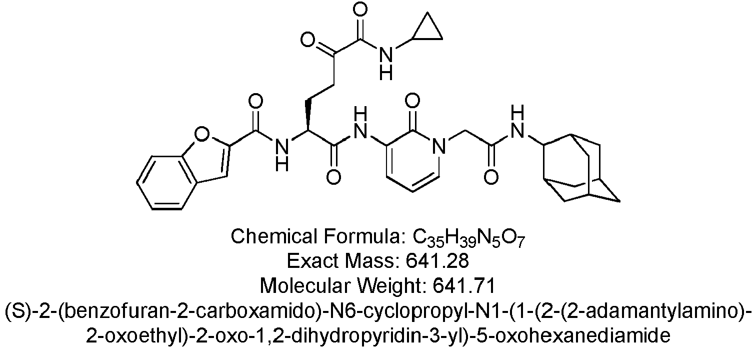

The synthesis of compound **II-121** was performed according to compound **II-2**, using cyclopropyl isocyanide instead of methyl isocyanide in step 4 (according to ZED3632).
Yield: 44 mg, 51% (last step)
ESI-MS: 642.4 [M+H]⁺

### 112 Preparation of compound II-122

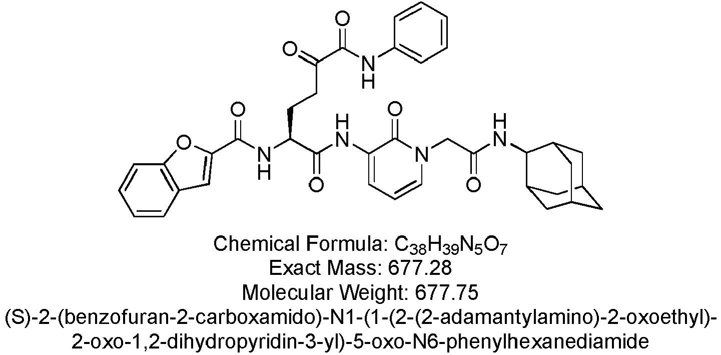

The synthesis of compound **II-122** was performed according to compound **II-2**, using phenyl isocyanide instead of methyl isocyanide in step 4 (according to ZED3632).
Yield: 37 mg, 56% (last step)
ESI-MS: 678.4 [M+H]⁺

### 113 Preparation of compound II-123

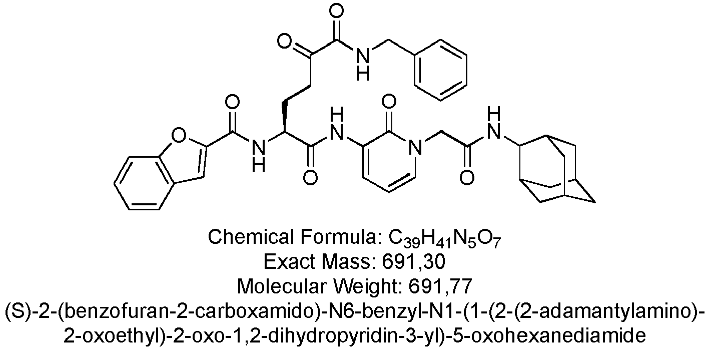

The synthesis of compound **II-123** was performed according to compound **II-2**, using benzyl isocyanide instead of methyl isocyanide in step 4 (according to ZED3632).
Yield: 46 mg, 52% (last step)
ESI-MS: 692.5 [M+H]⁺

### 114 Preparation of compound II-124

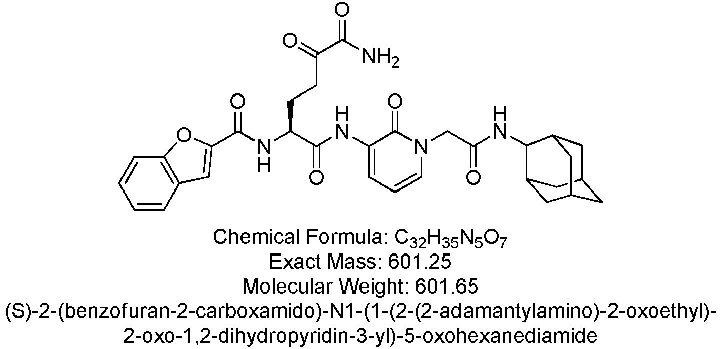

The synthesis of compound **II-124** was performed according to compound **II-118,** using benzofuran-2-carboxylic acid instead of 3-methylbenzo[b]furan-2-carboxylic acid in step 6 (according to ZED3264).
Yield: 96 mg, 81% (last step)
ESI-MS: 602.4 [M+H]⁺

### 115 Preparation of compound II-125

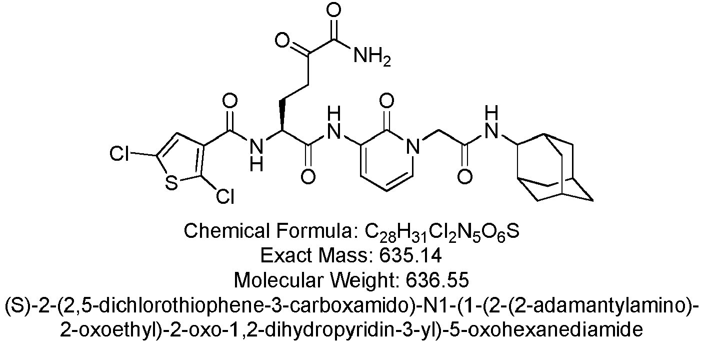

The synthesis of compound **II-125** was performed according to compound **II-124,** using 2,5-dichlorothiophene-3-carboxylic acid instead of benzofuran-2-carboxylic acid in step 6 (according to ZED3264).
Yield: 78 mg, 71% (last step)
ESI-MS: 636.3 / 638.3 [M+H]⁺

### 116 Preparation of compound II-126

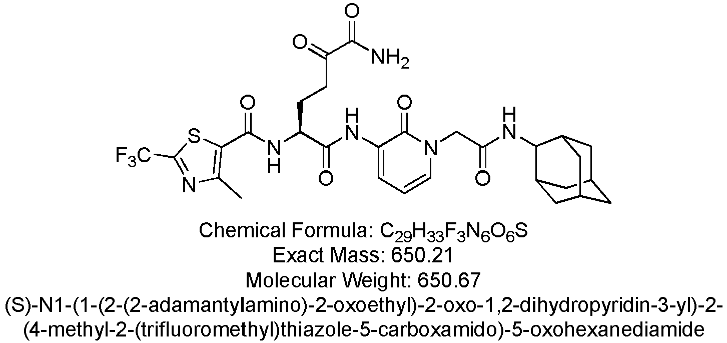

The synthesis of compound **II-126** was performed according to compound **II-124**, using 4-methyl-2-(trifluoromethyl)thiazole-5-carboxylic acid instead of benzofuran-2-carboxylic acid in step 6 (according to ZED3264).
Yield: 53 mg, 67% (last step)
ESI-MS: 651.3 [M+H]⁺

### 117 Preparation of compound II-127

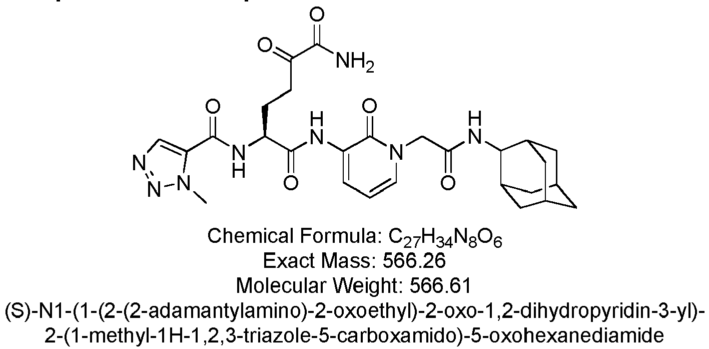

The synthesis of compound **II-127** was performed according to compound **II-124**, using 1-methyl-1H-1,2,3-triazole-5-carboxylic acid instead of benzofuran-2-carboxylic acid in step 6 (according to ZED3264).
Yield: 26 mg, 49% (last step)
ESI-MS: 567.3 [M+H]⁺

### 118 Preparation of compound II-128

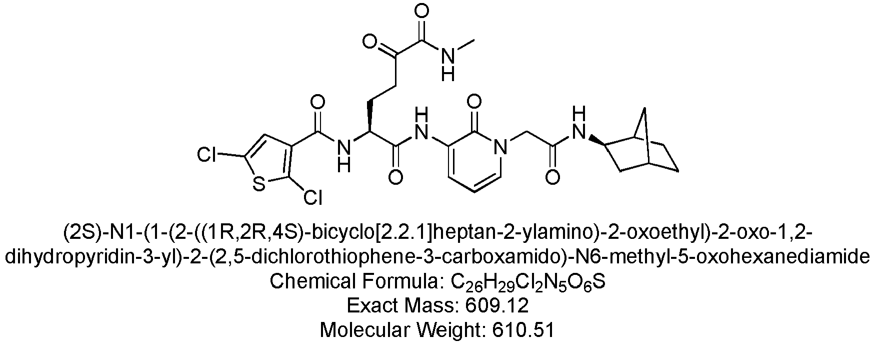

The synthesis of compound **II-128** was performed according to compound **II-97**, using 2,5-dichlorothiophene-3-carboxylic acid instead of 3-methylbenzo[b]furan-2-carboxylic acid in step 6 (according to ZED3264).
Yield: 53 mg, 73% (last step)
ESI-MS: 610.3 / 612.3 [M+H]⁺

### 119 Preparation of compound II-129

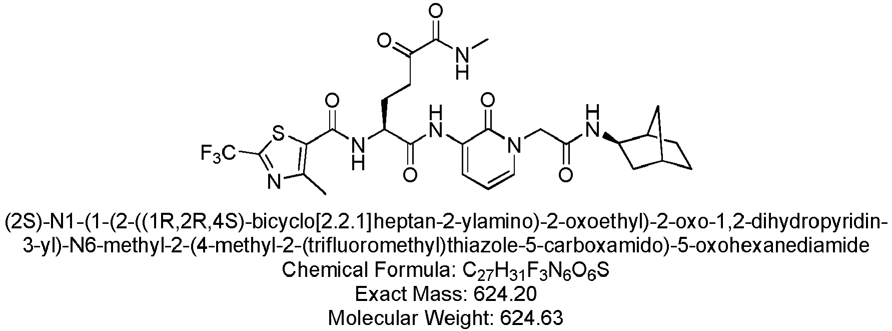

The synthesis of compound **II-129** was performed according to compound **II-97**, using 4-methyl-2-(trifluoromethyl)thiazole-5-carboxylic acid instead of 3-methylbenzo[b]furan-2-carboxylic acid in step 6 (according to ZED3264).
Yield: 42 mg, 60% (last step)
ESI-MS: 625.3 [M+H]⁺

### 120 Preparation of compound II-130

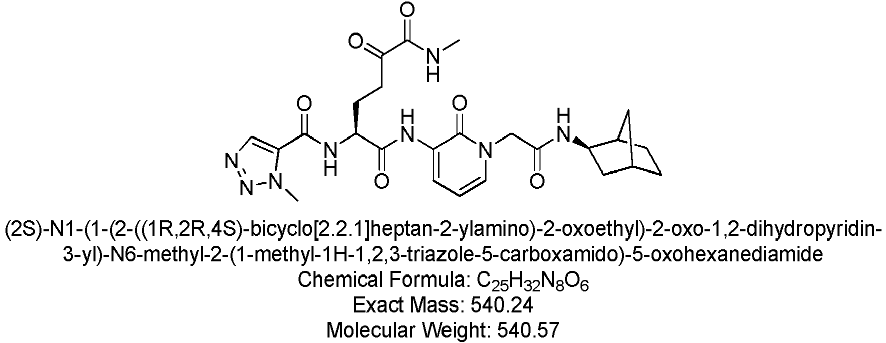

The synthesis of compound **II-130** was performed according to compound **II-97**, using 1-methyl-1H-1,2,3-triazole-5-carboxylic acid instead of 3-methylbenzo[b]furan-2-carboxylic acid in step 6 (according to ZED3264).
Yield: 15 mg, 39% (last step)
ESI-MS: 541.3 [M+H]⁺

### 121 Preparation of compound II-131

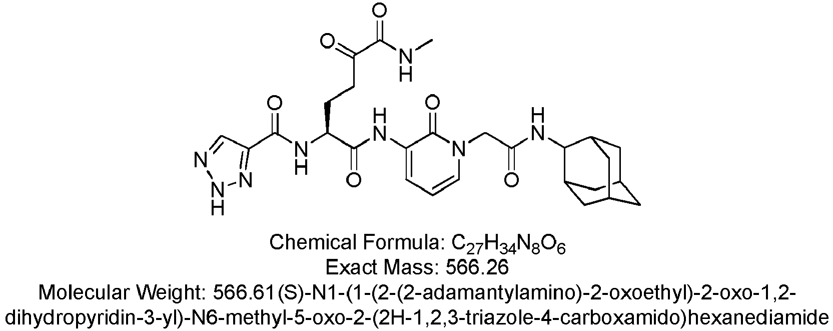

The synthesis of compound **II-131** was performed according to compound **II-3**, using 2H-1,2,3-triazole-4-carboxylic acid instead of 3-methylbenzo[b]furan-2-carboxylic acid in step 6 (according to ZED3264).
Yield: 28 mg, 56% (last step)
ESI-MS: 567.4 [M+H]⁺

### 122 Preparation of compound II-132

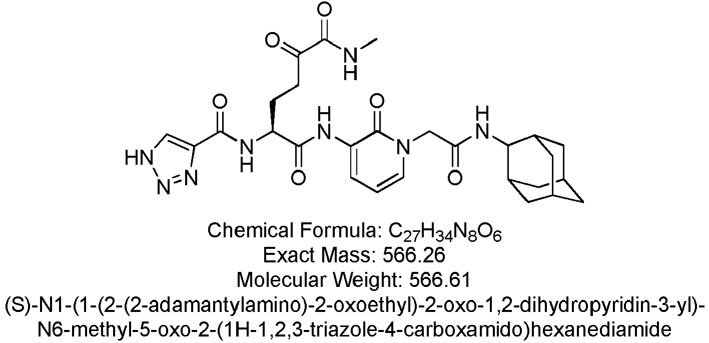

The synthesis of compound **II-132** was performed according to compound **II-3**, using 1H-1,2,3-triazole-4-carboxylic acid instead of 3-methylbenzo[b]furan-2-carboxylic acid in step 6 (according to ZED3264).
Yield: 14 mg, 45% (last step)
ESI-MS: 567.4 [M+H]⁺

### 123 Preparation of compound II-133

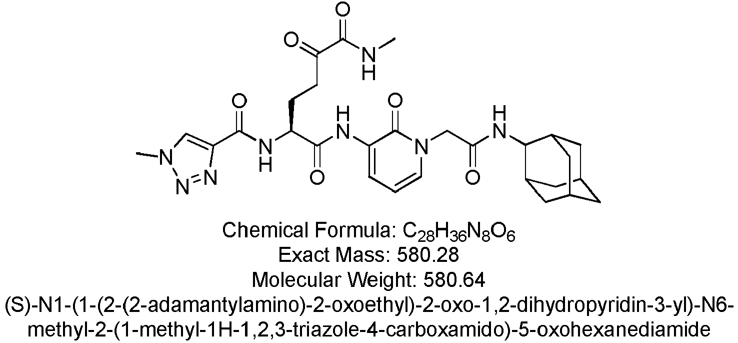

The synthesis of compound **II-133** was performed according to compound **II-3**, using 1-methyl-1H-1,2,3-triazole-4-carboxylic acid instead of 3-methylbenzo[b]furan-2-carboxylic acid in step 6 (according to ZED3264).
Yield: 36 mg, 61% (last step)
ESI-MS: 581.4 [M+H]⁺

### 124 Preparation of compound II-134

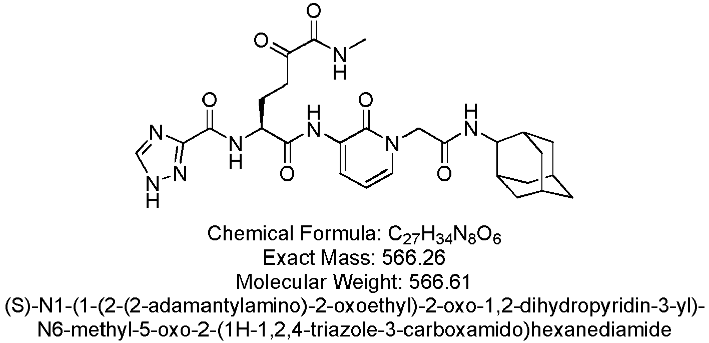

The synthesis of compound **II-134** was performed according to compound **II-3**, using 1H-1,2,4-triazole-3-carboxylic acid instead of 3-methylbenzo[b]furan-2-carboxylic acid in step 6 (according to ZED3264).
Yield: 21 mg, 43% (last step)
ESI-MS: 567.4 [M+H]⁺

### 125 Preparation of compound II-135

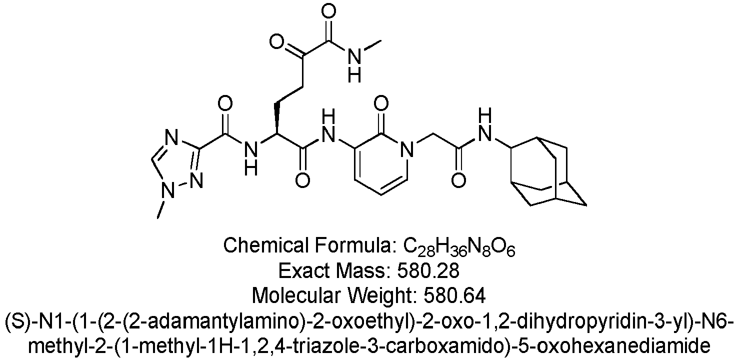

The synthesis of compound **II-135** was performed according to compound **II-3**, using 1-methyl-1H-1,2,4-triazole-3-carboxylic acid instead of 3-methylbenzo[b]furan-2-carboxylic acid in step 6 (according to ZED3264).
Yield: 39 mg, 67% (last step)
ESI-MS: 581.4 [M+H]⁺

### 126 Preparation of compound II-136

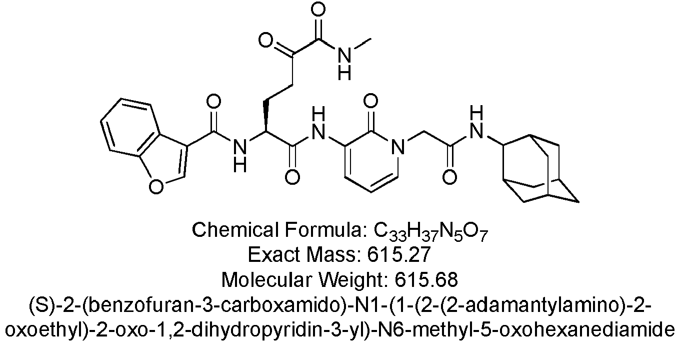

The synthesis of compound **II-136** was performed according to compound **II-3**, using benzofuran-3-carboxylic acid instead of 3-methylbenzo[b]furan-2-carboxylic acid in step 6 (according to ZED3264).
Yield: 56 mg, 71% (last step)
ESI-MS: 616.4 [M+H]⁺

### 127 Preparation of compound II-137

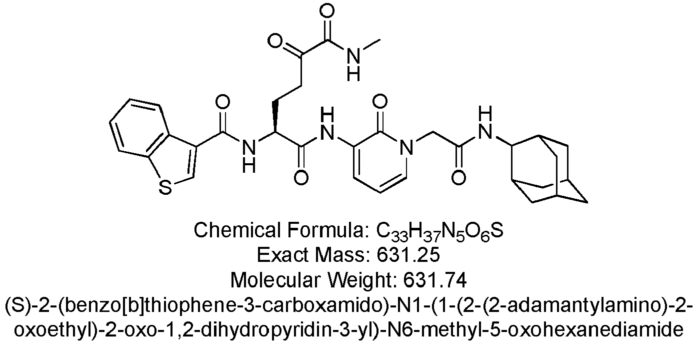

The synthesis of compound **II-137** was performed according to compound **II-3**, using benzo[b]thiophene-3-carboxylic acid instead of 3-methylbenzo[b]furan-2-carboxylic acid in step 6 (according to ZED3264).
Yield: 49 mg, 64% (last step)
ESI-MS: 632.4 [M+H]⁺

### 128 Preparation of compound II-138

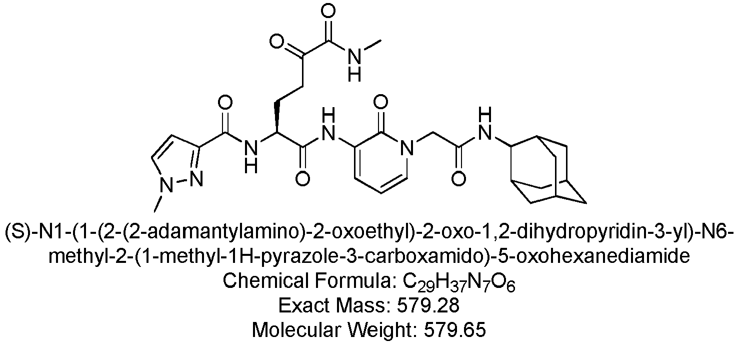

The synthesis of compound **II-138** was performed according to compound **II-3**, using 1-methyl-1H-pyrazole-3-carboxylic acid instead of 3-methylbenzo[b]furan-2-carboxylic acid in step 6 (according to ZED3264).
Yield: 43 mg, 59% (last step)
ESI-MS: 580.4 [M+H]⁺

### 129 Preparation of compound II-139

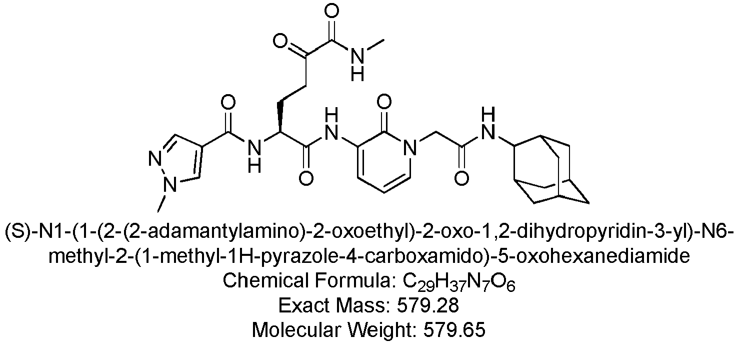

The synthesis of compound **II-139** was performed according to compound **II-3**, using 1-methyl-1H-pyrazole-4-carboxylic acid instead of 3-methylbenzo[b]furan-2-carboxylic acid in step 6 (according to ZED3264).
Yield: 53 mg, 62% (last step)
ESI-MS: 580.4 [M+H]⁺

### 130 Preparation of compound II-140

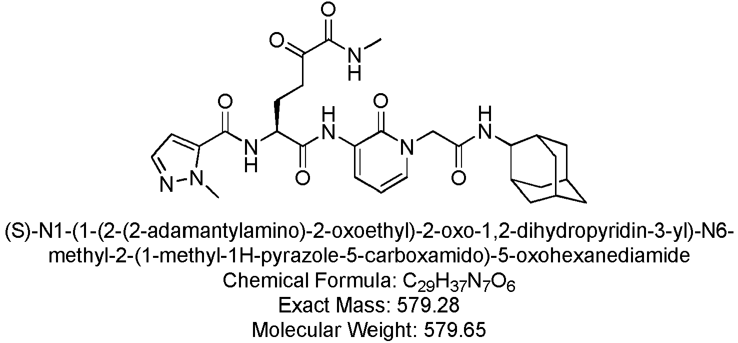

The synthesis of compound **II-140** was performed according to compound **II-3,** using 1-methyl-1H-pyrazole-5-carboxylic acid instead of 3-methylbenzo[b]furan-2-carboxylic acid in step 6 (according to ZED3264).
Yield: 39 mg, 48% (last step)
ESI-MS: 580.4 [M+H]⁺

### 131 Preparation of compound II-141

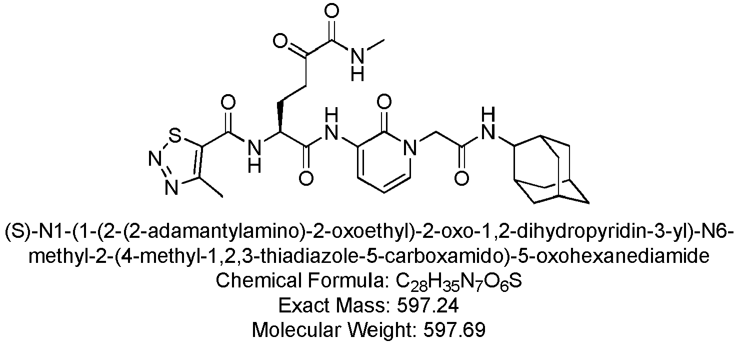

The synthesis of compound **II-141** was performed according to compound **II-3,** using 4-methyl-1,2,3-thiadiazole-5-carboxylic acid instead of 3-methylbenzo[b]furan-2-carboxylic acid in step 6 (according to ZED3264).
Yield: 54 mg, 58% (last step)
ESI-MS: 598.4 [M+H]⁺

### 132 Preparation of compound II-142

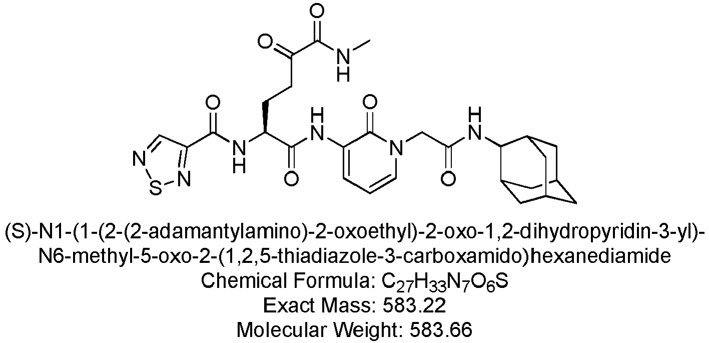

The synthesis of compound **II-142** was performed according to compound **II-3,** using 1,2,5-thiadiazole-3-carboxylic acid instead of 3-methylbenzo[b]furan-2-carboxylic acid in step 6 (according to ZED3264).
Yield: 36 mg, 43% (last step)
ESI-MS: 584.4 [M+H]⁺

### 133 Preparation of compound II-143

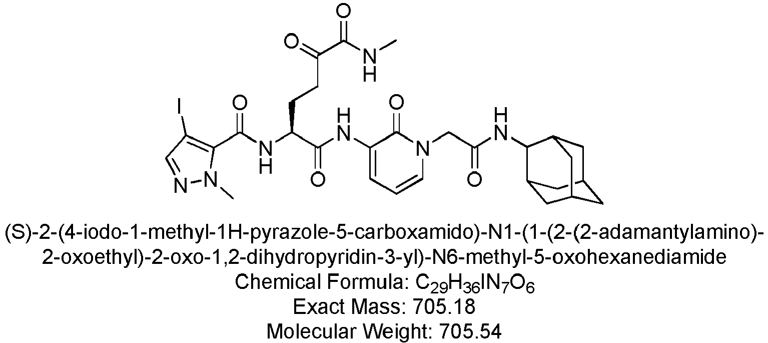

The synthesis of compound **II-143** was performed according to compound **II-3**, using 4-iodo-1-methyl-1H-pyrazole-5-carboxylic acid instead of 3-methylbenzo[b]furan-2-carboxylic acid in step 6 (according to ZED3264).
Yield: 24 mg, 49% (last step)
ESI-MS: 706.3 [M+H]⁺

### 134 Preparation of compound II-144

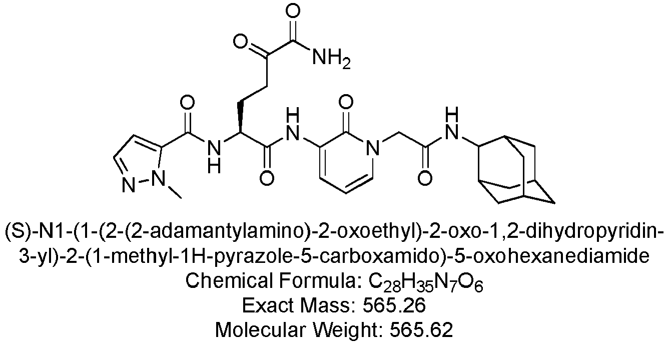

The synthesis of compound **II-144** was performed according to compound **II-118,** using 1-methyl-1H-pyrazole-5-carboxylic acid instead of 3-methylbenzo[b]furan-2-carboxylic acid in step 6 (according to ZED3264).
Yield: 26 mg, 68% (last step)
ESI-MS: 566.4 [M+H]⁺

### 135 Preparation of compound II-145

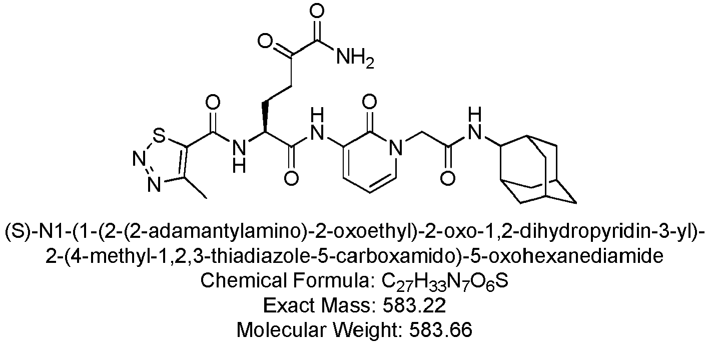

The synthesis of compound **II-145** was performed according to compound **II-118,** using 4-methyl-1,2,3-thiadiazole-5-carboxylic acid instead of 3-methylbenzo[b]furan-2-carboxylic acid in step 6 (according to ZED3264).
Yield: 35 mg, 62% (last step)
ESI-MS: 584.4 [M+H]⁺

### 136 Preparation of compound II-146

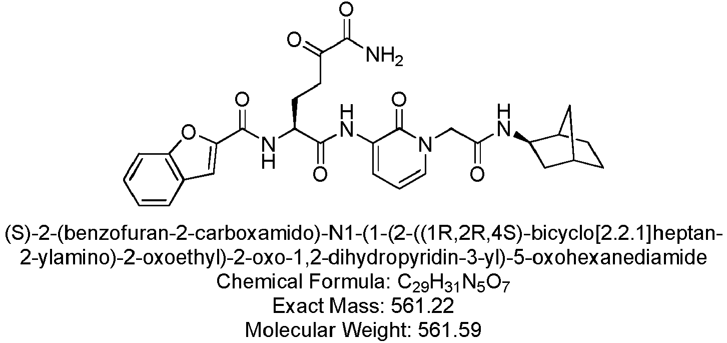

The synthesis of compound **II-146** was performed according to compound **II-118,** using benzofuran-2-carboxylic acid instead of 3-methylbenzo[b]furan-2-carboxylic acid in step 6 (according to ZED3264) and using exo-2-aminonorbornane instead of 2-adamantanamine in step 2 (according to ZED3905).
Yield: 43 mg, 60% (last step)
ESI-MS: 562.4 [M+H]⁺

### 137 Preparation of compound II-147

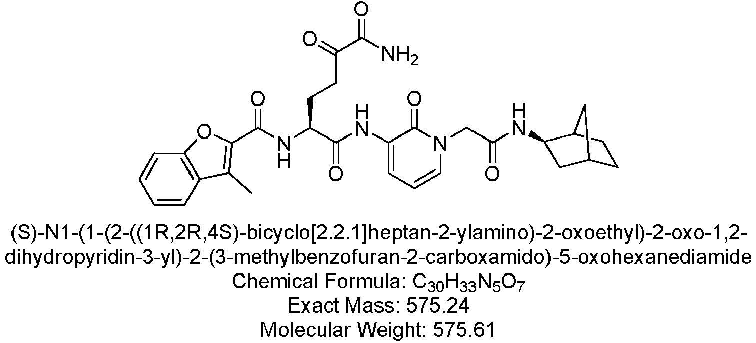

The synthesis of compound **II-147** was performed according to compound **II-118,** using using exo-2-aminonorbornane instead of 2-adamantanamine in step 2 (according to ZED3905).
Yield: 56 mg, 67% (last step)
ESI-MS: 576.4 [M+H]⁺

### 138 Preparation of compound II-148

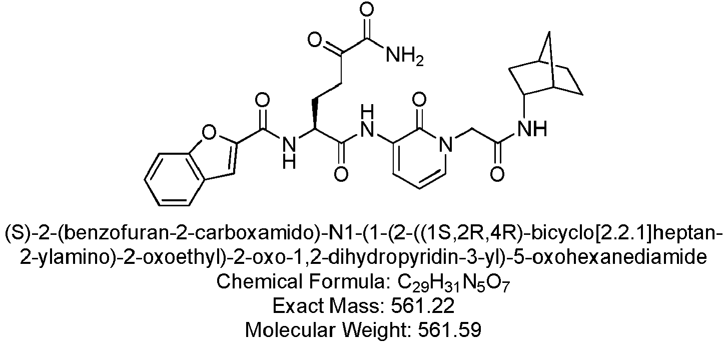

The synthesis of compound **II-148** was performed according to compound **II-118,** using benzofuran-2-carboxylic acid instead of 3-methylbenzo[b]furan-2-carboxylic acid in step 6 (according to ZED3264) and using (±)-endo-2-aminonorbornane instead of 2-adamantanamine in step 2 (according to ZED3905).
Yield: 49 mg, 66% (last step)
ESI-MS: 562.4 [M+H]⁺

### 139 Preparation of compound II-149

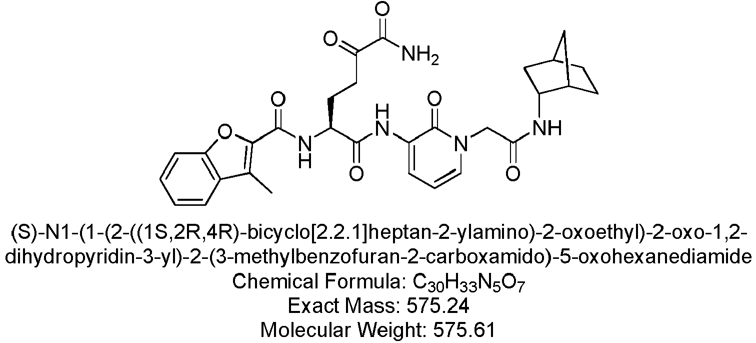

The synthesis of compound **II-149** was performed according to compound **II-118,** using using (±)-endo-2-aminonorbornane instead of 2-adamantanamine in step 2 (according to ZED3905).
Yield: 64 mg, 75% (last step)
ESI-MS: 576.4 [M+H]⁺

### 140 Preparation of compound II-150

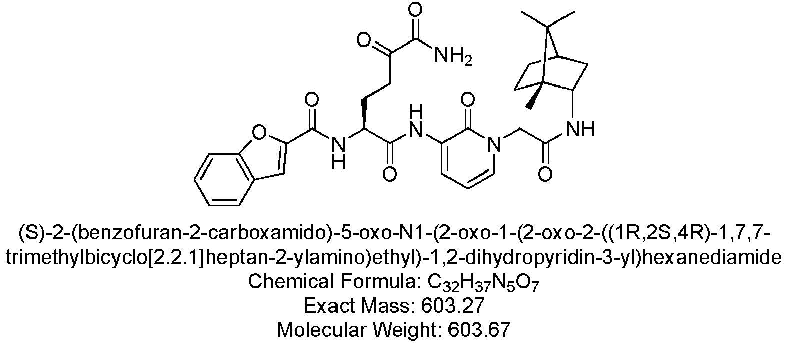

The synthesis of compound **II-150** was performed according to compound **II-118,** using benzofuran-2-carboxylic acid instead of 3-methylbenzo[b]furan-2-carboxylic acid in step 6 (according to ZED3264) and using (R)-(+)-bornylamine instead of 2-adamantanamine in step 2 (according to ZED3905).
Yield: 38 mg, 53% (last step)
ESI-MS: 604.4 [M+H]⁺

### 141 Preparation of compound II-151

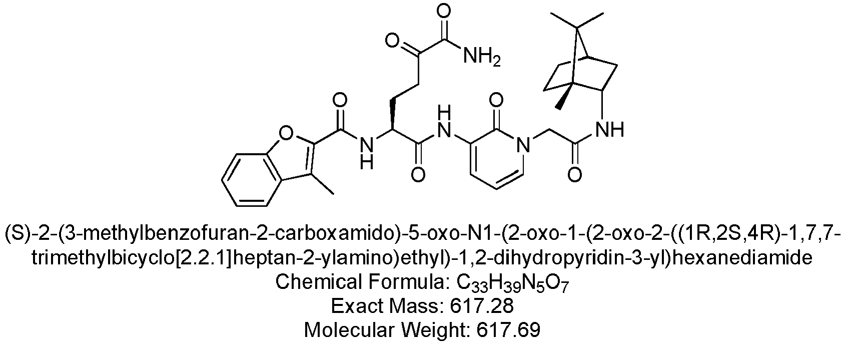

The synthesis of compound **II-151** was performed according to compound **II-118,** using using (R)-(+)-bornylamine instead of 2-adamantanamine in step 2 (according to ZED3905).
Yield: 31 mg, 59% (last step)
ESI-MS: 618.5 [M+H]⁺

### 142 Preparation of compound II-152

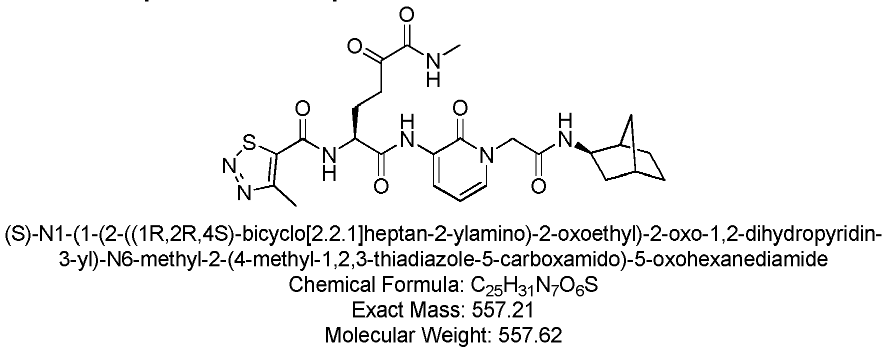

The synthesis of compound **II-152** was performed according to compound **II-94,** using 4-methyl-1,2,3-thiadiazole-5-carboxylic acid instead of 3-methylbenzo[b]furan-2-carboxylic acid in step 6 (according to ZED3264).
Yield: 53 mg, 68% (last step)
ESI-MS: 558.4 [M+H]⁺

### 143 Preparation of compound II-153

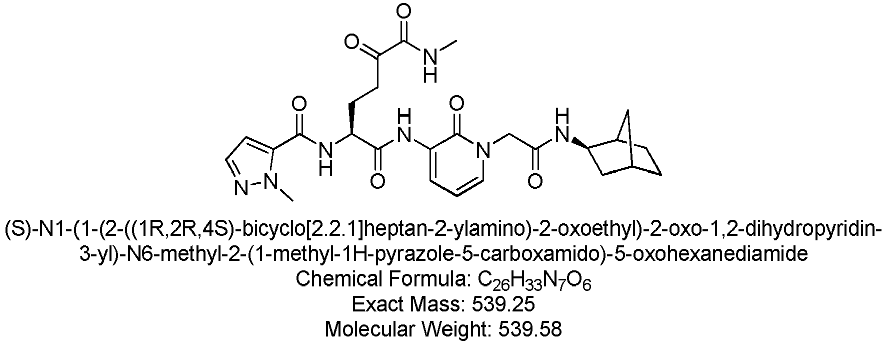

The synthesis of compound **II-153** was performed according to compound **II-94,** using 1-methyl-1H-pyrazole-5-carboxylic acid instead of 3-methylbenzo[b]furan-2-carboxylic acid in step 6 (according to ZED3264).
Yield: 60 mg, 67% (last step)
ESI-MS: 540.4 [M+H]⁺

### 144 Preparation of compound II-154

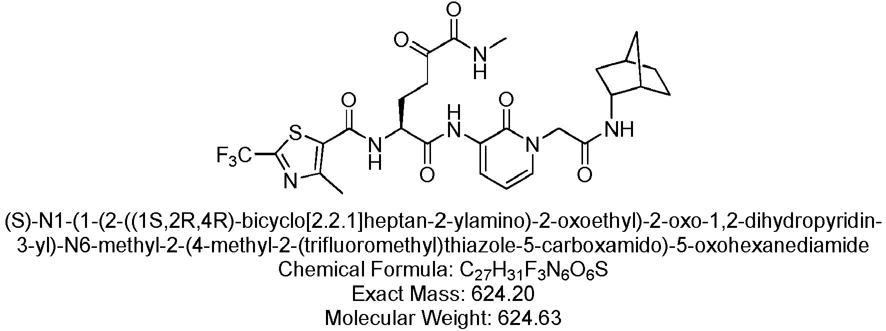

The synthesis of compound **II-154** was performed according to compound **II-90,** using 4-methyl-2-(trifluoromethyl)thiazole-5-carboxylic acid instead of 3-methylbenzo[b]furan-2-carboxylic acid in step 6 (according to ZED3264).
Yield: 68 mg, 74% (last step)
ESI-MS: 625.3 [M+H]⁺

### 145 Preparation of compound II-155

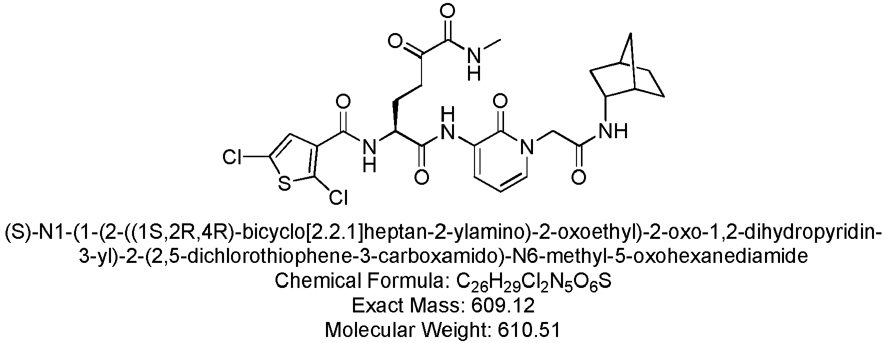

The synthesis of compound **II-155** was performed according to compound **II-90,** using 2,5-dichlorothiophene-3-carboxylic acid instead of 3-methylbenzo[b]furan-2-carboxylic acid in step 6 (according to ZED3264).
Yield: 74 mg, 68% (last step)
ESI-MS: 610.3 / 612.3 [M+H]⁺

### 146 Preparation of compound II-156

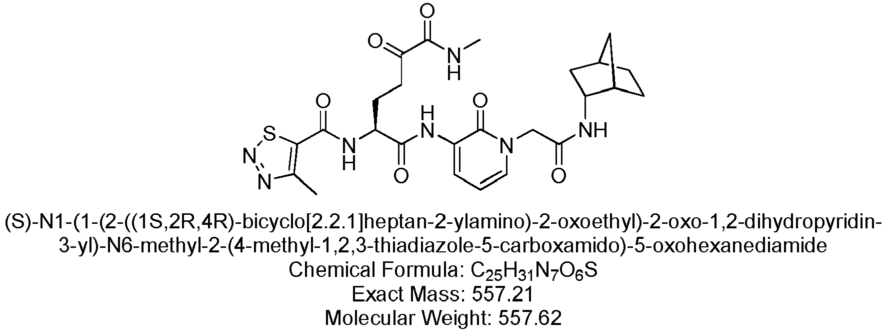

The synthesis of compound **II-156** was performed according to compound **II-90,** using 4-methyl-1,2,3-thiadiazole-5-carboxylic acid instead of 3-methylbenzo[b]furan-2-carboxylic acid in step 6 (according to ZED3264).
Yield: 56 mg, 61 % (last step)
ESI-MS: 558.4 [M+H]⁺

### 147 Preparation of compound II-157

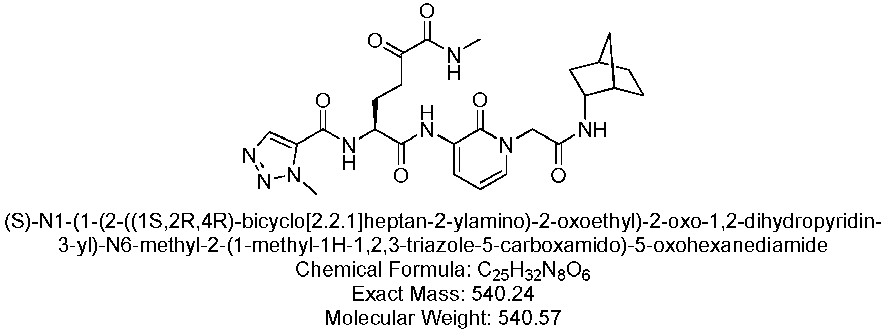

The synthesis of compound **II-157** was performed according to compound **II-90,** using 1-methyl-1H-1,2,3-triazole-5-carboxylic acid instead of 3-methylbenzo[b]furan-2-carboxylic acid in step 6 (according to ZED3264).
Yield: 34 mg, 52% (last step)
ESI-MS: 541.4 [M+H]⁺

### 148 Preparation of compound II-158

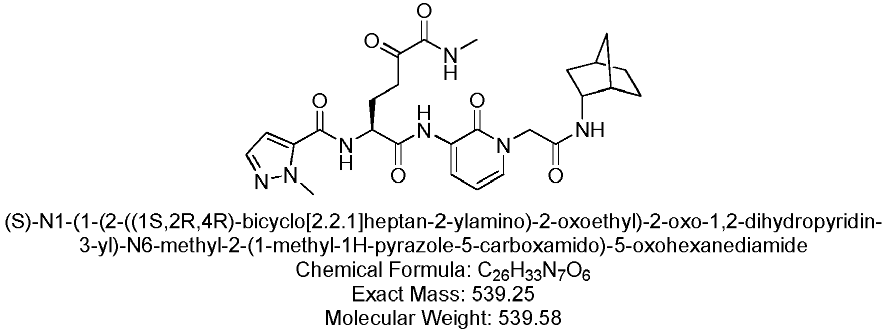

The synthesis of compound **II-158** was performed according to compound **II-90,** using 1-methyl-1H-pyrazole-5-carboxylic acid instead of 3-methylbenzo[b]furan-2-carboxylic acid in step 6 (according to ZED3264).
Yield: 55 mg, 64% (last step)
ESI-MS: 540.4 [M+H]⁺

### 149 Preparation of compound II-159

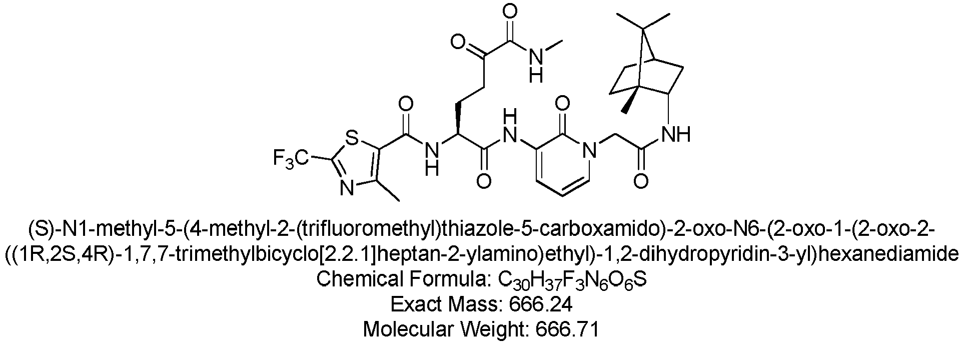

The synthesis of compound **II-159** was performed according to compound **II-92**, using 4-methyl-2-(trifluoromethyl)thiazole-5-carboxylic acid instead of 3-methylbenzo[b]furan-2-carboxylic acid in step 6 (according to ZED3264).
Yield: 52 mg, 57% (last step)
ESI-MS: 667.4 [M+H]⁺

### 150 Preparation of compound II-160

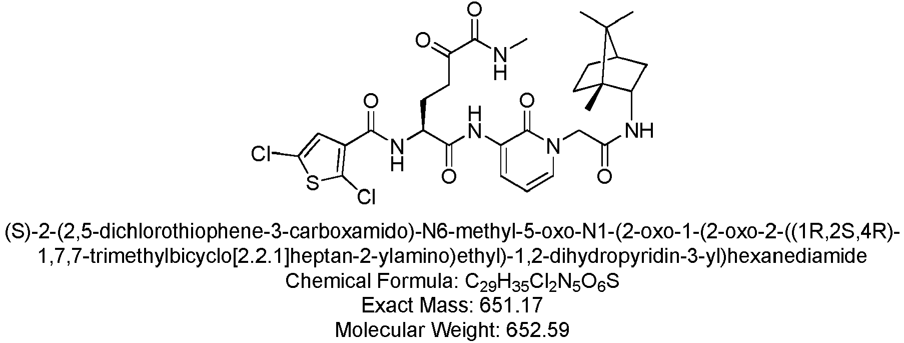

The synthesis of compound **II-160** was performed according to compound **II-92**, using 2,5-dichlorothiophene-3-carboxylic acid instead of 3-methylbenzo[b]furan-2-carboxylic acid in step 6 (according to ZED3264).
Yield: 66 mg, 61 % (last step)
ESI-MS: 652.3 / 654.3 [M+H]⁺

### 151 Preparation of compound II-161

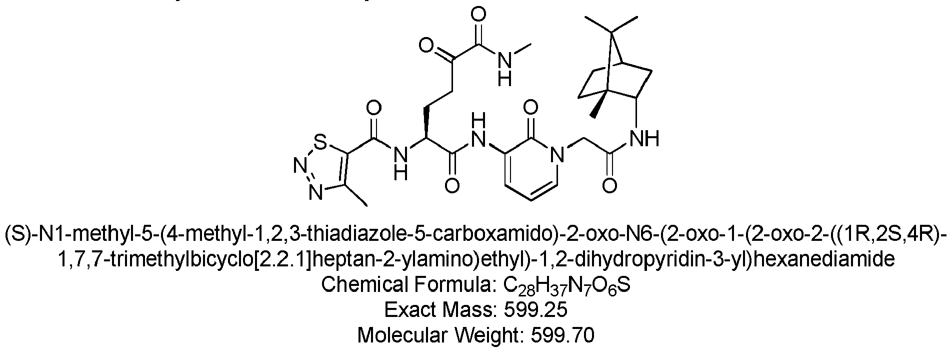

The synthesis of compound **II-161** was performed according to compound **II-92**, using 4-methyl-1,2,3-thiadiazole-5-carboxylic acid instead of 3-methylbenzo[b]furan-2-carboxylic acid in step 6 (according to ZED3264).
Yield: 41 mg, 51% (last step)
ESI-MS: 600.4 [M+H]⁺

### 152 Preparation of compound II-162

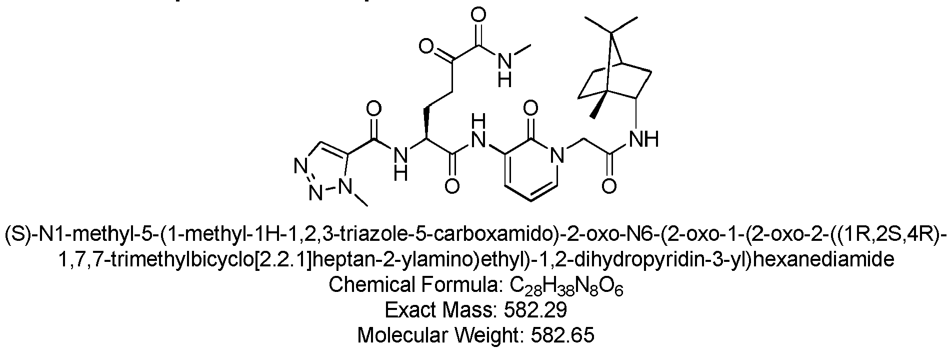

The synthesis of compound **II-162** was performed according to compound **II-92**, using 1-methyl-1H-1,2,3-triazole-5-carboxylic acid instead of 3-methylbenzo[b]furan-2-carboxylic acid in step 6 (according to ZED3264).
Yield: 28 mg, 46% (last step)
ESI-MS: 583.5 [M+H]⁺

### 153 Preparation of compound II-163

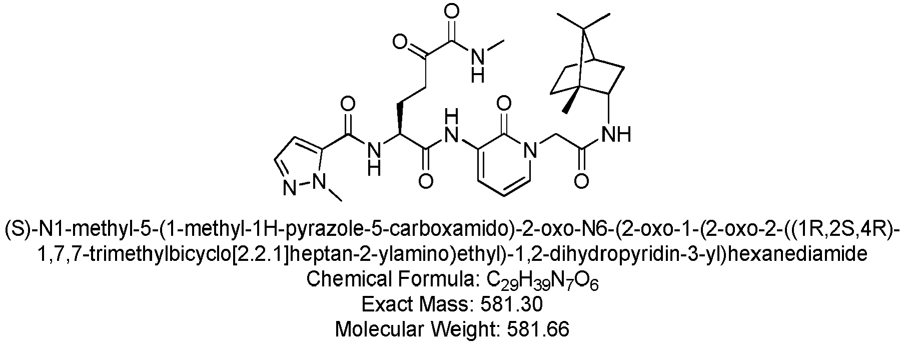

The synthesis of compound **II-163** was performed according to compound **II-92**, using 1-methyl-1H-pyrazole-5-carboxylic acid instead of 3-methylbenzo[b]furan-2-carboxylic acid in step 6 (according to ZED3264).
Yield: 44 mg, 58% (last step)
ESI-MS: 582.5 [M+H]⁺

### 154 Preparation of compound II-164

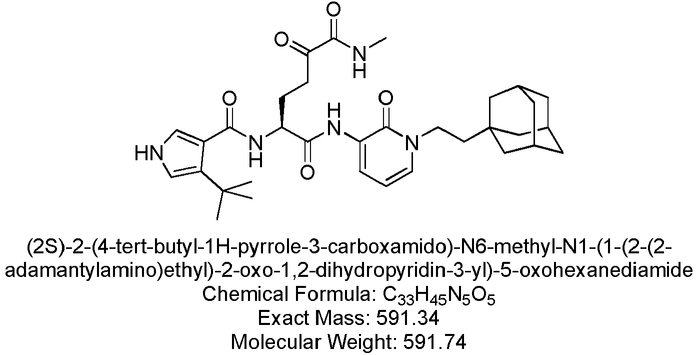

The synthesis of compound **II-164** was performed according to compound **II-107**, using 1-(2-bromoethyl)adamantane instead of 1-(bromomethyl)adamantane (according to ZED4893) and 5-tert-butyl-1H-pyrrole-3-carboxylic acid instead of 3-methyl-benzo[b]furan-2-carboxylic acid in step 6 (according to ZED3264).
Yield: 32 mg, 56% (last step)
ESI-MS: 592.5 [M+H]⁺

### 155 Preparation of compound II-165

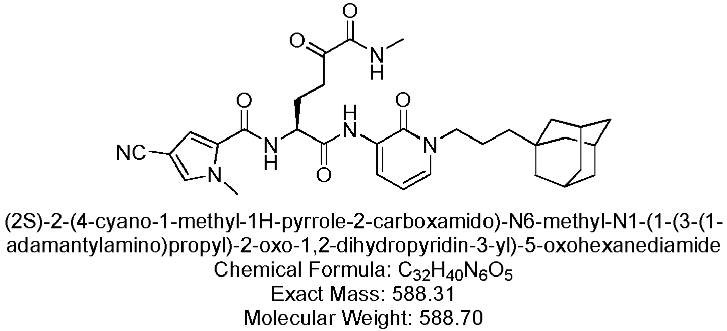

The synthesis of compound **II-165** was performed according to compound **II-107**, using 1-(3-bromopropyl)adamantane instead of 1-(bromomethyl)adamantane (according to ZED4893) and 4-cyano-1-methyl-1H-pyrrole-2-carboxylic acid instead of 3-methyl-benzo[b]furan-2-carboxylic acid in step 6 (according to ZED3264).
Yield: 27 mg, 48% (last step)
ESI-MS: 589.5 [M+H]⁺

### 156 Preparation of compound II-166

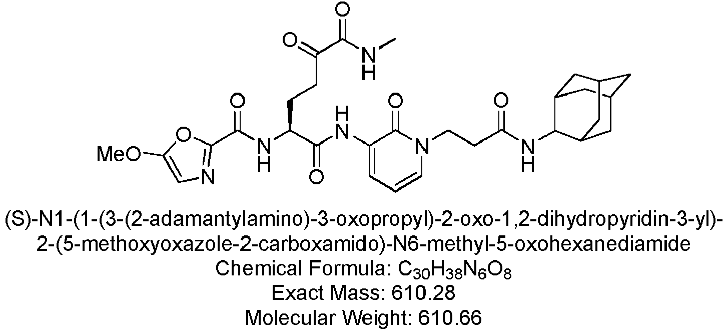

The synthesis of compound **II-166** was performed according to compound **II-3**, using 3-chloropropionic acid instead of chloroacetic acid (according to ZED1657) and 5-methoxyoxazole-2-carboxylic acid instead of 3-methylbenzo[b]furan-2-carboxylic acid in step 6 (according to ZED3264).
Yield: 31 mg, 59% (last step)
ESI-MS: 611.4 [M+H]⁺

### 157 Preparation of compound II-167

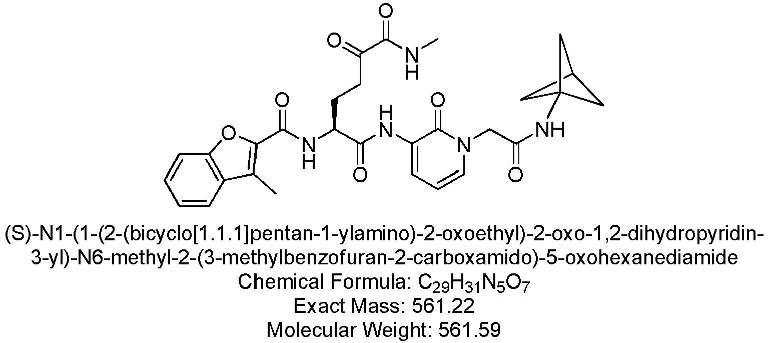

The synthesis of compound **II-167** was performed according to compound **II-3**, using 1-bicyclo[1.1.1]pentylamine instead of 2-adamantanamine in step 2 (according to ZED3905).
Yield: 45 mg, 67% (last step)
ESI-MS: 562.4 [M+H]⁺

### 158 Preparation of compound II-168

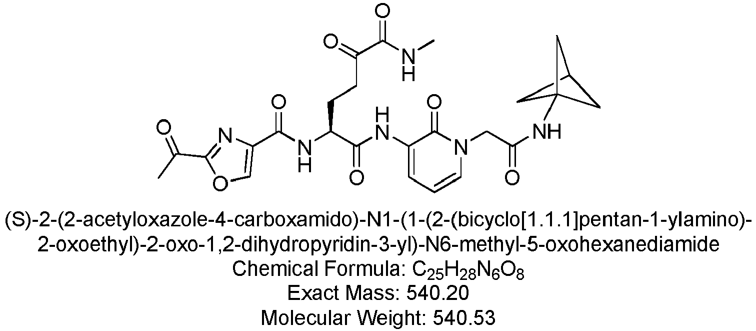

The synthesis of compound **II-168** was performed according to compound **II-167,** using 2-acetyloxazole-4-carboxylic acid instead of 3-methylbenzo[b]furan-2-carboxylic acid in step 6 (according to ZED3264).
Yield: 26 mg, 48% (last step)
ESI-MS: 541.4 [M+H]⁺

### 159 Preparation of compound II-169

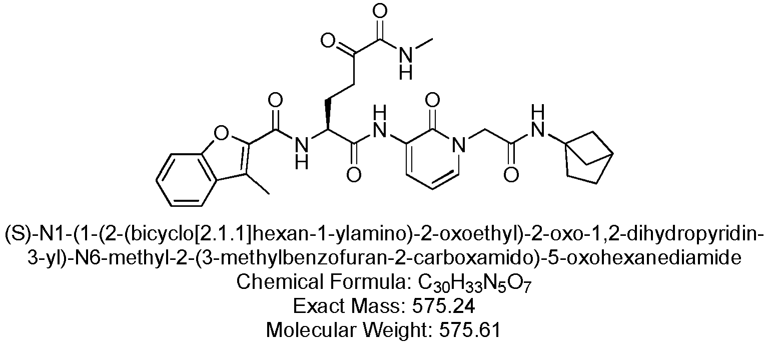

The synthesis of compound **II-169** was performed according to compound **II-3**, using bicyclo[2.1.1]hexan-1-amine instead of 2-adamantanamine in step 2 (according to ZED3905).
Yield: 33 mg, 61 % (last step)
ESI-MS: 576.4 [M+H]⁺

### 160 Preparation of compound II-170

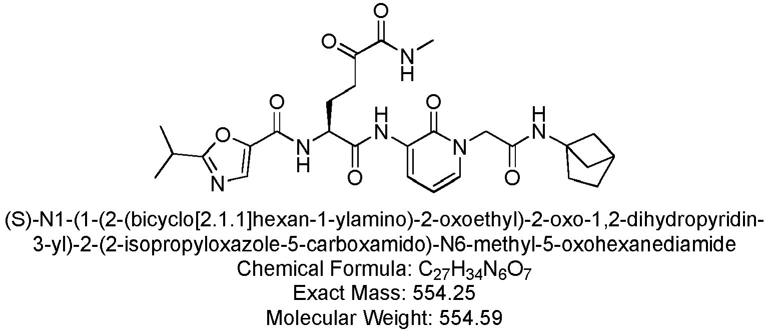

The synthesis of compound **II-170** was performed according to compound **II-169**, using 2-isopropyloxazole-5-carboxylic acid instead of 3-methylbenzo[b]furan-2-carboxylic acid in step 6 (according to ZED3264).
Yield: 32 mg, 54% (last step)
ESI-MS: 555.4 [M+H]⁺

### 161 Preparation of compound II-171

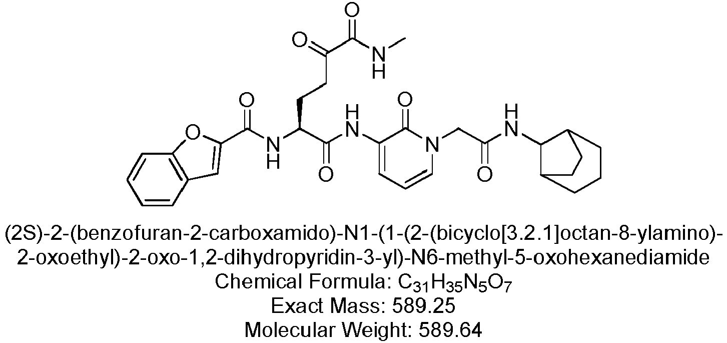

The synthesis of compound **II-171** was performed according to compound **II-2,** using bicyclo[3.2.1]octan-8-amine instead of 2-adamantanamine in step 2 (according to ZED3905).
Yield: 42 mg, 60% (last step)
ESI-MS: 590.4 [M+H]⁺

### 162 Preparation of compound II-172

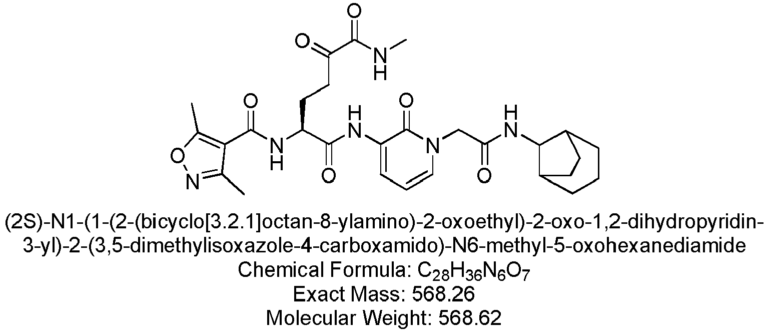

The synthesis of compound **II-172** was performed according to compound **II-171**, using 3,5-dimethylisoxazole-4-carboxylic acid instead of benzofuran-2-carboxylic acid in step 6 (according to ZED3264).
Yield: 35 mg, 58% (last step)
ESI-MS: 569.4 [M+H]⁺

### 163 Preparation of compound II-173

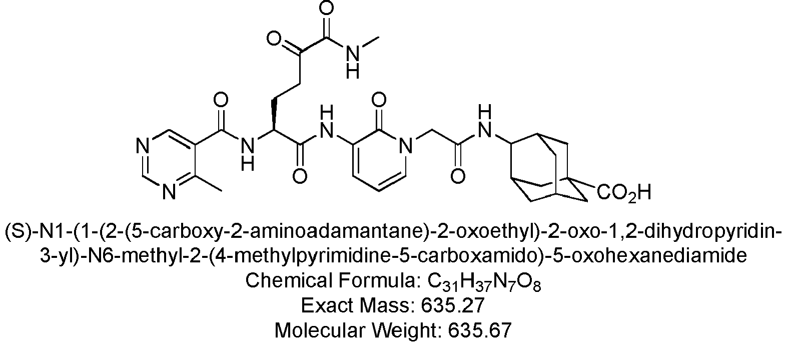

The synthesis of compound **II-173** was performed according to compound **II-3**, using 4-aminoadamantane-1-carboxylic acid instead of 2-adamantanamine in step 2 (according to ZED3905) and 4-methylpyrimidine-5-carboxylic acid instead of 3-methyl-benzo[b]furan-2-carboxylic acid in step 6 (according to ZED3264).
Yield: 25 mg, 49% (last step)
ESI-MS: 636.4 [M+H]⁺

### 164 Preparation of compound II-174

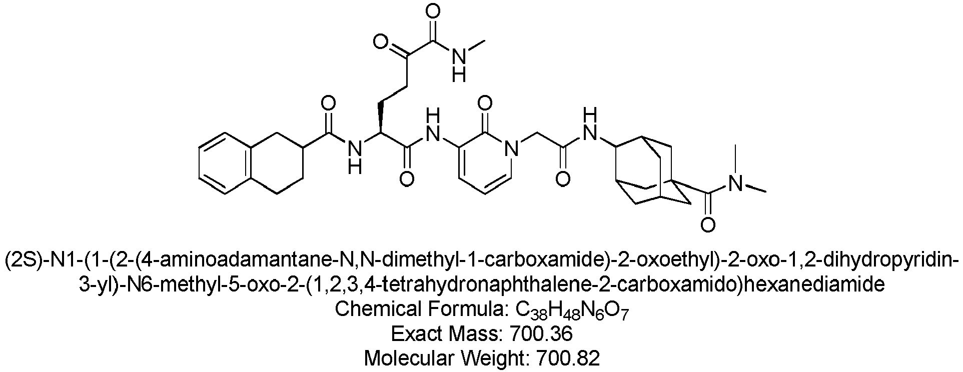

The synthesis of compound **II-174** was performed according to compound **II-3**, using 4-aminoadamantane-N,N-dimethyl-1-carboxamide instead of 2-adamantanamine in step 2 (according to ZED3905) and 1,2,3,4-tetrahydronaphthalene-2-carboxylic acid instead of 3-methylbenzo[b]furan-2-carboxylic acid in step 6 (according to ZED3264).
Yield: 33 mg, 56% (last step)
ESI-MS: 701.5 [M+H]⁺

### 165 Preparation of compound II-175

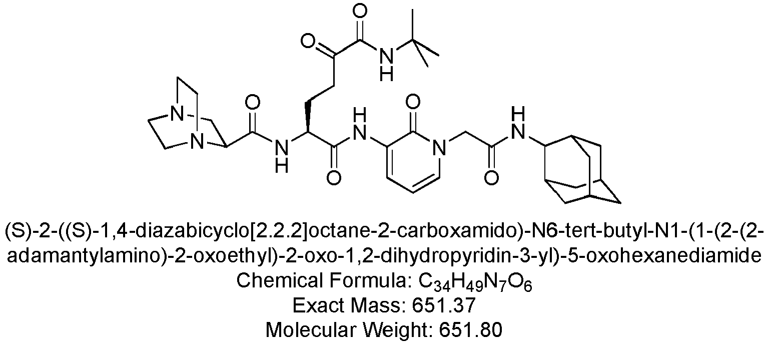

The synthesis of compound **II-175** was performed according to compound **II-3**, using tert-butyl isocyanide instead of methyl isocyanide in step 4 (according to ZED3632) and 1,4-diazabicyclo[2.2.2]octane-2-carboxylic acid instead of 3-methylbenzo[b]furan-2-carboxylic acid in step 6 (according to ZED3264).
Yield: 39 mg, 53% (last step)
ESI-MS: 652.5 [M+H]⁺

### 166 Preparation of compound II-176

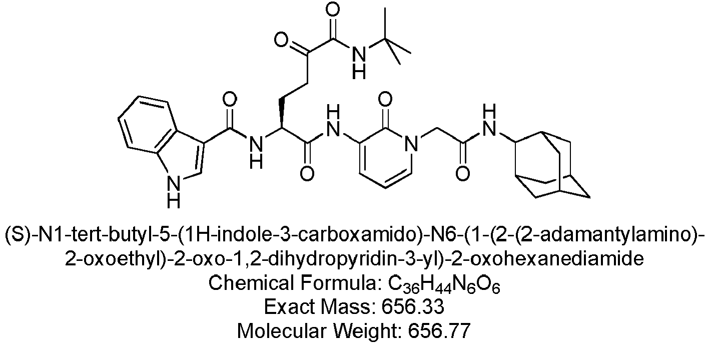

The synthesis of compound **II-176** was performed according to compound **II-3**, using tert-butyl isocyanide instead of methyl isocyanide in step 4 (according to ZED3632) and 1H-indole-3-carboxylic acid instead of 3-methylbenzo[b]furan-2-carboxylic acid in step 6 (according to ZED3264).
Yield: 52 mg, 62% (last step)
ESI-MS: 657.5 [M+H]⁺

### 167 Preparation of compound II-177

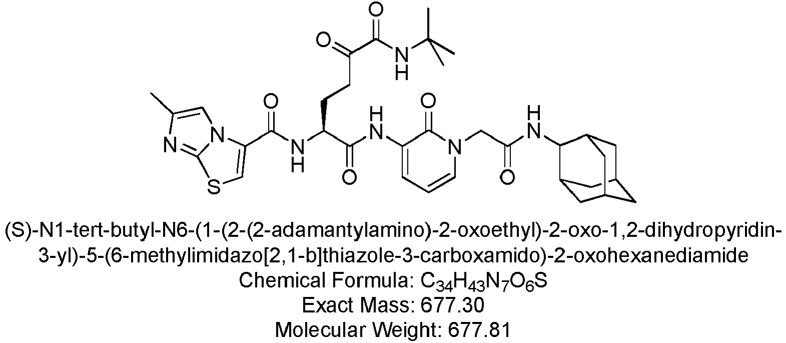

The synthesis of compound **II-177** was performed according to compound **II-3**, using tert-butyl isocyanide instead of methyl isocyanide in step 4 (according to ZED3632) and 6-methylimidazo[2,1-b][1,3]thiazole-3-carboxylic acid instead of 3-methylbenzo[b]furan-2-carboxylic acid in step 6 (according to ZED3264).
Yield: 42 mg, 53% (last step)
ESI-MS: 678.5 [M+H]⁺

### 168 Preparation of compound II-178

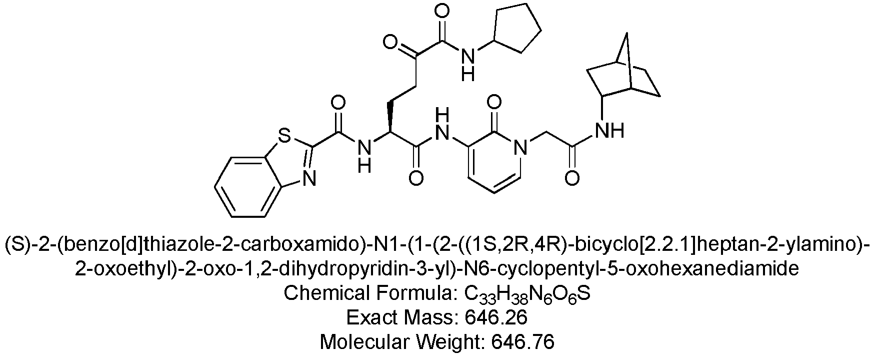

The synthesis of compound **II-178** was performed according to compound **II-90**, using cyclopentyl isocyanide instead of methyl isocyanide in step 4 (according to ZED3632) and 1,3-benzothiazole-2-carboxylic acid instead of 3-methylbenzo[b]furan-2-carboxylic acid in step 6 (according to ZED3264).
Yield: 54 mg, 61 % (last step)
ESI-MS: 647.4 [M+H]⁺

### 169 Preparation of compound II-179

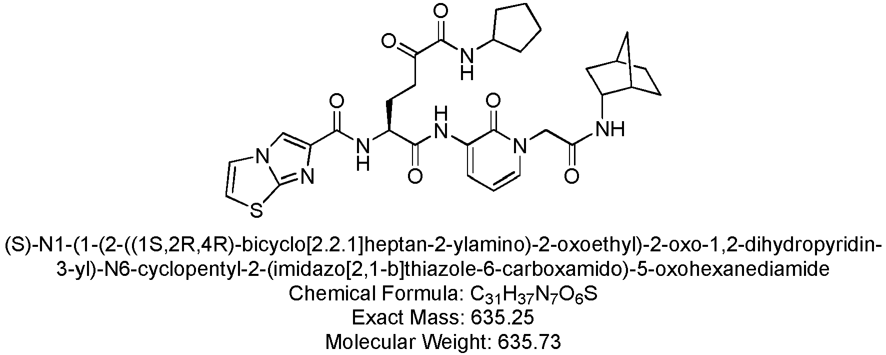

The synthesis of compound **II-179** was performed according to compound **II-90**, using cyclopentyl isocyanide instead of methyl isocyanide in step 4 (according to ZED3632) and imidazo[2,1-b][1,3]thiazole-6-carboxylic acid instead of 3-methylbenzo[b]furan-2-carboxylic acid in step 6 (according to ZED3264).
Yield: 36 mg, 51 % (last step)
ESI-MS: 636.4 [M+H]⁺

### 170 Preparation of compound II-180

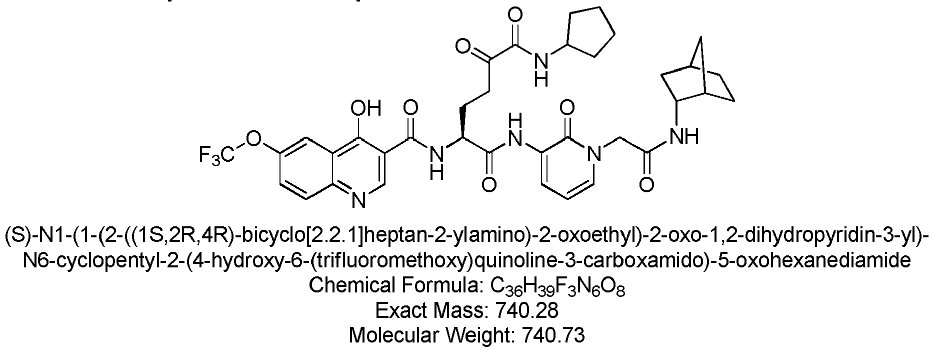

The synthesis of compound **II-180** was performed according to compound **II-90**, using cyclopentyl isocyanide instead of methyl isocyanide in step 4 (according to ZED3632) and 4-hydroxy-6-(trifluoromethoxy)quinoline-3-carboxylic acid instead of 3-methyl-benzo[b]furan-2-carboxylic acid in step 6 (according to ZED3264).
Yield: 19 mg, 41 % (last step)
ESI-MS: 741.5 [M+H]⁺

### 171 Preparation of compound II-181

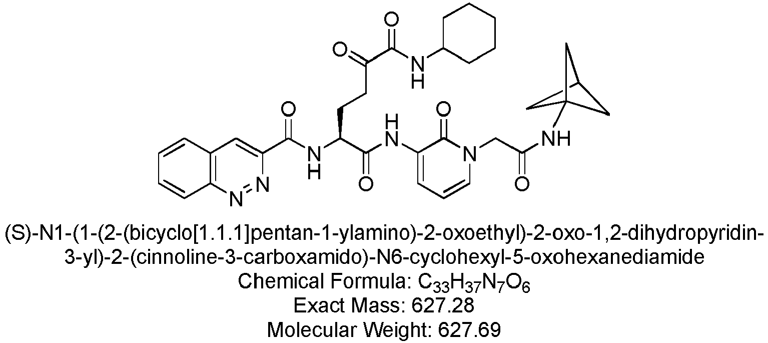

The synthesis of compound **II-181** was performed according to compound **II-167**, using cyclohexyl isocyanide instead of methyl isocyanide in step 4 (according to ZED3632) and 3-cinnolinecarboxylic acid instead of 3-methylbenzo[b]furan-2-carboxylic acid in step 6 (according to ZED3264).
Yield: 37 mg, 56% (last step)
ESI-MS: 628.5 [M+H]⁺

### 172 Preparation of compound II-182

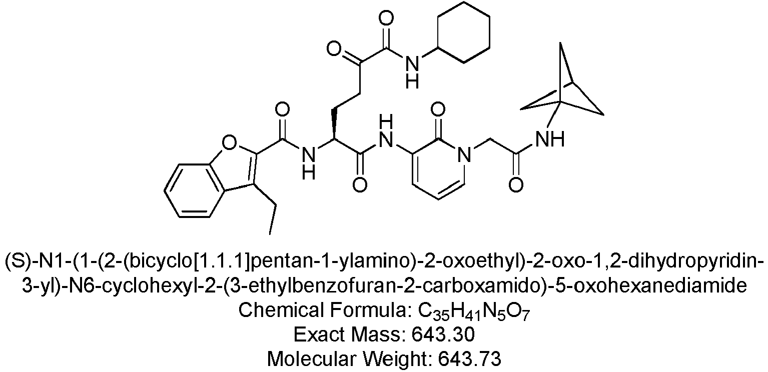

The synthesis of compound **II-182** was performed according to compound **II-167**, using cyclohexyl isocyanide instead of methyl isocyanide in step 4 (according to ZED3632) and 3-ethylbenzofuran-2-carboxylic acid instead of 3-methylbenzo[b]furan-2-carboxylic acid in step 6 (according to ZED3264).
Yield: 53 mg, 68% (last step)
ESI-MS: 644.5 [M+H]⁺

### 173 Preparation of compound II-183

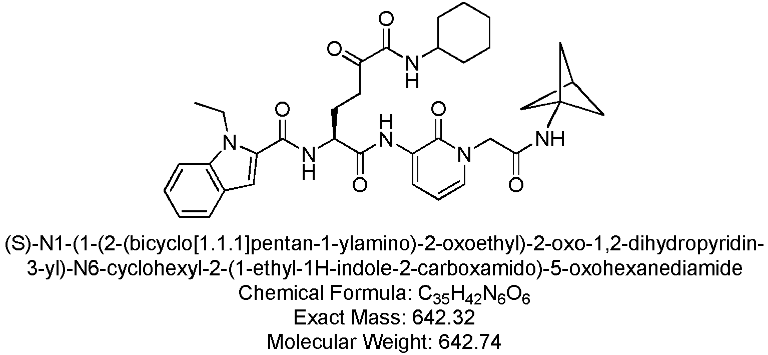

The synthesis of compound **II-183** was performed according to compound **II-167**, using cyclohexyl isocyanide instead of methyl isocyanide in step 4 (according to ZED3632) and 1-ethyl-1H-indole-2-carboxylic acid instead of 3-methylbenzo[b]furan-2-carboxylic acid in step 6 (according to ZED3264).
Yield: 41 mg, 57% (last step)
ESI-MS: 643.5 [M+H]⁺

### 174 Preparation of compound II-184

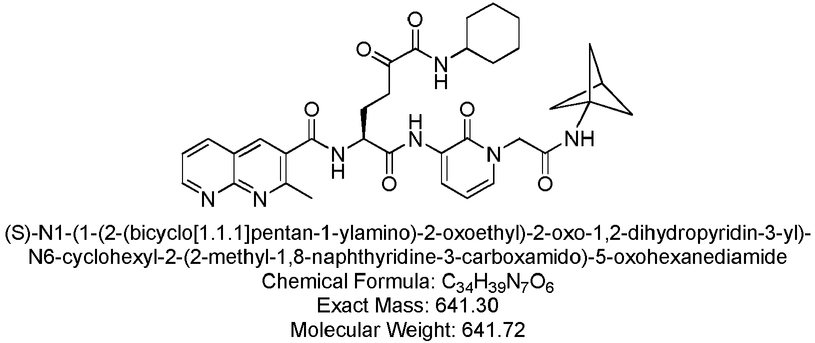

The synthesis of compound **II-184** was performed according to compound **II-167,** using cyclohexyl isocyanide instead of methyl isocyanide in step 4 (according to ZED3632) and 2-methyl-1,8-naphthyridine-3-carboxylic acid instead of 3-methylbenzo[b]furan-2-carboxylic acid in step 6 (according to ZED3264).
Yield: 26 mg, 54% (last step)
ESI-MS: 642.5 [M+H]⁺

### 175 Preparation of compound II-185

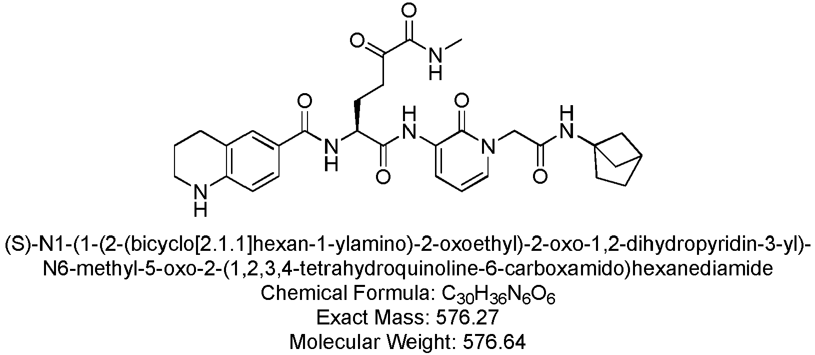

The synthesis of compound **II-185** was performed according to compound **II-169**, using N-Boc-1,2,3,4-tetrahydroquinoline-6-carboxylic acid instead of 3-methylbenzo[b]furan-2-carboxylic acid in step 6 (according to ZED3264). The final product was obtained by deprotection (DCM/TFA) as described above and purified by HPLC.
Yield: 39 mg, 61 % (last step)
ESI-MS: 577.4 [M+H]⁺

### 176 Preparation of compound II-186

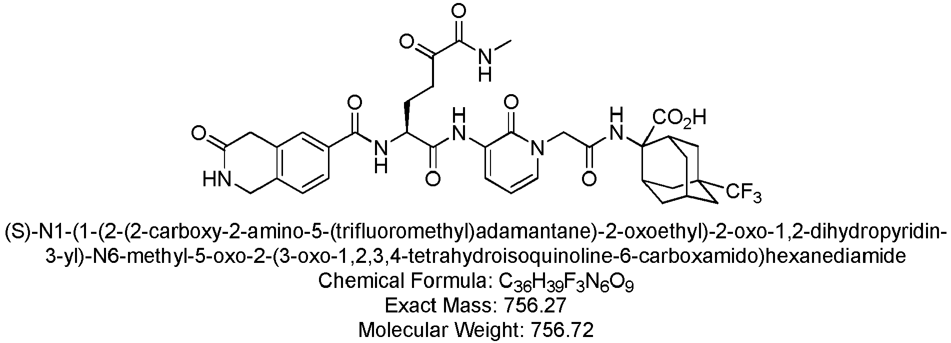

The synthesis of compound **II-186** was performed according to compound **II-3**, using 2-amino-5-(trifluoromethyl)adamantane-2-carboxylic acid instead of 2-adamantanamine in step 2 (according to ZED3905) and 3-oxo-1,2,3,4-tetrahydroisoquinoline-6-carboxylic acid instead of 3-methylbenzo[b]furan-2-carboxylic acid in step 6 (according to ZED3264).
Yield: 12 mg, 36% (last step)
ESI-MS: 757.4 [M+H]⁺

### 177 Preparation of compound II-187

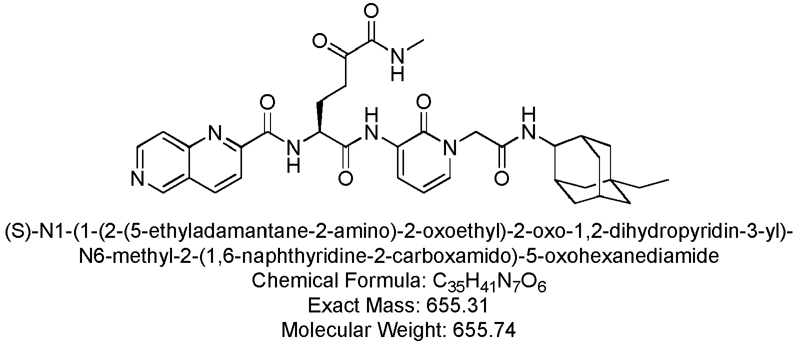

The synthesis of compound **II-187** was performed according to compound **II-3**, using 5-ethyladamantane-2-amine instead of 2-adamantanamine in step 2 (according to ZED3905) and 1,6-naphthyridine-2-carboxylic acid instead of 3-methylbenzo[b]furan-2-carboxylic acid in step 6 (according to ZED3264).
Yield: 26 mg, 51 % (last step)
ESI-MS: 656.5 [M+H]⁺

### 178 Preparation of compound II-188

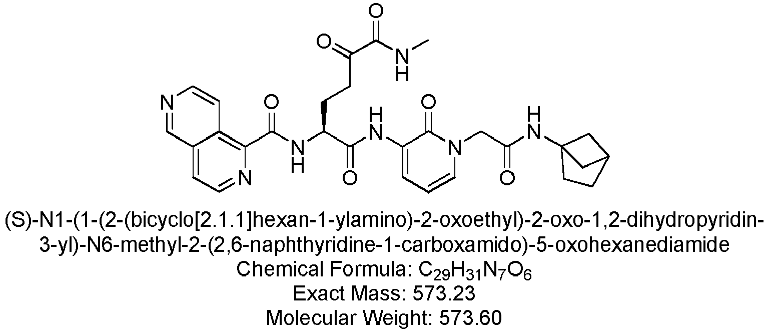

The synthesis of compound **II-188** was performed according to compound **II-169**, using 2,6-naphthyridine-1-carboxylic acid instead of 3-methylbenzo[b]furan-2-carboxylic acid in step 6 (according to ZED3264).
Yield: 33 mg, 56% (last step)
ESI-MS: 574.4 [M+H]⁺

### 179 Preparation of compound II-189

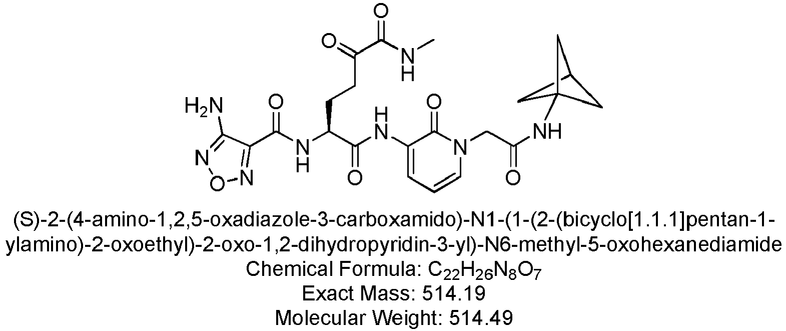

The synthesis of compound **II-189** was performed according to compound **II-167**, using 4-Boc-amino-1,2,5-oxadiazole-3-carboxylic acid instead of 3-methylbenzo[b]furan-2-carboxylic acid in step 6 (according to ZED3264). The amine was deprotected by TFA in the final step.
Yield: 16 mg, 69% (last step)
ESI-MS: 515.3 [M+H]⁺

### 180 Preparation of compound II-190

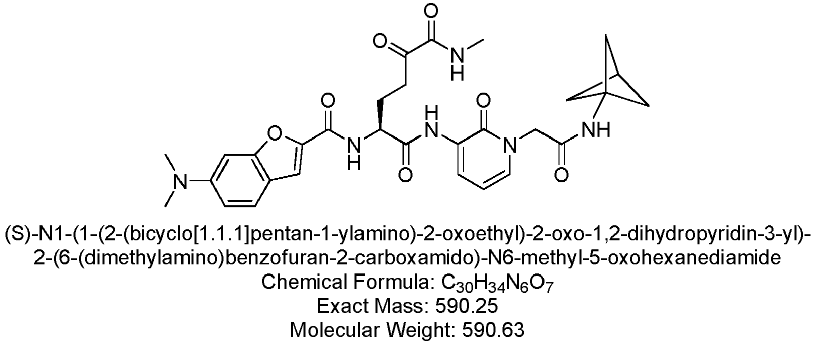

The synthesis of compound **II-190** was performed according to compound **II-167**, using 6-(dimethylamino)benzofuran-2-carboxylic acid instead of 3-methylbenzo[b]furan-2-carboxylic acid in step 6 (according to ZED3264).
Yield: 38 mg, 55% (last step)
ESI-MS: 591.4 [M+H]⁺

### 181 Preparation of compound II-191

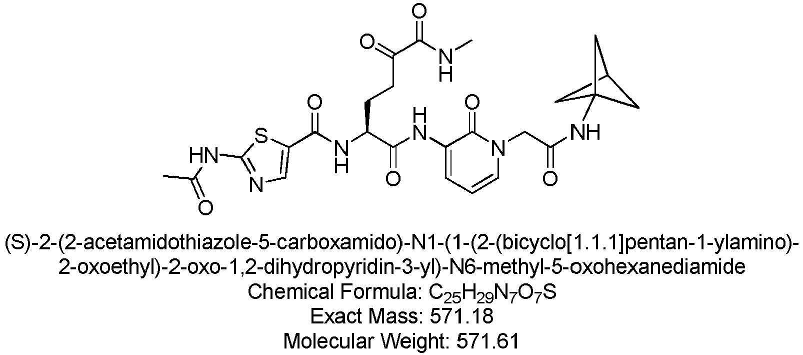

The synthesis of compound **II-191** was performed according to compound **II-167**, using 2-acetylamino-5-thiazolecarboxylic acid instead of 3-methylbenzo[b]furan-2-carboxylic acid in step 6 (according to ZED3264).
Yield: 21 mg, 46% (last step)
ESI-MS: 572.3 [M+H]⁺

### 182 Preparation of compound II-192

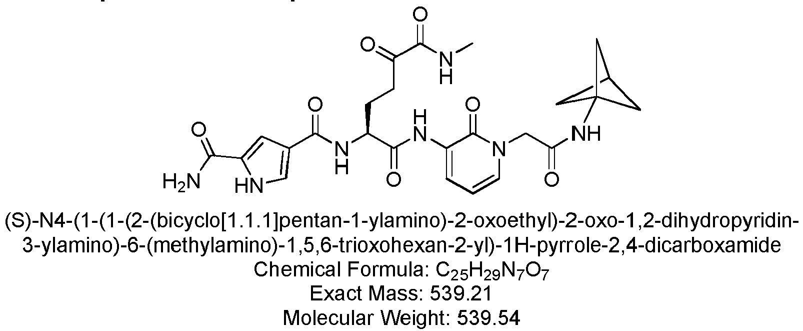

The synthesis of compound **II-192** was performed according to compound **II-167**, using 5-carbamoyl-1H-pyrrole-3-carboxylic acid instead of 3-methylbenzo[b]furan-2-carboxylic acid in step 6 (according to ZED3264).
Yield: 13 mg, 38% (last step)
ESI-MS: 540.4 [M+H]⁺

### 183 Preparation of compound II-193

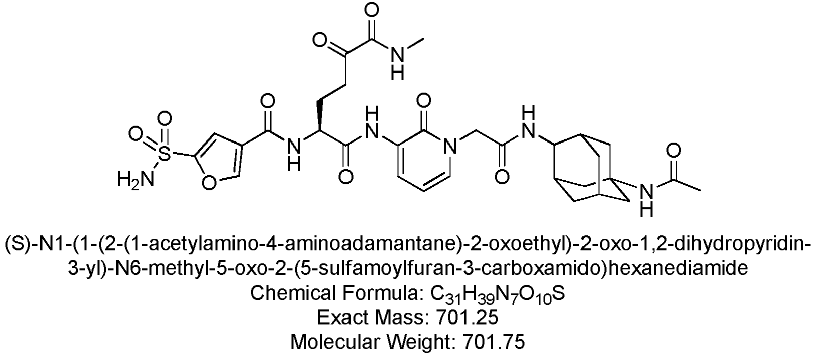

The synthesis of compound **II-193** was performed according to compound **II-3**, using 1-acetylamino-4-aminoadamantane instead of 2-adamantanamine in step 2 (according to ZED3905) and 5-sulfamoylfuran-3-carboxylic acid instead of 3-methylbenzo[b]furan-2-carboxylic acid in step 6 (according to ZED3264).
Yield: 10 mg, 27% (last step)
ESI-MS: 702.4 [M+H]⁺

### 184 Preparation of compound II-194

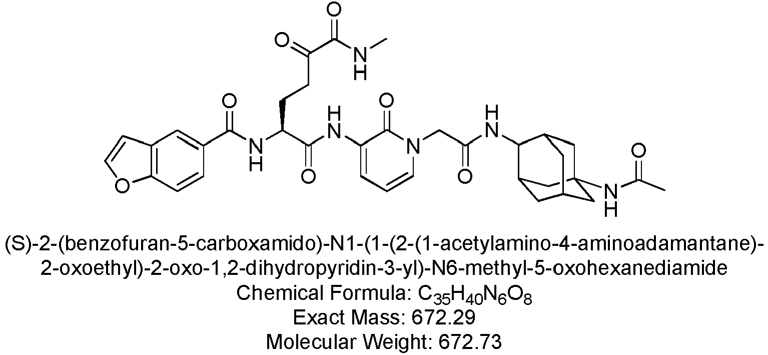

The synthesis of compound **II-194** was performed according to compound **II-3**, using 1-acetylamino-4-aminoadamantane instead of 2-adamantanamine in step 2 (according to ZED3905) and benzofuran-5-carboxylic acid instead of 3-methylbenzo[b]furan-2-carboxylic acid in step 6 (according to ZED3264).
Yield: 23 mg, 52% (last step)
ESI-MS: 673.5 [M+H]⁺

### 185 Preparation of compound II-195

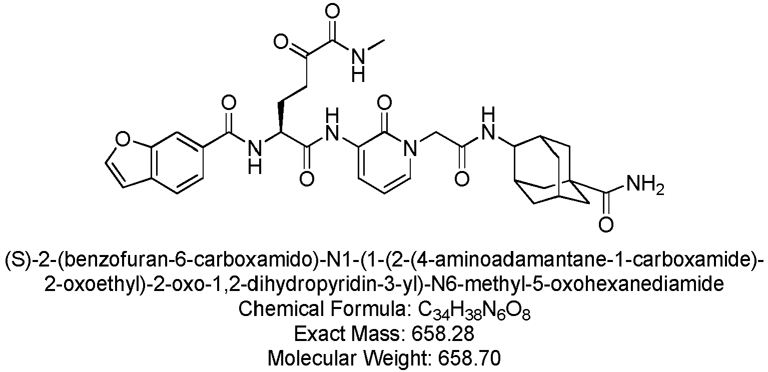

The synthesis of compound **II-195** was performed according to compound **II-3**, using 4-aminoadamantane-1-carboxamide instead of 2-adamantanamine in step 2 (according to ZED3905) and benzofuran-6-carboxylic acid instead of 3-methylbenzo[b]furan-2-carboxylic acid in step 6 (according to ZED3264).
Yield: 26 mg, 57% (last step)
ESI-MS: 659.4 [M+H]⁺

### 186 Preparation of compound II-196

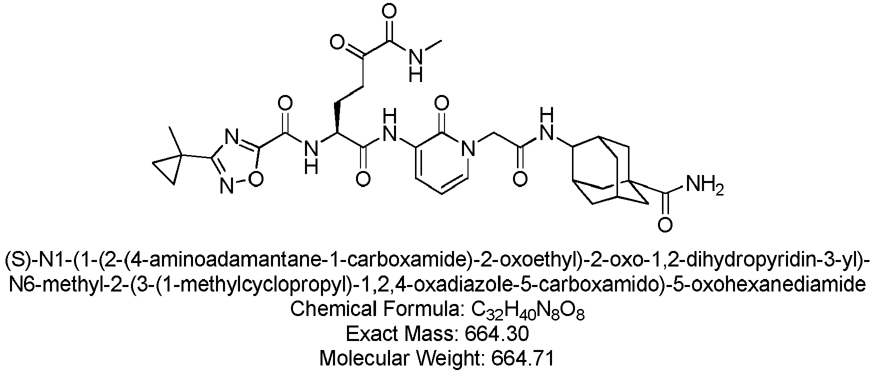

The synthesis of compound **II-196** was performed according to compound **II-3**, using 4-aminoadamantane-1-carboxamide instead of 2-adamantanamine in step 2 (according to ZED3905) and 3-(1-methylcyclopropyl)-1,2,4-oxadiazole-5-carboxylic acid instead of 3-methylbenzo[b]furan-2-carboxylic acid in step 6 (according to ZED3264).
Yield: 15 mg, 38% (last step)
ESI-MS: 665.5 [M+H]⁺

### 187 Preparation of compound II-197

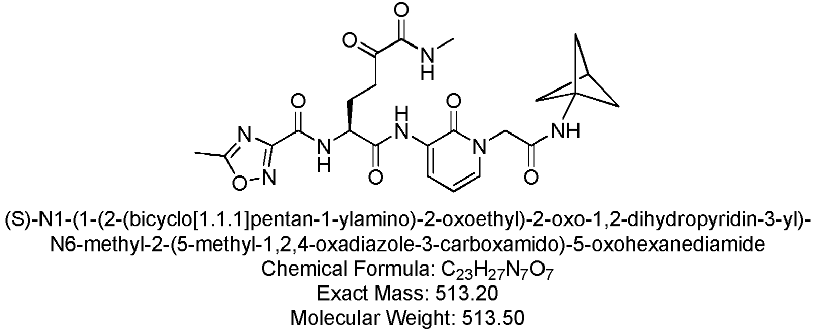

The synthesis of compound **II-197** was performed according to compound **II-167**, using 5-methyl-1,2,4-oxadiazole-3-carboxylic acid instead of 3-methylbenzo[b]furan-2-carboxylic acid in step 6 (according to ZED3264).
Yield: 36 mg, 58% (last step)
ESI-MS: 514.4 [M+H]⁺

### 188 Preparation of compound II-198

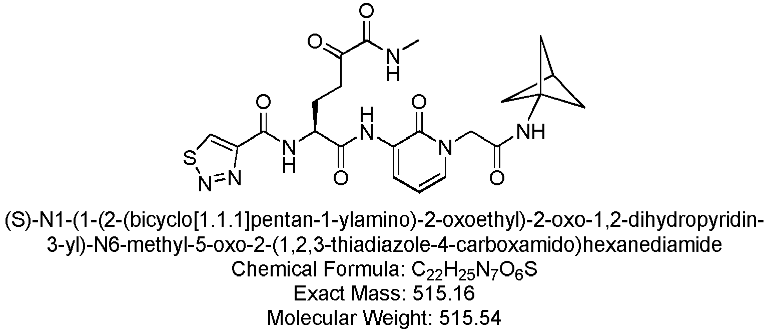

The synthesis of compound **II-198** was performed according to compound **II-167**, using 1,2,3-thiadiazole-4-carboxylic acid instead of 3-methylbenzo[b]furan-2-carboxylic acid in step 6 (according to ZED3264).
Yield: 29 mg, 48% (last step)
ESI-MS: 516.3 [M+H]⁺

### 189 Preparation of compound II-199

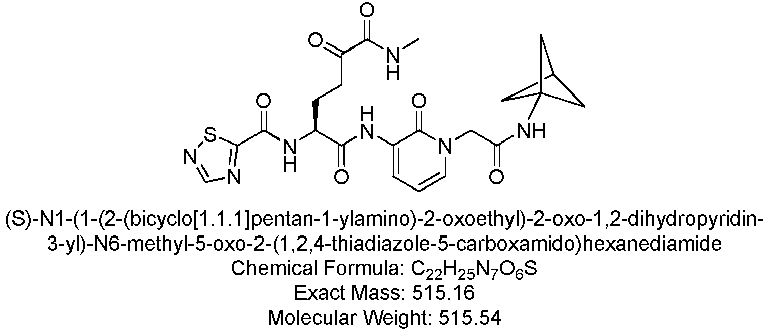

The synthesis of compound **II-199** was performed according to compound **II-167**, using 1,2,4-thiadiazole-5-carboxylic acid instead of 3-methylbenzo[b]furan-2-carboxylic acid in step 6 (according to ZED3264).
Yield: 35 mg, 56% (last step)
ESI-MS: 516.3 [M+H]⁺

### 190 Preparation of compound II-200

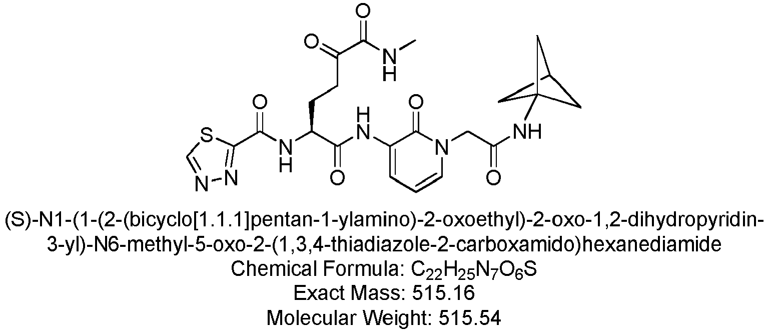

The synthesis of compound **II-200** was performed according to compound **II-167**, using 1,3,4-thiadiazole-2-carboxylic acid instead of 3-methylbenzo[b]furan-2-carboxylic acid in step 6 (according to ZED3264).
Yield: 24 mg, 41 % (last step)
ESI-MS: 516.3 [M+H]⁺

### 191 Preparation of compound II-201

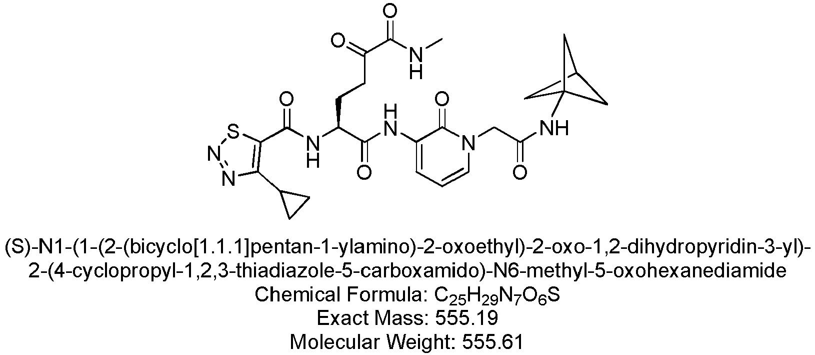

The synthesis of compound **II-201** was performed according to compound **II-167**, using 4-cyclopropyl-[1,2,3]thiadiazole-5-carboxylic acid instead of 3-methylbenzo[b]furan-2-carboxylic acid in step 6 (according to ZED3264).
Yield: 43 mg, 67% (last step)
ESI-MS: 556.4 [M+H]⁺

### 192 Preparation of compound II-202

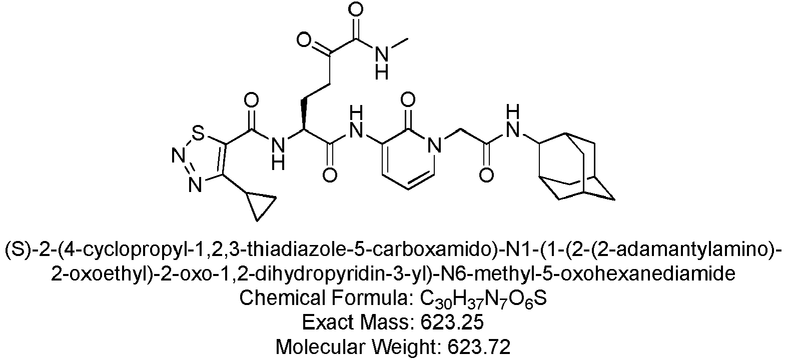

The synthesis of compound **II-202** was performed according to compound **II-3**, using 4-cyclopropyl-[1,2,3]thiadiazole-5-carboxylic acid instead of 3-methylbenzo[b]furan-2-carboxylic acid in step 6 (according to ZED3264).
Yield: 42 mg, 56% (last step)
ESI-MS: 624.4 [M+H]⁺

### 193 Preparation of compound II-203

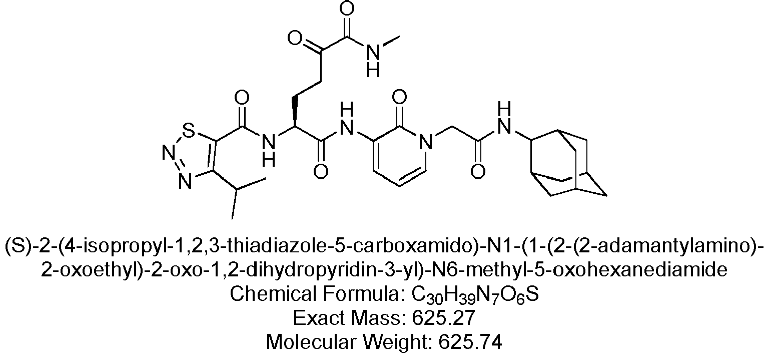

The synthesis of compound **II-203** was performed according to compound **II-3**, using 4-(propan-2-yl)-1,2,3-thiadiazole-5-carboxylic acid instead of 3-methylbenzo[b]furan-2-carboxylic acid in step 6 (according to ZED3264).
Yield: 36 mg, 52% (last step)
ESI-MS: 626.4 [M+H]⁺

### 194 Preparation of compound II-204

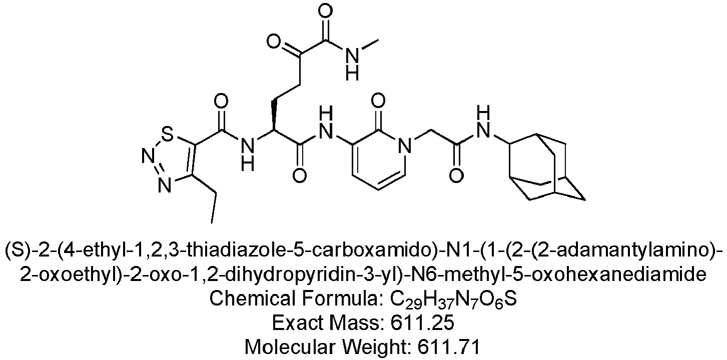

The synthesis of compound **II-204** was performed according to compound **II-3**, using 4-ethyl-1,2,3-thiadiazole-5-carboxylic acid instead of 3-methylbenzo[b]furan-2-carboxylic acid in step 6 (according to ZED3264).
Yield: 33 mg, 47% (last step)
ESI-MS: 612.4 [M+H]⁺

### 195 Preparation of compound II-205

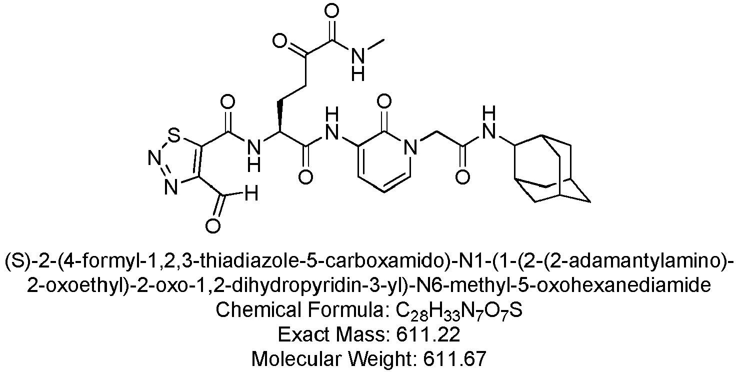

The synthesis of compound **II-205** was performed according to compound **II-3**, using 4-(hydroxymethyl)-1,2,3-thiadiazole-5-carboxylic acid instead of 3-methylbenzo[b]furan-2-carboxylic acid in step 6 (according to ZED3264).
Yield: 13 mg, 32% (last step)
ESI-MS: 612.4 [M+H]⁺

### 196 Preparation of compound II-206

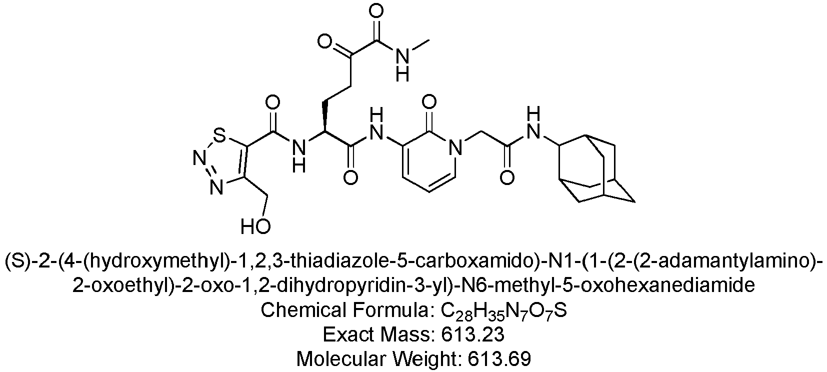

The synthesis of compound **II-206** was performed according to compound **II-3**, using 4-((tetrahydro-2H-pyran-2-yloxy)methyl)-1,2,3-thiadiazole-5-carboxylic acid instead of 3-methylbenzo[b]furan-2-carboxylic acid in step 6 (according to ZED3264). The tetrahydropyranyl (Thp) protecting group was cleaved by TFA in the final step.
Yield: 9 mg, 46% (last step)
ESI-MS: 614.4 [M+H]⁺

### 197 Preparation of compound II-207

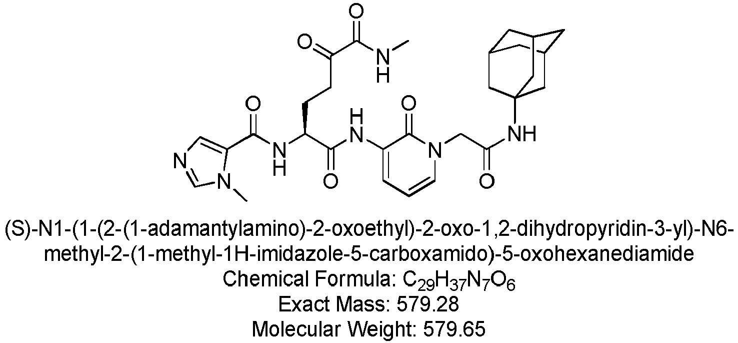

The synthesis of compound **II-207** was performed according to compound **II-3**, using 1-adamantanamine instead of 2-adamantanamine in step 2 (according to ZED3905) and 1-methyl-1H-imidazole-5-carboxylic acid instead of 3-methylbenzo[b]furan-2-carboxylic acid in step 6 (according to ZED3264).
Yield: 64 mg, 71 % (last step)
ESI-MS: 580.4 [M+H]⁺

### 198 Preparation of compound II-208

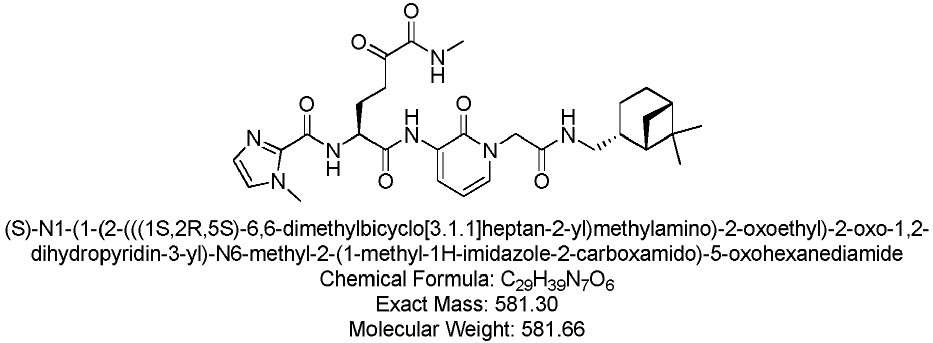

The synthesis of compound **II-208** was performed according to compound **II-3**, using (-)-cis-myrtanylamine instead of 2-adamantanamine in step 2 (according to ZED3905) and 1-methyl-1H-imidazole-2-carboxylic acid instead of 3-methylbenzo[b]furan-2-carboxylic acid in step 6 (according to ZED3264).
Yield: 43 mg, 59% (last step)
ESI-MS: 582.5 [M+H]⁺

### 199 Preparation of compound II-209

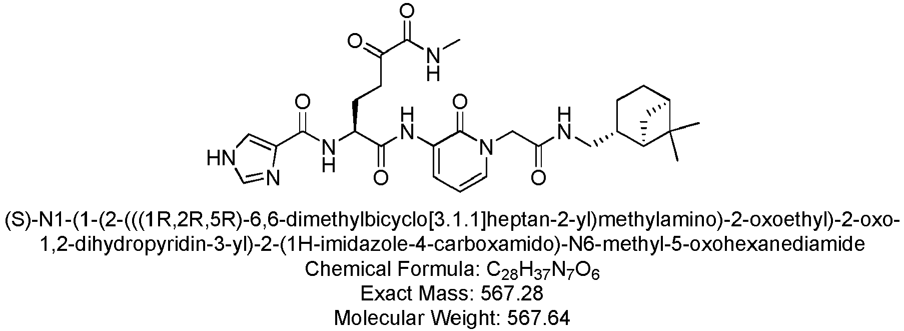

The synthesis of compound **II-209** was performed according to compound **II-3**, using (-)-cis-myrtanylamine instead of 2-adamantanamine in step 2 (according to ZED3905) and 1-methyl-1H-imidazole-2-carboxylic acid instead of 3-methylbenzo[b]furan-2-carboxylic acid in step 6 (according to ZED3264).
Yield: 31 mg, 55% (last step)
ESI-MS: 568.4 [M+H]⁺

### 200 Preparation of compound II-210

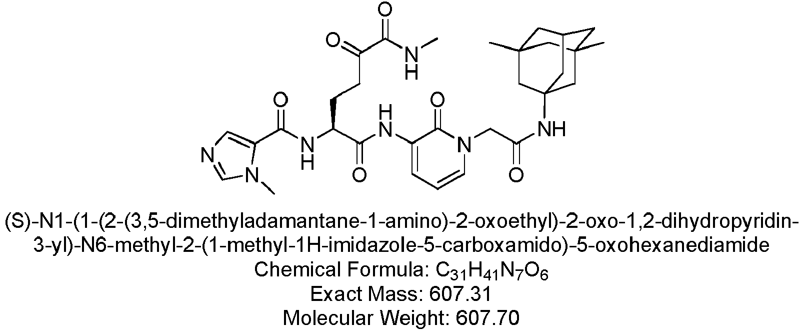

The synthesis of compound **II-210** was performed according to compound **II-3**, using 3,5-dimethyl-1-adamantanamine instead of 2-adamantanamine in step 2 (according to ZED3905) and 1-methyl-1H-imidazole-5-carboxylic acid instead of 3-methyl-benzo[b]furan-2-carboxylic acid in step 6 (according to ZED3264).
Yield: 46 mg, 66% (last step)
ESI-MS: 608.5 [M+H]⁺

### 201 Preparation of compound II-211

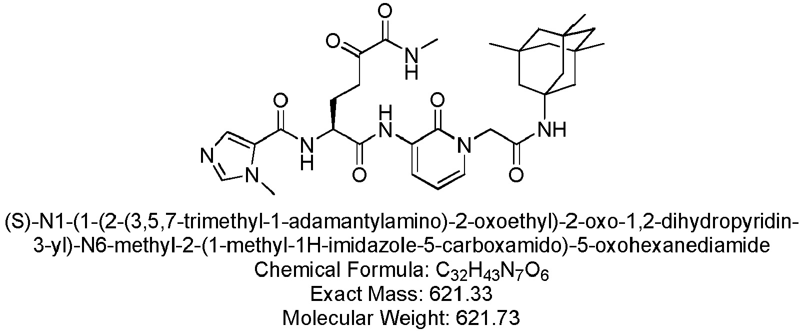

The synthesis of compound **II-211** was performed according to compound **II-3**, using 3,5,7-trimethyl-1-adamantanamine instead of 2-adamantanamine in step 2 (according to ZED3905) and 1-methyl-1H-imidazole-5-carboxylic acid instead of 3-methyl-benzo[b]furan-2-carboxylic acid in step 6 (according to ZED3264).
Yield: 53 mg, 70% (last step)
ESI-MS: 622.5 [M+H]⁺

### 202 Preparation of Reference Compound 6

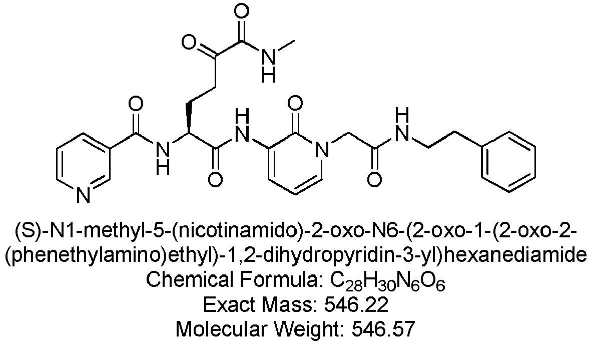

The synthesis of reference compound **6** was performed according to compound **II-3**, using 2-phenylethylamine instead of 2-adamantanamine in step 2 (according to ZED3905) and nicotinic acid instead of 3-methylbenzo[b]furan-2-carboxylic acid in step 6 (according to ZED3264).
Yield: 89 mg, 79% (last step)
ESI-MS: 547.4 [M+H]⁺

### Biological Examples

### Example B-1. inhibitory effect of the compounds according to the invention

### Transglutaminase assay

For the determination of potency of inhibitors against tissue transglutaminase, the incorporation of dansylcadaverine into dimethylcasein (Zedira product T036, Lorand et al., Anal Biochem, 1971, 44:221-31) was measured using recombinant human transglutaminase 2 (Zedira Product T022).

The tissue transglutaminase is diluted in buffer (50 mM Tris-HCl, 7.5 mM CaCl₂, 150 mM NaCl, pH = 7.4). The final concentration of TG2 in the assay is 10 nM.

A 10 mM inhibitor stock solution is prepared in DMSO, and from this a serial 1:2-fold dilution series is prepared also in DMSO. Each of the initial dilutions is subsequently diluted 1:50-fold with buffer (50 mM Tris-HCl, 7.5 mM CaCl₂, 150 mM NaCl, pH = 7.4) to yield the final working dilutions containing 2% (v/v) DMSO.

15 µl of inhibitor working dilution are added per well of a 96 well microtiter plate. As control, 15 µl of a 2% (v/v) DMSO solution prepared using the buffer mentioned above are added per well.

Immediately before starting the assay, 600 µl transglutaminase working solution are added to 11.4 ml assay buffer (50 mM Tris-HCl, 10 mM CaCl₂, 10 mM glutathione, 2.5% glycerol, 16.7 µM dansylcadaverine, 4 µM N,N-dimethylcasein, 200 mM NaCl, pH = 8.0). 285 µl of this reaction mix are added per well containing the inhibitor.

Increase in fluorescence is measured using λₑₓ = 330 nm and λₑₘ = 500 nm at 37°C for 30 min. A slope of the increase in fluorescence between 20 and 30 min is calculated for determination of the IC₅₀ value (inhibitor concentration at which 50% of the initial activity is blocked).

Analysis of enzymatic activity is performed by calculation of the slope of an increase in fluorescence intensity. IC₅₀ values are calculated by plotting the enzymatic activity (as percentage from control containing 2% DMSO instead of inhibitor) against the inhibitor concentration. IC₅₀ is defined as the inhibitor concentration blocking 50 % of initial enzyme activity.

The inhibitory activity of the inventive compounds in regard to tissue transglutaminase (TG2) is shown in the following table 1 using IC₅₀-values.

**Table 1. efficacy of reversible TG2 inhibitors**

| **A:** IC₅₀ < 40 nM, **B:** 40 nM ≤ IC₅₀ < 400 nM, **C:** 400 nM ≤ IC₅₀ < 2,500 nM, | | | |
|---|---|---|---|
| **D:** 2,500 nM ≤ IC₅₀ < 10,000 nM | | | |
| Compound | IC₅₀ TG2 [nM] | Compound | IC₅₀ TG2 [nM] |
| II-2 | A | II-117 | B |
| II-3 | A | II-118 | A |
| II-4 | A | II-119 | B |
| II-5 | A | II-120 | B |
| II-6 | A | II-121 | B |
| II-7 | A | II-122 | C |
| II-8 | A | II-123 | C |
| II-9 | A | II-124 | B |
| II-10 | A | II-125 | B |
| II-11 | A | II-126 | B |
| II-12 | B | II-127 | B |
| II-13 | A | II-128 | B |
| II-14 | A | II-129 | B |
| II-15 | A | II-130 | B |
| II-16 | A | II-131 | B |
| II-17 | B | II-132 | C |
| II-18 | B | II-133 | C |
| II-19 | B | II-134 | C |
| II-20 | C | II-135 | B |
| II-21 | A | II-136 | B |
| II-22 | A | II-137 | B |
| II-23 | B | II-138 | C |
| II-24 | B | II-139 | C |
| II-25 | B | II-140 | B |
| II-26 | B | II-141 | B |
| II-27 | B | II-142 | B |
| II-28 | A | II-143 | C |
| II-29 | A | II-144 | C |
| II-30 | A | II-145 | B |
| II-31 | B | II-146 | A |
| II-32 | B | II-147 | A |
| II-33 | B | II-148 | A |
| II-34 | B | II-149 | A |
| II-35 | B | II-150 | B |
| II-36 | B | II-151 | B |
| II-37 | B | II-152 | B |
| II-38 | C | II-153 | C |
| II-39 | B | II-154 | B |
| II-40 | A | II-155 | A |
| II-41 | B | II-156 | B |
| II-42 | B | II-157 | B |
| II-43 | C | II-158 | B |
| II-44 | A | II-159 | B |
| II-45 | A | II-160 | B |
| II-46 | B | II-161 | B |
| II-47 | A | II-162 | B |
| II-48 | A | II-163 | B |
| II-49 | A | II-164 | C |
| II-50 | A | II-165 | C |
| II-51 | B | II-166 | C |
| II-52 | C | II-167 | A |
| II-53 | B | II-168 | B |
| II-54 | B | II-169 | A |
| II-55 | B | II-170 | B |
| II-56 | B | II-171 | A |
| II-58 | B | II-172 | B |
| II-59 | B | II-173 | B |
| II-60 | B | II-174 | B |
| II-61 | B | II-175 | C |
| II-62 | A | II-176 | C |
| II-63 | A | II-177 | C |
| II-64 | B | II-178 | B |
| II-65 | B | II-179 | C |
| II-66 | C | II-180 | C |
| II-67 | C | II-181 | C |
| II-68 | B | II-182 | B |
| II-69 | A | II-183 | B |
| II-70 | A | II-184 | C |
| II-71 | A | II-185 | C |
| II-72 | A | II-186 | C |
| II-73 | B | II-187 | C |
| II-74 | C | II-188 | C |
| II-87 | B | II-189 | C |
| II-88 | B | II-190 | C |
| II-90 | B | II-191 | C |
| II-92 | B | II-192 | C |
| II-94 | A | II-193 | C |
| II-95 | B | II-194 | B |
| II-96 | B | II-195 | B |
| II-97 | B | II-196 | C |
| II-98 | B | II-197 | C |
| II-99 | B | II-198 | B |
| II-100 | B | II-199 | B |
| II-101 | B | II-200 | B |
| II-103 | B | II-201 | B |
| II-104 | B | II-202 | B |
| II-105 | B | II-203 | B |
| II-107 | B | II-204 | B |
| II-108 | B | II-205 | B |
| II-109 | B | II-206 | B |
| II-110 | B | II-207 | B |
| II-111 | A | II-208 | C |
| II-112 | A | II-209 | C |
| II-113 | B | II-210 | B |
| II-114 | B | II-211 | B |
| II-115 | B | Ref. 1 | C |
| II-116 | B | Ref. 2 | B |
| Ref. 6 | D | Ref. 3 | D |

### Ref. 1 (ZED3641)

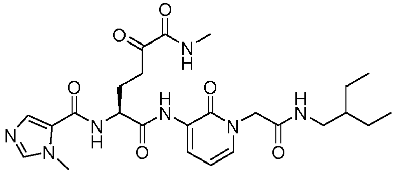

### Ref. 2 (ZED1227)

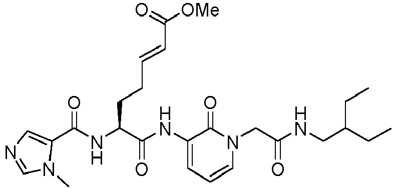

### Ref. 3 (A8, ZED1047)

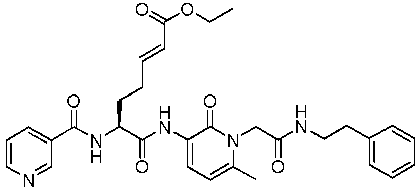

### Ref. 6

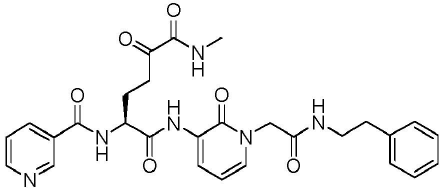

### Example B-2. logD values of the inventive compounds

In order to classify the inventive compounds according to their lipophilicity, LogD values (distribution coefficient) were determined by means of the well-established shake flask method, measuring the partition of a compound between an octanol and phosphate-buffered saline (PBS, pH 7.4) by HPLC.

The LogD is pH dependent and is a "predictor" for in-vivo properties. LogD combines lipophilicity (intrinsic structural property of the molecule, logP) and ionizability (pKa).

Compounds with a moderate lipophilicity (LogD values from 0 to 3) are usually advantaged for oral absorption, being in balance between solubility and permeability. However, sophisticated formulation of a compound might improve oral bioavailability for highly lipophilic compounds.

**Table 2. logd values of reversible TG2 inhibitors**

| **A:** logD < 1, B: 1 ≤ logD < 3, **C:** 3 ≤ logD < 5 | | | |
|---|---|---|---|
| Compound | logD | Compound | logD |
| II-2 | B | II-117 | C |
| II-3 | B | II-118 | B |
| II-4 | B | II-119 | C |
| II-5 | C | II-120 | C |
| II-6 | C | II-121 | C |
| II-7 | C | II-122 | C |
| II-8 | C | II-123 | C |
| II-9 | B | II-124 | B |
| II-10 | C | II-125 | B |
| II-11 | B | II-126 | A |
| II-12 | C | II-127 | A |
| II-13 | B | II-128 | B |
| II-14 | B | II-129 | B |
| II-15 | B | II-130 | A |
| II-16 | B | II-131 | A |
| II-17 | B | II-132 | A |
| II-18 | B | II-133 | A |
| II-19 | C | II-134 | A |
| II-20 | B | II-135 | A |
| II-21 | C | II-136 | B |
| II-22 | C | II-137 | C |
| II-23 | C | II-138 | A |
| II-24 | B | II-139 | A |
| II-25 | B | II-140 | A |
| II-26 | B | II-141 | A |
| II-27 | B | II-142 | B |
| II-28 | B | II-143 | A |
| II-29 | C | II-144 | A |
| II-30 | C | II-145 | A |
| II-31 | B | II-146 | A |
| II-32 | B | II-147 | A |
| II-33 | B | II-148 | A |
| II-34 | B | II-149 | A |
| II-35 | B | II-150 | B |
| II-36 | B | II-151 | B |
| II-37 | B | II-152 | A |
| II-38 | C | II-153 | A |
| II-39 | B | II-154 | B |
| II-40 | C | II-155 | B |
| II-41 | C | II-156 | A |
| II-42 | B | II-157 | A |
| II-43 | B | II-158 | A |
| II-44 | C | II-159 | B |
| II-45 | C | II-160 | B |
| II-46 | C | II-161 | A |
| II-47 | B | II-162 | A |
| II-48 | C | II-163 | A |
| II-49 | C | II-164 | C |
| II-50 | A | II-165 | C |
| II-51 | A | II-166 | B |
| II-52 | A | II-167 | A |
| II-53 | B | II-168 | A |
| II-54 | B | II-169 | A |
| II-55 | A | II-170 | A |
| II-56 | B | II-171 | A |
| II-58 | B | II-172 | A |
| II-59 | B | II-173 | A |
| II-60 | A | II-174 | A |
| II-61 | B | II-175 | B |
| II-62 | B | II-176 | C |
| II-63 | B | II-177 | C |
| II-64 | B | II-178 | C |
| II-65 | B | II-179 | B |
| II-66 | B | II-180 | C |
| II-67 | C | II-181 | B |
| II-68 | A | II-182 | B |
| II-69 | B | II-183 | B |
| II-70 | B | II-184 | B |
| II-71 | B | II-185 | A |
| II-72 | C | II-186 | A |
| II-73 | B | II-187 | C |
| II-74 | B | II-188 | A |
| II-87 | C | II-189 | A |
| II-88 | C | II-190 | A |
| II-90 | B | II-191 | A |
| II-92 | C | II-192 | A |
| II-94 | B | II-193 | A |
| II-95 | B | II-194 | A |
| II-96 | B | II-195 | A |
| II-97 | B | II-196 | A |
| II-98 | B | II-197 | A |
| II-99 | C | II-198 | A |
| II-100 | C | II-199 | A |
| II-101 | C | II-200 | A |
| II-103 | B | II-201 | A |
| II-104 | B | II-202 | A |
| II-105 | C | II-203 | B |
| II-107 | C | II-204 | B |
| II-108 | B | II-205 | A |
| II-109 | C | II-206 | A |
| II-110 | C | II-207 | A |
| II-111 | B | II-208 | B |
| II-112 | B | II-209 | B |
| II-113 | A | II-210 | B |
| II-114 | A | II-211 | B |
| II-115 | C | Ref. 1 | A |
| II-116 | C | Ref. 2 | B |
| Ref. 6 | A | Ref. 3 | B |

### Example B-3. Caco-2 permeability assay of the inventive compounds

Permeability coefficients (Pₐₚₚ values) were obtained from Caco-2 barrier studies predicting oral/intestinal bioavailability of the tested compounds. The assays were performed by using CacoReady^{™} ready-to-use kits from ReadyCell according to the manufacturers protocol.

It is considered that compounds bearing Pₐₚₚ values above 1×10⁻⁶ cm/s are classified as permeable whereas compounds bearing Pₐₚₚ values below 1×10⁻⁶ cm/s are classified as not permeable.

**Table 3. Caco2 permeability assay of reversible TG2 inhibitors**

| **A:** Pₐₚₚ < 1×10⁻⁶ cm/s, **B:** Pₐₚₚ ≥ 1×10⁻⁶ cm/s ≤ Pₐₚₚ < 10×10⁻⁶ cm/s | | | |
|---|---|---|---|
| **C:** Pₐₚₚ ≥ 10x1 0⁻⁶ cm/s | | | |
| Compound | Pₐₚₚ [cm/s] ×10⁻⁶ | Compound | Pₐₚₚ [cm/s] ×10⁻⁶ |
| II-2 | B | II-117 | B |
| II-3 | B | II-118 | B |
| II-4 | B | II-119 | B |
| II-5 | B | II-120 | B |
| II-6 | B | II-121 | B |
| II-7 | B | II-122 | B |
| II-8 | B | II-123 | B |
| II-9 | B | II-124 | B |
| II-10 | A | II-125 | B |
| II-11 | B | II-126 | B |
| II-12 | A | II-127 | A |
| II-13 | B | II-128 | B |
| II-14 | B | II-129 | A |
| II-15 | B | II-130 | A |
| II-16 | B | II-131 | A |
| II-17 | B | II-132 | A |
| II-18 | A | II-133 | B |
| II-19 | A | II-134 | A |
| II-20 | B | II-135 | B |
| II-21 | B | II-136 | B |
| II-22 | B | II-137 | B |
| II-23 | B | II-138 | A |
| II-24 | B | II-139 | A |
| II-25 | B | II-140 | A |
| II-26 | B | II-141 | A |
| II-27 | B | II-142 | A |
| II-28 | B | II-143 | A |
| II-29 | B | II-144 | A |
| II-30 | B | II-145 | A |
| II-31 | B | II-146 | B |
| II-32 | B | II-147 | B |
| II-33 | C | II-148 | B |
| II-34 | C | II-149 | B |
| II-35 | B | II-150 | B |
| II-36 | B | II-151 | B |
| II-37 | B | II-152 | A |
| II-38 | B | II-153 | A |
| II-39 | B | II-154 | B |
| II-40 | A | II-155 | B |
| II-41 | B | II-156 | A |
| II-42 | B | II-157 | A |
| II-43 | B | II-158 | A |
| II-44 | B | II-159 | B |
| II-45 | B | II-160 | B |
| II-46 | A | II-161 | A |
| II-47 | B | II-162 | A |
| II-48 | B | II-163 | A |
| II-49 | B | II-164 | B |
| II-50 | A | II-165 | B |
| II-51 | B | II-166 | B |
| II-52 | A | II-167 | B |
| II-53 | A | II-168 | A |
| II-54 | A | II-169 | B |
| II-55 | A | II-170 | B |
| II-56 | A | II-171 | B |
| II-58 | A | II-172 | B |
| II-59 | A | II-173 | A |
| II-60 | A | II-174 | A |
| II-61 | A | II-175 | A |
| II-62 | B | II-176 | B |
| II-63 | A | II-177 | B |
| II-64 | A | II-178 | B |
| II-65 | A | II-179 | B |
| II-66 | A | II-180 | A |
| II-67 | B | II-181 | A |
| II-68 | A | II-182 | B |
| II-69 | B | II-183 | B |
| II-70 | B | II-184 | A |
| II-71 | B | II-185 | A |
| II-72 | A | II-186 | A |
| II-73 | A | II-187 | B |
| II-74 | A | II-188 | A |
| II-87 | B | II-189 | A |
| II-88 | A | II-190 | A |
| II-90 | A | II-191 | A |
| II-92 | B | II-192 | A |
| II-94 | B | II-193 | A |
| II-95 | B | II-194 | A |
| II-96 | B | II-195 | A |
| II-97 | B | II-196 | A |
| II-98 | B | II-197 | A |
| II-99 | B | II-198 | A |
| II-100 | B | II-199 | A |
| II-101 | B | II-200 | A |
| II-103 | B | II-201 | A |
| II-104 | B | II-202 | A |
| II-105 | B | II-203 | A |
| II-107 | B | II-204 | A |
| II-108 | A | II-205 | A |
| II-109 | B | II-206 | A |
| II-110 | B | II-207 | A |
| II-111 | A | II-208 | A |
| II-112 | A | II-209 | A |
| II-113 | A | II-210 | A |
| II-114 | A | II-211 | A |
| II-115 | B | Ref. 1 | A |
| II-116 | B | Ref. 2 | A |
| Ref. 6 | A | Ref. 3 | A |

### Bioavailability studies

Presumed from promising Pₐₚₚ values (permeability coefficients, see below), the inventors had proven the oral bioavailability of the inhibitors of the present application by the representative compounds II-3, II-15, and II-28. For this selected set of representative compounds, pharmacokinetic profiles were determined in male C57BL/6 mice (N=3, each). Briefly, the compounds were administered as single-dose soluble, oral formulation [20 mg/ml in PBS / (2-Hydroxypropyl)-β-cyclodextrin formulation] at 200 mg/kg. Plasma samples were taken at (0, 0.25, 0.5, 1, 2, 4, and 6 hours) and analyzed by LC-MS to determine the concentration of the representative compounds.

The calculated pharmacokinetic parameters are summarized in the table.

| | Cₘₐₓ [ng/mL] | Cₘₐₓ [nM] | AUCt [ng/mL*h] | Kₑₗ [1/h] | MRT [h] | t_{1/2} [h] | CL/F [L/h/kg] | Vd/F [L/h/kg] | R² |
|---|---|---|---|---|---|---|---|---|---|
| II-3 | 13,594 | 21,588 | 12,023 | 1.0 | 1.0 | 0.7 | 16.6 | 16.3 | 0.87 |
| II-28 | 15,143 | 23,277 | 14,468 | 0.6 | 1.6 | 1.1 | 14.8 | 23.8 | 0.98 |
| II-15 | 32,322 | 48,626 | 36,878 | 0.9 | 1.1 | 0.9 | 6.1 | 7.5 | 0.93 |

| | | | | | | | | | |
|---|---|---|---|---|---|---|---|---|---|
| Cₘₐₓ: maximum plasma concentration. AUCt: area under the plasma concentration time curve from administration to last observed concentration at time t measured by trapezoidal rules. Kₑₗ: is estimated by the linear regression of the logarithm of the terminal concentration as a function of time. Point used to calculate the Kel are selected the 'Best Fit' option of Winnonlin. MRT: mean residence time. t_{1/2}: is calculated by application of the equation In2 / Kₑₗ. CL/F: apparent plasma clearance calculated as follow: Dose/AUCinf. V_{d}/F (L/kg): apparent volume of distribution following administration. The parameter are calculated as V_{d}/F = (CL/F) / Kₑₗ. R²: correlation coefficient. | | | | | | | | | |

| | IC₅₀ [nM] | IC₉₀ [nM] | Pₐₚₚ [cm/s] *10⁻⁶ |
|---|---|---|---|
| II-3 | 23 | 180 | 8.4 |
| II-28 | 24 | 240 | 7.4 |
| II-15 | 35 | 375 | 5.9 |

The corresponding PK profiles reveal that the plasma levels for all representative compounds exceed the IC₉₀ for 3.5 hours and the IC₅₀ during the whole study (6 hours). In summary, the high cₘₐₓ values observed in the PK-studies exceed the IC₉₀ levels >100-times. We therefore expect to occupy all active TG2 accessible.

Additionally, in a multiple-dosing PK study, II-3 was orally administered to 3 mice with 200 mg/kg doses (dose volume 10 mL/kg) twice daily (12 h intervals). After the animals were sacrificed, the liver and lung were removed. The homogenates of the respective tissues were analyzed by LC-MS to determine the concentration of the compound. The tissue concentration in lung and liver after the eighth dose (four days) was 6,800 and 10,400 ng/g, respectively, showing that the compound reaches the tissue at pharmacological active concentration.

## Claims

1. A compound of the general formula (**I**): 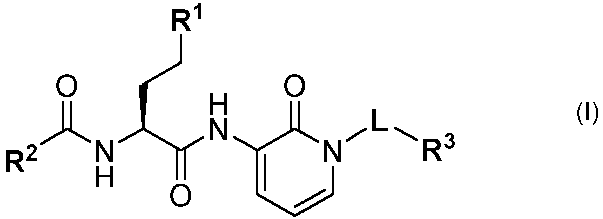 wherein
**L** represents -**L¹**- or **-L¹-L²-**; preferably **-L¹-L²-**;
**L¹** represents -CH₂-, -CH₂CH₂-, -CH₂CH₂CH₂-, -CH₂CO-, or -CH₂CH₂CO-;
**L²** represents a bond, **-NR^{N1}-**, -**NR^{N1}**CH₂-, -**NR^{N1}**CH₂CH₂-, or -NR^{N1}CH(CH₃)-;
**R¹** represents 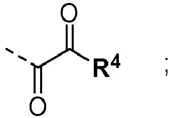
**R²** represents 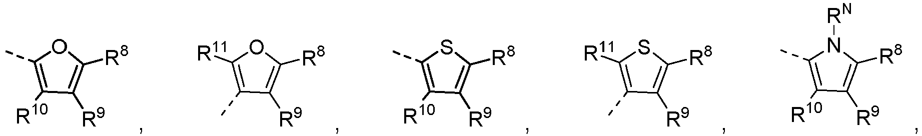 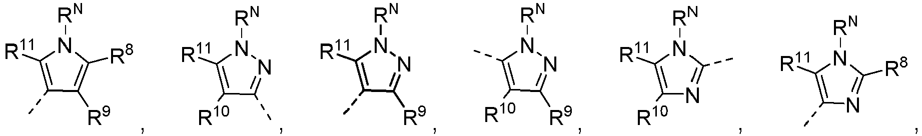 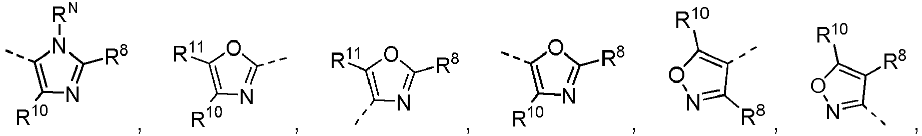 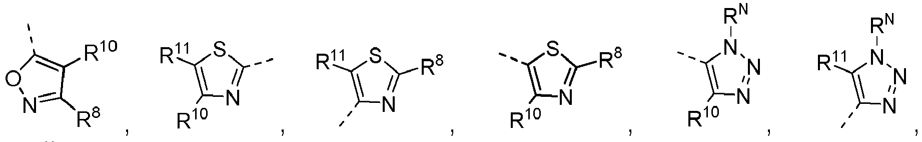 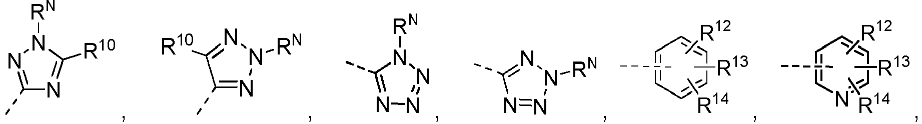 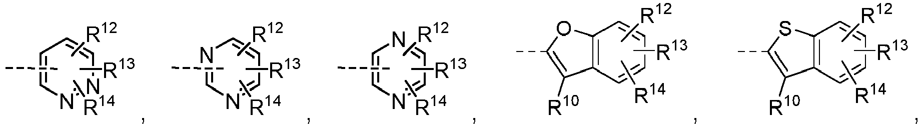 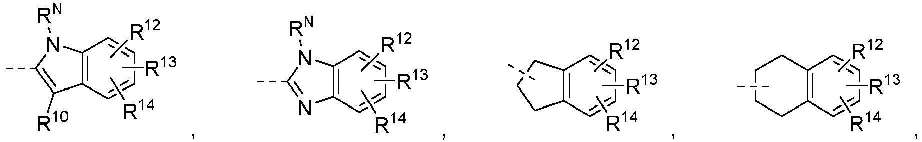 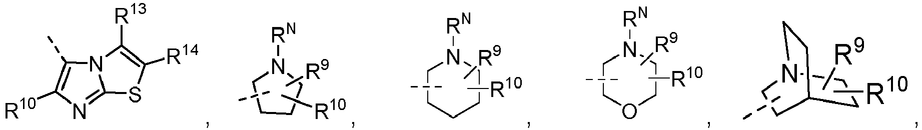 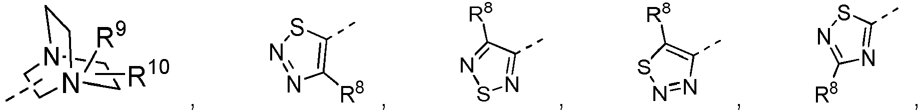 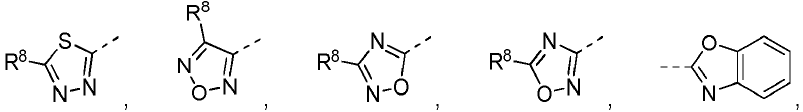 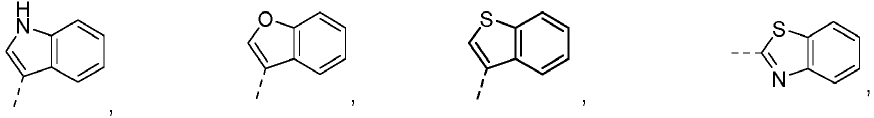 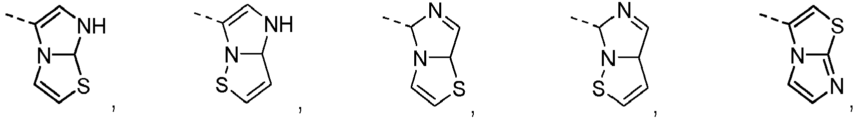 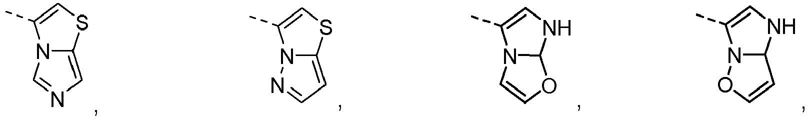 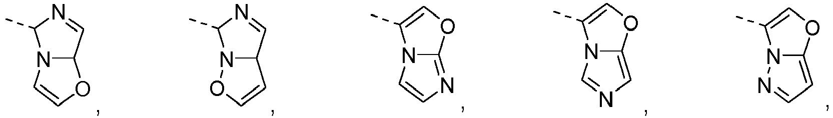 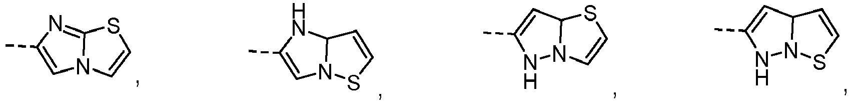 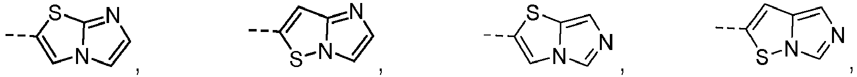 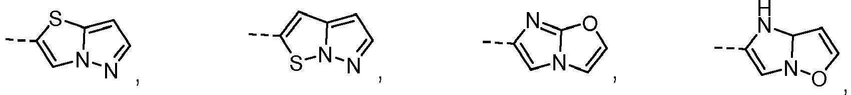 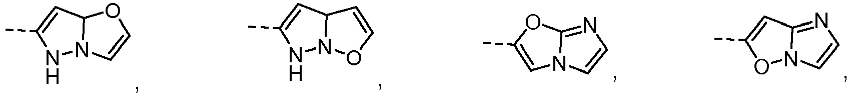 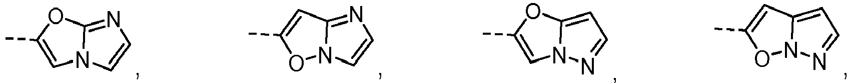 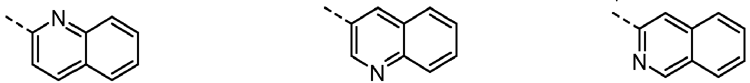 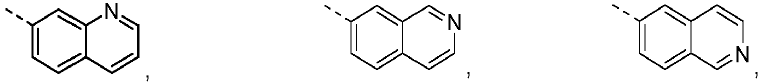 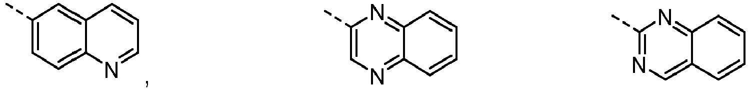 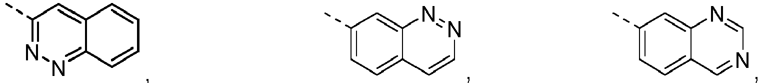 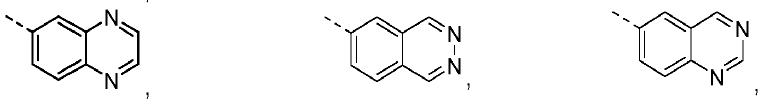 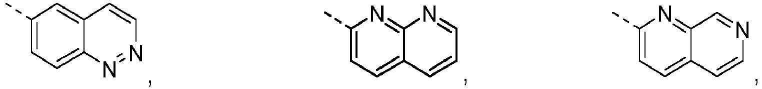 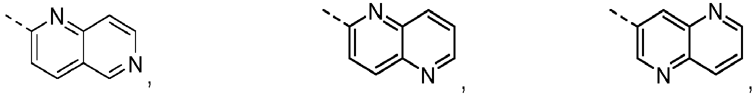 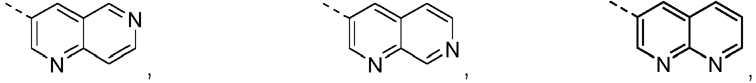 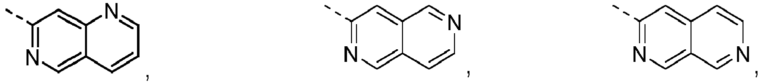 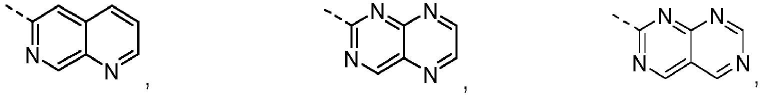 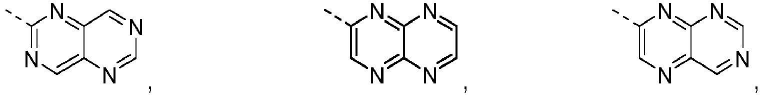 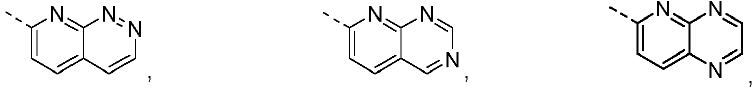 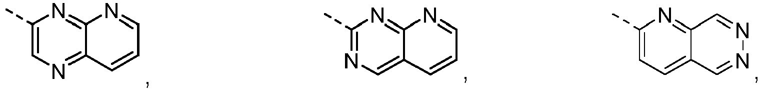 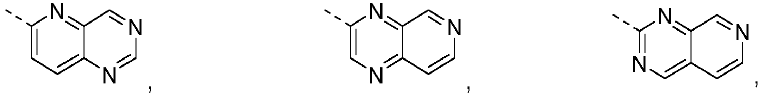 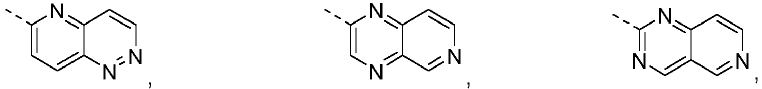 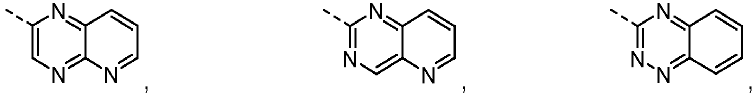 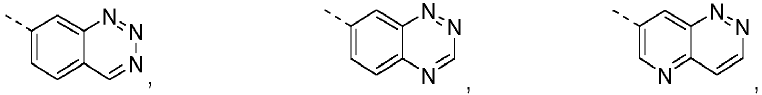 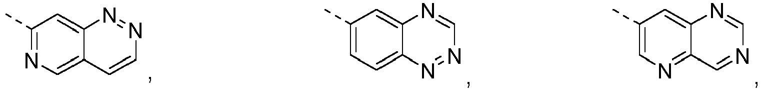 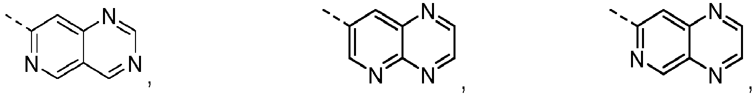 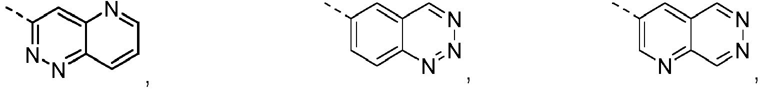 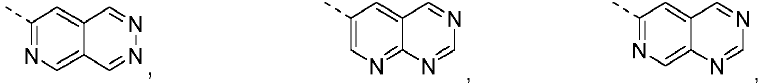 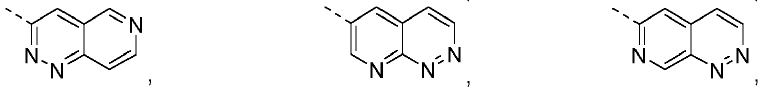 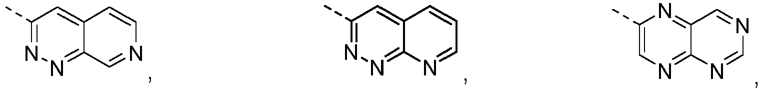 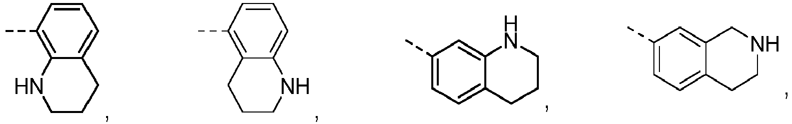 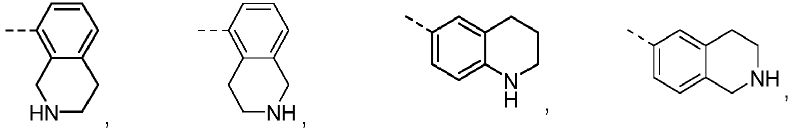 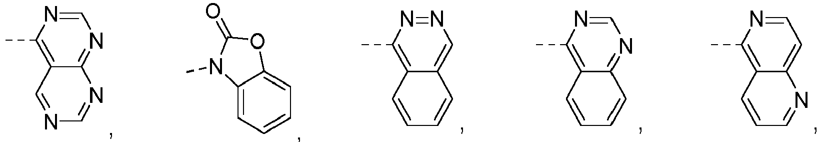 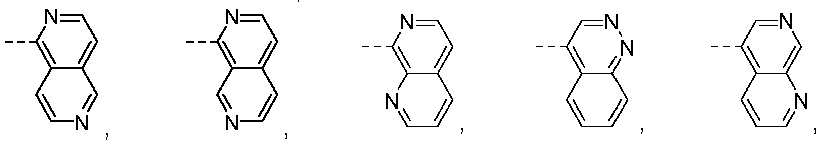 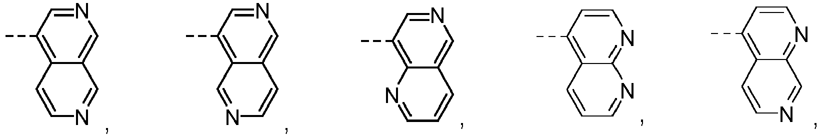 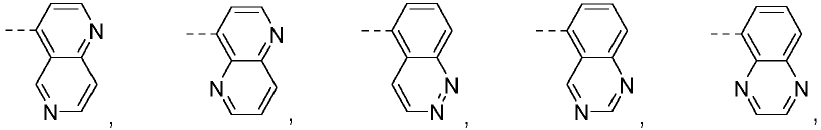 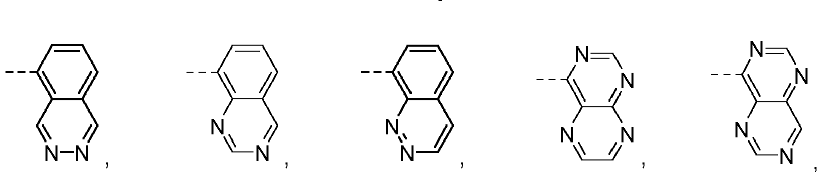 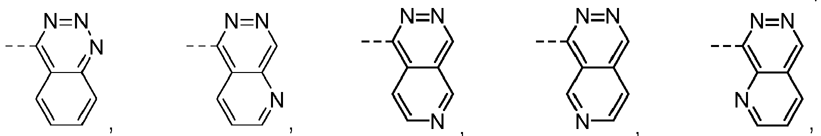 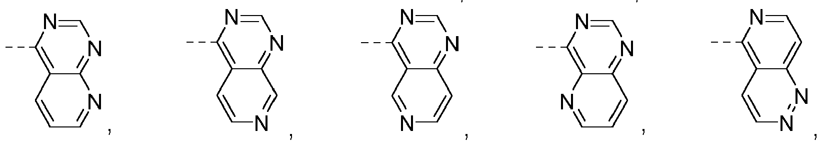 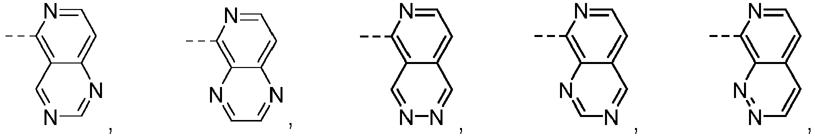 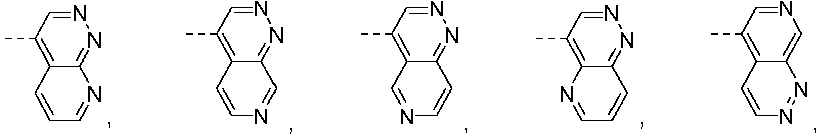 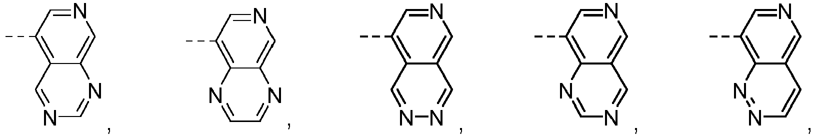 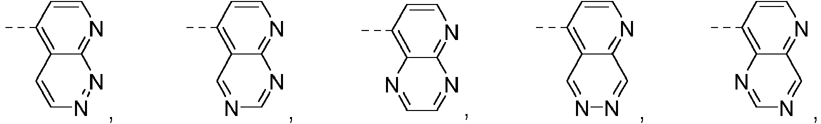 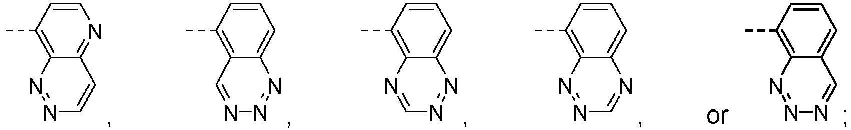
wherein the unsubstituted bicyclic residues can be substituted with 1 to 5 of the substituents **R⁹** - **R¹⁴** and **R^{N}** ; and preferably with 1 to 3 of the substituents **R¹¹** - **R¹³**;
**R³** represents bicyclo[1.1.1]pentyl, bicyclo[2.1.1]hexyl, bicyclo[2.2.1]heptyl, bicyclo[3.1.1]heptyl, bicyclo[2.2.2]octyl, bicyclo[3.2.1]octyl, bicyclo[3.2.2]nonyl, bicyclo[3.3.2]decyl, bicyclo[3.3.3]undecyl, 4-homoisotwistyl, adamantyl, diamantyl, hexamethylenetetraminyl and the afore-mentioned residues optionally contain one or more C=C double bond(s) and/or are optionally substituted by one or more of **R^{a}**, **R^{b}**, **R^{c}**, **R^{d}**, and **R^{e}**;
**R^{a}**, **R^{b}**, **R^{c}**, **R^{d}**, and **R^{e}** represent independently of each other -H, -F, -Cl, -Br, -CN, -OH, -CH₃, -CH₂CH₃, -CH₂CH₂CH₃, -CH(CH₃)₂, -CHF₂, -CF₃, -CH₂CF₃, -COCH₃, -COCH₂CH₃, -CO₂H, -CO₂CH₃, -CO₂C₂H₅, -CONH₂, -CONHCH₃, -CON(CH₃)₂, -CONHC₂H₅, -CH₂CO₂H, -CH₂CO₂CH₃, -CH₂CO₂C₂H₅, -CH₂CONH₂, -CH₂CONHCH₃, -CH₂CON(CH₃)₂, -CH₂CONHC₂H₅, -NHCOCH₃, -NHCOC₂H₅, -NHCOCF₃, -NHCOCH₂CF₃, -NHSO₂CH₃, -NHSO₂C₂H₅, -NHSO₂CHF₂, -NHSO₂CF₃, or -NHSO₂CH₂CF₃;
**R⁴** represents **-NR⁶R⁷**;
**R⁶** and **R⁷** represent independently of each other -H, -CH₃, -CH₂CH₂CH₃, -CH(CH₃)₂, -CH₂CH₂CH₂CH₃, -CH₂CH₂CH₂CH₂CH₃, -CH₂CH(CH₃)₂, -C(CH₃)₃, -CH₂CH=CH₂, -CH₂CH=CH(CH₃), -CH₂CH=C(CH₃)₂, -CH₂CH=CHCH₂CH₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -Ph, -CH₂-Ph, -CH₂OCH₃, -CH₂OCH₂CH₃, -CH₂CH₂OCH₃, -CH₂CH₂OCH₂CH₃, -CH₂CH₂NHCH₃, or -CH₂CH₂N(CH₃)₂, or **-NR⁶R⁷** is -N(C₂H₅)₂, 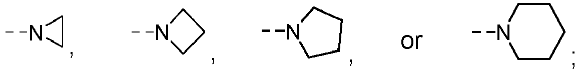
**R⁸, R⁹, R¹⁰, R¹¹, R¹², R¹³,** and **R¹⁴** represent independently of each other -H, -F, -Cl, -Br, -I, -OH, -CN, -NO₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -CH₂-cyclo-C₃H₅, -CH₂OH, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -OCH₃, -OC₂H₅, -OC₃H₇, -OCH(CH₃)₂, -OC(CH₃)₃, -OC₄H₉, -OCHF₂, -OCF₃, -OCH₂CF₃, -OC₂F₅, -OCH₂OCH₃, -O-cyclo-C₃H₅, -OCH₂-cyclo-C₃H₅, -O-C₂H₄-cyclo-C₃H₅, -CHO, -COCH₃, -COCF₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, -COOC(CH₃)₃, -OOC-CH₃, -OOC-CF₃, -OOC-C₂H₅, -OOC-C₃H₇, -OOC-CH(CH₃)₂, -OOC-C(CH₃)₃, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NHCOCH₃, -NHCOCF₃, -NHCOC₂H₅, -NHCOC₃H₇, -NHCOCH(CH₃)₂, -NHCOC(CH₃)₃, -CONH₂, -CONHCH₃, -CONHC₂H₅, -CONHC₃H₇, -CONHCH(CH₃)₂, -CONH-cyclo-C₃H₅, -CONHC(CH₃)₃, -CON(CH₃)₂, -CON(C₂H₅)₂, -CON(C₃H₇)₂, -CON[CH(CH₃)₂]₂, -CON[C(CH₃)₃]₂, -SO₂NH₂, -SO₂NHCH₃, -SO₂NHC₂H₅, -SO₂NHC₃H₇, -SO₂NHCH(CH₃)₂, -SO₂NH-cyclo-C₃H₅, -SO₂NHC(CH₃)₃, -SO₂N(CH₃)₂, -SO₂N(C₂H₅)₂, -SO₂N(C₃H₇)₂, -SO₂N[CH(CH₃)₂]₂, -SO₂N[C(CH₃)₃]₂, -NHSO₂CH₃, -NHSO₂CF₃, -NHSO₂C₂H₅, -NHSO₂C₃H₇, -NHSO₂CH(CH₃)₂, -NHSO₂C(CH₃)₃, -CH=CH₂, -CH₂-CH=CH₂, -C(CH₃)=CH₂, -CH=CH-CH₃, -C=CH, -C=C-CH₃, -CH₂-C=CH, -Ph, -O-Ph, -O-CH₂-Ph, 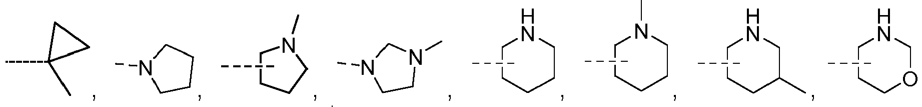 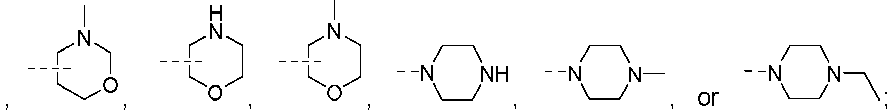
or **R⁸** and **R⁹** or **R⁹** and **R¹⁰** can form together one of the following five-membered or six-membered rings: 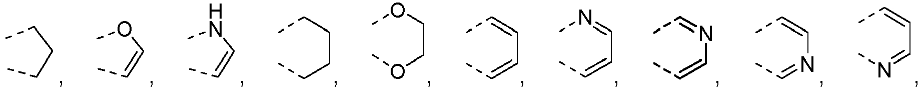 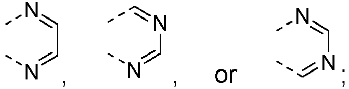
or **R¹²** and **R¹³** or **R¹³** and **R¹⁴** can form together one of the following five-membered or six-membered rings: 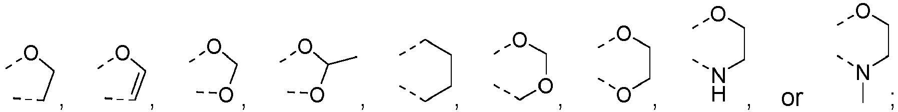
**R^{N}** represents -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -cyclo-C₄H₇, -cyclo-C₅H₉, -CH₂-cyclo-C₃H₅, -CH₂-cyclo-C₄H₇, -CH₂-cyclo-C₅H₉, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -CH₂-CH=CH₂, -CH₂-C=CH, -CHO, -COCH₃, -COC₂H₅, -COC₃H₇, -COCH(CH₃)₂, -COC(CH₃)₃, -CO-cyclo-C₃H₅, -CO-cyclo-C₄H₇, -CO-cyclo-C₅H₉, -COOCH₃, -COOC₂H₅, -COOC₃H₇, -COOCH(CH₃)₂, -COOC(CH₃)₃, -COOCH₂Ph, -SO₂CH₃, -SO₂CF₃, -SO₂C₂H₅, -SO₂C₃H₇, -SO₂CH(CH₃)₂, -SO₂-cyclo-C₃H₅, or -SO₂C(CH₃)₃;
**R^{N1}** represents -H, -CH₃, or -CH₂CH₃;
or a diastereomer, an enantiomer, a mixture of diastereomers, a mixture of enantiomers, a racemate, a solvate, a hydrate, or a pharmaceutically acceptable salt thereof.

2. The compound according to claim 1, wherein **R²** represents 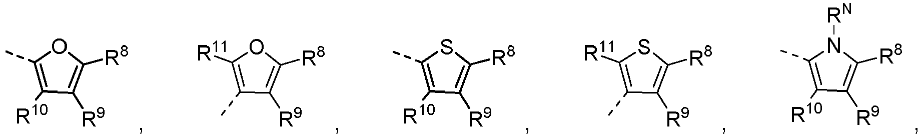 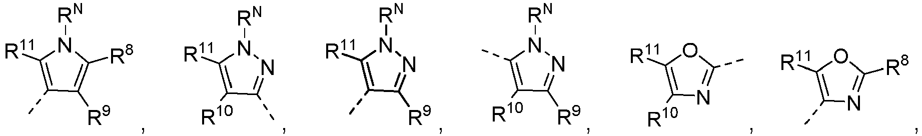 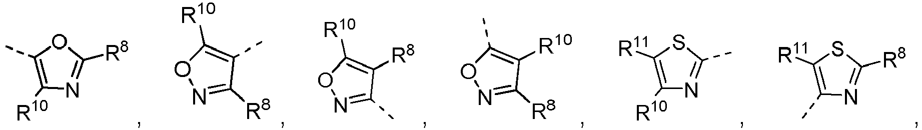 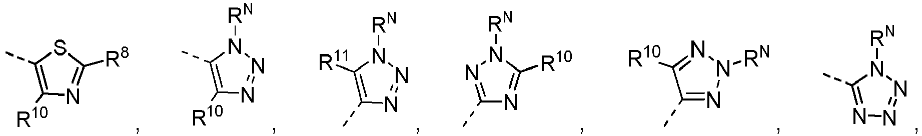 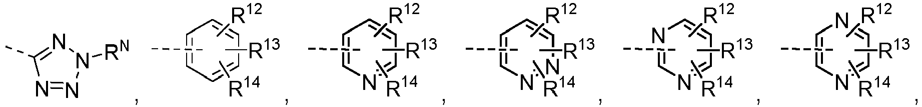 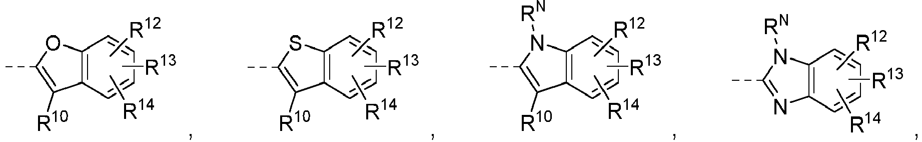 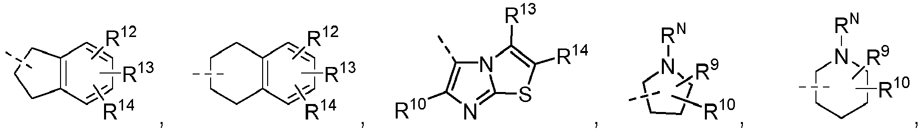 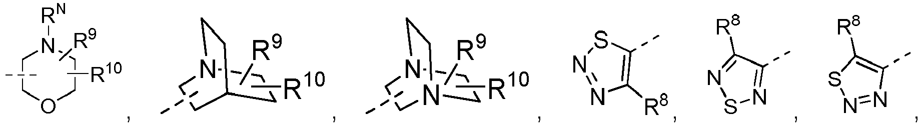 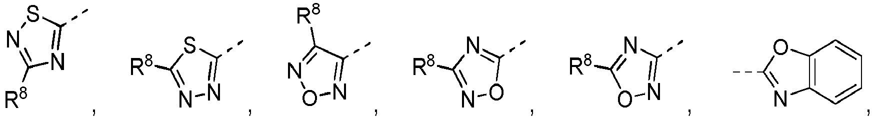 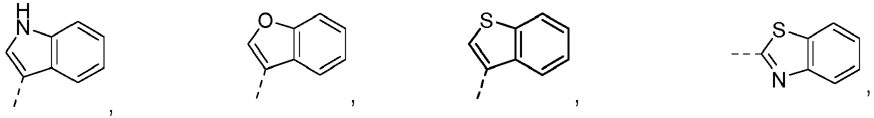 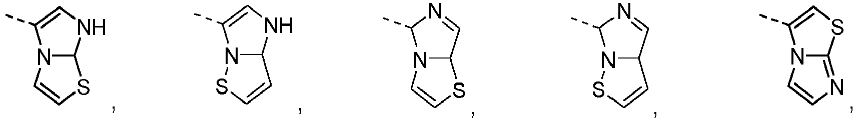 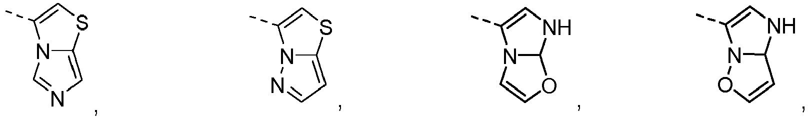 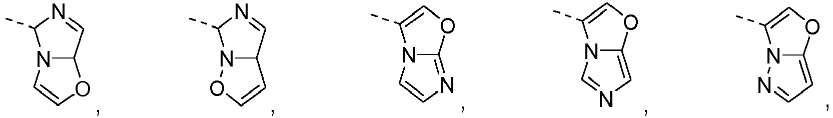 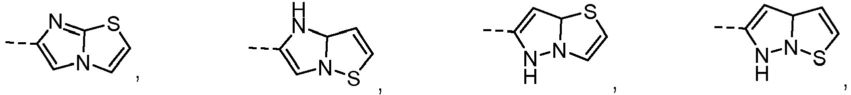 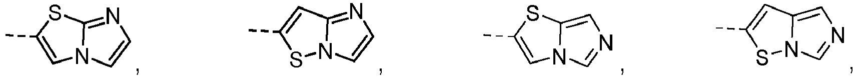 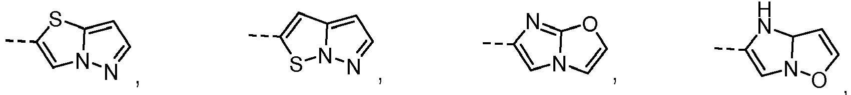 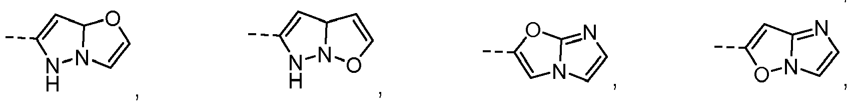 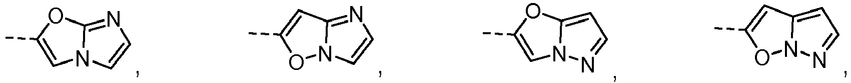 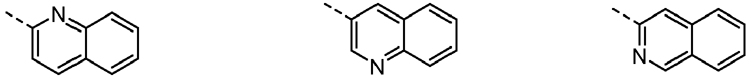 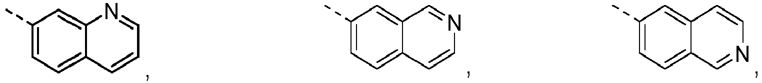 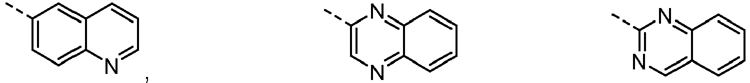 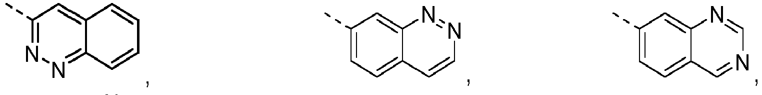 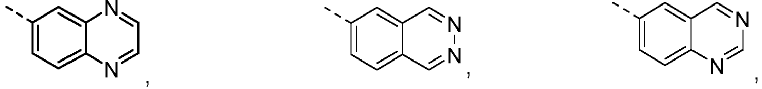 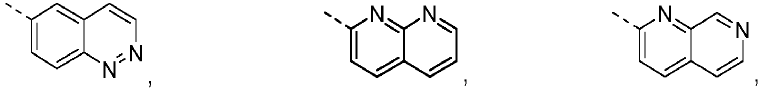 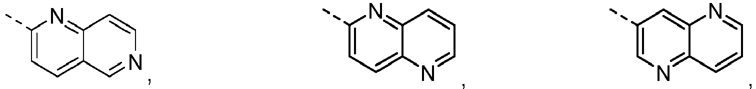 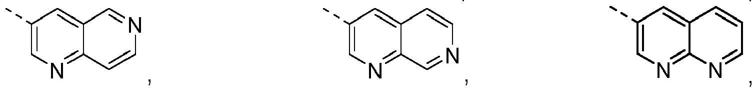 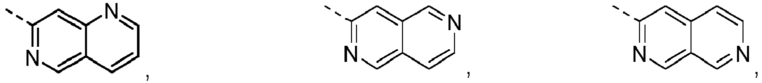 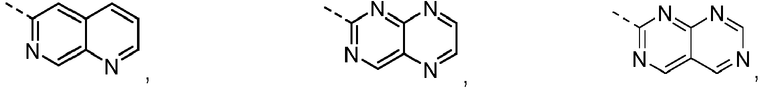 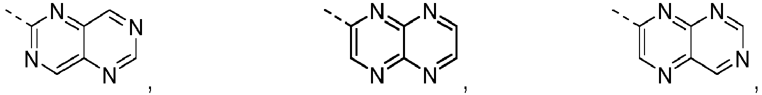 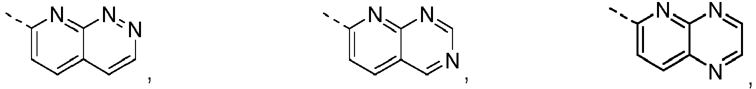 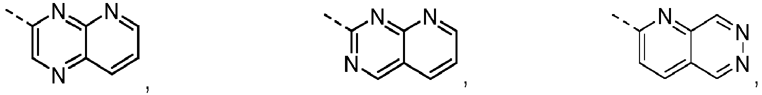 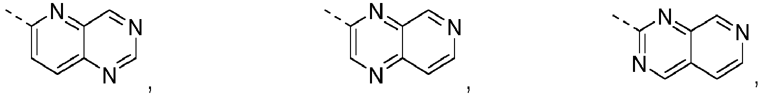 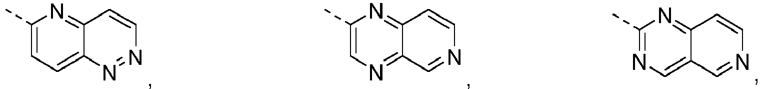 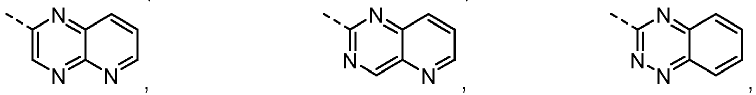 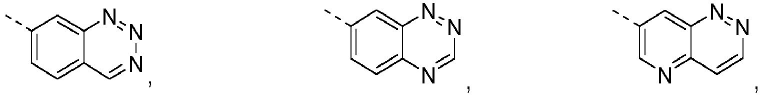 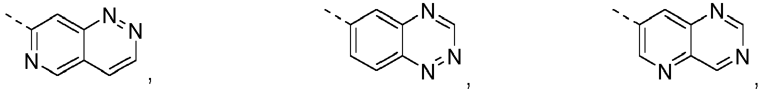 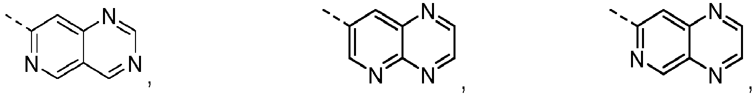 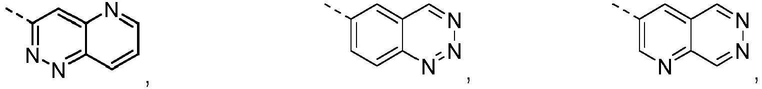 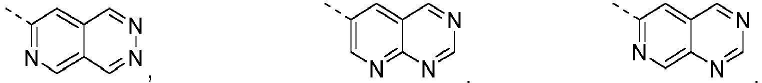 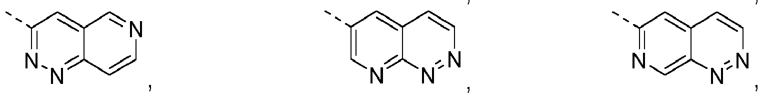 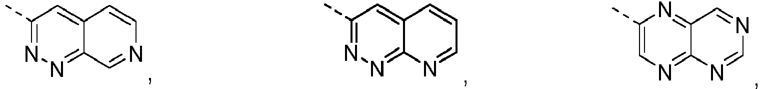 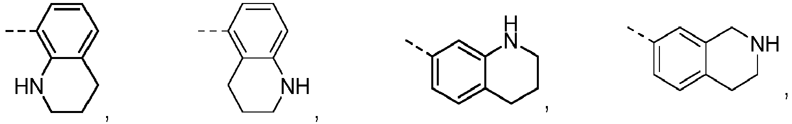 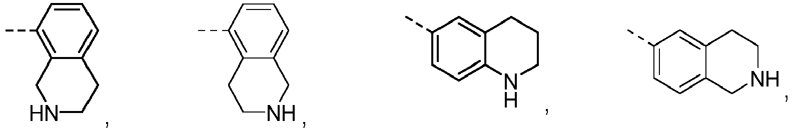 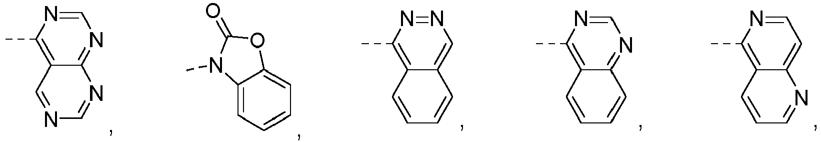 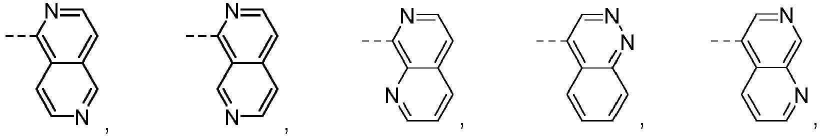 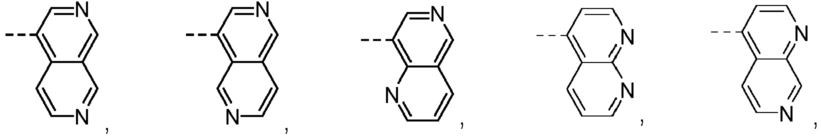 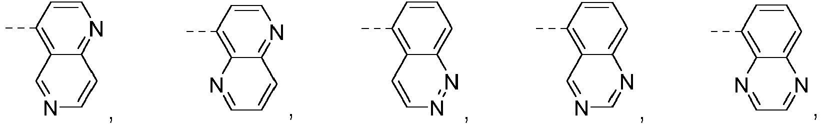 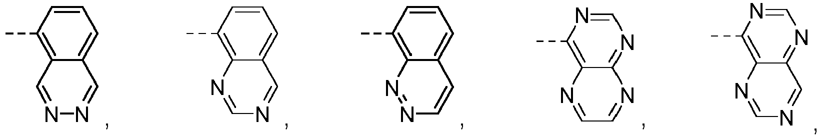 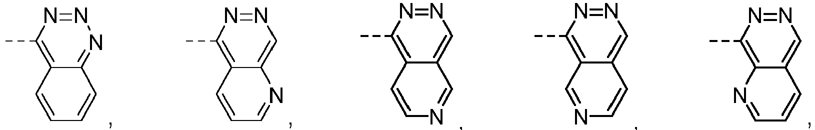 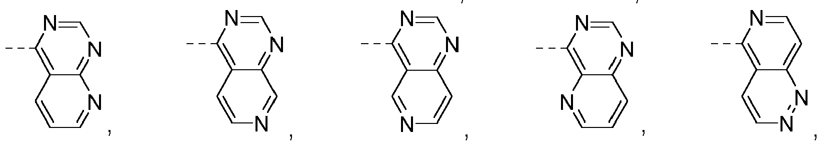 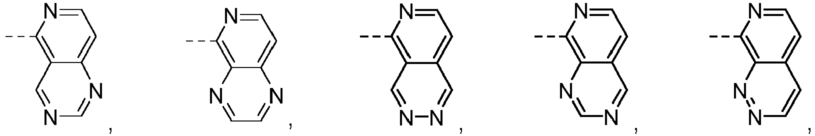 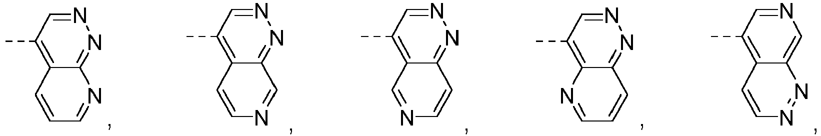 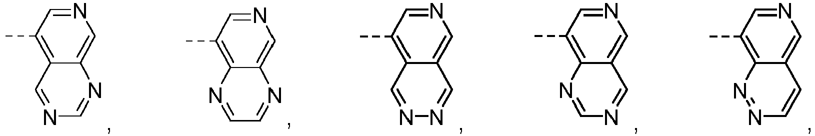 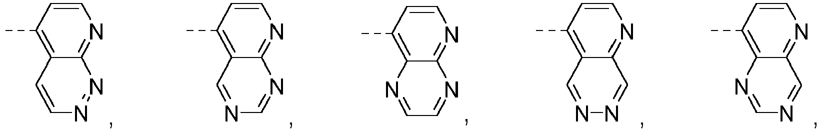 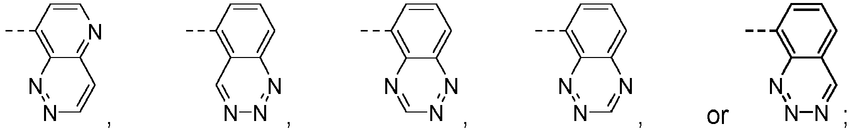 wherein the unsubstituted bicyclic residues can be substituted with 1 to 5 of the substituents **R⁹** - **R¹⁴** and **R^{N}** ; and preferably with 1 to 3 of the substituents **R¹¹** - **R¹³** and the substituents **R⁹** - **R¹⁴** and **R^{N}** have the meanings as defined in claim 1.

3. The compound according to claim 1, wherein **R²** represents 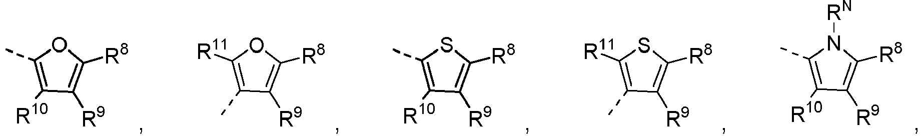 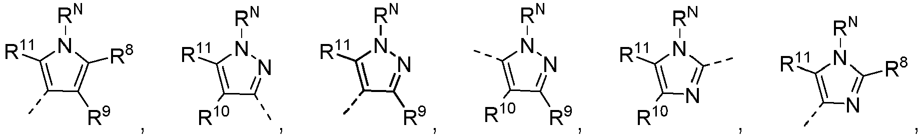 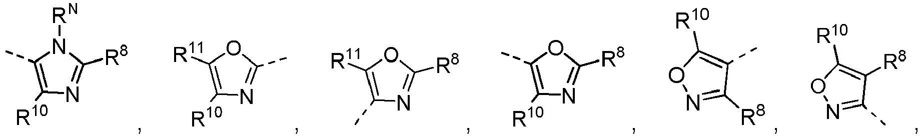 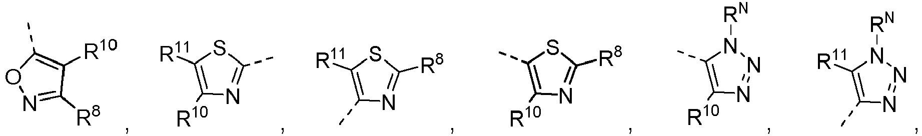 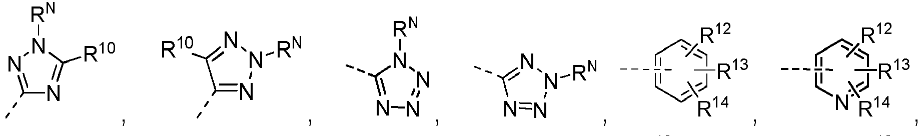 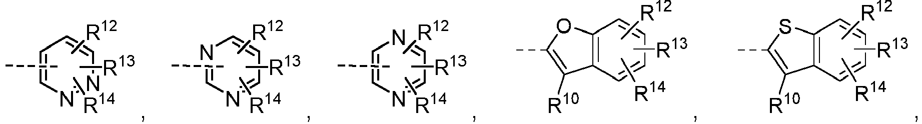 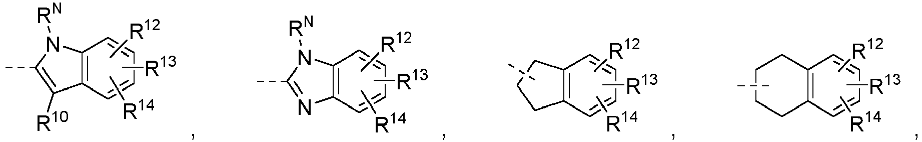 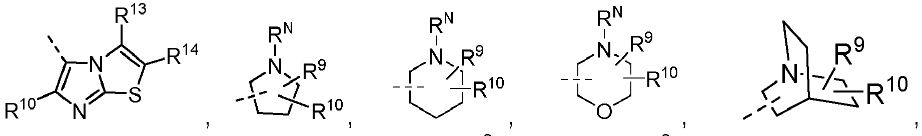 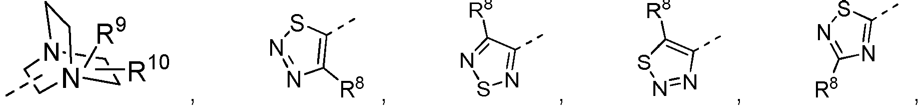 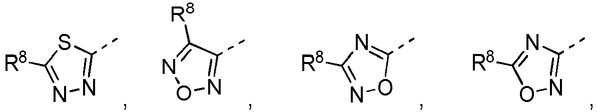 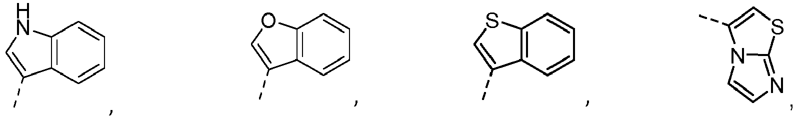 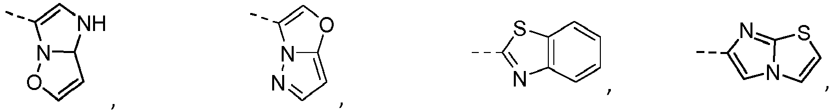 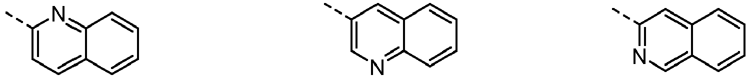 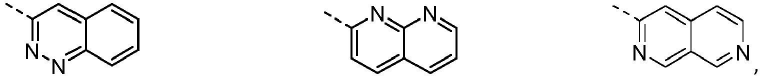 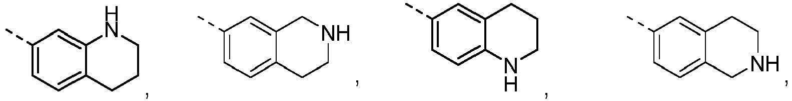 or 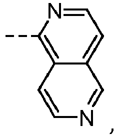 wherein the unsubstituted bicyclic residues can be substituted with 1 to 5 of the substituents **R⁹** - **R¹⁴** and **R^{N}** ; and preferably with 1 to 3 of the substituents **R¹¹** - **R¹³** and the substituents **R⁹** - **R¹⁴** and **R^{N}** have the meanings as defined in claim 1.

4. The compound according to claim 1, wherein the compound has the formula (Ib) 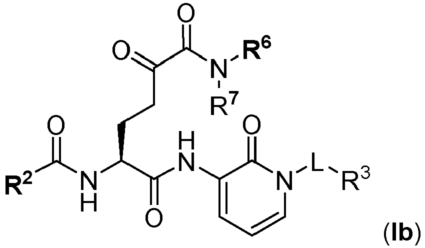 wherein
L represents **-L¹-** or **-L¹-L²-**; preferably **-L¹-L²-**;
**L¹** represents -CH₂-, -CH₂CH₂-, -CH₂CH₂CH₂-, -CH₂CO-, or -CH₂CH₂CO-;
**L²** represents a bond, -NR^{N1}-, -NR^{N1}CH₂-, -NR^{N1}CH₂CH₂-, or -NR^{N1}CH(CH₃)-;
**R²** represents 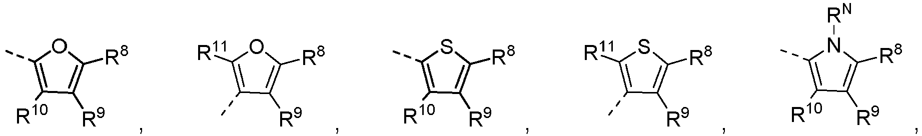 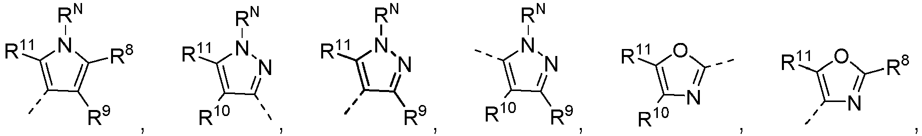 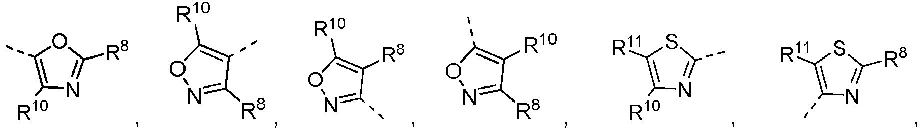 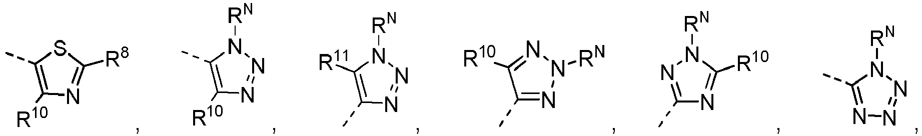 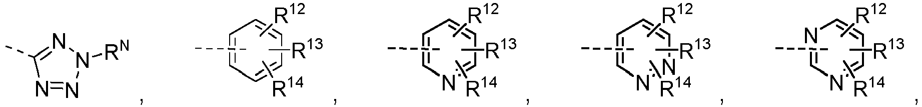 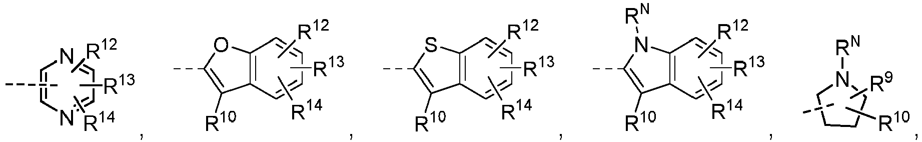 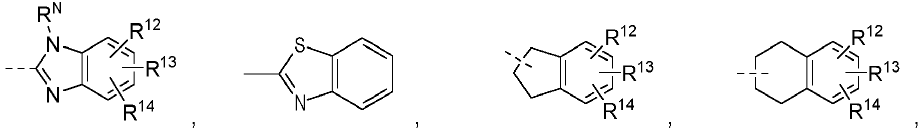 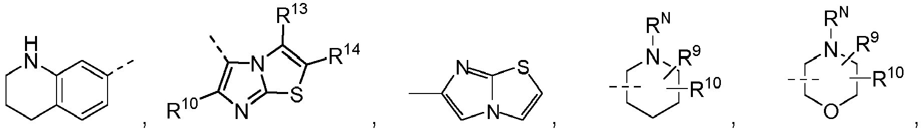 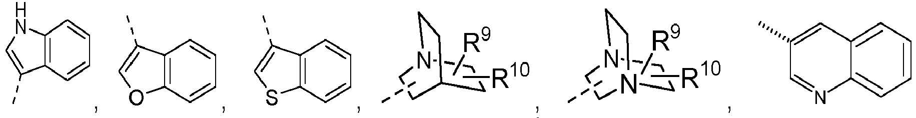 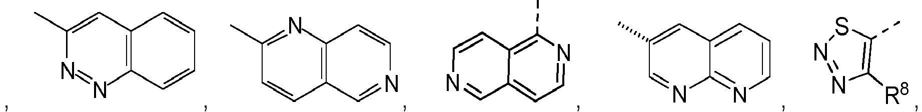 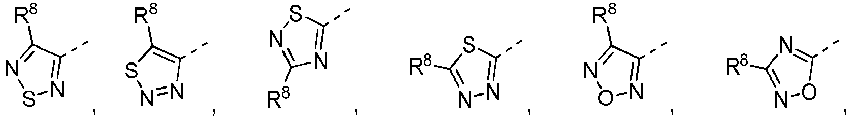 or 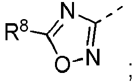
**R³** represents bicyclo[1.1.1]pentyl, bicyclo[2.1.1]hexyl, bicyclo[2.2.1]heptyl, bicyclo[3.1.1]heptyl, bicyclo[2.2.2]octyl, bicyclo[3.2.1]octyl, bicyclo[3.2.2]nonyl, bicyclo[3.3.2]decyl, bicyclo[3.3.3]undecyl, 4-homoisotwistyl, adamantyl, diamantyl, hexamethylenetetraminyl, and the afore-mentioned residues optionally contain one or more C=C double bond(s) and/or are substituted by one or more of **R^{a}**, **R^{b}**, **R^{c}**, **R^{d}**, and **R^{e}**;
**R^{a}**, **R^{b}**, **R^{c}**, **R^{d}**, and **R^{e}** represent independently of each other -H, -F, -Cl, -Br, -CN, -OH, -CH₃, -CH₂CH₃, -CH₂CH₂CH₃, -CH(CH₃)₂, -CHF₂, -CF₃, -CH₂CF₃, -COCH₃, -COCH₂CH₃, -CO₂H, -CO₂CH₃, -CO₂C₂H₅, -CONH₂, -CONHCH₃, -CON(CH₃)₂, -CONHC₂H₅, -CH₂CO₂H, -CH₂CO₂CH₃, -CH₂CO₂C₂H₅, -CH₂CONH₂, -CH₂CONHCH₃, -CH₂CON(CH₃)₂, -CH₂CONHC₂H₅, -NHCOCH₃, -NHCOC₂H₅, -NHCOCF₃, -NHCOCH₂CF₃, -NHSO₂CH₃, -NHSO₂C₂H₅, -NHSO₂CHF₂, -NHSO₂CF₃, or -NHSO₂CH₂CF₃;
**R⁴** represents **-NR⁶R⁷**;
**R⁶** and **R⁷** represent independently of each other -H, -CH₃, -CH₂CH₂CH₃, -CH(CH₃)₂, -CH₂CH₂CH₂CH₃, -CH₂CH₂CH₂CH₂CH₃, -CH₂CH(CH₃)₂, -C(CH₃)₃, -CH₂CH=CH₂, -CH₂CH=CH(CH₃), -CH₂CH=C(CH₃)₂, -CH₂CH=CHCH₂CH₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -Ph, -CH₂-Ph, -CH₂OCH₃, -CH₂OCH₂CH₃, -CH₂CH₂OCH₃, -CH₂CH₂OCH₂CH₃, -CH₂CH₂NHCH₃, -CH₂CH₂N(CH₃)₂, or **-NR⁶R⁷** is -N(C₂H₅)₂, 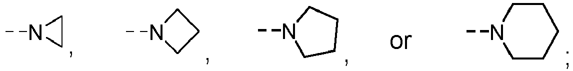
**R^{N}** represents -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -cyclo-C₄H₇, -cyclo-C₅H₉, -CH₂-cyclo-C₃H₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -CH₂-CH=CH₂, -CH₂-C=CH, -CHO, -COCH₃, -COC₂H₅, -COC₃H₇, -COCH(CH₃)₂, -COC(CH₃)₃, -COOCH₃, -COOC₂H₅, -COOC₃H₇, -COOCH(CH₃)₂, -COOC(CH₃)₃, -COOCH₂Ph, -SO₂CH₃, -SO₂CF₃, -SO₂C₂H₅, -SO₂C₃H₇, -SO₂CH(CH₃)₂, or -SO₂C(CH₃)₃;
**R^{N1}** represent -H, -CH₃, or -CH₂CH₃;
and **R⁸** - **R¹⁴** have the meanings as defined in formula (**I**);
or a diastereomer, an enantiomer, a mixture of diastereomers, a mixture of enantiomers, a racemate, a solvate, a hydrate, or a pharmaceutically acceptable salt thereof.

5. The compound according to any one of claims 1 - 4, wherein
**L¹** represents -CH₂-, or -CH₂CO-;
**L²** represents a bond, **-NR^{N1}-**, -N**R**^{N1}CH₂-, or -NR^{N1}CH(CH₃)-;
**R³** represents bicyclo[1.1.1]pentyl, bicyclo[2.1.1]hexyl, bicyclo[2.2.1]heptyl, bicyclo[3.1.1]heptyl, bicyclo[2.2.2]octyl, 4-homoisotwistyl, adamantyl, or diamantyl, and the afore-mentioned residues optionally contain one or more C=C double bond(s) and/or are substituted by one or more of **R^{a}**, **R^{b}**, **R^{c}**, **R^{d}**, and **R^{e}**;
and **R^{a}**, **R^{b}**, **R^{c}**, **R^{d}**, **R^{e}** and **R^{N1}** have the same meanings as defined in claim 1.

6. The compound according to any one of the claims 1 - 5, wherein
**R²** represents 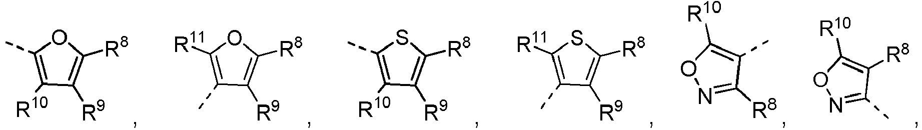 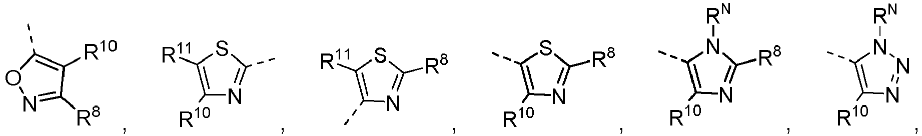 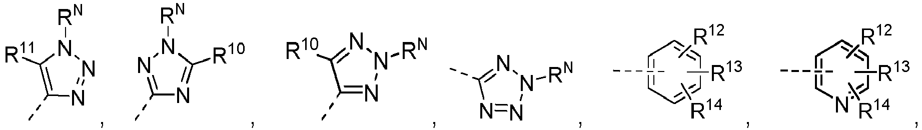 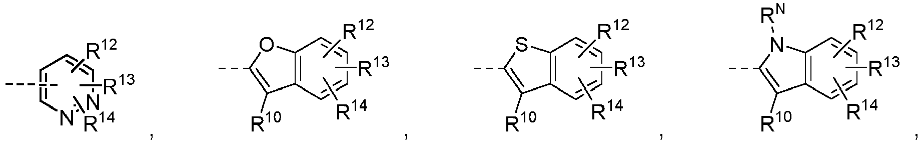 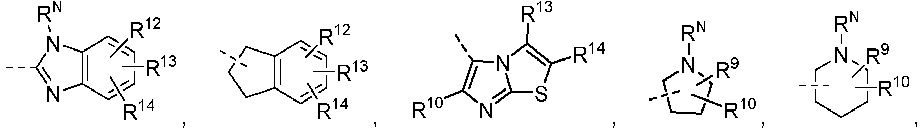 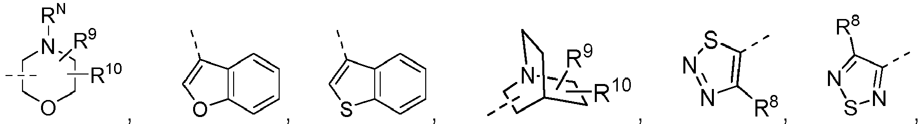 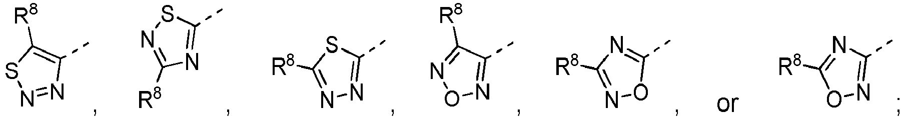 and **R⁸** - **R¹⁴** and **R^{N}** have the meanings as defined in claim 1 or 2.

7. The compound according to any one of the claims 1 - 6, wherein the compound has any one of the formulae (**IV-a**) - (**IV-o**) and (**V-a**) - (**V-d**): 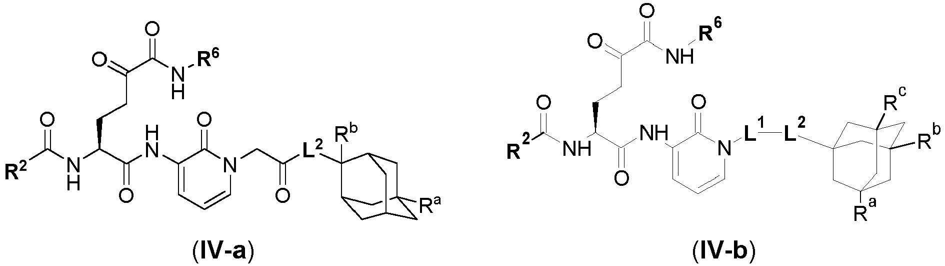 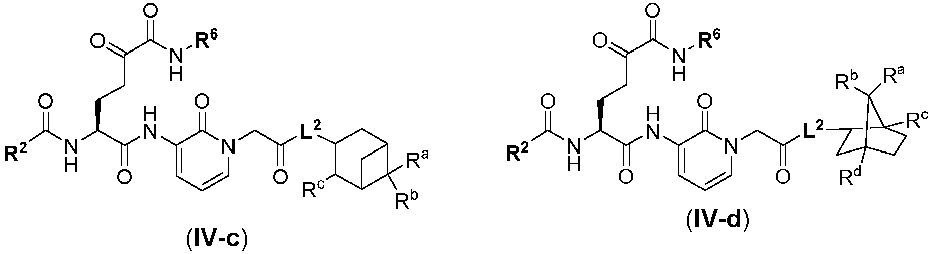 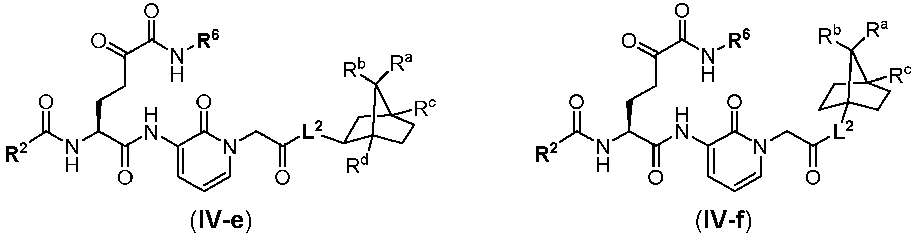 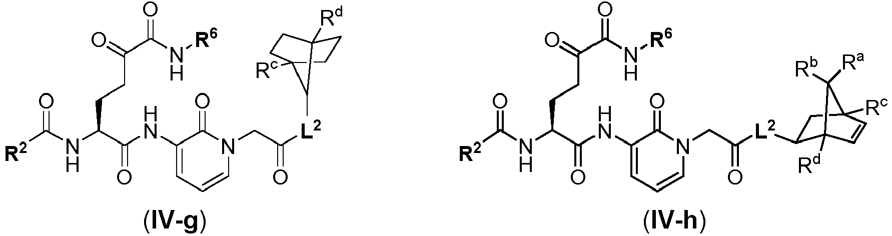 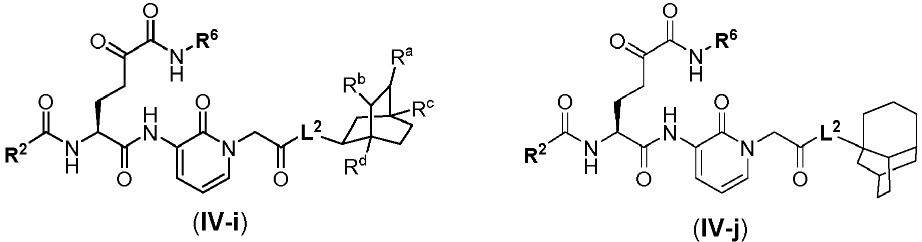 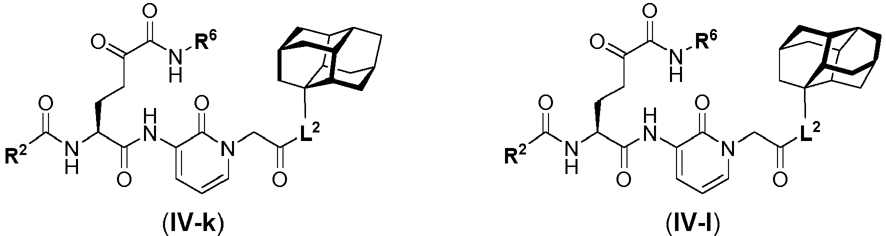 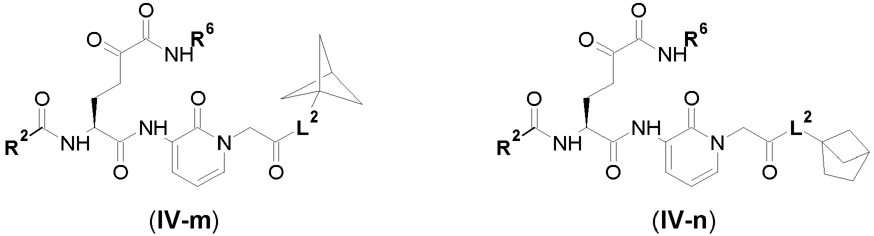 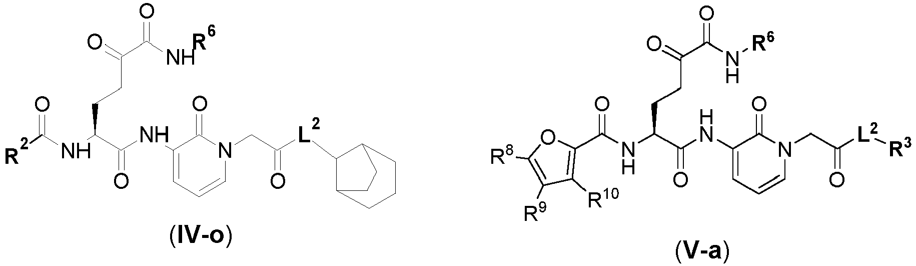 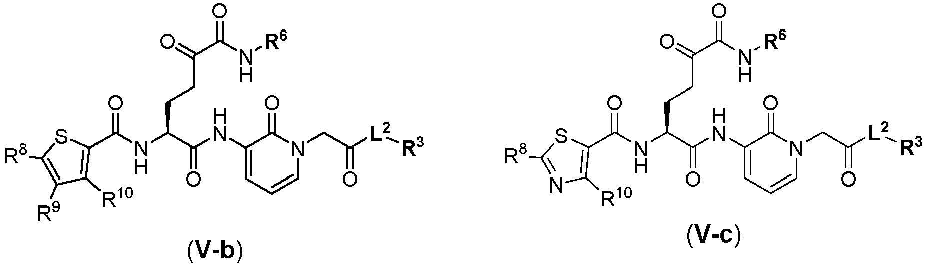 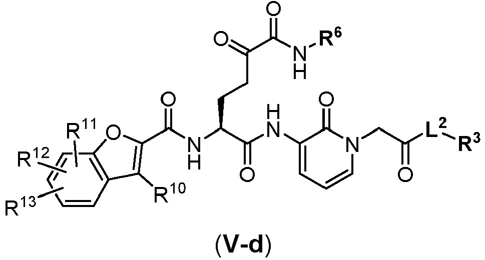 and **R²**, **R³**, **R⁶**, **R⁸**, **R⁹**, **R¹⁰**, **R¹¹**, **R¹²**, **R¹³**, **R^{a}**, **R^{b}**, **R^{c}**, **R^{d}** and **L²** have the same meanings as defined in claim 1.

8. The compound according to any one of the claims 1 - 7, wherein
**R²** represents 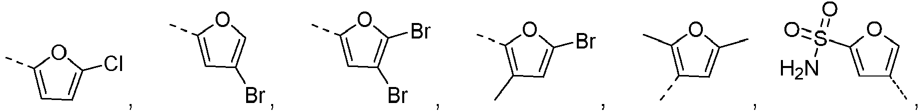 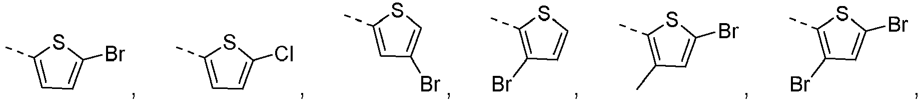 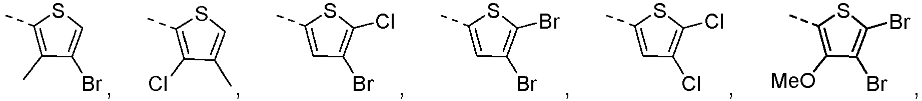 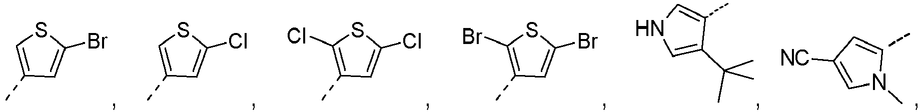 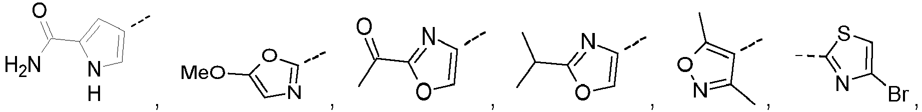 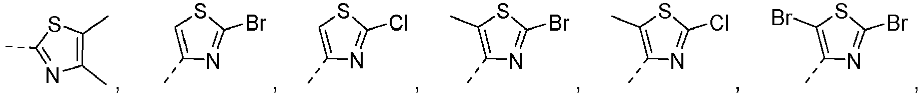 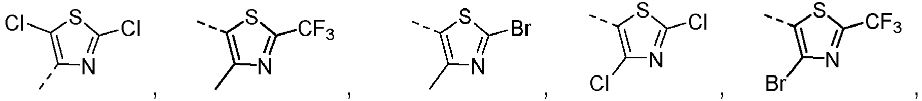 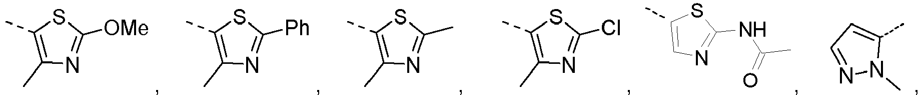 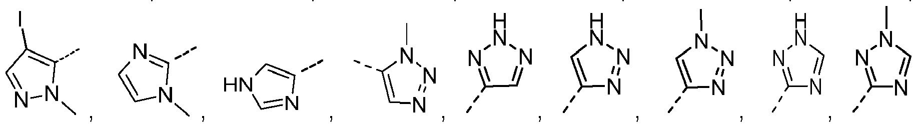 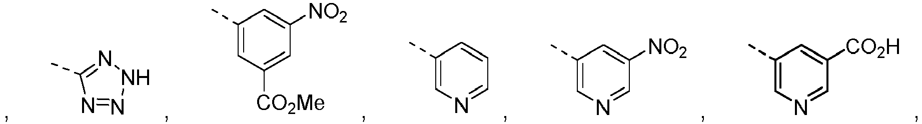 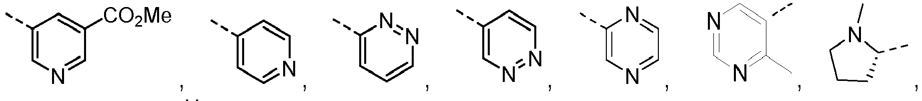 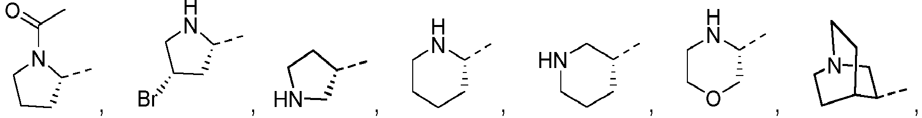 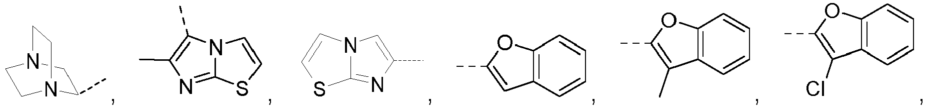 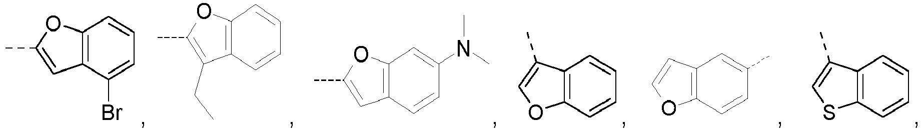 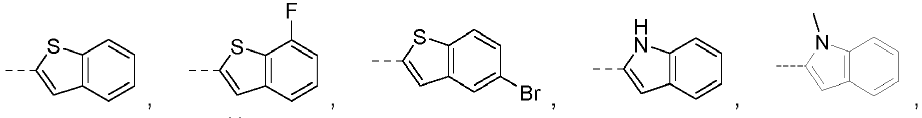 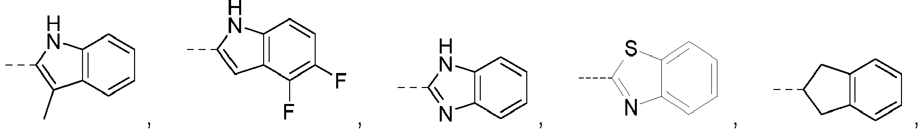 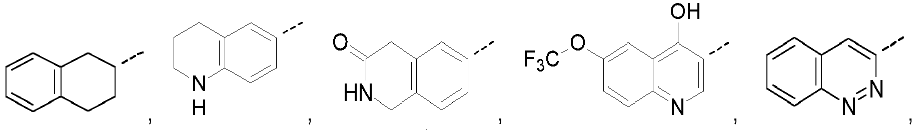 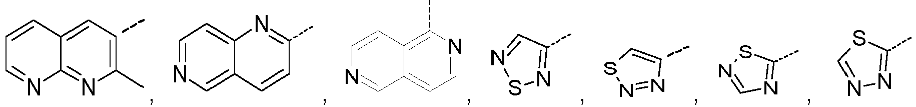 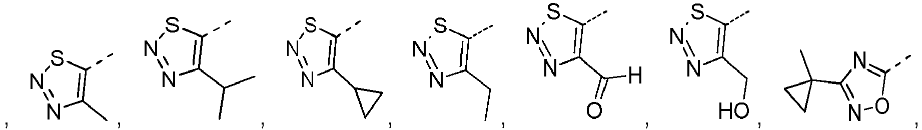 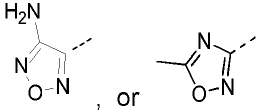 and
**R⁶** represents -H, -CH₃, -CH(CH₃)₂, -CH₂CH₂CH₃, -CH₂CH=CH₂, -CH₂CH₂CH₂CH₃, -CH₂CH(CH₃)₂, -C(CH₃)₃, -CH₂CH₂CH₂CH₂CH₃, -cyclo-C₃H₅, -cyclo-C₅H₉, -cyclo-C₆H₁₁ or -CH₂-cyclo-C₃H₅.

9. The compound according to claim 1 selected from the group consisting of:
| Compound | Name |
|---|---|
| **II-2**: | (S)-2-(benzofuran-2-carboxamido)-N1-(1-(2-(2-adamantylamino)-2-oxoethyl)-2-oxo-1,2-dihydropyridin-3-yl)-N6-methyl-5-oxohexanediamide |
| **II-3**: | (S)-N1-(1-(2-(2-adamantylamino)-2-oxoethyl)-2-oxo-1,2-dihydropyridin-3-yl)-N6-methyl-2-(3-methylbenzofuran-2-carboxamido)-5-oxohexanediamide |
| **II-4**: | (S)-2-(3-chlorobenzofuran-2-carboxamido)-N1-(1-(2-(2-adamantylamino)-2-oxoethyl)-2-oxo-1,2-dihydropyridin-3-yl)-N6-methyl-5-oxohexanediamide |
| **II-5**: | (S)-2-(4-bromobenzofuran-2-carboxamido)-N1 -(1 -(2-(2-adamantylamino)-2-oxoethyl)-2-oxo-1,2-dihydropyridin-3-yl)-N6-methyl-5-oxohexanediamide |
| **II-6**: | (S)-2-(4-bromobenzofuran-2-carboxamido)-N1-(1-(2-(2-adamantylamino)-2-oxoethyl)-2-oxo-1,2-dihydropyridin-3-yl)-N6-methyl-5-oxohexanediamide |
| **II-7:** | (S)-2-(benzo[b]thiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-8**: | (S)-2-(5-bromobenzo[b]thiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-9**: | (S)-2-(1H-indole-2-carboxamido)-N1-(1-(2-(2-adamantylamino)-2-oxoethyl)-2-oxo-1,2-dihydropyridin-3-yl)-N6-methyl-5-oxohexanediamide |
| **II-10**: | (S)-2-(4,5-difluoro-1H-indole-2-carboxamido)-N1-(1-(2-(2-adamantylamino)-2-oxoethyl)-2-oxo-1,2-dihydropyridin-3-yl)-N6-methyl-5-oxohexanediamide |
| **II-11**: | (S)-N1-(1-(2-(2-adamantylamino)-2-oxoethyl)-2-oxo-1,2-dihydropyridin-3-yl)-N6-methyl-2-(3-methyl-1H-indole-2-carboxamido)-5-oxohexanediamide |
| **II-12**: | (S)-2-(1H-benzo[d]imidazole-2-carboxamido)-N1-(1-(2-(2-adamantylamino)-2-oxoethyl)-2-oxo-1,2-dihydropyridin-3-yl)-N6-methyl-5-oxohexanediamide |
| **II-13**: | (S)-2-(2,3-dihydro-1H-indene-2-carboxamido)-N1-(1-(2-(2-adamantylamino)-2-oxoethyl)-2-oxo-1,2-dihydropyridin-3-yl)-N6-methyl-5-oxohexanediamide |
| **II-14:** | (S)--(2-bromo-4-methylthiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-15**: | (S)-N1-(1-(2-(2-adamantylamino)-2-oxoethyl)-2-oxo-1,2-dihydropyridin-3-yl)-N6-methyl-2-(4-methyl-2-(trifluoromethyl)thiazole-5-carboxamido)-5-oxohexanediamide |
| **II-16**: | (S)-2-(4-bromo-2-(trifluoromethyl)thiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-17**: | (S)-2-(2,4-dichlorothiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-18**: | (S)-N1-(1-(2-(2-adamantylamino)-2-oxoethyl)-2-oxo-1,2-dihydropyridin-3-yl)-2-(2-methoxy-4-methylthiazole-5-carboxamido)-N6-methyl-5-oxohexanediamide |
| **II-19**: | (S)-N1-(1-(2-(2-adamantylamino)-2-oxoethyl)-2-oxo-1,2-dihydropyridin-3-yl)-N6-methyl-2-(4-methyl-2-phenylthiazole-5-carboxam ido)-5-oxohexanediamide |
| **II-20**: | (S)-2-(2,4-dimethylthiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-21**: | (S)-2-(5-bromo-3-methylthiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-22**: | (S)-2-(3,5-dibromothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-23**: | (S)-2-(5-bromothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-24:** | (S)-2-(5-chlorothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-25**: | (S)-2-(5-bromo-3-methylfuran-2-carboxamido)-N1-(1-(2-(2-adamantylamino)-2-oxoethyl)-2-oxo-1,2-dihydropyridin-3-yl)-N6-methyl-5-oxohexanediamide |
| **II-26**: | (S)-2-(5-chlorofuran-2-carboxamido)-N1-(1-(2-(2-adamantylamino)-2-oxoethyl)-2-oxo-1,2-dihydropyridin-3-yl)-N6-methyl-5-oxohexanediamide |
| **II-27**: | (S)-2-(5-chlorothiophene-3-carboxamido)-N1-(1-(2-(2-adamantylamino)-2-oxoethyl)-2-oxo-1,2-dihydropyridin-3-yl)-N6-methyl-5-oxohexanediamide |
| **II-28**: | (S)-2-(2,5-dichlorothiophene-3-carboxamido)-N1-(1-(2-(2-adamantylamino)-2-oxoethyl)-2-oxo-1,2-dihydropyridin-3-yl)-N6-methyl-5-oxohexanediamide |
| **II-29**: | (S)-2-(2,5-dibromothiophene-3-carboxamido)-N1-(1-(2-(2-adamantylamino)-2-oxoethyl)-2-oxo-1,2-dihydropyridin-3-yl)-N6-methyl-5-oxohexanediamide |
| **II-30**: | (S)-2-(5-bromothiophene-3-carboxamido)-N1-(1-(2-(2-adamantylamino)-2-oxoethyl)-2-oxo-1,2-dihydropyridin-3-yl)-N6-methyl-5-oxohexanediamide |
| **II-31**: | (S)-2-(2-chloro-5-methylthiazole-4-carboxamido)-N1-(1-(2-(2-adamantylamino)-2-oxoethyl)-2-oxo-1,2-dihydropyridin-3-yl)-N6-methyl-5-oxohexanediamide |
| **II-32**: | (S)-2-(2,5-dichlorothiazole-4-carboxamido)-N1-(1-(2-(2-adamantylamino)-2-oxoethyl)-2-oxo-1,2-dihydropyridin-3-yl)-N6-methyl-5-oxohexanediamide |
| **II-33**: | (S)-2-(2,5-dibromothiazole-4-carboxamido)-N1-(1-(2-(2-adamantylamino)-2-oxoethyl)-2-oxo-1,2-dihydropyridin-3-yl)-N6-methyl-5-oxohexanediamide |
| **II-34:** | (S)-2-(2-bromo-5-methylthiazole-4-carboxamido)-N1-(1-(2-(2-adamantylamino)-2-oxoethyl)-2-oxo-1,2-dihydropyridin-3-yl)-N6-methyl-5-oxohexanediamide |
| **II-35:** | (S)-2-(2-bromothiazole-4-carboxamido)-N1-(1-(2-(2-adamantylamino)-2-oxoethyl)-2-oxo-1,2-dihydropyridin-3-yl)-N6-methyl-5-oxohexanediamide |
| **II-36**: | (S)-2-(2-chlorothiazole-4-carboxamido)-N1-(1-(2-(2-adamantylamino)-2-oxoethyl)-2-oxo-1,2-dihydropyridin-3-yl)-N6-methyl-5-oxohexanediamide |
| **II-37**: | (S)-2-(2,5-dimethylfuran-3-carboxamido)-N1-(1-(2-(2-adamantylamino)-2-oxoethyl)-2-oxo-1,2-dihydropyridin-3-yl)-N6-methyl-5-oxohexanediamide |
| **II-38**: | (S)-2-(4,5-dimethylthiazole-2-carboxamido)-N1-(1-(2-(2-adamantylamino)-2-oxoethyl)-2-oxo-1,2-dihydropyridin-3-yl)-N6-methyl-5-oxohexanediamide |
| **II-39:** | (S)-2-(4-bromothiazole-2-carboxamido)-N1-(1-(2-(2-adamantylamino)-2-oxoethyl)-2-oxo-1,2-dihydropyridin-3-yl)-N6-methyl-5-oxohexanediamide |
| **II-40:** | (S)-2-(4-bromothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-41**: | (S)-2-(4-bromo-3-methylthiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-42:** | (S)-2-(3-bromothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-43**: | (S)-2-(3-chloro-4-methylthiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-44:** | (S)-2-(4-bromo-5-chlorothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-45:** | (S)-2-(4,5-dibromothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-46:** | (S)-2-(4,5-dibromo-3-methoxythiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-47:** | (S)-2-(4-bromofuran-2-carboxamido)-N1-(1-(2-(2-adamantylamino)-2-oxoethyl)-2-oxo-1,2-dihydropyridin-3-yl)-N6-methyl-5-oxohexanediamide |
| **II-48:** | (S)-2-(4,5-dibromofuran-2-carboxamido)-N1-(1-(2-(2-adamantylamino)-2-oxoethyl)-2-oxo-1,2-dihydropyridin-3-yl)-N6-methyl-5-oxohexanediamide |
| **II-49:** | (S)-2-(4,5-dichlorothiophene-2-carboxamido)-N1-(1-(2-(2-adamantylamino)-2-oxoethyl)-2-oxo-1,2-dihydropyridin-3-yl)-N6-methyl-5-oxohexanediamide |
| **II-50**: | (S)-2-((S)-1-acetylpyrrolidine-2-carboxamido)-N1-(1-(2-(2-adamantylamino)-2-oxoethyl)-2-oxo-1,2-dihydropyridin-3-yl)-N6-methyl-5-oxohexanediamide |
| **II-51:** | (S)-N1-(1-(2-(2-adamantylamino)-2-oxoethyl)-2-oxo-1,2-dihydropyridin-3-yl)-N6-methyl-2-(1-methyl-1H-1,2,3-triazole-5-carboxamido)-5-oxohexanediamide |
| **II-52**: | (S)-N1-(1-(2-(2-adamantylamino)-2-oxoethyl)-2-oxo-1,2-dihydropyridin-3-yl)-N6-methyl-5-oxo-2-(2H-tetrazole-5-carboxamido)hexanediamide |
| **II-53:** | (S)-N1-(1-(2-(2-adamantylamino)-2-oxoethyl)-2-oxo-1,2-dihydropyridin-3-yl)-N6-methyl-5-oxo-2-(pyrazine-2-carboxamido)hexanediamide |
| **II-54:** | (S)-N1-(1-(2-(2-adamantylamino)-2-oxoethyl)-2-oxo-1,2-dihydropyridin-3-yl)-N6-methyl-2-((S)-1-methylpyrrolidine-2-carboxamido)-5-oxohexanediamide |
| **II-55:** | (S)-N1-(1-(2-(2-adamantylamino)-2-oxoethyl)-2-oxo-1,2-dihydropyridin-3-yl)-N6-methyl-5-oxo-2-((S)-pyrrolidine-3-carboxamido)hexanediamide |
| **II-56**: | (S)-2-((2S,4S)-4-bromopyrrolidine-2-carboxamido)-N1-(1-(2-(2-adamantylamino)-2-oxoethyl)-2-oxo-1,2-dihydropyridin-3-yl)-N6-methyl-5-oxohexanediamide |
| **II-58:** | (S)-N1-(1-(2-(2-adamantylamino)-2-oxoethyl)-2-oxo-1,2-dihydropyridin-3-yl)-N6-methyl-5-oxo-2-((S)-piperidine-2-carboxamido)hexanediamide |
| **II-59:** | (S)-N1-(1-(2-(2-adamantylamino)-2-oxoethyl)-2-oxo-1,2-dihydropyridin-3-yl)-N6-methyl-5-oxo-2-((R)-piperidine-3-carboxamido)hexanediamide |
| **II-60**: | (S)-N1-(1-(2-(2-adamantylamino)-2-oxoethyl)-2-oxo-1,2-dihydropyridin-3-yl)-N6-methyl-2-((R)-morpholine-3-carboxamido)-5-oxohexanediamide |
| **II-61**: | (S)-N1-(1-(2-(2-adamantylamino)-2-oxoethyl)-2-oxo-1,2-dihydropyridin-3-yl)-N6-methyl-5-oxo-2-(quinuclidine-3-carboxamido)hexanediamide |
| **II-62**: | (S)-methyl 3-(1-(1-(2-(2-adamantylamino)-2-oxoethyl)-2-oxo-1,2-dihydropyridin-3-ylamino)-6-(methylamino)-1,5,6-trioxohexan-2-ylcarbamoyl)-5-nitrobenzoate |
| **II-63:** | (S)-N1-(1-(2-(2-adamantylamino)-2-oxoethyl)-2-oxo-1,2-dihydropyridin-3-yl)-N6-methyl-2-(5-nitronicotinamido)-5-oxohexanediamide |
| **II-64:** | (S)-5-(1-(1-(2-(2-adamantylamino)-2-oxoethyl)-2-oxo-1,2-dihydropyridin-3-ylamino)-6-(methylamino)-1,5,6-trioxohexan-2-ylcarbamoyl)nicotinic acid |
| **II-65**: | (S)-methyl 5-(1-(1-(2-(2-adamantylamino)-2-oxoethyl)-2-oxo-1,2-dihydropyridin-3-ylamino)-6-(methylamino)-1,5,6-trioxohexan-2-ylcarbamoyl)nicotinate |
| **II-66**: | (S)-N1-(1-(2-(2-adamantylamino)-2-oxoethyl)-2-oxo-1,2-dihydropyridin-3-yl)-N6-methyl-2-(6-methylimidazo[2,1-b]thiazole-5-carboxamido)-5-oxohexanediamide |
| **II-67**: | (S)-N1-(1-(2-(2-adamantyl(methyl)amino)-2-oxoethyl)-2-oxo-1,2-dihydropyridin-3-yl)-N6-methyl-2-(3-methylbenzofuran-2-carboxamido)-5-oxohexanediamide |
| **II-68**: | (S)-N1-(1-(2-(5-hydroxyadamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-69**: | (S)-N1-(1-(2-(5-fluoroadamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-70:** | (S)-N1-(1-(2-(5-chloroadamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-71**: | (S)-N1-(1-(2-(5-bromoadamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-72**: | (S)-N1-(1-(2-(5-methyladamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-73:** | (S)-N 1-(1-(2-(2-carbonitrileadamantane-2-amino)-2-oxoethyl)-2-oxo-1,2-dihydropyridin-3-yl)-N6-methyl-2-(3-methylbenzofuran-2-carboxamido)-5-oxohexanediamide |
| **II-74:** | (S)-N1-(1-(2-(2-methyl adamantane-2-carboxylate-2-amino)-2-oxoethyl)-2-oxo-1,2-dihydropyridin-3-yl)-N6-methyl-2-(3-methylbenzofuran-2-carboxamido)-5-oxohexanediamide |
| **II-87**: | (S)-N1-(1-(2-(1-adamantylmethylamino)-2-oxoethyl)-2-oxo-1,2-dihydropyridin-3-yl)-N6-methyl-2-(3-methylbenzofuran-2-carboxamido)-5-oxohexanediamide |
| **II-88**: | (S)-N1-(1-(2-(1-(1-adamantyl)ethanamino)-2-oxoethyl)-2-oxo-1,2-dihydropyridin-3-yl)-N6-methyl-2-(3-methylbenzofuran-2-carboxamido)-5-oxohexanediamide |
| **II-90**: | (S)-N1-(1-(2-((1R,2S,4S)-bicyclo[2.2.1]heptan-2-ylamino)-2-oxoethyl)-2-oxo-1,2-dihydropyridin-3-yl)-N6-methyl-2-(3-methylbenzofuran-2-carboxamido)-5-oxohexanediamide |
| **II-92**: | (S)-N1-methyl-5-(3-methylbenzofuran-2-carboxamido)-2-oxo-N6-(2-oxo-1-(2-oxo-2-((1S,2S,4R)-1,7,7-trimethylbicyclo[2.2.1]heptan-2-ylamino)ethyl)-1,2-dihydropyridin-3-yl)hexanediamide |
| **II-94:** | (S)-N1-(1-(2-((1R,2R,4S)-bicyclo[2.2.1]heptan-2-ylamino)-2-oxoethyl)-2-oxo-1,2-dihydropyridin-3-yl)-N6-methyl-2-(3-methylbenzofuran-2-carboxamido)-5-oxohexanediamide |
| **II-95**: | (S)-N1-(1-(2-(bicyclo[2.2.1]heptan-1-ylamino)-2-oxoethyl)-2-oxo-1,2-dihydropyridin-3-yl)-N6-methyl-2-(3-methylbenzofuran-2-carboxamido)-5-oxohexanediamide |
| **II-96**: | (S)-N1-(1-(2-(bicyclo[2.2.1]heptan-7-ylamino)-2-oxoethyl)-2-oxo-1,2-dihydropyridin-3-yl)-N6-methyl-2-(3-methylbenzofuran-2-carboxamido)-5-oxohexanediamide |
| **II-97**: | (S)-N1-(1-(2-(bicyclo[2.2.1]hept-5-en-2-ylamino)-2-oxoethyl)-2-oxo-1,2-dihydropyridin-3-yl)-N6-methyl-2-(3-methylbenzofuran-2-carboxamido)-5-oxohexanediamide |
| **II-98**: | (2S)-N1-(1-(2-(bicyclo[2.2.2]octan-2-ylamino)-2-oxoethyl)-2-oxo-1,2-dihydropyridin-3-yl)-N6-methyl-2-(3-methylbenzofuran-2-carboxamido)-5-oxohexanediamide |
| **II-99**: | (S)-N1-methyl-5-(3-methylbenzofuran-2-carboxamido)-2-oxo-N6-(2-oxo-1-(2-oxo-2-((1R,2R,4R)-1,7,7-trimethylbicyclo[2.2.1]heptan-2-ylamino)ethyl)-1,2-dihydropyridin-3-yl)hexanediamide |
| **II-100:** | (S)-N1-methyl-5-(3-methylbenzofuran-2-carboxamido)-2-oxo-N6-(2-oxo-1-(2-oxo-2-((1R,2R,3R,5S)-2,6,6-trimethylbicyclo[3.1.1]heptan-3-ylamino)ethyl)-1,2-dihydropyridin-3-yl)hexanediamide |
| **II-101**: | (S)-N1-methyl-5-(3-methylbenzofuran-2-carboxamido)-2-oxo-N6-(2-oxo-1-(2-oxo-2-((1S,2S,3S,5R)-2,6,6-trimethylbicyclo[3.1.1]heptan-3-ylamino)ethyl)-1,2-dihydropyridin-3-yl)hexanediamide |
| **II-103:** | (S)-N1-(1-(2-(4-homoisotwistane-3-amino)-2-oxoethyl)-2-oxo-1,2-dihydropyridin-3-yl)-N6-methyl-2-(3-methylbenzofuran-2-carboxamido)-5-oxohexanediamide |
| **II-104:** | (S)-N1-(1-(2-(diamantane-1-amino)-2-oxoethyl)-2-oxo-1,2-dihydropyridin-3-yl)-N6-methyl-2-(3-methylbenzofuran-2-carboxamido)-5-oxohexanediamide |
| **II-105:** | (S)-N1-(1-(2-(diamantane-4-amino)-2-oxoethyl)-2-oxo-1,2-dihydropyridin-3-yl)-N6-methyl-2-(3-methylbenzofuran-2-carboxamido)-5-oxohexanediamide |
| **II-107:** | (S)-N1-(1-(1-adamantylmethyl)-2-oxo-1,2-dihydropyridin-3-yl)-N6-methyl-2-(3-methylbenzofuran-2-carboxamido)-5-oxohexanediamide |
| **II-108:** | (2S)-N1-(1-((3-hydroxy-1-adamantyl)methyl)-2-oxo-1,2-dihydropyridin-3-yl)-N6-methyl-2-(3-methylbenzofuran-2-carboxamido)-5-oxohexanediamide |
| **II-109:** | (2S)-N1-(1-((3-bromo-1-adamantyl)methyl)-2-oxo-1,2-dihydropyridin-3-yl)-N6-methyl-2-(3-methylbenzofuran-2-carboxamido)-5-oxohexanediamide |
| **II-110:** | (S)-N1-(1-(2-adamantylmethyl)-2-oxo-1,2-dihydropyridin-3-yl)-N6-methyl-2-(3-methylbenzofuran-2-carboxamido)-5-oxohexanediamide |
| **II-111**: | (S)-N1-(1-(2-(2-adamantylamino)-2-oxoethyl)-2-oxo-1,2-dihydropyridin-3-yl)-N6-methyl-2-(nicotinamido)-5-oxohexanediamide |
| **II-112**: | (S)-2-(isonicotinamido)-N1-(1-(2-(2-adamantylamino)-2-oxoethyl)-2-oxo-1,2-dihydropyridin-3-yl)-N6-methyl-5-oxohexanediamide |
| **II-113**: | (S)-N1-(1-(2-(2-adamantylamino)-2-oxoethyl)-2-oxo-1,2-dihydropyridin-3-yl)-N6-methyl-5-oxo-2-(pyridazine-4-carboxamido)hexanediamide |
| **II-114:** | (S)-N1-(1-(2-(2-adamantylamino)-2-oxoethyl)-2-oxo-1,2-dihydropyridin-3-yl)-N6-methyl-5-oxo-2-(pyridazine-3-carboxamido)hexanediamide |
| **II-115**: | (S)-N1-cyclopropyl-N6-(1-(2-(2-adamantylamino)-2-oxoethyl)-2-oxo-1,2-dihydropyridin-3-yl)-5-(3-methylbenzofuran-2-carboxamido)-2-oxohexanediamide |
| **II-116**: | (S)-N1-(1-(2-(2-adamantylamino)-2-oxoethyl)-2-oxo-1,2-dihydropyridin-3-yl)-2-(3-methylbenzofuran-2-carboxamido)-5-oxoN6-pentylhexanediamide |
| **II-117**: | (S)-N1-allyl-N6-(1-(2-(2-adamantylamino)-2-oxoethyl)-2-oxo-1,2-dihydropyridin-3-yl)-5-(3-methylbenzofuran-2-carboxamido)-2-oxohexanediamide |
| **II-118**: | (S)-N1-(1-(2-(2-adamantylamino)-2-oxoethyl)-2-oxo-1,2-dihydropyridin-3-yl)-2-(3-methylbenzofuran-2-carboxamido)-5-oxohexanediamide |
| **II-119**: | (S)-N1-allyl-5-(benzofuran-2-carboxamido)-N6-(1-(2-(2-adamantylamino)-2-oxoethyl)-2-oxo-1,2-dihydropyridin-3-yl)-2-oxohexanediamide |
| **II-120**: | (S)-2-(benzofuran-2-carboxamido)-N6-isopropyl-N1-(1-(2-(2-adamantylamino)-2-oxoethyl)-2-oxo-1,2-dihydropyridin-3-yl)-5-oxohexanediamide |
| **II-121**: | (S)-2-(benzofuran-2-carboxamido)-N6-cyclopropyl-N1-(1 -(2-(2-adamantylamino)-2-oxoethyl)-2-oxo-1,2-dihydropyridin-3-yl)-5-oxohexanediamide |
| **II-122**: | (S)-2-(benzofuran-2-carboxamido)-N1-(1-(2-(2-adamantylamino)-2-oxoethyl)-2-oxo-1,2-dihydropyridin-3-yl)-5-oxo-N6-phenylhexanediamide |
| **II-123**: | (S)-2-(benzofuran-2-carboxamido)-N6-benzyl-N1-(1-(2-(2-adamantylamino)-2-oxoethyl)-2-oxo-1,2-dihydropyridin-3-yl)-5-oxohexanediamide |
| **II-124:** | (S)-2-(benzofuran-2-carboxamido)-N1-(1-(2-(2-adamantylamino)-2-oxoethyl)-2-oxo-1,2-dihydropyridin-3-yl)-5-oxohexanediamide |
| **II-125**: | (S)-2-(2,5-dichlorothiophene-3-carboxamido)-N1-(1-(2-(2-adamantylamino)-2-oxoethyl)-2-oxo-1,2-dihydropyridin-3-yl)-5-oxohexanediamide |
| **II-126**: | (S)-N1-(1-(2-(2-adamantylamino)-2-oxoethyl)-2-oxo-1,2-dihydropyridin-3-yl)-2-(4-methyl-2-(trifluoromethyl)thiazole-5-carboxamido)-5-oxohexanediamide |
| **II-127**: | (S)-N1-(1-(2-(2-adamantylamino)-2-oxoethyl)-2-oxo-1,2-dihydropyridin-3-yl)-2-(1-methyl-1H-1,2,3-triazole-5-carboxamido)-5-oxohexanediamide |
| **II-128**: | (2S)-N1-(1-(2-((1R,2R,4S)-bicyclo[2.2.1]heptan-2-ylamino)-2-oxoethyl)-2-oxo-1,2-dihydropyridin-3-yl)-2-(2,5-dichlorothiophene-3-carboxamido)-N6-methyl-5-oxohexanediamide |
| **II-129**: | (2S)-N1-(1-(2-((1R,2R,4S)-bicyclo[2.2.1]heptan-2-ylamino)-2-oxoethyl)-2-oxo-1,2-dihydropyridin-3-yl)-N6-methyl-2-(4-methyl-2-(trifluoromethyl)thiazole-5-carboxamido)-5-oxohexanediamide |
| **II-130**: | (2S)-N1-(1-(2-((1R,2R,4S)-bicyclo[2.2.1]heptan-2-ylamino)-2-oxoethyl)-2-oxo-1,2-dihydropyridin-3-yl)-N6-methyl-2-(1-methyl-1H-1,2,3-triazole-5-carboxamido)-5-oxohexanediamide |
| **II-131**: | (S)-N1-(1-(2-(2-adamantylamino)-2-oxoethyl)-2-oxo-1 ,2-dihydropyridin-3-yl)-N6-methyl-5-oxo-2-(2H-1,2,3-triazole-4-carboxamido)hexanediamide |
| **II-132**: | (S)-N1-(1-(2-(2-adamantylamino)-2-oxoethyl)-2-oxo-1,2-dihydropyridin-3-yl)-N6-methyl-5-oxo-2-(1H-1,2,3-triazole-4-carboxamido)hexanediamide |
| **II-133**: | (S)-N1-(1-(2-(2-adamantylamino)-2-oxoethyl)-2-oxo-1,2-dihydropyridin-3-yl)-N6-methyl-2-(1-methyl-1H-1,2,3-triazole-4-carboxamido)-5-oxohexanediamide |
| **II-134**: | (S)-N1-(1-(2-(2-adamantylamino)-2-oxoethyl)-2-oxo-1,2-dihydropyridin-3-yl)-N6-methyl-5-oxo-2-(1H-1,2,4-triazole-3-carboxamido)hexanediamide |
| **II-135**: | (S)-N1-(1-(2-(2-adamantylamino)-2-oxoethyl)-2-oxo-1,2-dihydropyridin-3-yl)-N6-methyl-2-(1-methyl-1H-1,2,4-triazole-3-carboxamido)-5-oxohexanediamide |
| **II-136**: | (S)-2-(benzofuran-3-carboxamido)-N1-(1-(2-(2-adamantylamino)-2-oxoethyl)-2-oxo-1,2-dihydropyridin-3-yl)-N6-methyl-5-oxohexanediamide |
| **II-137**: | (S)-2-(benzo[b]thiophene-3-carboxamido)-N1-(1-(2-(2-adamantylamino)-2-oxoethyl)-2-oxo-1,2-dihydropyridin-3-yl)-N6-methyl-5-oxohexanediamide |
| **II-138** | (S)-N1-(1-(2-(2-adamantylamino)-2-oxoethyl)-2-oxo-1,2-dihydropyridin-3-yl)-N6-methyl-2-(1-methyl-1H-pyrazole-3-carboxamido)-5-oxohexanediamide |
| **II-139** | (S)-N1-(1-(2-(2-adamantylamino)-2-oxoethyl)-2-oxo-1,2-dihydropyridin-3-yl)-N6-methyl-2-(1-methyl-1H-pyrazole-4-carboxamido)-5-oxohexanediamide |
| **II-140** | (S)-N1-(1-(2-(2-adamantylamino)-2-oxoethyl)-2-oxo-1,2-dihydropyridin-3-yl)-N6-methyl-2-(1-methyl-1H-pyrazole-5-carboxamido)-5-oxohexanediamide |
| **II-141** | (S)-N1-(1-(2-(2-adamantylamino)-2-oxoethyl)-2-oxo-1,2-dihydropyridin-3-yl)-N6-methyl-2-(4-methyl-1,2,3-thiadiazole-5-carboxamido)-5-oxohexanediamide |
| **II-142** | (S)-N1-(1-(2-(2-adamantylamino)-2-oxoethyl)-2-oxo-1,2-dihydropyridin-3-yl)-N6-methyl-5-oxo-2-(1,2,5-thiadiazole-3-carboxamido)hexanediamide |
| **II-143** | (S)-2-(4-iodo-1-methyl-1H-pyrazole-5-carboxamido)-N1-(1-(2-(2-adamantylamino)-2-oxoethyl)-2-oxo-1,2-dihydropyridin-3-yl)-N6-methyl-5-oxohexanediamide |
| **II-144** | (S)-N1-(1-(2-(2-adamantylamino)-2-oxoethyl)-2-oxo-1,2-dihydropyridin-3-yl)-2-(1-methyl-1H-pyrazole-5-carboxamido)-5-oxohexanediamide |
| **II-145** | (S)-N1-(1-(2-(2-adamantylamino)-2-oxoethyl)-2-oxo-1,2-dihydropyridin-3-yl)-2-(4-methyl-1,2,3-thiadiazole-5-carboxamido)-5-oxohexanediamide |
| **II-146** | (S)-2-(benzofuran-2-carboxamido)-N1-(1-(2-((1R,2R,4S)-bicyclo[2.2.1]heptan-2-ylamino)-2-oxoethyl)-2-oxo-1,2-dihydropyridin-3-yl)-5-oxohexanediamide |
| **II-147** | (S)-N1-(1-(2-((1R,2R,4S)-bicyclo[2.2.1]heptan-2-ylamino)-2-oxoethyl)-2-oxo-1,2-dihydropyridin-3-yl)-2-(3-methylbenzofuran-2-carboxam ido)-5-oxohexanediamide |
| **II-148** | (S)-2-(benzofuran-2-carboxamido)-N1-(1-(2-((1S,2R,4R)-bicyclo[2.2.1]heptan-2-ylamino)-2-oxoethyl)-2-oxo-1,2-dihydropyridin-3-yl)-5-oxohexanediamide |
| **II-149** | (S)-N1-(1-(2-((1S,2R,4R)-bicyclo[2.2.1]heptan-2-ylamino)-2-oxoethyl)-2-oxo-1,2-dihydropyridin-3-yl)-2-(3-methylbenzofuran-2-carboxamido)-5-oxohexanediamide |
| **II-150** | (S)-2-(benzofuran-2-carboxamido)-5-oxo-N1-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-151** | (S)-2-(3-methylbenzofuran-2-carboxamido)-5-oxo-N1-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-152** | (S)-N1-(1-(2-((1R,2R,4S)-bicyclo[2.2.1]heptan-2-ylamino)-2-oxoethyl)-2-oxo-1,2-dihydropyridin-3-yl)-N6-methyl-2-(4-methyl-1,2,3-thiadiazole-5-carboxamido)-5-oxohexanediamide |
| **II-153** | (S)-N1-(1-(2-((1R,2R,4S)-bicyclo[2.2.1]heptan-2-ylamino)-2-oxoethyl)-2-oxo-1,2-dihydropyridin-3-yl)-N6-methyl-2-(1-methyl-1H-pyrazole-5-carboxamido)-5-oxohexanediamide |
| **II-154** | (S)-N1-(1-(2-((1S,2R,4R)-bicyclo[2.2.1]heptan-2-ylamino)-2-oxoethyl)-2-oxo-1,2-dihydropyridin-3-yl)-N6-methyl-2-(4-methyl-2-(trifluoromethyl)thiazole-5-carboxamido)-5-oxohexanediamide |
| **II-155** | (S)-N1-(1-(2-((1S,2R,4R)-bicyclo[2.2.1]heptan-2-ylamino)-2-oxoethyl)-2-oxo-1,2-dihydropyridin-3-yl)-2-(2,5-dichlorothiophene-3-carboxamido)-N6-methyl-5-oxohexanediamide |
| **II-156** | (S)-N1-(1-(2-((1S,2R,4R)-bicyclo[2.2.1]heptan-2-ylamino)-2-oxoethyl)-2-oxo-1,2-dihydropyridin-3-yl)-N6-methyl-2-(4-methyl-1,2,3-thiadiazole-5-carboxamido)-5-oxohexanediamide |
| **II-157** | (S)-N1-(1-(2-((1S,2R,4R)-bicyclo[2.2.1]heptan-2-ylamino)-2-oxoethyl)-2-oxo-1,2-dihydropyridin-3-yl)-N6-methyl-2-(1-methyl-1H-1,2,3-triazole-5-carboxamido)-5-oxohexanediamide |
| **II-158** | (S)-N1-(1-(2-((1S,2R,4R)-bicyclo[2.2.1]heptan-2-ylamino)-2-oxoethyl)-2-oxo-1,2-dihydropyridin-3-yl)-N6-methyl-2-(1-methyl-1H-pyrazole-5-carboxamido)-5-oxohexanediamide |
| **II-159** | (S)-N 1-methyl-5-(4-methyl-2-(trifluoromethyl)thiazole-5-carboxamido)-2-oxo-N6-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-160** | (S)-2-(2,5-dichlorothiophene-3-carboxamido)-N6-methyl-5-oxo-N1-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-161** | (S)-N1-methyl-5-(4-methyl-1,2,3-thiadiazole-5-carboxamido)-2-oxoN6-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-162** | (S)-N1-methyl-5-(1-methyl-1H-1,2,3-triazole-5-carboxamido)-2-oxo-N6-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-163** | (S)-N1-methyl-5-(1-methyl-1 H-pyrazole-5-carboxamido)-2-oxo-N6-(2-oxo-1-(2-oxo-2-((1R,2S,4R)-1,7,7-trimethylbicyclo[2.2.1]heptan-2-ylamino)ethyl)-1,2-dihydropyridin-3-yl)hexanediamide |
| **II-164** | (2S)-2-(4-tert-butyl-1H-pyrrole-3-carboxamido)-N6-methyl-N1-(1-(2-(1-adamantylamino)ethyl)-2-oxo-1,2-dihydropyridin-3-yl)-5-oxohexanediamide |
| **II-165** | (2S)-2-(4-cyano-1-methyl-1H-pyrrole-2-carboxamido)-N6-methyl-N1-(1-(3-(1-adamantylamino)propyl)-2-oxo-1,2-dihydropyridin-3-yl)-5-oxohexanediamide |
| **II-166** | (S)-N1-(1-(3-(2-adamantylamino)-3-oxopropyl)-2-oxo-1,2-dihydropyridin-3-yl)-2-(5-methoxyoxazole-2-carboxamido)-N6-methyl-5-oxohexanediamide |
| **II-167** | (S)-N1-(1-(2-(bicyclo[1.1.1]pentan-1-ylam ino)-2-oxoethyl)-2-oxo-1,2-dihydropyridin-3-yl)-N6-methyl-2-(3-methylbenzofuran-2-carboxamido)-5-oxohexanediamide |
| **II-168** | (S)-2-(2-acetyloxazole-4-carboxamido)-N1-(1-(2-(bicyclo[1.1.1]pentan-1-ylamino)-2-oxoethyl)-2-oxo-1,2-dihydropyridin-3-yl)-N6-methyl-5-oxohexanediamide |
| **II-169** | (S)-N1-(1-(2-(bicyclo[2.1.1]hexan-1-ylamino)-2-oxoethyl)-2-oxo-1,2-dihydropyridin-3-yl)-N6-methyl-2-(3-methylbenzofuran-2-carboxamido)-5-oxohexanediamide |
| **II-170** | (S)-N1-(1-(2-(bicyclo[2.1.1]hexan-1-ylamino)-2-oxoethyl)-2-oxo-1,2-dihydropyridin-3-yl)-2-(2-isopropyloxazole-5-carboxamido)-N6-methyl-5-oxohexanediamide |
| **II-171** | (2S)-2-(benzofuran-2-carboxamido)-N1-(1-(2-(bicyclo[3.2.1]octan-8-ylamino)-2-oxoethyl)-2-oxo-1,2-dihydropyridin-3-yl)-N6-methyl-5-oxohexanediamide |
| **II-172** | (2S)-N1-(1-(2-(bicyclo[3.2.1]octan-8-ylamino)-2-oxoethyl)-2-oxo-1,2-dihydropyridin-3-yl)-2-(3,5-dimethylisoxazole-4-carboxamido)-N6-methyl-5-oxohexanediamide |
| **II-173** | (S)-N1-(1-(2-(5-carboxy-2-aminoadamantane)-2-oxoethyl)-2-oxo-1,2-dihydropyridin-3-yl)-N6-methyl-2-(4-methylpyrimidine-5-carboxamido)-5-oxohexanediamide |
| **II-174** | (2S)-N 1-(1-(2-(4-aminoadamantane-N, N-dimethyl-1-carboxamide)-2-oxoethyl)-2-oxo-1,2-dihydropyridin-3-yl)-N6-methyl-5-oxo-2-(1,2,3,4-tetrahydronaphthalene-2-carboxamido)hexanediamide |
| **II-175** | (S)-2-((S)-1,4-diazabicyclo[2.2.2]octane-2-carboxamido)-N6-tert-butyl-N1-(1-(2-(2-adamantylamino)-2-oxoethyl)-2-oxo-1,2-dihydropyridin-3-yl)-5-oxohexanediamide |
| **II-176** | (S)-N1-tert-butyl-5-(1H-indole-3-carboxamido)-N6-(1-(2-(2-adamantylamino)-2-oxoethyl)-2-oxo-1,2-dihydropyridin-3-yl)-2-oxohexanediamide |
| **II-177** | (S)-N1-tert-butyl-N6-(1-(2-(2-adamantylamino)-2-oxoethyl)-2-oxo-1,2-dihydropyridin-3-yl)-5-(6-methylimidazo[2,1-b]thiazole-3-carboxamido)-2-oxohexanediamide |
| **II-178** | (S)-2-(benzo[d]thiazole-2-carboxamido)-N1-(1-(2-((1S,2R,4R)-bicyclo[2.2.1]heptan-2-ylamino)-2-oxoethyl)-2-oxo-1,2-dihydropyridin-3-yl)-N6-cyclopentyl-5-oxohexanediamide |
| **II-179** | (S)-N1-(1-(2-((1S,2R,4R)-bicyclo[2.2.1]heptan-2-ylamino)-2-oxoethyl)-2-oxo-1,2-dihydropyridin-3-yl)-N6-cyclopentyl-2-(imidazo[2,1-b]thiazole-6-carboxamido)-5-oxohexanediamide |
| **II-180** | (S)-N1-(1-(2-((1S,2R,4R)-bicyclo[2.2.1]heptan-2-ylamino)-2-oxoethyl)-2-oxo-1,2-dihydropyridin-3-yl)-N6-cyclopentyl-2-(4-hydroxy-6-(trifluoromethoxy)quinoline-3-carboxamido)-5-oxohexanediamide |
| **II-181** | (S)-N1-(1-(2-(bicyclo[1.1.1]pentan-1-ylam ino)-2-oxoethyl)-2-oxo-1,2-dihydropyridin-3-yl)-2-(cinnoline-3-carboxamido)-N6-cyclohexyl-5-oxohexanediamide |
| **II-182** | (S)-N1-(1-(2-(bicyclo[1.1.1]pentan-1-ylam ino)-2-oxoethyl)-2-oxo-1,2-dihydropyridin-3-yl)-N6-cyclohexyl-2-(3-ethylbenzofuran-2-carboxamido)-5-oxohexanediamide |
| **II-183** | (S)-N1-(1-(2-(bicyclo[1.1.1]pentan-1-ylamino)-2-oxoethyl)-2-oxo-1,2-dihydropyridin-3-yl)-N6-cyclohexyl-2-(1-ethyl-1H-indole-2-carboxamido)-5-oxohexanediamide |
| **II-184** | (S)-N1-(1-(2-(bicyclo[1.1.1]pentan-1-ylamino)-2-oxoethyl)-2-oxo-1,2-dihydropyridin-3-yl)-N6-cyclohexyl-2-(2-methyl-1,8-naphthyridine-3-carboxamido)-5-oxohexanediamide |
| **II-185** | (S)-N1-(1-(2-(bicyclo[2.1.1]hexan-1-ylamino)-2-oxoethyl)-2-oxo-1,2-dihydropyridin-3-yl)-N6-methyl-5-oxo-2-(1,2,3,4-tetrahydroquinoline-6-carboxamido)hexanediamide |
| **II-186** | (S)-N1-(1-(2-(2-carboxy-2-amino-5-(trifluoromethyl)adamantane)-2-oxoethyl)-2-oxo-1,2-dihydropyridin-3-yl)-N6-methyl-5-oxo-2-(3-oxo-1,2,3,4-tetrahydroisoquinoline-6-carboxamido)hexanediamide |
| **II-187** | (S)-N1-(1-(2-(5-ethyladamantane-2-amino)-2-oxoethyl)-2-oxo-1,2-dihydropyridin-3-yl)-N6-methyl-2-(1,6-naphthyridine-2-carboxamido)-5-oxohexanediamide |
| **II-188** | (S)-N1-(1-(2-(bicyclo[2.1.1]hexan-1-ylamino)-2-oxoethyl)-2-oxo-1,2-dihydropyridin-3-yl)-N6-methyl-2-(2,6-naphthyridine-1-carboxamido)-5-oxohexanediamide |
| **II-189** | (S)-2-(4-amino-1,2,5-oxadiazole-3-carboxamido)-N1-(1-(2-(bicyclo[1.1.1]pentan-1-ylamino)-2-oxoethyl)-2-oxo-1,2-dihydropyridin-3-yl)-N6-methyl-5-oxohexanediamide |
| **II-190** | (S)-N1-(1-(2-(bicyclo[1.1.1]pentan-1-ylamino)-2-oxoethyl)-2-oxo-1,2-dihydropyridin-3-yl)-2-(6-(dimethylamino)benzofuran-2-carboxamido)-N6-methyl-5-oxohexanediam ide |
| **II-191** | (S)-2-(2-acetamidothiazole-5-carboxamido)-N1-(1-(2-(bicyclo[1.1.1]pentan-1-ylamino)-2-oxoethyl)-2-oxo-1,2-dihydropyridin-3-yl)-N6-methyl-5-oxohexanediamide |
| **II-192** | (S)-N4-(1-(1-(2-(bicyclo[1.1.1]pentan-1-ylam ino)-2-oxoethyl)-2-oxo-1,2-dihydropyridin-3-ylamino)-6-(methylamino)-1,5,6-trioxohexan-2-yl)-1H-pyrrole-2,4-dicarboxamide |
| **II-193** | (S)-N1-(1-(2-(1-acetylamino-4-am inoadamantane)-2-oxoethyl)-2-oxo-1,2-dihydropyridin-3-yl)-N6-methyl-5-oxo-2-(5-sulfamoylfuran-3-carboxamido)hexanediamide |
| **II-194** | (S)-2-(benzofuran-5-carboxamido)-N1-(1-(2-(1-acetylamino-4-aminoadamantane)-2-oxoethyl)-2-oxo-1,2-dihydropyridin-3-yl)-N6-methyl-5-oxohexanediamide |
| **II-195** | (S)-2-(benzofuran-6-carboxamido)-N1-(1-(2-(4-aminoadamantane-1-carboxamide)-2-oxoethyl)-2-oxo-1,2-dihydropyridin-3-yl)-N6-methyl-5-oxohexanediamide |
| **II-196** | (S)-N1-(1-(2-(4-aminoadamantane-1-carboxamide)-2-oxoethyl)-2-oxo-1,2-dihydropyridin-3-yl)-N6-methyl-2-(3-(1-methylcyclopropyl)-1,2,4-oxadiazole-5-carboxamido)-5-oxohexanediamide |
| **II-197** | (S)-N1-(1-(2-(bicyclo[1.1.1]pentan-1-ylam ino)-2-oxoethyl)-2-oxo-1,2-dihydropyridin-3-yl)-N6-methyl-2-(5-methyl-1,2,4-oxadiazole-3-carboxamido)-5-oxohexanediamide |
| **II-198** | (S)-N1-(1-(2-(bicyclo[1.1.1]pentan-1-ylamino)-2-oxoethyl)-2-oxo-1,2-dihydropyridin-3-yl)-N6-methyl-5-oxo-2-(1,2,3-thiadiazole-4-carboxamido)hexanediamide |
| **II-199** | (S)-N1-(1-(2-(bicyclo[1.1.1]pentan-1-ylamino)-2-oxoethyl)-2-oxo-1,2-dihydropyridin-3-yl)-N6-methyl-5-oxo-2-(1,2,4-thiadiazole-5-carboxamido)hexanediamide |
| **II-200** | (S)-N1-(1-(2-(bicyclo[1.1.1]pentan-1-ylamino)-2-oxoethyl)-2-oxo-1,2-dihydropyridin-3-yl)-N6-methyl-5-oxo-2-(1,3,4-thiadiazole-2-carboxamido)hexanediamide |
| **II-201** | (S)-N1-(1-(2-(bicyclo[1.1.1]pentan-1-ylamino)-2-oxoethyl)-2-oxo-1,2-dihydropyridin-3-yl)-2-(4-cyclopropyl-1,2,3-thiadiazole-5-carboxamido)-N6-methyl-5-oxohexanediam ide |
| **II-202** | (S)-2-(4-cyclopropyl-1,2, 3-thiadiazole-5-carboxam ido)-N1-(1-(2-(2-adamantylamino)-2-oxoethyl)-2-oxo-1,2-dihydropyridin-3-yl)-N6-methyl-5-oxohexanediamide |
| **II-203** | (S)-2-(4-isopropyl-1,2,3-thiadiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-204** | (S)-2-(4-ethyl-1,2,3-thiadiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-205** | (S)-2-(4-formyl-1,2,3-thiadiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-206** | (S)-2-(4-(hydroxymethyl)-1,2,3-thiadiazole-5-carboxamido)-N1-(1-(2-(2-adamantylamino)-2-oxoethyl)-2-oxo-1,2-dihydropyridin-3-yl)-N6-methyl-5-oxohexanediamide |
| **II-207** | (S)-N1-(1-(2-(1-adamantylamino)-2-oxoethyl)-2-oxo-1,2-dihydropyridin-3-yl)-N6-methyl-2-(1-methyl-1H-imidazole-5-carboxamido)-5-oxohexanediamide |
| **II-208** | (S)-N1-(1-(2-(((1S,2R,5S)-6,6-dimethylbicyclo[3.1.1]heptan-2-yl)methylamino)-2-oxoethyl)-2-oxo-1,2-dihydropyridin-3-yl)-N6-methyl-2-(1-methyl-1H-imidazole-2-carboxamido)-5-oxohexanediamide |
| **II-209** | (S)-N1-(1-(2-(((1R,2R,5R)-6,6-dimethylbicyclo[3.1.1]heptan-2-yl)methylamino)-2-oxoethyl)-2-oxo-1,2-dihydropyridin-3-yl)-2-(1H-imidazole-4-carboxamido)-N6-methyl-5-oxohexanediamide |
| **II-210** | (S)-N1-(1-(2-(3,5-dimethyladamantane-1-amino)-2-oxoethyl)-2-oxo-1,2-dihydropyridin-3-yl)-N6-methyl-2-(1-methyl-1H-imidazole-5-carboxamido)-5-oxohexanediamide |
| **II-211** | (S)-N 1-(1-(2-(3,5,7-trimethyl-1-adamantylamino)-2-oxoethyl)-2-oxo-1,2-dihydropyridin-3-yl)-N6-methyl-2-(1-methyl-1H-imidazole-5-carboxamido)-5-oxohexanediamide |
or a pharmaceutically acceptable salt thereof.

10. A pharmaceutical composition comprising a compound of any one of the claims 1 - 9 as an active ingredient, together with at least one pharmaceutically acceptable carrier, excipient and/or diluent.

11. Compound according to any one of the claims 1 - 9 for use in medicine.

12. Compound according to any one of the claims 1 - 9 or the pharmaceutical composition according to claim 10 for use in the treatment or prophylaxis of autoimmune and inflammatory diseases, vascular diseases, fibrotic diseases, liver diseases, cholestatic liver diseases, cancer, neurodegenerative diseases, ocular diseases, and skin disorders.

13. Compound for use, or the pharmaceutical composition for use according to claim 12, wherein
the autoimmune and inflammatory diseases comprise multiple sclerosis, celiac disease, Duhring-Brocq-disease (dermatitis herpetiformis), gluten ataxia, gluten neuropathy, diabetes, rheumatoid arthritis, Graves' disease, inflammatory bowel disease, systemic lupus erythematosus psoriasis, and gingivitis; wherein the vascular diseases comprise atherosclerosis, thrombosis, vascular stiffness; wherein the fibrotic diseases affect the lung, the kidney, the liver, the skin or the gut like cystic fibrosis, kidney fibrosis and diabetic nephropathy, intestinal fibrosis, idiopathic lung fibrosis, liver fibrosis; wherein the liver diseases comprise alcoholic hepatitis, alcoholic steatohepatitis, nonalcoholic steatohepatitis, non-alcoholic fatty liver disease, liver cirrhosis, autoimmune hepatitis or liver inflammation; wherein the cholestatic liver diseases comprise primary biliary cholangitis and primary sclerosing cholangitis; wherein the cancer comprises glioblastoma, melanoma, pancreatic cancer, renal cell carcinoma, meningioma, and breast cancer, wherein the neurodegenerative diseases comprise Parkinson's disease, Huntington's disease, or Alzheimer's disease, wherein the ocular diseases comprise glaucoma, cataracts, macular degeneration, or uveitis; and wherein the skin disorders comprise acne, psoriasis, scarring, and skin aging.

14. Compound for use, or the pharmaceutical composition for use according to any one of the claims 12 and 13 in the treatment or prophylaxis of celiac disease.

15. A method for producing the compound of formula (**Ib**) according to claim 1 or 4 comprising:
**Step 1B:** providing a compound **4b** 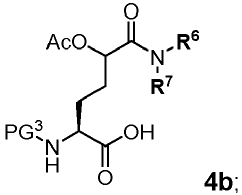
**Step 2B:** performing coupling reaction of the compound **4b** with a compound **5** 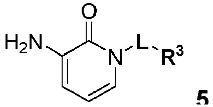 to obtain a compound **6b** 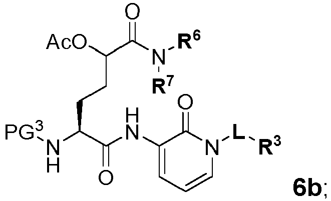
**Step 3B:** deprotecting an amino protecting group PG³ to obtain a compound **7b** 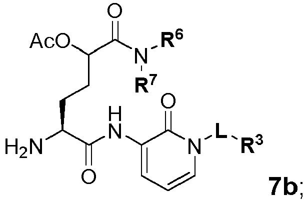
**Step 4B:** performing coupling reaction of the compound **7b** with a carboxylic acid (**R²**-CO₂H **8**) to obtain a compound **9b** 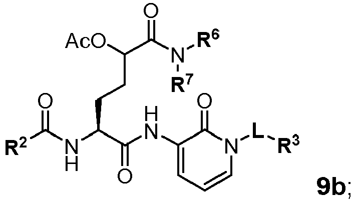
**Step 5B:** performing oxidation reaction of the compound **9b** to produce the compound of the formula (**Ib**) 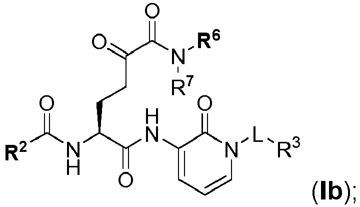
wherein **L**, **R²**, **R³**, **R⁶** and **R⁷** have the same meanings as defined in claim 1, and **PG³** is an amino protecting group.

## Patentansprüche

1. Eine Verbindung der allgemeinen Formel (**I**): 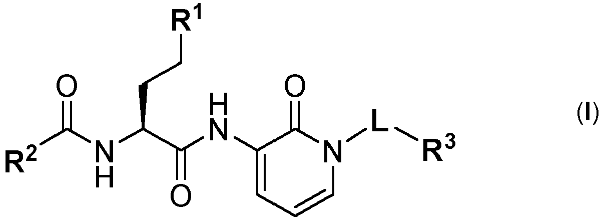 wobei
**L** -**L¹**- oder -**L¹**-**L²**- darstellt; bevorzugt -**L¹**-**L²**-;
**L¹** -CH₂-, -CH₂CH₂-, -CH₂CH₂CH₂-, -CH₂CO- oder -CH₂CH₂CO-darstellt;
**L²** eine Bindung, -N**R**^{N1}-, -N**R**^{N1}CH₂-, -N**R**^{N1}CH₂CH₂- oder -NR^{N1}CH(CH₃)- darstellt;
**R¹** 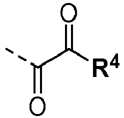 darstellt;
**R2** 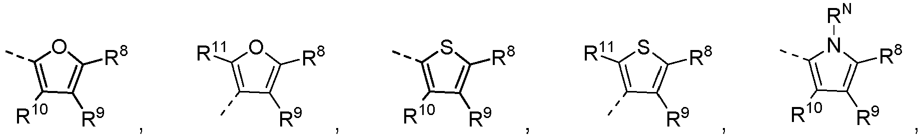 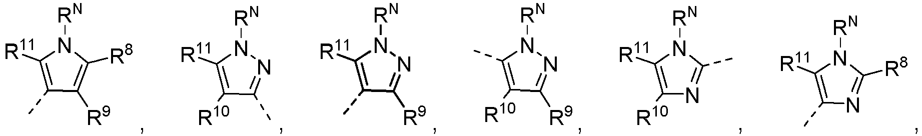 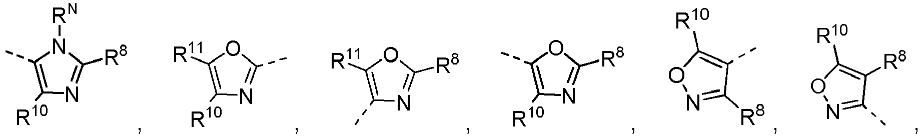 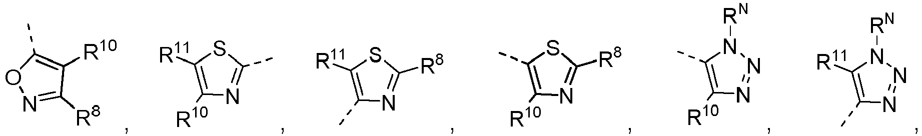 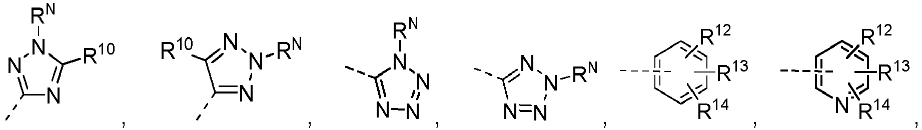 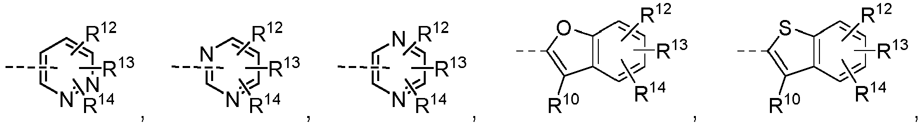 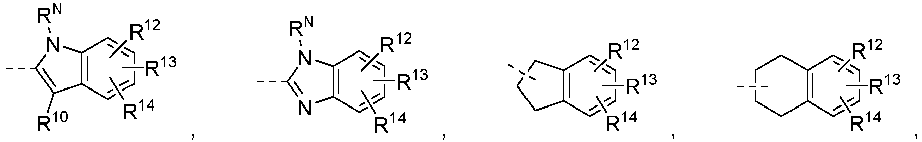 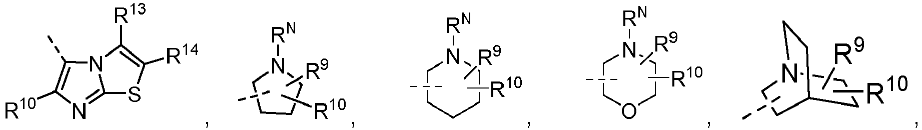 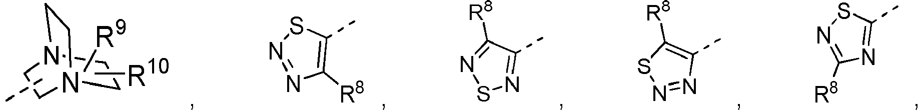 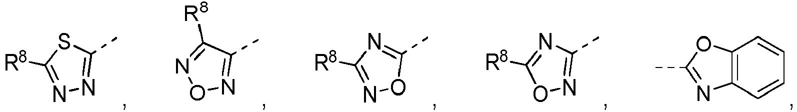 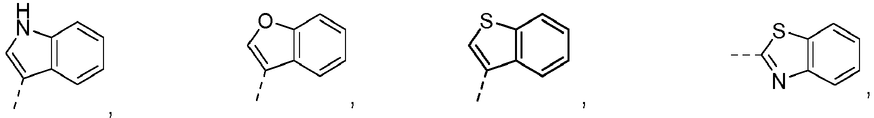 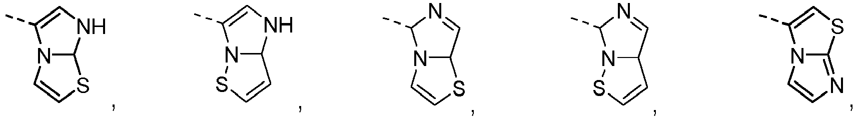 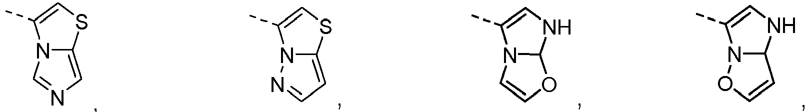 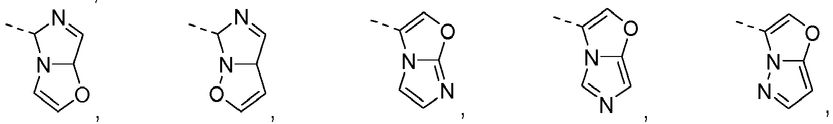 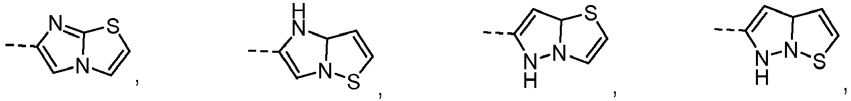 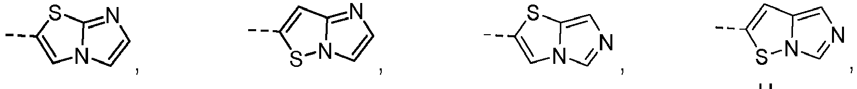 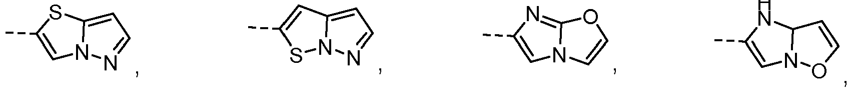 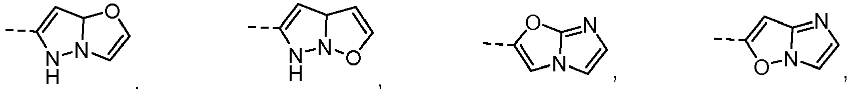 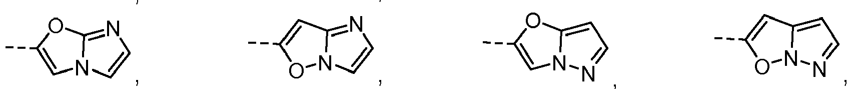 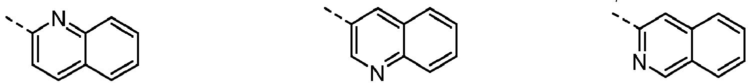 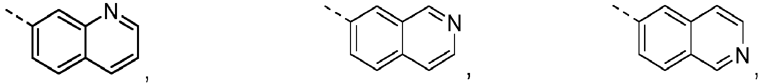 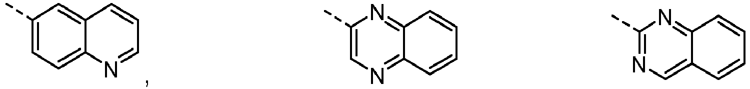 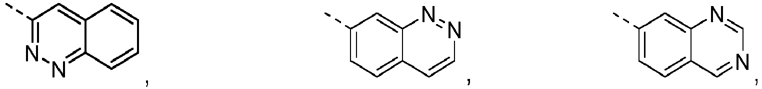 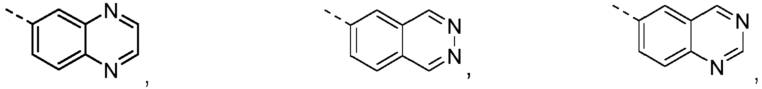 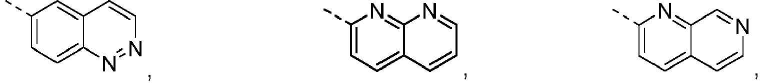 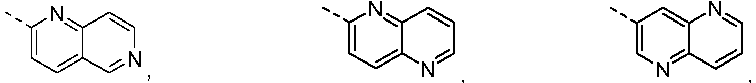 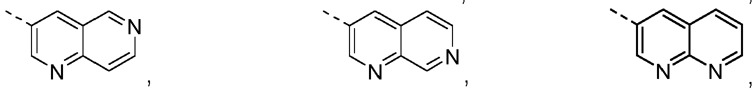 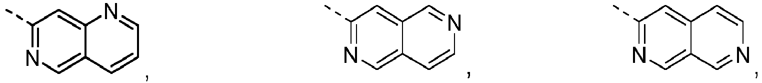 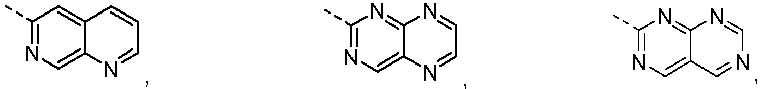 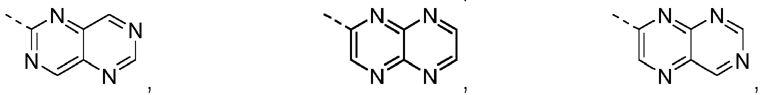 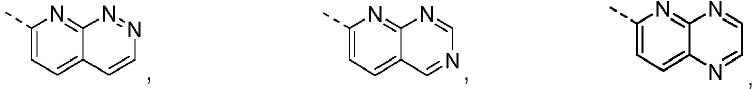 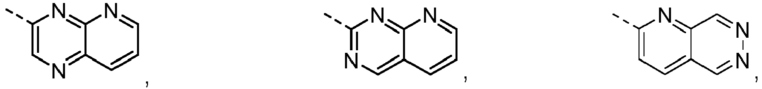 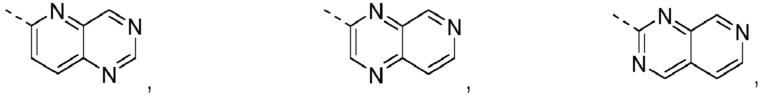 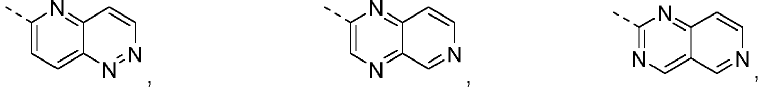 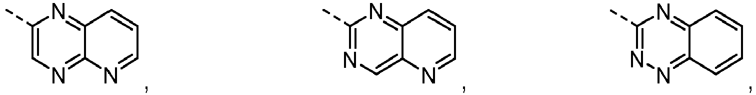 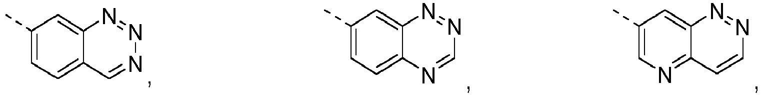 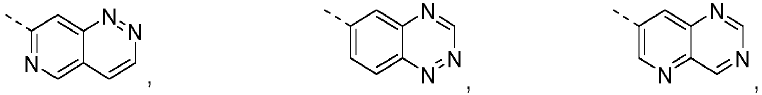 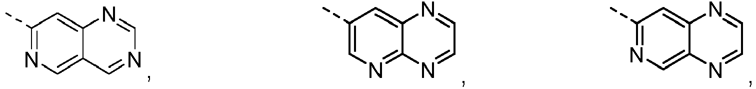 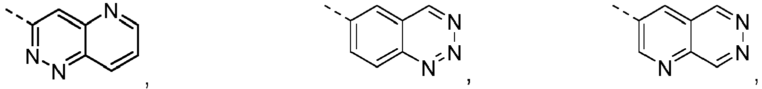 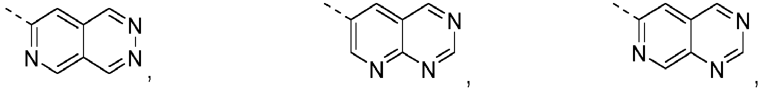 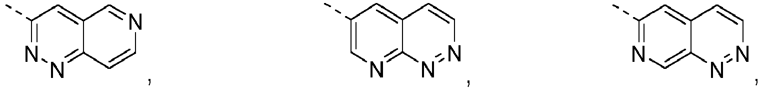 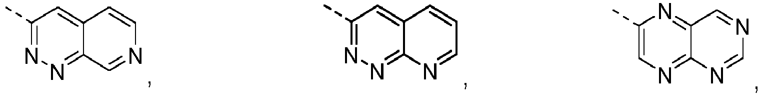 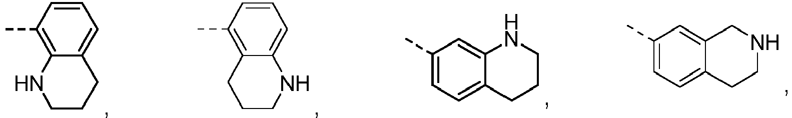 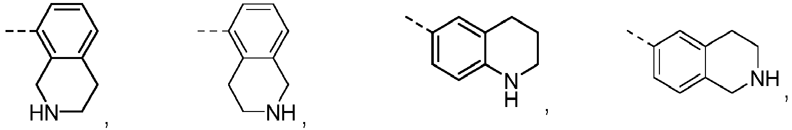 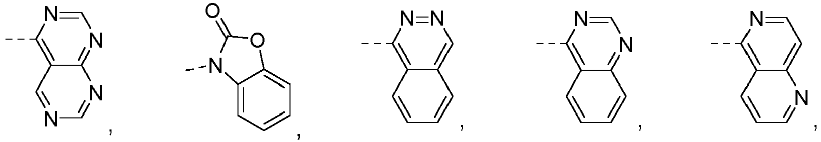 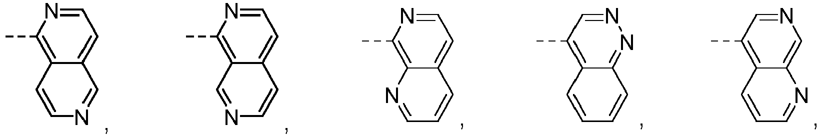 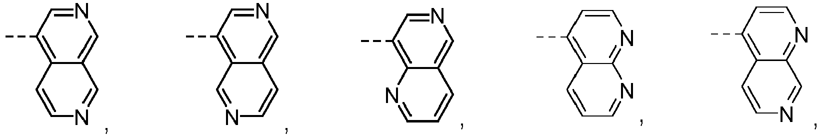 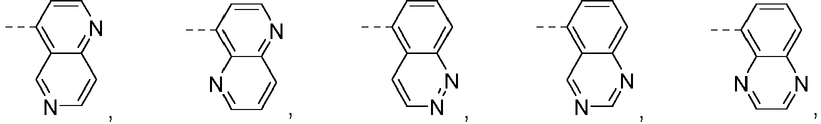 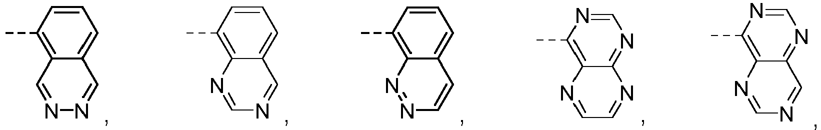 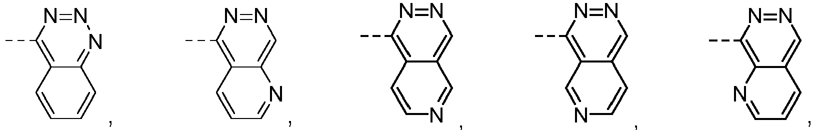 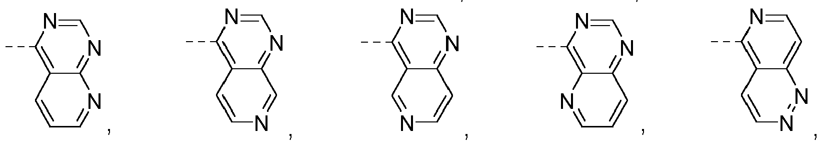 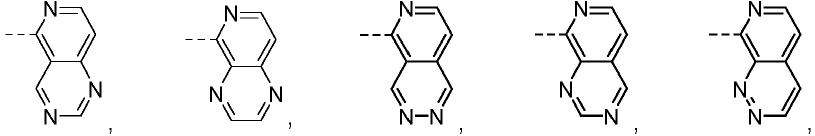 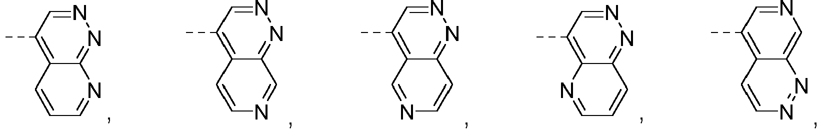 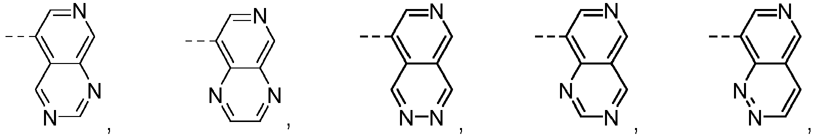 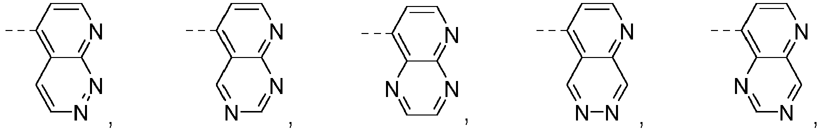 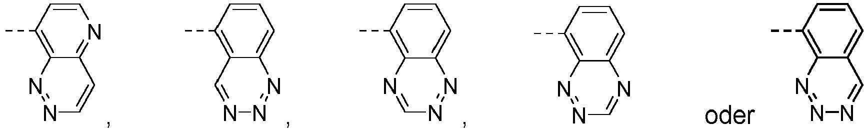
darstellt;
wobei die unsubstituierten bicyclischen Reste mit 1 bis 5 der Substituenten **R⁹** - **R¹⁴** und **R^{N}** substituiert sein können; und bevorzugt mit 1 bis 3 der Substituenten **R¹¹** - **R¹³**;
**R³** Bicyclo[1.1.1]pentyl, Bicyclo[2.1.1]hexyl, Bicyclo[2.2.1]heptyl, Bbicyclo[3.1.1]heptyl, Bicyclo[2.2.2]octyl, Bicyclo[3.2.1]octyl, Bicyclo[3.2.2]nonyl, Bicyclo[3.3.2]decyl, Bicyclo[3.3.3]undecyl, 4-Homoisotwistyl, Adamantyl, Diamantyl, Hexamethylentetraminyl und die vorgenannten Reste optional eine oder mehrere C=C-Doppelbindung(en) enthalten und/oder optional mit einem oder mehreren von **R^{a}**, **R^{b}**, **R^{c}**, **R^{d}** und **R^{e}** substituiert sind;
**R^{a}**, **R^{b}**, **R^{c}**, **R^{d}** und **R^{e}** unabhängig voneinander -H, -F, -Cl, -Br, -CN, -OH, -CH₃, -CH₂CH₃, -CH₂CH₂CH₃, -CH(CH₃)₂, -CHF₂, -CF₃, -CH₂CF₃, -COCH₃, -COCH₂CH₃, -CO₂H, -CO₂CH₃, -CO₂C₂H₅, -CONH₂, -CONHCH₃, -CON(CH₃)₂, -CONHC₂H₅, -CH₂CO₂H, -CH₂CO₂CH₃, -CH₂CO₂C₂H₅, -CH₂CONH₂, -CH₂CONHCH₃, -CH₂CON(CH₃)₂, -CH₂CONHC₂H₅, -NHCOCH₃, -NHCOC₂H₅, -NHCOCF₃, -NHCOCH₂CF₃, -NHSO₂CH₃, -NHSO₂C₂H₅, -NHSO₂CHF₂, -NHSO₂CF₃ oder -NHSO₂CH₂CF₃ darstellen;
**R⁴ -NR⁶R⁷** darstellt;
**R⁶** und **R⁷** unabhängig voneinander -H, -CH₃, -CH₂CH₂CH₃, -CH(CH₃)₂, -CH₂CH₂CH₂CH₃, -CH₂CH₂CH₂CH₂CH₃, -CH₂CH(CH₃)₂, -C(CH₃)₃, -CH₂CH=CH₂, -CH₂CH=CH(CH₃), -CH₂CH=C(CH₃)₂, -CH₂CH=CHCH₂CH₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -Ph, -CH₂-Ph, -CH₂OCH₃, -CH₂OCH₂CH₃, -CH₂CH₂OCH₃, -CH₂CH₂OCH₂CH₃, -CH₂CH₂NHCH₃ oder -CH₂CH₂N(CH₃)₂ darstellen,
oder **-NR⁶R⁷** -N(C₂H₅)₂, 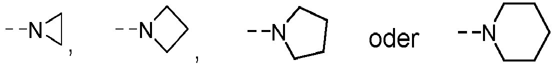 ist;
**R⁸**, **R⁹**, **R¹⁰**, **R¹¹**, **R¹²**, **R¹³** und **R¹⁴** unabhängig voneinander -H, -F, -Cl, -Br, -I, -OH, -CN, -NO₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -CH₂-cyclo-C₃H₅, -CH₂OH, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -OCH₃, -OC₂H₅, -OC₃H₇, -OCH(CH₃)₂, -OC(CH₃)₃, -OC₄H₉, -OCHF₂, -OCF₃, -OCH₂CF₃, -OC₂F₅, -OCH₂OCH₃, -O-cyclo-C₃H₅, -OCH₂-cyclo-C₃H₅, -O-C₂H₄-cyclo-C₃H₅, -CHO, -COCH₃, -COCF₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, -COOC(CH₃)₃, -OOC-CH₃, -OOC-CF₃, -OOC-C₂H₅, -OOC-C₃H₇, -OOC-CH(CH₃)₂, -OOC-C(CH₃)₃, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NHCOCH₃, -NHCOCF₃, -NHCOC₂H₅, -NHCOC₃H₇, -NHCOCH(CH₃)₂, -NHCOC(CH₃)₃, -CONH₂, -CONHCH₃, -CONHC₂H₅, -CONHC₃H₇, -CONHCH(CH₃)₂, -CONH-cyclo-C₃H₅, -CONHC(CH₃)₃, -CON(CH₃)₂, -CON(C₂H₅)₂, -CON(C₃H₇)₂, -CON[CH(CH₃)₂]₂, -CON[C(CH₃)₃]₂, -SO₂NH₂, -SO₂NHCH₃, -SO₂NHC₂H₅, -SO₂NHC₃H₇, -SO₂NHCH(CH₃)₂, -SO₂NH-cyclo-C₃H₅, -SO₂NHC(CH₃)₃, -SO₂N(CH₃)₂, -SO₂N(C₂H₅)₂, -SO₂N(C₃H₇)₂, -SO₂N[CH(CH₃)₂]₂, -SO₂N[C(CH₃)₃]₂, -NHSO₂CH₃, -NHSO₂CF₃, -NHSO₂C₂H₅, -NHSO₂C₃H₇, -NHSO₂CH(CH₃)₂, -NHSO₂C(CH₃)₃, -CH=CH₂, -CH₂-CH=CH₂, -C(CH₃)=CH₂, -CH=CH-CH₃, -C≡CH, -C≡C-CH₃, -CH₂-C≡CH, -Ph, -O-Ph, -O-CH₂-Ph, 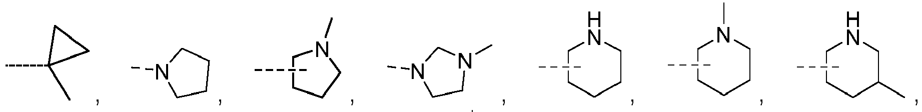 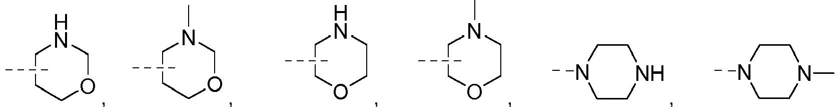 oder 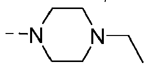 darstellen;
oder **R⁸** und **R⁹** oder **R⁹** und **R¹⁰** gemeinsam einen der folgenden fünf- oder sechsgliedrigen Ringe bilden können: 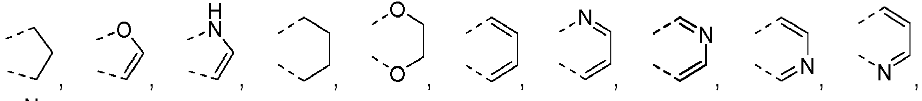 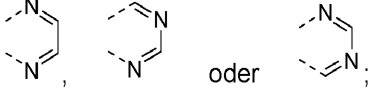
oder **R¹²** und **R¹³** oder **R¹³** und R¹⁴ gemeinsam einen der folgenden fünf- oder sechsgliedrigen Ringe bilden können: 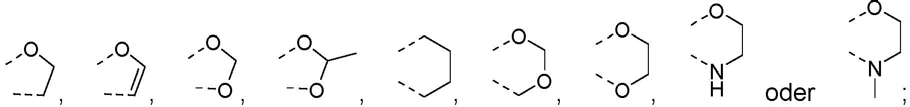
**R^{N}** -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -cyclo-C₄H₇, -cyclo-C₅H₉, -CH₂-cyclo-C₃H₅, -CH₂-cyclo-C₄H₇, -CH₂-cyclo-C₅H₉, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -CH₂-CH=CH₂, -CH₂-C≡CH, -CHO, -COCH₃, -COC₂H₅, -COC₃H₇, -COCH(CH₃)₂, -COC(CH₃)₃, -CO-cyclo-C₃H₅, -CO-cyclo-C₄H₇, -CO-cyclo-C₅H₉, -COOCH₃, -COOC₂H₅, -COOC₃H₇, -COOCH(CH₃)₂, -COOC(CH₃)₃, -COOCH₂Ph, -SO₂CH₃, -SO₂CF₃, -SO₂C₂H₅, -SO₂C₃H₇, -SO₂CH(CH₃)₂, -SO₂-cyclo-C₃H₅ oder -SO₂C(CH₃)₃ darstellt;
**R^{N1}** -H, -CH₃ oder -CH₂CH₃ darstellt;
oder ein Diastereomer, ein Enantiomer, ein Diastereomerengemisch, ein Enantiomerengemisch, ein Racemat, ein Solvat, ein Hydrat oder ein pharmazeutisch verträgliches Salz davon.

2. Die Verbindung gemäß Anspruch 1, wobei **R²** 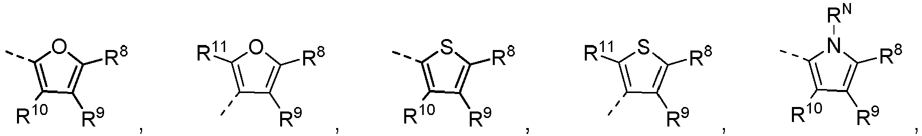 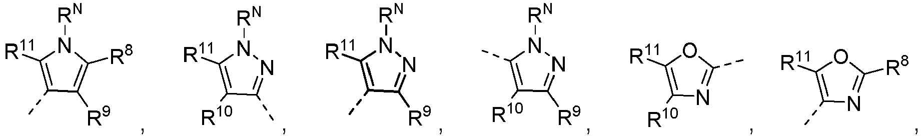 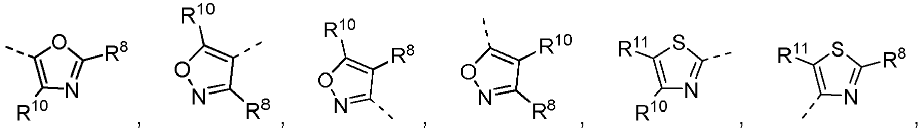 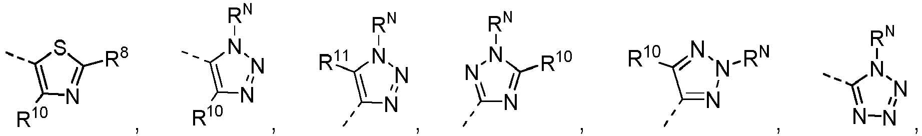 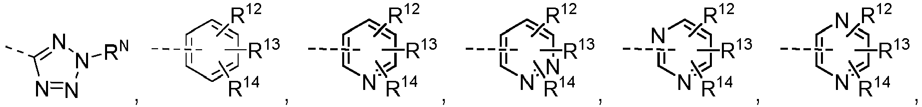 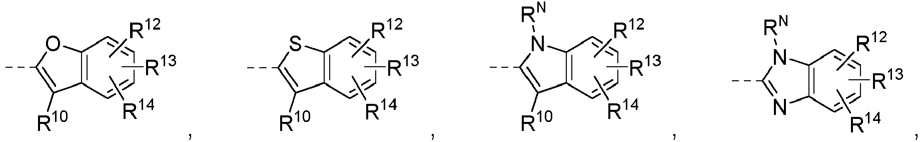 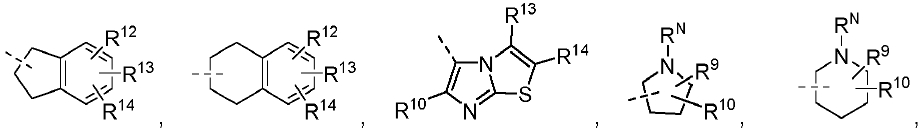 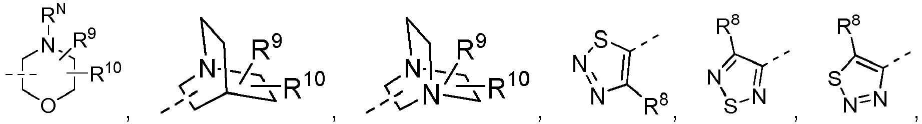 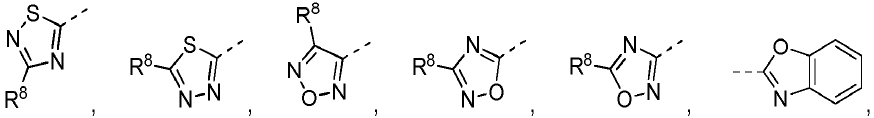 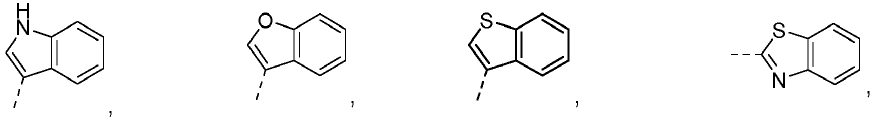 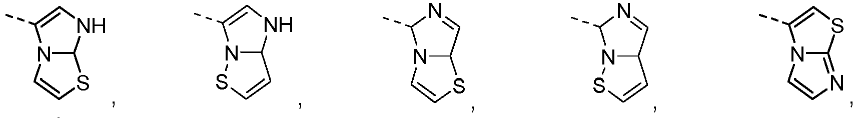 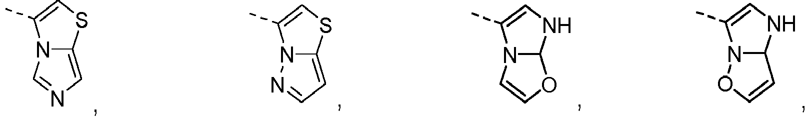 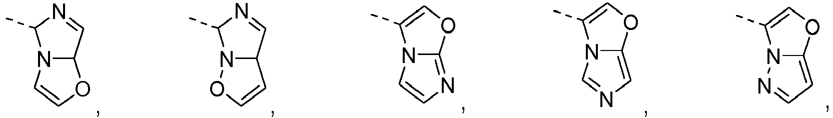 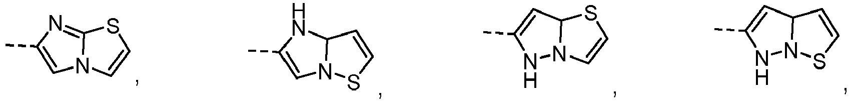 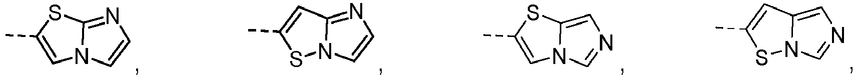 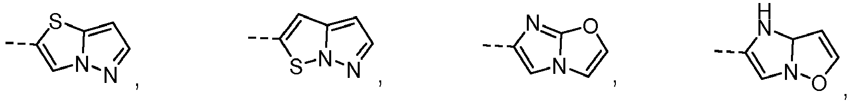 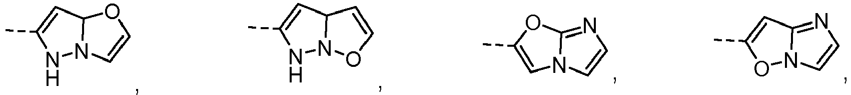 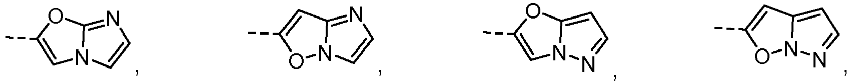 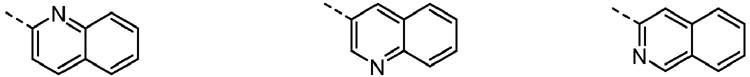 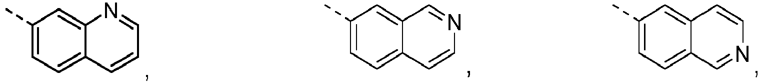 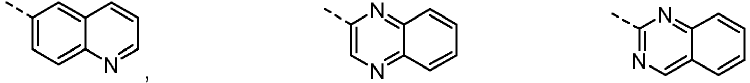 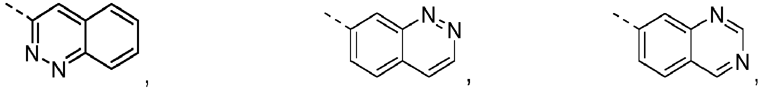 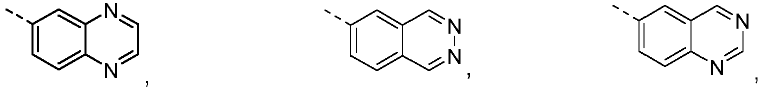 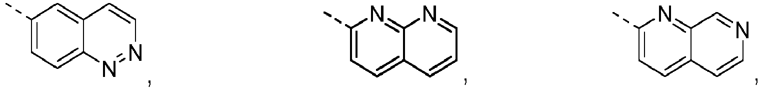 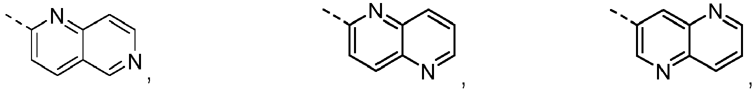 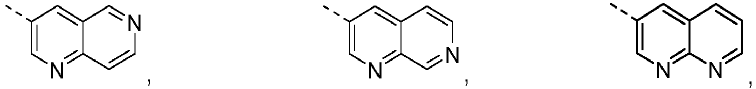 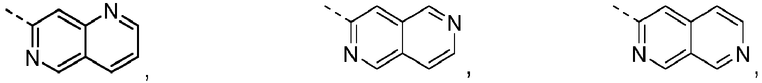 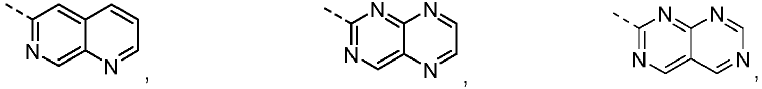 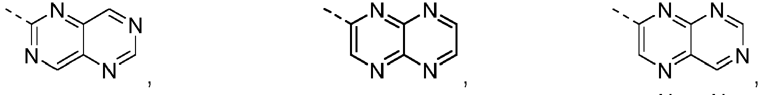 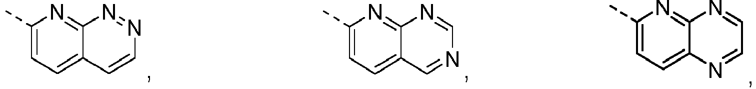 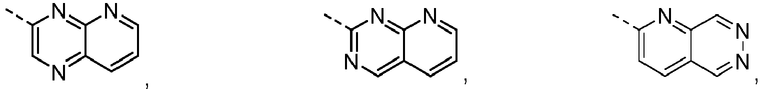 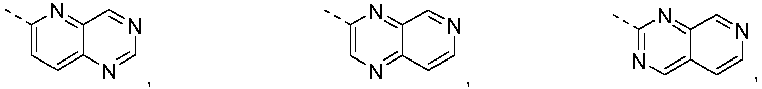 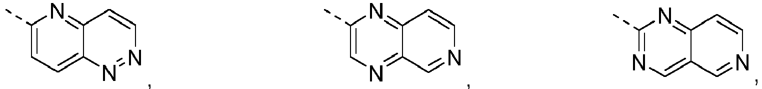 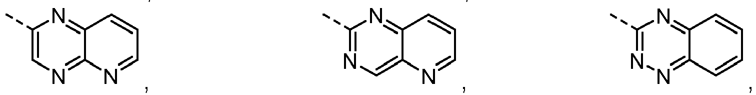 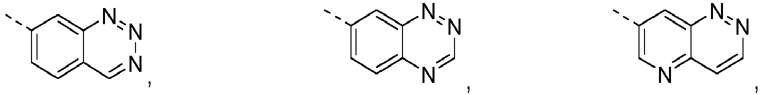 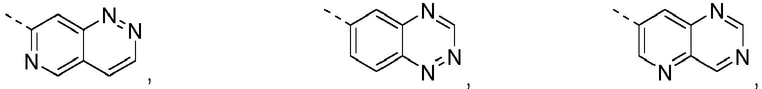 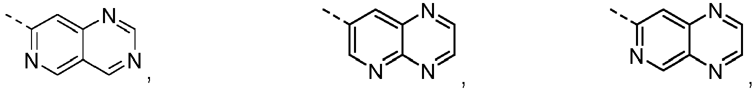 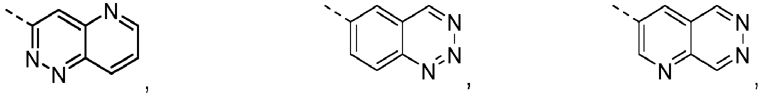 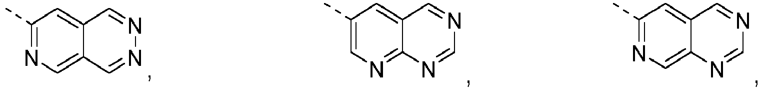 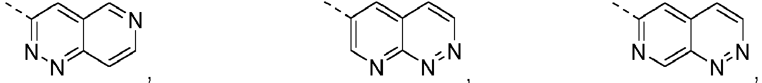 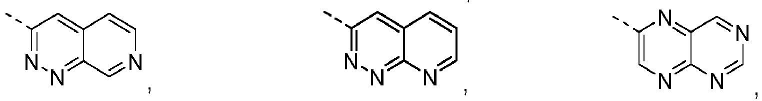 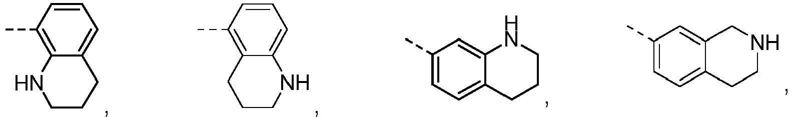 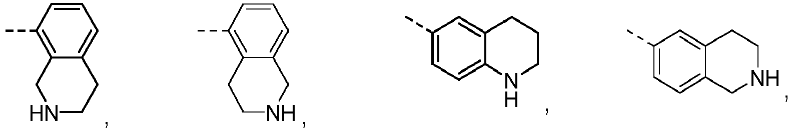 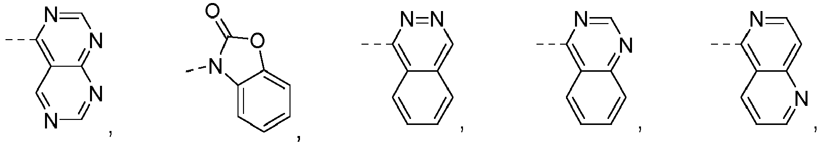 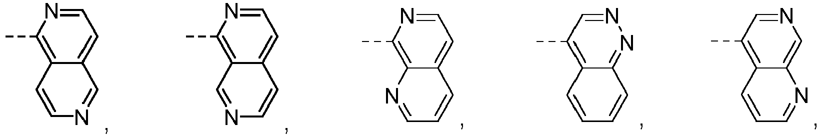 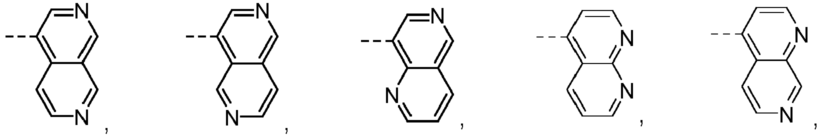 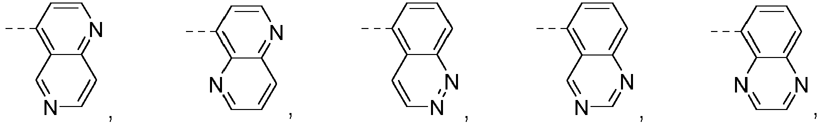 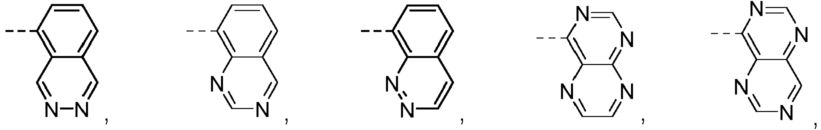 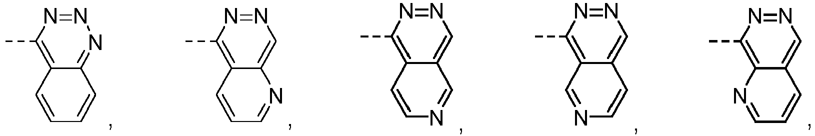 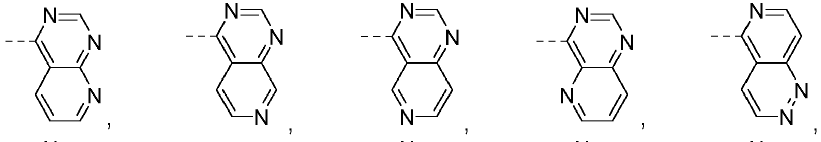 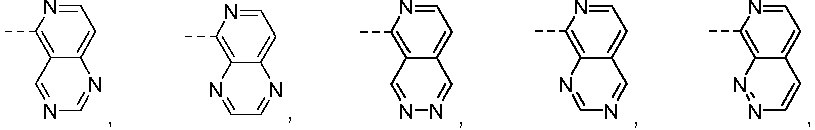 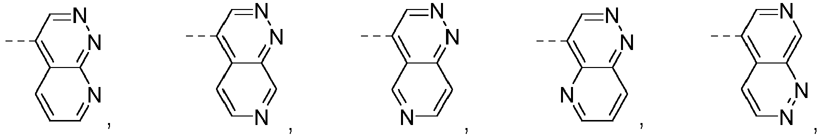 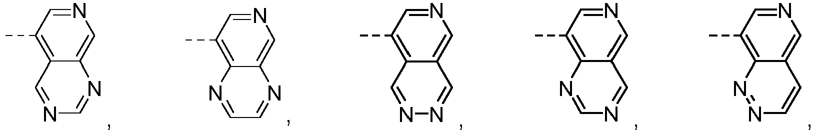 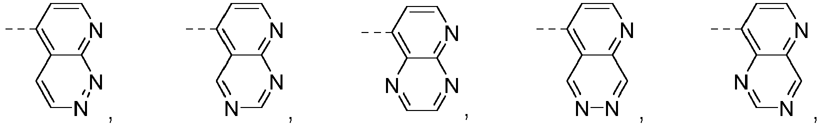 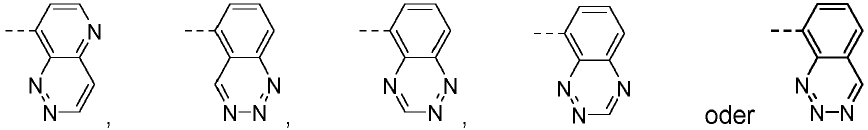 darstellt;
wobei die unsubstituierten bicyclischen Reste mit 1 bis 5 der Substituenten **R⁹** - **R¹⁴** und **R^{N}** substituiert sein können; und bevorzugt mit 1 bis 3 der Substituenten **R¹¹** - **R¹³** und die Substituenten **R⁹** - **R¹⁴** und **R^{N}** die in Anspruch 1 definierten Bedeutungen haben.

3. Die Verbindung gemäß Anspruch 1, wobei **R²** 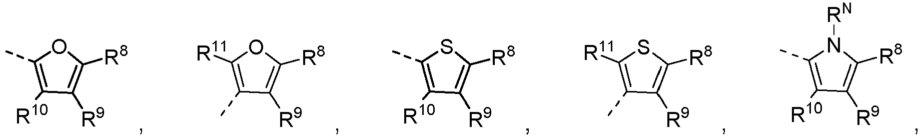 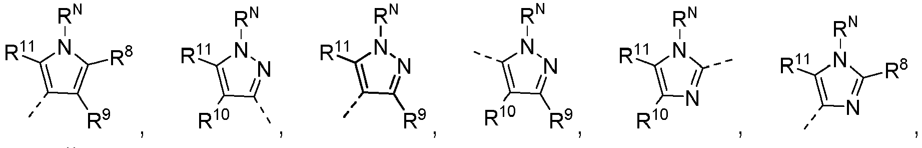 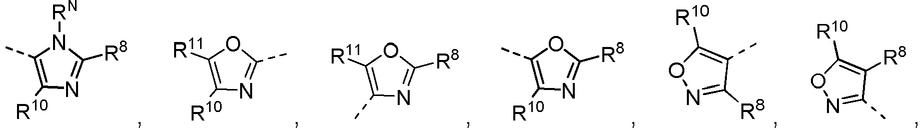 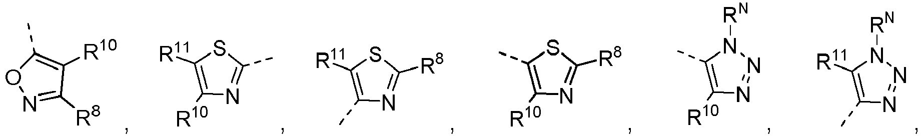 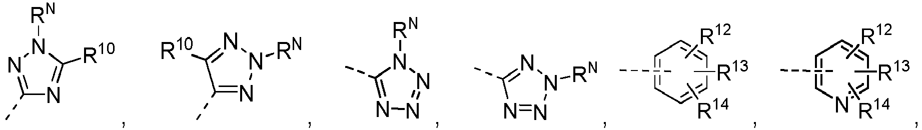 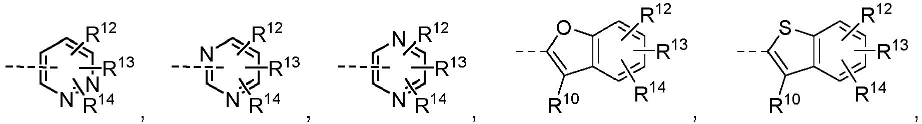 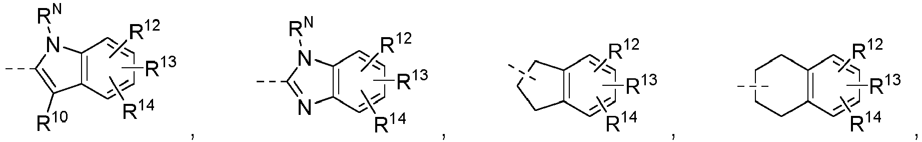 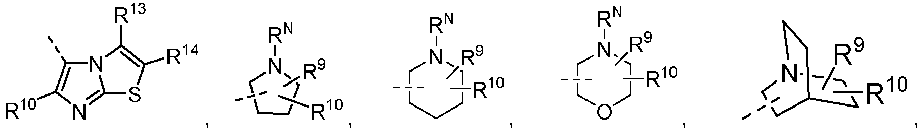 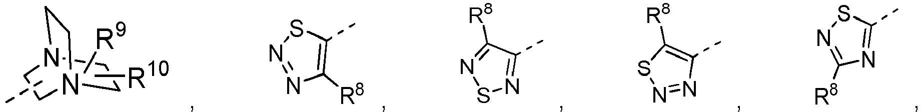 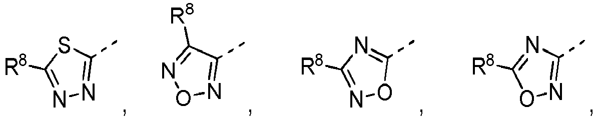 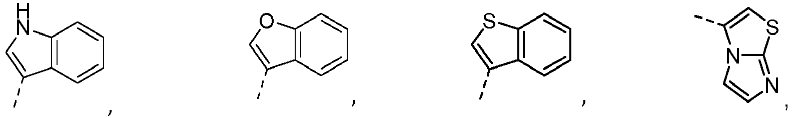 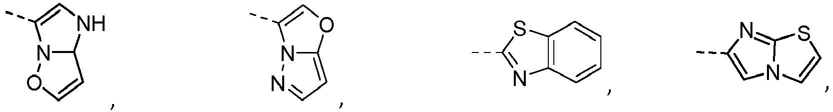 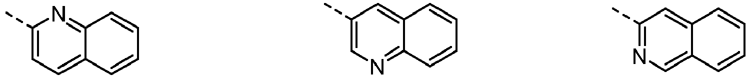 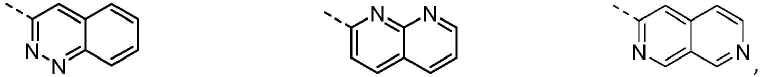 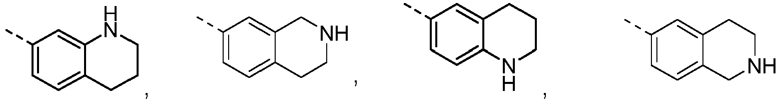 oder 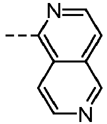 darstellt,
wobei die unsubstituierten bicyclischen Reste mit 1 bis 5 der Substituenten **R⁹** - **R¹⁴** und **R^{N}** substituiert sein können; und bevorzugt mit 1 bis 3 der Substituenten **R¹¹** - **R¹³** und die Substituenten **R⁹** - **R¹⁴** und **R^{N}** die in Anspruch 1 definierten Bedeutungen haben.

4. Die Verbindung gemäß Anspruch 1, wobei die Verbindung die Formel (**Ib**) ausweist 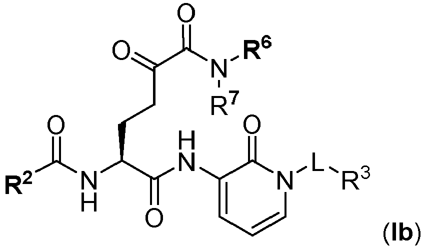 wobei
**L -L¹-** oder **-L¹-L²-** darstellt; bevorzugt **-L¹-L²-;**
**L¹** -CH₂-, -CH₂CH₂-, -CH₂CH₂CH₂-, -CH₂CO- oder -CH₂CH₂CO-darstellt;
**L²** eine Bindung, -NR^{N1}-, -NR^{N1}CH₂-, -NR^{N1}CH₂CH₂- oder -NR^{N1}CH(CH₃)-darstellt;
**R²** 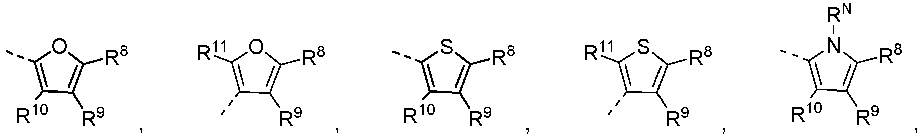 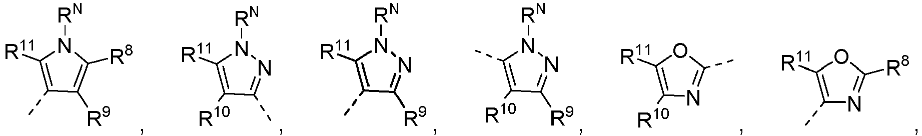 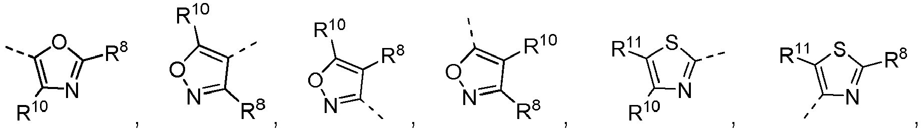 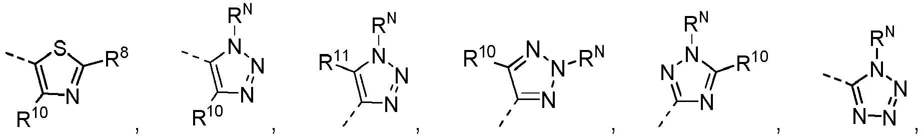 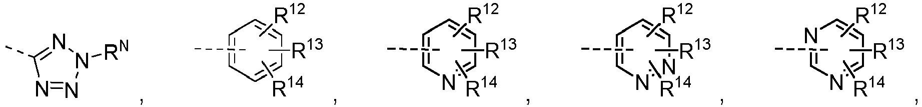 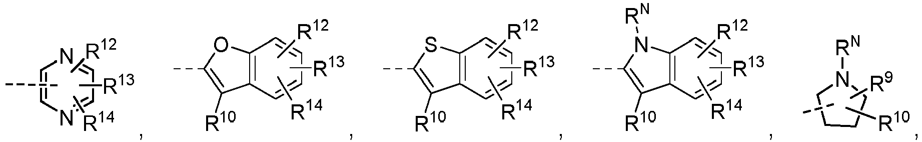 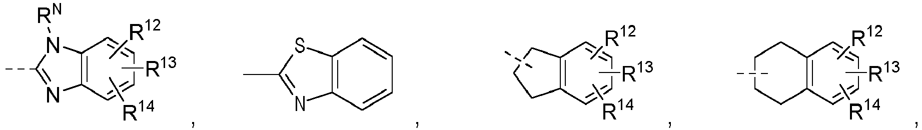 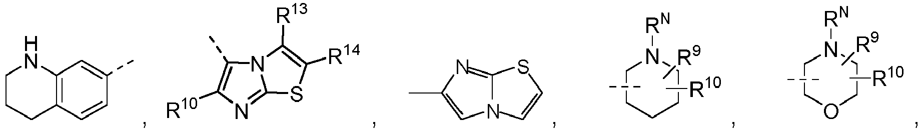 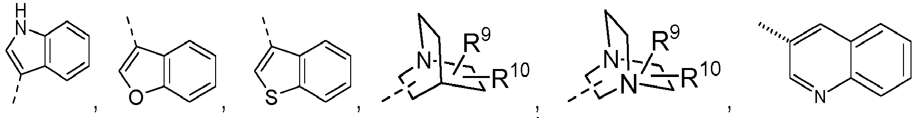 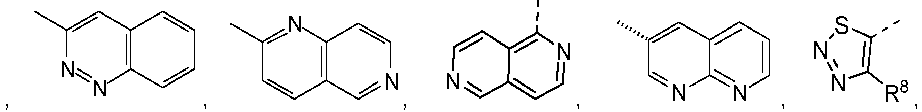 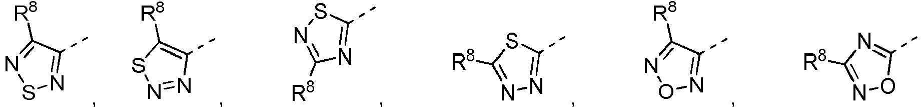 oder 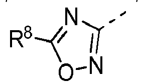 darstellt;
**R³** Bicyclo[1.1.1]pentyl, Bicyclo[2.1.1]hexyl, Bicyclo[2.2.1]heptyl, Bicyclo[3.1.1]heptyl, Bicyclo[2.2.2]octyl, Bicyclo[3.2.1]octyl, Bicyclo[3.2.2]nonyl, Bicyclo[3.3.2]decyl, Bicyclo[3.3.3]undecyl, 4-Homoisotwistyl, Adamantyl, Diamantyl, Hexamethylentetraminyl, und die vorgenannten Reste optional eine oder mehrere C=C-Doppelbindung(en) enthalten und/oder optional mit einem oder mehreren von **R^{a}, R^{b}, R^{c}, R^{d}** und **R^{e}** substituiert sind;
**R^{a}, R^{b}, R^{c}, R^{d}** und **R^{e}** unabhängig voneinander -H, -F, -Cl, -Br, -CN, -OH, -CH₃, -CH₂CH₃, -CH₂CH₂CH₃, -CH(CH₃)₂, -CHF₂, -CF₃, -CH₂CF₃, -COCH₃, -COCH₂CH₃, -CO₂H, -CO₂CH₃, -CO₂C₂H₅, -CONH₂, -CONHCH₃, -CON(CH₃)₂, -CONHC₂H₅, -CH₂CO₂H, -CH₂CO₂CH₃, -CH₂CO₂C₂H₅, -CH₂CONH₂, -CH₂CONHCH₃, -CH₂CON(CH₃)₂, -CH₂CONHC₂H₅, -NHCOCH₃, -NHCOC₂H₅, -NHCOCF₃, -NHCOCH₂CF₃, -NHSO₂CH₃, -NHSO₂C₂H₅, -NHSO₂CHF₂, -NHSO₂CF₃ oder -NHSO₂CH₂CF₃ darstellen;
**R⁴ -NR⁶R⁷** darstellt;
**R⁶** und **R⁷** unabhängig voneinander -H, -CH₃, -CH₂CH₂CH₃, -CH(CH₃)₂, -CH₂CH₂CH₂CH₃, -CH₂CH₂CH₂CH₂CH₃, -CH₂CH(CH₃)₂, -C(CH₃)₃, -CH₂CH=CH₂, -CH₂CH=CH(CH₃), -CH₂CH=C(CH₃)₂, -CH₂CH=CHCH₂CH₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -Ph, -CH₂-Ph, -CH₂OCH₃, -CH₂OCH₂CH₃, -CH₂CH₂OCH₃, -CH₂CH₂OCH₂CH₃, -CH₂CH₂NHCH₃, -CH₂CH₂N(CH₃)₂ darstellen,
oder **-NR⁶R⁷** -N(C₂H₅)₂, 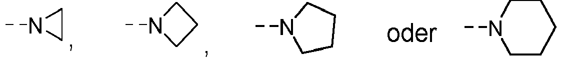 ist;
**R^{N}** -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -cyclo-C₄H₇, -cyclo-C₅H₉, -CH₂-cyclo-C₃H₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -CH₂-CH=CH₂, -CH₂-C≡CH, -CHO, -COCH₃, -COC₂H₅, -COC₃H₇, -COCH(CH₃)₂, -COC(CH₃)₃, -COOCH₃, -COOC₂H₅, -COOC₃H₇, -COOCH(CH₃)₂, -COOC(CH₃)₃, -COOCH₂Ph, -SO₂CH₃, -SO₂CF₃, -SO₂C₂H₅, -SO₂C₃H₇, -SO₂CH(CH₃)₂ oder -SO₂C(CH₃)₃ darstellt;
**R^{N1}** -H, -CH₃ oder -CH₂CH₃ darstellt;
und **R⁸** - **R¹⁴** die in Formel (**I**) definierten Bedeutungen haben;
oder ein Diastereomer, ein Enantiomer, ein Diastereomerengemisch, ein Enantiomerengemisch, ein Racemat, ein Solvat, ein Hydrat oder ein pharmazeutisch verträgliches Salz davon.

5. Die Verbindung gemäß einem der Ansprüche 1 - 4, wobei
**L¹** -CH₂- oder -CH₂CO- darstellt;
**L²** eine Bindung, **-NR^{N1}-, -NR^{N1}**CH₂- oder -NR^{N1}CH(CH₃)- darstellt;
**R³** Bicyclo[1.1.1]pentyl, Bicyclo[2.1.1]hexyl, Bicyclo[2.2.1]heptyl, Bicyclo[3.1.1]heptyl, Bicyclo[2.2.2]octyl, 4-Homoisotwistyl, Adamantyl, oder Diamantyl, und die vorgenannten Reste optional eine oder mehrere C=C-Doppelbindung(en) enthalten und/oder optional mit einem oder mehreren von **R^{a}, R^{b}, R^{c}, R^{d}** und **R^{e}** substituiert sind;
und **R^{a}, R^{b}, R^{c}, R^{d}, R^{e}** und **R^{N1}** die in Anspruch 1 definierten Bedeutungen haben.

6. Die Verbindung gemäß einem der Ansprüche 1 - 5, wobei
**R2** 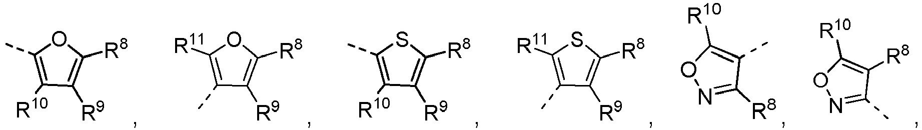 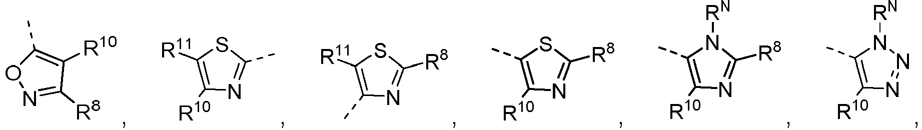 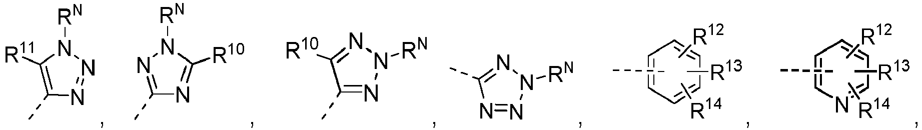 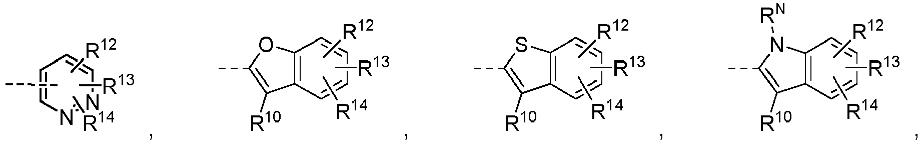 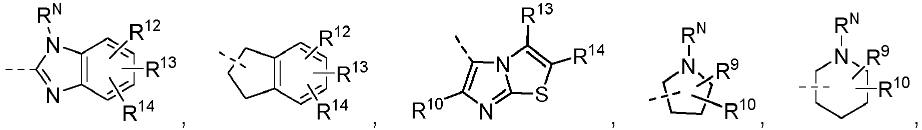 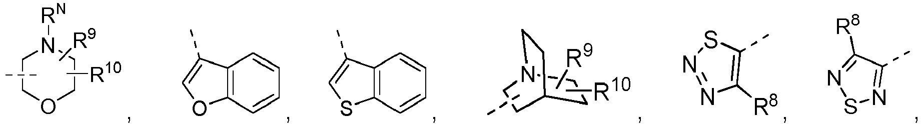 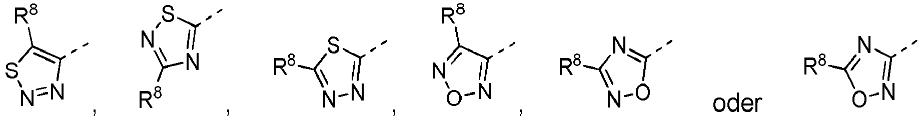
darstellt;
und **R⁸** - **R¹⁴** und **R^{N}** die in Anspruch 1 oder 2 definierten Bedeutungen haben.

7. Die Verbindung gemäß einem der Ansprüche 1 - 6, wobei die Verbindung eine der Formeln **(IV-a)** - **(IV-o)** und **(V-a)** - **(V-d)** aufweist: 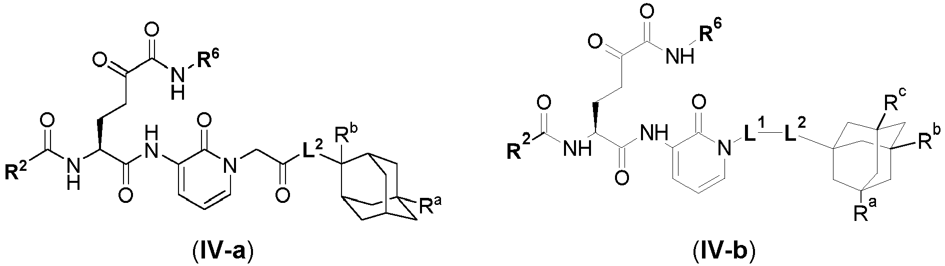 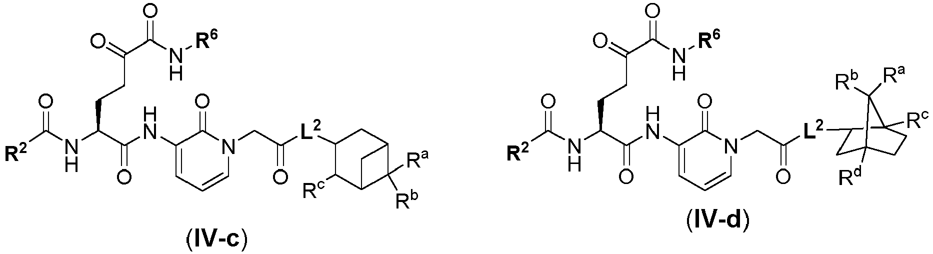 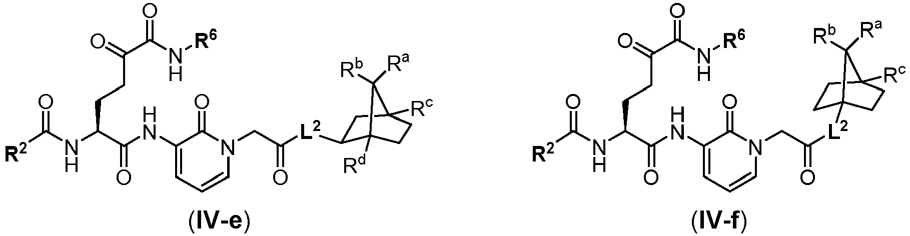 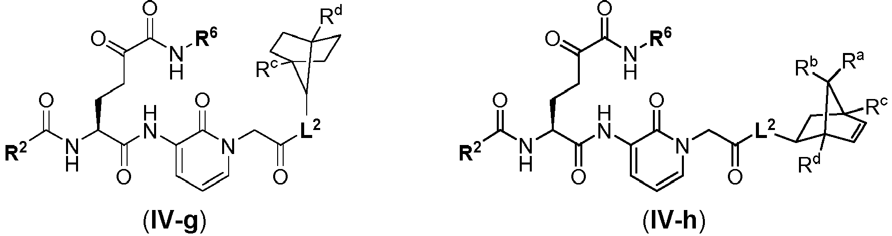 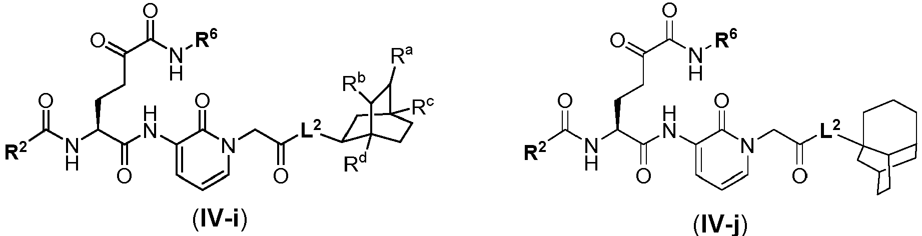 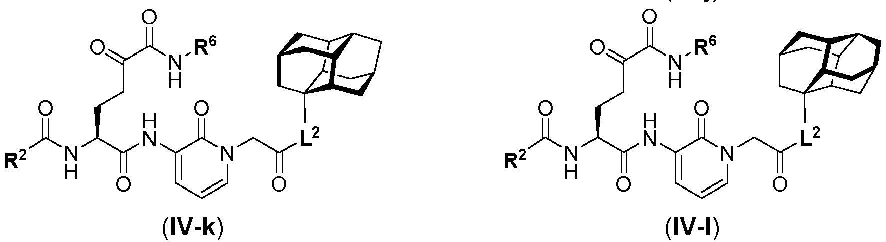 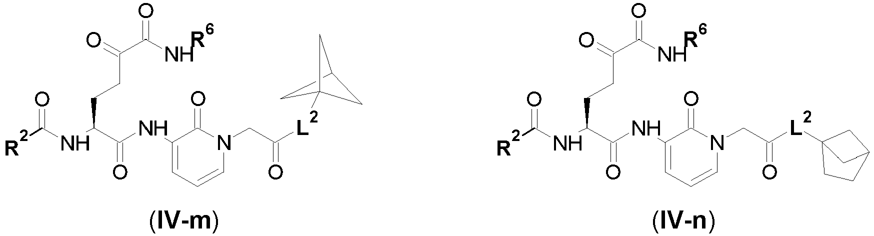 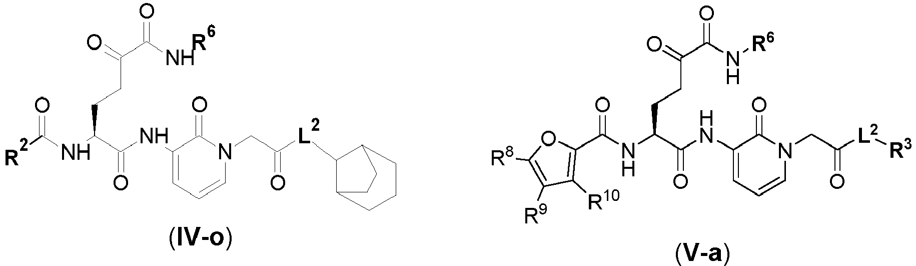 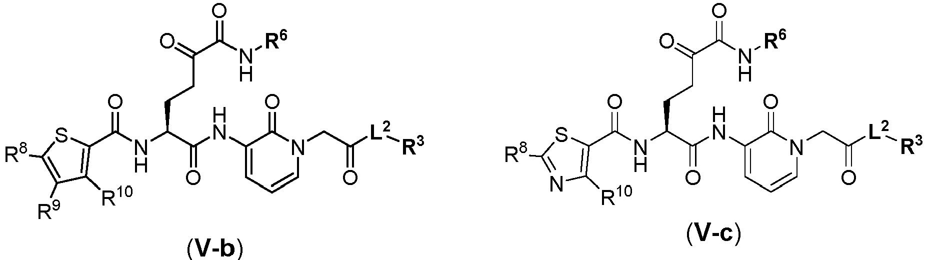 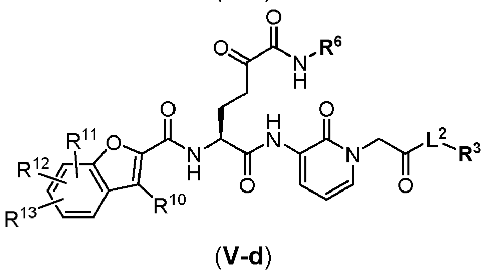 und **R², R³, R⁶, R⁸, R⁹, R¹⁰, R¹¹, R¹², R¹³, R^{a}, R^{b}, R^{c}, R^{d}** und **L²** die in Anspruch 1 definierten Bedeutungen haben.

8. Die Verbindung gemäß einem der Ansprüche 1 - 7, wobei
**R²** 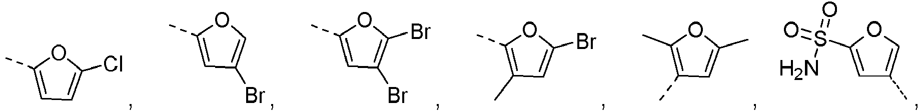 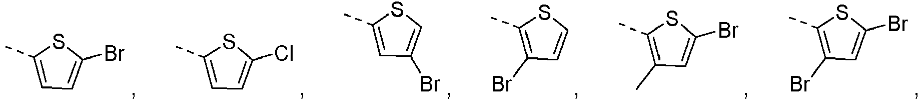 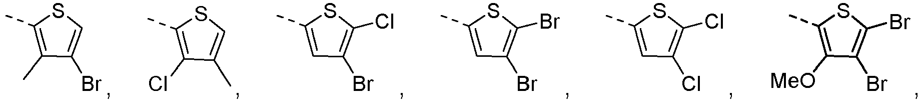 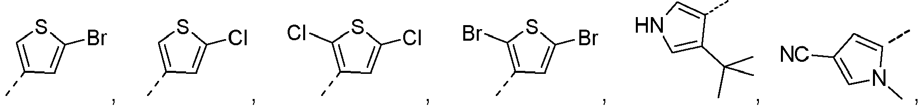 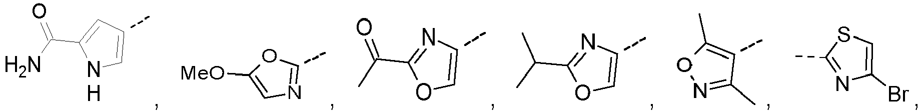 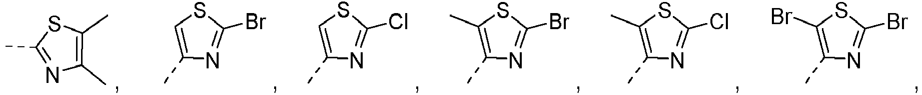 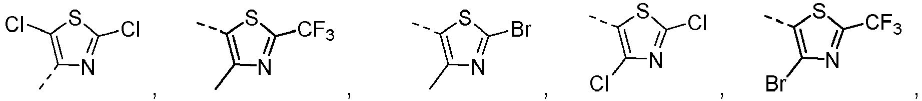 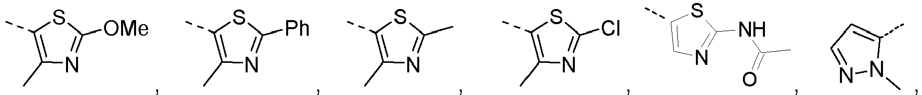 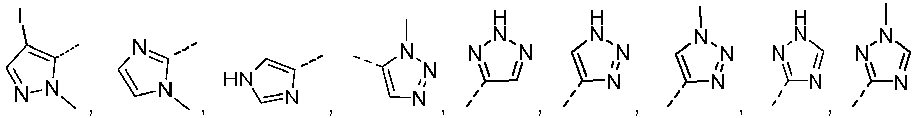 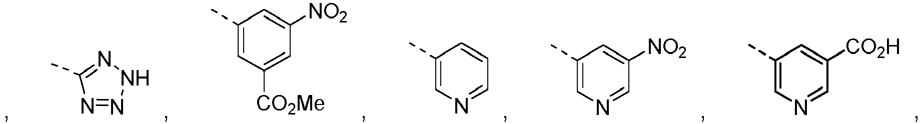 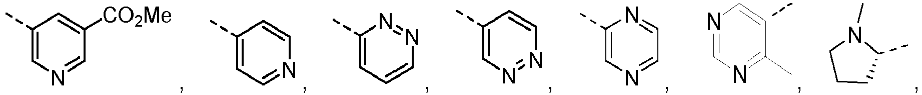 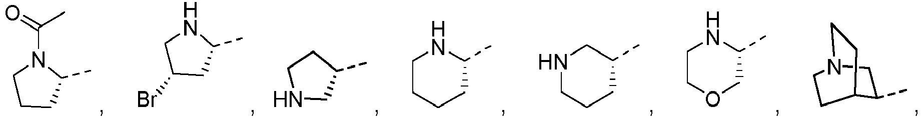 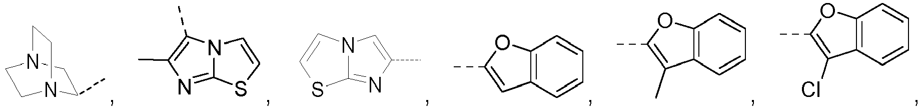 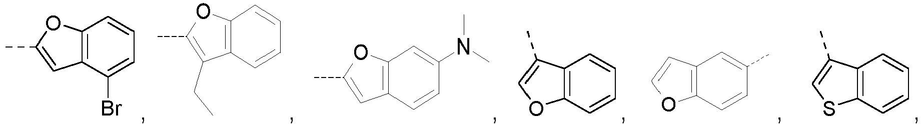 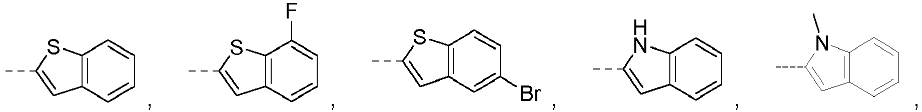 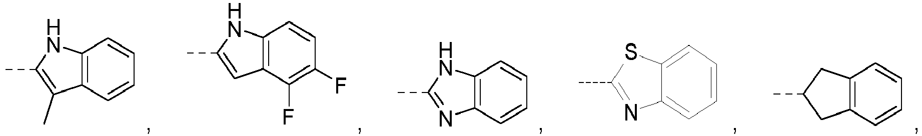 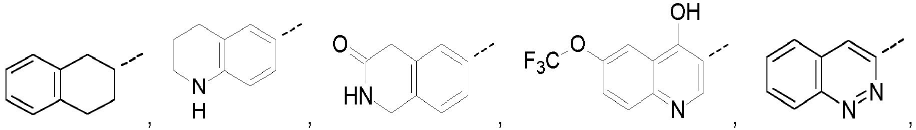 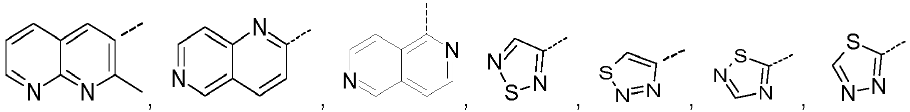 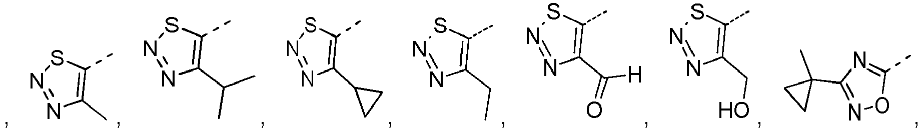 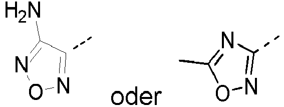 darstellt und
**R⁶** -H, -CH₃, -CH(CH₃)₂, -CH₂CH₂CH₃, -CH₂CH=CH₂, -CH₂CH₂CH₂CH₃, -CH₂CH(CH₃)₂, -C(CH₃)₃, -CH₂CH₂CH₂CH₂CH₃, -cyclo-C₃H₅, -cyclo-C₅H₉, -cyclo-C₆H₁₁ oder -CH₂-cyclo-C₃H₅ darstellt.

9. Die Verbindung gemäß Anspruch 1 ausgewählt aus der Gruppe bestehend aus:
| Verbindung | Name |
|---|---|
| **II-2:** | (S)-2-(Benzofuran-2-carboxamido)-N1-(1-(2-(2-adamantylamino)-2-oxoethyl)-2-oxo-1,2-dihydropyridin-3-yl)-N6-methyl-5-oxohexandiamid |
| **II-3:** | (S)-N1-(1-(2-(2-Adamantylamino)-2-oxoethyl)-2-oxo-1,2-dihydropyridin-3-yl)-N6-methyl-2-(3-methylbenzofuran-2-carboxamido)-5-oxohexandiamid |
| **II-4:** | (S)-2-(3-Chlorbenzofuran-2-carboxamido)-N1-(1-(2-(2-adamantylamino)-2-oxoethyl)-2-oxo-1,2-dihydropyridin-3-yl)-N6-methyl-5-oxohexandiamid |
| **II-5:** | (S)-2-(4-Brombenzofuran-2-carboxamido)-N1-(1-(2-(2-adamantylamino)-2-oxoethyl)-2-oxo-1,2-dihydropyridin-3-yl)-N6-methyl-5-oxohexandiamid |
| **II-6:** | (S)-2-(4-Brombenzofuran-2-carboxamido)-N1-(1-(2-(2-adamantylamino)-2-oxoethyl)-2-oxo-1,2-dihydropyridin-3-yl)-N6-methyl-5-oxohexandiamid |
| **II-7:** | (S)-2-(Benzo[b]thiophen-2-carboxamido)-N1-(1-(2-(2-adamantylamino)-2-oxoethyl)-2-oxo-1,2-dihydropyridin-3-yl)-N6-methyl-5-oxohexandiamid |
| **II-8:** | (S)-2-(5-Brombenzo[b]thiophen-2-carboxamido)-N1-(1-(2-(2-adamantylamino)-2-oxoethyl)-2-oxo-1,2-dihydropyridin-3-yl)-N6-methyl-5-oxohexandiamid |
| **II-9:** | (S)-2-(1H-Indol-2-carboxamido)-N1-(1-(2-(2-adamantylamino)-2-oxoethyl)-2-oxo-1,2-dihydropyridin-3-yl)-N6-methyl-5-oxohexandiamid |
| **II-10:** | (S)-2-(4,5-Difluor-1H-indole-2-carboxamido)-N1-(1-(2-(2-adamantylamino)-2-oxoethyl)-2-oxo-1,2-dihydropyridin-3-yl)-N6-methyl-5-oxohexandiamid |
| **II-11:** | (S)-N1-(1-(2-(2-Adamantylamino)-2-oxoethyl)-2-oxo-1,2-dihydropyridin-3-yl)-N6-methyl-2-(3-methyl-1H-indol-2-carboxamido)-5-oxohexandiamid |
| **II-12:** | (S)-2-(1H-Benzo[d]imidazol-2-carboxamido)-N1-(1-(2-(2-adamantylamino)-2-oxoethyl)-2-oxo-1,2-dihydropyridin-3-yl)-N6-methyl-5-oxohexandiamid |
| **II-13:** | (S)-2-(2,3-Dihydro-1H-inden-2-carboxamido)-N1-(1-(2-(2-adamantylamino)-2-oxoethyl)-2-oxo-1,2-dihydropyridin-3-yl)-N6-methyl-5-oxohexandiamid |
| **II-14:** | (S)--(2-Brom-4-methylthiazol-5-carboxamido)-N1-(1-(2-(2-adamantylamino)-2-oxoethyl)-2-oxo-1,2-dihydropyridin-3-yl)-N6-methyl-5-oxohexandiamid |
| **II-15:** | (S)-N1-(1-(2-(2-Adamantylamino)-2-oxoethyl)-2-oxo-1,2-dihydropyridin-3-yl)-N6-methyl-2-(4-methyl-2-(trifluormethyl)thiazole-5-carboxamido)-5-oxohexandiamid |
| **II-16:** | (S)-2-(4-Brom-2-(trifluormethyl)thiazol-5-carboxamido)-N1-(1-(2-(2-adamantylamino)-2-oxoethyl)-2-oxo-1,2-dihydropyridin-3-yl)-N6-methyl-5-oxohexandiamid |
| **II-17:** | (S)-2-(2,4-Dichlorthiazol-5-carboxamido)-N1-(1-(2-(2-adamantylamino)-2-oxoethyl)-2-oxo-1,2-dihydropyridin-3-yl)-N6-methyl-5-oxohexandiamid |
| **II-18:** | (S)-N1-(1-(2-(2-Adamantylamino)-2-oxoethyl)-2-oxo-1,2-dihydropyridin-3-yl)-2-(2-methoxy-4-methylthiazol-5-carboxamido)-N6-methyl-5-oxohexandiamid |
| **II-19:** | (S)-N1-(1-(2-(2-Adamantylamino)-2-oxoethyl)-2-oxo-1,2-dihydropyridin-3-yl)-N6-methyl-2-(4-methyl-2-phenylthiazol-5-carboxamido)-5-oxohexandiamid |
| **II-20:** | (S)-2-(2,4-Dimethylthiazol-5-carboxamido)-N1-(1-(2-(2-adamantylamino)-2-oxoethyl)-2-oxo-1,2-dihydropyridin-3-yl)-N6-methyl-5-oxohexandiamid |
| **II-21:** | (S)-2-(5-Brom-3-methylthiophen-2-carboxamido)-N1-(1-(2-(2-adamantylamino)-2-oxoethyl)-2-oxo-1,2-dihydropyridin-3-yl)-N6-methyl-5-oxohexandiamid |
| **II-22:** | (S)-2-(3,5-Dibromthiophen-2-carboxamido)-N1-(1-(2-(2-adamantylamino)-2-oxoethyl)-2-oxo-1,2-dihydropyridin-3-yl)-N6-methyl-5-oxohexandiamid |
| **II-23:** | (S)-2-(5-Bromthiophen-2-carboxamido)-N1-(1-(2-(2-adamantylamino)-2-oxoethyl)-2-oxo-1,2-dihydropyridin-3-yl)-N6-methyl-5-oxohexandiamid |
| **II-24:** | (S)-2-(5-Chlorthiophen-2-carboxamido)-N1-(1-(2-(2-adamantylamino)-2-oxoethyl)-2-oxo-1,2-dihydropyridin-3-yl)-N6-methyl-5-oxohexandiamid |
| **II-25:** | (S)-2-(5-Brom-3-methylfuran-2-carboxamido)-N1-(1-(2-(2-adamantylamino)-2-oxoethyl)-2-oxo-1,2-dihydropyridin-3-yl)-N6-methyl-5-oxohexandiamid |
| **II-26:** | (S)-2-(5-Chlorfuran-2-carboxamido)-N1-(1-(2-(2-adamantylamino)-2-oxoethyl)-2-oxo-1,2-dihydropyridin-3-yl)-N6-methyl-5-oxohexandiamid |
| **II-27:** | (S)-2-(5-Chlorthiophen-3-carboxamido)-N1-(1-(2-(2-adamantylamino)-2-oxoethyl)-2-oxo-1,2-dihydropyridin-3-yl)-N6-methyl-5-oxohexandiamid |
| **II-28:** | (S)-2-(2,5-Dichlorthiophen-3-carboxamido)-N1-(1-(2-(2-adamantylamino)-2-oxoethyl)-2-oxo-1,2-dihydropyridin-3-yl)-N6-methyl-5-oxohexandiamid |
| **II-29:** | (S)-2-(2,5-Dibromthiophen-3-carboxamido)-N1-(1-(2-(2-adamantylamino)-2-oxoethyl)-2-oxo-1,2-dihydropyridin-3-yl)-N6-methyl-5-oxohexandiamid |
| **II-30:** | (S)-2-(5-Bromthiophen-3-carboxamido)-N1-(1-(2-(2-adamantylamino)-2-oxoethyl)-2-oxo-1,2-dihydropyridin-3-yl)-N6-methyl-5-oxohexandiamid |
| **II-31:** | (S)-2-(2-Chlor-5-methylthiazol-4-carboxamido)-N1-(1-(2-(2-adamantylamino)-2-oxoethyl)-2-oxo-1,2-dihydropyridin-3-yl)-N6-methyl-5-oxohexandiamid |
| **II-32:** | (S)-2-(2,5-Dichlorthiazol-4-carboxamido)-N1-(1-(2-(2-adamantylamino)-2-oxoethyl)-2-oxo-1,2-dihydropyridin-3-yl)-N6-methyl-5-oxohexandiamid |
| **II-33:** | (S)-2-(2,5-Dibromthiazol-4-carboxamido)-N1-(1-(2-(2-adamantylamino)-2-oxoethyl)-2-oxo-1,2-dihydropyridin-3-yl)-N6-methyl-5-oxohexandiamid |
| **II-34:** | (S)-2-(2-Brom-5-methylthiazol-4-carboxamido)-N1-(1-(2-(2-adamantylamino)-2-oxoethyl)-2-oxo-1,2-dihydropyridin-3-yl)-N6-methyl-5-oxohexandiamid |
| **II-35:** | (S)-2-(2-Bromthiazol-4-carboxamido)-N1-(1-(2-(2-adamantylamino)-2-oxoethyl)-2-oxo-1,2-dihydropyridin-3-yl)-N6-methyl-5-oxohexandiamid |
| **II-36:** | (S)-2-(2-Chlorthiazol-4-carboxamido)-N 1-(1-(2-(2-adamantylamino)-2-oxoethyl)-2-oxo-1,2-dihydropyridin-3-yl)-N6-methyl-5-oxohexandiamid |
| **II-37:** | (S)-2-(2,5-Dimethylfuran-3-carboxamido)-N1-(1-(2-(2-adamantylamino)-2-oxoethyl)-2-oxo-1,2-dihydropyridin-3-yl)-N6-methyl-5-oxohexandiamid |
| **II-38:** | (S)-2-(4,5-Dimethylthiazol-2-carboxamido)-N1-(1-(2-(2-adamantylamino)-2-oxoethyl)-2-oxo-1,2-dihydropyridin-3-yl)-N6-methyl-5-oxohexandiamid |
| **II-39:** | (S)-2-(4-Bromthiazol-2-carboxamido)-N 1-(1-(2-(2-adamantylamino)-2-oxoethyl)-2-oxo-1,2-dihydropyridin-3-yl)-N6-methyl-5-oxohexandiamid |
| **II-40:** | (S)-2-(4-Bromthiophen-2-carboxamido)-N1-(1-(2-(2-adamantylamino)-2-oxoethyl)-2-oxo-1,2-dihydropyridin-3-yl)-N6-methyl-5-oxohexandiamid |
| **II-41:** | (S)-2-(4-Brom-3-methylthiophen-2-carboxamido)-N1-(1-(2-(2-adamantylamino)-2-oxoethyl)-2-oxo-1,2-dihydropyridin-3-yl)-N6-methyl-5-oxohexandiamid |
| **II-42:** | (S)-2-(3-Bromthiophen-2-carboxamido)-N1-(1-(2-(2-adamantylamino)-2-oxoethyl)-2-oxo-1,2-dihydropyridin-3-yl)-N6-methyl-5-oxohexandiamid |
| **II-43:** | (S)-2-(3-Chlor-4-methylthiophen-2-carboxamido)-N1-(1-(2-(2-adamantylamino)-2-oxoethyl)-2-oxo-1,2-dihydropyridin-3-yl)-N6-methyl-5-oxohexandiamid |
| **II-44:** | (S)-2-(4-Brom-5-chlorthiophen-2-carboxamido)-N1-(1-(2-(2-adamantylamino)-2-oxoethyl)-2-oxo-1,2-dihydropyridin-3-yl)-N6-methyl-5-oxohexandiamid |
| **II-45:** | (S)-2-(4,5-Dibromthiophene-2-carboxamido)-N1-(1-(2-(2-adamantylamino)-2-oxoethyl)-2-oxo-1,2-dihydropyridin-3-yl)-N6-methyl-5-oxohexandiamid |
| **II-46:** | (S)-2-(4,5-Dibrom-3-methoxythiophen-2-carboxamido)-N1-(1-(2-(2-adamantylamino)-2-oxoethyl)-2-oxo-1,2-dihydropyridin-3-yl)-N6-methyl-5-oxohexandiamid |
| **II-47:** | (S)-2-(4-Bromfuran-2-carboxamido)-N1-(1-(2-(2-adamantylamino)-2-oxoethyl)-2-oxo-1,2-dihydropyridin-3-yl)-N6-methyl-5-oxohexandiamid |
| **II-48:** | (S)-2-(4,5-Dibromfuran-2-carboxamido)-N1-(1-(2-(2-adamantylamino)-2-oxoethyl)-2-oxo-1,2-dihydropyridin-3-yl)-N6-methyl-5-oxohexandiamid |
| **II-49:** | (S)-2-(4,5-Dichlorthiophen-2-carboxamido)-N1-(1-(2-(2-adamantylamino)-2-oxoethyl)-2-oxo-1,2-dihydropyridin-3-yl)-N6-methyl-5-oxohexandiamid |
| **II-50:** | (S)-2-((S)-1-Acetylpyrrolidin-2-carboxamido)-N1-(1-(2-(2-adamantylamino)-2-oxoethyl)-2-oxo-1,2-dihydropyridin-3-yl)-N6-methyl-5-oxohexandiamid |
| **II-51**: | (S)-N1-(1-(2-(2-Adamantylamino)-2-oxoethyl)-2-oxo-1,2-dihydropyridin-3-yl)-N6-methyl-2-(1-methyl-1H-1,2,3-triazol-5-carboxamido)-5-oxohexandiamid |
| **II-52:** | (S)-N1-(1-(2-(2-Adamantylamino)-2-oxoethyl)-2-oxo-1,2-dihydropyridin-3-yl)-N6-methyl-5-oxo-2-(2H-tetrazol-5-carboxamido)hexandiamid |
| **II-53:** | (S)-N1-(1-(2-(2-Adamantylamino)-2-oxoethyl)-2-oxo-1,2-dihydropyridin-3-yl)-N6-methyl-5-oxo-2-(pyrazine-2-carboxamido)hexandiamid |
| **II-54:** | (S)-N1-(1-(2-(2-Adamantylamino)-2-oxoethyl)-2-oxo-1,2-dihydropyridin-3-yl)-N6-methyl-2-((S)-1-methylpyrrolidin-2-carboxamido)-5-oxohexandiamid |
| **II-55:** | (S)-N1-(1-(2-(2-Adamantylamino)-2-oxoethyl)-2-oxo-1,2-dihydropyridin-3-yl)-N6-methyl-5-oxo-2-((S)-pyrrolidin-3-carboxamido)hexandiamid |
| **II-56:** | (S)-2-((2S,4S)-4-Brompyrrolidin-2-carboxamido)-N1-(1-(2-(2-adamantylamino)-2-oxoethyl)-2-oxo-1,2-dihydropyridin-3-yl)-N6-methyl-5-oxohexandiamid |
| **II-58:** | (S)-N1-(1-(2-(2-Adamantylamino)-2-oxoethyl)-2-oxo-1,2-dihydropyridin-3-yl)-N6-methyl-5-oxo-2-((S)-piperidin-2-carboxamido)hexandiamid |
| **II-59:** | (S)-N1-(1-(2-(2-Adamantylamino)-2-oxoethyl)-2-oxo-1,2-dihydropyridin-3-yl)-N6-methyl-5-oxo-2-((R)-piperidin-3-carboxamido)hexandiamid |
| **II-60:** | (S)-N1-(1-(2-(2-Adamantylamino)-2-oxoethyl)-2-oxo-1,2-dihydropyridin-3-yl)-N6-methyl-2-((R)-morpholin-3-carboxamido)-5-oxohexandiamid |
| **II-61:** | (S)-N1-(1-(2-(2-Adamantylamino)-2-oxoethyl)-2-oxo-1,2-dihydropyridin-3-yl)-N6-methyl-5-oxo-2-(chinuclidine-3-carboxamido)hexandiamid |
| **II-62:** | (S)-Methyl 3-(1-(1-(2-(2-adamantylamino)-2-oxoethyl)-2-oxo-1,2-dihydropyridin-3-ylamino)-6-(methylamino)-1,5,6-trioxohexan-2-ylcarbamoyl)-5-nitrobenzoat |
| **II-63:** | (S)-N1-(1-(2-(2-Adamantylamino)-2-oxoethyl)-2-oxo-1,2-dihydropyridin-3-yl)-N6-methyl-2-(5-nitronicotinamido)-5-oxohexandiamid |
| **II-64:** | (S)-5-(1-(1-(2-(2-Adamantylamino)-2-oxoethyl)-2-oxo-1,2-dihydropyridin-3-ylamino)-6-(methylamino)-1,5,6-trioxohexan-2-ylcarbamoyl)nikotinsäure |
| **II-65:** | (S)-Methyl 5-(1-(1-(2-(2-adamantylamino)-2-oxoethyl)-2-oxo-1,2-dihydropyridin-3-ylamino)-6-(methylamino)-1,5,6-trioxohexan-2-ylcarbamoyl)nicotinat |
| **II-66:** | (S)-N1-(1-(2-(2-Adamantylamino)-2-oxoethyl)-2-oxo-1,2-dihydropyridin-3-yl)-N6-methyl-2-(6-methylimidazo[2,1-b]thiazole-5-carboxamido)-5-oxohexandiamid |
| **II-67:** | (S)-N1-(1-(2-(2-Adamantyl(methyl)amino)-2-oxoethyl)-2-oxo-1,2-dihydropyridin-3-yl)-N6-methyl-2-(3-methylbenzofuran-2-carboxamido)-5-oxohexandiamid |
| **II-68:** | (S)-N1-(1-(2-(5-Hydroxyadamantan-2-amino)-2-oxoethyl)-2-oxo-1,2-dihydropyridin-3-yl)-N6-methyl-2-(3-methylbenzofuran-2-carboxamido)-5-oxohexandiamid |
| **II-69:** | (S)-N1-(1-(2-(5-Fluoradamantan-2-amino)-2-oxoethyl)-2-oxo-1,2-dihydropyridin-3-yl)-N6-methyl-2-(3-methylbenzofuran-2-carboxamido)-5-oxohexandiamid |
| **II-70:** | (S)-N1-(1-(2-(5-Chloradamantan-2-amino)-2-oxoethyl)-2-oxo-1,2-dihydropyridin-3-yl)-N6-methyl-2-(3-methylbenzofuran-2-carboxamido)-5-oxohexandiamid |
| **II-71:** | (S)-N1-(1-(2-(5-Bromadamantan-2-amino)-2-oxoethyl)-2-oxo-1,2-dihydropyridin-3-yl)-N6-methyl-2-(3-methylbenzofuran-2-carboxamido)-5-oxohexandiamid |
| **II-72:** | (S)-N1-(1-(2-(5-Methyladamantan-2-amino)-2-oxoethyl)-2-oxo-1,2-dihydropyridin-3-yl)-N6-methyl-2-(3-methylbenzofuran-2-carboxamido)-5-oxohexandiamid |
| **II-73:** | (S)-N1-(1-(2-(2-Carbonitriladamantan-2-amino)-2-oxoethyl)-2-oxo-1,2-dihydropyridin-3-yl)-N6-methyl-2-(3-methylbenzofuran-2-carboxamido)-5-oxohexandiamid |
| **II-74:** | (S)-N1-(1-(2-(2-Methyl-adamantan-2-carboxylat-2-amino)-2-oxoethyl)-2-oxo-1,2-dihydropyridin-3-yl)-N6-methyl-2-(3-methylbenzofuran-2-carboxamido)-5-oxohexandiamid |
| **II-87:** | (S)-N1-(1-(2-(1-Adamantylmethylamino)-2-oxoethyl)-2-oxo-1,2-dihydropyridin-3-yl)-N6-methyl-2-(3-methylbenzofuran-2-carboxamido)-5-oxohexandiamid |
| **II-88:** | (S)-N1-(1-(2-(1-(1-Adamantyl)ethanamino)-2-oxoethyl)-2-oxo-1,2-dihydropyridin-3-yl)-N6-methyl-2-(3-methylbenzofuran-2-carboxamido)-5-oxohexandiamid |
| **II-90:** | (S)-N1-(1-(2-((1R,2S,4S)-Bicyclo[2.2.1]heptan-2-ylamino)-2-oxoethyl)-2-oxo-1,2-dihydropyridin-3-yl)-N6-methyl-2-(3-methylbenzofuran-2-carboxamido)-5-oxohexandiamid |
| **II-92:** | (S)-N1-Methyl-5-(3-methylbenzofuran-2-carboxamido)-2-oxo-N6-(2-oxo-1-(2-oxo-2-((1S,2S,4R)-1,7,7-trimethylbicyclo[2.2.1]heptan-2-ylamino)ethyl)-1,2-dihydropyridin-3-yl)hexandiamid |
| **II-94:** | (S)-N1-(1-(2-((1R,2R,4S)-Bicyclo[2.2.1]heptan-2-ylamino)-2-oxoethyl)-2-oxo-1,2-dihydropyridin-3-yl)-N6-methyl-2-(3-methylbenzofuran-2-carboxamido)-5-oxohexandiamid |
| **II-95:** | (S)-N1-(1-(2-(Bicyclo[2.2.1]heptan-1-ylamino)-2-oxoethyl)-2-oxo-1,2-dihydropyridin-3-yl)-N6-methyl-2-(3-methylbenzofuran-2-carboxamido)-5-oxohexandiamid |
| **II-96:** | (S)-N1-(1-(2-(Bicyclo[2.2.1]heptan-7-ylam ino)-2-oxoethyl)-2-oxo-1,2-dihydropyridin-3-yl)-N6-methyl-2-(3-methylbenzofuran-2-carboxamido)-5-oxohexandiamid |
| **II-97:** | (S)-N1-(1-(2-(Bicyclo[2.2.1]hept-5-en-2-ylam ino)-2-oxoethyl)-2-oxo-1,2-dihydropyridin-3-yl)-N6-methyl-2-(3-methylbenzofuran-2-carboxamido)-5-oxohexandiamid |
| **II-98:** | (2S)-N1-(1-(2-(Bicyclo[2.2.2]octan-2-ylamino)-2-oxoethyl)-2-oxo-1,2-dihydropyridin-3-yl)-N6-methyl-2-(3-methylbenzofuran-2-carboxamido)-5-oxohexandiamid |
| **II-99:** | (S)-N1-Methyl-5-(3-methylbenzofuran-2-carboxamido)-2-oxo-N6-(2-oxo-1-(2-oxo-2-((1R,2R,4R)-1,7,7-trimethylbicyclo[2.2.1]heptan-2-ylamino)ethyl)-1,2-dihydropyridin-3-yl)hexandiamid |
| **II-100:** | (S)-N1-Methyl-5-(3-methylbenzofuran-2-carboxamido)-2-oxo-N6-(2-oxo-1-(2-oxo-2-((1R,2R,3R,5S)-2,6,6-trimethylbicyclo[3.1.1]heptan-3-ylamino)ethyl)-1,2-dihydropyridin-3-yl)hexandiamid |
| **II-101**: | (S)-N1-Methyl-5-(3-methylbenzofuran-2-carboxamido)-2-oxo-N6-(2-oxo-1-(2-oxo-2-((1S,2S,3S,5R)-2,6,6-trimethylbicyclo[3.1.1]heptan-3-ylamino)ethyl)-1,2-dihydropyridin-3-yl)hexandiamid |
| **II-103:** | (S)-N1-(1-(2-(4-Homoisotwistan-3-amino)-2-oxoethyl)-2-oxo-1,2-dihydropyridin-3-yl)-N6-methyl-2-(3-methylbenzofuran-2-carboxamido)-5-oxohexandiamid |
| **II-104:** | (S)-N1-(1-(2-(Diamantan-1-amino)-2-oxoethyl)-2-oxo-1,2-dihydropyridin-3-yl)-N6-methyl-2-(3-methylbenzofuran-2-carboxamido)-5-oxohexandiamid |
| **II-105:** | (S)-N1-(1-(2-(Diamantan-4-amino)-2-oxoethyl)-2-oxo-1,2-dihydropyridin-3-yl)-N6-methyl-2-(3-methylbenzofuran-2-carboxamido)-5-oxohexandiamid |
| **II-107:** | (S)-N1-(1-(1-Adamantylmethyl)-2-oxo-1,2-dihydropyridin-3-yl)-N6-methyl-2-(3-methylbenzofuran-2-carboxamido)-5-oxohexandiamid |
| **II-108:** | (2S)-N1-(1-((3-Hydroxy-1-adamantyl)methyl)-2-oxo-1,2-dihydropyridin-3-yl)-N6-methyl-2-(3-methylbenzofuran-2-carboxamido)-5-oxohexandiamid |
| **II-109:** | (2S)-N1-(1-((3-Brom-1-adamantyl)methyl)-2-oxo-1,2-dihydropyridin-3-yl)-N6-methyl-2-(3-methylbenzofuran-2-carboxamido)-5-oxohexandiamid |
| **II-110:** | (S)-N1-(1-(2-Adamantylmethyl)-2-oxo-1,2-dihydropyridin-3-yl)-N6-methyl-2-(3-methylbenzofuran-2-carboxamido)-5-oxohexandiamid |
| **II-111:** | (S)-N1-(1-(2-(2-Adamantylamino)-2-oxoethyl)-2-oxo-1,2-dihydropyridin-3-yl)-N6-methyl-2-(nicotinamido)-5-oxohexandiamid |
| **II-112:** | (S)-2-(Isonicotinamido)-N1-(1-(2-(2-adamantylamino)-2-oxoethyl)-2-oxo-1,2-dihydropyridin-3-yl)-N6-methyl-5-oxohexandiamid |
| **II-113:** | (S)-N1-(1-(2-(2-Adamantylamino)-2-oxoethyl)-2-oxo-1,2-dihydropyridin-3-yl)-N6-methyl-5-oxo-2-(pyridazine-4-carboxamido)hexandiamid |
| **II-114:** | (S)-N1-(1-(2-(2-Adamantylamino)-2-oxoethyl)-2-oxo-1,2-dihydropyridin-3-yl)-N6-methyl-5-oxo-2-(pyridazin-3-carboxamido)hexandiamid |
| **II-115:** | (S)-N1-Cyclopropyl-N6-(1-(2-(2-adamantylamino)-2-oxoethyl)-2-oxo-1,2-dihydropyridin-3-yl)-5-(3-methylbenzofuran-2-carboxamido)-2-oxohexandiamid |
| **II-116:** | (S)-N1-(1-(2-(2-Adamantylamino)-2-oxoethyl)-2-oxo-1,2-dihydropyridin-3-yl)-2-(3-methylbenzofuran-2-carboxam ido)-5-oxo-N6-pentylhexandiamid |
| **II-117:** | (S)-N1-Allyl-N6-(1-(2-(2-adamantylamino)-2-oxoethyl)-2-oxo-1,2-dihydropyridin-3-yl)-5-(3-methylbenzofuran-2-carboxamido)-2-oxohexandiamid |
| **II-118:** | (S)-N1-(1-(2-(2-Adamantylamino)-2-oxoethyl)-2-oxo-1,2-dihydropyridin-3-yl)-2-(3-methylbenzofuran-2-carboxamido)-5-oxohexandiamid |
| **II-119:** | (S)-N1-Allyl-5-(benzofuran-2-carboxamido)-N6-(1-(2-(2-adamantylamino)-2-oxoethyl)-2-oxo-1,2-dihydropyridin-3-yl)-2-oxohexandiamid |
| **II-120:** | (S)-2-(Benzofuran-2-carboxamido)-N6-isopropyl-N1-(1-(2-(2-adamantylamino)-2-oxoethyl)-2-oxo-1,2-dihydropyridin-3-yl)-5-oxohexandiamid |
| **II-121**: | (S)-2-(Benzofuran-2-carboxamido)-N6-cyclopropyl-N1-(1-(2-(2-adamantylamino)-2-oxoethyl)-2-oxo-1,2-dihydropyridin-3-yl)-5-oxohexandiamid |
| **II-122:** | (S)-2-(Benzofuran-2-carboxamido)-N1-(1-(2-(2-adamantylamino)-2-oxoethyl)-2-oxo-1,2-dihydropyridin-3-yl)-5-oxo-N6-phenylhexandiamid |
| **II-123:** | (S)-2-(Benzofuran-2-carboxamido)-N6-benzyl-N1-(1-(2-(2-adamantylamino)-2-oxoethyl)-2-oxo-1,2-dihydropyridin-3-yl)-5-oxohexandiamid |
| **II-124:** | (S)-2-(Benzofuran-2-carboxamido)-N1-(1-(2-(2-adamantylamino)-2-oxoethyl)-2-oxo-1,2-dihydropyridin-3-yl)-5-oxohexandiamid |
| **II-125:** | (S)-2-(2,5-Dichlorthiophen-3-carboxamido)-N1-(1-(2-(2-adamantylamino)-2-oxoethyl)-2-oxo-1,2-dihydropyridin-3-yl)-5-oxohexandiamid |
| **II-126:** | (S)-N1-(1-(2-(2-Adamantylamino)-2-oxoethyl)-2-oxo-1,2-dihydropyridin-3-yl)-2-(4-methyl-2-(trifluormethyl)thiazol-5-carboxamido)-5-oxohexandiamid |
| **II-127:** | (S)-N1-(1-(2-(2-Adamantylamino)-2-oxoethyl)-2-oxo-1,2-dihydropyridin-3-yl)-2-(1-methyl-1H-1,2,3-triazol-5-carboxamido)-5-oxohexandiamid |
| **II-128:** | (2S)-N1-(1-(2-((1R,2R,4S)-Bicyclo[2.2.1]heptan-2-ylamino)-2-oxoethyl)-2-oxo-1,2-dihydropyridin-3-yl)-2-(2,5-dichlorthiophen-3-carboxamido)-N6-methyl-5-oxohexandiamid |
| **II-129:** | (2S)-N1-(1-(2-((1R,2R,4S)-Bicyclo[2.2.1]heptan-2-ylamino)-2-oxoethyl)-2-oxo-1,2-dihydropyridin-3-yl)-N6-methyl-2-(4-methyl-2-(trifluormethyl)thiazol-5-carboxamido)-5-oxohexandiamid |
| **II-130:** | (2S)-N1-(1-(2-((1R,2R,4S)-Bicyclo[2.2.1]heptan-2-ylamino)-2-oxoethyl)-2-oxo-1,2-dihydropyridin-3-yl)-N6-methyl-2-(1-methyl-1H-1,2,3-triazol-5-carboxamido)-5-oxohexandiamid |
| **II-131**: | (S)-N1-(1-(2-(2-Adamantylamino)-2-oxoethyl)-2-oxo-1 ,2-dihydropyridin-3-yl)-N6-methyl-5-oxo-2-(2H-1,2,3-triazol-4-carboxamido)hexandiamid |
| **II-132:** | (S)-N1-(1-(2-(2-Adamantylamino)-2-oxoethyl)-2-oxo-1,2-dihydropyridin-3-yl)-N6-methyl-5-oxo-2-(1H-1,2,3-triazol-4-carboxamido)hexandiamid |
| **II-133:** | (S)-N1-(1-(2-(2-Adamantylamino)-2-oxoethyl)-2-oxo-1,2-dihydropyridin-3-yl)-N6-methyl-2-(1-methyl-1H-1,2,3-triazol-4-carboxamido)-5-oxohexandiamid |
| **II-134:** | (S)-N1-(1-(2-(2-Adamantylamino)-2-oxoethyl)-2-oxo-1,2-dihydropyridin-3-yl)-N6-methyl-5-oxo-2-(1H-1,2,4-triazol-3-carboxamido)hexandiamid |
| **II-135:** | (S)-N1-(1-(2-(2-Adamantylamino)-2-oxoethyl)-2-oxo-1,2-dihydropyridin-3-yl)-N6-methyl-2-(1-methyl-1H-1,2,4-triazol-3-carboxamido)-5-oxohexandiamid |
| **II-136:** | (S)-2-(Benzofuran-3-carboxamido)-N1-(1-(2-(2-adamantylamino)-2-oxoethyl)-2-oxo-1,2-dihydropyridin-3-yl)-N6-methyl-5-oxohexandiamid |
| **II-137:** | (S)-2-(Benzo[b]thiophen-3-carboxamido)-N1-(1-(2-(2-adamantylamino)-2-oxoethyl)-2-oxo-1,2-dihydropyridin-3-yl)-N6-methyl-5-oxohexandiamid |
| **II-138** | (S)-N1-(1-(2-(2-Adamantylamino)-2-oxoethyl)-2-oxo-1,2-dihydropyridin-3-yl)-N6-methyl-2-(1-methyl-1H-pyrazol-3-carboxamido)-5-oxohexandiamid |
| **II-139:** | (S)-N1-(1-(2-(2-Adamantylamino)-2-oxoethyl)-2-oxo-1,2-dihydropyridin-3-yl)-N6-methyl-2-(1-methyl-1H-pyrazol-4-carboxamido)-5-oxohexanediamid |
| **II-140** | (S)-N1-(1-(2-(2-Adamantylamino)-2-oxoethyl)-2-oxo-1,2-dihydropyridin-3-yl)-N6-methyl-2-(1-methyl-1H-pyrazol-5-carboxamido)-5-oxohexandiamid |
| **II-141** | (S)-N1-(1-(2-(2-Adamantylamino)-2-oxoethyl)-2-oxo-1,2-dihydropyridin-3-yl)-N6-methyl-2-(4-methyl-1,2,3-thiadiazol-5-carboxamido)-5-oxohexandiamid |
| **II-142** | (S)-N1-(1-(2-(2-Adamantylamino)-2-oxoethyl)-2-oxo-1,2-dihydropyridin-3-yl)-N6-methyl-5-oxo-2-(1,2,5-thiadiazol-3-carboxamido)hexandiamid |
| **II-143** | (S)-2-(4-Iod-1-methyl-1H-pyrazol-5-carboxamido)-N1-(1-(2-(2-adamantylamino)-2-oxoethyl)-2-oxo-1,2-dihydropyridin-3-yl)-N6-methyl-5-oxohexandiamid |
| **II-144** | (S)-N1-(1-(2-(2-Adamantylamino)-2-oxoethyl)-2-oxo-1,2-dihydropyridin-3-yl)-2-(1-methyl-1H-pyrazol-5-carboxamido)-5-oxohexandiamid |
| **II-145** | (S)-N1-(1-(2-(2-Adamantylamino)-2-oxoethyl)-2-oxo-1,2-dihydropyridin-3-yl)-2-(4-methyl-1,2,3-thiadiazol-5-carboxamido)-5-oxohexandiamid |
| **II-146** | (S)-2-(Benzofuran-2-carboxamido)-N1-(1-(2-((1R,2R,4S)-bicyclo[2.2.1]heptan-2-ylamino)-2-oxoethyl)-2-oxo-1,2-dihydropyridin-3-yl)-5-oxohexandiamid |
| **II-147** | (S)-N1-(1-(2-((1R,2R,4S)-Bicyclo[2.2.1]heptan-2-ylamino)-2-oxoethyl)-2-oxo-1,2-dihydropyridin-3-yl)-2-(3-methylbenzofuran-2-carboxamido)-5-oxohexandiamid |
| **II-148** | (S)-2-(Benzofuran-2-carboxamido)-N1-(1-(2-((1S,2R,4R)-bicyclo[2.2.1]heptan-2-ylamino)-2-oxoethyl)-2-oxo-1,2-dihydropyridin-3-yl)-5-oxohexandiamid |
| **II-149** | (S)-N1-(1-(2-((1S,2R,4R)-Bicyclo[2.2.1]heptan-2-ylamino)-2-oxoethyl)-2-oxo-1,2-dihydropyridin-3-yl)-2-(3-methylbenzofuran-2-carboxamido)-5-oxohexandiamid |
| **II-150** | (S)-2-(Benzofuran-2-carboxamido)-5-oxo-N1-(2-oxo-1-(2-oxo-2-((1R,2S,4R)-1,7,7-trimethylbicyclo[2.2.1]heptan-2-ylamino)ethyl)-1,2-dihydropyridin-3-yl)hexandiamid |
| **II-151** | (S)-2-(3-Methylbenzofuran-2-carboxamido)-5-oxo-N1-(2-oxo-1-(2-oxo-2-((1R,2S,4R)-1,7,7-trimethylbicyclo[2.2.1]heptan-2-ylamino)ethyl)-1,2-dihydropyridin-3-yl)hexandiamid |
| **II-152** | (S)-N1-(1-(2-((1R,2R,4S)-Bicyclo[2.2.1]heptan-2-ylamino)-2-oxoethyl)-2-oxo-1,2-dihydropyridin-3-yl)-N6-methyl-2-(4-methyl-1,2,3-thiadiazol-5-carboxamido)-5-oxohexandiamid |
| **II-153** | (S)-N1-(1-(2-((1R,2R,4S)-Bicyclo[2.2.1]heptan-2-ylamino)-2-oxoethyl)-2-oxo-1,2-dihydropyridin-3-yl)-N6-methyl-2-(1-methyl-1H-pyrazol-5-carboxamido)-5-oxohexandiamid |
| **II-154** | (S)-N1-(1-(2-((1S,2R,4R)-Bicyclo[2.2.1]heptan-2-ylamino)-2-oxoethyl)-2-oxo-1,2-dihydropyridin-3-yl)-N6-methyl-2-(4-methyl-2-(trifluormethyl)thiazol-5-carboxamido)-5-oxohexandiamid |
| **II-155** | (S)-N1-(1-(2-((1S,2R,4R)-Bicyclo[2.2.1]heptan-2-ylamino)-2-oxoethyl)-2-oxo-1,2-dihydropyridin-3-yl)-2-(2,5-dichlorthiophen-3-carboxamido)-N6-methyl-5-oxohexandiamid |
| **II-156** | (S)-N1-(1-(2-((1S,2R,4R)-Bicyclo[2.2.1]heptan-2-ylamino)-2-oxoethyl)-2-oxo-1,2-dihydropyridin-3-yl)-N6-methyl-2-(4-methyl-1,2,3-thiadiazol-5-carboxamido)-5-oxohexandiamid |
| **II-157** | (S)-N1-(1-(2-((1S,2R,4R)-Bicyclo[2.2.1]heptan-2-ylamino)-2-oxoethyl)-2-oxo-1,2-dihydropyridin-3-yl)-N6-methyl-2-(1-methyl-1H-1,2,3-triazol-5-carboxamido)-5-oxohexandiamid |
| **II-158** | (S)-N1-(1-(2-((1S,2R,4R)-Bicyclo[2.2.1]heptan-2-ylamino)-2-oxoethyl)-2-oxo-1,2-dihydropyridin-3-yl)-N6-methyl-2-(1-methyl-1H-pyrazol-5-carboxamido)-5-oxohexandiamid |
| **II-159** | (S)-N1-Methyl-5-(4-methyl-2-(trifluormethyl)thiazol-5-carboxamido)-2-oxo-N6-(2-oxo-1-(2-oxo-2-((1R,2S,4R)-1,7,7-trimethylbicyclo[2.2.1]heptan-2-ylamino)ethyl)-1,2-dihydropyridin-3-yl)hexandiamid |
| **II-160** | (S)-2-(2,5-Dichlorthiophen-3-carboxamido)-N6-methyl-5-oxo-N1-(2-oxo-1-(2-oxo-2-((1R,2S,4R)-1,7,7-trimethylbicyclo[2.2.1]heptan-2-ylamino)ethyl)-1,2-dihydropyridin-3-yl)hexandiamid |
| **II-161** | (S)-N1-Methyl-5-(4-methyl-1,2,3-thiadiazol-5-carboxamido)-2-oxo-N6-(2-oxo-1-(2-oxo-2-((1R,2S,4R)-1,7,7-trimethylbicyclo[2.2.1]heptan-2-ylamino)ethyl)-1,2-dihydropyridin-3-yl)hexandiamid |
| **II-162** | (S)-N1-Methyl-5-(1-methyl-1H-1,2,3-triazol-5-carboxamido)-2-oxo-N6-(2-oxo-1-(2-oxo-2-((1R,2S,4R)-1,7,7-trimethylbicyclo[2.2.1]heptan-2-ylamino)ethyl)-1,2-dihydropyridin-3-yl)hexandiamid |
| **II-163** | (S)-N1-Methyl-5-(1-methyl-1H-pyrazol-5-carboxamido)-2-oxo-N6-(2-oxo-1-(2-oxo-2-((1R,2S,4R)-1,7,7-trimethylbicyclo[2.2.1]heptan-2-ylamino)ethyl)-1,2-dihydropyridin-3-yl)hexandiamid |
| **II-164** | (2S)-2-(4-tert-Butyl-1H-pyrrol-3-carboxamido)-N6-methyl-N1-(1-(2-(1-adamantylamino)ethyl)-2-oxo-1,2-dihydropyridin-3-yl)-5-oxohexandiamid |
| **II-165** | (2S)-2-(4-Cyano-1-methyl-1H-pyrrol-2-carboxamido)-N6-methyl-N1-(1-(3-(1-adamantylamino)propyl)-2-oxo-1,2-dihydropyridin-3-yl)-5-oxohexandiamid |
| **II-166** | (S)-N1-(1-(3-(2-adamantylamino)-3-oxopropyl)-2-oxo-1,2-dihydropyridin-3-yl)-2-(5-methoxyoxazol-2-carboxamido)-N6-methyl-5-oxohexandiamid |
| **II-167** | (S)-N1-(1-(2-(Bicyclo[1.1.1]pentan-1-ylamino)-2-oxoethyl)-2-oxo-1,2-dihydropyridin-3-yl)-N6-methyl-2-(3-methylbenzofuran-2-carboxamido)-5-oxohexandiamid |
| **II-168** | (S)-2-(2-Acetyloxazol-4-carboxamido)-N1-(1-(2-(bicyclo[1.1.1]pentan-1-ylamino)-2-oxoethyl)-2-oxo-1,2-dihydropyridin-3-yl)-N6-methyl-5-oxohexandiamid |
| **II-169** | (S)-N1-(1-(2-(Bicyclo[2.1.1]hexan-1-ylamino)-2-oxoethyl)-2-oxo-1,2-dihydropyridin-3-yl)-N6-methyl-2-(3-methylbenzofuran-2-carboxamido)-5-oxohexandiamid |
| **II-170** | (S)-N1-(1-(2-(Bicyclo[2.1.1]hexan-1-ylamino)-2-oxoethyl)-2-oxo-1,2-dihydropyridin-3-yl)-2-(2-isopropyloxazol-5-carboxamido)-N6-methyl-5-oxohexandiamid |
| **II-171** | (2S)-2-(Benzofuran-2-carboxamido)-N1-(1-(2-(bicyclo[3.2.1]octan-8-ylamino)-2-oxoethyl)-2-oxo-1,2-dihydropyridin-3-yl)-N6-methyl-5-oxohexandiamid |
| **II-172** | (2S)-N1-(1-(2-(Bicyclo[3.2.1]octan-8-ylamino)-2-oxoethyl)-2-oxo-1,2-dihydropyridin-3-yl)-2-(3,5-dimethylisoxazol-4-carboxamido)-N6-methyl-5-oxohexandiamid |
| **II-173** | (S)-N1-(1-(2-(5-Carboxy-2-aminoadamantan)-2-oxoethyl)-2-oxo-1,2-dihydropyridin-3-yl)-N6-methyl-2-(4-methylpyrimidin-5-carboxamido)-5-oxohexandiamid |
| **II-174** | (2S)-N1-(1-(2-(4-Aminoadamantan-N,N-dimethyl-1-carboxamid)-2-oxoethyl)-2-oxo-1,2-dihydropyridin-3-yl)-N6-methyl-5-oxo-2-(1,2,3,4-tetrahydronaphthalin-2-carboxamido)hexandiamid |
| **II-175** | (S)-2-((S)-1,4-Diazabicyclo[2.2.2]octan-2-carboxamido)-N6-tert-butyl-N1-(1-(2-(2-adamantylamino)-2-oxoethyl)-2-oxo-1,2-dihydropyridin-3-yl)-5-oxohexandiamid |
| **II-176** | (S)-N1-tert-Butyl-5-(1H-indol-3-carboxamido)-N6-(1-(2-(2-adamantylamino)-2-oxoethyl)-2-oxo-1,2-dihydropyridin-3-yl)-2-oxohexandiamid |
| **II-177** | (S)-N1-tert-Butyl-N6-(1-(2-(2-adamantylamino)-2-oxoethyl)-2-oxo-1,2-dihydropyridin-3-yl)-5-(6-methylimidazo[2,1-b]thiazol-3-carboxamido)-2-oxohexandiamid |
| **II-178** | (S)-2-(Benzo[d]thiazol-2-carboxamido)-N1-(1-(2-((1S,2R,4R)-bicyclo[2.2.1]heptan-2-ylamino)-2-oxoethyl)-2-oxo-1,2-dihydropyridin-3-yl)-N6-cyclopentyl-5-oxohexandiamid |
| **II-179** | (S)-N1-(1-(2-((1S,2R,4R)-Bicyclo[2.2.1]heptan-2-ylamino)-2-oxoethyl)-2-oxo-1,2-dihydropyridin-3-yl)-N6-cyclopentyl-2-(imidazo[2,1-b]thiazol-6-carboxamido)-5-oxohexandiamid |
| **II-180** | (S)-N1-(1-(2-((1S,2R,4R)-Bicyclo[2.2.1]heptan-2-ylamino)-2-oxoethyl)-2-oxo-1,2-dihydropyridin-3-yl)-N6-cyclopentyl-2-(4-hydroxy-6-(trifluormethoxy)chinolin-3-carboxamido)-5-oxohexandiamid |
| **II-181** | (S)-N1-(1-(2-(Bicyclo[1.1.1]pentan-1-ylamino)-2-oxoethyl)-2-oxo-1,2-dihydropyridin-3-yl)-2-(cinnolin-3-carboxamido)-N6-cyclohexyl-5-oxohexandiamid |
| **II-182** | (S)-N1-(1-(2-(Bicyclo[1.1.1]pentan-1-ylamino)-2-oxoethyl)-2-oxo-1,2-dihydropyridin-3-yl)-N6-cyclohexyl-2-(3-ethylbenzofuran-2-carboxamido)-5-oxohexandiamid |
| **II-183** | (S)-N1-(1-(2-(Bicyclo[1.1.1]pentan-1-ylamino)-2-oxoethyl)-2-oxo-1,2-dihydropyridin-3-yl)-N6-cyclohexyl-2-(1-ethyl-1H-indol-2-carboxamido)-5-oxohexandiamid |
| **II-184** | (S)-N1-(1-(2-(Bicyclo[1.1.1]pentan-1-ylamino)-2-oxoethyl)-2-oxo-1,2-dihydropyridin-3-yl)-N6-cyclohexyl-2-(2-methyl-1,8-naphthyridin-3-carboxamido)-5-oxohexandiamid |
| **II-185** | (S)-N1-(1-(2-(Bicyclo[2.1.1]hexan-1-ylamino)-2-oxoethyl)-2-oxo-1,2-dihydropyridin-3-yl)-N6-methyl-5-oxo-2-(1,2,3,4-tetrahydrochinolin-6-carboxamido)hexandiamid |
| **II-186** | (S)-N1-(1-(2-(2-Carboxy-2-amino-5-(trifluormethyl)adamantan)-2-oxoethyl)-2-oxo-1,2-dihydropyridin-3-yl)-N6-methyl-5-oxo-2-(3-oxo-1,2,3,4-tetrahydroisochinolin-6-carboxamido)hexandiamid |
| **II-187** | (S)-N1-(1-(2-(5-Ethyladamantan-2-amino)-2-oxoethyl)-2-oxo-1,2-dihydropyridin-3-yl)-N6-methyl-2-(1,6-naphthyridin-2-carboxamido)-5-oxohexandiamid |
| **II-188** | (S)-N1-(1-(2-(Bicyclo[2.1.1]hexan-1-ylamino)-2-oxoethyl)-2-oxo-1,2-dihydropyridin-3-yl)-N6-methyl-2-(2,6-naphthyridin-1-carboxamido)-5-oxohexandiamid |
| **II-189** | (S)-2-(4-Amino-1,2,5-oxadiazol-3-carboxamido)-N1-(1-(2-(bicyclo[1.1.1]pentan-1-ylamino)-2-oxoethyl)-2-oxo-1,2-dihydropyridin-3-yl)-N6-methyl-5-oxohexandiamid |
| **II-190** | (S)-N1-(1-(2-(Bicyclo[1.1.1]pentan-1-ylamino)-2-oxoethyl)-2-oxo-1,2-dihydropyridin-3-yl)-2-(6-(dimethylamino)benzofuran-2-carboxamido)-N6-methyl-5-oxohexandiamid |
| **II-191** | (S)-2-(2-Acetamidothiazol-5-carboxamido)-N1-(1-(2-(bicyclo[1.1.1]pentan-1-ylamino)-2-oxoethyl)-2-oxo-1,2-dihydropyridin-3-yl)-N6-methyl-5-oxohexandiamid |
| **II-192** | (S)-N4-(1-(1-(2-(Bicyclo[1.1.1]pentan-1-ylamino)-2-oxoethyl)-2-oxo-1,2-dihydropyridin-3-ylamino)-6-(methylamino)-1,5,6-trioxohexan-2-yl)-1H-pyrrol-2,4-dicarboxamid |
| **II-193** | (S)-N1-(1-(2-(1-Acetylamino-4-aminoadamantan)-2-oxoethyl)-2-oxo-1,2-dihydropyridin-3-yl)-N6-methyl-5-oxo-2-(5-sulfamoylfuran-3-carboxamido)hexandiamid |
| **II-194** | (S)-2-(Benzofuran-5-carboxamido)-N1-(1-(2-(1-acetylamino-4-aminoadamantan)-2-oxoethyl)-2-oxo-1,2-dihydropyridin-3-yl)-N6-methyl-5-oxohexandiamid |
| **II-195** | (S)-2-(Benzofuran-6-carboxamido)-N1-(1-(2-(4-aminoadamantan-1-carboxamid)-2-oxoethyl)-2-oxo-1,2-dihydropyridin-3-yl)-N6-methyl-5-oxohexandiamid |
| **II-196** | (S)-N1-(1-(2-(4-Aminoadamantan-1-carboxamid)-2-oxoethyl)-2-oxo-1,2-dihydropyridin-3-yl)-N6-methyl-2-(3-(1-methylcyclopropyl)-1,2,4-oxadiazol-5-carboxamido)-5-oxohexandiamid |
| **II-197** | (S)-N1-(1-(2-(Bicyclo[1.1.1]pentan-1-ylamino)-2-oxoethyl)-2-oxo-1,2-dihydropyridin-3-yl)-N6-methyl-2-(5-methyl-1,2,4-oxadiazol-3-carboxamido)-5-oxohexandiamid |
| **II-198** | (S)-N1-(1-(2-(Bicyclo[1.1.1]pentan-1-ylamino)-2-oxoethyl)-2-oxo-1,2-dihydropyridin-3-yl)-N6-methyl-5-oxo-2-(1,2,3-thiadiazol-4-carboxamido)hexandiamid |
| **II-199** | (S)-N1-(1-(2-(Bicyclo[1.1.1]pentan-1-ylamino)-2-oxoethyl)-2-oxo-1,2-dihydropyridin-3-yl)-N6-methyl-5-oxo-2-(1,2,4-thiadiazol-5-carboxamido)hexanediamid |
| **II-200** | (S)-N1-(1-(2-(Bicyclo[1.1.1]pentan-1-ylamino)-2-oxoethyl)-2-oxo-1,2-dihydropyridin-3-yl)-N6-methyl-5-oxo-2-(1,3,4-thiadiazol-2-carboxamido)hexandiamid |
| **II-201** | (S)-N1-(1-(2-(Bicyclo[1.1.1]pentan-1-ylamino)-2-oxoethyl)-2-oxo-1,2-dihydropyridin-3-yl)-2-(4-cyclopropyl-1,2,3-thiadiazol-5-carboxamido)-N6-methyl-5-oxohexandiamid |
| **II-202** | (S)-2-(4-Cyclopropyl-1,2,3-thiadiazol-5-carboxamido)-N1-(1-(2-(2-adamantylamino)-2-oxoethyl)-2-oxo-1,2-dihydropyridin-3-yl)-N6-methyl-5-oxohexandiamid |
| **II-203** | (S)-2-(4-Isopropyl-1,2,3-thiadiazol-5-carboxamido)-N1-(1-(2-(2-adamantylamino)-2-oxoethyl)-2-oxo-1,2-dihydropyridin-3-yl)-N6-methyl-5-oxohexandiamid |
| **II-204** | (S)-2-(4-Ethyl-1,2,3-thiadiazol-5-carboxamido)-N1-(1-(2-(2-adamantylamino)-2-oxoethyl)-2-oxo-1,2-dihydropyridin-3-yl)-N6-methyl-5-oxohexandiamid |
| **II-205** | (S)-2-(4-Formyl-1,2,3-thiadiazol-5-carboxamido)-N1-(1-(2-(2-adamantylamino)-2-oxoethyl)-2-oxo-1,2-dihydropyridin-3-yl)-N6-methyl-5-oxohexandiamid |
| **II-206** | (S)-2-(4-(Hydroxymethyl)-1,2,3-thiadiazol-5-carboxamido)-N1-(1-(2-(2-adamantylamino)-2-oxoethyl)-2-oxo-1,2-dihydropyridin-3-yl)-N6-methyl-5-oxohexandiamid |
| **II-207** | (S)-N1-(1-(2-(1-Adamantylamino)-2-oxoethyl)-2-oxo-1,2-dihydropyridin-3-yl)-N6-methyl-2-(1-methyl-1H-imidazol-5-carboxamido)-5-oxohexandiamid |
| **II-208** | (S)-N1-(1-(2-(((1S,2R,5S)-6,6-Dimethylbicyclo[3.1.1]heptan-2-yl)methylamino)-2-oxoethyl)-2-oxo-1,2-dihydropyridin-3-yl)-N6-methyl-2-(1-methyl-1H-imidazol-2-carboxamido)-5-oxohexandiamid |
| **II-209** | (S)-N1-(1-(2-(((1R,2R,5R)-6,6-Dimethylbicyclo[3.1.1]heptan-2-yl)methylamino)-2-oxoethyl)-2-oxo-1,2-dihydropyridin-3-yl)-2-(1H-imidazol-4-carboxamido)-N6-methyl-5-oxohexandiamid |
| **II-210** | (S)-N1-(1-(2-(3,5-Dimethyladamantan-1-amino)-2-oxoethyl)-2-oxo-1,2-dihydropyridin-3-yl)-N6-methyl-2-(1-methyl-1H-imidazol-5-carboxamido)-5-oxohexandiamid |
| **II-211** | (S)-N1-(1-(2-(3,5,7-Trimethyl-1-adamantylamino)-2-oxoethyl)-2-oxo-1,2-dihydropyridin-3-yl)-N6-methyl-2-(1-methyl-1H-imidazol-5-carboxamido)-5-oxohexandiamid |
oder ein pharmazeutisch verträgliches Salz davon.

10. Eine pharmazeutische Zusammensetzung umfassend eine Verbindung nach einem der Ansprüche 1 - 9 als einen aktiven Inhaltstoff, zusammen mit mindestens einem pharmazeutisch verträglichen Träger, Hilfsstoff und/oder Verdünnungsmittel.

11. Verbindung gemäß einem der Ansprüche 1 - 9 zur Verwendung in der Medizin.

12. Verbindung gemäß einem der Ansprüche 1 - 9 oder pharmazeutische Zusammensetzung gemäß Anspruch 10 zur Verwendung bei der Behandlung oder in der Prophylaxe von Autoimmun- und Entzündungserkrankungen, Gefäßerkrankungen, fibrotischen Erkrankungen, Lebererkrankungen, cholestatischen Lebererkrankungen, Krebs, neurodegenerativen Erkrankungen, Augenerkrankungen und Hautstörungen.

13. Verbindung zur Verwendung oder die pharmazeutische Zusammensetzung zur Verwendung gemäß Anspruch 12, wobei die Autoimmun- und Entzündungserkrankungen Multiple Sklerose, Zöliakie, Duhring-Brocq-Krankheit (Dermatitis herpetiformis), Gluten-Ataxie, Gluten-Neuropathie, Diabetes, rheumatoide Arthritis, Graves-Krankheit, entzündliche Darmerkrankung, systemischer Lupus erythematodes, Psoriasis und Gingivitis umfassen; wobei die Gefäßerkrankungen Atherosklerose, Thrombose, Gefäßversteifung umfassen; die fibrotischen Erkrankungen die Lunge, die Niere, die Leber, die Haut oder den Darm betreffen wie zystische Fibrose, Nierenfibrose und diabetische Nephropathie, Darmfibrose, idiopathische Lungenfibrose, Leberfibrose; wobei die Lebererkrankungen alkoholische Hepatitis, alkoholische Steatohepatitis, nichtalkoholische Steatohepatitis, nichtalkoholische Fettlebererkrankung, Leberzirrhose, Autoimmunhepatitis oder Leberentzündung umfassen; wobei die cholestatischen Lebererkrankungen primär biliäre Cholangitis und primär sklerosierende Cholangitis umfassen; wobei Krebs Glioblastom, Melanom, Bauchspeicheldrüsenkrebs, Nierenzellkarzinom, Meningiom und Brustkrebs umfasst, woebi die neurodegenerativen Erkrankungen Parkinson-Krankheit, Huntington-Krankheit oder Alzheimer-Krankheit umfassen; wobei die Augenerkrankungen Glaukom, Katarakt, Makuladegeneration oder Uveitis umfassen; und wobei die Hautstörungen Akne, Psoriasis, Narbenbildung und Hautalterung umfassen.

14. Verbindung zur Verwendung oder die pharmazeutische Zusammensetzung zur Verwendung gemäß einem der Ansprüche 12 und 13 bei der Behandlung oder in der Prophylaxe von Zöliakie.

15. Ein Verfahren zum Herstellen der Verbindung der Formel (**Ib**) gemäß Anspruch 1 oder 4 umfassend:
**Schritt 1B:** Bereitstellen einer Verbindung **4b** 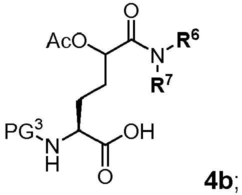
**Schritt 2B:** Durchführen einer Kupplungsreaktion der Verbindung **4b** mit einer Verbindung **5** 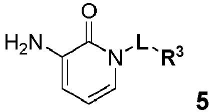 um eine Verbindung **6b** zu erhalten 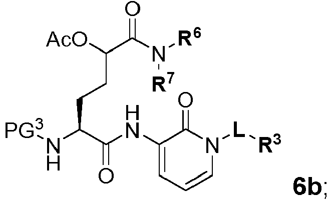
**Schritt 3B:** Entschützen einer Aminoschutzgruppe PG³, um eine Verbindung **7b** zu erhalten 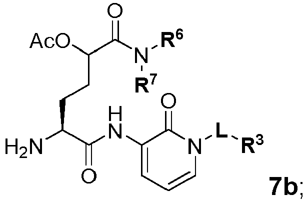
**Schritt** 4B: Durchführen einer Kupplungsreaktion der Verbindung **7b** mit einer Carbonsäure (**R²**-CO₂H **8**), um eine Verbindung **9b** zu erhalten 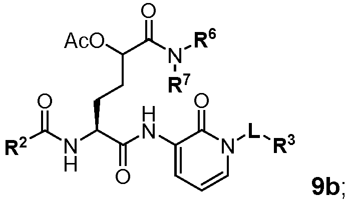
**Schritt 5B:** Durchführen einer Oxidationsreaktion der Verbindung **9b** , um die Verbindung der Formel (**Ib**) herzustellen 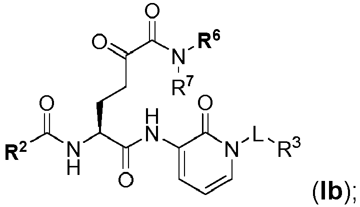
wobei **L, R²**, **R³**, **R⁶** und **R⁷** die in Anspruch 1 definierten Bedeutungen haben, und **PG³** eine Aminoschutzgruppe ist.

## Revendications

1. Un composé de la formule générale (**I**) : 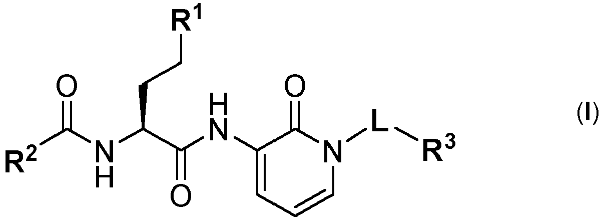 où
**L** représente -**L¹-** ou **-L¹-L²-** ; de préférence **-L¹-L²-** ;
**L¹** représente -CH₂-, -CH₂CH₂-, -CH₂CH₂CH₂-, -CH₂CO-, ou -CH₂CH₂CO- ;
**L²** représente une liaison **-NR^{N1}-,** -**NR^{N1}**CH₂-, -**NR^{N1}**CH₂CH₂-, ou -NR^{N1}CH(CH₃)- ;
**R¹** représente 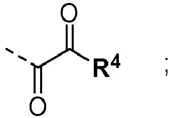
**R²** représente 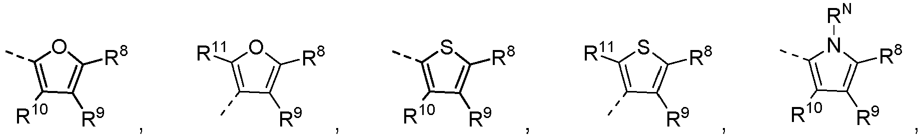 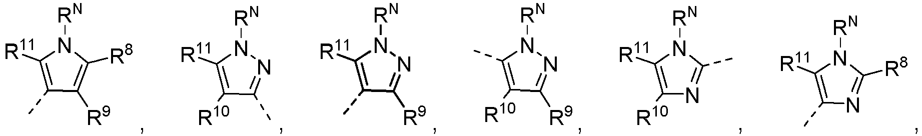 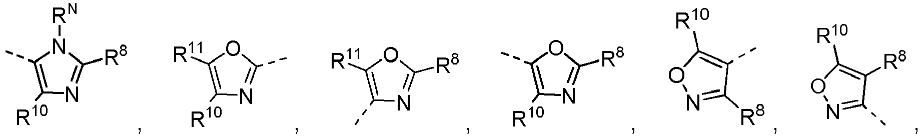 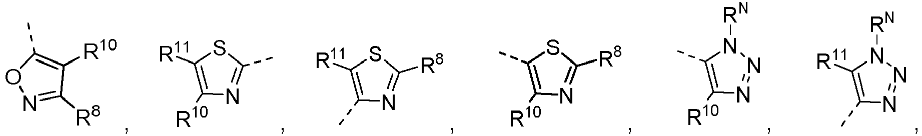 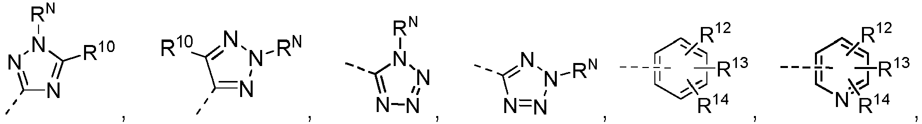 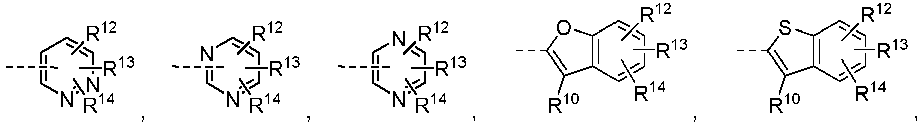 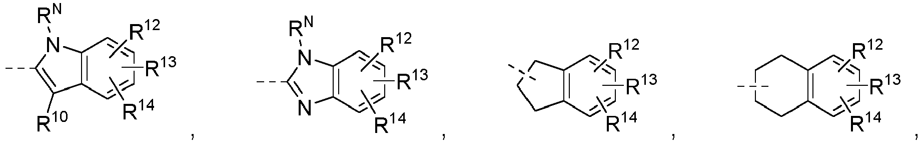 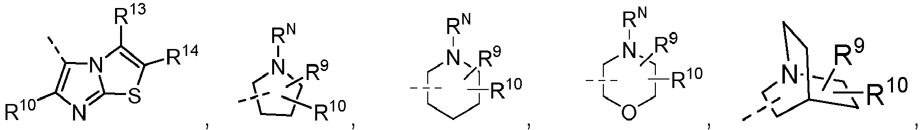 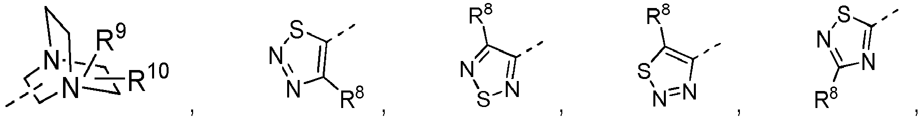 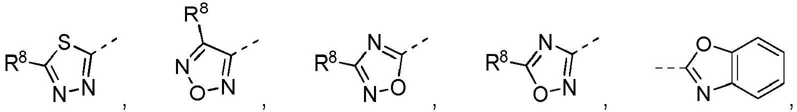 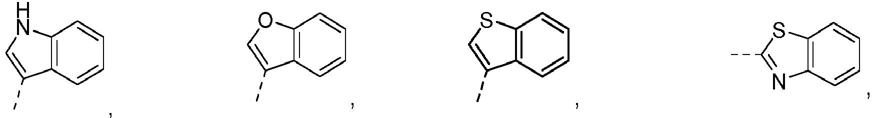 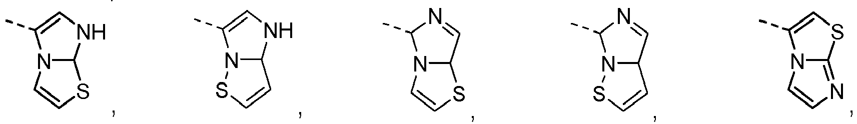 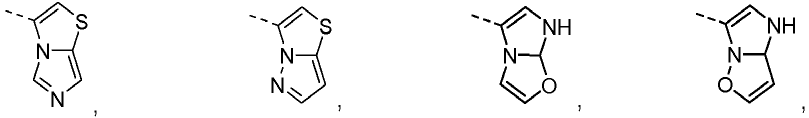 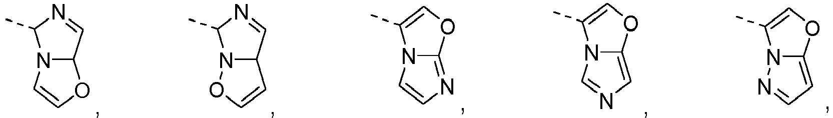 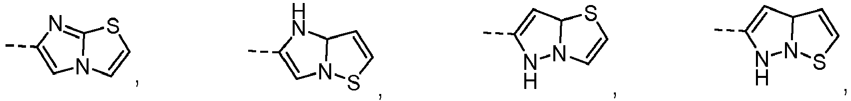 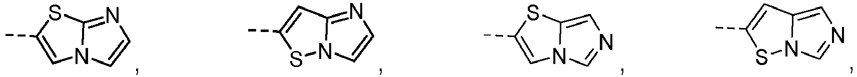 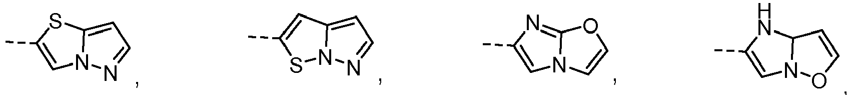 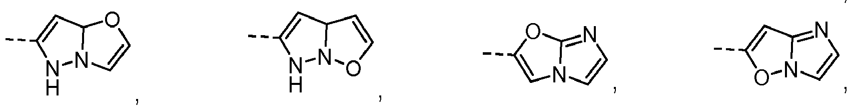 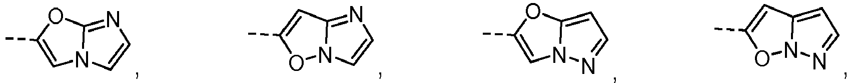 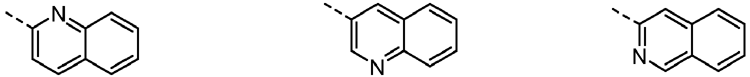 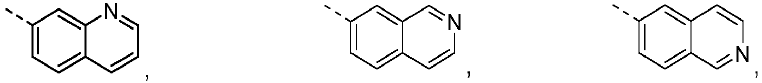 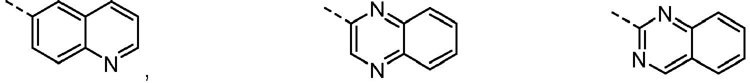 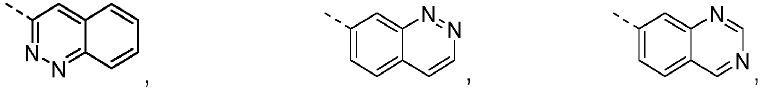 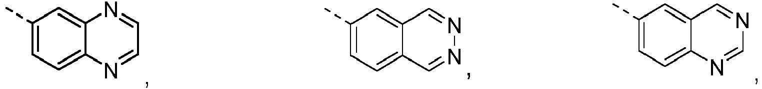 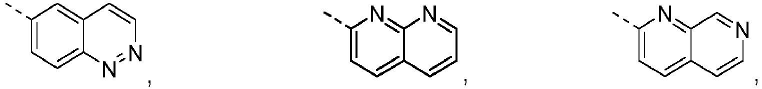 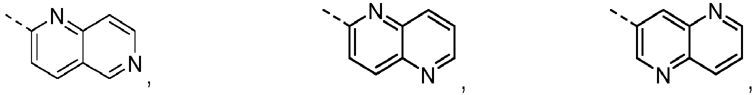 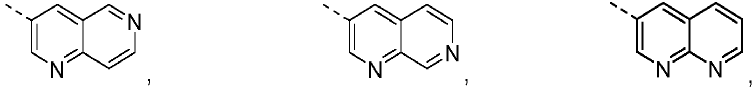 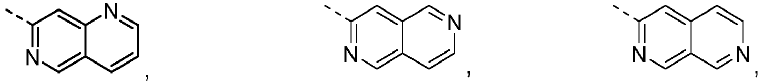 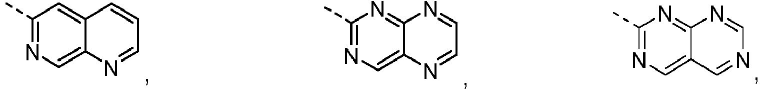 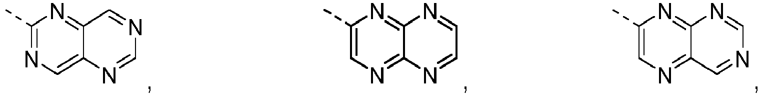 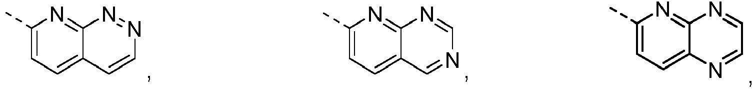 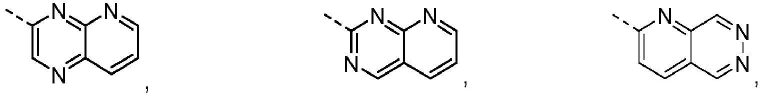 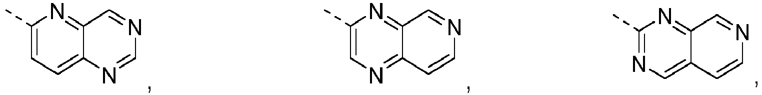 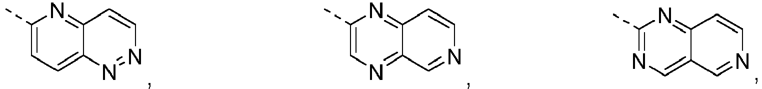 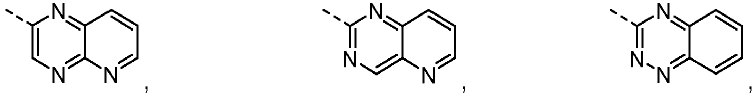 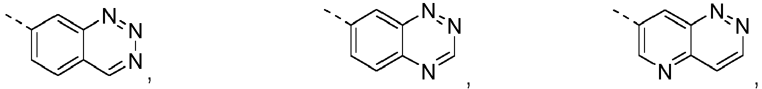 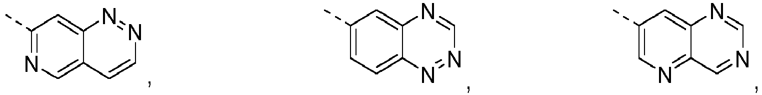 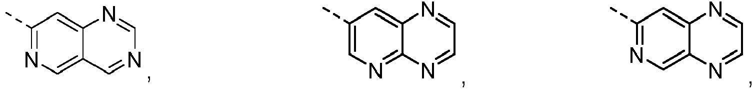 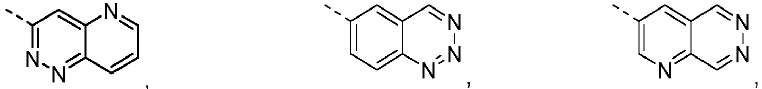 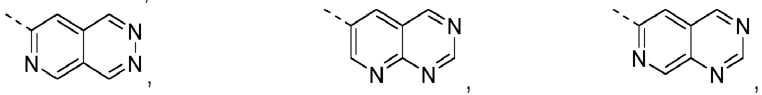 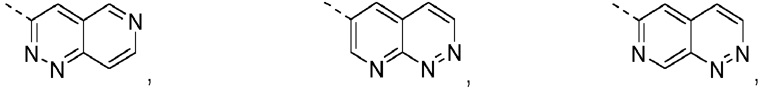 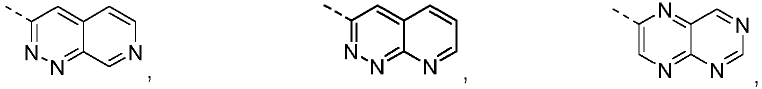 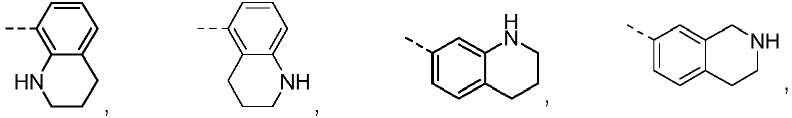 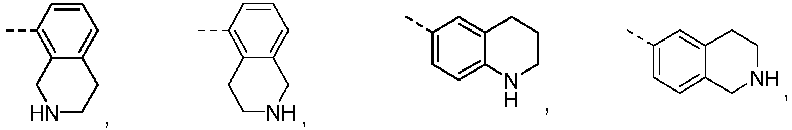 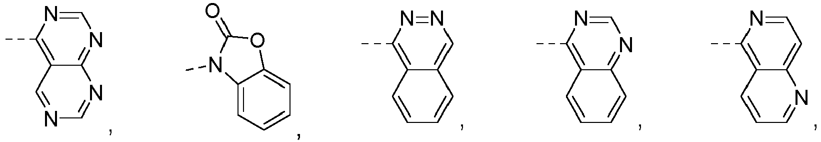 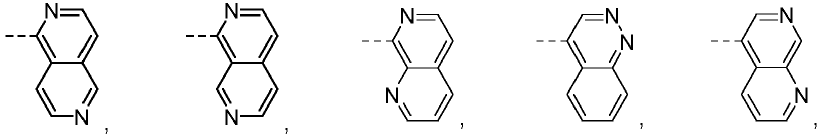 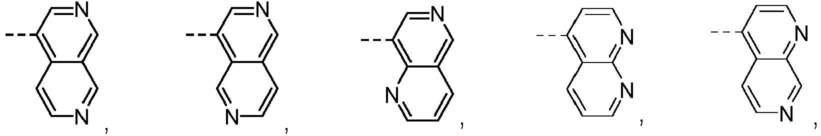 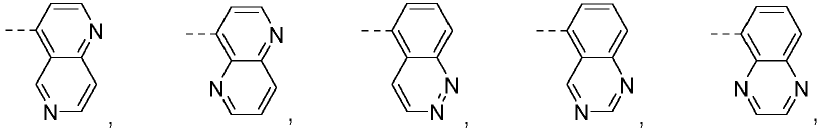 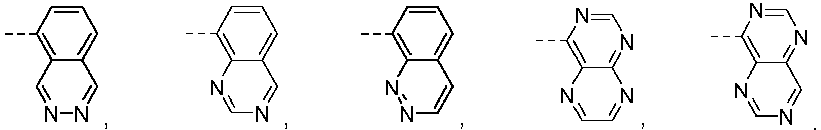 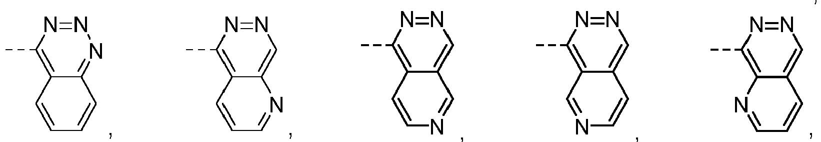 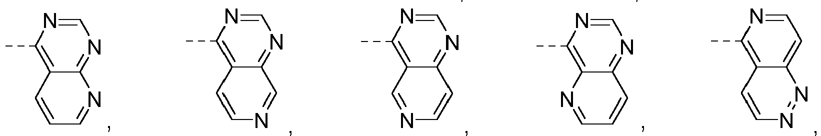 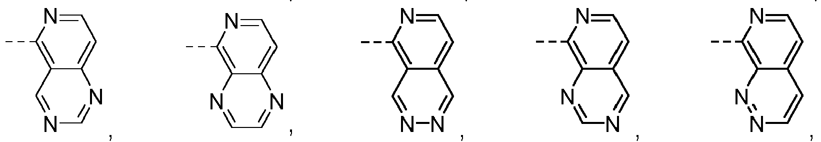 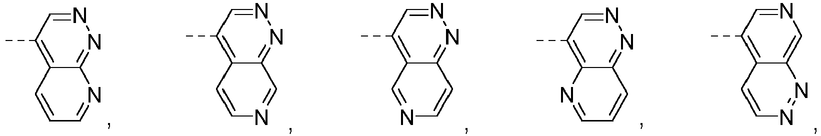 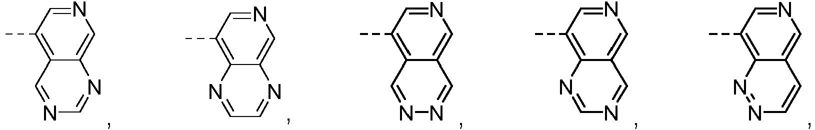 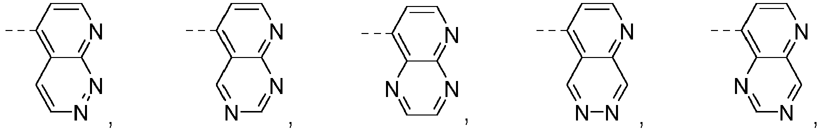 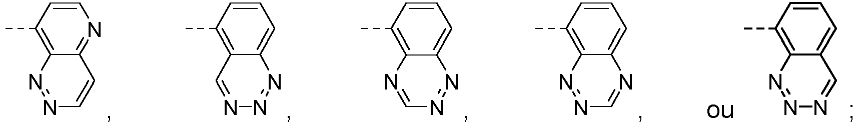
où les résidus bicycliques non substitués peuvent être substitués par 1 à 5 des substituants **R⁹** - **R¹⁴** et **R^{N}** ; de préférence par 1 à 3 des substituants **R¹¹** - **R¹³** ;
**R³** représente un bicyclo[1.1.1]pentyle, un bicyclo[2.1.1]hexyle, un bicyclo[2.2.1]heptyle, un bicyclo[3.1.1]heptyle, un bicyclo[2.2.2]octyle, un bicyclo[3.2.1]octyle, un bicyclo[3.2.2]nonyle, un bicyclo[3.3.2]décyle, un bicyclo[3.3.3]undécyl, un 4-homoisotwistyle, un adamantyle, un diamantyle, un hexaméthylène-tétraminyle et les résidus susmentionnés contiennent éventuellement une ou plusieurs double(s) liaison(s) C=C et/ou sont éventuellement substitués par un ou plusieurs des éléments **R^{a}, R^{b}, R^{c}, R^{d},** et **R^{e}** ;
**R^{a}, R^{b}, R^{c}, R^{d},** et **R^{e}** représentent indépendamment les uns des autres -H, -F,-Cl, -Br,-CN, -OH, -CH₃, -CH₂CH₃, -CH₂CH₂CH₃, -CH(CH₃)₂, -CHF₂, -CF₃, -CH₂CF₃, -COCH₃, -COCH₂CH₃, -CO₂H, -CO₂CH₃, -CO₂C₂H₅, -CONH₂, -CONHCH₃, -CON(CH₃)₂, -CONHC₂H₅, -CH₂CO₂H, -CH₂CO₂CH₃, -CH₂CO₂C₂H₅, -CH₂CONH₂, -CH₂CONHCH₃, -CH₂CON(CH₃)₂, -CH₂CONHC₂H₅, -NHCOCH₃, -NHCOC₂H₅, -NHCOCF₃, -NHCOCH₂CF₃, -NHSO₂CH₃, -NHSO₂C₂H₅, -NHSO₂CHF₂, -NHSO₂CF₃, ou-NHSO₂CH₂CF₃ ;
**R⁴** représente **-NR⁶R⁷** ;
**R⁶** et **R⁷** représentent indépendamment l'un de l'autre -H, -CH₃, -CH₂CH₂CH₃, -CH(CH₃)₂, -CH₂CH₂CH₂CH₃, -CH₂CH₂CH₂CH₂CH₃, -CH₂CH(CH₃)₂, -C(CH₃)₃, -CH₂CH=CH₂, -CH₂CH=CH(CH₃), -CH₂CH=C(CH₃)₂, -CH₂CH=CHCH₂CH₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -Ph, -CH₂-Ph, -CH₂OCH₃, -CH₂OCH₂CH₃,-CH₂CH₂OCH₃, -CH₂CH₂OCH₂CH₃, -CH₂CH₂NHCH₃, ou -CH₂CH₂N(CH₃)₂,
ou **-NR⁶R⁷** est - N(C₂H₅)₂, 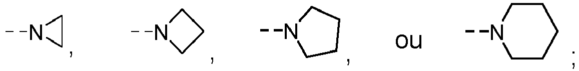
**R⁸, R⁹, R¹⁰, R¹¹, R¹², R¹³,** et **R¹⁴** représentent indépendamment les uns des autres -H, -F, -Cl, -Br, -I, -OH, -CN, -NO₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -CH₂-cyclo-C₃H₅, -CH₂OH, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -OCH₃, -OC₂H₅, -OC₃H₇, -OCH(CH₃)₂, -OC(CH₃)₃, -OC₄H₉, -OCHF₂, -OCF₃, -OCH₂CF₃, -OC₂F₅, -OCH₂OCH₃, -O-cyclo-C₃H₅, -OCH₂-cyclo-C₃H₅, -O-C₂H₄-cyclo-C₃H₅, -CHO, -COCH₃, -COCF₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, -COOC(CH₃)₃, -OOC-CH₃, -OOC-CF₃, -OOC-C₂H₅, -OOC-C₃H₇, -OOC-CH(CH₃)₂, -OOC-C(CH₃)₃, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, - N[C(CH₃)₃]₂,
-NHCOCH₃, -NHCOCF₃, -NHCOC₂H₅, -NHCOC₃H₇, -NHCOCH(CH₃)₂, -NHCOC(CH₃)₃, -CONH₂, -CONHCH₃, -CONHC₂H₅, -CONHC₃H₇, -CONHCH(CH₃)₂, -CONH-cyclo-C₃H₅, -CONHC(CH₃)₃, -CON(CH₃)₂, -CON(C₂H₅)₂, -CON(C₃H₇)₂, -CON[CH(CH₃)₂]₂, -CON[C(CH₃)₃]₂, -SO₂NH₂, -SO₂NHCH₃, -SO₂NHC₂H₅, -SO₂NHC₃H₇, -SO₂NHCH(CH₃)₂, -SO₂NH-cyclo-C₃H₅, -SO₂NHC(CH₃)₃, -SO₂N(CH₃)₂, -SO₂N(C₂H₅)₂, -SO₂N(C₃H₇)₂, -SO₂N[CH(CH₃)₂]₂, -SO₂N[C(CH₃)₃]₂, -NHSO₂CH₃, -NHSO₂CF₃, -NHSO₂C₂H₅, -NHSO₂C₃H₇, -NHSO₂CH(CH₃)₂, -NHSO₂C(CH₃)₃, -CH=CH₂, -CH₂-CH=CH₂, -C(CH₃)=CH₂, -CH=CH-CH₃, -C≡CH, -C≡C-CH₃, -CH₂-C≡CH, -Ph, -O-Ph, -O-CH₂-Ph, 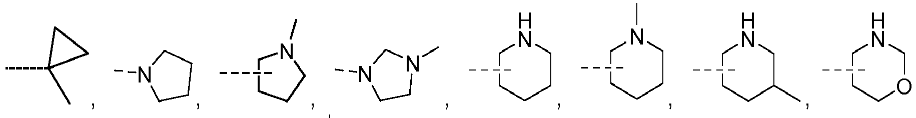 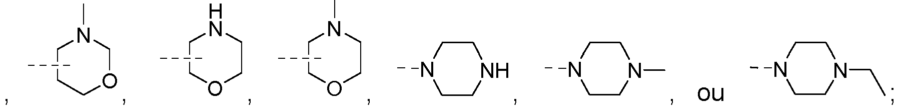
ou **R⁸** et **R⁹** ou **R⁹** et **R¹⁰** peuvent former ensemble l'un des cycles à cinq ou six chaînons suivants : 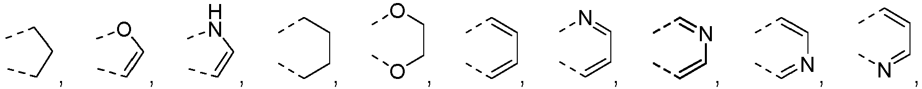 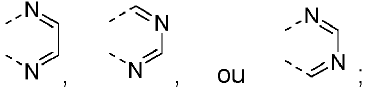
ou **R¹²** et **R¹³** ou **R¹³** et **R¹⁴** peuvent former ensemble l'un des cycles à cinq ou six chaînons suivants : 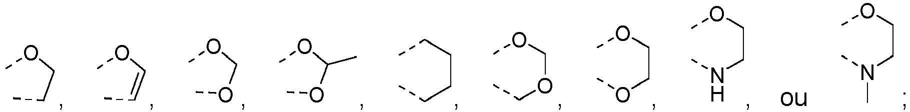
**R^{N}** représente -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -cyclo-C₄H₇, -cyclo-C₅H₉, -CH₂-cyclo-C₃H₅, -CH₂-cyclo-C₄H₇, -CH₂-cyclo-C₅H₉, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -CH₂-CH=CH₂, -CH₂-C≡CH, -CHO, -COCH₃, -COC₂H₅, -COC₃H₇, -COCH(CH₃)₂, -COC(CH₃)₃, -CO-cyclo-C₃H₅, -CO-cyclo-C₄H₇, -CO-cyclo-C₅H₉, -COOCH₃, -COOC₂H₅, -COOC₃H₇, -COOCH(CH₃)₂, -COOC(CH₃)₃, -COOCH₂Ph, -SO₂CH₃, -SO₂CF₃, -SO₂C₂H₅, -SO₂C₃H₇, -SO₂CH(CH₃)₂, -SO₂-cyclo-C₃H₅, ou -SO₂C(CH₃)₃ ;
**R^{N1}** représente -H, -CH₃, ou -CH₂CH₃ ;
ou un diastéréomère, un énantiomère, un mélange de diastéréomères, un mélange d'énantiomères, un racémate, un solvate, un hydrate ou un sel pharmaceutiquement acceptable de celui-ci.

2. Le composé conformément à la revendication 1, où **R²** représente 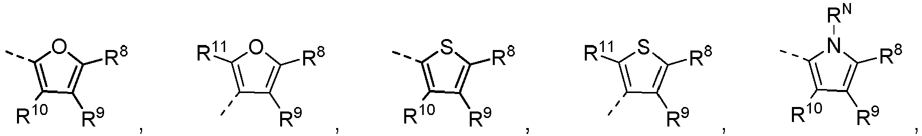 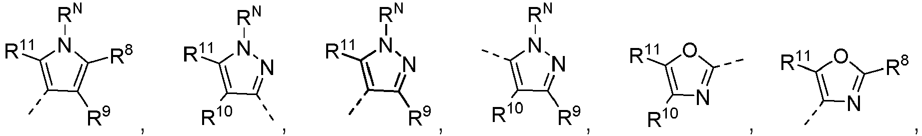 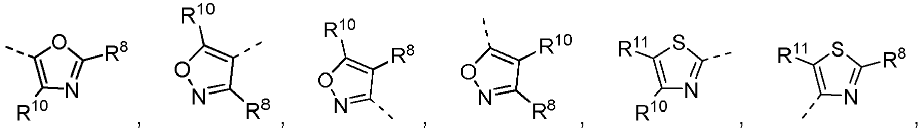 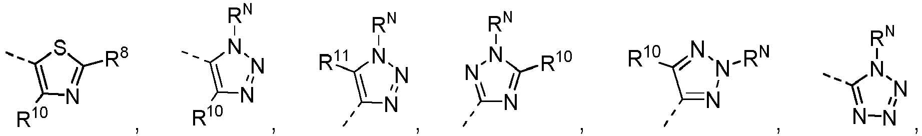 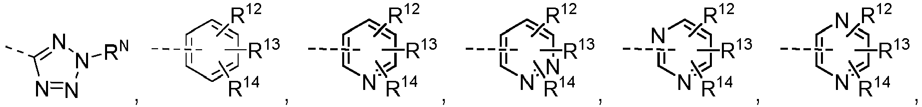 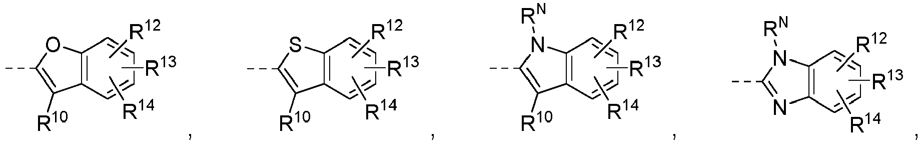 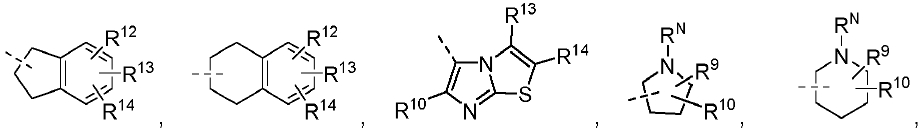 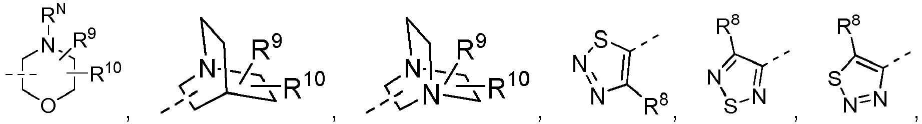 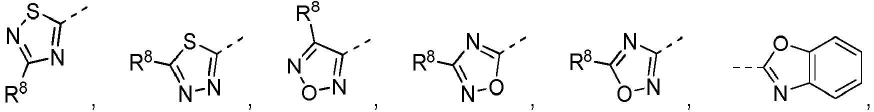 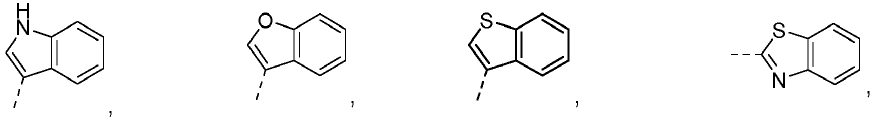 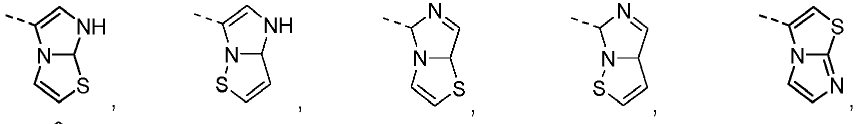 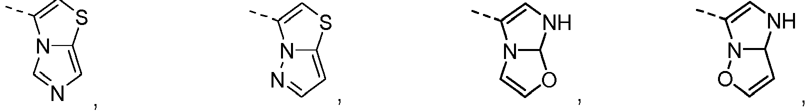 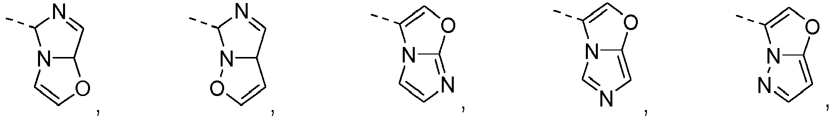 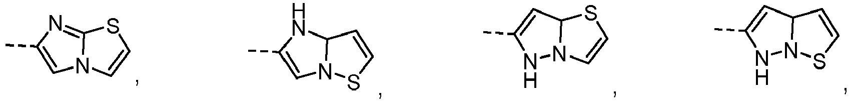 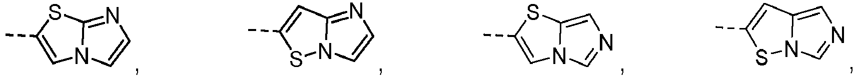 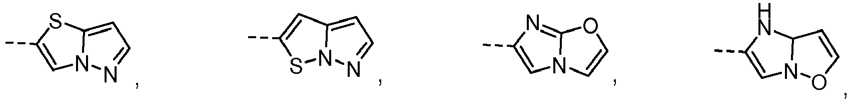 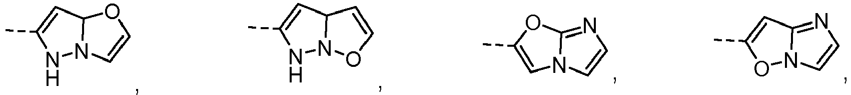 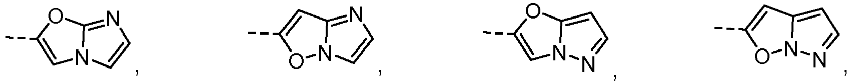 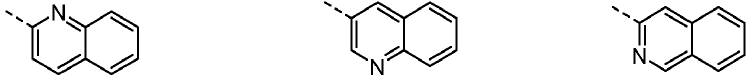 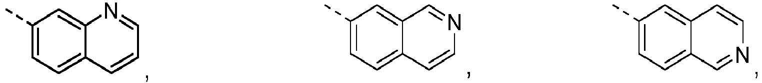 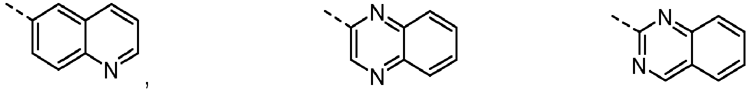 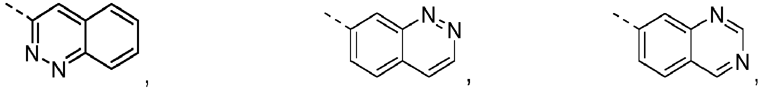 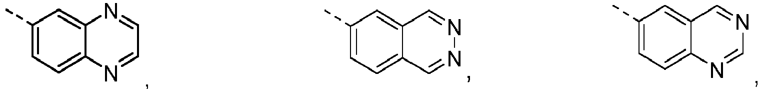 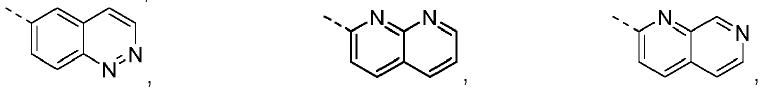 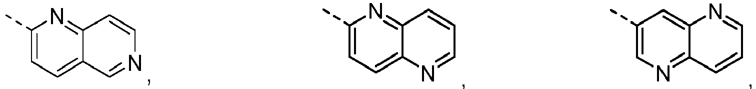 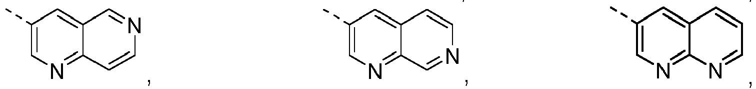 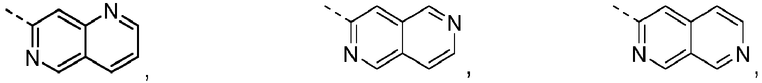 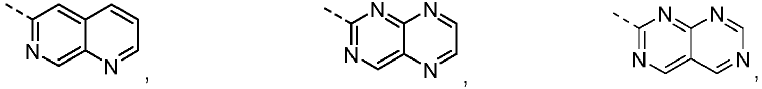 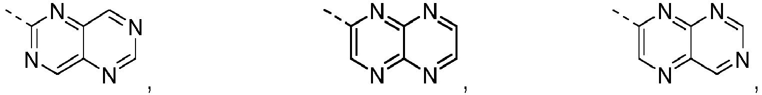 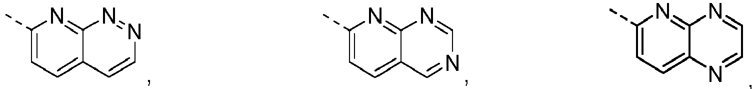 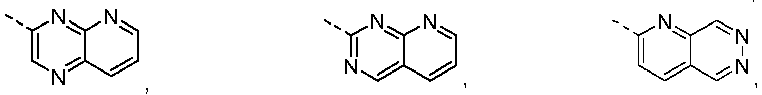 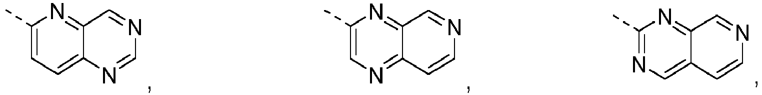 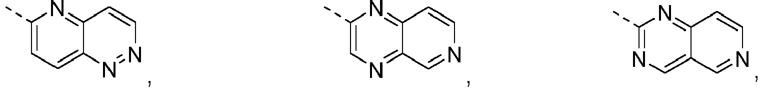 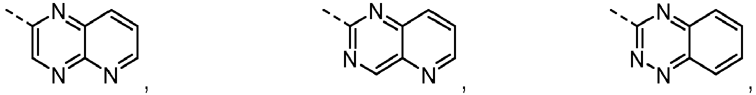 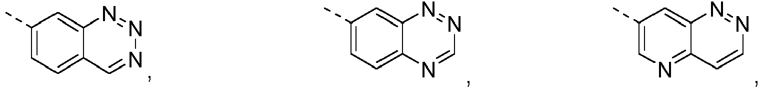 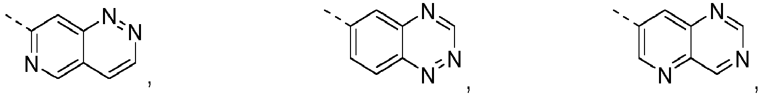 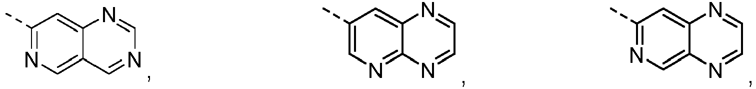 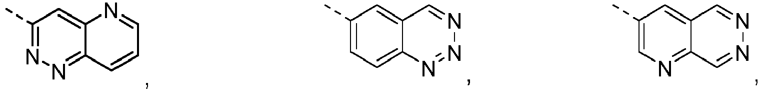 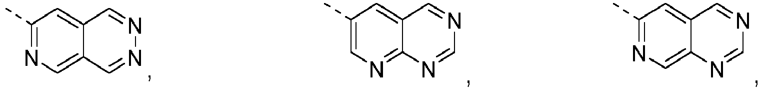 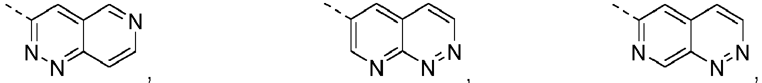 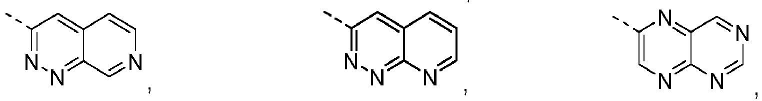 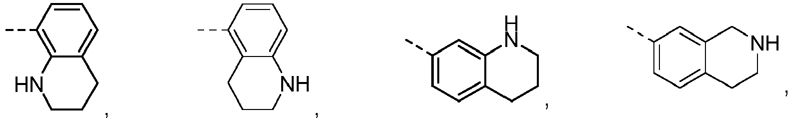 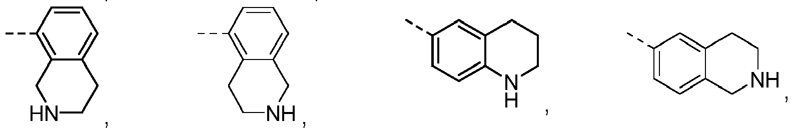 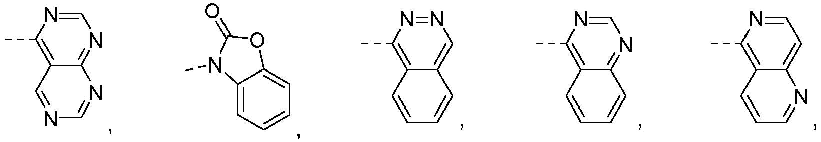 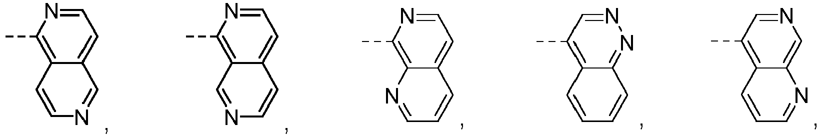 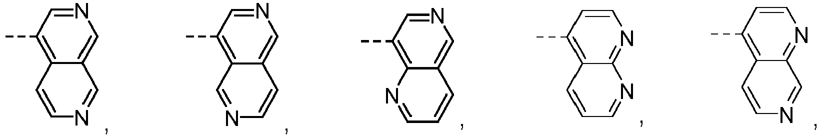 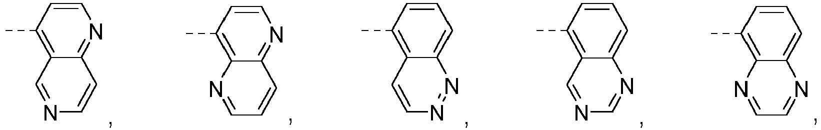 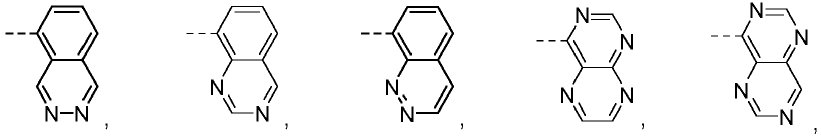 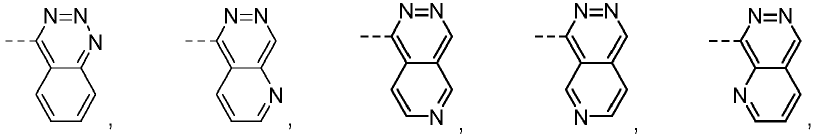 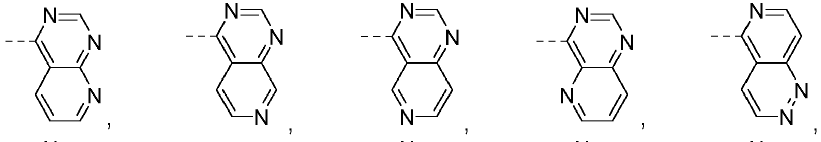 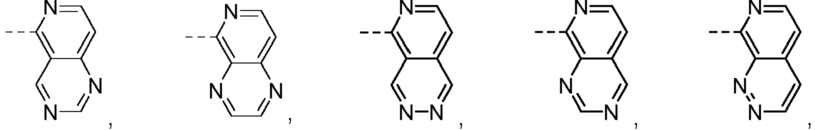 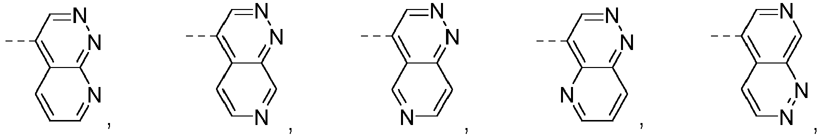 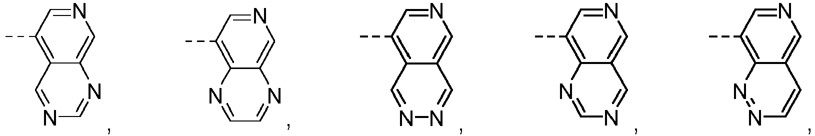 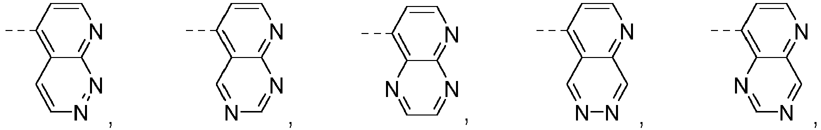 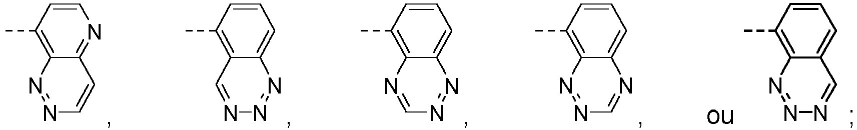 où les résidus bicycliques non substitués peuvent être substitués par 1 à 5 des substituants **R⁹** - **R¹⁴** et **R^{N}** ; de préférence par 1 à 3 des substituants **R¹¹** - **R¹³** et les substituants **R⁹** - **R¹⁴** et **R^{N}** ont la signification définie dans la revendication 1.

3. Le composé conformément à la revendication 1, où **R²** représente 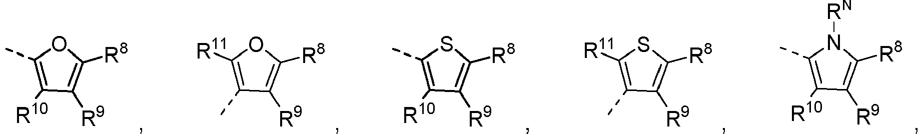 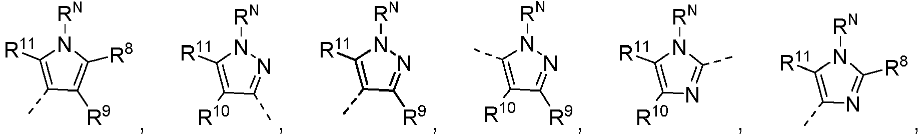 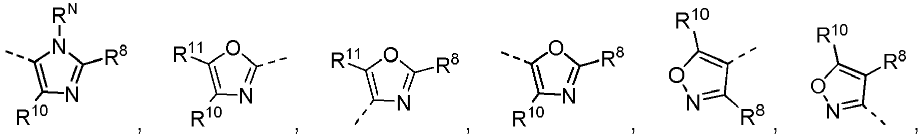 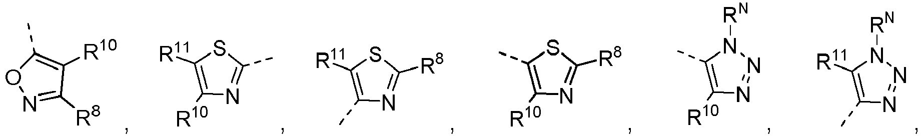 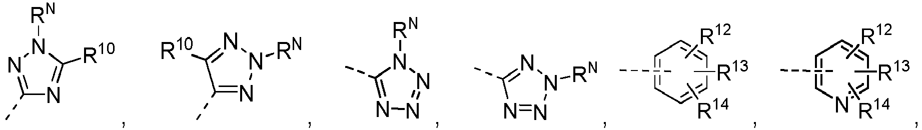 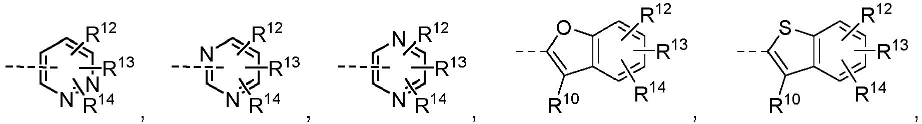 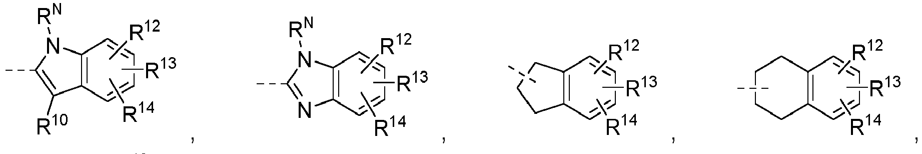 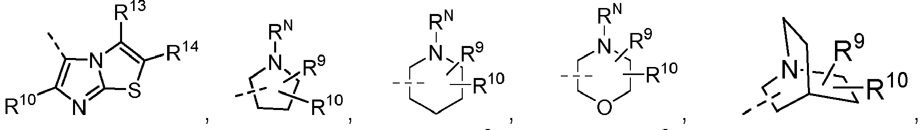 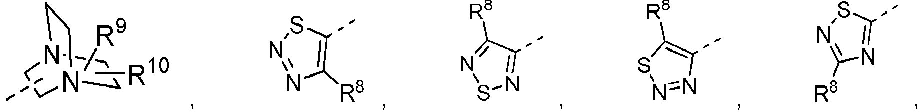 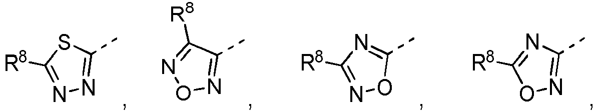 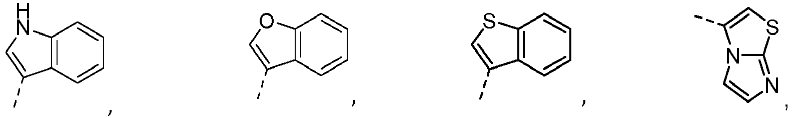 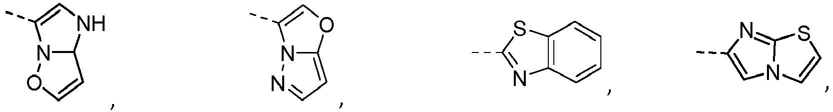 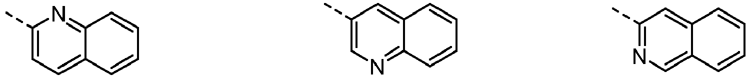 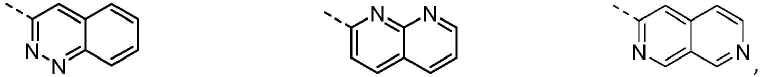 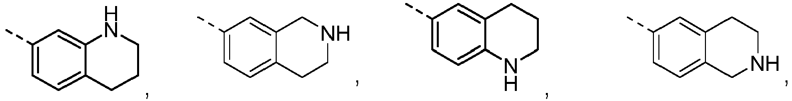 ou 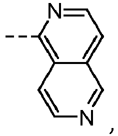 où les résidues bicycliques non substitués peuvent être substitués par 1 à 5 des substituants **R⁹** - **R¹⁴** et **R^{N}** ; de préférence par 1 à 3 des substituants **R¹¹** - **R¹³** et les substituants **R⁹** - **R¹⁴** et **R^{N}** ont la signification définie dans la revendication 1.

4. Le composé conforme à la revendication 1, où ledit composé a la formule (**Ib**) 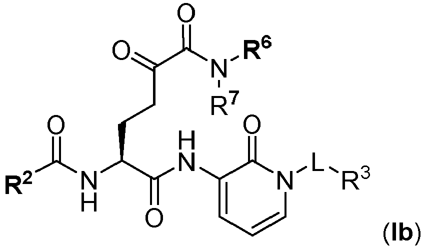 où
**L** représente -**L¹**- ou -**L¹**-**L²**- ; de préférence -**L¹**-**L²**- ;
**L¹** représente -CH₂-, -CH₂CH₂-, -CH₂CH₂CH₂-, -CH₂CO-, ou -CH₂CH₂CO- ;
**L²** représente une liaison -NR^{N1}-, -NR^{N1}CH₂-, -NR^{N1}CH₂CH₂-, ou -NR^{N1}CH(CH₃)- ;
**R²** représente 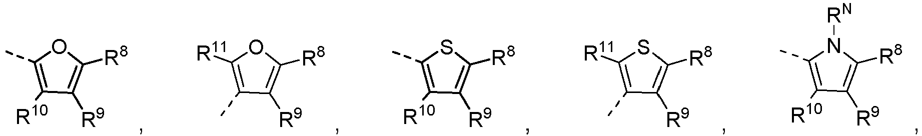 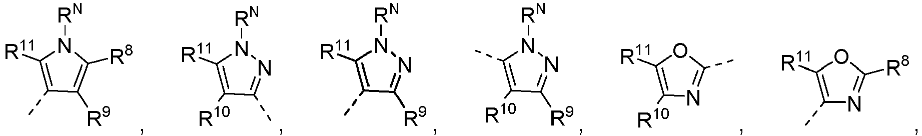 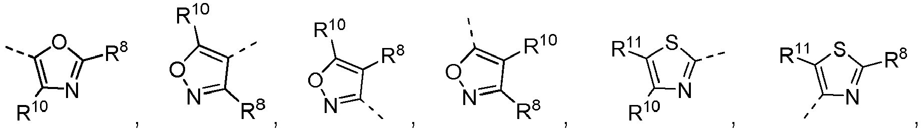 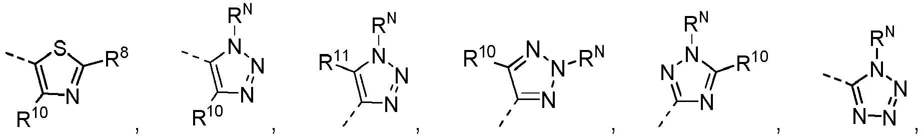 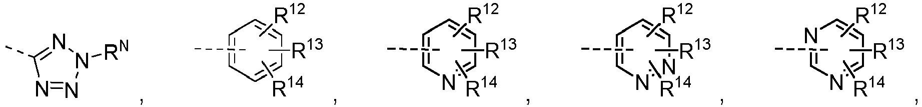 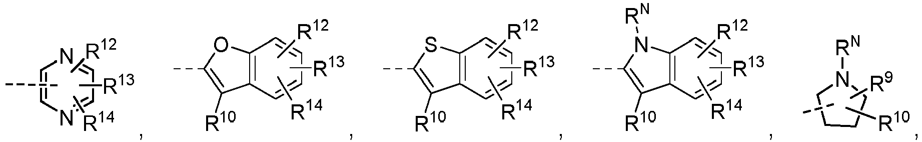 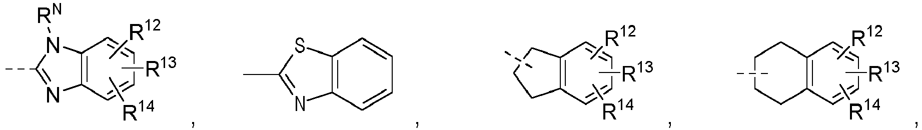 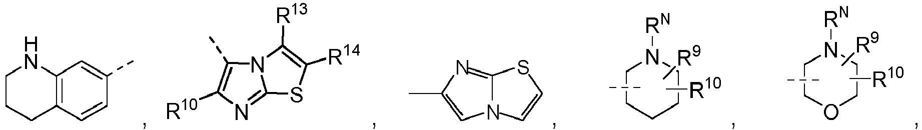 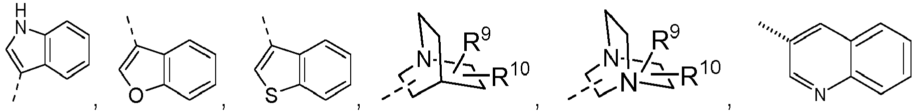 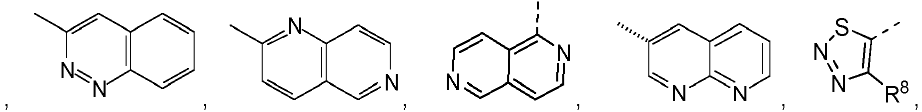 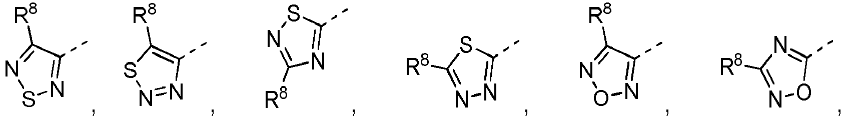 ou 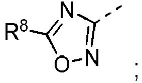
**R³** représente un bicyclo[1.1.1]pentyle, un bicyclo[2.1.1]hexyle, un bicyclo[2.2.1]heptyle, un bicyclo[3.1.1]heptyle, un bicyclo[2.2.2]octyle, un bicyclo[3.2.1]octyle, un bicyclo[3.2.2]nonyle, un bicyclo[3.3.2]décyle, un bicyclo[3.3.3]undécyl, un 4-homoisotwistyle, un adamantyle, un diamantyle, un hexaméthylène-tétraminyle et les résidus susmentionnés contiennent éventuellement une ou plusieurs double(s) liaison(s) C=C et/ou sont substitués par un ou plusieurs des éléments **R^{a}**, **R^{b}**, **R^{c}**, **R^{d}**, et **R^{e}** ;
**R^{a}**, **R^{b}**, **R^{c}**, **R^{d}**, et **R^{e}** représentent indépendamment les uns des autres -H, -F, -Cl, -Br, -CN, -OH, -CH₃, -CH₂CH₃, -CH₂CH₂CH₃, -CH(CH₃)₂, -CHF₂, -CF₃, -CH₂CF₃, -COCH₃, -COCH₂CH₃, -CO₂H, -CO₂CH₃, -CO₂C₂H₅, -CONH₂, -CONHCH₃, -CON(CH₃)₂, -CONHC₂H₅, -CH₂CO₂H, -CH₂CO₂CH₃, -CH₂CO₂C₂H₅, -CH₂CONH₂, -CH₂CONHCH₃, -CH₂CON(CH₃)₂, -CH₂CONHC₂H₅, -NHCOCH₃, -NHCOC₂H₅, -NHCOCF₃, -NHCOCH₂CF₃, -NHSO₂CH₃, -NHSO₂C₂H₅, -NHSO₂CHF₂, -NHSO₂CF₃, ou -NHSO₂CH₂CF₃ ;
**R⁴** représente -N**R⁶R⁷** ;
**R⁶** et **R⁷** représentent indépendamment les uns des autres -H, -CH₃, -CH₂CH₂CH₃, -CH(CH₃)₂, -CH₂CH₂CH₂CH₃, -CH₂CH₂CH₂CH₂CH₃, -CH₂CH(CH₃)₂, -C(CH₃)₃, -CH₂CH=CH₂, -CH₂CH=CH(CH₃), -CH₂CH=C(CH₃)₂, -CH₂CH=CHCH₂CH₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -Ph, -CH₂-Ph, -CH₂OCH₃, -CH₂OCH₂CH₃, -CH₂CH₂OCH₃, -CH₂CH₂OCH₂CH₃, -CH₂CH₂NHCH₃, -CH₂CH₂N(CH₃)₂,
ou -N**R⁶R⁷** est - N(C₂H₅)₂, 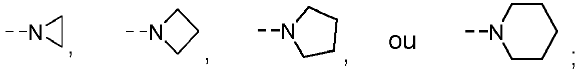
**R^{N}** représente -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -cyclo-C₄H₇, -cyclo-C₅H₉, -CH₂-cyclo-C₃H₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -CH₂-CH=CH₂, -CH₂-C≡CH, -CHO, -COCH₃, -COC₂H₅, -COC₃H₇, -COCH(CH₃)₂, -COC(CH₃)₃, -COOCH₃, -COOC₂H₅, - COOC₃H₇,
-COOCH(CH₃)₂, -COOC(CH₃)₃, -COOCH₂Ph, -SO₂CH₃, -SO₂CF₃, -SO₂C₂H₅, -SO₂C₃H₇, -SO₂CH(CH₃)₂, ou -SO₂C(CH₃)₃ ;
**R^{N1}** représente -H, -CH₃, ou -CH₂CH₃ ;
et **R⁸** - **R¹⁴** ont les significations définies dans la formule (**I**) ;
ou un diastéréomère, un énantiomère, un mélange de diastéréomères, un mélange d'énantiomères, un racémate, un solvate, un hydrate ou un sel pharmaceutiquement acceptable de celui-ci.

5. Le composé conforme à l'une des revendications 1 à 4, où
**L¹** représente -CH₂-, ou -CH₂CO- ;
**L²** représente une liaison -N**R^{N1}**-, -N**R^{N1}**CH₂- ou -NR^{N1}CH(CH₃)- ;
**R³** représente un bicyclo[1.1.1]pentyle, un bicyclo[2.1.1]hexyle, un bicyclo[2.2.1]heptyle, un bicyclo[3.1.1]heptyle, un bicyclo[2.2.2]octyle, un 4-homoisotwistyle, un adamantyle ou un diamantyle et les résidus susmentionnés contiennent éventuellement une ou plusieurs double(s) liaison(s) C=C et/ou sont substitués par un ou plusieurs des éléments **R^{a}**, **R^{b}**, **R^{c}**, **R^{d}** et **R^{e}** ;
et **R^{a}**, **R^{b}**, **R^{c}**, **R^{d}**, **R^{e}** ainsi que **R^{N1}** ont les mêmes significations que celles définies dans la revendication 1.

6. Le composé conforme à l'une des revendications 1 à 5, où
**R²** représente 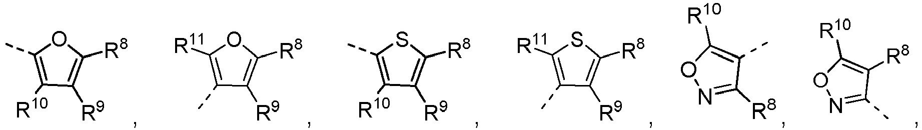 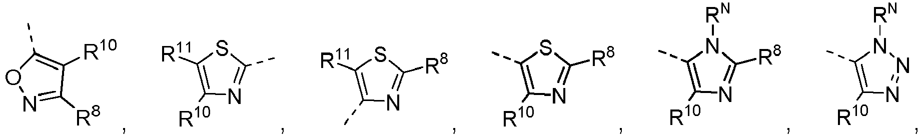 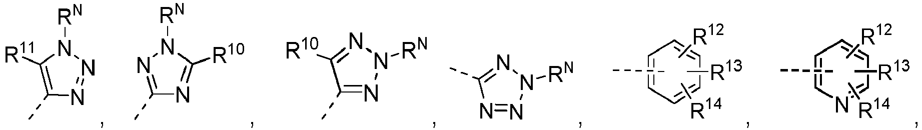 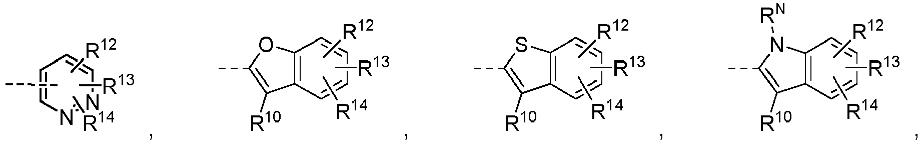 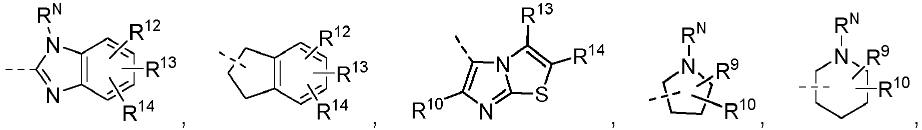 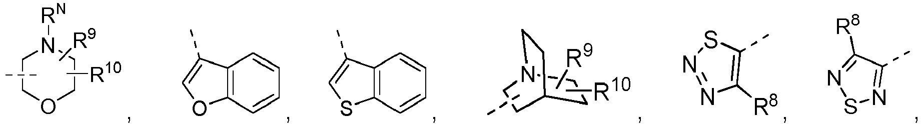 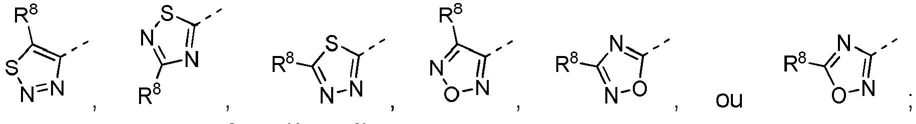 et les substituants **R⁸** - **R¹⁴** et **R^{N}** ont les significations définies dans la revendication 1 ou 2.

7. Le composé conforme à l'une des revendications 1 à 6, où ledit composé a une des formules (**IV-a**) - (**IV-o**) et (**V-a**) - (**V-d**) : 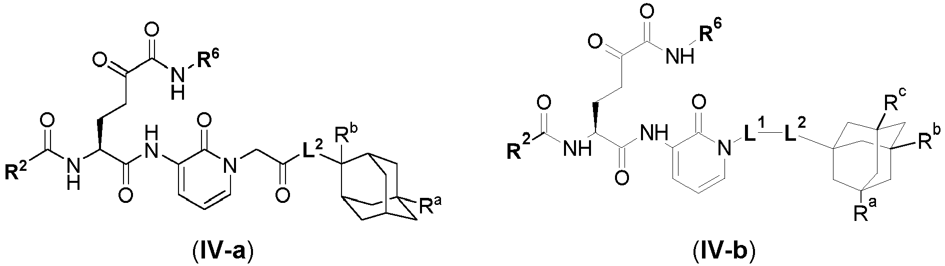 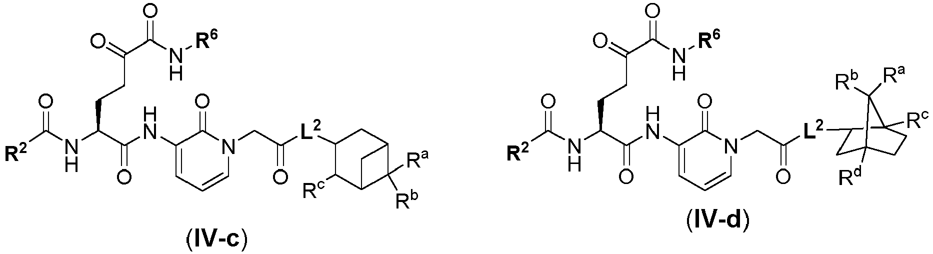 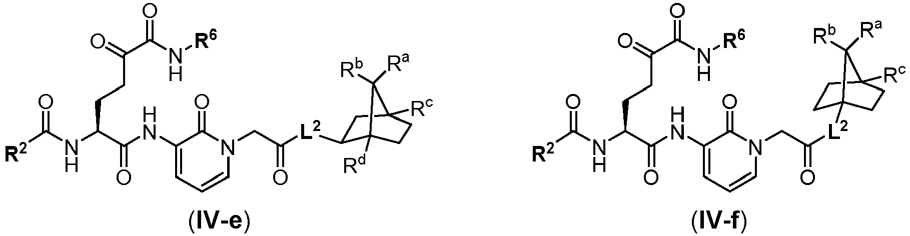 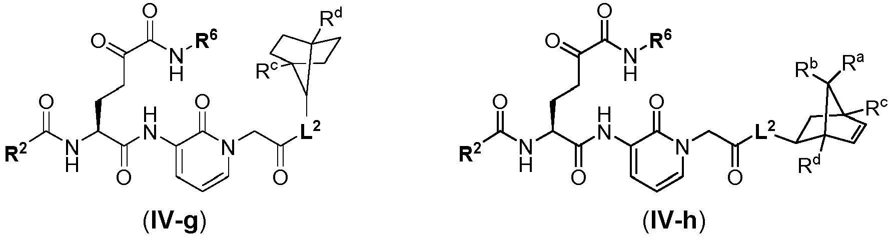 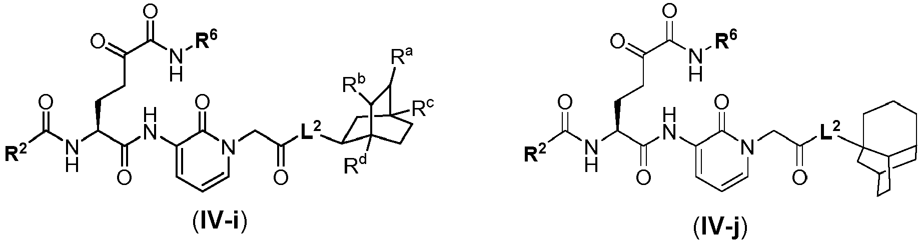 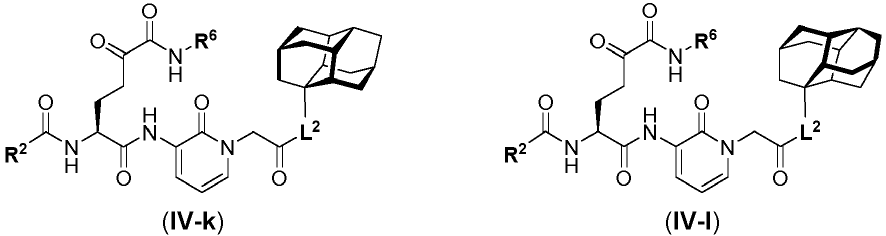 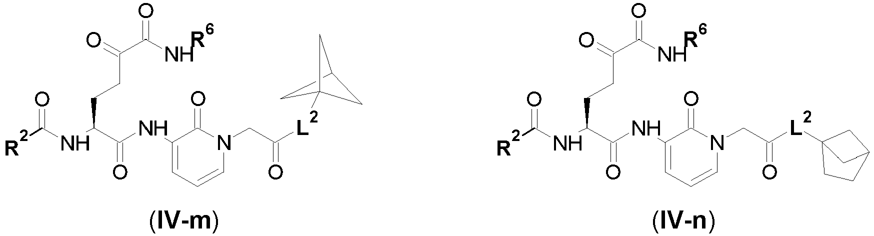 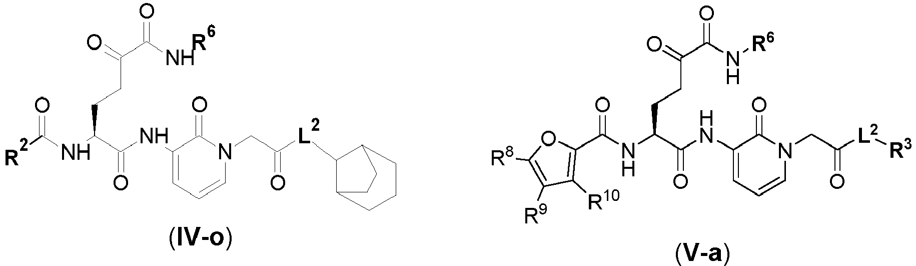 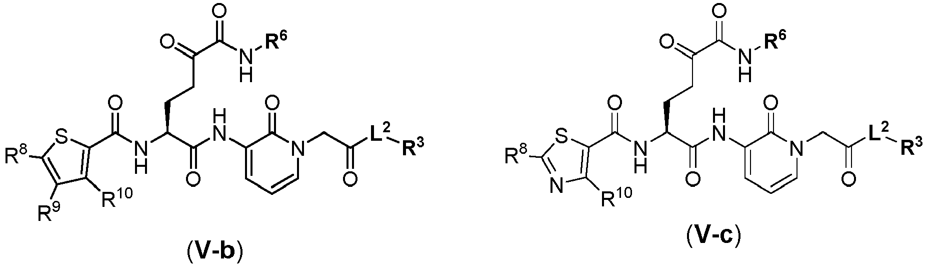 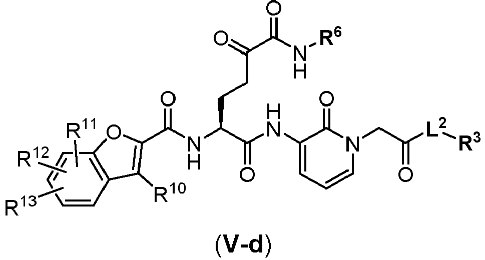 et **R²**, **R³**, **R⁶**, **R⁸**, **R⁹**, **R¹⁰**, **R¹¹**, **R¹²**, **R¹³**, **R^{a}**, **R^{b}**, **R^{c}**, **R^{d}** ainsi que **L²** ont les mêmes significations que celles définies à la revendication 1.

8. Le composé conforme à l'une des revendications 1 à 7, où
**R²** représente 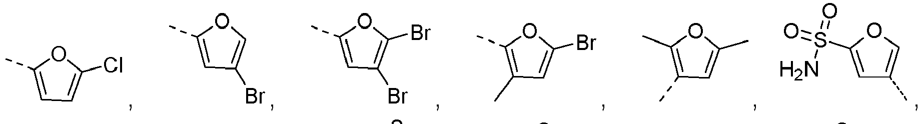 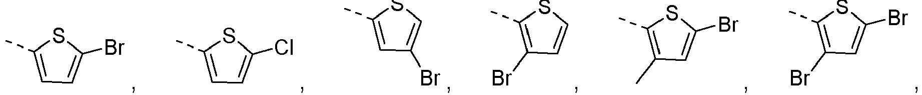 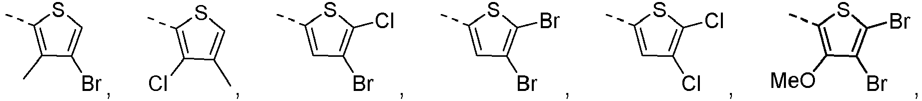 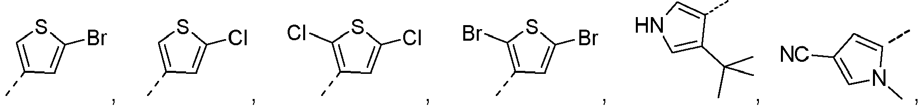 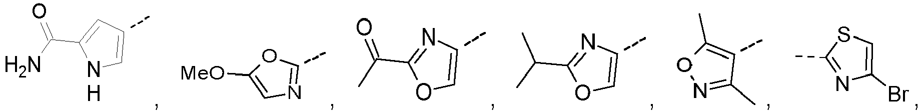 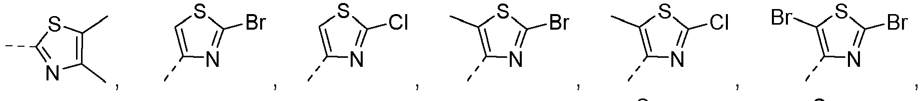 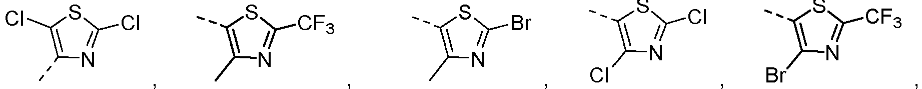 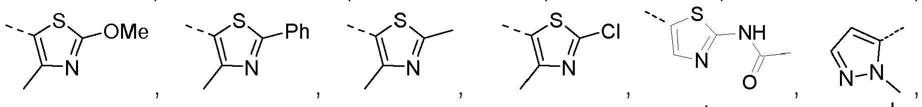 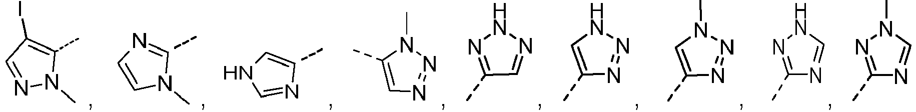 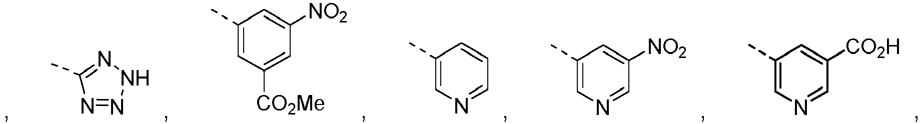 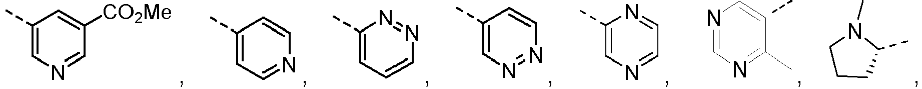 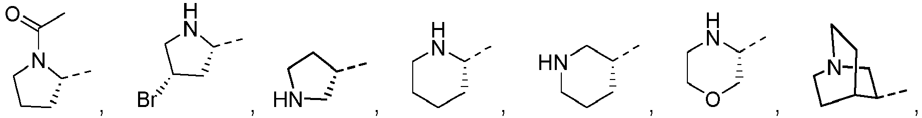 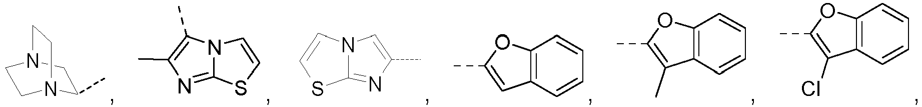 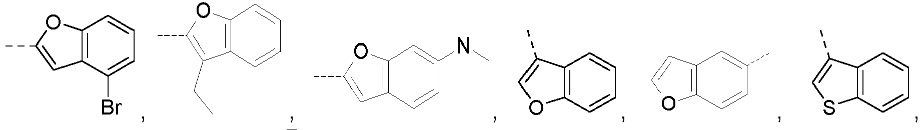 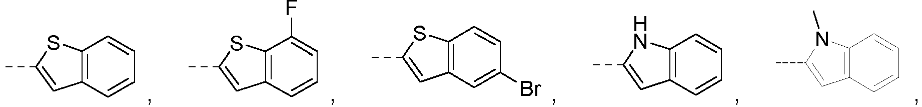 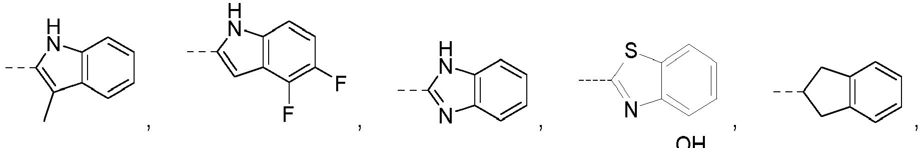 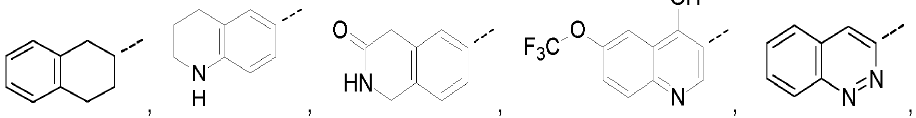 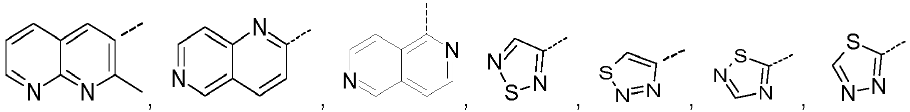 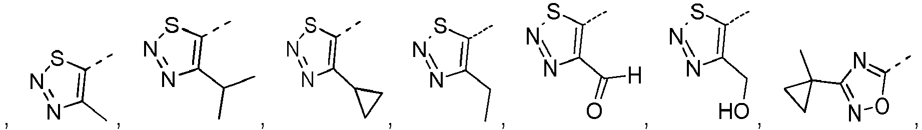 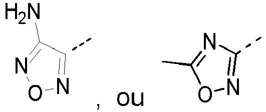 et
**R⁶** représente -H, -CH₃, -CH(CH₃)₂, -CH₂CH₂CH₃, -CH₂CH=CH₂, -CH₂CH₂CH₂CH₃, -CH₂CH(CH₃)₂, -C(CH₃)₃, -CH₂CH₂CH₂CH₂CH₃, -cyclo-C₃H₅, -cyclo-C₅H₉, -cyclo-C₆H₁₁ ou -CH₂-cyclo-C₃H₅.

9. Le composé conformé à la revendication 1, sélectionné parmi le groupe constitué de :
| Composé | Nom |
|---|---|
| **II-2** : | (S)-2-(benzofuran-2-carboxamido)-N1-(1-(2-(2-adamantylamino)-2-oxoéthyl)-2-oxo-1,2-dihydropyridin-3-yl)-N6-méthyl-5-oxohexanediamide |
| **II-3** : | (S)-N1-(1-(2-(2-adamantylamino)-2-oxoéthyl)-2-oxo-1,2-dihydropyridin-3-yl)-N6-méthyl-2-(3-méthylbenzofuran-2-carboxamido)-5-oxohexanediamide |
| **II-4** : | (S)-2-(3-chlorobenzofuran-2-carboxamido)-N1-(1-(2-(2-adamantylamino)-2-oxoéthyl)-2-oxo-1,2-dihydropyridin-3-yl)-N6-méthyl-5-oxohexanediamide |
| **II-5** : | (S)-2-(4-bromobenzofuran-2-carboxamido)-N1-(1-(2-(2-adamantylamino)-2-oxoéthyl)-2-oxo-1,2-dihydropyridin-3-yl)-N6-méthyl-5-oxohexanediamide |
| **II-6** : | (S)-2-(4-bromobenzofuran-2-carboxamido)-N1-(1-(2-(2-adamantylamino)-2-oxoéthyl)-2-oxo-1,2-dihydropyridin-3-yl)-N6-méthyl-5-oxohexanediamide |
| **II-7** : | (S)-2-(benzo[b]thiophène-2-carboxamido)-N1-(1-(2-(2-adamantylamino)-2-oxoéthyl)-2-oxo-1,2-dihydropyridin-3-yl)-N6-méthyl-5-oxohexanediamide |
| **II-8** : | (S)-2-(5-bromobenzo[b]thiophène-2-carboxamido)-N1-(1-(2-(2-adamantylamino)-2-oxoéthyl)-2-oxo-1,2-dihydropyridin-3-yl)-N6-méthyl-5-oxohexanediamide |
| **II-9** : | (S)-2-(1H-indole-2-carboxamido)-N1-(1-(2-(2-adamantylamino)-2-oxoéthyl)-2-oxo-1,2-dihydropyridin-3-yl)-N6-méthyl-5-oxohexanediamide |
| **II-10** : | (S)-2-(4,5-difluoro-1H-indole-2-carboxamido)-N1-(1-(2-(2-adamantylamino)-2-oxoéthyl)-2-oxo-1,2-dihydropyridin-3-yl)-N6-méthyl-5-oxohexanediamide |
| **II-11** : | (S)-N1-(1-(2-(2-adamantylamino)-2-oxoéthyl)-2-oxo-1,2-dihydropyridin-3-yl)-N6-méthyl-2-(3-méthyl-1H-indole-2-carboxamido)-5-oxohexanediamide |
| **II-12** : | (S)-2-(1H-benzo[d]imidazole-2-carboxamido)-N1-(1-(2-(2-adamantylamino)-2-oxoéthyl)-2-oxo-1,2-dihydropyridin-3-yl)-N6-méthyl-5-oxohexanediamide |
| **II-13** : | (S)-2-(2,3-dihydro-1H-indène-2-carboxamido)-N1-(1-(2-(2-adamantylamino)-2-oxoéthyl)-2-oxo-1,2-dihydropyridin-3-yl)-N6-méthyl-5-oxohexanediamide |
| **II-14** : | (S)--(2-bromo-4-méthylthiazole-5-carboxamido)-N1-(1-(2-(2-adamantylamino)-2-oxoéthyl)-2-oxo-1,2-dihydropyridin-3-yl)-N6-méthyl-5-oxohexanediamide |
| **II-15** : | (S)-N1-(1-(2-(2-adamantylamino)-2-oxoéthyl)-2-oxo-1,2-dihydropyridin-3-yl)-N6-méthyl-2-(4-méthyl-2-(trifluorométhyl)thiazole-5-carboxamido)-5-oxohexanediamide |
| **II-16** : | (S)-2-(4-bromo-2-(trifluorométhyl)thiazole-5-carboxamido)-N1-(1-(2-(2-adamantylamino)-2-oxoéthyl)-2-oxo-1,2-dihydropyridin-3-yl)-N6-méthyl-5-oxohexanediamide |
| **II-17** : | (S)-2-(2,4-dichlorothiazole-5-carboxamido)-N1-(1-(2-(2-adamantylamino)-2-oxoéthyl)-2-oxo-1,2-dihydropyridin-3-yl)-N6-méthyl-5-oxohexanediamide |
| **II-18** : | (S)-N1-(1-(2-(2-adamantylamino)-2-oxoéthyl)-2-oxo-1,2-dihydropyridin-3-yl)-2-(2-méthoxy-4-méthylthiazole-5-carboxamido)-N6-méthyl-5-oxohexanediamide |
| **II-19** : | (S)-N1-(1-(2-(2-adamantylamino)-2-oxoéthyl)-2-oxo-1,2-dihydropyridin-3-yl)-N6-méthyl-2-(4-méthyl-2-phénylthiazole-5-carboxamido)-5-oxohexanediamide |
| **II-20** : | (S)-2-(2,4-diméthylthiazole-5-carboxamido)-N1-(1-(2-(2-adamantylamino)-2-oxoéthyl)-2-oxo-1,2-dihydropyridin-3-yl)-N6-méthyl-5-oxohexanediamide |
| **II-21** : | (S)-2-(5-bromo-3-méthylthiophène-2-carboxamido)-N1-(1-(2-(2-adamantylamino)-2-oxoéthyl)-2-oxo-1,2-dihydropyridin-3-yl)-N6-méthyl-5-oxohexanediamide |
| **II-22** : | (S)-2-(3,5-dibromothiophène-2-carboxamido)-N1-(1-(2-(2-adamantylamino)-2-oxoéthyl)-2-oxo-1,2-dihydropyridin-3-yl)-N6-méthyl-5-oxohexanediamide |
| **II-23** : | (S)-2-(5-bromothiophène-2-carboxamido)-N1-(1-(2-(2-adamantylamino)-2-oxoéthyl)-2-oxo-1,2-dihydropyridin-3-yl)-N6-méthyl-5-oxohexanediamide |
| **II-24** : | (S)-2-(5-chlorothiophène-2-carboxamido)-N1-(1-(2-(2-adamantylamino)-2-oxoéthyl)-2-oxo-1,2-dihydropyridin-3-yl)-N6-méthyl-5-oxohexanediamide |
| **II-25** : | (S)-2-(5-bromo-3-méthylfuran-2-carboxamido)-N1-(1-(2-(2-adamantylamino)-2-oxoéthyl)-2-oxo-1,2-dihydropyridin-3-yl)-N6-méthyl-5-oxohexanediamide |
| **II-26** : | (S)-2-(5-chlorofuran-2-carboxamido)-N1-(1-(2-(2-adamantylamino)-2-oxoéthyl)-2-oxo-1,2-dihydropyridin-3-yl)-N6-méthyl-5-oxohexanediamide |
| **II-27** : | (S)-2-(5-chlorothiophène-3-carboxamido)-N1-(1-(2-(2-adamantylamino)-2-oxoéthyl)-2-oxo-1,2-dihydropyridin-3-yl)-N6-méthyl-5-oxohexanediamide |
| **II-28** : | (S)-2-(2,5-dichlorothiophène-3-carboxamido)-N1-(1-(2-(2-adamantylamino)-2-oxoéthyl)-2-oxo-1,2-dihydropyridin-3-yl)-N6-méthyl-5-oxohexanediamide |
| **II-29** : | (S)-2-(2,5-dibromothiophène-3-carboxamido)-N1-(1-(2-(2-adamantylamino)-2-oxoéthyl)-2-oxo-1,2-dihydropyridin-3-yl)-N6-méthyl-5-oxohexanediamide |
| **II-30** : | (S)-2-(5-bromothiophène-3-carboxamido)-N1-(1-(2-(2-adamantylamino)-2-oxoéthyl)-2-oxo-1,2-dihydropyridin-3-yl)-N6-méthyl-5-oxohexanediamide |
| **II-31** : | (S)-2-(2-chloro-5-méthylthiazole-4-carboxamido)-N1-(1-(2-(2-adamantylamino)-2-oxoéthyl)-2-oxo-1,2-dihydropyridin-3-yl)-N6-méthyl-5-oxohexanediamide |
| **II-32** : | (S)-2-(2,5-dichlorothiazole-4-carboxamido)-N1-(1-(2-(2-adamantylamino)-2-oxoéthyl)-2-oxo-1,2-dihydropyridin-3-yl)-N6-méthyl-5-oxohexanediamide |
| **II-33** : | (S)-2-(2,5-dibromothiazole-4-carboxamido)-N1-(1-(2-(2-adamantylamino)-2-oxoéthyl)-2-oxo-1,2-dihydropyridin-3-yl)-N6-méthyl-5-oxohexanediamide |
| **II-34** : | (S)-2-(2-bromo-5-méthylthiazole-4-carboxamido)-N1-(1-(2-(2-adamantylamino)-2-oxoéthyl)-2-oxo-1,2-dihydropyridin-3-yl)-N6-méthyl-5-oxohexanediamide |
| **II-35** : | (S)-2-(2-bromothiazole-4-carboxamido)-N1-(1-(2-(2-adamantylamino)-2-oxoéthyl)-2-oxo-1,2-dihydropyridin-3-yl)-N6-méthyl-5-oxohexanediamide |
| **II-36** : | (S)-2-(2-chlorothiazole-4-carboxamido)-N1-(1-(2-(2-adamantylamino)-2-oxoéthyl)-2-oxo-1,2-dihydropyridin-3-yl)-N6-méthyl-5-oxohexanediamide |
| **II-37** : | (S)-2-(2,5-diméthylfuran-3-carboxamido)-N1-(1-(2-(2-adamantylamino)-2-oxoéthyl)-2-oxo-1,2-dihydropyridin-3-yl)-N6-méthyl-5-oxohexanediamide |
| **II-38** : | (S)-2-(4,5-diméthylthiazole-2-carboxamido)-N1-(1-(2-(2-adamantylamino)-2-oxoéthyl)-2-oxo-1,2-dihydropyridin-3-yl)-N6-méthyl-5-oxohexanediamide |
| **II-39** : | (S)-2-(4-bromothiazole-2-carboxamido)-N1-(1-(2-(2-adamantylamino)-2-oxoéthyl)-2-oxo-1,2-dihydropyridin-3-yl)-N6-méthyl-5-oxohexanediamide |
| **II-40** : | (S)-2-(4-bromothiophène-2-carboxamido)-N1-(1-(2-(2-adamantylamino)-2-oxoéthyl)-2-oxo-1,2-dihydropyridin-3-yl)-N6-méthyl-5-oxohexanediamide |
| **II-41** : | (S)-2-(4-bromo-3-méthylthiophène-2-carboxamido)-N1-(1-(2-(2-adamantylamino)-2-oxoéthyl)-2-oxo-1,2-dihydropyridin-3-yl)-N6-méthyl-5-oxohexanediamide |
| **II-42** : | (S)-2-(3-bromothiophène-2-carboxamido)-N1-(1-(2-(2-adamantylamino)-2-oxoéthyl)-2-oxo-1,2-dihydropyridin-3-yl)-N6-méthyl-5-oxohexanediamide |
| **II-43** : | (S)-2-(3-chloro-4-méthylthiophène-2-carboxamido)-N1-(1-(2-(2-adamantylamino)-2-oxoéthyl)-2-oxo-1,2-dihydropyridin-3-yl)-N6-méthyl-5-oxohexanediamide |
| **II-44** : | (S)-2-(4-bromo-5-chlorothiophène-2-carboxamido)-N1-(1-(2-(2-adamantylamino)-2-oxoéthyl)-2-oxo-1,2-dihydropyridin-3-yl)-N6-méthyl-5-oxohexanediamide |
| **II-45** : | (S)-2-(4,5-dibromothiophène-2-carboxamido)-N1-(1-(2-(2-adamantylamino)-2-oxoéthyl)-2-oxo-1,2-dihydropyridin-3-yl)-N6-méthyl-5-oxohexanediamide |
| **II-46** : | (S)-2-(4,5-dibromo-3-méthoxythiophène-2-carboxamido)-N1-(1-(2-(2-adamantylamino)-2-oxoéthyl)-2-oxo-1,2-dihydropyridin-3-yl)-N6-méthyl-5-oxohexanediamide |
| **II-47** : | (S)-2-(4-bromofuran-2-carboxamido)-N1-(1-(2-(2-adamantylamino)-2-oxoéthyl)-2-oxo-1,2-dihydropyridin-3-yl)-N6-méthyl-5-oxohexanediamide |
| **II-48** : | (S)-2-(4,5-dibromofuran-2-carboxamido)-N1-(1-(2-(2-adamantylamino)-2-oxoéthyl)-2-oxo-1,2-dihydropyridin-3-yl)-N6-méthyl-5-oxohexanediamide |
| **II-49** : | (S)-2-(4,5-dichlorothiophène-2-carboxamido)-N1-(1-(2-(2-adamantylamino)-2-oxoéthyl)-2-oxo-1,2-dihydropyridin-3-yl)-N6-méthyl-5-oxohexanediamide |
| **II-50** : | (S)-2-((S)-1-acétylpyrrolidine-2-carboxamido)-N 1-(1-(2-(2-adamantylamino)-2-oxoéthyl)-2-oxo-1,2-dihydropyridin-3-yl)-N6-méthyl-5-oxohexanediamide |
| **II-51** : | (S)-N1-(1-(2-(2-adamantylamino)-2-oxoéthyl)-2-oxo-1,2-dihydropyridin-3-yl)-N6-méthyl-2-(1-méthyl-1H-1,2,3-triazole-5-carboxamido)-5-oxohexanediamide |
| **II-52** : | (S)-N1-(1-(2-(2-adamantylamino)-2-oxoéthyl)-2-oxo-1,2-dihydropyridin-3-yl)-N6-méthyl-5-oxo-2-(2H-tétrazole-5-carboxamido)hexanediamide |
| **II-53** : | (S)-N1-(1-(2-(2-adamantylamino)-2-oxoéthyl)-2-oxo-1,2-dihydropyridin-3-yl)-N6-méthyl-5-oxo-2-(pyrazine-2-carboxamido)hexanediamide |
| **II-54** : | (S)-N1-(1-(2-(2-adamantylamino)-2-oxoéthyl)-2-oxo-1,2-dihydropyridin-3-yl)-N6-méthyl-2-((S)-1-méthylpyrrolidine-2-carboxamido)-5-oxohexanediamide |
| **II-55** : | (S)-N1-(1-(2-(2-adamantylamino)-2-oxoéthyl)-2-oxo-1,2-dihydropyridin-3-yl)-N6-méthyl-5-oxo-2-((S)-pyrrolidine-3-carboxamido)hexanediamide |
| **II-56** : | (S)-2-((2S,4S)-4-bromopyrrolidine-2-carboxamido)-N1-(1-(2-(2-adamantylamino)-2-oxoéthyl)-2-oxo-1,2-dihydropyridin-3-yl)-N6-méthyl-5-oxohexanediamide |
| **II-58** : | (S)-N1-(1-(2-(2-adamantylamino)-2-oxoéthyl)-2-oxo-1,2-dihydropyridin-3-yl)-N6-méthyl-5-oxo-2-((S)-piperidine-2-carboxamido)hexanediamide |
| **II-59** : | (S)-N1-(1-(2-(2-adamantylamino)-2-oxoéthyl)-2-oxo-1,2-dihydropyridin-3-yl)-N6-méthyl-5-oxo-2-((R)-piperidine-3-carboxamido)hexanediamide |
| **II-60** : | (S)-N1-(1-(2-(2-adamantylamino)-2-oxoéthyl)-2-oxo-1,2-dihydropyridin-3-yl)-N6-méthyl-2-((R)-morpholine-3-carboxamido)-5-oxohexanediamide |
| **II-61** : | (S)-N1-(1-(2-(2-adamantylamino)-2-oxoéthyl)-2-oxo-1,2-dihydropyridin-3-yl)-N6-méthyl-5-oxo-2-(quinuclidine-3-carboxamido)hexanediamide |
| **II-62** : | (S) 3-(1-(1-(2-(2-adamantylamino)-2-oxoéthyl)-2-oxo-1,2-dihydropyridin-3-ylamino)-6-(méthylamino)-1,5,6-trioxohexan-2-ylcarbamoyl)-5-nitrobenzoate de méthyle |
| **II-63** : | (S)-N1-(1-(2-(2-adamantylamino)-2-oxoéthyl)-2-oxo-1,2-dihydropyridin-3-yl)-N6-méthyl-2-(5-nitronicotinamido)-5-oxohexanediamide |
| **II-64** : | Acide (S)-5-(1-(1-(2-(2-adamantylamino)-2-oxoéthyl)-2-oxo-1 ,2-dihydropyridin-3-ylamino)-6-(méthylamino)-1,5,6-trioxohexan-2-ylcarbamoyl)-nicotinique |
| **II-65** : | (S)-méthyle 5-(1-(1-(2-(2-adamantylamino)-2-oxoéthyl)-2-oxo-1,2-dihydropyridin-3-ylamino)-6-(méthylamino)-1,5,6-trioxohexan-2-ylcarbamoyl)nicotinate |
| **II-66** : | (S)-N1-(1-(2-(2-adamantylamino)-2-oxoéthyl)-2-oxo-1,2-dihydropyridin-3-yl)-N6-méthyl-2-(6-méthylimidazo[2,1-b]thiazole-5-carboxamido)-5-oxohexanediamide |
| **II-67** : | (S)-N1-(1-(2-(2-adamantyl(méthyl)amino)-2-oxoéthyl)-2-oxo-1,2-dihydropyridin-3-yl)-N6-méthyl-2-(3-méthylbenzofuran-2-carboxamido)-5-oxohexanediamide |
| **II-68** : | (S)-N1-(1-(2-(5-hydroxyadamantane-2-amino)-2-oxoéthyl)-2-oxo-1,2-dihydropyridin-3-yl)-N6-méthyl-2-(3-méthylbenzofuran-2-carboxamido)-5-oxohexanediamide |
| **II-69** : | (S)-N1-(1-(2-(5-fluoroadamantane-2-amino)-2-oxoéthyl)-2-oxo-1,2-dihydropyridin-3-yl)-N6-méthyl-2-(3-méthylbenzofuran-2-carboxam ido)-5-oxohexanediamide |
| **II-70** : | (S)-N1-(1-(2-(5-chloroadamantane-2-amino)-2-oxoéthyl)-2-oxo-1,2-dihydropyridin-3-yl)-N6-méthyl-2-(3-méthylbenzofuran-2-carboxamido)-5-oxohexanediamide |
| **II-71** : | (S)-N1-(1-(2-(5-bromoadamantane-2-amino)-2-oxoéthyl)-2-oxo-1,2-dihydropyridin-3-yl)-N6-méthyl-2-(3-méthylbenzofuran-2-carboxamido)-5-oxohexanediamide |
| **II-72** : | (S)-N1-(1-(2-(5-méthyladamantane-2-amino)-2-oxoéthyl)-2-oxo-1,2-dihydropyridin-3-yl)-N6-méthyl-2-(3-méthylbenzofuran-2-carboxamido)-5-oxohexanediamide |
| **II-73** : | (S)-N1-(1-(2-(2-carbonitrileadamantane-2-amino)-2-oxoéthyl)-2-oxo-1,2-dihydropyridin-3-yl)-N6-méthyl-2-(3-méthylbenzofuran-2-carboxamido)-5-oxohexanediamide |
| **II-74** : | (S)-N1-(1-(2-(2-méthyl adamantane-2-carboxylate-2-amino)-2-oxoéthyl)-2-oxo-1,2-dihydropyridin-3-yl)-N6-méthyl-2-(3-méthylbenzofuran-2-carboxamido)-5-oxohexanediamide |
| **II-87** : | (S)-N1-(1-(2-(1-adamantylméthylamino)-2-oxoéthyl)-2-oxo-1,2-dihydropyridin-3-yl)-N6-méthyl-2-(3-méthylbenzofuran-2-carboxamido)-5-oxohexanediamide |
| **II-88** : | (S)-N1-(1-(2-(1-(1-adamantyl)éthanamino)-2-oxoéthyl)-2-oxo-1,2-dihydropyridin-3-yl)-N6-méthyl-2-(3-méthylbenzofuran-2-carboxamido)-5-oxohexanediamide |
| **II-90** : | (S)-N1-(1-(2-((1R,2S,4S)-bicyclo[2.2.1]heptan-2-ylamino)-2-oxoéthyl)-2-oxo-1,2-dihydropyridin-3-yl)-N6-méthyl-2-(3-méthylbenzofuran-2-carboxamido)-5-oxohexanediamide |
| **II-92** : | (S)-N1-méthyl-5-(3-méthylbenzofuran-2-carboxamido)-2-oxo-N6-(2-oxo-1-(2-oxo-2-((1S,2S,4R)-1,7,7-triméthylbicyclo[2.2.1]heptan-2-ylamino)éthyl)-1,2-dihydropyridin-3-yl)hexanediamide |
| **II-94** : | (S)-N1-(1-(2-((1R,2R,4S)-bicyclo[2.2.1]heptan-2-ylamino)-2-oxoéthyl)-2-oxo-1,2-dihydropyridin-3-yl)-N6-méthyl-2-(3-méthylbenzofuran-2-carboxamido)-5-oxohexanediamide |
| **II-95** : | (S)-N1-(1-(2-(bicyclo[2.2.1]heptan-1-ylamino)-2-oxoéthyl)-2-oxo-1,2-dihydropyridin-3-yl)-N6-méthyl-2-(3-méthylbenzofuran-2-carboxamido)-5-oxohexanediamide |
| **II-96** : | (S)-N1-(1-(2-(bicyclo[2.2.1]heptan-7-ylamino)-2-oxoéthyl)-2-oxo-1,2-dihydropyridin-3-yl)-N6-méthyl-2-(3-méthylbenzofuran-2-carboxamido)-5-oxohexanediamide |
| **II-97** : | (S)-N1-(1-(2-(bicyclo[2.2.1]hept-5-en-2-ylamino)-2-oxoéthyl)-2-oxo-1,2-dihydropyridin-3-yl)-N6-méthyl-2-(3-méthylbenzofuran-2-carboxamido)-5-oxohexanediamide |
| **II-98** : | (2S)-N1-(1-(2-(bicyclo[2.2.2]octan-2-ylamino)-2-oxoéthyl)-2-oxo-1,2-dihydropyridin-3-yl)-N6-méthyl-2-(3-méthylbenzofuran-2-carboxamido)-5-oxohexanediamide |
| **II-99** : | (S)-N1-méthyl-5-(3-méthylbenzofuran-2-carboxamido)-2-oxo-N6-(2-oxo-1-(2-oxo-2-((1R,2R,4R)-1,7,7-triméthylbicyclo[2.2.1]heptan-2-ylamino)éthyl)-1,2-dihydropyridin-3-yl)hexanediamide |
| **II-100** : | (S)-N1-méthyl-5-(3-méthylbenzofuran-2-carboxamido)-2-oxo-N6-(2-oxo-1-(2-oxo-2-((1R,2R,3R,5S)-2,6,6-triméthylbicyclo[3.1.1]heptan-3-ylamino)éthyl)-1,2-dihydropyridin-3-yl)hexanediamide |
| **II-101** : | (S)-N1-méthyl-5-(3-méthylbenzofuran-2-carboxamido)-2-oxo-N6-(2-oxo-1-(2-oxo-2-((1S,2S,3S,5R)-2,6,6-triméthylbicyclo[3.1.1]heptan-3-ylamino)éthyl)-1,2-dihydropyridin-3-yl)hexanediamide |
| **II-103** : | (S)-N1-(1-(2-(4-homoisotwistane-3-amino)-2-oxoéthyl)-2-oxo-1,2-dihydropyridin-3-yl)-N6-méthyl-2-(3-méthylbenzofuran-2-carboxamido)-5-oxohexanediamide |
| **II-104** : | (S)-N1-(1-(2-(diamantane-1-amino)-2-oxoéthyl)-2-oxo-1,2-dihydropyridin-3-yl)-N6-méthyl-2-(3-méthylbenzofuran-2-carboxamido)-5-oxohexanediamide |
| **II-105** : | (S)-N1-(1-(2-(diamantane-4-amino)-2-oxoéthyl)-2-oxo-1,2-dihydropyridin-3-yl)-N6-méthyl-2-(3-méthylbenzofuran-2-carboxamido)-5-oxohexanediamide |
| **II-107** : | (S)-N1-(1-(1-adamantylméthyl)-2-oxo-1,2-dihydropyridin-3-yl)-N6-méthyl-2-(3-méthylbenzofuran-2-carboxamido)-5-oxohexanediamide |
| **II-108** : | (2S)-N1-(1-((3-hydroxy-1-adamantyl)méthyl)-2-oxo-1,2-dihydropyridin-3-yl)-N6-méthyl-2-(3-méthylbenzofuran-2-carboxamido)-5-oxohexanediamide |
| **II-109** : | (2S)-N1-(1-((3-bromo-1-adamantyl)méthyl)-2-oxo-1,2-dihydropyridin-3-yl)-N6-méthyl-2-(3-méthylbenzofuran-2-carboxamido)-5-oxohexanediamide |
| **II-110 :** | (S)-N1-(1-(2-adamantylméthyl)-2-oxo-1,2-dihydropyridin-3-yl)-N6-méthyl-2-(3-méthylbenzofuran-2-carboxamido)-5-oxohexanediamide |
| **II-111** : | (S)-N1-(1-(2-(2-adamantylamino)-2-oxoéthyl)-2-oxo-1,2-dihydropyridin-3-yl)-N6-méthyl-2-(nicotinamido)-5-oxohexanediamide |
| **II-112 :** | (S)-2-(isonicotinamido)-N1-(1-(2-(2-adamantylamino)-2-oxoéthyl)-2-oxo-1,2-dihydropyridin-3-yl)-N6-méthyl-5-oxohexanediamide |
| **II-113 :** | (S)-N1-(1-(2-(2-adamantylamino)-2-oxoéthyl)-2-oxo-1,2-dihydropyridin-3-yl)-N6-méthyl-5-oxo-2-(pyridazine-4-carboxamido)hexanediamide |
| **II-114 :** | (S)-N1-(1-(2-(2-adamantylamino)-2-oxoéthyl)-2-oxo-1,2-dihydropyridin-3-yl)-N6-méthyl-5-oxo-2-(pyridazine-3-carboxamido)hexanediamide |
| **II-115 :** | (S)-N1-cyclopropyl-N6-(1-(2-(2-adamantylamino)-2-oxoéthyl)-2-oxo-1,2-dihydropyridin-3-yl)-5-(3-méthylbenzofuran-2-carboxamido)-2-oxohexanediamide |
| **II-116 :** | (S)-N1-(1-(2-(2-adamantylamino)-2-oxoéthyl)-2-oxo-1,2-dihydropyridin-3-yl)-2-(3-méthylbenzofuran-2-carboxam ido)-5-oxo-N6-pentylhexanediamide |
| **II-117 :** | (S)-N1-allyl-N6-(1-(2-(2-adamantylamino)-2-oxoéthyl)-2-oxo-1,2-dihydropyridin-3-yl)-5-(3-méthylbenzofuran-2-carboxamido)-2-oxohexanediamide |
| **II-118** : | (S)-N1-(1-(2-(2-adamantylamino)-2-oxoéthyl)-2-oxo-1,2-dihydropyridin-3-yl)-2-(3-méthylbenzofuran-2-carboxamido)-5-oxohexanediamide |
| **II-119** : | (S)-N1-allyl-5-(benzofuran-2-carboxamide)-N6-(-(2-(2-adamantylamino)-2-oxoéthyl)-2-oxo-1,2-dihydropyridin-3-yl)-2-oxohexanediamide |
| **II-120** : | (S)-2-(benzofuran-2-carboxamido)-N6-isopropyl-N1-(1-(2-(2-adamantylamino)-2-oxoéthyl)-2-oxo-1,2-dihydropyridin-3-yl)-5-oxohexanediamide, |
| **II-121 :** | (S)-2-(benzofuran-2-carboxamido)-N6-cyclopropyl-N1-(1-(2-(2-adamantylamino)-2-oxoéthyl)-2-oxo-1,2-dihydropyridin-3-yl)-5-oxohexanediamide, |
| **II-122** : | (S)-2-(benzofuran-2-carboxamido)-N1-(1-(2-(2-adamantylamino)-2-oxoéthyl)-2-oxo-1,2-dihydropyridin-3-yl)-5-oxo-N6-phénylhexanediamide |
| **II-123** : | (S)-2-(benzofuran-2-carboxamido)-N6-benzyl-N1-(1-(2-(2-adamantylamino)-2-oxoéthyl)-2-oxo-1,2-dihydropyridin-3-yl)-5-oxohexanediamide |
| **II-124** : | (S)-2-(benzofuran-2-carboxamido)-N1-(1-(2-(2-adamantylamino)-2-oxoéthyl)-2-oxo-1,2-dihydropyridin-3-yl)-5-oxohexanediamide |
| **II-125 :** | (S)-2-(2,5-dichlorothiophène-3-carboxamido)-N1-(1-(2-(2-adamantylamino)-2-oxoéthyl)-2-oxo-1,2-dihydropyridin-3-yl)-5-oxohexanediamide |
| **II-126** : | (S)-N1-(1-(2-(2-adamantylamino)-2-oxoéthyl)-2-oxo-1,2-dihydropyridin-3-yl)-2-(4-méthyl-2-(trifluorométhyl)thiazole-5-carboxamido)-5-oxohexanediamide |
| **II-127** : | (S)-N1-(1-(2-(2-adamantylamino)-2-oxoéthyl)-2-oxo-1,2-dihydropyridin-3-yl)-2-(1-méthyl-1H-1,2,3-triazole-5-carboxamido)-5-oxohexanediamide |
| **II-128** : | (2S)-N1-(1-(2-((1R,2R,4S)-bicyclo[2.2.1]heptan-2-ylamino)-2-oxoéthyl)-2-oxo-1,2-dihydropyridin-3-yl)-2-(2,5-dichlorothiophène-3-carboxamido)-N6-méthyl-5-oxohexanediamide |
| **II-129** : | (2S)-N1-(1-(2-((1R,2R,4S)-bicyclo[2.2.1]heptan-2-ylamino)-2-oxoéthyl)-2-oxo-1,2-dihydropyridin-3-yl)-N6-méthyl-2-(4-méthyl-2-(trifluorométhyl)thiazole-5-carboxamido)-5-oxohexanediamide |
| **II-130** : | (2S)-N1-(1-(2-((1R,2R,4S)-bicyclo[2.2.1]heptan-2-ylamino)-2-oxoéthyl)-2-oxo-1,2-dihydropyridin-3-yl)-N6-méthyl-2-(1-méthyl-1H-1,2,3-triazole-5-carboxamido)-5-oxohexanediamide |
| **II-131** : | (S)-N1-(1-(2-(2-adamantylamino)-2-oxoéthyl)-2-oxo-1,2-dihydropyridin-3-yl)-N6-méthyl-5-oxo-2-(2H-1,2,3-triazole-4-carboxamido)hexanediamide |
| **II-132** : | (S)-N1-(1-(2-(2-adamantylamino)-2-oxoéthyl)-2-oxo-1,2-dihydropyridin-3-yl)-N6-méthyl-5-oxo-2-(1H-1,2,3-triazole-4-carboxamido)hexanediamide |
| **II-133** : | (S)-N1-(1-(2-(2-adamantylamino)-2-oxoéthyl)-2-oxo-1,2-dihydropyridin-3-yl)-N6-méthyl-2-(1-méthyl-1H-1,2,3-triazole-4-carboxamido)-5-oxohexanediamide |
| **II-134** : | (S)-N1-(1-(2-(2-adamantylamino)-2-oxoéthyl)-2-oxo-1,2-dihydropyridin-3-yl)-N6-méthyl-5-oxo-2-(1H-1,2,4-triazole-3-carboxamido)hexanediamide |
| **II-135** : | (S)-N1-(1-(2-(2-adamantylamino)-2-oxoéthyl)-2-oxo-1,2-dihydropyridin-3-yl)-N6-méthyl-2-(1-méthyl-1H-1,2,4-triazole-3-carboxamido)-5-oxohexanediamide |
| **II-136** : | (S)-2-(benzofuran-3-carboxamido)-N1-(1-(2-(2-adamantylamino)-2-oxoéthyl)-2-oxo-1,2-dihydropyridin-3-yl)-N6-méthyl-5-oxohexanediamide |
| **II-137** : | (S)-2-(benzo[b]thiophène-3-carboxamido)-N1 -(1 -(2-(2-adamantylamino)-2-oxoéthyl)-2-oxo-1,2-dihydropyridin-3-yl)-N6-méthyl-5-oxohexanediamide |
| **II-138** | (S)-N1-(1-(2-(2-adamantylamino)-2-oxoéthyl)-2-oxo-1,2-dihydropyridin-3-yl)-N6-méthyl-2-(1-méthyl-1H-pyrazole-3-carboxamido)-5-oxohexanediamide |
| **II-139** | (S)-N1-(1-(2-(2-adamantylamino)-2-oxoéthyl)-2-oxo-1,2-dihydropyridin-3-yl)-N6-méthyl-2-(1-méthyl-1H-pyrazole-4-carboxamido)-5-oxohexanediamide |
| **II-140** | (S)-N1-(1-(2-(2-adamantylamino)-2-oxoéthyl)-2-oxo-1,2-dihydropyridin-3-yl)-N6-méthyl-2-(1-méthyl-1H-pyrazole-5-carboxamido)-5-oxohexanediamide |
| **II-141** | (S)-N1-(1-(2-(2-adamantylamino)-2-oxoéthyl)-2-oxo-1,2-dihydropyridin-3-yl)-N6-méthyl-2-(4-méthyl-1,2,3-thiadiazole-5-carboxamido)-5-oxohexanediamide |
| **II-142** | (S)-N1-(1-(2-(2-adamantylamino)-2-oxoéthyl)-2-oxo-1,2-dihydropyridin-3-yl)-N6-méthyl-5-oxo-2-(1,2,5-thiadiazole-3-carboxamido)hexanediamide |
| **II-143** | (S)-2-(4-iodo-1-méthyl-1H-pyrazole-5-carboxamido)-N1-(1-(2-(2-adamantylamino)-2-oxoéthyl)-2-oxo-1,2-dihydropyridin-3-yl)-N6-méthyl-5-oxohexanediamide |
| **II-144** | (S)-N1-(1-(2-(2-adamantylamino)-2-oxoéthyl)-2-oxo-1,2-dihydropyridin-3-yl)-2-(1-méthyl-1H-pyrazole-5-carboxamido)-5-oxohexanediamide |
| **II-145** | (S)-N1-(1-(2-(2-adamantylamino)-2-oxoéthyl)-2-oxo-1,2-dihydropyridin-3-yl)-2-(4-méthyl-1,2,3-thiadiazole-5-carboxamido)-5-oxohexanediamide |
| **II-146** | (S)-2-(benzofuran-2-carboxamido)-N1-(1-(2-((1R,2R,4S)-bicyclo[2.2.1]heptan-2-ylamino)-2-oxoéthyl)-2-oxo-1,2-dihydropyridin-3-yl)-5-oxohexanediamide |
| **II-147** | (S)-N1-(1-(2-((1R,2R,4S)-bicyclo[2.2.1]heptan-2-ylamino)-2-oxoéthyl)-2-oxo-1,2-dihydropyridin-3-yl)-2-(3-méthylbenzofuran-2-carboxamido)-5-oxohexanediamide |
| **II-148** | (S)-2-(benzofuran-2-carboxamido)-N1-(1-(2-((1S,2R,4R)-bicyclo[2.2.1]heptan-2-ylamino)-2-oxoéthyl)-2-oxo-1,2-dihydropyridin-3-yl)-5-oxohexanediamide |
| **II-149** | (S)-N1-(1-(2-((1S,2R,4R)-bicyclo[2.2.1]heptan-2-ylamino)-2-oxoéthyl)-2-oxo-1,2-dihydropyridin-3-yl)-2-(3-méthylbenzofuran-2-carboxamido)-5-oxohexanediamide |
| **II-150** | (S)-2-(benzofuran-2-carboxamido)-5-oxo-N1-(2-oxo-1-(2-oxo-2-((1R,2S,4R)-1,7,7-triméthylbicyclo[2.2.1]heptan-2-ylamino)éthyl)-1,2-dihydropyridin-3-yl)hexanediamide |
| **II-151** | (S)-2-(3-méthylbenzofuran-2-carboxamido)-5-oxo-N1-(2-oxo-1-(2-oxo-2-((1R,2S,4R)-1,7,7-triméthylbicyclo[2.2.1]heptan-2-ylamino)éthyl)-1,2-dihydropyridin-3-yl)hexanediamide |
| **II-152** | (S)-N1-(1-(2-((1R,2R,4S)-bicyclo[2.2.1]heptan-2-ylamino)-2-oxoéthyl)-2-oxo-1,2-dihydropyridin-3-yl)-N6-méthyl-2-(4-méthyl-1,2,3-thiadiazole-5-carboxamido)-5-oxohexanediamide |
| **II-153** | (S)-N1-(1-(2-((1R,2R,4S)-bicyclo[2.2.1]heptan-2-ylamino)-2-oxoéthyl)-2-oxo-1,2-dihydropyridin-3-yl)-N6-méthyl-2-(1-méthyl-1H-pyrazole-5-carboxamido)-5-oxohexanediamide |
| **II-154** | (S)-N1-(1-(2-((1S,2R,4R)-bicyclo[2.2.1]heptan-2-ylamino)-2-oxoéthyl)-2-oxo-1,2-dihydropyridin-3-yl)-N6-méthyl-2-(4-méthyl-2-(trifluorométhyl)thiazole-5-carboxamido)-5-oxohexanediamide |
| **II-155** | (S)-N1-(1-(2-((1S,2R,4R)-bicyclo[2.2.1]heptan-2-ylamino)-2-oxoéthyl)-2-oxo-1,2-dihydropyridin-3-yl)-2-(2,5-dichlorothiophène-3-carboxamido)-N6-méthyl-5-oxohexanediamide |
| **II-156** | (S)-N1-(1-(2-((1S,2R,4R)-bicyclo[2.2.1]heptan-2-ylamino)-2-oxoéthyl)-2-oxo-1,2-dihydropyridin-3-yl)-N6-méthyl-2-(4-méthyl-1,2,3-thiadiazole-5-carboxamido)-5-oxohexanediamide |
| **II-157** | (S)-N1-(1-(2-((1S,2R,4R)-bicyclo[2.2.1]heptan-2-ylamino)-2-oxoéthyl)-2-oxo-1,2-dihydropyridin-3-yl)-N6-méthyl-2-(1-méthyl-1H-1,2,3-triazole-5-carboxamido)-5-oxohexanediamide |
| **II-158** | (S)-N1-(1-(2-((1S,2R,4R)-bicyclo[2.2.1]heptan-2-ylamino)-2-oxoéthyl)-2-oxo-1,2-dihydropyridin-3-yl)-N6-méthyl-2-(1-méthyl-1H-pyrazole-5-carboxamido)-5-oxohexanediamide |
| **II-159** | (S)-N 1-méthyl-5-(4-méthyl-2-(trifluorométhyl)thiazole-5-carboxamido)-2-oxo-N6-(2-oxo-1-(2-oxo-2-((1R,2S,4R)-1,7,7-triméthylbicyclo[2.2.1]heptan-2-ylamino)éthyl)-1,2-dihydropyridin-3-yl)hexanediamide |
| **II-160** | (S)-2-(2,5-dichlorothiophène-3-carboxamido)-N6-méthyl-5-oxo-N1-(2-oxo-1-(2-oxo-2-((1R,2S,4R)-1,7,7-triméthylbicyclo[2.2.1]heptan-2-ylamino)éthyl)-1,2-dihydropyridin-3-yl)hexanediamide |
| **II-161** | (S)-N1-méthyl-5-(4-méthyl-1,2,3-thiadiazole-5-carboxamido)-2-oxo-N6-(2-oxo-1-(2-oxo-2-((1R,2S,4R)-1,7,7-triméthylbicyclo[2.2.1]heptan-2-ylamino)éthyl)-1,2-dihydropyridin-3-yl)hexanediamide |
| **II-162** | (S)-N1-méthyl-5-(1-méthyl-1H-1,2,3-triazole-5-carboxamido)-2-oxo-N6-(2-oxo-1-(2-oxo-2-((1R,2S,4R)-1,7,7-triméthylbicyclo[2.2.1]heptan-2-ylamino)éthyl)-1,2-dihydropyridin-3-yl)hexanediamide |
| **II-163** | (S)-N1-méthyl-5-(1-méthyl-1H-pyrazole-5-carboxamido)-2-oxo-N6-(2-oxo-1-(2-oxo-2-((1R,2S,4R)-1,7,7-triméthylbicyclo[2.2.1]heptan-2-ylamino)éthyl)-1,2-dihydropyridin-3-yl)hexanediamide |
| **II-164** | (2S)-2-(4-tert-butyl-1H-pyrrole-3-carboxamido)-N6-méthyl-N1-(1-(2-(1-adamantylamino)éthyl)-2-oxo-1,2-dihydropyridin-3-yl)-5-oxohexanediamide |
| **II-165** | (2S)-2-(4-cyano-1-méthyl-1H-pyrrole-2-carboxamido)-N6-méthyl-N1-(1-(3-(1-adamantylamino)propyl)-2-oxo-1,2-dihydropyridin-3-yl)-5-oxohexanediamide |
| **II-166** | (S)-N1-(1-(3-(2-adamantylamino)-3-oxopropyl)-2-oxo-1,2-dihydropyridin-3-yl)-2-(5-méthoxyoxazole-2-carboxamido)-N6-méthyl-5-oxohexanediamide |
| **II-167** | (S)-N1-(1-(2-(bicyclo[1.1.1]pentan-1-ylamino)-2-oxoéthyl)-2-oxo-1,2-dihydropyridin-3-yl)-N6-méthyl-2-(3-méthylbenzofuran-2-carboxamido)-5-oxohexanediamide |
| **II-168** | (S)-2-(2-acétyloxazole-4-carboxamido)-N1-(1-(2-(bicyclo[1.1.1]pentan-1-ylamino)-2-oxoéthyl)-2-oxo-1,2-dihydropyridin-3-yl)-N6-méthyl-5-oxohexanediamide |
| **II-169** | (S)-N1-(1-(2-(bicyclo[2.1.1]hexan-1-ylamino)-2-oxoéthyl)-2-oxo-1,2-dihydropyridin-3-yl)-N6-méthyl-2-(3-méthylbenzofuran-2-carboxamido)-5-oxohexanediamide |
| **II-170** | (S)-N1-(1-(2-(bicyclo[2.1.1]hexan-1-ylamino)-2-oxoéthyl)-2-oxo-1,2-dihydropyridin-3-yl)-2-(2-isopropyloxazole-5-carboxamido)-N6-méthyl-5-oxohexanediamide |
| II-171 | (2S)-2-(benzofuran-2-carboxamido)-N1-(1-(2-(bicyclo[3.2.1]octan-8-ylamino)-2-oxoéthyl)-2-oxo-1,2-dihydropyridin-3-yl)-N6-méthyl-5-oxohexanediamide |
| **II-172** | (2S)-N1-(1-(2-(bicyclo[3.2.1]octan-8-ylamino)-2-oxoéthyl)-2-oxo-1,2-dihydropyridin-3-yl)-2-(3,5-diméthylisoxazole-4-carboxamido)-N6-méthyl-5-oxohexanediamide |
| **II-173** | (S)-N 1 -(1 -(2-(5-carboxy-2-aminoadamantane)-2-oxoéthyl)-2-oxo-1,2-dihydropyridin-3-yl)-N6-méthyl-2-(4-méthylpyrimidine-5-carboxamido)-5-oxohexanediamide |
| **II-174** | (2S)-N 1-(1-(2-(4-aminoadamantane-N, N-diméthyl-1-carboxamide)-2-oxoéthyl)-2-oxo-1,2-dihydropyridin-3-yl)-N6-méthyl-5-oxo-2-(1,2,3,4-tétrahydronaphtalène-2-carboxamido)hexanediamide |
| **II-175** | (S)-2-((S)-1,4-diazabicyclo[2.2.2]octane-2-carboxamido)-N6-tert-butyl-N1-(1-(2-(2-adamantylamino)-2-oxoéthyl)-2-oxo-1,2-dihydropyridin-3-yl)-5-oxohexanediamide |
| **II-176** | (S)-N1-tert-butyl-5-(1H-indole-3-carboxamido)-N6-(1-(2-(2-adamantylamino)-2-oxoéthyl)-2-oxo-1,2-dihydropyridin-3-yl)-2-oxohexanediamide |
| **II-177** | (S)-N1-tert-butyl-N6-(1-(2-(2-adamantylamino)-2-oxoéthyl)-2-oxo-1,2-dihydropyridin-3-yl)-5-(6-méthylimidazo[2,1-b]thiazole-3-carboxamido)-2-oxohexanediamide |
| **II-178** | (S)-2-(benzo[d]thiazole-2-carboxamido)-N1-(1-(2-((1S,2R,4R)-bicyclo[2.2.1]heptan-2-ylamino)-2-oxoéthyl)-2-oxo-1,2-dihydropyridin-3-yl)-N6-cyclopentyl-5-oxohexanediamide |
| **II-179** | (S)-N1-(1-(2-((1S,2R,4R)-bicyclo[2.2.1]heptan-2-ylamino)-2-oxoéthyl)-2-oxo-1,2-dihydropyridin-3-yl)-N6-cyclopentyl-2-(imidazo[2,1-b]thiazole-6-carboxamido)-5-oxohexanediamide |
| **II-180** | (S)-N1-(1-(2-((1S,2R,4R)-bicyclo[2.2.1]heptan-2-ylamino)-2-oxoéthyl)-2-oxo-1,2-dihydropyridin-3-yl)-N6-cyclopentyl-2-(4-hydroxy-6-(trifluorométhoxy)quinoline-3-carboxamido)-5-oxohexanediamide |
| **II-181** | (S)-N1-(1-(2-(bicyclo[1.1.1]pentan-1-ylam ino)-2-oxoéthyl)-2-oxo-1,2-dihydropyridin-3-yl)-2-(cinnoline-3-carboxamido)-N6-cyclohexyl-5-oxohexanediamide |
| **II-182** | (S)-N1-(1-(2-(bicyclo[1.1.1]pentan-1-ylam ino)-2-oxoéthyl)-2-oxo-1,2-dihydropyridin-3-yl)-N6-cyclohexyl-2-(3-éthylbenzofuran-2-carboxamido)-5-oxohexanediamide |
| **II-183** | (S)-N1-(1-(2-(bicyclo[1.1.1]pentan-1-ylam ino)-2-oxoéthyl)-2-oxo-1,2-dihydropyridin-3-yl)-N6-cyclohexyl-2-(1-éthyl-1H-indole-2-carboxamido)-5-oxohexanediamide |
| **II-184** | (S)-N1-(1-(2-(bicyclo[1.1.1]pentan-1-ylam ino)-2-oxoéthyl)-2-oxo-1,2-dihydropyridin-3-yl)-N6-cyclohexyl-2-(2-méthyl-1,8-naphthyridine-3-carboxamido)-5-oxohexanediamide |
| **II-185** | (S)-N1-(1-(2-(bicyclo[2.1.1]hexan-1-ylamino)-2-oxoéthyl)-2-oxo-1,2-dihydropyridin-3-yl)-N6-méthyl-5-oxo-2-(1,2,3,4-tétrahydroquinoline-6-carboxamido)hexanediamide |
| **II-186** | (S)-N1-(1-(2-(2-carboxy-2-amino-5-(trifluorométhyl)adamantane)-2-oxoéthyl)-2-oxo-1,2-dihydropyridin-3-yl)-N6-méthyl-5-oxo-2-(3-oxo-1,2,3,4-tétrahydroisoquinoline-6-carboxamido)hexanediamide |
| **II-187** | (S)-N1-(1-(2-(5-éthyladamantane-2-amino)-2-oxoéthyl)-2-oxo-1,2-dihydropyridin-3-yl)-N6-méthyl-2-(1,6-naphthyridine-2-carboxamido)-5-oxohexanediamide |
| **II-188** | (S)-N1-(1-(2-(bicyclo[2.1.1]hexan-1-ylamino)-2-oxoéthyl)-2-oxo-1,2-dihydropyridin-3-yl)-N6-méthyl-2-(2,6-naphthyridine-1-carboxamido)-5-oxohexanediamide |
| **II-189** | (S)-2-(4-amino-1,2,5-oxadiazole-3-carboxamido)-N1-(1-(2-(bicyclo[1.1.1]pentan-1-ylamino)-2-oxoéthyl)-2-oxo-1,2-dihydropyridin-3-yl)-N6-méthyl-5-oxohexanediamide |
| **II-190** | (S)-N1-(1-(2-(bicyclo[1.1.1]pentan-1-ylam ino)-2-oxoéthyl)-2-oxo-1,2-dihydropyridin-3-yl)-2-(6-(diméthylamino)benzofuran-2-carboxamido)-N6-méthyl-5-oxohexanediamide |
| **II-191** | (S)-2-(2-acétamidothiazole-5-carboxamido)-N1-(1-(2-(bicyclo[1.1.1]pentan-1-ylamino)-2-oxoéthyl)-2-oxo-1,2-dihydropyridin-3-yl)-N6-méthyl-5-oxohexanediamide |
| **II-192** | (S)-N4-(1-(1-(2-(bicyclo[1.1.1]pentan-1-ylam ino)-2-oxoéthyl)-2-oxo-1,2-dihydropyridin-3-ylamino)-6-(méthylamino)-1,5,6-trioxohexan-2-yl)-1H-pyrrole-2,4-dicarboxamide |
| **II-193** | (S)-N1-(1-(2-(1-acétylamino-4-am inoadamantane)-2-oxoéthyl)-2-oxo-1,2-dihydropyridin-3-yl)-N6-méthyl-5-oxo-2-(5-sulfamoylfuran-3-carboxamido)hexanediamide |
| **II-194** | (S)-2-(benzofuran-5-carboxamido)-N1-(1-(2-(1-acétylamino-4-aminoadamantane)-2-oxoéthyl)-2-oxo-1,2-dihydropyridin-3-yl)-N6-méthyl-5-oxohexanediamide |
| **II-195** | (S)-2-(benzofuran-6-carboxamido)-N1-(1-(2-(4-aminoadamantane-1-carboxamide)-2-oxoéthyl)-2-oxo-1,2-dihydropyridin-3-yl)-N6-méthyl-5-oxohexanediamide |
| **II-196** | (S)-N1-(1-(2-(4-aminoadamantane-1-carboxamide)-2-oxoéthyl)-2-oxo-1,2-dihydropyridin-3-yl)-N6-méthyl-2-(3-(1-méthylcyclopropyl)-1,2,4-oxadiazole-5-carboxamido)-5-oxohexanediamide |
| **II-197** | (S)-N1-(1-(2-(bicyclo[1.1.1]pentan-1-ylam ino)-2-oxoéthyl)-2-oxo-1,2-dihydropyridin-3-yl)-N6-méthyl-2-(5-méthyl-1,2,4-oxadiazole-3-carboxamido)-5-oxohexanediamide |
| **II-198** | (S)-N1-(1-(2-(bicyclo[1.1.1]pentan-1-ylam ino)-2-oxoéthyl)-2-oxo-1,2-dihydropyridin-3-yl)-N6-méthyl-5-oxo-2-(1,2,3-thiadiazole-4-carboxamido)hexanediamide |
| **II-199** | (S)-N1-(1-(2-(bicyclo[1.1.1]pentan-1-ylamino)-2-oxoéthyl)-2-oxo-1,2-dihydropyridin-3-yl)-N6-méthyl-5-oxo-2-(1,2,4-thiadiazole-5-carboxamido)hexanediamide |
| **II-200** | (S)-N1-(1-(2-(bicyclo[1.1.1]pentan-1-ylamino)-2-oxoéthyl)-2-oxo-1,2-dihydropyridin-3-yl)-N6-méthyl-5-oxo-2-(1,3,4-thiadiazole-2-carboxamido)hexanediamide |
| **II-201** | (S)-N1-(1-(2-(bicyclo[1.1.1]pentan-1-ylamino)-2-oxoéthyl)-2-oxo-1,2-dihydropyridin-3-yl)-2-(4-cyclopropyl-1,2,3-thiadiazole-5-carboxamido)-N6-méthyl-5-oxohexanediamide |
| **II-202** | (S)-2-(4-cyclopropyl-1,2,3-thiadiazole-5-carboxamido)-N1-(1-(2-(2-adamantylamino)-2-oxoéthyl)-2-oxo-1,2-dihydropyridin-3-yl)-N6-méthyl-5-oxohexanediamide |
| **II-203** | (S)-2-(4-isopropyl-1,2,3-thiadiazole-5-carboxamido)-N1-(1-(2-(2-adamantylamino)-2-oxoéthyl)-2-oxo-1,2-dihydropyridin-3-yl)-N6-méthyl-5-oxohexanediamide |
| **II-204** | (S)-2-(4-éthyl-1,2, 3-thiadiazole-5-carboxamido)-N1-(1-(2-(2-adamantylamino)-2-oxoéthyl)-2-oxo-1,2-dihydropyridin-3-yl)-N6-méthyl-5-oxohexanediamide |
| **II-205** | (S)-2-(4-formyl-1,2,3-thiadiazole-5-carboxamido)-N1-(1-(2-(2-adamantylamino)-2-oxoéthyl)-2-oxo-1,2-dihydropyridin-3-yl)-N6-méthyl-5-oxohexanediamide |
| **II-206** | (S)-2-(4-hydroxyméthyl-1,2,3-thiadiazole-5-carboxamido)-N1-(1-(2-(2-adamantylamino)-2-oxoéthyl)-2-oxo-1,2-dihydropyridin-3-yl)-N6-méthyl-5-oxohexanediamide |
| **II-207** | (S)-N1-(1-(2-(1-adamantylamino)-2-oxoéthyl)-2-oxo-1,2-dihydropyridin-3-yl)-N6-méthyl-2-(1-méthyl-1H-imidazole-5-carboxamido)-5-oxohexanediamide |
| **II-208** | (S)-N1-(1-(2-(((1S,2R,5S)-6,6-diméthylbicyclo[3.1.1]heptan-2-yl)méthylamino)-2-oxoéthyl)-2-oxo-1,2-dihydropyridin-3-yl)-N6-méthyl-2-(1-méthyl-1H-imidazole-2-carboxamido)-5-oxohexanediamide |
| **II-209** | (S)-N1-(1-(2-(((1R,2R,5R)-6,6-diméthylbicyclo[3.1.1]heptan-2-yl)méthylamino)-2-oxoéthyl)-2-oxo-1,2-dihydropyridin-3-yl)-2-(1H-imidazole-4-carboxamido)-N6-méthyl-5-oxohexanediamide |
| **II-210** | (S)-N1-(1-(2-(3,5-diméthyladamantane-1-amino)-2-oxoéthyl)-2-oxo-1,2-dihydropyridin-3-yl)-N6-méthyl-2-(1-méthyl-1H-imidazole-5-carboxamido)-5-oxohexanediamide |
| **II-211** | (S)-N 1-(1-(2-(3,5,7-triméthyl-1-adamantylamino)-2-oxoéthyl)-2-oxo-1,2-dihydropyridin-3-yl)-N6-méthyl-2-(1-méthyl-1H-imidazole-5-carboxamido)-5-oxohexanediamide |
ou un sel de formule acceptable dans le domaine pharmaceutique.

10. Une composition pharmaceutique comprenant un composé conforme à l'une des revendications 1 à 9 en tant que principe actif, combiné à au moins un support, un excipient et/ou un diluant pharmaceutiquement acceptable.

11. Le composé conforme à l'une des revendications 1 à 9, destiné au domaine médical.

12. Le composé selon l'une des revendications 1 à 9 ou composition pharmaceutique conformément à la revendication 10 pour utilisation dans le traitement ou la prophylaxie de
maladies auto-immunes et inflammatoires, maladies vasculaires, maladies fibrotiques, maladies du foie, maladies cholestatiques du foie, cancers, maladies neurodégénératives, maladies oculaires et maladies de la peau.

13. Le composé ou la composition pharmaceutique à utiliser selon la revendication 12, où les maladies auto-immunes et inflammatoires comprennent la sclérose en plaques, la maladie coeliaque, la maladie de Duhring-Brocq (dermatite herpétiforme), l'ataxie au gluten, la neuropathie associée au gluten, le diabète, la polyarthrite rhumatoïde, la maladie de Graves, les maladies inflammatoires de l'intestin, le lupus érythémateux systémique, le psoriasis et la gingivite ; les maladies vasculaires comprennent l'athérosclérose, la thrombose, la rigidité vasculaire ; les maladies fibrotiques affectent les poumons, les reins, le foie, la peau ou l'intestin, comme la fibrose kystique, la fibrose rénale et la néphropathie diabétique, la fibrose intestinale, la fibrose pulmonaire idiopathique, la fibrose hépatique ; les maladies du foie comprennent l'hépatite alcoolique, la stéatohépatite alcoolique, la stéatohépatite non alcoolique, la cirrhose du foie, l'hépatite auto-immune ou l'inflammation du foie ; les maladies cholestatiques du foie comprennent la cholangite biliaire primitive et la cholangite sclérosante primitive ; les cancers comprennent le glioblastome, le mélanome, le cancer du pancréas, le carcinome rénal, le méningiome et le cancer du sein ; les maladies neurodégénératives comprennent la maladie de Parkinson, la chorée de Huntington ou la maladie d'Alzheimer ; les maladies oculaires comprennent le glaucome, la cataracte, la dégénérescence maculaire ou l'uvéite ; et les troubles cutanés comprennent l'acné, le psoriasis, la cicatrisation et le vieillissement cutané.

14. Le composé ou la composition pharmaceutique destiné(e) à une utilisation conforme à l'une des revendications 12 à 13 dans le traitement ou la prophylaxie de la maladie coeliaque.

15. Une méthode de production du composé de formule (**Ib**) conformément à la revendication 1 ou 4, comprenant :
**Étape 1B :** l'obtention d'un composé **4b** 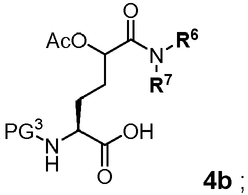
**Étape 2B** : une réaction de couplage du composé **4b** avec un composé 5 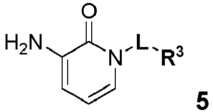 pour obtenir un composé **6b** 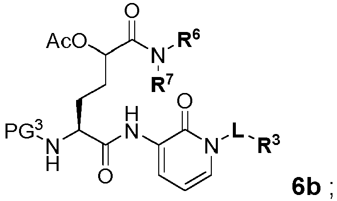
**Étape 3B :** la déprotection d'un groupe protecteur d'amines PG³ pour obtenir un composé **7b** 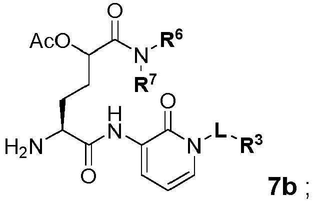
**Étape 4B** : une réaction de couplage du composé **7b** avec un acide carboxylique (**R²**-CO₂H**8**) pour obtenir un composé **9b** 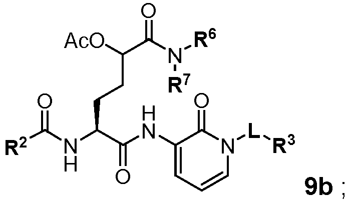
**Étape 5B** : une réaction d'oxydation du composé **9b** pour produire le composé de formule (**Ib**) 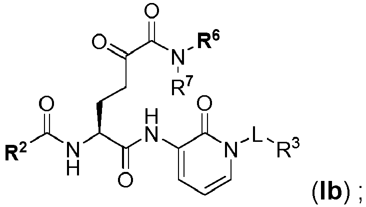
où **L, R², R³, R⁶ et R⁷** ont les mêmes significations que celles définies dans la revendication 1, et **PG³** est un groupe protecteur d'amines.
